(19) Europäisches Patentamt / European Patent Office / Office européen des brevets

(11) **EP 3 011 032 B1**

(12) **EUROPEAN PATENT SPECIFICATION**

(45) Date of publication and mention of the grant of the patent:
**16.10.2019 Bulletin 2019/42**

(51) Int Cl.:
*C12N 15/63* (2006.01)    *C12N 15/10* (2006.01)
*C12N 9/22* (2006.01)

(21) Application number: **14735795.8**

(22) Date of filing: **11.06.2014**

(86) International application number:
**PCT/US2014/041808**

(87) International publication number:
**WO 2014/204728 (24.12.2014 Gazette 2014/52)**

(54) **DELIVERY, ENGINEERING AND OPTIMIZATION OF SYSTEMS, METHODS AND COMPOSITIONS FOR TARGETING AND MODELING DISEASES AND DISORDERS OF POST MITOTIC CELLS**

FREISETZUNG, MANIPULATION UND OPTIMIERUNG VON SYSTEME, VERFAHREN UND ZUSAMMENSETZUNGEN FÜR TARGETING UND MODELLIERUNG VON ERKRANKUNGEN UND STÖRUNGEN POSTMITOTISCHER ZELLEN

DÉLIVRANCE, MODIFICATION ET OPTIMISATION DE SYSTÈMES, PROCÉDÉS ET COMPOSITIONS POUR CIBLER ET MODÉLISER DES MALADIES ET DES TROUBLES LIÉS AUX CELLULES POST-MITOTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2013 US 201361836123 P**
**17.07.2013 US 201361847537 P**
**05.08.2013 US 201361862355 P**
**28.08.2013 US 201361871301 P**
**12.12.2013 PCT/US2013/074667**
**12.12.2013 US 201361915203 P**
**15.04.2014 US 201461979573 P**

(43) Date of publication of application:
**27.04.2016 Bulletin 2016/17**

(60) Divisional application:
**19171576.2**

(73) Proprietors:
• **The Broad Institute, Inc.**
**Cambridge, MA 02142 (US)**
• **Massachusetts Institute of Technology**
**Cambridge, MA 02142 (US)**

(72) Inventors:
• **ZHANG, Feng**
**Cambridge, MA 02139 (US)**
• **HEIDENREICH, Matthias**
**Somerville, MA 02144 (US)**
• **SWIECH, Lukasz**
**Boston, MA 02120 (US)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**WO-A2-2013/082519     FR-A1- 2 872 170**
**US-A1- 2006 178 297**

• **L. CONG ET AL: "Multiplex Genome Engineering Using CRISPR/Cas Systems", SCIENCE, vol. 339, no. 6121, 15 February 2013 (2013-02-15), pages 819-823, XP055102030, ISSN: 0036-8075, DOI: 10.1126/science.1231143 & L. CONG ET AL: "Supplementary Material to : Multiplex Genome Engineering Using CRISPR/Cas Systems", SCIENCE, vol. 339, no. 6121, 3 January 2013 (2013-01-03), pages 819-823, XP055067744, ISSN: 0036-8075, DOI: 10.1126/science.1231143**
• **WANG HAOYI ET AL: "One-Step Generation of Mice Carrying Mutations in Multiple Genes by CRISPR/Cas-Mediated Genome Engineering", CELL, vol. 153, no. 4, 9 May 2013 (2013-05-09), pages 910-918, XP028538358, ISSN: 0092-8674, DOI: 10.1016/J.CELL.2013.04.025**

**(Cont. next page)**

- WOONG Y HWANG ET AL: "Efficient genome editing in zebrafish using a CRISPR-Cas system", NATURE BIOTECHNOLOGY, vol. 31, no. 3, 29 January 2013 (2013-01-29), pages 227-229, XP055086625, ISSN: 1087-0156, DOI: 10.1038/nbt.2501
- M. JINEK ET AL: "A Programmable Dual-RNA-Guided DNA Endonuclease in Adaptive Bacterial Immunity", SCIENCE, vol. 337, no. 6096, 17 August 2012 (2012-08-17), pages 816-821, XP055067740, ISSN: 0036-8075, DOI: 10.1126/science.1225829 & M. JINEK ET AL: "A Programmable Dual-RNA-Guided DNA Endonuclease in Adaptive Bacterial Immunity (Supplementary Material)", SCIENCE, vol. 337, no. 6096, 28 June 2012 (2012-06-28), pages 816-821, XP055067747, ISSN: 0036-8075, DOI: 10.1126/science.1225829
- G. GASIUNAS ET AL: "PNAS Plus: Cas9-crRNA ribonucleoprotein complex mediates specific DNA cleavage for adaptive immunity in bacteria", PROCEEDINGS OF THE NATIONAL ACADEMY OF SCIENCES, vol. 109, no. 39, 25 September 2012 (2012-09-25), pages E2579-E2586, XP055068588, ISSN: 0027-8424, DOI: 10.1073/pnas.1208507109
- JIANG WENYAN ET AL: "RNA-guided editing of bacterial genomes using CRISPR-Cas systems", NATURE BIOTECHNOLOGY,, vol. 31, no. 3, 29 January 2013 (2013-01-29), pages 233-239, XP002699849,
- BLAKE WIEDENHEFT ET AL: "RNA-guided genetic silencing systems in bacteria and archaea", NATURE, vol. 482, no. 7385, 15 February 2012 (2012-02-15), pages 331-338, XP055116249, ISSN: 0028-0836, DOI: 10.1038/nature10886
- ELITZA DELTCHEVA ET AL: "CRISPR RNA maturation by trans-encoded small RNA and host factor RNase III", NATURE, vol. 471, no. 7340, 31 March 2011 (2011-03-31), pages 602-607, XP055068535, ISSN: 0028-0836, DOI: 10.1038/nature09886
- MICHAEL P TERNS ET AL: "CRISPR-based adaptive immune systems", CURRENT OPINION IN MICROBIOLOGY, vol. 14, no. 3, 1 June 2011 (2011-06-01), pages 321-327, XP055097823, ISSN: 1369-5274, DOI: 10.1016/j.mib.2011.03.005
- GAJ THOMAS ET AL: "ZFN, TALEN, and CRISPR/Cas-based methods for genome engineering", TRENDS IN BIOTECHNOLOGY, vol. 31, no. 7, 1 July 2013 (2013-07-01), pages 397-405, XP028571313, ISSN: 0167-7799, DOI: 10.1016/J.TIBTECH.2013.04.004
- LUKE A. GILBERT ET AL: "CRISPR-Mediated Modular RNA-Guided Regulation of Transcription in Eukaryotes", CELL, vol. 154, no. 2, 1 July 2013 (2013-07-01), pages 442-451, XP055115843, ISSN: 0092-8674, DOI: 10.1016/j.cell.2013.06.044
- KRZYSZTOF CHYLINSKI ET AL: "The tracrRNA and Cas9 families of type II CRISPR-Cas immunity systems", RNA BIOLOGY, vol. 10, no. 5, 1 May 2013 (2013-05-01), pages 726-737, XP055116068, ISSN: 1547-6286, DOI: 10.4161/rna.24321
- INCONTRO SALVATORE ET AL: "Efficient, Complete Deletion of Synaptic Proteins using CRISPR.", NEURON, vol. 83, no. 5, 3 September 2014 (2014-09-03), pages 1051-1057, XP002730291, ISSN: 1097-4199
- STRAUB CHRISTOPH ET AL: "CRISPR/CAS9-MEDIATED GENE KNOCK-DOWN IN POST-MITOTIC NEURONS.", PLOS ONE, vol. 9, no. 8, E105584, August 2014 (2014-08), pages 1-5, XP002730292,
- BAENA-LOPEZ LUIS ALBERTO ET AL: "Accelerated homologous recombination and subsequent genome modification in Drosophila", DEVELOPMENT (CAMBRIDGE), vol. 140, no. 23, December 2013 (2013-12), pages 4818-4825, XP002730293, ISSN: 0950-1991

**Description**

FIELD OF THE INVENTION

The present invention generally relates to the delivery, engineering, optimization and therapeutic applications of systems, methods, and compositions used for the control of gene expression involving sequence targeting, such as genome perturbation or gene-editing, that relate to Clustered Regularly Interspaced Short Palindromic Repeats (CRISPR) and components thereof. In particular, the present invention relates to aspects related to delivery to post-mitotic cells, including but not limited to the brain or kidney, for gene therapy of conditions in said cells, understanding gene function in said cells and the creation of models comprising said cells.

BACKGROUND OF THE INVENTION

Recent advances in genome sequencing techniques and analysis methods have significantly accelerated the ability to catalog and map genetic factors associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications. Although genome-editing techniques such as designer zinc fingers, transcription activator-like effectors (TALEs), or homing meganucleases are available for producing targeted genome perturbations, there remains a need for new genome engineering technologies that are affordable, easy to set up, scalable, and amenable to targeting multiple positions within the eukaryotic genome.

SUMMARY OF THE INVENTION

The CRISPR-Cas system does not require the generation of customized proteins to target specific sequences but rather a single Cas enzyme can be programmed by a short RNA molecule to recognize a specific DNA target. Adding the CRISPR-Cas system to the repertoire of genome sequencing techniques and analysis methods may significantly simplify the methodology and accelerate the ability to catalog and map genetic factors associated with a diverse range of biological functions and diseases. To utilize the CRISPR-Cas system effectively for genome editing without deleterious effects, it is critical to understand aspects of engineering, optimization and cell-type/tissue/organ specific delivery of these genome engineering tools, which are aspects of the claimed invention.

There exists a pressing need for alternative and robust systems and techniques for nucleic sequence targeting with a wide array of applications. Aspects of this invention address this need and provide related advantages. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

In one aspect, the disclosure provides methods for using one or more elements of a CRISPR-Cas system. The CRISPR complex of the invention provides an effective means for modifying a target polynucleotide. The CRISPR complex of the invention has a wide variety of utilities including modifying (e.g., deleting, inserting, translocating, inactivating, activating) a target polynucleotide in a multiplicity of cell types in various tissues and organs. As such the CRISPR complex of the invention has a broad spectrum of applications in, e.g., gene or genome editing, gene therapy, drug discovery, drug screening, disease diagnosis, and prognosis. In vivo, in vitro and ex vivo uses are envisaged.

Aspects of the invention relate to Cas9 enzymes having improved post-mitotic cell targeting specificity in a CRISPR-Cas9 system having guide RNAs having optimal activity, smaller in length than wild-type Cas9 enzymes and nucleic acid molecules coding therefor, and chimeric Cas9 enzymes, as well as methods of improving the target specificity of a Cas9 enzyme or of designing a CRISPR-Cas9 system comprising designing or preparing guide RNAs having optimal activity and/or selecting or preparing a Cas9 enzyme having a smaller size or length than wild-type Cas9 whereby packaging a nucleic acid coding therefor into a delivery vector is more advanced as there is less coding therefor in the delivery vector than for wild-type Cas9, and/or generating chimeric Cas9 enzymes.

Also provided are uses of the present sequences, vectors, enzymes or systems, in medicine. Also provided are uses of the same in gene or genome editing. This is in relation to post-mitotic cell tissues or cells, whether in or ex vivo,

In an additional aspect of the invention, a Cas9 enzyme may comprise one or more mutations and may be used as a generic DNA binding protein with or without fusion to a functional domain. The mutations may be artificially introduced mutations or gain- or loss-of-function mutations. The mutations may include but are not limited to mutations in one of the catalytic domains (D10 and H840) in the RuvC and HNH catalytic domains, respectively. Further mutations have been characterized. In one aspect of the invention, the transcriptional activation domain may be VP64. In other aspects of the invention, the transcriptional repressor domain may be KRAB or SID4X. Other aspects of the invention relate to the mutated Cas 9 enzyme being fused to domains which include but are not limited to a transcriptional activator, repressor, a recombinase, a transposase, a histone remodeler, a demethylase, a DNA methyltransferase, a cryptochrome, a light inducible/controllable domain or a chemically inducible/controllable domain.

In a further embodiment, the disclosure provides for methods to generate mutant tracrRNA and direct repeat sequences or mutant chimeric guide sequences that allow for enhancing performance of these RNAs in cells. Aspects of the disclosure also provide for selection of said sequences.

Aspects of the disclosure also provide for methods of simplifying the cloning and delivery of components of the CRISPR complex. In the preferred embodiment of the disclosure, a suitable promoter, such as the U6 promoter, is amplified with a DNA oligo and added onto the guide RNA. The resulting PCR product can then be transfected into cells to drive expression of the guide RNA. Aspects of the disclosure also relate to the guide RNA being transcribed *in vitro* or ordered from a synthesis company and directly transfected.

In one aspect, the disclosure provides for methods to improve activity by using a more active polymerase. In a preferred embodiment, the expression of guide RNAs under the control of the T7 promoter is driven by the expression of the T7 polymerase in the cell. In an advantageous embodiment, the cell is a eukaryotic cell. In a preferred embodiment the eukaryotic cell is a human cell. In a more preferred embodiment the human cell is a patient specific cell.

In one aspect, the disclosure provides for methods of reducing the toxicity of Cas enzymes. In certain aspects, the Cas enzyme is any Cas9 as described herein, for instance any naturally-occurring bacterial Cas9 as well as any chimaeras, mutants, homologs or orthologs. In a preferred embodiment, the Cas9 is delivered into the cell in the form of mRNA. This allows for the transient expression of the enzyme thereby reducing toxicity. In another preferred embodiment, the disclosure also provides for methods of expressing Cas9 under the control of an inducible promoter, and the constructs used therein.

In another aspect, the disclosure provides for methods of improving the *in vivo* applications of the CRISPR-Cas system. In the preferred embodiment, the Cas enzyme is wildtype Cas9 or any of the modified versions described herein, including any naturally-occurring bacterial Cas9 as well as any chimaeras, mutants, homologs or orthologs. An advantageous aspect of the invention provides for the selection of Cas9 homologs that are easily packaged into viral vectors for delivery. Cas9 orthologs typically share the general organization of 3-4 RuvC domains and a HNH domain. The 5' most RuvC domain cleaves the non-complementary strand, and the HNH domain cleaves the complementary strand. All notations are in reference to the guide sequence.

The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue (D10) is mutated to alanine to convert Cas9 into a complementary-strand nicking enzyme. Similarly, the conserved His and Asn residues in the HNH domains are mutated to Alanine to convert Cas9 into a non-complementary-strand nicking enzyme. In some embodiments, both sets of mutations may be made, to convert Cas9 into a non-cutting enzyme.

In some embodiments, the CRISPR enzyme is a type I or III CRISPR enzyme, preferably a type II CRISPR enzyme. This type II CRISPR enzyme may be any Cas enzyme. A preferred Cas enzyme may be identified as Cas9 as this can refer to the general class of enzymes that share homology to the biggest nuclease with multiple nuclease domains from the type II CRISPR system. Most preferably, the Cas9 enzyme is from, or is derived from, spCas9 or saCas9. By derived, Applicants mean that the derived enzyme is largely based, in the sense of having a high degree of sequence homology with, a wildtype enzyme, but that it has been mutated (modified) in some way as described herein.

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes.* However, it will be appreciated that this invention includes many more Cas9s from other species of microbes, such as SpCas9, SaCas9, St1Cas9 and so forth. Further examples are provided herein. The skilled person will be able to determine appropriate corresponding residues in Cas9 enzymes other than SpCas9 by comparison of the relevant amino acid sequences. Thus, where a specific amino acid replacement is referred to using the SpCas9 numbering, then, unless the context makes it apparent this is not intended to refer to other Cas9 enzymes, the disclosure is intended to encompass corresponding modifications in other Cas9 enzymes. SaCas9 is particularly advantageous.

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species is known.

In further embodiments, the disclosure

provides for methods of enhancing the function of Cas9 by generating chimeric Cas9 proteins. Chimeric Cas9 proteins chimeric Cas9s may be new Cas9 containing fragments from more than one naturally occurring Cas9. These methods may comprise fusing N-terminal fragments of one Cas9 homolog with C-terminal fragments of another Cas9 homolog. These methods also allow for the selection of new properties displayed by the chimeric Cas9 proteins.

It will be appreciated that in the present methods, where the organism is an animal or a plant, the modification may occur *ex vivo* or *in vitro,* for instance in a cell culture and in some instances not *in vivo.* In other embodiments, it may occur *in vivo.*

In one aspect, the invention provides a method of modifying an organism or a non-human organism by manipulation of a target sequence in a genomic locus of interest comprising: delivering a non-naturally occurring or engineered composition comprising :

  A) -

    I. a CRISPR-Cas system RNA polynucleotide sequence, optionally a chimeric RNA (chiRNA) polynucleotide sequence, wherein the polynucleotide sequence comprises:

      (a) a guide sequence capable of hybridizing to a target sequence in a eukaryotic cell,
      (b) a tracr mate sequence, and
      (c) a tracr sequence, and

    II. a polynucleotide sequence encoding a CRISPR enzyme comprising at least one or more nuclear localization sequences,

  wherein (a), (b) and (c) are arranged in a 5' to 3' orientation,
  wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence and the polynucleotide sequence encoding a CRISPR enzyme is DNA or RNA,
  or
  (B)

    I. polynucleotides comprising:

      (a) a guide sequence capable of hybridizing to a target sequence in a eukaryotic cell, and
      (b) at least one or more tracr mate sequences,

    II. a polynucleotide sequence encoding a CRISPR enzyme, and
    III. a polynucleotide sequence comprising a tracr sequence,

  wherein when transcribed, the tracr mate sequence hybridizes to the tracr sequence and the guide sequence directs sequence-specific binding of a CRISPR complex to the target sequence, and
  wherein the CRISPR complex comprises the CRISPR enzyme complexed with (1) the guide sequence that is hybridized to the target sequence, and (2) the tracr mate sequence that is hybridized to the tracr sequence, and the polynucleotide sequence encoding a CRISPR enzyme is DNA or RNA.

The following applies equally to all aspects of the invention. The target sequence is most preferably a post-mitotic cell target sequence. Where liver may be mentioned herein, this is will understood to be reference to post-mitotic cells in general, especially kidney or brain cells. The post-mitotic cell may be in or from (i.e. the source of the cells or the cell type) any one of the following organs or may be organoids or *ex vivo* models or collections of cells comprising cells of the:

  Kidney, such as glomerulus cells;

Digestive System including the stomach, pancreas, duodenum, ileum and/or colon;

Heart;

Lung;

Brain, in particular neurones, and/or CNS in general;

Eye, including retinal tissue;

Ear, including the inner ear;

Skin;

Muscle;

Bone; and/or

Liver in general, although this is excluded in some embodiments as it is also the subject of a separate application.

Brain and Kidney are particularly preferred. In some embodiments, the cell is a brain cell, such as a neurone. In some embodiments, the cell is a kidney cell.

Preferred kidney cells in clude any one or more of:

- Kidney glomerulus parietal cell;
- Kidney glomerulus podocyte;
- Kidney proximal tubule brush border cell;
- Loop of Henle thin segment cell;
- Thick ascending limb cell;
- Kidney distal tubule cell;
- Kidney collecting duct cell; and
- Interstitial kidney cells.

Preferred exaples of kidney targets are provided in the table below uner the section entitled kidney, as well as in Table B. Any one or more of these targets is preferred. Examples 1 and 18 also target Kidney cells (albeit stem cels, which are not post-mitotic cells), but the teaching re delivery may be applicable.

In some particularly preferred embodiments, manipulation invokes a phenotypic change in the cell.

In some embodiments, the phenotypic change may be invoked in or maintained in the cell *in vivo.* Either the cell is transfected in vivo or is extracted, transfected ex vivo and then reinserted (transplanted) back into the same or a different host.

Expression of the CRISPR enzyme, and optionally the guide sequence, may be under the control of a promoter specific for the post-mitotic cell, for instance comprised within an expression cassette capable of expressing the enzyme and the optional guide in said post-mitotic cell. In other words, the CRISPR enzyme, and optionally the guide sequence, is/are operably linked to said promoter specific for the post-mitotic cell. AAV vector systems are particularly preferred, especially where the post-mitotic cell is a a neurone.

The promoter for the CRISPR enzyme and the optional promoter for the guide sequence may be the same or different.

The following also applies to any method, use or composition describe herein. The CRISPR-Cas system RNA may be a chimeric RNA (chiRNA). The CRISPR-Cas system may be a multiplexed CRISPR enzyme system further comprising multiple chimeras and/or multiple multiguide sequences and a single tracr sequence. The CRISPR enzyme may be a nuclease directing cleavage of both strands at the location of the target sequence. The CRISPR enzyme may comprise one or more mutations. The CRISPR enzyme may comprise one or more mutations D10A, E762A, H840A, N854A, N863A or D986A. The one or more mutations may be in a RuvCI domain of the CRISPR enzyme. The CRISPR enzyme may be a nickase directing cleavage at the location of the target sequence. The nickase may be a double nickase. At least two or more NLS are preferred.

The CRISPR enzyme may be type II, preferably a Cas and most preferably a Cas9. Reference to Cas or Cas9 (for instance in CRISPR-Cas or CRISR Cas9) will be understood to be any Cas, most preferably Cas9 and particularly Sa or Sp Cas9 (encompassing all mutations such as D10A to provided DSB, nickase or dual nickase function).

The CRISPR enzyme may have one or more mutations in a catalytic domain, wherein when transcribed, the tracr mate sequence hybridizes to the tracr sequence and the guide sequence directs sequence-specific binding of a CRISPR complex to the target sequence, and wherein the enzyme further comprises a functional domain. The functional domain may be a transcriptional activation domain. The transcriptional activation domain may be VP64.

The methods may further comprise minimizing off-target modifications by manipulation of a first and a second target sequence on opposite strands of a DNA duplex in a genomic locus of interest in a cell comprising delivering a non-naturally occurring or engineered composition comprising:

I. a CRISPR-Cas system chimeric RNA (chiRNA) polynucleotide sequence, wherein the polynucleotide sequence comprises:

(a) a first guide sequence capable of hybridizing to the first target sequence,
(b) a first tracr mate sequence,
(c) a first tracr sequence,
(d) a second guide sequence capable of hybridizing to the second target sequence,
(e) a second tracr mate sequence, and
(f) a second tracr sequence, and

optionally, wherein a linker sequence is present between the first tracr sequence and the second guide sequence, whereby the first guide sequence and the second guide sequence are in tandem; and
II. a polynucleotide sequence encoding a CRISPR enzyme comprising at least one or more nuclear localization sequences, wherein (a), (b), (c), (d), (e) and (f) are arranged in a 5' to 3' orientation, wherein the polynucleotide sequence comprises a linker sequence between the first tracr sequence and the second guide sequence, whereby the first guide sequence and the second guide sequence are in tandem, and wherein when transcribed, the first and the second tracr mate sequence hybridize to the first and second tracr sequence respectively and the first and the second guide sequence directs sequence-specific binding of a first and a second CRISPR complex to the first and second target sequences respectively,
or
II. a second regulatory element operably linked to an enzyme-coding sequence encoding a CRISPR enzyme, and wherein components I and II are located on the same or different vectors of the system, and when transcribed, a first tracr mate sequence hybridizes to a first tracr sequence and the first and the second guide sequence directs sequence-specific binding of a first and a second CRISPR complex to the first and second target sequences respectively;
wherein the first CRISPR complex comprises the CRISPR enzyme complexed with (1) the first guide sequence that is hybridized to the first target sequence, and (2) the first tracr mate sequence that is hybridized to the first tracr sequence,
wherein the second CRISPR complex comprises the CRISPR enzyme complexed with (1) the second guide sequence that is hybridized to the second target sequence, and (2) the second tracr mate sequence that is hybridized to the second tracr sequence,
wherein the polynucleotide sequence encoding a CRISPR enzyme is DNA or RNA, and
wherein the first guide sequence directs cleavage of one strand of the DNA duplex near the first target sequence and the second guide sequence directs cleavage of other strand near the second target sequence inducing a double strand break, thereby modifying the organism or the non-human organism by minimizing off-target modifications.

In some embodiments, the second alternative above (B) is preferred. The first alternative (A) is particularly preferred, however. This applies to all aspects of the invention featuring the two alternative CRISPR approaches.

It will be appreciated that the present application is directed to post-mitotic cells, whether that is an organ per se or a tissue within it or simply one or post-mitotic cells, such as neurones. Neurones and kidney cells are preferred. The post-mitotic cells may be comprised within a vertebrate animal, either a patient (in the sense of an animal in need of CRISPR-directed gene therapy) or a model organism, or may be in cell culture , an organoid or other ex vivo tissue, such a "liver on a chip" for instance where hepatocytes are seeded and grown on a scaffold. Harvested hepatocytes from un-transplanted organs are also a useful target. With the development of 3-D printing techniques being applied to biology, printed tissues are within grasp and it is entirely feasible that liver cells or tissues printed in this way to create an organoid or onto a chip could also be targeted. Non-liver alternatives are also envisaged, particularly for kidney tissues or other post-mitotic cells/tissues.

Thus, provided is a model organism comprising post-mitotic cells, such as neurones or kidney cells, to which the present CRISPR-Cas system has been delivered. Similarly, also provided is an *ex vivo* collection of two or more post-mitotic cells, such as neurones or kidney cells, to which the present CRISPR-Cas system has been delivered. Such collections may include post-mitotic organs, organoids, cells populating a scaffold ('kidney on a chip'). Methods of creating such models or collections are also provided.

In particular, such post-mitotic cells may express, or comprise polynucleotides capable of expressing, a Cas enzyme. As discussed herein, this has the advantage of providing a ready model for interrogating gene function through gene perturbation, including knock down. This is particularly useful in studying conditions of the post-mitotic cells, such as the kidney or brain, such as those listed herein, as well as broader conditions such as obesity.

Methods of interrogating post-mitotic cell gene function are also provided herein. These typically comprise delivering to post-mitotic cells, either *in* or *ex vivo,* the CRISPR-Cas system. However, if the cells already comprise Cas, whether expressed as a protein or encoded by polynucleotides already comprised within the cells, then only the CRISPR polynucleotide needs to be delivered. The method may include extraction from and, optionally, re-insertion back into the post-mitotic cell. By delivering, it is meant actually physical delivery of the polynucleotides to the nucleus of the cell, but also transfection. Therefore, delivery should also be read as including transfection unless otherwise apparent.

Also provided is a method of inducing gene perturbation in one or more post-mitotic cells, comprising transducing a first population of cells with a CRISPR-Cas system
to thereby alter the genome of the first population of cells to obtain a
second population of cells. The method may be *ex vivo* or *in vitro,* for instance in a cell culture or in an *ex vivo* or *in vitro* model (such as an organoid or 'post-mitotic cell on a chip'). Alternatively, the method may be *in vivo,* in which case it may also include isolating the first
population of cells from the subject, and transplanting the second population of cells (back) into the subject. Gene perturbation may be for one or more, or two or more, or three or more, or four or more genes. The gene perturbation may be a reduction in gene function (i.e. activity in the encoded gene product). This may be, for instance, induced through alteration of the genome of the first population of cells to obtain the second population of cells, wherein the second population of cells has a defective genotype, such as a monogenic condition, which is absent in the first population of cells. This may require a corresponding repair template , as discussed herein, to provide the defective sequence or it may be through induction of a DSB. In particular, the gene perturbation is a gene knockdown. In some embodiments, the post-mitotic cell is most preferably a kidney or brain (neuron) cell or a liver cell, such as a primary hepatocyte.

Alternatively, the gene perturbation may be an increase in gene function (i.e. activity in the encoded gene product). This may be, for instance, induced through alteration of the genome of the first population of cells to obtain the second population of cells, wherein the first population of cells has a defective genotype, such as a monogenic condition, which is absent in (i.e. corrected for in) the second population of cells. This may require a corresponding repair template, as discussed herein, to provide the corrected sequence.

If multiplexing is used, then a mixture of reduction of one or more genes and increase of one or more genes is envisaged. This may be achieved through provision of one or more of the guides (in the multiplex) and corresponding repair templates may be used to reduce function, whilst one or more of the guides and their corresponding templates may be used to increase function.

Also provided is a method of interrogating function of one or more genes in one or more post-mitotic cells, comprising determining changes in expression of the one or more genes in the first populations of post-mitotic cells, inducing said gene perturbation in said first population to provide said second population with an altered genome (or genotype), and determining changes in expression of the one or more genes in the second population of post-mitotic cells, thereby interrogating the function of the one or more genes. In some embodiments, the post-mitotic cell is most preferably a kidney or brain (neuron) cell or a liver cell, such as a primary hepatocyte.

Also provided is a model and a method of creating said model. The model may be an animal comprising a post-mitotic cell (an *in vivo* model) or it may be an *ex vivo* or *in vitro* model, such as a post-mitotic organoid or 'post-mitotic cell on a chip' or the collection of post-mitotic cells, such as a on a scaffold, as described herein. The post-mitotic cells of either model will preferably be transfected with Cas9. Accordingly, there is specifically provided a model comprising one or more post-mitotic cells comprising the CRISPR enzyme, preferably a Cas9 such as Sa or SpCas9. The model cells may have been transfected or transduced with the second regulatory element provided herein, which is second regulatory element operably linked to an enzyme-coding sequence encoding a CRISPR enzyme comprising at list one or more nuclear localization sequences (NLSs). The model may be, as described above, an *in vivo* model or it may be an *ex vivo* or *in vitro* model. Such a model allows rapid interrogation of function of one or more genes, as only the CRISPR-Cas system polynucleotide sequence (comprising one or more guide sequences targeting said one or more genes) needs to be delivered to perturb the function of said gene. In other words, methods of interrogating gene function in such models may comprise only delivery of the CRISPR-Cas system polynucleotide sequence (comprising the one or more guide sequences), the Cas (CRISPR enzyme) having already been provided in the cell(s) of the model. Methods of creating such models are also provided, comprising transducing or transfecting one or more post-mitotic cells in a first population of post-mitotic cells with a second regulatory element operably linked to an enzyme-coding sequence encoding a CRISPR enzyme comprising at list one or more nuclear localization sequences (NLSs) as described herein to thereby provide one or more second population of post-mitotic cells comprising or expressing the CRISPR enzyme. In some embodiments, the post-mitotic cell is most preferably a kidney or brain (neuron) cell or a liver cell, such as a primary hepatocyte.

Methods of creating gene perturbed models, in particular gene knock down models, are also provided. These methods may typically comprise inducing gene perturbation in one or more genes, as described herein, in a first population of cells to thereby provide a second population of cells with an altered genome (or genotype). The second population of cells may then be seeded into a scaffold or onto a chip, for instance, to thereby provide an *ex vivo* or *in vitro* model. Alternatively, the second population of may be comprised within an *in vivo* animal.

Methods of gene therapy are also envisaged. For instance, correction of one or more deficient genotypes (for example single point mutations) is achievable through the use of the present CRISPR-Cas system in the post-mitotic cells discussed herein (including the models). Monogenic conditions associated with the post-mitotic are particularly preferred and are exemplified herein, see Example 36 where the CRISPR-Cas9 system target was ApoB, a lipid metabolism gene, was effective at inducing a phenotypic change in vivo. Example 38 is also instructive in relation to phenotypic behaviour changes seen in vivo in the brain of mice transduced with the present system. Compositions for use in gene therapy are also provided.

Although various Cas enzymes are envisaged, Cas9 is particularly preferred and we have shown particular efficacy in the liver for SaCa9. Tracr sequence from Sa is also preferred if the Cas enzyme is an Sa Cas enzyme. A suitable PAM in such circumstance is NNGRR.

Although one guide may be used, so-called multiplexing with two, three, four or more guides, is particularly useful in interrogation of gene function and model creation (to provide multiple gene knock downs), but also in gene therapy where multiple defective genotypes are to be corrected (either multiple errors in a single gene or, more likely, multiple errors spread across several genes). Alternatively, multiplexing with two guides is useful in a dual nickase approach to reduce off-target effects or simply selection of multiple targets within one gene to ensure Cas recruitment. Triple and quadruple guides are preferred. Reference to gene herein is made interchangeably with genomic locus.

The intron approach described here is also useful in this regard, where the guide is positioned within the Cas intron.

Preferred means of delivery include the methods described by Kanasty below, such as LNP, especially where only the guide is to be delivered or it is to be delivered alone. However, viral vectors including lentiviral and AAV are generally preferred for the liver as they have been successful to date. Of these, AAV is preferred and especially serotype 8, with AAV2/8 shown to be effective. Some preferred targets, to the extent that they are present in or conditions of the kidney are metabolic disorders, such as any one of: Amyloid neuropathy (TTR, PALB); Amyloidosis (APOA1, APP, AAA, CVAP, AD1, GSN, FGA, LYZ, TTR, PALB); Cirrhosis (KRT18, KRT8, CIRH1A, NAIC, TEX292, KIAA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD1, PKD2, PKD4, PKDTS, PRKCSH, G19P1, PCLD, SEC63). Other preffered targets include any one or more of: PCSK9, HMGCR, APOB, LDLR, ANGPTL3, F8, F9/FIX, AAT, FAH, HPD, TAT, ATP7B, UGT1A1, OTC, ARH.

It will be appreciated that methods of altering expression in the post-mitotic cell do not involve alteration of the germline, which may be excluded on moral grounds. In fact, although transfection of stem cells is envisaged and certainly preferred in some embodiments, neurones or kidney cells are particularly preferred, particularly where they may show or be stimulated to show some regeneration.

Type II CRISPRS are particularly preferred, especially for use in eukaryotes, as in the present case, where livers are only found in eukaryotes, particularly vertebrate animals, in any case.

Use of the CRISPR-Cas systems to invoke a phenotypic change is a particular advantage, especially *in vivo.* We have shown this in the present application.

Where therapeutic applications are envisaged, or for other genome engineering in the post-mitotic cells, then where a correction is required it will be appreciated that following nicking or cleavage of the genomic DNA target, then correction via the HDR pathway is preferred. For gene knockdown, NHEJ is advantageous, however, correction via the HDR pathway is preferred for therapy. In such circumstances, it is preferable to deliver a repair template. This is most preferably ssDNA although RNA via a retroviral vector to provide a corresponding DNA template is also possible. The skilled person can readily put the invention into practice from the herein teachings contributing to the knowledge in the art; and in this regard mention is made that the skilled person from the herein teachings contributing to the knowledge in the art can readily appreciate and implement considerations as to homologous arm length. Mention is made of patent applications and publications including herein inventor Zhang, including those cited herein. The repair template is preferably co-delivered with one or more elements of the CRISPR-Cas system.

Also provided is a method of altering expression of at least one post-mitotic cell gene product comprising introducing into a eukaryotic liver cell, for example a hepatocyte, containing and expressing a DNA molecule having a target sequence and encoding the gene product, an engineered, non-naturally occurring Clustered Regularly Interspaced Short Palindromic Repeats (CRISPR)-CRISPR associated (Cas) (CRISPR-Cas) system comprising one or more vectors comprising:

a) a first regulatory element operable in a eukaryotic cell operably linked to at least one nucleotide sequence encoding a CRISPR-Cas system guide RNA that hybridizes with the target sequence, and
b) a second regulatory element operable in a eukaryotic cell operably linked to a nucleotide sequence encoding a Type-II Cas9 protein,

wherein components (a) and (b) are located on same or different vectors of the system, whereby the guide RNA targets the target sequence and the Cas9 protein cleaves the DNA molecule, whereby expression of the at least one post-mitotic cell gene product is altered; and, wherein the Cas9 protein and the guide RNA do not naturally occur together. Reference below to targets will be understood to be post-mitotic cell targets or genes otherwise expressed in the post-mitotic cell unless otherwise apparent

Any or all of the polynucleotide sequence encoding a CRISPR enzyme, guide sequence, tracr mate sequence or tracr sequence, may be RNA. The polynucleotides encoding the sequence encoding a CRISPR enzyme, the guide sequence, tracr mate sequence or tracr sequence may be RNA and may be delivered via liposomes, nanoparticles, exosomes, microvesicles, or a gene-gun.

It will be appreciated that where reference is made to a polynucleotide, which is RNA and is said to 'comprise' a feature such a tracr mate sequence, the RNA sequence includes the feature. Where the polynucleotide is DNA and is said to comprise a feature such a tracr mate sequence, the DNA sequence is or can be transcribed into the RNA including the feature at issue. Where the feature is a protein, such as the CRISPR enzyme, the DNA or RNA sequence referred to is, or can be, translated (and in the case of DNA transcribed first).

Accordingly, in certain embodiments the disclosure
provides a method of modifying
post-mitotic cells of an organism, e.g., mammal including human or a non-human mammal or organism by manipulation of a target sequence in a genomic locus of interest comprising delivering a non-naturally occurring or engineered composition comprising a viral or plasmid vector system comprising one or more viral or plasmid vectors operably encoding a composition for expression thereof, wherein the composition comprises: (A) a non-naturally occurring or engineered composition comprising a vector system comprising one or more vectors comprising

I. a first regulatory element operably linked to a CRISPR-Cas system chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence, and II. a second regulatory element operably linked to an enzyme-coding sequence encoding a CRISPR enzyme comprising at least one or more nuclear localization sequences (or optionally at least one or more nuclear localization sequences as some embodiments can involve no NLS), wherein (a), (b) and (c) are arranged in a 5' to 3' orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence, or (B) a non-naturally occurring or engineered composition comprising a vector system comprising one or more vectors comprising I. a first regulatory element operably linked to (a) a guide sequence capable of hybridizing to a target sequence in a eukaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence. In some embodiments, components I, II and III are located on the same vector. In other embodiments, components I and II are located on the same vector, while component III is located on another vector. In other embodiments, components I and III are located on the same vector, while component II is located on another vector. In other embodiments, components II and III are located on the same vector, while component I is located on another vector. In other embodiments, each of components I, II and III is located on different vectors. The discosure
also
provides a viral or plasmid vector system as described herein.

Preferably, the vector is a viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided. In some embodiments, one or more of the viral or plasmid vectors may be delivered via liposomes, nanoparticles, exosomes, microvesicles, or a gene-gun.

By manipulation of a target sequence, Applicants also mean the epigenetic manipulation of a target sequence. This may be of the chromatin state of a target sequence, such as by modification of the methylation state of the target sequence (i.e. addition or removal of methylation or methylation patterns or CpG islands), histone modification, increasing or reducing accessibility to the target sequence, or by promoting 3D folding.

It will be appreciated that where reference is made to a method of modifying an organism or mammal including human or a non-human mammal or organism by manipulation of a target sequence in a genomic locus of interest, this may apply to the organism (or mammal) as a whole or just a single cell or population of cells from that organism (if the organism is multicellular). In the case of humans, for instance, Applicants envisage, *inter alia,* a single cell or a population of cells and these may preferably be modified *ex vivo* and then re-introduced. In this case, a biopsy or other tissue or biological fluid sample may be necessary. Stem cells are also particularly preferred in this regard. But, of course, *in vivo* embodiments are also envisaged.

In certain embodiments the disclosure
provides a method of treating or inhibiting a
condition caused by a defect in a target sequence in a genomic locus of interest in a subject (e.g., mammal or human) or a non-human subject (e.g., mammal) in need thereof comprising modifying the subject or a non-human subject by manipulation of the target sequence and wherein the condition is susceptible to treatment or inhibition by manipulation of the target sequence comprising providing treatment comprising: delivering a non-naturally occurring or engineered composition comprising an AAV or lentivirus vector system comprising one or more AAV or lentivirus vectors operably encoding a composition for expression thereof, wherein the target sequence is manipulated by the composition when expressed, wherein the composition comprises: (A) a non-naturally occurring or engineered composition comprising a vector system comprising one or more vectors comprising I. a first regulatory element operably linked to a CRISPR-Cas system chimeric RNA (chiRNA) polynucleotide sequence, wherein the polynucleotide sequence comprises (a) a guide sequence capable of hybridizing to a target
sequence in a eukaryotic cell, (b) a tracr mate sequence, and (c) a tracr sequence, and II. a second regulatory element operably linked to an enzyme-coding sequence encoding a CRISPR enzyme comprising at least one or more nuclear localization sequences (or optionally at least one or more nuclear localization sequences as some embodiments can involve no NLS) wherein (a), (b) and (c) are arranged in a 5' to 3' orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence, or (B) a non-naturally occurring or engineered composition comprising a vector system comprising one or more vectors comprising I. a first regulatory element operably linked to (a) a guide sequence capable of hybridizing to a target sequence in a eukaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence. In some embodiments, components I, II and III are located on the same vector. In other embodiments, components I and II are located on the same vector, while component III is located on another vector. In other embodiments, components I and III are located on the same vector, while component II is located on another vector. In other embodiments, components II and III are located on the same vector, while component I is located on another vector. In other embodiments, each of components I, II and III is located on different vectors. The disclosure also provides a viral (e.g. AAV or lentivirus) vector system as
described herein. , and can be part of a vector system as described herein.

Some methods of the invention can include inducing expression. The organism or subject is a eukaryote (including mammal including human) or a non-human eukaryote or a non-human animal or a non-human mammal, provided it has post-mitotic cell (e.g. the brain or kidney) and corresponding function. In some embodiments, the organism or subject is a non-human animal, and may be an arthropod, for example, an insect, or may be a nematode. In some methods of the invention the organism or subject is a mammal or a non-human mammal. A non-human mammal may be for example a rodent (preferably a mouse or a rat), an ungulate, or a primate. In some methods of the invention the viral vector is an AAV or a lentivirus, and can be part of a vector system as described herein. In some methods of the invention the CRISPR enzyme is a Cas9. In some methods of the invention the expression of the guide sequence is under the control of the T7 promoter and is driven by the expression of T7 polymerase.

The disclosure
in some embodiments comprehends a method of delivering a CRISPR
enzyme comprising delivering to a cell mRNA encoding the CRISPR enzyme. In some of these methods the CRISPR enzyme is a Cas9.

The disclosure
also provides methods of preparing the vector systems of the disclosure,
in particular the viral vector systems as described herein. The discosure
in some embodiments

comprehends a method of preparing the AAV comprising transfecting plasmid(s) containing or consisting essentially of nucleic acid molecule(s) coding for the AAV into AAV-infected cells, and supplying AAV rep and/or cap obligatory for replication and packaging of the AAV. In some embodiments the AAV rep and/or cap obligatory for replication and packaging of the AAV are supplied by transfecting the cells with helper plasmid(s) or helper virus(es). In some embodiments the helper virus is a poxvirus, adenovirus, herpesvirus or baculovirus. In some embodiments the poxvirus is a vaccinia virus. In some embodiments the cells are mammalian cells. And in some embodiments the cells are insect cells and the helper virus is baculovirus. In other embodiments, the virus is a lentivirus.

The invention further comprehends a composition of the invention or a CRISPR enzyme thereof (including or alternatively mRNA encoding the CRISPR enzyme) for use in medicine or in therapy. In some embodiments the invention comprehends a composition according to the invention or a CRISPR enzyme thereof (including or alternatively mRNA encoding the CRISPR enzyme) for use in a method according to the invention. In some embodiments the invention provides for the use of a composition of the invention or a CRISPR enzyme thereof (including or alternatively mRNA encoding the CRISPR enzyme) in *ex vivo* gene or genome editing. In certain embodiments the invention comprehends use of a composition of the invention or a CRISPR enzyme thereof (including or alternatively mRNA encoding the CRISPR enzyme) in the manufacture of a medicament for *ex vivo* gene or genome editing or for use in a method according of the invention. The invention comprehends in some embodiments a composition of the invention or a CRISPR enzyme thereof (including or alternatively mRNA encoding the CRISPR enzyme), wherein the target sequence is flanked at its 3' end by a PAM (protospacer adjacent motif) sequence comprising 5'-motif, especially where the Cas9 is (or is derived from) *S. pyogenes* or *S. aureus* Cas9. For example, a suitable PAM is 5'-NRG or 5'-NNGRR (where N is any Nucleotide) for SpCas9 or SaCas9 enzymes (or derived enzymes), respectively, as mentioned below.

It will be appreciated that SpCas9 or SaCas9 are those from or derived from *S. pyogenes* or *S. aureus* Cas9. It may of course, be mutated or otherwise changed from the wild type to suit the intended use, as described herein. The dual nickase D10A mutant or variant is preferred, especially in combination with two overlapping guides directed as opposing sites on differing strands of the same chromosome.

Apects of the invention comprehend improving the specificity of a CRISPR enzyme, e.g. Cas9, mediated gene targeting and reducing the likelihood of off-target modification by the CRISPR enzyme, e.g. Cas9. The invention in some embodiments comprehends a method of modifying an organism or a non-human organism by minimizing off-target modifications by manipulation of a first and a second target sequence on opposite strands of a DNA duplex in a genomic locus of interest in a cell comprising delivering a non-naturally occurring or engineered composition comprising:

I. a first CRISPR-Cas system chimeric RNA (chiRNA) polynucleotide sequence, wherein the first polynucleotide sequence comprises:

> (a) a first guide sequence capable of hybridizing to the first target sequence,
> (b) a first tracr mate sequence, and
> (c) a first tracr sequence,

I. a second CRISPR-Cas system chiRNA polynucleotide sequence, wherein the second polynucleotide sequence comprises:

> (a) a second guide sequence capable of hybridizing to the second target sequence,
> (b) a second tracr mate sequence, and
> (c) a second tracr sequence, and

III. a polynucleotide sequence encoding a CRISPR enzyme comprising at least one or more nuclear localization sequences and comprising one or more mutations, wherein (a), (b) and (c) are arranged in a 5' to 3' orientation, wherein when transcribed, the first and the second tracr mate sequence hybridize to the first and second tracr sequence respectively and the first and the second guide sequence directs sequence-specific binding of a first and a second CRISPR complex to the first and second target sequences respectively, wherein the first CRISPR complex comprises the CRISPR enzyme complexed with (1) the first guide sequence that is hybridized to the first target sequence, and (2) the first tracr mate sequence that is hybridized to the first tracr sequence, wherein the second CRISPR complex comprises the CRISPR enzyme complexed with (1) the second guide sequence that is hybridized to the second target sequence, and (2) the second tracr mate sequence that is hybridized to the second tracr sequence, wherein the polynucleotide sequence encoding a CRISPR enzyme is DNA or RNA, and wherein the first guide sequence directs cleavage of one strand of the DNA duplex near the first target sequence and the second guide sequence directs cleavage of the other strand near the second target sequence inducing a double strand break, thereby modifying the organism or the non-human organism by minimizing off-target modifications.

In some methods of the invention any or all of the polynucleotide sequence encoding the CRISPR enzyme, the first and the second guide sequence, the first and the second tracr mate sequence or the first and the second tracr sequence, is/are RNA. In further embodiments of the invention the polynucleotides encoding the sequence encoding the CRISPR enzyme, the first and the second guide sequence, the first and the second tracr mate sequence or the first and the second tracr sequence, is/are RNA and are delivered via liposomes, nanoparticles, exosomes, microvesicles, or a gene-gun. In certain embodiments of the invention, the first and second tracr mate sequence share 100% identity and/or the first and second tracr sequence share 100% identity. In some embodiments, the polynucleotides may be comprised within a vector system comprising one or more vectors. In preferred embodiments of the invention the CRISPR enzyme is a Cas9 enzyme, e.g. SpCas9. In an aspect of the invention the CRISPR enzyme comprises one or more mutations in a catalytic domain, wherein the one or more mutations are selected from the group consisting of D10A, E762A, H840A, N854A, N863A and D986A. In a highly preferred embodiment the CRISPR enzyme has the D10A mutation. In preferred embodiments, the first CRISPR enzyme has one or more mutations such that the enzyme is a complementary strand nicking enzyme, and the second CRISPR enzyme has one or more mutations such that the enzyme is a non-complementary strand nicking enzyme. Alternatively the first enzyme may be a non-complementary strand nicking enzyme, and the second enzyme may be a complementary strand nicking enzyme.

In preferred methods of the invention the first guide sequence directing cleavage of one strand of the DNA duplex near the first target sequence and the second guide sequence directing cleavage of the other strand near the second target sequence results in a 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs. Most preferably, the overlap is between 5 and -1 base pairs.

The invention in some embodiments comprehends a method of modifying an organism or a non-human organism by minimizing off-target modifications by manipulation of a first and a second target sequence on opposite strands of a DNA duplex in a genomic locus of interest in a cell comprising delivering a non-naturally occurring or engineered composition comprising a vector system comprising one or more vectors comprising

I. a first regulatory element operably linked to

(a) a first guide sequence capable of hybridizing to the first target sequence, and
(b) at least one or more tracr mate sequences,

II. a second regulatory element operably linked to

(a) a second guide sequence capable of hybridizing to the second target sequence, and
(b) at least one or more tracr mate sequences,

III. a third regulatory element operably linked to an enzyme-coding sequence encoding a CRISPR enzyme, and
IV. a fourth regulatory element operably linked to a tracr sequence,

wherein components I, II, III and IV are located on the same or different vectors of the system, when transcribed, the tracr mate sequence hybridizes to the tracr sequence and the first and the second guide sequence direct sequence-specific binding of a first and a second CRISPR complex to the first and second target sequences respectively, wherein the first CRISPR complex comprises the CRISPR enzyme complexed with (1) the first guide sequence that is hybridized to the first target sequence, and (2) the tracr mate sequence that is hybridized to the tracr sequence, wherein the second CRISPR complex comprises the CRISPR enzyme complexed with (1) the second guide sequence that is hybridized to the second target sequence, and (2) the tracr mate sequence that is hybridized to the tracr sequence, wherein the polynucleotide sequence encoding a CRISPR enzyme is DNA or RNA, and wherein the first guide sequence directs cleavage of one strand of the DNA duplex near the first target sequence and the second guide sequence directs cleavage of the other strand near the second target sequence inducing a double strand break, thereby modifying the organism or the non-human organism by minimizing off-target modifications.

The disclosure

also provides a vector system as described herein. The system may

comprise one, two, three or four different vectors. Components I, II, III and IV may thus be located on one, two, three or four different vectors, and all combinations for possible locations of the components are herein envisaged, for example: components I, II, III and IV can be located on the same vector; components I, II, III and IV can each be located on different vectors; components I, II, II I and IV may be located on a total of two or three different vectors, with all combinations of locations envisaged, etc.

In some methods of the invention any or all of the polynucleotide sequence encoding the CRISPR enzyme, the first and the second guide sequence, the first and the second tracr mate sequence or the first and the second tracr sequence, is/are RNA. In further embodiments of the invention the first and second tracr mate sequence share 100% identity and/or the first and second tracr sequence share 100% identity. In preferred embodiments of the invention the CRISPR enzyme is a Cas9 enzyme, e.g. SpCas9. In an aspect of the invention the CRISPR enzyme comprises one or more mutations in a catalytic domain, wherein the one or more mutations are selected from the group consisting of D10A, E762A, H840A, N854A, N863A and D986A. In a highly preferred embodiment the CRISPR enzyme has the D10A mutation. In preferred embodiments, the first CRISPR enzyme has one or more mutations such that the enzyme is a complementary strand nicking enzyme, and the second CRISPR enzyme has one or more mutations such that the enzyme is a non-complementary strand nicking enzyme. Alternatively the first enzyme may be a non-complementary strand nicking enzyme, and the second enzyme may be a complementary strand nicking enzyme. In a further embodiment of the invention, one or more of the viral vectors are delivered via liposomes, nanoparticles, exosomes, microvesicles, or a gene-gun.

In preferred methods of the invention the first guide sequence directing cleavage of one strand of the DNA duplex near the first target sequence and the second guide sequence directing cleavage of other strand near the second target sequence results in a 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs.

The invention in some embodiments comprehends a method of modifying a genomic locus of interest by minimizing off-target modifications by introducing into a cell containing and expressing a double stranded DNA molecule encoding a gene product of interest an engineered, non-naturally occurring CRISPR-Cas system comprising a Cas protein having one or more mutations and two guide RNAs that target a first strand and a second strand of the DNA molecule respectively, whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together.

In preferred methods of the invention the Cas protein nicking each of the first strand and the second strand of the DNA molecule encoding the gene product results in a 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs.

Embodiments of the invention also comprehend the guide RNAs comprising a guide sequence fused to a tracr mate sequence and a tracr sequence. In an aspect of the invention the Cas protein is codon optimized for expression in a eukaryotic cell, preferably a mammalian cell or a human cell. In further embodiments of the invention the Cas protein is a type II CRISPR-Cas protein, e.g. a Cas 9 protein. In a highly preferred embodiment the Cas protein is a Cas9 protein, e.g. SpCas9. In aspects of the invention the Cas protein has one or more mutations selected from the group consisting of D10A, E762A, H840A, N854A, N863A and D986A. In a highly preferred embodiment the Cas protein has the D10A mutation.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein.

The disclosure

also comprehends an engineered, non-naturally occurring CRISPR-Cas

system comprising a Cas protein having one or more mutations and two guide RNAs that target a first strand and a second strand respectively of a double stranded DNA molecule encoding a gene product in a cell, whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together.

In aspects of the invention the guide RNAs may comprise a guide sequence fused to a tracr mate sequence and a tracr sequence. In an embodiment of the invention the Cas protein is a type II CRISPR-Cas protein. In an aspect of the invention the Cas protein is codon optimized for expression in a eukaryotic cell, preferably a mammalian cell or a human cell. In further embodiments of the invention the Cas protein is a type II CRISPR-Cas protein, e.g. a Cas 9 protein. In a highly preferred embodiment the Cas protein is a Cas9 protein, e.g. SpCas9. In aspects of the invention the Cas protein has one or more mutations selected from the group consisting of D10A, E762A, H840A, N854A, N863A and D986A. In a highly preferred embodiment the Cas protein has the D10A mutation.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein.

The disclosure also comprehends an engineered, non-naturally occurring vector system comprising one or more vectors comprising:

a) a first regulatory element operably linked to each of two CRISPR-Cas system guide RNAs that target a first strand and a second strand respectively of a double stranded DNA molecule encoding a gene product,
b) a second regulatory element operably linked to a Cas protein,

wherein components (a) and (b) are located on same or different vectors of the system, whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together.

In aspects of the invention the guide RNAs may comprise a guide sequence fused to a tracr mate sequence and a tracr sequence. In an embodiment of the invention the Cas protein is a type II CRISPR-Cas protein. In an aspect of the invention the Cas protein is codon optimized for expression in a eukaryotic cell, preferably a mammalian cell or a human cell. In further embodiments of the invention the Cas protein is a type II CRISPR-Cas protein, e.g. a Cas 9 protein. In a highly preferred embodiment the Cas protein is a Cas9 protein, e.g. SpCas9. In aspects of the invention the Cas protein has one or more mutations selected from the group consisting of D10A, E762A, H840A, N854A, N863A and D986A. In a highly preferred embodiment the Cas protein has the D10A mutation.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein. In preferred embodiments of the invention the vectors of the system are viral vectors. In a further embodiment, the vectors of the system are delivered via liposomes, nanoparticles, exosomes, microvesicles, or a gene-gun.

In one aspect, the disclosure provides a method of modifying a target polynucleotide in a post-mitotic eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expressed from a gene comprising the target sequence. In some embodiments, the method further comprises delivering one or more vectors to said eukaryotic cell, wherein the one or more vectors drive expression of one or more of: the CRISPR enzyme, the guide sequence linked to the tracr mate sequence, and the tracr sequence. In some embodiments, said vectors are delivered to the eukaryotic cell in a subject. In some embodiments, said modifying takes place in said eukaryotic cell in a cell culture. In some embodiments, the method further comprises isolating said eukaryotic cell from a subject prior to said modifying. In some embodiments, the method further comprises returning said eukaryotic cell and/or cells derived therefrom to said subject.

In one aspect, the disclosure provides a method of modifying expression of a polynucleotide in a post-mitotic eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. In some embodiments, the method further comprises delivering one or more vectors to said eukaryotic cells, wherein the one or more vectors drive expression of one or more of: the CRISPR enzyme, the guide sequence linked to the tracr mate sequence, and the tracr sequence.

In one aspect, the disclosure provides a method of generating a model post-mitotic eukaryotic cell comprising a mutated disease gene. In some embodiments, a disease gene is any gene associated with an increase in the risk of having or developing a disease. In some embodiments, the method comprises (a) introducing one or more vectors into a eukaryotic cell, wherein the one or more vectors drive expression of one or more of: a CRISPR enzyme, a guide sequence linked to a tracr mate sequence, and a tracr sequence; and (b) allowing a CRISPR complex to bind to a target polynucleotide to effect cleavage of the target polynucleotide within said disease gene, wherein the CRISPR complex comprises the CRISPR enzyme complexed with (1) the guide sequence that is hybridized to the target sequence within the target polynucleotide, and (2) the tracr mate sequence that is hybridized to the tracr sequence, thereby generating a model eukaryotic cell comprising a mutated disease gene. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expression from a gene comprising the target sequence.

In one aspect the disclosure provides for a method of selecting one or more post- mitotic cell(s) by introducing one or more mutations in a gene in the one or more cell (s), the method comprising: introducing one or more vectors into the cell (s), wherein the one or more vectors drive expression of one or more of: a CRISPR enzyme, a guide sequence linked to a tracr mate sequence, a tracr sequence, and an editing template; wherein the editing template comprises the one or more mutations that abolish CRISPR enzyme cleavage; allowing homologous recombination of the editing template with the target polynucleotide in the cell(s) to be selected; allowing a CRISPR complex to bind to a target polynucleotide to effect cleavage of the target polynucleotide within said gene, wherein the CRISPR complex comprises the CRISPR enzyme complexed with (1) the guide sequence that is hybridized to the target sequence within the target polynucleotide, and (2) the tracr mate sequence that is hybridized to the tracr sequence, wherein binding of the CRISPR complex to the target polynucleotide induces cell death, thereby allowing one or more prokaryotic cell(s) in which one or more mutations have been introduced to be selected. In a preferred embodiment, the CRISPR enzyme is Cas9. Aspects of the invention allow for selection of specific cells without requiring a selection marker or a two-step process that may include a counter-selection system.

In one aspect, the disclosure provides for methods of modifying a target polynucleotide in a post-mitotic eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In other embodiments, this diclosure provides a method of modifying expression of a polynucleotide in a post-mitotic eukaryotic cell. The method comprises increasing or decreasing expression of a target polynucleotide by using a CRISPR complex that binds to the polynucleotide.

Where desired, to effect the modification of the expression in a cell, one or more vectors comprising a tracr sequence, a guide sequence linked to the tracr mate sequence, a sequence encoding a CRISPR enzyme is delivered to a cell. In some methods, the one or more vectors comprises a regulatory element operably linked to an enzyme-coding sequence encoding said CRISPR enzyme comprising a nuclear localization sequence; and a regulatory element operably linked to a tracr mate sequence and one or more insertion sites for inserting a guide sequence upstream of the tracr mate sequence. When expressed, the guide sequence directs sequence-specific binding of a CRISPR complex to a target sequence in a cell. Typically, the CRISPR complex comprises a CRISPR enzyme complexed with (1) the guide sequence that is hybridized to the target sequence, and (2) the tracr mate sequence that is hybridized to the tracr sequence.

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein is not produced.

In certain embodiments, the CRISPR enzyme comprises one or more mutations selected from the group consisting of D10A, E762A, H840A, N854A, N863A or D986A and/or the one or more mutations is in a RuvC1 or HNH domain of the CRISPR enzyme or is a mutation as otherwise as discussed herein. In some embodiments, the CRISPR enzyme has one or more muations in a catalytic domain, wherein when transcribed, the tracr mate sequence hybridizes to the tracr sequence and the guide sequence directs sequence-specific binding of a CRISPR complex to the target sequence, and wherein the enzyme further comprises a functional domain. In some embodiments, the functional domain is a transcriptional activation domain, preferably VP64. In some embodiments, the functional domain is a transcription repression domain, preferably KRAB. In some embodiments, the transcription repression domain is SID, or concatemers of SID (eg SID4X). In some embodiments, the functional domain is an epigenetic modifying domain, such that an epigenetic modifying enzyme is provided. In some embodiments, the functional domain is an activation domain, which may be the P65 activation domain.

In some embodiments, the CRISPR enzyme is a type I or III CRISPR enzyme, but is preferably a type II CRISPR enzyme. This type II CRISPR enzyme may be any Cas enzyme. A Cas enzyme may be identified as Cas9 as this can refer to the general class of enzymes that share homology to the biggest nuclease with multiple nuclease domains from the type II CRISPR system. Most preferably, the Cas9 enzyme is from, or is derived from, spCas9 or saCas9. By derived, Applicants mean that the derived enzyme is largely based, in the sense of having a high degree of sequence homology with, a wildtype enzyme, but that it has been mutated (modified) in some way as described herein.

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes.* However, it will be appreciated that this invention includes many more Cas9s from other species of microbes, such as SpCas9, SaCa9, St1Cas9 and so forth.

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species is known.

Preferably, delivery is in the form of a vector which may be a viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided. A vector may mean not only a viral or yeast system (for instance, where the nucleic acids of interest may be operably linked to and under the control of (in terms of expression, such as to ultimately provide a processed RNA) a promoter), but also direct delivery of nucleic acids into a host cell. While in herein methods the vector may be a viral vector and this is advantageously an AAV, other viral vectors as herein discussed can be employed, such as lentivirus. For example, baculoviruses may be used for expression in insect cells. These insect cells may, in turn be useful for producing large quantities of further vectors, such as AAV or lentivirus vectors adapted for delivery. Also envisaged is a method of delivering the present CRISPR enzyme comprising delivering to a cell mRNA encoding the CRISPR enzyme. It will be appreciated that in certain embodiments the CRISPR enzyme is truncated, and/or comprised of less than one thousand amino acids or less than four thousand amino acids, and/or is a nuclease or nickase, and/or is codon-optimized, and/or comprises one or more mutations, and/or comprises a chimeric CRISPR enzyme, and/or the other options as herein discussed. AAV and lentiviral vectors are preferred.

In certain embodiments, the target sequence is flanked or followed, at its 3' end, by a PAM suitable for the CRISPR enzyme, typically a Cas and in particular a Cas9.

For example, a suitable PAM is 5'-NRG or 5'-NNGRR for SpCas9 or SaCas9 enzymes (or derived enzymes), respectively.

It will be appreciated that SpCas9 or SaCas9 are those from or derived from *S. pyogenes* or *S. aureus* Cas9.

These and other embodiments are disclosed or are obvious from and encompassed by, the following Detailed Description.

BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

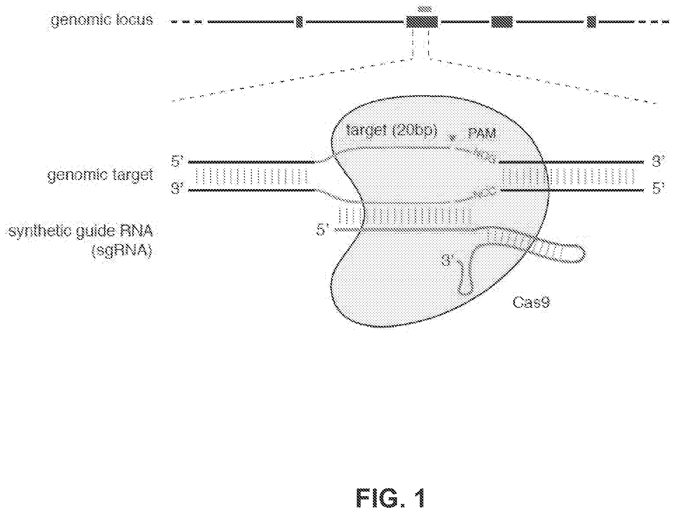

Figure 1 shows a schematic model of the CRISPR system. The Cas9 nuclease from Streptococcus pyogenes (yellow) is targeted to genomic DNA by a synthetic guide RNA (sgRNA) consisting of a 20-nt guide sequence (blue) and a scaffold (red). The guide sequence base-pairs with the DNA target (blue), directly upstream of a requisite 5'-NGG protospacer adjacent motif (PAM; magenta), and Cas9 mediates a double-stranded break (DSB) ~3 bp upstream of the PAM (red triangle).

Figure 2A-F shows an exemplary CRISPR system, a possible mechanism of action, an example adaptation for expression in eukaryotic cells, and results of tests assessing nuclear localization and CRISPR activity.

Figure 3A-D shows results of an evaluation of SpCas9 specificity for an example target.

Figure 4A-G show an exemplary vector system and results for its use in directing homologous recombination in eukaryotic cells.

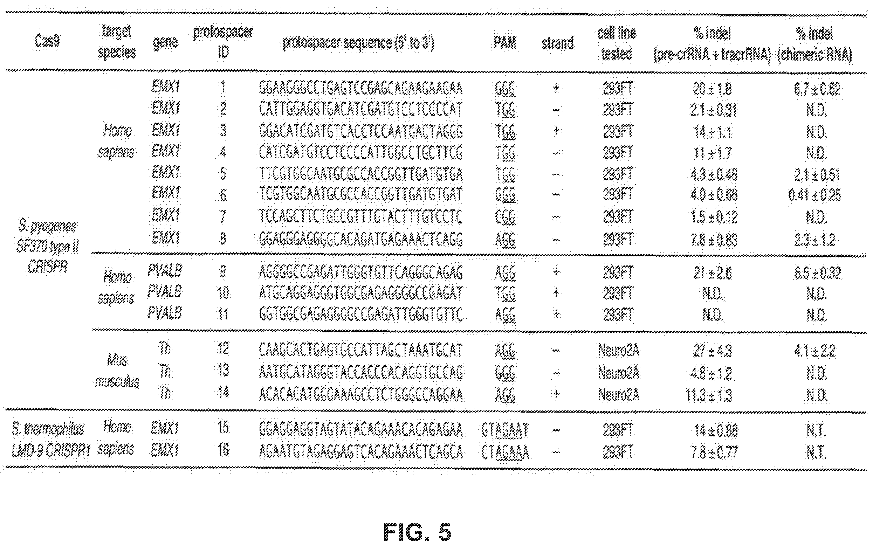

Figure 5 provides a table of protospacer sequences and summarizes modification efficiency results for protospacer targets designed based on exemplary S. pyogenes and S. thermophilus CRISPR systems with corresponding PAMs against loci in human and mouse genomes. Cells were transfected with Cas9 and either pre-crRNA/tracrRNA or chimeric RNA, and analyzed 72 hours after transfection. Percent indels are calculated based on Surveyor assay results from indicated cell lines (N=3 for all protospacer targets, errors are S.E.M., N.D. indicates not detectable using the Surveyor assay, and N.T. indicates not tested in this study).

Figure 6A-C shows a comparison of different tracrRNA transcripts for Cas9-mediated gene targeting.

Figure 7 shows a schematic of a surveyor nuclease assay for detection of double strand break-induced micro-insertions and -deletions.

Figure 8A-B shows exemplary bicistronic expression vectors for expression of CRISPR system elements in eukaryotic cells.

Figure 9A-C shows histograms of distances between adjacent S. pyogenes SF370 locus 1 PAM (NGG) (Figure 9A) and S. thermophilus LMD9 locus 2 PAM (NNAGAAW) (Figure 9B) in the human genome; and distances for each PAM by chromosome (Chr) (Figure 9C).

Figure 10A-D shows an exemplary CRISPR system, an example adaptation for expression in eukaryotic cells, and results of tests assessing CRISPR activity.

Figure 11A-C shows exemplary manipulations of a CRISPR system for targeting of genomic loci in mammalian cells.

Figure 12A-B shows the results of a Northern blot analysis of crRNA processing in mammalian cells.

Figure 13A-B shows an exemplary selection of protospacers in the human PVALB and mouse Th loci.

Figure 14 shows example protospacer and corresponding PAM sequence targets of the S. thermophilus CRISPR system in the human EMX1 locus.

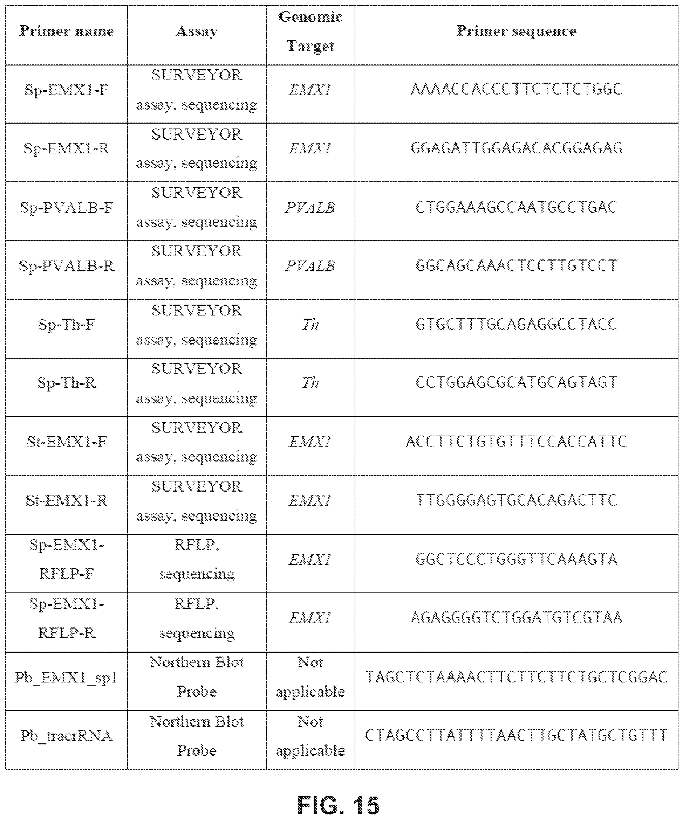

Figure 15 provides a table of sequences for primers and probes used for Surveyor, RFLP, genomic sequencing, and Northern blot assays.

Figure 16A-C shows exemplary manipulation of a CRISPR system with chimeric RNAs and results of SURVEYOR assays for system activity in eukaryotic cells.

Figure 17A-B shows a graphical representation of the results of SURVEYOR assays for CRISPR system activity in eukaryotic cells.

Figure 18 shows an exemplary visualization of some S. pyogenes Cas9 target sites in the human genome using the UCSC genome browser.

Figure 19A-D shows a circular depiction of the phylogenetic analysis revealing five families of Cas9s, including three groups of large Cas9s (~1400 amino acids) and two of small Cas9s (~1100 amino acids).

Figure 20A-F shows the linear depiction of the phylogenetic analysis revealing five families of Cas9s, including three groups of large Cas9s (~1400 amino acids) and two of small Cas9s (~1100 amino acids).

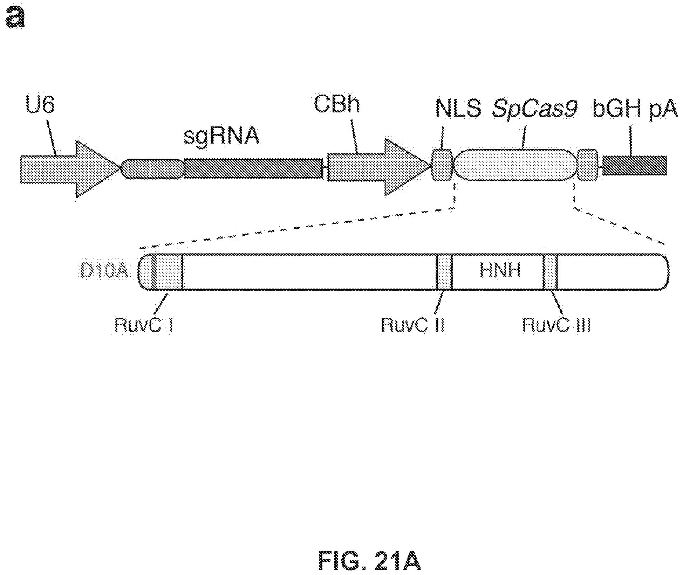

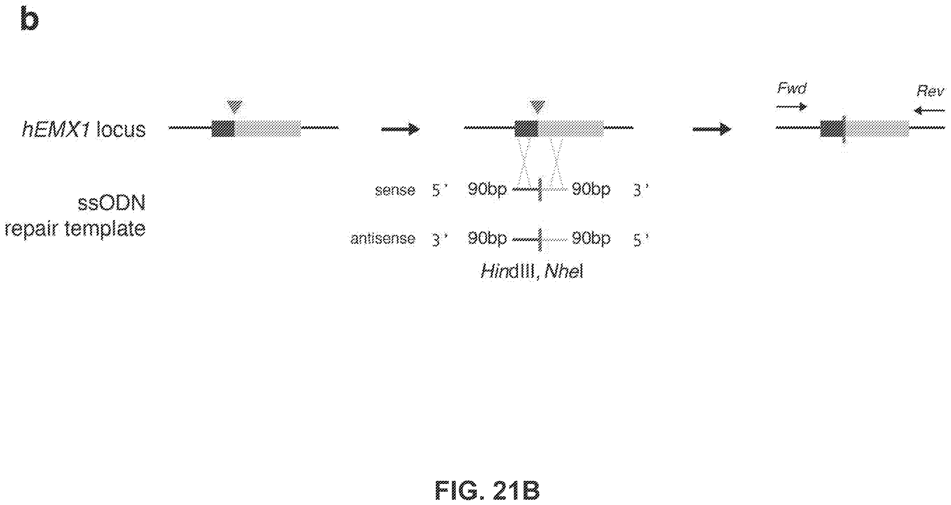

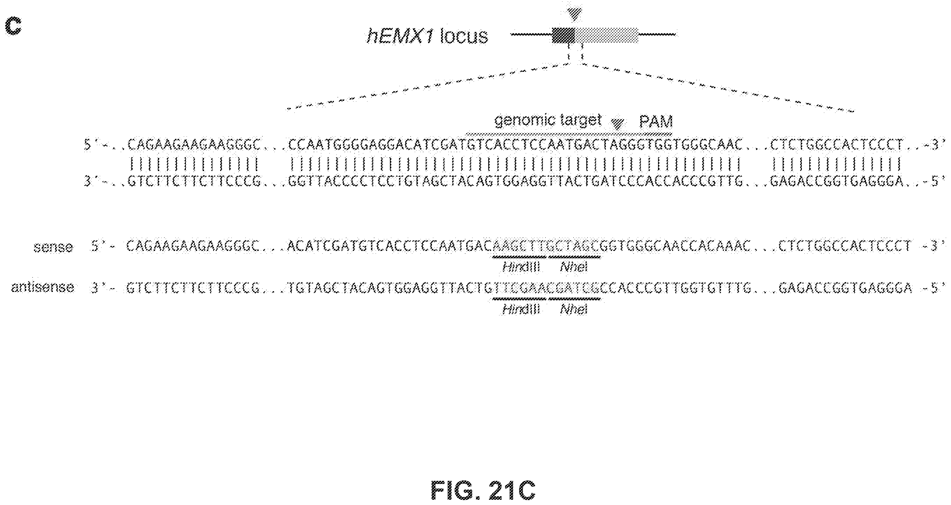

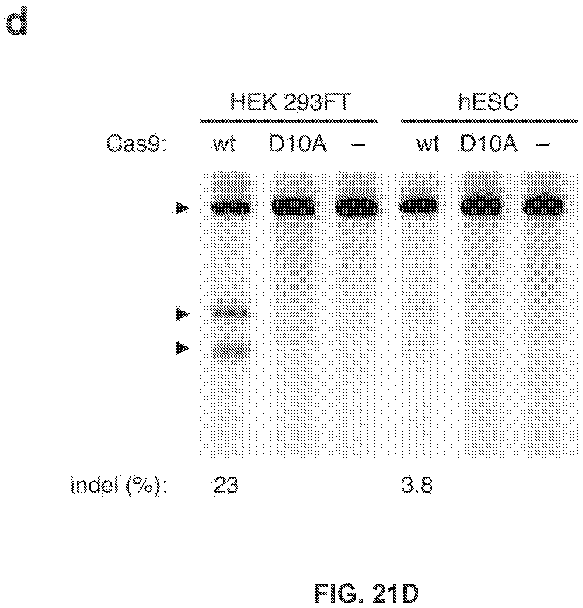

Figure 21A-D shows genome editing via homologous recombination. (a) Schematic of SpCas9 nickase, with D10A mutation in the RuvC I catalytic domain. (b) Schematic representing homologous recombination (HR) at the human EMX1 locus using either sense or antisense single stranded oligonucleotides as repair templates. Red arrow above indicates sgRNA cleavage site; PCR primers for genotyping (Tables J and K) are indicated as arrows in right panel. (c) Sequence of region modified by HR. d, SURVEYOR assay for wildtype (wt) and nickase (D10A) SpCas9-mediated indels at the EMX1 target 1 locus (n=3). Arrows indicate positions of expected fragment sizes.

Figure 22A-B shows single vector designs for SpCas9.

Figure 23 shows a graph representing the length distribution of Cas9 orthologs.

Figure 24A-M shows sequences where the mutation points are located within the SpCas9 gene.

Figure 25A shows the Conditional Cas9, Rosa26 targeting vector map.

Figure 25B shows the Constitutive Cas9, Rosa26 targeting vector map.

Figure 26 shows a schematic of the important elements in the Constitutive and Conditional Cas9 constructs.

Figure 27 shows delivery and *in vivo* mouse brain Cas9 expression data.

Figure 28 shows RNA delivery of Cas9 and chimeric RNA into cells (A) Delivery of a GFP reporter as either DNA or mRNA into Neuro-2A cells. (B) Delivery of Cas9 and chimeric RNA against the Icam2 gene as RNA results in cutting for one of two spacers tested. (C) Delivery of Cas9 and chimeric RNA against the F7 gene as RNA results in cutting for one of two spacers tested.

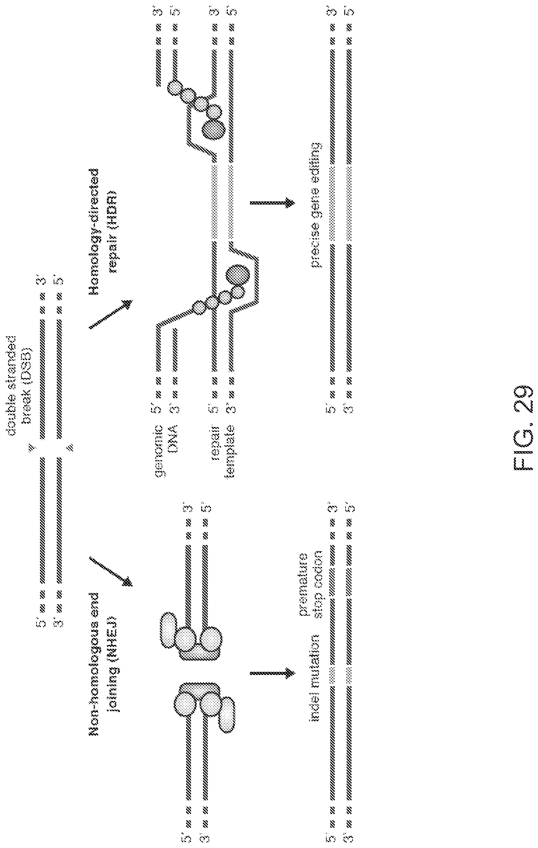

Figure 29 shows how DNA double-strand break (DSB) repair promotes gene editing. In the error-prone non-homologous end joining (NHEJ) pathway, the ends of a DSB are processed by endogenous DNA repair machineries and rejoined together, which can result in random insertion/deletion (indel) mutations at the site of junction. Indel mutations occurring within the coding region of a gene can result in frame-shift and a premature stop codon, leading to gene knockout. Alternatively, a repair template in the form of a plasmid or single-stranded oligodeoxynucleotides (ssODN) can be supplied to leverage the homology-directed repair (HDR) pathway, which allows high fidelity and precise editing.

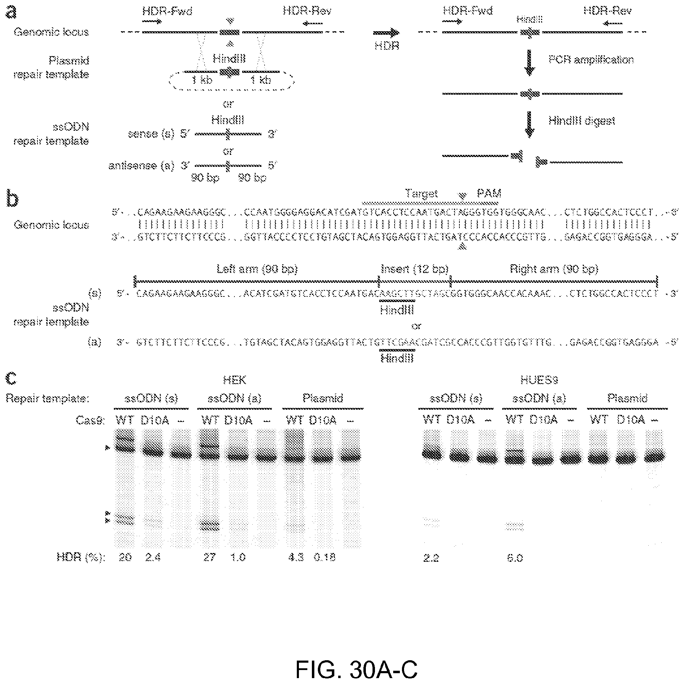

Figure 30A-C shows anticipated results for HDR in HEK and HUES9 cells. (a) Either a targeting plasmid or an ssODN (sense or antisense) with homology arms can be used to edit the sequence at a target genomic locus cleaved by Cas9 (red triangle). To assay the efficiency of HDR, we introduced a HindIII site (red bar) into the target locus, which was PCR-amplified with primers that anneal outside of the region of homology. Digestion of the PCR product with HindIII reveals the occurrence of HDR events. (b) ssODNs, oriented in either the sense or the antisense (s or a) direction relative to the locus of interest, can be used in combination with Cas9 to achieve efficient HDR-mediated editing at the target locus. A minimal homology region of 40 bp, and preferably 90 bp, is recommended on either side of the modification (red bar). (c) Example of the effect of ssODNs on HDR in the EMX1 locus is shown using both wild-type Cas9 and Cas9 nickase (D10A). Each ssODN contains homology arms of 90 bp flanking a 12-bp insertion of two restriction sites.

Figure 31A-C shows the repair strategy for Cystic Fibrosis delta F508 mutation.

Figure 32A-B (a) shows a schematic of the GAA repeat expansion in FXN intron 1 and (b) shows a schematic of the strategy adopted to excise the GAA expansion region using the CRISPR/Cas system.

Figure 33 shows a screen for efficient SpCas9 mediated targeting of Tet1-3 and Dnmtl, 3a and 3b gene loci. Surveyor assay on DNA from transfected N2A cells demonstrates efficient DNA cleavage by using different gRNAs.

Figure 34 shows a strategy of multiplex genome targeting using a 2-vector system in an AAV1/2 delivery system. Tet1-3 and Dnmtl, 3a and 3b gRNA under the control of the U6 promoter. GFP-KASH under the control of the human synapsin promoter. Restriction sides shows simple gRNA replacement strategy by subcloning. HA-tagged SpCas9 flanked by two nuclear localization signals (NLS) is shown. Both vectors are delivered into the brain by AAV1/2 virus in a 1:1 ratio.

Figure 35 shows verification of multiplex DNMT targeting vector #1 functionality using Surveyor assay. N2A cells were co-transfected with the DNMT targeting vector #1 (+) and the SpCas9 encoding vector for testing SpCas9 mediated cleavage of DNMTs genes family loci. gRNA only (-) is negative control. Cells were harvested for DNA purification and downstream processing 48 h after transfection.

Figure 36 shows verification of multiplex DNMT targeting vector #2 functionality using Surveyor assay. N2A cells were co-transfected with the DNMT targeting vector #1 (+) and the SpCas9 encoding vector for testing SpCas9 mediated cleavage of DNMTs genes family loci. gRNA only (-) is negative control. Cells were harvested for DNA purification and downstream processing 48 h after transfection.

Figure 37 shows schematic overview of short promoters and short polyA versions used for HA-SpCas9 expression in vivo. Sizes of the encoding region from L-ITR to R-ITR are shown on the right.

Figure 38 shows schematic overview of short promoters and short polyA versions used for HA-SaCas9 expression in vivo. Sizes of the encoding region from L-ITR to R-ITR are shown on the right.

Figure 39 shows expression of SpCas9 and SaCas9 in N2A cells. Representative Western blot of HA-tagged SpCas9 and SaCas9 versions under the control of different short promoters and with or short polyA (spA) sequences. Tubulin is loading control. mCherry (mCh) is a transfection control. Cells were harvested and further processed for Western blotting 48 h after transfection.

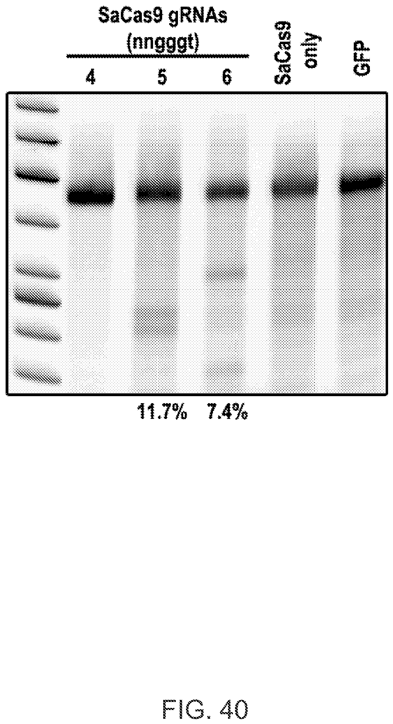

Figure 40 shows screen for efficient SaCas9 mediated targeting of Tet3 gene locus. Surveyor assay on DNA from transfected N2A cells demonstrates efficient DNA cleavage by using different gRNAs with NNGGGT PUM sequence. GFP transfected cells and cells expressing only SaCas9 are controls.

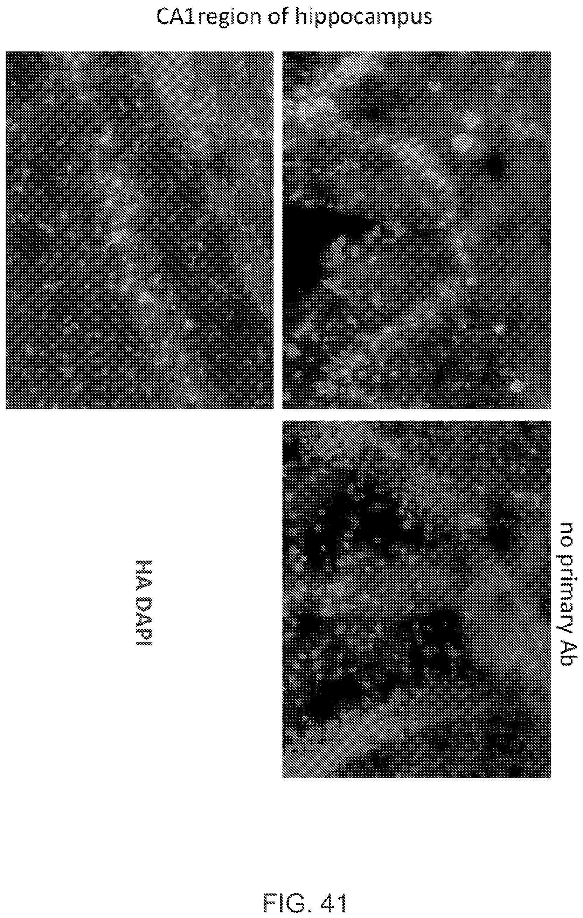

Figure 41 shows expression of HA-SaCas9 in the mouse brain. Animals were injected into dentate gyri with virus driving expression of HA-SaCas9 under the control of human Synapsin promoter. Animals were sacrificed 2 weeks after surgery. HA tag was detected using rabbit monoclonal antibody C29F4 (Cell Signaling). Cell nuclei stained in blue with DAPI stain.

Figure 42 shows expression of SpCas9 and SaCas9 in cortical primary neurons in culture 7 days after transduction. Representative Western blot of HA-tagged SpCas9 and SaCas9 versions under the control of different promoters and with bgh or short polyA (spA) sequences. Tubulin is loading control.

Figure 43 shows LIVE/DEAD stain of primary cortical neurons 7 days after transduction with AAV1 particles carrying SpCas9 with different promoters and multiplex gRNAs constructs (example shown on the last panel for DNMTs). Neurons after AAV transduction were compared with control untransduced neurons. Red nuclei indicate permeabilized, dead cells (second line of panels). Live cells are marked in green color (third line of panels).

Figure 44 shows LIVE/DEAD stain of primary cortical neurons 7 days after transduction with AAV1 particles carrying SaCas9 with different promoters. Red nuclei indicate permeabilized, dead cells (second line of panels). Live cells are marked in green color (third line of panels).

Figure 45 shows comparison of morphology of neurons after transduction with AAV1 virus carrying SpCas9 and gRNA multiplexes for TETs and DNMTs genes loci. Neurons without transduction are shown as a control.

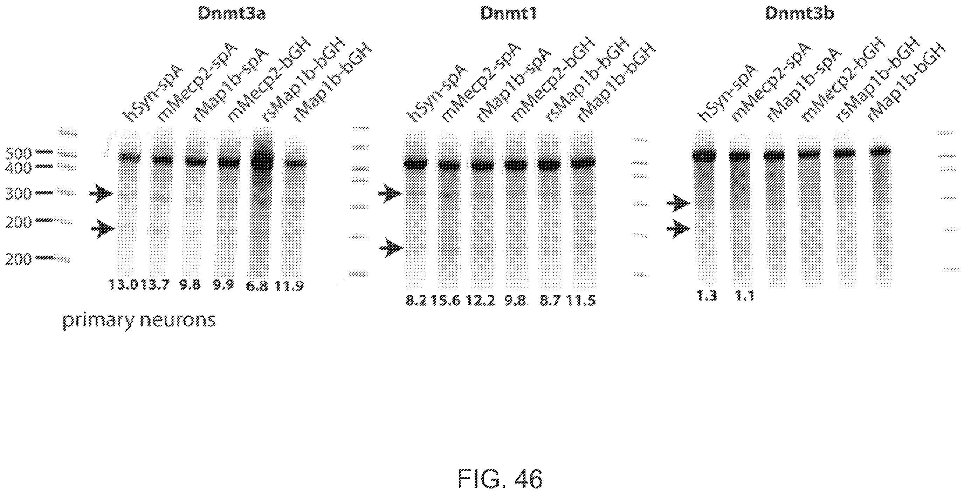

Figure 46 shows verification of multiplex DNMT targeting vector #1 functionality using Surveyor assay in primary cortical neurons. Cells were co-transduced with the DNMT targeting vector #1 and the SpCas9 viruses with different promoters for testing SpCas9 mediated cleavage of DNMTs genes family loci.

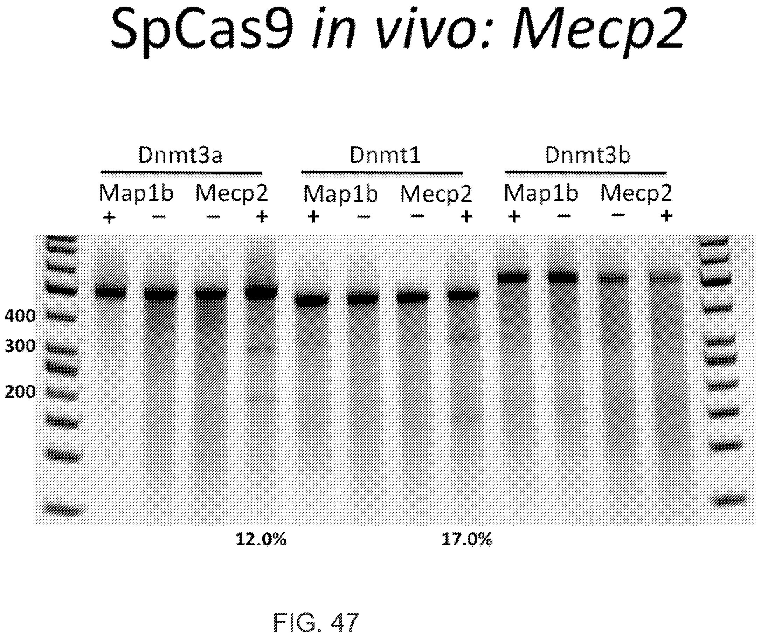

Figure 47 shows in vivo efficiency of SpCas9 cleavage in the brain. Mice were injected with AAV1/2 virus carrying gRNA multiplex targeting DNMT family genes loci together with SpCas9 viruses under control of 2 different promoters: mouse Mecp2 and rat Maplb. Two weeks after injection brain tissue was extracted and nuclei were prepped and sorted using FACS, based on the GFP expression driven by Synapsin promoter from gRNA multiplex construct. After gDNA extraction Surveyor assay was run. + indicates GFP positive nuclei and - control, GFP-negative nuclei from the same animal. Numbers on the gel indicate assessed SpCas9 efficiency.

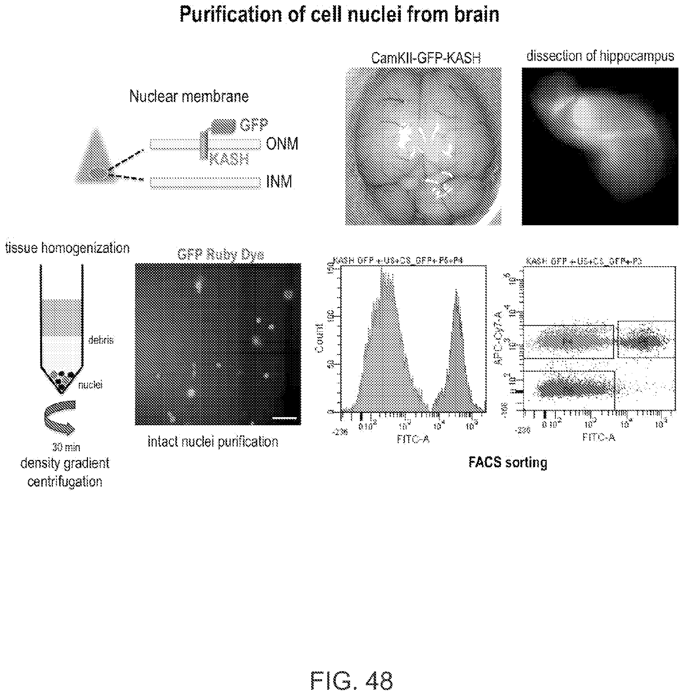

Figure 48 shows purification of GFP-KASH labeled cell nuclei from hippocampal neurons. The outer nuclear membrane (ONM) of the cell nuclear membrane is tagged with a fusion of GFP and the KASH protein transmembrane domain. Strong GFP expression in the brain after one week of stereotactic surgery and AAV1/2 injection. Density gradient centrifugation step to purify cell nuclei from intact brain. Purified nuclei are shown. Chromatin stain by Vybrant® DyeCycle™ Ruby Stain is shown in red, GFP labeled nuclei are green. Representative FACS profile of GFP+ and GFP- cell nuclei (Magenta: Vybrant® DyeCycle™ Ruby Stain, Green: GFP).

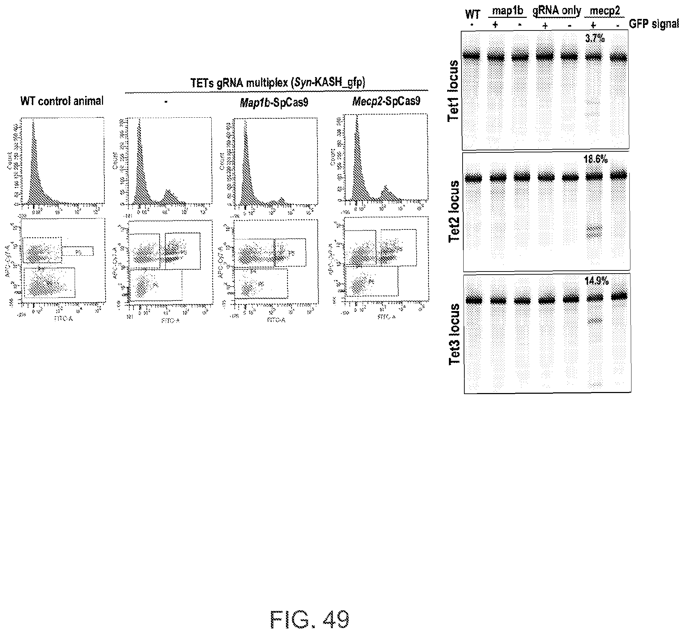

Figure 49 shows efficiency of SpCas9 cleavage in the mouse brain. Mice were injected with AAV1/2 virus carrying gRNA multiplex targeting TET family genes loci together with SpCas9 viruses under control of 2 different promoters: mouse Mecp2 and rat Maplb. Three weeks after injection brain tissue was extracted, nuclei were prepped and sorted using FACS, based on the GFP expression driven by Synapsin promoter from gRNA multiplex construct. After gDNA extraction Surveyor assay was run. + indicates GFP positive nuclei and - control, GFP-negative nuclei from the same animal. Numbers on the gel indicate assessed SpCas9 efficiency.

Figure 50 shows GFP-KASH expression in cortical neurons in culture. Neurons were transduced with AAV1 virus carrying gRNA multiplex constructs targeting TET genes loci. The strongest signal localize around cells nuclei due to KASH domain localization.

Figure 51 shows (top) a list of spacing (as indicated by the pattern of arrangement for two PAM sequences) between pairs of guide RNAs. Only guide RNA pairs satisfying patterns 1, 2, 3, 4 exhibited indels when used with SpCas9(D10A) nickase. (bottom) Gel images showing that combination of SpCas9(D10A) with pairs of guide RNA satisfying patterns 1, 2, 3, 4 led to the formation of indels in the target site.

Figure 52 shows a list of U6 reverse primer sequences used to generate U6-guide RNA expression casssettes. Each primer needs to be paired with the U6 forward primer "gcactgagggcctatttcccatgattc" (SEQ ID NO: 1) to generate amplicons containing U6 and the desired guide RNA.

Figure 53 shows a Genomic sequence map from the human Emx1 locus showing the locations of the 24 patterns listed in Figure 33.

Figure 54 shows on (right) a gel image indicating the formation of indels at the target site when variable 5' overhangs are present after cleavage by the Cas9 nickase targeted by different pairs of guide RNAs. on (left) a table indicating the lane numbers of the gel on the right and various parameters including identifying the guide RNA pairs used and the length of the 5' overhang present following cleavage by the Cas9 nickase.

Figure 55 shows a Genomic sequence map from the human Emx1 locus showing the locations of the different pairs of guide RNAs that result in the gel patterns of Fig. 54 (right) and which are further described in Example 35.

Figure 56A shows that guide (target) 1 induced the highest percentage of indels in ApoB.

Figure 56B shows the results of a Surveyor nuclease gel assay for indel formation efficiency, 4 weeks post-injection.

Figure 57B shows oil red staining to detect hepatic lipid accumulation phenotype in vivo following AAV-Cas9-sgRNA delivery. The scale bar in each square represents 20 micrometres.

Figure 58 shows that 21 ntds/base pairs (bp), represented by the grey bars is the optimal spacer length, at least compared to 20 or 22 base pairs (represented by the black and the white bars, respectively) across a range of targets and within two different genes (AAVS1 and EMX1).

Figure 59 shows whether a guide sequence could be inserted into the Cas9 intronic sequence

Figure 60 shows that the full-length H1 promoter (grey bar) is still weaker than U6 promoter (black bar), as the U6 shows increased indel percentage formation for each target tested.

Figure 61 shows that short H1 promoter is weaker than the full-length H1

Figure 62 shows distance between the 5' ends of two guide sequences in a construct measured in relation to the cleavage efficiency of the D10A SaCas9 double nickase.

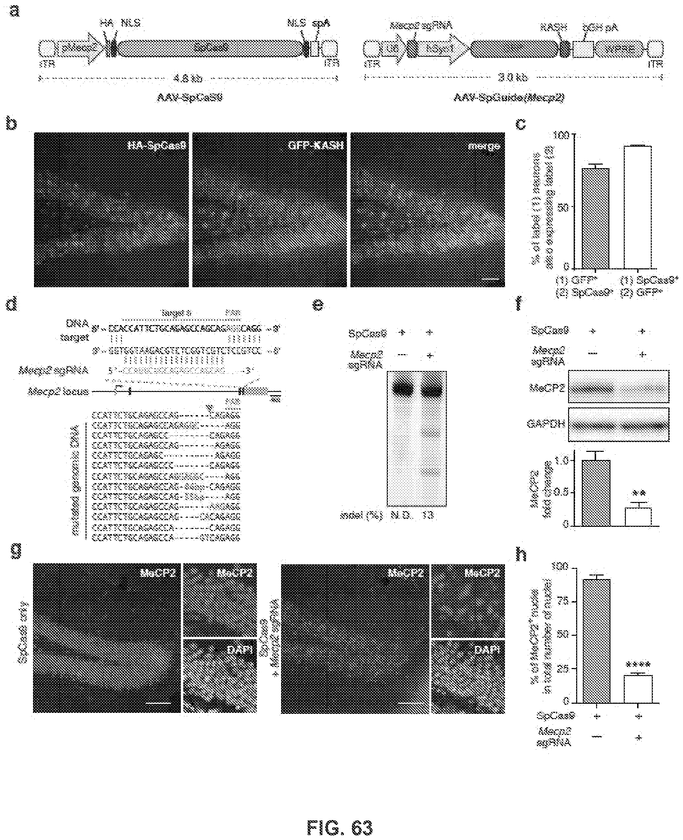

Figure 63 (Example 38) shows CRISPR-Cas9 system delivery and targeting of *Mecp2* locus in the mouse brain. **(a)** AAV-SpCas9 and AAV-SpGuide(*Mecp2*) expression vectors. The sgRNA vector contains encoding sequence of the GFP-KASH fusion protein for identification of transduced neurons, **(b)** Expression of HA-Cas9 and GFP-KASH in the dorsal dentate gyrus (DG) of mouse hippocampus. Scale bar, 100 μm. **(c)** Quantification of cells efficiently targeted by the dual-vector Cas9-CRISPR system. **(d)** Graphical representation of the mouse *Mecp2* locus showing Cas9 target location; sgRNA indicated in blue. PAM sequence marked in purple. Representative mutation patterns detected by sequencing of *Mecp2* locus were shown below: green - wild-type sequence; red dashes - deleted bases; red bases: insertion or mutations; red arrowhead indicates CRISPR-Cas9 cutting site. **(e)** SURVEYOR™ assay gel showing modification of the *Mecp2* locus, 2 weeks after AAV delivery in the DG region. **(f)** Western blot analysis of MeCP2 protein expression in the targeted brain region and quantification of MeCP2 protein levels in dorsal DG (*t*-test, \*\**p*<0.001, n=4 from 3 animals, error bars: s.e.m.). **(g)** Images of the dorsal DG region, 2 weeks after CRISPR-Cas9 targeting of *Mecp2* locus. Scale bar, 150 μm. **(h)** Quantification of MeCP2 positive cells population within all detected cells (DAPI staining) in the targeted brain region in compare to control collateral site (*t*-test, \*\*\*\**p*<0.0001, n=290 and 249 cells from 2 animals, respectively; error bars: s.e.m). (ITR - inverted terminal repeat; HA - hemagglutinin tag; NLS - nuclear localization signal; spA - synthetic polyadenylation signal; U6 - PolIII promoter; sgRNA - single guide RNA; hSyn - human synapsin 1 promoter; GFP- green fluorescent protein; KASH - Klarsicht, ANC1, Syne Homology nuclear transmembrane domain; bGH pA - bovine growth hormone polyadenylatio signal; WPRE - Woodchuck Hepatitis virus posttranscriptional regulatory element).

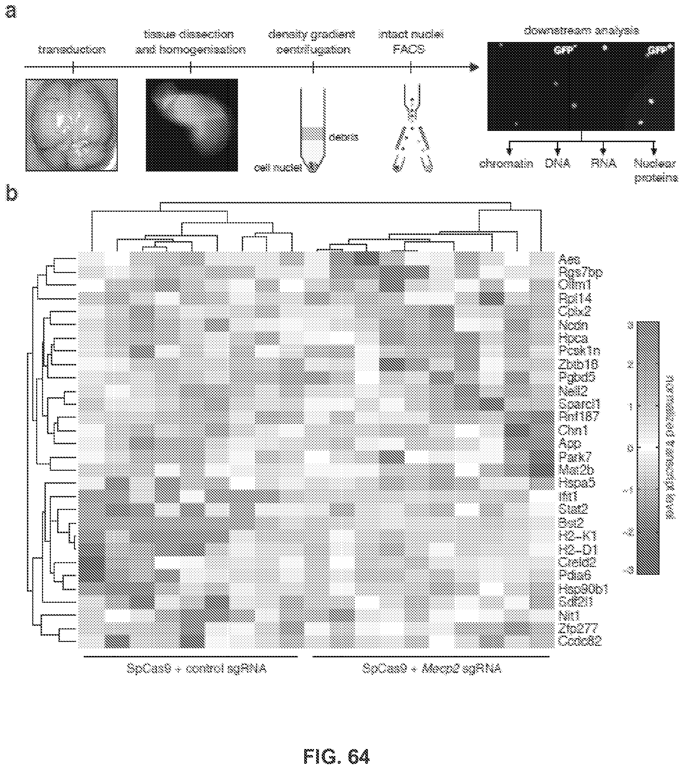

Figure 64 (Example 38) shows analysis of gene expression in Cas9-mediated MeCP2 knockdown neurons, **(a)** Strategy for cell nuclei purification of CRISPR-Cas9 targeted cells from the mouse brain. **(b)** Hierarchical clustering of differentially expressed genes (t-test, p<0.01, n=19 populations of sorted nuclei from 8 animals) detected by RNAseq. Relative log2(TPM+1) expression levels of genes are normalized for each row and displayed in red-blue color scale. Each column represents a population of targeted 100 neuronal nuclei FACS sorted from the isolated, dentate gyrus population of cells, either from control or *Mecp2* sgRNA transduced animals, as indicated.

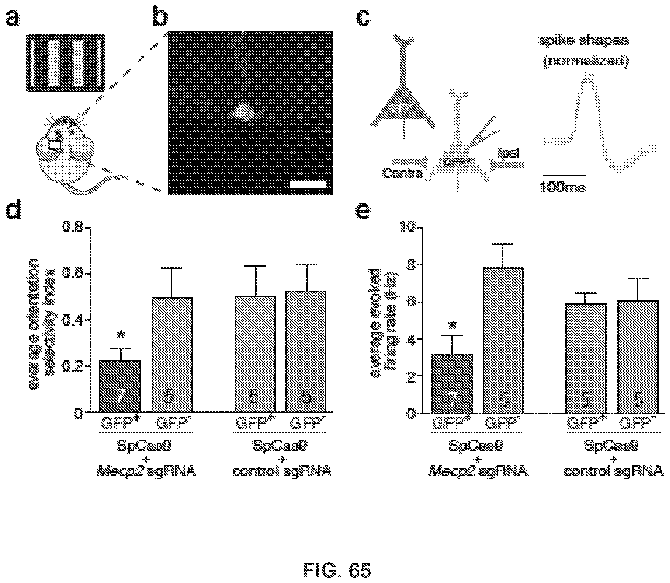

Figure 65 (Example 38) shows cell-autonomous defects in cellular response properties of neurons after CRISPR-mediated MeCP2 knockdown, (a) Cartoon showing *in vivo* experiment configuration from mouse visual cortex and visual stimulation parameter. GFP⁺ neuron is shown. Scale bar, 20 μm. (b) Cartoon showing recording configuration in layer 2/3 excitatory neurons that receive both contra- and ipsilateral eye specific input. Genome modified GFP⁺ cells are in green whereas unmodified cells are in gray. Normalized spike shape shows regular spiking excitatory neurons. (c,d) Average OSI (c) and evoked FR (d) were measured from GFP⁺ cells expressing *Mecp2* and control sgRNA, respectively (t-test, *p<0.05; numbers in graph indicate numbers of recorded cells; n=2-3 animals; error bars: s.e.m).

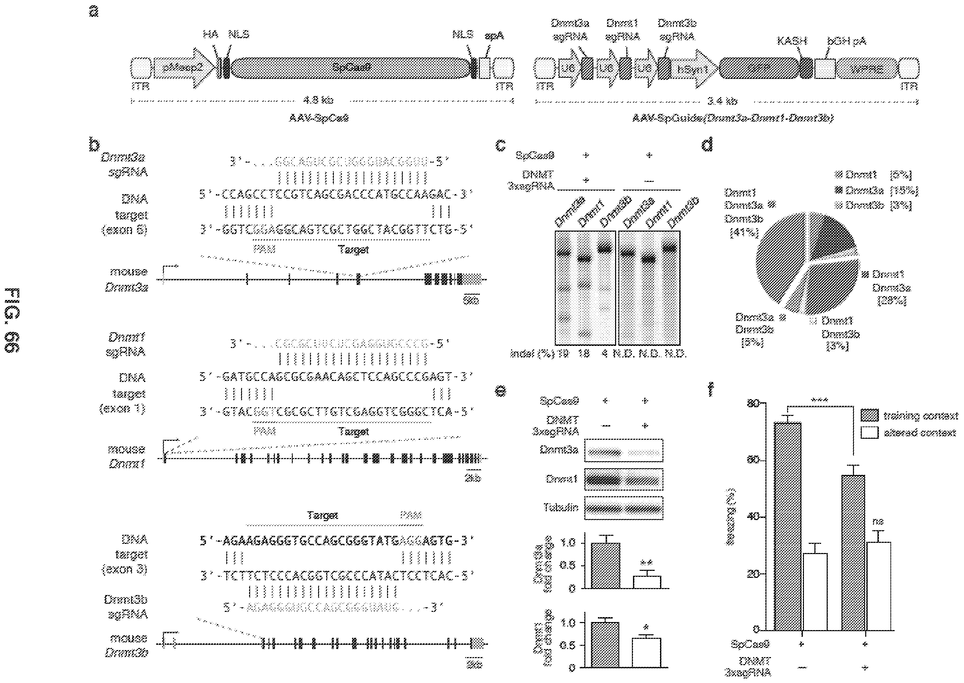

Figure 66 (Example 38) shows simultaneous, multiplex gene editing in the mouse brain. (a) Schematic illustration of CRISPR-Cas9 system designed for multiplex genome targeting. (b) Graphical representation of targeted DNMT mouse loci. Guide RNAs are indicated in blue. PAM sequences are marked in purple. (c) SURVEYOR™ assay gel showing modification of DNMTs loci in FACS sorted GFP-KASH positive cells, 4 weeks after AAV delivery in the DG region. (d) Deep sequencing-based analysis of DNMTs loci modification in single cells, showing co-occurrence of modification in multiple loci. (e) Western blot analysis for Dnmt3a and Dnmtl proteins after in vivo delivery of CRISPR-Cas9 system targeting DNMT family genes (top). Western blot quantification of Dnmt3a and Dnmtl protein levels in DG after in vivo CRISPR-Cas9 targeting (bottom; t-test, **p<0.001, *p<0.05, Dnmt3a: n=7; Dnmtl: n=5 from 5 animals; error bars: s.e.m). (f) Contextual learning deficits, 8 weeks after targeting of DNMT genes using SpCas9 in the DG region of hippocampus, tested in training and altered context (t-test, ***p<0.0001, n=18 animals, 2 independent experiments; error bars: s.e.m).

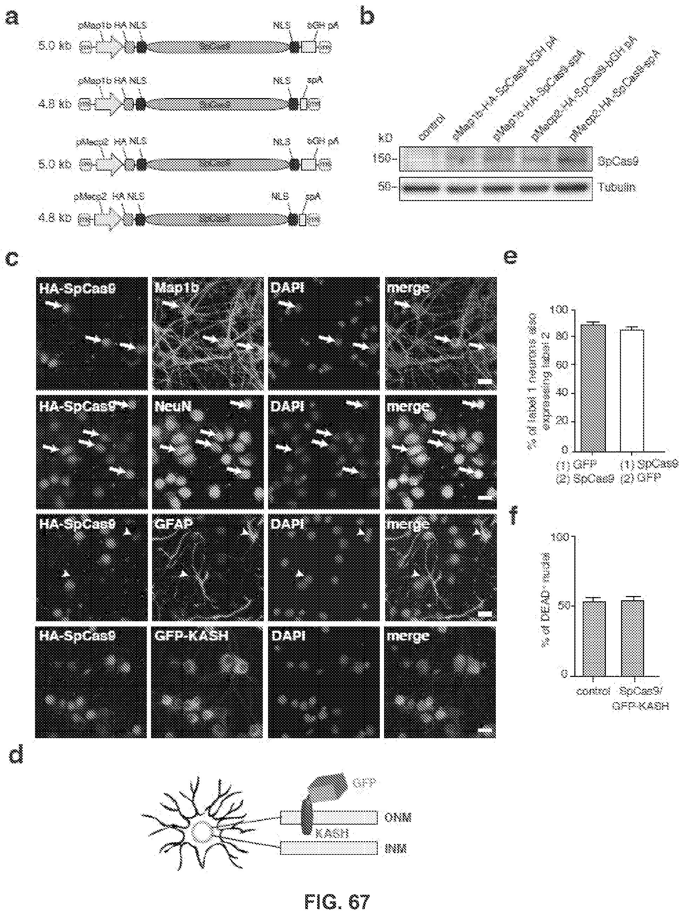

Figure 67 (Example 38) chows cloning and expression of HA-tagged SpCas9 (HA-SpCas9) for AAV packaging, (a) Schematic overview of different cloning strategies to minimize SpCas9 expression cassette size using short rat *Map1b* promotor (pMaplb), a truncated version of the mouse *Mecp2* promoter (pMecp2) and a short polyA motif (spA). **(b)** Western blot analysis of primary cortical neuron culture expressing HA-SpCas9 using different SpCas9 expression cassettes. (c) *Mecp2* promoter drives HA-SpCas9 (red) expression in neurons (Maplb, NeuN; arrows) but not in astroglia (GFAP, arrowheads). Co-expressioin of HA-SpCas9 with GFP-KASH is shown (bottom). Nuclei were labeled with DAPI (blue). Scale bars, 20 μm. **(d)** Schematic overview of GFP-labeling. Enhanced green fluorescent protein (GFP) fused to the nuclear transmembrane KASH domain and integration of GFP-KASH to the outer nuclear membrane is illustrated. **(e)** Co-infection efficiency calculation, showing populations of cell expressing both HA-SpCas9 and GFP-KASH (n=973 neurons from 3 cultures; error bars: s.e.m). **(f)** Cells were stained with LIFE/DEAD® kit 7 days after virus delivery. Quantification of DAPI⁺ and dead (DEAD⁺) cells (control n=518 DAPI⁺ nuclei; SpCas9/GFP-KASH n=1003 DAPI⁺ nuclei from 2 cultures; error bars: s.e.m). (ITR-inverted terminal repeat; HA - hemagglutinin tag; NLS - nuclear localization signal; spA-synthetic polyadenylation signal; U6 - PolIII promoter; sgRNA - single guide RNA; hSyn - human synapsin 1 promoter; GFP- green fluorescent protein; KASH - Klarsicht, ANC1, Syne Homology nuclear transmembrane domain;

bGH pA - bovine growth hormone polyadenylation signal; WPRE - Woodchuck Hepatitis virus posttranscriptional regulatory element).

Figure 68 (Example 38) shows targeting of *Mecp2* in Neuro-2a cells, (a) *Mecp2* targeting sequences and corresponding protospacer adjacent motifs (PAM). (b) Evaluation of 6 *Mecp2* sgRNAs co-transfected with SpCas9 into Neuro-2a cells. Locus modification efficiencies were analyzed 48 h after transfection using SURVEYOR™ assay.

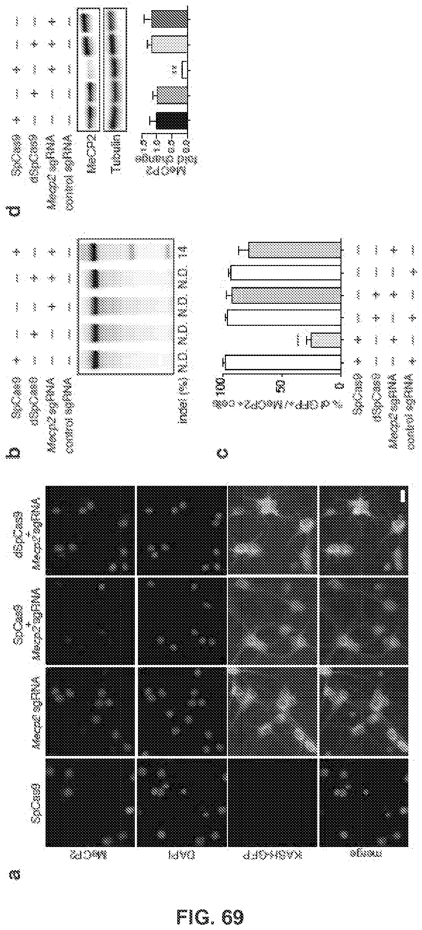

Figure 69 (Example 38) shows CRISPR-SpCas9 targeting of *Mecp2* in primary cortical neurons, (a) Immunofluorescent staining of MeCP2 (red) in cultured neurons 7 days after AAV-CRISPR transduction (green, GFP-KASH). Nuclei were labeled with DAPI (blue). Scale bar, 20 μm. **(b)** Evaluation of *Mecp2* locus targeting using SpCas9 or dSpCas9, together with *Mecp2* sgRNA or control (targeting bacterial *lacZ* gene) sgRNA, using SURVEYOR™ assay gel. **(c)** Quantification of MeCP2 positive nuclei in targeted population of neurons (GFP⁺). **(d)** Western blot of MeCP2 protein levels after CRISPR-SpCas9 targeting of *Mecp2* locus and quantification of MeCP2 protein levels (t-test, **p<0.001, n=5 from 3 cultures, error bars: s.e.m).

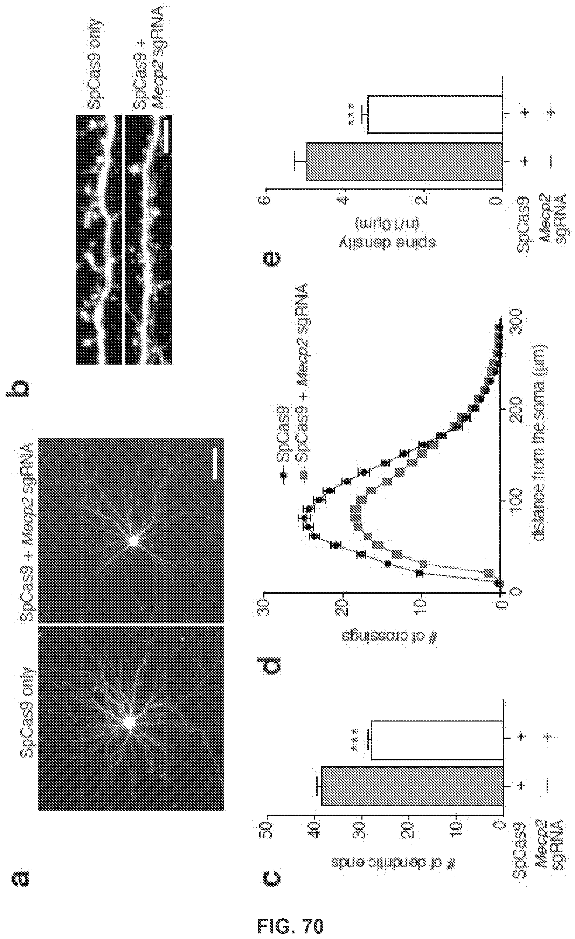

Figure 70 (Example 38) shows morphological changes in dendritic tree of neurons after SpCas9-mediated MeCP2 knockdown *in vitro.* (a) Reduced complexity of dendritic tree in neurons after CRISPR-SpCas9 targeting of *Mecp2* locus. Scale bar, 20 μm. (b) Changes in dendritic spines morphology in neurons targeted with SpCas9 and *Mecp2* sgRNA. Scale bar, 10 μm. Morphology of cells was visualized with co-transfection with mCherry construct. Cells for morphology analysis were chosen based on the result of Mecp2 staining, (c) Dendritic tree morphology assessed with number of dendritic ends and (d) Sholl analysis (*t*-test, ***p<0.0001, n=40 from 2 cultures), (e) Spine density quantification (*t*-test, ***p<0.0001, n=40 from 2 cultures, error bars: s.e.m).

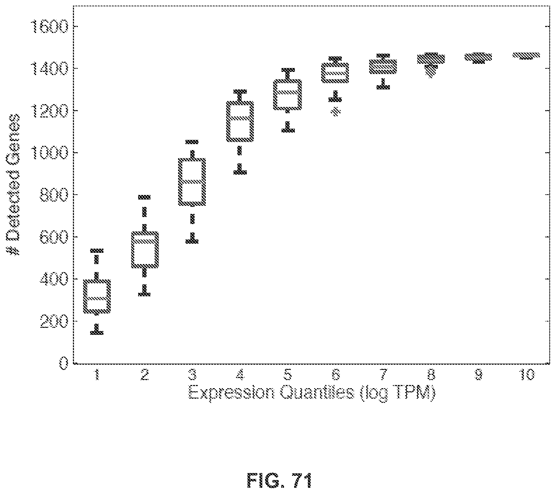

Figure 71 (Example 38) shows RNAseq of neuronal nuclei from control animals and SpCas9-mediated Mecp2 knockdown. Box plot presenting the number of detected genes across the RNA-seq libraries (19 libraries each of 100 nuclei taken from control sgRNA or *Mecp2* sgRNA transduced nuclei; n=4 animals/group) per quantile of expression level. All genes are divided to 10 quantiles by their mean log2(TPM+1) expression level, then for each quantile the number of genes that are detected (log2(TPM+1)>2) was counted in each sample. The three target sequences shown are SEQ ID NO: _545_, SEQ ID NO: _546_ and SEQ ID NO: _547_, for Dnmt3a, Dnmtl and Dnmt3b, respectively.

Figure 72 (Example 38) shows multiplex genome targeting of DNMT family members *in vitro*. **(a)** *Dnmt3a, Dnmt1* and *Dnmt3b* targeting sequences and corresponding protospacer adjacent motifs (PAM). **(b)** SURVEYOR™ nuclease assay analysis of Neuro-2a cells 48 hours after transfection with SpCas9 and DNMT 3xsgRNA vector targeting *Dnmt3a, Dnmt1* and *Dnmt3b* loci. Efficient genome editing of all three targeted genes is shown.

Figure 73 (Example 38) shows next generation sequencing of targeted *Dnmt3a, Dnmt1* and *Dnmt3b* loci. Examples of sequencing results of mutated *Dnmt3a* **(a),** *Dnmt1* **(b)** and *Dnmt3b* (c) loci after *in vivo* delivery of SpCas9 and DNMT 3xsgRNA into the mouse dentate gyrus. Green: wild-type sequence, red dashes: deleted bases, red bases: insertion or mutations. Red arrowheads indicate CRISPR-SpCas9 cutting site. The full sequences used in this figure are provide as SEQ ID NO: 3, SEQ ID NO: 4 , and SEQ ID NO: 5 for the Dnmt3a, the Dnmt1 and the Dnmt3b loci, respectively. They are:

SEQ ID NO: (Dnmt3a):
CCT CCG TGT CAG CGA CCC ATG CCA A
SEQ ID NO: (Dnmtl):
CCA GCG TCG AAC AGC TCC AGC CCG
SEQ ID NO: (Dnmt3b)
AGA GGG TGC CAG CGG GTA TAT GAG G

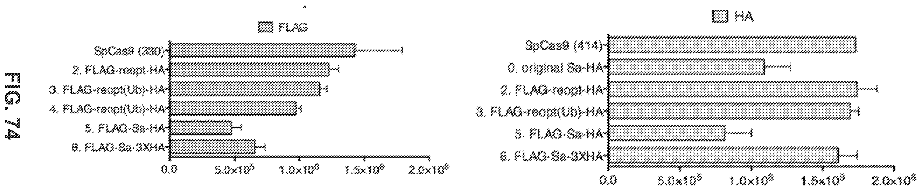

Figure 74 shows SaCas9 protein sequences are codon optimized ("reopt") and have their ubiquitination signals removed ("reopt(Ub)") for enhanced expression. Protein blots against FLAG- and HA-tagged SaCas9 show approximately 2-fold increased expression of optimized SaCas9 (reopt, #2-4) relative to the original constructs (#0, 5, and 6), and similar level as SpCas9 (SpCas9 330, top bar left panel; SpCas9 414, top bar right panel). The addition of 3xHA tagging (right panel #6) improves detection signal over the 1xHA tag (right panel #5) by ~2 fold.

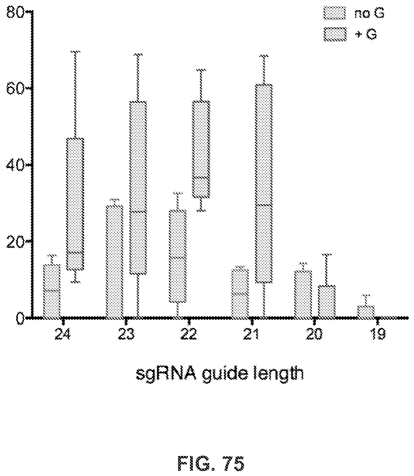

Figure 75 shows indel efficiency using sgRNAs transcribed by U6 promoter as-is (grey, left hand bar for each number of ntds) or appending a "G" (blue, right hand bar for each number of ntds and with a thicker border) to 5'-most position of sgRNA for SaCas9. Total sgRNA spacer lengths (including G) are indicated on the x-axis. Graph represents with aggregated data from 5 sgRNAs.

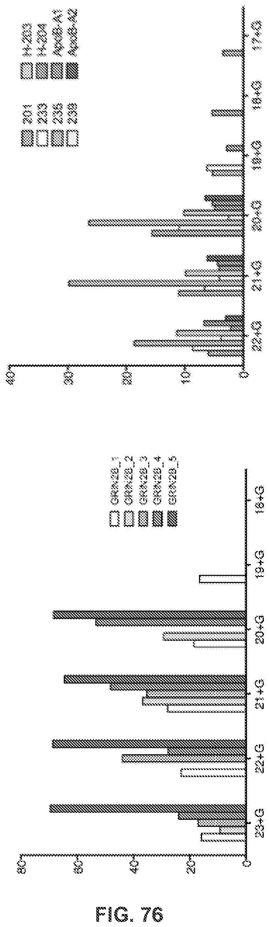

Figure 76 shows optimization of sgRNA spacer length (x axis). Graphs show indel formation with different lengths of sgRNA spacer in HEK (left) and Hepa (right) cells.

The figures herein are for illustrative purposes only and are not necessarily drawn to scale.

## DETAILED DESCRIPTION OF THE INVENTION

Also with respect to general information on CRISPR-Cas Systems, mention is made

➤ Multiplex genome engineering using CRISPR/Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013);

➤ RNA-guided editing of bacterial genomes using CRISPR-Cas systems. Jiang W., Bikard D., Cox D., Zhang F, Marraffini LA. Nat Biotechnol Mar;31(3):233-9 (2013);

➤ One-Step Generation of Mice Carrying Mutations in Multiple Genes by CRISPR/Cas-Mediated Genome Engineering. Wang H., Yang H., Shivalila CS., Dawlaty MM., Cheng AW., Zhang F., Jaenisch R. Cell May 9;153(4):910-8 (2013);

➤ Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. 2013 Aug 22;500(7463):472-6. doi: 10.1038/Nature12466. Epub 2013 Aug 23;

> Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5. (2013);

> DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013);

> Genome engineering using the CRISPR-Cas9 system. Ran, FA., Hsu, PD., Wright, J., Agarwala, V., Scott, DA., Zhang, F. Nature Protocols Nov;8(11):2281-308. (2013);

> Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013). [Epub ahead of print];

> Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27. (2014). 156(5):935-49;

> Genome-wide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. (2014) Apr 20. doi: 10.1038/nbt.2889, and

> Development and Applications of CRISPR-Cas9 for Genome Engineering, Hsu et al, Cell 157, 1262-1278 (June 5, 2014) (Hsu 2014),

and discussed briefly below:

- Cong *et al.* engineered type II CRISPR/Cas systems for use in eukaryotic cells based on both *Streptococcus thermophilus* Cas9 and also *Streptoccocus pyogenes* Cas9 and demonstrated that Cas9 nucleases can be directed by short RNAs to induce precise cleavage of DNA in human and mouse cells. Their study further showed that Cas9 as converted into a nicking enzyme can be used to facilitate homology-directed repair in eukaryotic cells with minimal mutagenic activity. Additionally, their study demonstrated that multiple guide sequences can be encoded into a single CRISPR array to enable simultaneous editing of several at endogenous genomic loci sites within the mammalian genome, demonstrating easy programmability and wide applicability of the RNA-guided nuclease technology. This ability to use RNA to program sequence specific DNA cleavage in cells defined a new class of genome engineering tools. These studies further showed that other CRISPR loci are likely to be transplantable into mammalian cells and can also mediate mammalian genome cleavage. Importantly, it can be envisaged that several aspects of the CRISPR/Cas system can be further improved to increase its efficiency and versatility.

- Jiang *et al.* used the clustered, regularly interspaced, short palindromic repeats (CRISPR)-associated Cas9 endonuclease complexed with dual-RNAs to introduce precise mutations in the genomes of *Streptococcus pneumoniae* and *Escherichia coli.* The approach relied on dual-RNA:Cas9-directed cleavage at the targeted genomic site to kill unmutated cells and circumvents the need for selectable markers or counter-selection systems. The study reported reprogramming dual-RNA:Cas9 specificity by changing the sequence of short CRISPR RNA (crRNA) to make single- and multinucleotide changes carried on editing templates. The study showed that simultaneous use of two crRNAs enabled multiplex mutagenesis. Furthermore, when the approach was used in combination with recombineering, in S. *pneumoniae,* nearly 100% of cells that were recovered using the described approach contained the desired mutation, and in *E. coli,* 65% that were recovered contained the mutation.

- Konermann *et al.* addressed the need in the art for versatile and robust technologies that enable optical and chemical modulation of DNA-binding domains based CRISPR Cas9 enzyme and also Transcriptional Activator Like Effectors

- As discussed in the present specification, the Cas9 nuclease from the microbial CRISPR-Cas system is targeted to specific genomic loci by a 20 nt guide sequence, which can tolerate certain mismatches to the DNA target and thereby promote undesired off-target mutagenesis. To address this, Ran *et al.* described an approach that combined a Cas9 nickase mutant with paired guide RNAs to introduce targeted double-strand breaks. Because individual nicks in the genome are repaired with high fidelity, simultaneous nicking *via* appropriately offset guide RNAs is required for double-stranded breaks and extends the number of specifically recognized bases for target cleavage. The authors demonstrated that using paired nicking can reduce off-target activity by 50- to 1,500-fold in cell lines and to facilitate gene knockout in mouse zygotes without sacrificing on-target cleavage efficiency. This versatile strategy enables a wide variety of genome editing applications that require high specificity.

- Hsu *et al.* characterized SpCas9 targeting specificity in human cells to inform the selection of target sites and avoid off-target effects. The study evaluated >700 guide RNA variants and SpCas9-induced indel mutation levels at >100 predicted genomic off-target loci in 293T and 293FT cells. The authors that SpCas9 tolerates mismatches between guide RNA and target DNA at different positions in a sequence-dependent manner, sensitive to the number, position and distribution of mismatches. The authors further showed that SpCas9-mediated cleavage is unaffected by DNA methylation and that the dosage of SpCas9 and sgRNA can be titrated to minimize off-target modification. Additionally, to facilitate mammalian genome engineering applications, the authors reported providing a web-based software tool to guide the selection and validation of target sequences as well as off-target analyses.

• Ran *et al.* described a set of tools for Cas9-mediated genome editing *via* non-homologous end joining (NHEJ) or homology-directed repair (HDR) in mammalian cells, as well as generation of modified cell lines for downstream functional studies. To minimize off-target cleavage, the authors further described a double-nicking strategy using the Cas9 nickase mutant with paired guide RNAs. The protocol provided by the authors

experimentally derived guidelines for the selection of target sites, evaluation of cleavage efficiency and analysis of off-target activity. The studies showed that beginning with target design, gene modifications can be achieved within as little as 1-2 weeks, and modified clonal cell lines can be derived within 2-3 weeks.

• Shalem *et al.* described a new way to interrogate gene function on a genome-wide scale. Their studies showed that delivery of a genome-scale CRISPR-Cas9 knockout (GeCKO) library targeted 18,080 genes with 64,751 unique guide sequences enabled both negative and positive selection screening in human cells. First, the authors showed use of the GeCKO library to identify genes essential for cell viability in cancer and pluripotent stem cells. Next, in a melanoma model, the authors screened for genes whose loss is involved in resistance to vemurafenib, a therapeutic that inhibits mutant protein kinase BRAF. Their studies showed that the highest-ranking candidates included previously validated genes NF1 and MED12 as well as novel hits NF2, CUL3, TADA2B, and TADA1. The authors observed a high level of consistency between independent guide RNAs targeting the same gene and a high rate of hit confirmation, and thus demonstrated the promise of genome-scale screening with Cas9.

• Nishimasu *et al.* reported the crystal structure of *Streptococcus pyogenes* Cas9 in complex with sgRNA and its target DNA at 2.5 A° resolution. The structure revealed a bilobed architecture composed of target recognition and nuclease lobes, accommodating the sgRNA:DNA heteroduplex in a positively charged groove at their interface. Whereas the recognition lobe is essential for binding sgRNA and DNA, the nuclease lobe contains the HNH and RuvC nuclease domains, which are properly positioned for cleavage of the complementary and non-complementary strands of the target DNA, respectively. The nuclease lobe also contains a carboxyl-terminal domain responsible for the interaction with the protospacer adjacent motif (PAM). This high-resolution structure and accompanying functional analyses have revealed the molecular mechanism of RNA-guided DNA targeting by Cas9, thus paving the way for the rational design of new, versatile genome-editing technologies.

• Wu *et al.* mapped genome-wide binding sites of a catalytically inactive Cas9 (dCas9) from *Streptococcus pyogenes* loaded with single guide RNAs (sgRNAs) in mouse embryonic stem cells (mESCs). The authors showed that each of the four sgRNAs tested

targets dCas9 to between tens and thousands of genomic sites, frequently characterized by a 5-nucleotide seed region in the sgRNA and an NGG protospacer adjacent motif (PAM). Chromatin inaccessibility decreases dCas9 binding to other sites with matching seed sequences; thus 70% of off-target sites are associated with genes. The authors showed that targeted sequencing of 295 dCas9 binding sites in mESCs transfected with catalytically active Cas9 identified only one site mutated above background levels. The authors proposed a two-state model for Cas9 binding and cleavage, in which a seed match triggers binding but extensive pairing with target DNA is required for cleavage.

• Hsu 2014 is a review article that discusses generally CRISPR-Cas9 history from yogurt to genome editing, including genetic screening of cells, that is in the information, data and findings of the applications in the lineage of this specification filed prior to June 5, 2014. The general teachings of Hsu 2014 do not involve the specific models, animals of the instant specification.

The disclosure
relates to the engineering and optimization of systems, methods and
compositions used for the control of gene expression involving sequence targeting, such as genome perturbation or gene-editing, that relate to the CRISPR-Cas system and components thereof. In advantageous embodiments, the Cas enzyme is Cas9.

The CRISPRS-Cas polynucleotide sequence is generally referred to herein as the guide, or even as guide RNA (sgRNA), although it will be appreciated that this terminology was not as commonplace previously. Furthermore, reference is made herein to a CRISPR-Cas9 system, although it will be appreciated that this is a broad reference to any Cas, provided it has a nuclease function either to induce a DSB, a nick or a double nick, although Cas9 is preferred and SaCas9 is particularly preferred.

Some of the key points in the present liver data are summarised below an flow through to post-mitotic cells in general, as liver cells are typically post-mitotic:

**AAV2/8**

Preferred delivery for the CRISPR-Cas system is through a viral vector. This vector may be a lentiviral vector or an AAV vector, as discussed at some length herein. Whet we have particularly showed is that AAV is a preferred example of a viral vector. Within that, we gone on to show that AAV8 and in particular AAV2/8 (AAV8 packaged with AAV2 packaging signal ITR) is useful in delivery to the liver, especially in vivo.

**Phenotypic Changes seen In Vivo**

As discussed elsewhere, we have been able to show, in vivo, that phenotypic change can be detected. This is a significant step forward as a deficiency often levelled at RNAi is that no lasting effect is seen. With the present invention, phenotypic change can be seen in the liver for the first time. A preferred arrangement to achieve this is to use that in Example 36. Important elements of this are preferred alone or in combination, namely:

- Sa Cas9;
- Use of a chimeric guide RNA comprising the guide, tracr sequence and tracr mate;
- For the tracr sequence, Sa tracr is preferable to recruit the Sa Cas9;
- AAV8 or more preferably AAV2/8;
- For experimental purposes, Rosa26 is a useful negative control;
- Although use of the CMV promoter in an AAV vector is helpful, use of a liver-specific promoter (for liver targeting) such as TBG is particularly effective;
- The target or targets may be wide-ranging as CRISPR has been shown to have broad applicability across targets, once they guides are successfully delivered and the Css9 enzymes are suitably expressed. However, preferred targets in the liver (against which the guides may be designed) nevertheless include one or more of: PCSK9; Hmgcr; SERPINA1; ApoB; and.or LDL.

Accordingly, in some embodiments it is particularly preferred that the Cas enzyme is an Sa Cas9. Preferably, the CRISPRS-Cas polynucleotide sequence is chimeric and preferably includes an Sa tracr where the Cas9 is an Sa Cas9. A viral vector may be used which is preferably AAV2/8. Furthermore, a liver-specific promoter is ideal and a preferred example is TBG. All of these may be used in combination to provide a chimeric CRISPRS-Cas polynucleotide sequence including an Sa tracr, wherein the Cas9 is an SaCas9, and the vector is AAV2/8, with at least the Cas9 under the control of a liver-specific such as TBG. Any of the above targets may be sued with this system, in particular ApoB due to its importance in obesity.

Yin and Anderson's later Nature Biotech Paper (NBT 2884, referenced herein) provides further support for the in vivo phenotypic changes that we have already shown.

Additional data that we provide in then adds further support by demonstrating efficient in vivo editing of somatic liver tissue via Cas9. Moreover, delivery via AAV2/8 and the use of an SaCas9 again show the usefulness of this particular approach in vivo. The preferred ApoB was again targeted.

Later examples 36 and 37 show excellent in vivo data for effecicacy in inducing a phenotypic change in vivo: specifically ApoB, a lipid metabolism gene, whilst Example 38 shows the applicability of the technique to post-mitotic cells, of which liver is an important example. Example 39 shows that multiple epitope tags are preferable for detection purposes.

Although viral vectors are preferred, in some embodiments, the use of cell penetrating peptides is a viable alternative and so is also preferred.

Example 36 showed that both genotypic and, crucially, phenotypic changes are seen with CRISPR-Cas systems. Not only that, but the CRISPR-Cas9 system was effective at inducing a phenotypic change *in vivo.* Specifically, the target was ApoB, a lipid metabolism gene. What is so encouraging is that ApoB can be said to be the "gold-standard" in liver delivery, and is widely used in mouse models of obesity. Liver is a preferred post-mitotic cell in some embodiments, although it may also be excluced in others. Either way, this work provides proof of principle that a phenotypic change is seen, even *in vivo,* and this is equally applicable to other post-mitotic cells. Indeed, Example 38 provides further proof of this in a separate tissue, brain, with post-mitotic neurones. Delivery in Example 36 was via intravenous injection. An AAV vector was used, as well as a Liver-specific promoter (TBG) for Cas9. Delivery through expression from a viral vector as seen here is an improvement over Anderson/Yin's (NBT 2884) use of hydrodynamic delivery as the delivery method, because hydrodynamic delivery requires several mls of fluid to be injected which is stressful on the murine body and can be fatal. Hydrodynamic delivery is best suited for delivery of plasmid (naked) DNA, whereas we have shown that packaging the guide and Cas9 sequences within a viral delivery vector is preferable in terms of greatly increased efficiency. Indeed, only relatively small volumes need to be introduced, and this can be done intravenously (i.v.), which is likely to be much more acceptable therapeutically. What was particularly encouraging was that not only was a genotypic change seen in a "gold-standard" gene for liver such as ApoB, but phenotypic changes were also recorded. Previous work with PCSK9 had shown genotypic, but not phenotypic changes, so the phenotypic changes seen with ApoB validate the plausibility of CRISPR delivery to, and its ability to effect phenotypic change in, the Liver. This is in combination with the more therapeutically acceptable means of delivery (i.v. compared to hydrodynamic delivery). As such, viral delivery of CRISPR-Cas9 system (guide and Cas9) is preferred, especially intravenously).

Potential targets include: PCSK9, HMGCR, APOB, LDLR, ANGPTL3, F8, F9/FIX, AAT, FAH, HPD, TAT, ATP7B, UGT1A1, OTC, ARH.

Accordingly, provided are methods of inducing a phenotypic change *in vivo* comprising administering the CRISPR-Cas9 system to the target cells, for instance the liver. Suitable delivery routes are described herein but i.v. injection is preferred in some embodiments. Viral vectors are preferred, particularly AAV, in particular AAV serotype 2/8.

Also provided is a CRISPR-Cas9 system comprising one or more guides targeting lipid metabolism genes, for instance ApoB. Methods of treating obesity, comprising administering said CRISPR-Cas9 system, are also envisaged. A mouse model comprising one or more liver gene knock down(s), especially of lipid metabolism gene(s), for instance including ApoB, are preferred.

Liver specific promoters for the Cas9 will be apparent but may include those mentioned herein. A preferred example is TBG.

As shown in Example 37, the guide may be 18-23 nucleotides in length. It may be 18-22, or 19-22, or 18-21, 20-22, but is preferably 22 and most preferably 21 nucleotides in length.

Also provided is proof of principle of successful packaging of a guide sequence into a SaCas9 intron. Accordingly, the CRISPR-Cas9 systems, wherein one or more guide sequences are packaged (positioned or inserted) into a Cas9 intron, are preferred.

The HI promoter can be used and may be preferable in some circumstances.

Expanding on the work by Ran (Cell, 154, 21 Aug 2013), the degree of overlap in the dual guide approach using a D10A Double-Nickase was investigated. Optimal results were shown between -5 and +1 bp (5' to 5'). Accordingly, it is prefer to use a dual guide approach to minimise off target effects. These preferably overlap, or come close to overlapping, at their 5' ends, on different stands of DNA at the genomic target . Preferably, the overlap is in the range of -5 to +1 bp. In these instances, it will be appreciated that the Cas9 is a double nickase, such as the preferred D10A variant.

Multiple or repeat epitope Tags are preferred for the Cas9. In particular, a triple epitope tag was shown in Example 39 to improve detection. The tag is preferably a repeat, more preferably a triple repeat. HA is a preferred Cas9 epitope tag. A triple HA epitope tag is, therefore, preferred in some embodiments.

Example 38 presents the following specific points. It provides:

- a first demonstration of successful AAV-mediated Cas9 delivery in vivo as well as efficient genome modification in post-mitotic neurons;
- for the development of a nuclear tagging technique which enables easy isolation of neuronal nuclei from Cas9 and sgRNA-expressing cells;
- a demonstration of applications toward RNAseq analysis of neuronal transcriptome;
  how electrophysiological studies and other techniques can be integrated with Cas9-mediated genome perturbation to determine phenotypic changes; and
- a demonstration of multiplex targeting and the ability to study gene function on rodent behavior using Cas9-mediated genome editing.

Based on this, it can be seen that Example 38 provides further proof of concept in two main areas:

- in the understanding and testing of gene function, including the creation and testing of models; and
- in gene therapy.

An additional aspect, discussed further below, is in relation to a method for Nuclear Tagging.

It will be appreciated that reference to CRISPR-Cas9 systems herein is a short-hand for referring to the Cas9 enzymes provided herein in combination with the guides or guides used to target one or more genomic sequences. Reference to guide(s) includes sgRNA, as well as the chimeric polynucleotide sequences described herein which comprises the guide sequences capabale of hybridising to target sequences in the genome of the subject, a tracr mate sequence and a tracr sequence.

The data essentially shows phenotypic changes resulting from gene knock down using two separate CRISPR-Cas9 systems according to the invention (guide RNA in combination with a Cas9 enzyme), in this case to successfully perturb gene function. The chosen tissue was brain tissue, but the results provide proof of principle for a wide range of post-mitotic tissues. This is an important distinction, because previous work has focussed on dividing cells (i.e. pre-mitotic).

In other words, whereas SpCas9 has been broadly used to engineer dividing cells, we demonstrate that SpCas9 can also be used to engineer the genome of postmitotic neurons. This is done with high efficiency via NHEJ-mediated indel generation to create knock downs, but therapeutic uses involving correction via the HDR mechanism (upon provision of a repair template) are also envisaged. Both are dependent on successful delivery and functional expression of the Cas9 and RNA guide or guides, which is shown here.

The fact that genotypic changes induced by the CRISPR-Cas9 systems then results in a phenotypic change is also important for both of the above areas (gene function and gene therapy).

The first CRISPR-Cas9 system employed guide sequences directed at (targeting) *Mecp2.* A dual vector CRISPR-Cas9 system, with one vector comprising the guide and one comprising the Cas9, was successfully employed showing further proof of principle for such dual vector systems. The dual vector CRISPR-Cas9 system was successfully delivered, via stereotactical injection, to two separate locations in the brain, specifically the Hippocampal dentate gyrus and the visual cortex. In both cases, gene perturbation of the same gene, *Mecp2,* was seen indicating that the dual vector system was successfully delivered and acted as expected, with transcription and functional activity in the Cas9 enzyme (in this case an SpCas9), and successful recruitment of the Cas9 to the genomic target sequence by the guide sequences.

AAV-mediated in vivo delivery of SpCas9 and sgRNA provides a rapid and powerful technology for achieving precise genomic perturbations within intact neural circuits. AS such, the vector used was an AAV vector, adding further evidence for their use in general and in dual vector CRISPR-Cas9 systems in particular, especially in post-mitotic cells and tissues, and in particular in the brain.

It will of course be appreciated that the choice of promoter is important in achieving expression from the CRISPR-Cas9 system, in particular the Cas9 or both guide(s) and Cas9. Suitable examples for cell and cell lifecycle stage specificity can be determined from the literature. Nevertheless, we provide some non-limiting examples: TBG, a liver-specific promoter and is used here to drive expression of SaCas9; the HI promoter; a truncated HI promoter; the U6 promoter. Also, as guides do not necessarily need a specific promoter, one or more guides could similarly packaged into a/the Cas9 intron.

The second CRISPR-Cas9 system used included a multiplex approach. One key advantage of the SpCas9 system is its ability to facilitate multiplex genome editing. This second system successfully targeted three or more genes from the same family (in this case, *Dmnt1, 3a* and *3b)* by including suitable guides and resulted in stable knockouts of multiple genes. This has broad implications for probing the function of not only individual genes, but also whole gene families, in the tissues of living animals. This is particularly important for tissues such as the brain where this has not been possible before, or could only be achieved through long years of classical genetics. We have shown that single or multiple gene perturbation (even complete knock down) can occur in post-mitotic cells in a normal animal. However, this could equally apply to a model organism (for instance one *already* carrying a gene mutation or perturbation or comprising altered expression of some kind) or a transgenic organism, lending a quick alternative to existing methods of producing model organisms and using model organisms to understand gene function. Further guides (and/or whole CRISPR-Cas9 systems) could be employed to make later rounds of gene perturbations and/or reinstatements (restoring gene function for instance by correction of the perturbed gene through provision, for instance, of a repair template, such as ssDNA suitable for HDR) within the same organism.

In fact, in general, SpCas9-mediated targeting of single or multiple genes can recapitulate morphological, electrophysiological, and behavioral phenotypes observed using classical, more time-consuming genetic mouse models.

Alternatively to knocking down whole gene families or related genes, the data here also provides proof of principle that simultaneous knock down or three or more unrelated genes is equally feasible. This is applicable across all tissues, but is particularly strongly presented in respect of post-mitotic tissues, especially the brain.

Another useful aspect of the work is that it showed that a combined, or integrated, approach could be taken to studying gene function, employing CRISPR to effect a genotypic change and then using classical tools such as electrophysiology (particularly relevant to brain and CNS tissue), biochemical, sequencing, electrophysiological, and/or behavioral readouts to establish what, if any, phenotypic changes result from the genotypic change induced by the CRISPR-Cas9 system. For example in the brain, this allows us to study the function of individual as wells as groups of genes in neural processes and their roles in brain disorders *in vivo.*

The successful perturbation of genes in this work is equally applicable to correction or reinstatement of gene function, i.e. the use of CRISPR-Cas9 systems in gene therapy. This is particularly in relation to targeting post-mitotic cells, especially the brain.

In general, the use of CRISPR-Cas9 systems show improvements over existing techniques such as Zn fingers, which take a long time to design and produce and cannot multiplex and shRNA, which has too many off-target effects whereas CRISPR off-target effects can be minimised by using double-nicakse approaches.

## Targeting of Tissues

The work herein supports the use of CRISPR-Cas9 systems to target genes in post-mitotic cells through delivery of the CRISPR-Cas9 system to the appropriate location (i.e. to cells within the organs or tissues of interest). Preferred tissues are within the following organs:

- Kidney;
- Digestive System including the stomach, pancreas, duodenum, ileum and/or colon;
- Heart;
- Lung;
- Brain, in particular neurones, and/or CNS in general;
- Eye, including retinal tissue;
- Ear, including the inner ear;
- Skin;
- Muscle;
- Bone; and/or
- Liver in general.

It will be appreciated that many of the above may comprise pre-mitotic cells, but that this aspect of the invention is directed to post-mitotic cells or tissues within those organs.

It is preferred that the organ is the kidney or the brain. Within the brain, the data specifically shows delivery to the Hippocampal dentate gyrus and the visual cortex, which are preferred tissues, although other tissues including any one or more of the following: primary motor cortex, primary auditoty cortex, primary somatosensory cortex, cerebellum, main olfactory bulb, prefrontal cortex, endopiriform nucleus, amygdala, substantia nigra, striatum, pallidum, thalamus, hypothalamus, Parabranchial nucleus, superior olivary complex, cochlear nuclei, mammillary nuclei, are also preferred in some embodiments.

Cells from the brain, and neurons in particular, are especially preferred.

The choice of promoter to drive expression of the CRISPR-Cas9 system, especially the Cas9 is important, as mentioned above. To be considered when selecting a promoter are the cell cycle stage (early/late) and the cell type as promoters will be specific for one of more cell types and cell-cycle stages. Suitable promoters may include any one or more of the following, in some embodiments:

| Cell Type | Promoter |
| --- | --- |
| Excitatory neurons | CamkII |
| Fast spiking interneurons | Parvalbumin |
| All interneurons | vGAT |
| Dopaminoceptive neurons | DR1 |
| Dopaminoceptive neurons | DR2 |
| Astroglia | GFAP |
| Activated neurons | Arc |

The dual vector CRISPR-Cas9 system used in targeting the brain, in particular the Hippocampal dentate gyrus, packaged SpCas9 and sgRNA expression cassettes on two separate viral vectors. Cas9s, in particular SpCAs9s, are therefore preferably delivered by adenoviral vectors, especially AAV (i.e. as AAV-SpCas9). Guides are preferably delivered as sgRNA expression cassettes by adenoviral vectors, especially AAV (i.e. as AAV-SpGuide). A preferred route for this tissue (the Hippocampal dentate gyrus) and for the brain in general is stereotactical injection.

## Understanding And Testing Of Gene Function, And The Creation And Use Of Models To So Do

Conditions which could be looked at include Huntingdon's, but essentially include any condition found in post-mitotic cells and especially those that may benefit from being studied *in vivo* or lack a useful model.

As mentioned above, CRISPR-Cas9 systems can be used to interrogate the function of one or more genes in post-mitotic cells. This may be achieved through delivery and expression of the CRISPR-Cas9 system to the post-mitotic cell, wherein the guide(s) of the CRISPR-Cas9 system are designed to recruit the Cas9 to the genomic target or targets of interest. Equally, where the Cas9 is already comprised within the post-mitotic cell, protein (transcribed) form, then delivery of the guides to the post-mitotic cell will suffice. Where the Cas9 is already comprised within the post-mitotic cell, in polynucleotide (untranscribed), then delivery of the guides to the post-mitotic cell as well as induction of transcription of the Cas9 polynucleotide will be necessary. Having the Cas9 under the control of an inducible or repressible promoter, such as the tet (tetracycline) on-off system may be advantageous here.

One aspect that is particularly promising is the integration of CRISPR techniques with phenotypic assays to determine the phenotypic changes, if any, resulting from gene perturbations, especially knock downs. For instance, Example 40 shows what can be achieved with targeted genomic perturbations coupled with quantitative readouts to provide insights into the biological function of specific genomic elements. In particular, Cas9-mediated in vivo genome editing in the brain can also be coupled with electrophysiological recording to study the effect of genomic perturbation on specific cell types or circuit components. In a broader sense, use of the CRISPR-Cas9 systems (to provide Cas9-mediated genomic perturbations) can be combined with biochemical, sequencing, electrophysiological, and behavioral analysis to study the function of the targeted genomic element.

Thus in one aspect, there is provided: a method of interrogating the function of one or more genes in a post-mitotic cell, comprising: inducing a deficient genotype or gene knock down as described below; and determining changes in expression of the one or more genes in the condition thereby interrogating the function of the one or more genes.

Optionally, the method may also include: transplanting the second population of cells into the subject thereby inducing the condition associated with the deficient genotype or gene knock down. This may be prior to the determining step.

The following applies broadly to appropriate aspects of the invention. The cell may be in a subject, such as a human, animal or model organism, so that gene function is interrogated *in vivo.* However, it is also envisaged that the cell may be ex vivo, for instance in a cell culture or in a model organ or organoid. In some embodiments, the method may include isolation a first population of cells from the subject, optionally culturing them and transducing them with one or more CRISPR-Cas9 systems. Further optional culturing may follow. Transplantation of the transduced cells back into the subject may then occur.

The cell may be frrom any of the tissues or organs described herein. The brain is one preferred example, providing for said method of interrogating the function of one or more genes, wherein the post-mitotic cell is a brain cell, for instance a neurone. Particularly in vivo, this allows for the interrogation of gene function on animal behaviour. The animal is preferably a mammal, for instance a rodent. Given the complexity of the nervous system, which consists of intricate networks of heterogeneous cell types, being able to efficiently edit the genome of neurons in vivo enables direct testing of gene function in relevant cell types embedded in native contexts. This is supported by our data where knockout mice showed impaired memory consolidation when tested under trained context conditions Results herein demonstrate that CRIPSR-Cas9-mediated knockout of DNMT family members in dentate gyrus neurons is sufficient to probe the function of genes in behavioral tasks.

This shows the versatility of Cas9s in facilitating targeted gene knockout in the mammalian brain in vivo, for studying genes functions and, in particular, for dissection of neuronal circuits. Introducing stable knockouts of multiple genes in the brain of living animals will have potentially far-reaching applications, such as causal interrogation of multigenic mechanisms in physiological and neuropathological conditions.

The specifics of this work are that Applicants chose the mouse Mecp2 promoter (235 bp, pMecp2)7 and a minimal polyadenylation signal (48 bp, spA) based on their ability to achieve sufficient levels of SpCas9 expression in cultured primary mouse cortical neurons. Mecp2 gene, plays a principal role in Rett syndrome, a type of autism spectrum disorder. To target Mecp2, we first designed several sgRNAs targeting exon 3 of the mouse Mecp2 gene and evaluated their efficacy using Neuro-2a cells. The most efficient sgRNA was identified using the SURVEYOR nuclease assay. The delivery was via stereotactical injection of a mixture (1:1 ratio) of high titer AAV-SpCas9 and AAV-SpGuide. We also successfully tested the possibility of multiplex genome editing in the brain Applicants designed a multiplex sgRNA expression vector consisting of three sgRNAs in tandem, along with GFP-KASH for nuclei labelling.

Thus, also provided are methods of inducing conditions characterised by one or more gene knockdowns in a post-mitotic cell. Examples of such conditions are numerous, but may include Rett syndrome, as exemplified. Suitable promoters will be apparent, and the Mecp2 promoter is ideal for Rett syndrome. One way to select a promoter to drive expression of the CRISPR-Cas9 system, in particular the Cas9, is to select the promoter for the gene of interest.

Thus in one aspect, there is provided: a method of inducing conditions characterised by one or more deficient genes (or genotyoes) or gene knockdowns in a post-mitotic cell, comprising:

transducing a first population of cells with a non-naturally occurring or engineered composition comprising a vector system comprising one or more vectors comprising

I. a first regulatory element operably linked to a CRISPR-Cas system chimeric RNA (chiRNA) polynucleotide sequence, wherein the polynucleotide sequence comprises

(a) one, two, thee, four or more guide sequences capable of hybridizing to three or more target sequences in genome of the subject,
(b) a tracr mate sequence, and
(c) a tracr sequence, and

II. a second regulatory element operably linked to an enzyme-coding sequence encoding a CRISPR enzyme comprising at list one or more nuclear localization sequences (NLSs), wherein (a), (b) and (c) are arranged in a 5' to 3' orientation,

wherein components I and II are located on the same or different vectors of the system, wherein when transcribed, the tracr mate sequence hybridizes to the tracr sequence and the guide sequence direct sequence-specific binding of CRISPR complexes to the target sequence,
wherein the CRISPR complex comprises the CRISPR enzyme complexed with (1) the guide sequence that is hybridized to the target sequence, and (2) the tracr mate sequence that is hybridized to the tracr sequence,
wherein the CRISPR enzyme alters the genome of the first population of cells to obtain a second population of cells bearing the one or more deficient genes or knocked down genes.

Optionally, the method may also include: isolating a first population of cells from the subject.

Optionally, the method may also include: transplanting the second population of cells into the subject thereby inducing the proliferative condition.

This may involve inducing a non-functional (which include partially non-functional) genotype into the target cell, to thereby provide a model for study (including future restoration of the functional genotype).

CRISPR-Cas9 systems can also be used to facilitate the study of gene functions in cellular assays by enabling targeted knockout in post-mitotic neurons.

Methods for delivering nucleotides to neuronal cells are well known and reviewed in The Journal of Neuroscience, by Karra and Dahm (5 May 2010, 30(18): 6171-6177; doi: 10.1523/JNEUROSCI.0183-10.2010). Examples include electrical transfection methods (such as electroporation, nucleofection, and single-cell electroporation); chemical transfection methods (such as Ca2+ phosphate co/precipitation and lipofection); viral delivery (such as Adenoviral, Adeno-Associated Virus (AAV), Lentiviral and Herpes Simplex Virus); and physical transfection methods (such as microinjection and biolistics (DNA-coated gold particles). All of these can be used for delivery of the CRISPR-Cas9 system, but lipofection or viral methods are preferred, especially AAV or Lentiviral.

## Models

Models are provided with single or multiple genes knocked down. An example would be a rodent model for Rett syndrome, a Mecp2 knock down. Others include Dmnt family knock downs, specifically Dmnt1, 3a and 3b knock downs. As such, models studying neurological conditions are provided. All that needs to be done is to identify the target genes of interest, design suitable guide(s) and include these in a suitable CRISPR-Cas9 system and deliver it to the post-mitotic cell(s) whether in vivo or ex vivo, as required. For instance, the models may have altered dendritic tree morphology and/or spine density are provided.

As mentioned above, models tissues are also provided, such as oraganoids or "Liver on a chip" or non-liver equivalents thereof such as ear, kidney and brain tissues, for instance on a chip or supported in a scaffold. Animal models and model tissues are preferred. These may be already transformed with Cas9 so that they comprise Cas9 in nucleotide or protein form, as mentioned above. These have the advantage that Cas9 does not need to be delivered alongside the guide(s) and this in turn may allow for a much greater degree of (guide) multiplexing to be accommodated within the delivery vectors. Again, use of inducible or repressible systems such as tet-on or tet-off, may be advantageous here.

All of these models are obtainable using the CRISPR-Cas9 system as described above. Due to the versatility of the CRISPR-Cas9 system, the range of possible models, whether human, rodent, mammalian or otherwise is hugely diverse and this can be established by simple selection of appropriates guide(s). Methods of creating such models are also provided comprising

## Gene Therapy

The data in Example 40 focuses on gene perturbation, primarily knock down. Gene knock down is likely to be only a small, if important, part of the total quorum of possible applications of CRISPR-Cas9 systems to gene therapy. As already shown in the Yin and Anderson paper (Nature Biotech 2884 published online 30 March 2014), a functional phenotype can be restored following correction of a deficient mutation in hereditary tyrosinemia type I (HTI), an otherwise fatal condition caused by mutation of fumarylacetoacetate hydrolase (FAH) (G to A in the last nucleotide in exon 8) which causes skipping of exon 8 during splicing and results in the formation of a truncated, unstable FAH protein, leading to accumulation of toxic metabolites. Correction of the A mutation back to the wild-type G geneotype resulted in a restored phenotype.

As such, the approaches taken herein demonstrate that the instant invention can plausibly be applied to gene therapy. In particular, the dual vector approach, the nuclear tagging approach, the specifics of the brain delivery (the form of injection, the promoters and/or viral vectors used), as well as the multiplexing (use of multiple guides for multiple targets either within the same or within different genes) could equally be applied to correctional gene therapy (i.e. where a deficient genotype is corrected) as to the exemplified gene knock down. The main difference between correctional gene therapy and gene knock down is that in order to correct a deficient genotype, such as a point mutation (for instance in Cystic Fibrosis, see ref Schwank et al, Cell Stem Cell 13, 653-658 5 Dec 2013), it is advantageous to provide a repair template to stimulate the HDR mechanism and ideally provide a suitable Cas9 nickase as well.

Accordingly, the present vectors preferably target post-mitotic cells. Where the guide or guides target a deficient genotype, are preferably also provided with a repair template, for instance ssDNA corresponding to the corrected sequence (a genotype providing functional phenotype). Repair templates are described herein. The Cas9 may be provided in the same or a different vector from the guide or guides. The vectors are preferably viral vectors, more preferably adenoviral vectors and most preferably AAV vectors. Delivery to the cells is preferably by intravenous injection or by stereotactic injection, as appropriate. The selection of the promoter may be important and advantageous examples are provided herein.

Methods of treating genetic diseases or conditions caused by, or associated with, a deficient genotype in post-mitotic cells are provided, comprising delivery of the CRISPR-Cas9 system to the appropriate cell. A deficient genotype may be the non-wild type genotype. In particular, single point mutations and/or monogenic disorders are especially suited to treatment using CRISPR-Cas9 systems. Where multiple genes require editing or correcting, then a multiplex approach may be used to target them all simultaneously. Alternatively, two or more rounds of different CRISPR-Cas9 systems could be envisaged. Preferably, the wild-type genotype is corrected for. It does not necessarily have to be the most common genotype, provided that function is restored or improved in the phenotype.

An example of a restored phenotype is the restoration of hearing to restore VGLUT3 function and hence hearing in the inner ear of mice (Omar Akil, Rebecca P. Seal, Kevin Burke, Chuansong Wang, Aurash Alemi, Matthew During, Robert H. Edwards, Lawrence R. Lustig. Restoration of Hearing in the VGLUT3 Knockout Mouse Using Virally Mediated Gene Therapy. Neuron, 2012; 75 (2): 283 DOI: 10.1016/j.neuron.2012.05.019). This was using AAV-mediated delivery of VGLUT3 itself, but it is entirely plausible that CRISPR-Cas9 system could also be used, preferably also using AAV vectors, to target the cells of the inner ear and correct the non-functional VGLUT3 genotype, with similar phenotypic consequences, namely restoration of hearing. As such, delivery of the CRISPR-Cas9 system to the inner ear, preferably using AAV vectors, is preferred, thus treating hearing loss. Indeed, restoration of gene function in sensory organs such as the eye, including the retina, nose and ear (particularly the inner ear) is preferred.

A relatively recent overview, which includes a discussion of disorders in post-mitotic tissues (eye, ear and beyond) is Kaufmann et al (EMBO Mol Med (2013(, 5, p1642-1661). This confirms the usefulness of AAV in the correction of monogenic disorders in post-mitotic tissues. It states that "in combination with other characteristics such as low inflammatory activity, they have shown to have an excellent safety profile and are therefore highly attractive tools for *in vivo gene* therapy. Indeed, Glybera® is a recombinant AAV for direct intramuscular injection..." The paper, with citations, reviews gene therapy in the retina, central nervous system, liver, skeletal and cardiac muscle as target tissues. And, with citations, indicates that "initial studies exploited the prototype AAV serotype 2 vector, the portfolio of AAV vectors has recently been expanded to include additional serotypes and even engineered capsids." Kaufmann and the documents cited in Kaufmann are hereby incorporated herein by reference.

## RNAseq Analysis of the Transcriptome

The combination of SpCas9-mediated genome perturbation and population level RNAseq analysis provides a way to characterize transcriptional regulation and suggest genes that may be important to specific functions or disease processes in the cells under consideration. In particular, the cells are from the brain, in particular neurones. Fast-acting techniques such as a CRISPR-Cas9 system are advantageous in studying the transcriptome, which is, by its nature, transient. As such, the use of CRISPR-Cas9 systems according to the present invention in analysis of the transcriptome (RNAseq) are provided.

## Nuclear Tagging Method

To facilitate immunofluorescence identification of SpCas9-expressing neurons, we tagged SpCas9 with a HA-epitope tag (derived from human influenza hemagluttinin, a general epitope tag widely used in expression vectors).

For the AAV-SpGuide vector, we packaged an U6-sgRNA expression cassette as well as the green fluorescent protein (GFP)-fused with the KASH nuclear trans-membrane domain driven by the human Synapsin I promoter. The GFP-KASH fusion protein directs GFP to the outer nuclear membrane and enables fluorescence-based identification and purification of intact neuronal nuclei transduced by AAV-SpGuide.

Accordingly, the vectors are preferably adapted in a similar fashion. Thus, the vectors are provided wherein the Cas9 is tagged with an epitope tag, such as the HA-epitope tag. The Cas9 may be any of the Cas9s described herein, for instance Sp or SaCas9 and may be any variant (such as D10A double nickase etc), provide that it is or can be tagged appropriately.

The vectors may also be adapted so that the guide RNA is packaged within an expression cassette, which comprises:

- a reporter protein; and
- optionally, a suitable promoter for the guide RNA, such as U6;

wherein the reporter protein is fused with a nuclear trans-membrane domain operably linked to a suitable promoter therefor.

The reporter protein is preferably a fluorescent protein, for instance one of green, red or yellow fluorescent proteins (GFP, RFP, YFP) and so forth.

Examples of nuclear trans-membrane domains include KASH-like domains, Sun2 domains, LEM domains. In some preferred embodiments, the nuclear trans-membrane domain is the KASH nuclear trans-membrane. Preferably, the promoter for the trans-membrane domain is the human Synapsin I promoter; see also documents cited herein.

This tagging approach may be used within single or dual vector systems, but preferably within dual vector systems as space is limited in single vector systems and the need for separate tags lessened as well.

Furthermore, each aspect of this tagging technique can be used independently of the other, so that epitope tagging of the Cas9 can be used alone, or the reporter/fluorescent protein cassette approach can be used alone, or more preferably both can be used together.

Kanasty and Anderson (Nature Materials, Vol 12 Nov 2013) is a useful review, initially submitted on 11 March 2013 and published online on 23 October 2013 of delivery of RNAi. Due to the similarities between RNAi and CRISPR guide sequences, the teaching of this and other art in respect of RNAi is informative for the mechanisms of delivering the guides in our CRISPR-Cas9 system. Some of the techniques described are also be suitable for delivery of the Cas9 as well. In some instance is may be useful to deliver the guides of our CRISPR-Cas9 system separately from the Cas9. This may be as part of a dual-vector delivery system, where the vectors are considered in the broadest light as simply any means of delivery, rather than specifically viral vectors. It is envisaged that the Cas9 may be delivered via a viral vector and that guides specific to genomic targets are delivered separately. As discussed herein, the guides could be delivered via the same vector types as the Cas9, for example a dual-vector system where the Cas9 is delivered in an AAV vector and the guide(s) are delivered in a separate AAV vector. This can be done substantially contemporaneously (i.e. co-delivery), but it could also be done at separate points in time, separated even by weeks or months. For example, if a first round of CRISPR-Cas9 systems have been delivered, but then it is subsequently required to provide further guides, then the original Cas9 which is hopefully still functional in the target cells may be re-used. If the Cas9 is under the control of an inducible promoter, then induction of transcription of new CAs9 in the target cells is preferred. Equally, if a CAs9-expressing model provided for herein is used, then only delivery of guide(s) is necessary. Accordingly, where delivery of guide(s) is required separately from Cas9, then it may be delivered in much the same way as RNAi. As such, the review by Kanasty is helpful in pointing out a number of known approaches that are suitable, with particular focus on the liver, although the means of delivery are generally appropriate for a broad range of cells. Examples include:

- "Liposomal delivery system, as well as siRNA conjugated to lipophilic molecules, interact with serum lipoproteins and subsequently gain entry into hepatocytes that take up those lipoproteins;"
- PEGylation;
- Conjugates such as:

  a. Dynamic Polyconjugates (DPCs, 10nm nanoparticles), which have been shown to deliver RNAi to successfully supress ApoB (thereby crossing over with our work on targeting ApoB via a CRISPR-Cas9 system); and
  b. Triantennary GalNAc conjugates
  c. are "both highly effective" especially GalNAc;

- Other nanoparticles include:

d. Cyclodextrin Polymer nanoparticles (CDP), including additional formulation components such as adamantine-PEG (AD-PEG) and adamantine-PEG-transferrin (AD-PEG-Tf) ;

e. Lipid Nanoparticles (LNP), including cationic or ionisable lipids, shielding lipids, cholesterol and endogenous or exogenous targeting ligands. An example of an endogenous targeting ligand is Retinol Binding protein (RBP) useful for targeting hepatic and pancreatic stellate cells, which express the RBP receptor. An example of an exogenous targeting ligand is GalNac, which also targets the liver via the asialoglycoprotein receptor on hepatocytes. A combined approach is seen in Anlylams ALN-VSP;

- "Fenestrations in the liver endothelium allow molecules 100-200 nm in diameter to diffuse out of the bloodstream and gain access to the hepatocytes and other liver cells";
- Ligands such as GalNAc are suitable for delivery to non-parenchymal liver cells expressing the mannose receptor, and to hepatocytes where conjugation of suitable siRNA to a GalNAc ligand has been shown to successfully supress PCSK9; and
- Oligonucleotide nanoparticles (ONPs) composed of composed of complimentary DNA fragments designed to hybridise into a pre-defined 3D structure. Using suitable 3' overhand sequences, 6 siRNA strands could be attached to each particle, even at a specified position. The hydrodynamic diameter was about 29nm.

These approaches are preferred in some embodiments for delivery of at least the guides for a CRISPR-Cas9 system. Especially preferred are Dynamic Polyconjugates or the use of an endogenous targeting ligands such as Retinol Binding protein or exogenous targeting ligands such as GalNac.

Particle delivery systems and/or formulations:

Several types of particle delivery systems and/or formulations are known to be useful in a diverse spectrum of biomedical applications. In general, a particle is defined as a small object that behaves as a whole unit with respect to its transport and properties. Particles are further classified according to diameter Coarse particles cover a range between 2,500 and 10,000 nanometers. Fine particles are sized between 100 and 2,500 nanometers. Ultrafine particles, or nanoparticles, are generally between 1 and 100 nanometers in size. The basis of the 100-nm limit is the fact that novel properties that differentiate particles from the bulk material typically develop at a critical length scale of under 100 nm.

As used herein, a particle delivery system/formulation is defined as any biological delivery system/formulation which includes a particle in accordance with the present invention. A particle in accordance with the present invention is any entity having a greatest dimension (e.g. diameter) of less than 100 microns ($\mu$m). In some embodiments, inventive particles have a greatest dimension of less than 10 $\mu$m. In some embodiments, inventive particles have a greatest dimension of less than 2000 nanometers (nm). In some embodiments, inventive particles have a greatest dimension of less than 1000 nanometers (nm). In some embodiments, inventive particles have a greatest dimension of less than 900 nm, 800 nm, 700 nm, 600 nm, 500 nm, 400 nm, 300 nm, 200 nm, or 100 nm. Typically, inventive particles have a greatest dimension (e.g., diameter) of 500 nm or less. In some embodiments, inventive particles have a greatest dimension (e.g., diameter) of 250 nm or less. In some embodiments, inventive particles have a greatest dimension (e.g., diameter) of 200 nm or less. In some embodiments, inventive particles have a greatest dimension (e.g., diameter) of 150 nm or less. In some embodiments, inventive particles have a greatest dimension (e.g., diameter) of 100 nm or less. Smaller particles, e.g., having a greatest dimension of 50 nm or less are used in some embodiments of the invention. In some embodiments, inventive particles have a greatest dimension ranging between 25 nm and 200 nm.

Particle characterization (including e.g., characterizing morphology, dimension, etc.) is done using a variety of different techniques. Common techniques are electron microscopy (TEM, SEM), atomic force microscopy (AFM), dynamic light scattering (DLS), X-ray photoelectron spectroscopy (XPS), powder X-ray diffraction (XRD), Fourier transform infrared spectroscopy (FTIR), matrix-assisted laser desorption/ionization time-of-flight mass spectrometry(MALDI-TOF), ultraviolet-visible spectroscopy, dual polarisation interferometry and nuclear magnetic resonance (NMR). Characterization (dimension measurements) may be made as to native particles (i.e., preloading) or after loading of the cargo (herein cargo refers to e.g., one or more components of CRISPR-Cas system e.g., CRISPR enzyme or mRNA or guide RNA, or any combination thereof, and may include additional carriers and/or excipients) to provide particles of an optimal size for delivery for any *in vitro, ex vivo* and/or *in vivo* application of the present invention. In certain preferred embodiments, particle dimension (e.g., diameter) characterization is based on measurements using dynamic laser scattering (DLS). Mention is made of US Patent No. 8,709,843; US Patent No. 6,007,845; US Patent No. 5,855,913; US Patent No. 5,985,309; US. Patent No. 5,543,158; and the publication by James E. Dahlman and Carmen Barnes et al. Nature Nanotechnology (2014) published online 11 May 2014, doi:10.1038/nnano.2014.84, concerning particles, methods of making and using them and measurements thereof.

Particles delivery systems within the scope of the present invention may be provided in any form, including but not limited to solid, semi-solid, emulsion, or colloidal particles. As such any of the delivery systems described herein, including but not limited to, e.g., lipid-based systems, liposomes, micelles, microvesicles, exosomes, or gene gun may be provided as particle delivery systems within the scope of the present invention.

Nanoparticles

In terms of this invention, it is preferred to have one or more components of CRISPR complex, e.g., CRISPR enzyme or mRNA or guide RNA delivered using nanoparticles or lipid envelopes. Other delivery systems or vectors are may be used in conjunction with the nanoparticle aspects of the invention.

In general, a "nanoparticle" refers to any particle having a diameter of less than 1000 nm. In certain preferred embodiments, nanoparticles of the invention have a greatest dimension (e.g., diameter) of 500 nm or less. In other preferred embodiments, nanoparticles of the invention have a greatest dimension ranging between 25 nm and 200 nm. In other preferred embodiments, nanoparticles of the invention have a greatest dimension of 100 nm or less. In other preferred embodiments, nanoparticles of the invention have a greatest dimension ranging between 35 nm and 60 nm.

Nanoparticles encompassed in the present invention may be provided in different forms, e.g., as solid nanoparticles (e.g., metal such as silver, gold, iron, titanium), non-metal, lipid-based solids, polymers), suspensions of nanoparticles, or combinations thereof. Metal, dielectric, and semiconductor nanoparticles may be prepared, as well as hybrid structures (e.g., core-shell nanoparticles). Nanoparticles made of semiconducting material may also be labeled quantum dots if they are small enough (typically sub 10 nm) that quantization of electronic energy levels occurs. Such nanoscale particles are used in biomedical applications as drug carriers or imaging agents and may be adapted for similar purposes in the present invention.

Semi-solid and soft nanoparticles have been manufactured, and are within the scope of the present invention. A prototype nanoparticle of semi-solid nature is the liposome. Various types of liposome nanoparticles are currently used clinically as delivery systems for anticancer drugs and vaccines. Nanoparticles with one half hydrophilic and the other half hydrophobic are termed Janus particles and are particularly effective for stabilizing emulsions. They can self-assemble at water/oil interfaces and act as solid surfactants.

US Patent No. 8,709,843, provides a drug delivery system for targeted delivery of therapeutic agent-containing particles to tissues, cells, and intracellular compartments. The invention provides targeted particles comprising comprising polymer conjugated to a surfactant, hydrophilic polymer or lipid.

US Patent No. 6,007,845, provides particles which have a core of a multiblock copolymer formed by covalently linking a multifunctional compound with one or more hydrophobic polymers and one or more hydrophilic polymers, and conatin a biologically active material.

US Patent No. 5,855,913, provides a particulate composition having aerodynamically light particles having a tap density of less than 0.4 g/cm3 with a mean diameter of between 5 $\mu$m and 30 $\mu$m, incorporating a surfactant on the surface thereof for drug delivery to the pulmonary system.

US Patent No. 5,985,309, provides particles incorporating a surfactant and/or a hydrophilic or hydrophobic complex of a positively or negatively charged therapeutic or diagnostic agent and a charged molecule of opposite charge for delivery to the pulmonary system.

US. Patent No. 5,543,158, provides biodegradable injectable nanoparticles having a biodegradable solid core containing a biologically active material and poly(alkylene glycol) moieties on the surface.

WO2012135025 (also published as US20120251560), describes conjugated polyethyleneimine (PEI) polymers and conjugated aza-macrocycles (collectively referred to as "conjugated lipomer" or "lipomers"). In certain embodiments, it can envisioned that such conjugated lipomers can be used in the context of the CRISPR-Cas system to achieve in vitro, ex vivo and in vivo genomic perturbations to modify gene expression, including modulation of protein expression.

In one embodiment, the nanoparticle may be epoxide-modified lipid-polymer, advantageously 7C1 (see, e.g., James E. Dahlman and Carmen Barnes et al. Nature Nanotechnology (2014) published online 11 May 2014, doi:10.1038/nnano.2014.84). C71 was synthesized by reacting C15 epoxide-terminated lipids with PEI600 at a 14:1 molar ratio, and was formulated with C14PEG2000 to produce nanoparticles (diameter between 35 and 60 nm) that were stable in PBS solution for at least 40 days.

An epoxide-modified lipid-polymer may be utilized to deliver the CRISPR-Cas system to pulmonary, cardiovascular or renal cells, however, one of skill in the art may adapt the system to deliver to other target organs. Dosage ranging from about 0.05 to about 0.6 mg/kg are envisioned. Dosages over several days or weeks are also envisioned, with a total dosage of about 2 mg/kg.

An advantage of the present methods is that the CRISPR system avoids off-target binding and its resulting side effects. This is achieved using systems arranged to have a high degree of sequence specificity for the target DNA.

## Cas9

Cas9 optimization may be used to enhance function or to develop new functions, one can generate chimeric Cas9 proteins. Examples that the Applicants have generated are provided in Example 6. Chimeric Cas9 proteins can be made by combining fragments from different Cas9 homologs. For example, two example chimeric Cas9 proteins from the Cas9s described herein. For example, Applicants fused the N-term of St1Cas9 (fragment from this protein is in bold) with C-term of SpCas9. The benefit of making chimeric Cas9s include any or all of: reduced toxicity; improved expression in eukaryotic cells; enhanced specificity; reduced molecular weight of protein, for example, making the protein smaller by combining the smallest domains from different Cas9 homologs; and/or altering the PAM sequence requirement.

The Cas9 may be used as a generic DNA binding protein. For example, and as shown in Example 7, Applicants used Cas9 as a generic DNA binding protein by mutating the two catalytic domains (D10 and H840) responsible for cleaving both strands of the DNA target. In order to upregulate gene transcription at a target locus Applicants fused a transcriptional activation domain (VP64) to Cas9. Other transcriptional activation domains are known. As shown in Example 17, transcriptional activation is possible. As also shown in Example 17, gene repression (in this case of the beta-catenin gene) is possible using a Cas9 repressor (DNA-binding domain) that binds to the target gene sequence, thus repressing its activity.

Cas9 and one or more guide RNA can be delivered using adeno associated virus (AAV), lentivirus, adenovirus or other plasmid or viral vector types, in particular, using formulations and doses from, for example, US Patents Nos. 8,454,972 (formulations, doses for adenovirus), 8,404,658 (formulations, doses for AAV) and 5,846,946 (formulations, doses for DNA plasmids) and from clinical trials and publications regarding the clinical trials involving lentivirus, AAV and adenovirus. For examples, for AAV, the route of administration, formulation and dose can be as in US Patent No. 8,454,972 and as in clinical trials involving AAV. For Adenovirus, the route of administration, formulation and dose can be as in US Patent No. 8,404,658 and as in clinical trials involving adenovirus. For plasmid delivery, the route of administration, formulation and dose can be as in US Patent No 5,846,946 and as in clinical studies involving plasmids. Doses may be based on or extrapolated to an average 70 kg individual, and can be adjusted for patients, subjects, mammals of different weight and species. Frequency of administration is within the ambit of the medical or veterinary practitioner (e.g., physician, veterinarian), depending on usual factors including the age, sex, general health, other conditions of the patient or subject and the particular condition or symptoms being addressed.

The viral vectors can be injected into the tissue of interest. For cell-type specific genome modification, the expression of Cas9 can be driven by a cell-type specific promoter. For example, liver-specific expression might use the Albumin promoter and neuron-specific expression might use the Synapsin I promoter.

## Transgenic animals

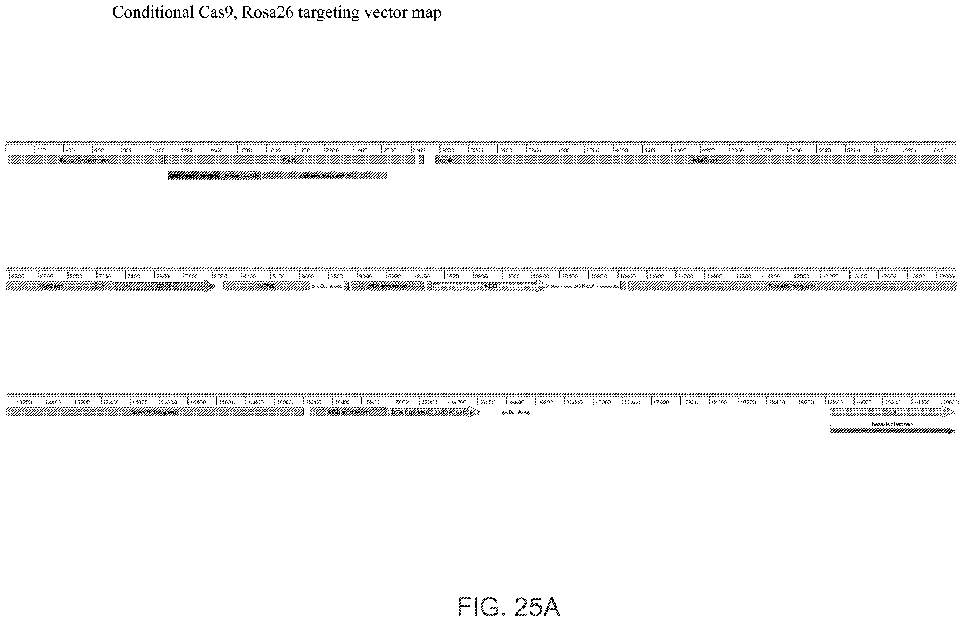

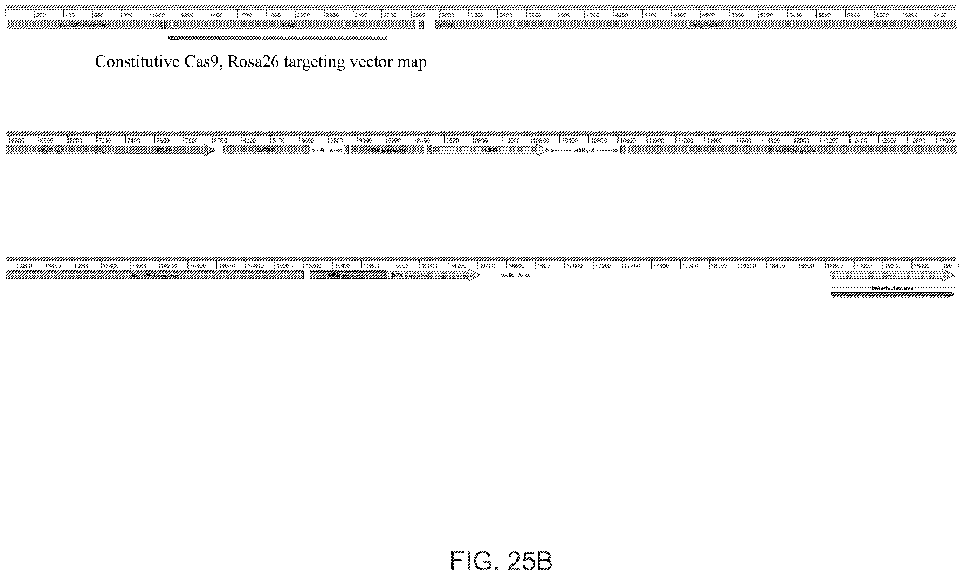

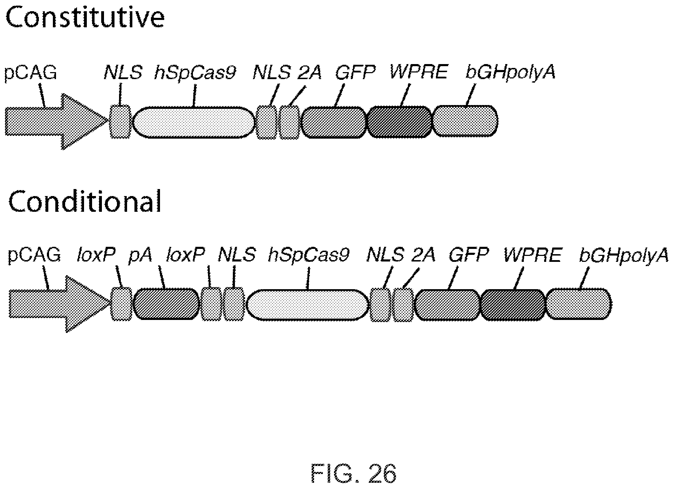

Transgenic animals (models) are also provided and the following applies equally to ex vivo model tissues and collections of tissues, such as organoids, liver on a chip and so forth. Preferred examples include animals comprising Cas9, in terms of polynucleotides encoding Cas9 or the protein itself. Mice, rats and rabbits are preferred. To generate transgenic mice with the constructs, as exemplified herein one may inject pure, linear DNA into the pronucleus of a zygote from a pseudo pregnant female, e.g. a CB56 female. Founders may then be identified, genotyped, and backcrossed to CB57 mice. The constructs may then be cloned and optionally verified, for instance by Sanger sequencing. Knock outs are envisaged where for instance one or more genes are knocked out in a model. However, are knockins are also envisaged (alone or in combination). An example knockin Cas9 mouse was generated and this is exemplified, but Cas9 knockins are preferred. To generate a Cas9 knock in mice one may target the same constitutive and conditional constructs to the Rosa26 locus, as described herein (Figs. 25A-B and 26). Methods of US Patent Publication Nos. 20120017290 and 20110265198 assigned to Sangamo BioSciences, Inc. directed to targeting the Rosa locus may be modified to utilize the CRISPR Cas system. In another embodiment, the methods of US PatentPublication No. 20130236946 assigned to Cellectis directed to targeting the Rosa locus may also be modified to utilize the CRISPR Cas system.

Utility of the conditional Cas9 mouse: Applicants have shown in 293 cells that the Cas9 conditional expression construct can be activated by co-expression with Cre. Applicants also show that the correctly targeted R1 mESCs can have active Cas9 when Cre is expressed. Because Cas9 is followed by the P2A peptide cleavage sequence and then EGFP Applicants identify successful expression by observing EGFP. Applicants have shown Cas9 activation in mESCs. This same concept is what makes the conditional Cas9 mouse so useful. Applicants may cross their conditional Cas9 mouse with a mouse that ubiquitously expresses Cre (ACTB-Cre line) and may arrive at a mouse that expresses Cas9 in every cell. It should only take the delivery of chimeric RNA to induce genome editing in embryonic or adult mice. Interestingly, if the conditional Cas9 mouse is crossed with a mouse expressing Cre under a tissue specific promoter, there should only be Cas9 in the tissues that also express Cre. This approach may be used to edit the genome in only precise tissues by delivering chimeric RNA to the same tissue.

As mentioned above, transgenic animals are also provided. In this regard, transgenic animals, especially mammals such as livestock (cows, sheep, goats and pigs), but also poultry and edible insects, are preferred.

## Adeno associated virus (AAV)

In terms of *in vivo* delivery, AAV is advantageous over other viral vectors for a couple of reasons:

Low toxicity (this may be due to the purification method not requiring ultra centrifugation of cell particles that can activate the immune response)

Low probability of causing insertional mutagenesis because it doesn't integrate into the host genome.

AAV has a packaging limit of 4.5 or 4.75 Kb. This means that Cas9 as well as a promoter and transcription terminator have to be all fit into the same viral vector. Constructs larger than 4.5 or 4.75 Kb will lead to significantly reduced virus production. SpCas9 is quite large, the gene itself is over 4.1 Kb, which makes it difficult for packing into AAV. Therefore embodiments of the invention include utilizing homologs of Cas9 that are shorter. For example:

| Species | Cas9 Size |
|---|---|
| Corynebacter diphtheriae | 3252 |
| Eubacterium ventriosum | 3321 |
| Streptococcus pasteurianus | 3390 |
| Lactobacillus farciminis | 3378 |
| Sphaerochaeta globus | 3537 |
| Azospirillum B510 | 3504 |
| Gluconacetobacter diazotrophicus | 3150 |
| Neisseria cinerea | 3246 |
| Roseburia intestinalis | 3420 |
| Parvibaculum lavamentivorans | 3111 |
| Staphylococcus aureus | 3159 |
| Nitratifractor salsuginis DSM 16511 | 3396 |
| Campylobacter lari CF89-12 | 3009 |
| Streptococcus thermophilus LMD-9 | 3396 |

These species are therefore, in general, preferred Cas9 species. Applicants have shown delivery and *in vivo* mouse brain Cas9 expression data.

Two ways to package Cas9 coding nucleic acid molecules, e.g., DNA, into viral vectors to mediate genome modification *in vivo* are preferred:

To achieve NHEJ-mediated gene knockout:

Single virus vector:
Vector containing two or more expression cassettes:

Promoter-Cas9 coding nucleic acid molecule -terminator
Promoter-gRNA1-terminator
Promoter-gRNA2-terminator
Promoter-gRNA(N)-terminator (up to size limit of vector)

Double virus vector:

Vector 1 containing one expression cassette for driving the expression of Cas9
Promoter-Cas9 coding nucleic acid molecule-terminator
Vector 2 containing one more expression cassettes for driving the expression of one or more guideRNAs
Promoter-gRNA1-terminator
Promoter-gRNA(N)-terminator (up to size limit of vector)

To mediate homology-directed repair. In addition to the single and double virus vector approaches described above, an additional vector is used to deliver a homology-direct repair template.

Promoter used to drive Cas9 coding nucleic acid molecule expression can include:

AAV ITR can serve as a promoter: this is advantageous for eliminating the need for an additional promoter element (which can take up space in the vector). The additional space freed up can be used to drive the expression of additional elements (gRNA, etc.). Also, ITR activity is relatively weaker, so can be used to reduce toxicity due to over expression of Cas9.

For ubiquitous expression, can use promoters: CMV, CAG, CBh, PGK, SV40, Ferritin heavy or light chains, etc.

For brain expression, can use promoters: Synapsinl for all neurons, CaMKIIalpha for excitatory neurons, GAD67 or GAD65 or VGAT for GABAergic neurons, etc.

For liver expression, can use Albumin promoter.

For lung expression, can use SP-B.

For endothelial cells, can use ICAM.

For hematopoietic cells can use IFNbeta or CD45.

For Osteoblasts can use OG-2.

Promoter used to drive guide RNA can include:

**Pol III promoters such as U6 or H1**

**Use of Pol II promoter and intronic cassettes to express gRNA**

As to AAV, the AAV can be AAV1, AAV2, AAV5 or any combination thereof. One can select the AAV of the AAV with regard to the cells to be targeted; e.g., one can select AAV serotypes 1, 2, 5 or a hybrid capsid AAV1, AAV2, AAV5 or any combination thereof for targeting brain or neuronal cells; and one can select AAV4 for targeting cardiac tissue. AAV8 is useful for delivery to the liver. The above promoters and vectors are preferred individually.

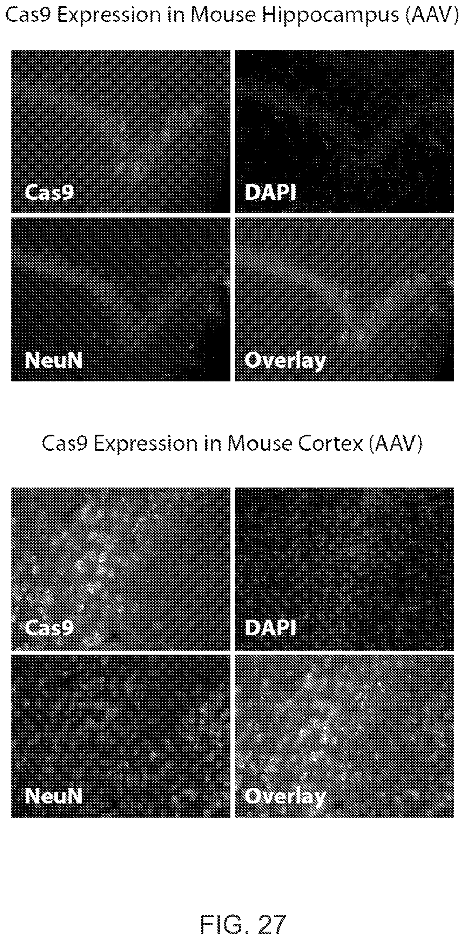

RNA delivery is also a useful method of *in vivo* delivery. Fig. 27 shows delivery and *in vivo* mouse brain Cas9 expression data. It is possible to deliver Cas9 and gRNA (and, for instance, HR repair template) into cells using liposomes or nanoparticles. Thus delivery of the CRISPR enzyme, such as a Cas9 and/or delivery of the RNAs of the invention may be in RNA form and via microvesicles, liposomes or nanoparticles. For example, Cas9 mRNA and gRNA can be packaged into liposomal particles for delivery *in vivo.* Liposomal transfection reagents such as lipofectamine from Life Technologies and other reagents on the market can effectively deliver RNA molecules into the liver.

Enhancing NHEJ or HR efficiency is also helpful for delivery. It is preferred that NHEJ efficiency is enhanced by co-expressing end-processing enzymes such as Trex2 (Dumitrache et al. Genetics. 2011 August; 188(4): 787-797). It is preferred that HR efficiency is increased by transiently inhibiting NHEJ machineries such as Ku70 and Ku86. HR efficiency can also be increased by co-expressing prokaryotic or eukaryotic homologous recombination enzymes such as RecBCD, RecA.

Various means of delivery are described herein, and further discussed in this section.

Viral delivery: The CRISPR enzyme, for instance a Cas9, and/or any of the present RNAs, for instance a guide RNA, can be delivered using adeno associated virus (AAV), lentivirus, adenovirus or other viral vector types, or combinations thereof. Cas9 and one or more guide RNAs can be packaged into one or more viral vectors. In some embodiments, the viral vector is delivered to the tissue of interest by, for example, an intramuscular injection, while other times the viral delivery is via intravenous, transdermal, intranasal, oral, mucosal, or other delivery methods. Such delivery may be either via a single dose, or multiple doses. One skilled in the art understands that the actual dosage to be delivered herein may vary greatly depending upon a variety of factors, such as the vector chose, the target cell, organism, or tissue, the general condition of the subject to be treated, the degree of transformation/modification sought, the administration route, the administration mode, the type of transformation/modification sought, etc.

Such a dosage may further contain, for example, a carrier (water, saline, ethanol, glycerol, lactose, sucrose, calcium phosphate, gelatin, dextran, agar, pectin, peanut oil, sesame oil, etc.), a diluent, a pharmaceutically-acceptable carrier (e.g., phosphate-buffered saline), a pharmaceutically-acceptable excipient, and/or other compounds known in the art. Such a dosage formulation is readily ascertainable by one skilled in the art. The dosage may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include microcrystalline cellulose, carboxymethylcellulose sodium, polysorbate 80, phenylethyl alcohol, chlorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991).

In an embodiment herein the delivery is via an adenovirus, which may be at a single booster dose containing at least $1 \times 10^5$ particles (also referred to as particle units, pu) of adenoviral vector. In an embodiment herein, the dose preferably is at least about $1 \times 10^6$ particles (for example, about $1 \times 10^6$-$1 \times 10^{12}$ particles), more preferably at least about $1 \times 10^7$ particles, more preferably at least about $1 \times 10^8$ particles (e.g., about $1 \times 10^8$-$1 \times 10^{11}$ particles or about $1 \times 10^8$-$1 \times 10^{12}$ particles), and most preferably at least about $1 \times 10°$ particles (e.g., about $1 \times 10^9$-$1 \times 10^{10}$ particles or about $1 \times 10^9$-$1 \times 10^{12}$ particles), or even at least about $1 \times 10^{10}$ particles (e.g., about $1 \times 10^{10}$-$1 \times 10^{12}$ particles) of the adenoviral vector. Alternatively, the dose comprises no more than about $1 \times 10^{14}$ particles, preferably no more than about $1 \times 10^{13}$ particles, even more preferably no more than about $1 \times 10^{12}$ particles, even more preferably no more than about $1 \times 10^{11}$ particles, and most preferably no more than about $1 \times 10^{10}$ particles (e.g., no more than about $1 \times 10^9$ articles). Thus, the dose may contain a single dose of adenoviral vector with, for example, about $1 \times 10^6$ particle units (pu), about $2 \times 10^6$ pu, about $4 \times 10^6$ pu, about $1 \times 10^7$ pu, about $2 \times 10^7$ pu, about $4 \times 10^7$ pu, about $1 \times 10^8$ pu, about $2 \times 10^8$ pu, about $4 \times 10^8$ pu, about $1 \times 10^9$ pu, about $2 \times 10^9$ pu, about $4 \times 10^9$ pu, about $1 \times 10^{10}$ pu, about $2 \times 10^{10}$ pu, about $4 \times 10^{10}$ pu, about $1 \times 10^{11}$ pu, about $2 \times 10^{11}$ pu, about $4 \times 10^{11}$ pu, about $1 \times 10^{12}$ pu, about $2 \times 10^{12}$ pu, or about $4 \times 10^{12}$ pu of adenoviral vector. See, for example, the adenoviral vectors in U.S. Patent No. 8,454,972 B2 to Nabel, et. al., granted on June 4, 2013; and the dosages at col 29, lines 36-58 thereof. In an embodiment herein, the adenovirus is delivered via multiple doses.

In an embodiment herein, the delivery is via an AAV. A therapeutically effective dosage for in vivo delivery of the AAV to a human is believed to be in the range of from about 20 to about 50 ml of saline solution containing from about $1 \times 10^{10}$ to about $1 \times 10^{10}$ functional AAV/ml solution. The dosage may be adjusted to balance the therapeutic benefit against any side effects. In an embodiment herein, the AAV dose is generally in the range of concentrations of from about $1 \times 10^5$ to $1 \times 10^{50}$ genomes AAV, from about $1 \times 10^8$ to $1 \times 10^{20}$ genomes AAV, from about $1 \times 10^{10}$ to about $1 \times 10^{16}$ genomes, or about $1 \times 10^{11}$ to about $1 \times 10^{16}$ genomes AAV. A human dosage may be about $1 \times 10^{13}$ genomes AAV. Such concentrations may be delivered in from about 0.001 ml to about 100 ml, about 0.05 to about 50 ml, or about 10 to about 25 ml of a carrier solution. Other effective dosages can be readily established by one of ordinary skill in the art through routine trials establishing dose response curves. See, for example, U.S. Patent No. 8,404,658 B2 to Hajjar, et al., granted on March 26, 2013, at col. 27, lines 45-60.

In an embodiment herein the delivery is via a plasmid. In such plasmid compositions, the dosage should be a sufficient amount of plasmid to elicit a response. For instance, suitable quantities of plasmid DNA in plasmid compositions can be from about 0.1 to about 2 mg, or from about 1 $\mu$g to about 10 $\mu$g.

The doses herein are based on an average 70 kg individual. The frequency of administration is within the ambit of the medical or veterinary practitioner (e.g., physician, veterinarian), or scientist skilled in the art. Mice used in experiments are about 20g. From that which is administered to a 20g mouse, one can extrapolate to a 70kg individual.

## Lentivirus

Lentiviruses are complex retroviruses that have the ability to infect and express their genes in both mitotic and post-mitotic cells. The most commonly known lentivirus is the human immunodeficiency virus (HIV), which uses the envelope glycoproteins of other viruses to target a broad range of cell types.

Lentiviruses may be prepared as follows. After cloning pCasES10 (which contains a lentiviral transfer plasmid backbone), HEK293FT at low passage (p=5) were seeded in a T-75 flask to 50% confluence the day before transfection in DMEM with 10% fetal bovine serum and without antibiotics. After 20 hours, media was changed to OptiMEM (serum-free) media and transfection was done 4 hours later. Cells were transfected with 10 $\mu$g of lentiviral transfer plasmid (pCasES10) and the following packaging plasmids: 5 $\mu$g of pMD2.G (VSV-g pseudotype), and 7.5ug of psPAX2 (gag/pol/rev/tat). Transfection was done in 4mL OptiMEM with a cationic lipid delivery agent (50uL Lipofectamine 2000 and 100ul Plus reagent). After 6 hours, the media was changed to antibiotic-free DMEM with 10% fetal bovine serum.

Lentivirus may be purified as follows. Viral supernatants were harvested after 48 hours. Supernatants were first cleared of debris and filtered through a 0.45um low protein binding (PVDF) filter. They were then spun in a ultracentrifuge for 2 hours at 24,000 rpm. Viral pellets were resuspended in 50ul of DMEM overnight at 4C. They were then aliquotted and immediately frozen at -80C.

In another embodiment, minimal non-primate lentiviral vectors based on the equine infectious anemia virus (EIAV) are also contemplated, especially for ocular gene therapy (see, e.g., Balagaan, J Gene Med 2006; 8: 275 - 285, Published online 21 November 2005 in Wiley InterScience (www.interscience.wiley.com). DOI: 10.1002/jgm.845). In another embodiment, RetinoStat®, an equine infectious anemia virus-based lentiviral gene therapy vector that expresses angiostatic proteins endostain and angiostatin that is delivered via a subretinal injection for the treatment of the web form of age-related macular degeneration is also contemplated (see, e.g., Binley et al., HUMAN GENE THERAPY 23:980-991 (September 2012)) may be modified for the CRISPR-Cas system system.

In another embodiment, self-inactivating lentiviral vectors with an siRNA targeting a common exon shared by HIV tat/rev, a nucleolar-localizing TAR decoy, and an anti-CCR5-specific hammerhead ribozyme (see, e.g., DiGiusto et al. (2010) Sci Transl Med 2:36ra43) may be used/and or adapted to the CRISPR-Cas system. A minimum of $2.5 \times 10^6$ CD34+ cells per kilogram patient weight may be collected and prestimulated for 16 to 20 hours in X-VIVO 15 medium (Lonza) containing 2 micro mol/ L-glutamine, stem cell factor (100 ng/ml), Flt-3 ligand (Flt-3L) (100 ng/ml), and thrombopoietin (10 ng/ml) (CellGenix) at a density of $2 \times 10^6$ cells/ml. Prestimulated cells may be transduced with lentiviral at a multiplicity of infection of 5 for 16 to 24 hours in 75-cm$^2$ tissue culture flasks coated with fibronectin (25 mg/cm$^2$) (RetroNectin,Takara Bio Inc.).

Lentiviral vectors have been disclosed as in the treatment for Parkinson's Disease, see, e.g., US Patent Publication No. 20120295960 and US Patent Nos. 7303910 and 7351585. Lentiviral vectors have also been disclosed for the treatment of ocular diseases, see e.g., US Patent Publication Nos. 20060281180, 20090007284, US20110117189; US20090017543; US20070054961, US20100317109. Lentiviral vectors have also been disclosed for delivery to the train, see, e.g., US Patent Publication Nos. US20110293571; US20110293571, US20040013648, US20070025970, US20090111106 and US Patent No. US7259015.

## RNA delivery

RNA delivery: The CRISPR enzyme, for instance a Cas9, and/or any of the present RNAs, for instance a guide RNA, can also be delivered in the form of RNA. Cas9 mRNA can be generated using *in vitro* transcription. For example, Cas9 mRNA can be synthesized using a PCR cassette containing the following elements: T7_promoter-kozak sequence (GCCACC)-Cas9-3' UTR from beta globin-polyA tail (a string of 120 or more adenines). The cassette can be used for transcription by T7 polymerase. Guide RNAs can also be transcribed using in vitro transcription from a cassette containing T7_promoter-GG-guide RNA sequence.

To enhance expression and reduce toxicity, the CRISPR enzyme and/or guide RNA can be modified using pseudo-U or 5-Methyl-C.

mRNA delivery methods are especially promising for liver delivery currently. In particular, for AAV8 is particularly preferred for delivery to the liver.

## Nanoparticles

CRISPR enzyme mRNA and guide RNA may be delivered simultaneously using nanoparticles or lipid envelopes.

For example, Su X, Fricke J, Kavanagh DG, Irvine DJ ("In vitro and in vivo mRNA delivery using lipid-enveloped pH-responsive polymer nanoparticles" Mol Pharm. 2011 Jun 6;8(3):774-87. doi: 10.1021/mp100390w. Epub 2011 Apr 1) describes biodegradable core-shell structured nanoparticles with a poly(β-amino ester) (PBAE) core enveloped by a phospholipid bilayer shell. These were developed for in vivo mRNA delivery. The pH-responsive PBAE component was chosen to promote endosome disruption, while the lipid surface layer was selected to minimize toxicity of the polycation core. Such are, therefore, preferred for delivering RNA.

In one embodiment, nanoparticles based on self assembling bioadhesive polymers are contemplated, which may be applied to oral delivery of peptides, intravenous delivery of peptides and nasal delivery of peptides, all to the brain. Other embodiments, such as oral absorption and ocular deliver of hydrophobic drugs are also contemplated. The molecular envelope technology involves an engineered polymer envelope which is protected and delivered to the site of the disease (see, e.g., Mazza, M. et al. ACSNano, 2013. 7(2): 1016-1026; Siew, A., et al. Mol Pharm, 2012. 9(1):14-28; Lalatsa, A., et al. J Contr Rel, 2012. 161(2):523-36; Lalatsa, A., et al., Mol Pharm, 2012. 9(6):1665-80; Lalatsa, A., et al. Mol Pharm, 2012. 9(6):1764-74; Garrett, N.L., et al. J Biophotonics, 2012. 5(5-6):458-68; Garrett, N.L., et al. J Raman Spect, 2012. 43(5):681-688; Ahmad, S., et al. J Royal Soc Interface 2010. 7:S423-33; Uchegbu, I.F. Expert Opin Drug Deliv, 2006. 3(5):629-40; Qu, X.,et al. Biomacromolecules, 2006. 7(12):3452-9 and Uchegbu, I.F., et al. Int J Pharm, 2001. 224:185-199). Doses of about 5 mg/kg are contemplated, with single or multiple doses, depending on the target tissue.

In one embodiment, nanoparticles that can deliver RNA to a cancer cell to stop tumor growth developed by Dan Anderson's lab at MIT may be used/and or adapted to the CRISPR Cas system. In particular, the Anderson lab developed fully automated, combinatorial systems for the synthesis, purification, characterization, and formulation of new biomaterials and nanoformulations. See, e.g., Alabi et al., Proc Natl Acad Sci U S A. 2013 Aug 6;110(32): 12881-6; Zhang et al., Adv Mater. 2013 Sep 6;25(33):4641-5; Jiang et al., Nano Lett. 2013 Mar 13;13(3):1059-64; Karagiannis et al., ACS Nano. 2012 Oct 23;6(10):8484-7; Whitehead et al., ACS Nano. 2012 Aug 28;6(8):6922-9 and Lee et al., Nat Nanotechnol. 2012 Jun 3;7(6):389-93.

US patent Publication No. 20110293703 relates to lipidoid compounds are also particularly useful in the administration of polynucleotides, which may be applied to deliver the CRISPR Cas system. In one aspect, the aminoalcohol lipidoid compounds are combined with an agent to be delivered to a cell or a subject to form microparticles, nanoparticles, liposomes, or micelles. The agent to be delivered by the particles, liposomes, or micelles may be in the form of a gas, liquid, or solid, and the agent may be a polynucleotide, protein, peptide, or small molecule. The minoalcohol lipidoid compounds may be combined with other aminoalcohol lipidoid compounds, polymers (synthetic or natural), surfactants, cholesterol, carbohydrates, proteins, lipids, etc. to form the particles. These particles may then optionally be combined with a pharmaceutical excipient to form a pharmaceutical composition.

US Patent Publication No. 20110293703 also provides methods of preparing the aminoalcohol lipidoid compounds. One or more equivalents of an amine are allowed to react with one or more equivalents of an epoxide-terminated compound under suitable conditions to form an aminoalcohol lipidoid compound. In certain embodiments, all the amino groups of the amine are fully reacted with the epoxide-terminated compound to form tertiary amines. In other embodiments, all the amino groups of the amine are not fully reacted with the epoxide-terminated compound to form tertiary amines thereby resulting in primary or secondary amines in the aminoalcohol lipidoid compound. These primary or secondary amines are left as is or may be reacted with another electrophile such as a different epoxide-terminated compound. As will be appreciated by one skilled in the art, reacting an amine with less than excess of epoxide-terminated compound will result in a plurality of different aminoalcohol lipidoid compounds with various numbers of tails. Certain amines may be fully functionalized with two epoxide-derived compound tails while other molecules will not be completely functionalized with epoxide-derived compound tails. For example, a diamine or polyamine may include one, two, three, or four epoxide-derived compound tails off the various amino moieties of the molecule resulting in primary, secondary, and tertiary amines. In certain embodiments, all the amino groups are not fully functionalized. In certain embodiments, two of the same types of epoxide-terminated compounds are used. In other embodiments, two or more different epoxide-terminated compounds are used. The synthesis of the aminoalcohol lipidoid compounds is performed with or without solvent, and the synthesis may be performed at higher temperatures ranging from 30.-100 C., preferably at approximately 50.-90 C. The prepared aminoalcohol lipidoid compounds may be optionally purified. For example, the mixture of aminoalcohol lipidoid compounds may be purified to yield an aminoalcohol lipidoid compound with a particular number of epoxide-derived compound tails. Or the mixture may be purified to yield a particular stereo- or regioisomer. The aminoalcohol lipidoid compounds may also be alkylated using an alkyl halide (e.g., methyl iodide) or other alkylating agent, and/or they may be acylated.

US Patent Publication No. 20110293703 also provides libraries of aminoalcohol lipidoid compounds prepared by the inventive methods. These aminoalcohol lipidoid compounds may be prepared and/or screened using high-throughput techniques involving liquid handlers, robots, microtiter plates, computers, etc. In certain embodiments, the aminoalcohol lipidoid compounds are screened for their ability to transfect polynucleotides or other agents (e.g., proteins, peptides, small molecules) into the cell.

US Patent Publication No. 20130302401 relates to a class of poly(beta-amino alcohols) (PBAAs) has been prepared using combinatorial polymerization. The inventive PBAAs may be used in biotechnology and biomedical applications as coatings (such as coatings of films or multilayer films for medical devices or implants), additives, materials, excipients, non-biofouling agents, micropatterning agents, and cellular encapsulation agents. When used as surface coatings, these PBAAs elicited different levels of inflammation, both in vitro and in vivo, depending on their chemical structures. The large chemical diversity of this class of materials allowed us to identify polymer coatings that inhibit macrophage activation in vitro. Furthermore, these coatings reduce the recruitment of inflammatory cells, and reduce fibrosis, following the subcutaneous implantation of carboxylated polystyrene microparticles. These polymers may be used to form polyelectrolyte complex capsules for cell encapsulation. The invention may also have many other biological applications such as antimicrobial coatings, DNA or siRNA delivery, and stem cell tissue engineering. The teachings of US Patent Publication No. 20130302401 may be applied to the CRISPR Cas system.

In another embodiment, lipid nanoparticles (LNPs) are contemplated. In particular, an antitransthyretin small interfering RNA encapsulated in lipid nanoparticles (see, e.g., Coelho et al., N Engl J Med 2013;369:819-29) may be applied to the CRISPR Cas system

Doses of about 0.01 to about 1 mg per kg of body weight administered intravenously are contemplated. Medications to reduce the risk of infusion-related reactions are contemplated, such as dexamethasone, acetampinophen, diphenhydramine or cetirizine, and ranitidine are contemplated. Multiple doses of about 0.3 mg per kilogram every 4 weeks for five doses are also contemplated.

LNPs have been shown to be highly effective in delivering siRNAs to the liver (see, e.g., Tabernero et al., Cancer Discovery, April 2013, Vol. 3, No. 4, pages 363-470) and are therefore contemplated for delivering CRISPR Cas to the liver. A dosage of about four doses of 6 mg/kg of the LNP (or RNA of the CRISPR-Cas system) every two weeks may be contemplated. Tabernero et al. demonstrated that tumor regression was observed after the first 2 cycles of LNPs dosed at 0.7 mg/kg, and by the end of 6 cycles the patient had achieved a partial response with complete regression of the lymph node metastasis and substantial shrinkage of the liver tumors. A complete response was obtained after 40 doses in this patient, who has remained in remission and completed treatment after receiving doses over 26 months. Two patients with RCC and extrahepatic sites of disease including kidney, lung, and lymph nodes that were progressing following prior therapy with VEGF pathway inhibitors had stable disease at all sites for approximately 8 to 12 months, and a patient with PNET and liver metastases continued on the extension study for 18 months (36 doses) with stable disease.

However, the charge of the LNP must be taken into consideration. As cationic lipids combined with negatively charged lipids to induce nonbilayer structures that facilitate intracellular delivery. Because charged LNPs are rapidly cleared from circulation following intravenous injection, ionizable cationic lipids with pKa values below 7 were developed (see, e.g., Rosin et al, Molecular Therapy, vol. 19, no. 12, pages 1286-2200, Dec. 2011). Negatively charged polymers such as siRNA oligonucleotides may be loaded into LNPs at low pH values

(e.g., pH 4) where the ionizable lipids display a positive charge. However, at physiological pH values, the LNPs exhibit a low surface charge compatible with longer circulation times. Four species of ionizable cationic lipids have been focused upon, namely 1,2-dilineoyl-3-dimethylammonium-propane (DLinDAP), 1,2-dilinoleyloxy-3-N,N-dimethylaminopropane (DLinDMA), 1,2-dilinoleyloxy-keto-N,N-dimethyl-3-aminopropane (DLinKDMA), and 1,2-dilinoleyl-4-(2-dimethylaminoethyl)-[1,3]-dioxolane (DLinKC2-DMA). It has been shown that LNP siRNA systems containing these lipids exhibit remarkably different gene silencing properties in hepatocytes in vivo, with potencies varying according to the series DLinKC2-DMA>DLinKDMA>DLinDMA>>DLinDAP employing a Factor VII gene silencing model (see, e.g., Rosin et al, Molecular Therapy, vol. 19, no. 12, pages 1286-2200, Dec. 2011). A dosage of 1 $\mu$g/ml levels may be contemplated, especially for a formulation containing DLinKC2-DMA.

Preparation of LNPs and CRISPR Cas encapsulation may be used/and or adapted from Rosin et al, Molecular Therapy, vol. 19, no. 12, pages 1286-2200, Dec. 2011). The cationic lipids 1,2-dilineoyl-3-dimethylammonium-propane (DLinDAP), 1,2-dilinoleyloxy-3-N,N-dimethylaminopropane (DLinDMA), 1,2-dilinoleyloxyketo-N,N-dimethyl-3-aminopropane (DLinK-DMA), 1,2-dilinoleyl-4-(2-dimethylaminoethyl)-[1,3]-dioxolane (DLinKC2-DMA), (3-o-[2''-(methoxypolyethyleneglycol 2000) succinoyl]-1,2-dimyristoyl-sn-glycol (PEG-S-DMG), and R-3-[($\omega$-methoxy-poly(ethylene glycol)2000) carbamoyl]-1,2-dimyristyloxlpropyl-3-amine (PEG-C-DOMG) may be provided by Tekmira Pharmaceuticals (Vancouver, Canada) or synthesized. Cholesterol may be purchased from Sigma (St Louis, MO). The specific CRISPR Cas RNA may be encapsulated in LNPs containing DLinDAP, DLinDMA, DLinK-DMA, and DLinKC2-DMA (cationic lipid:DSPC:CHOL: PEGS-DMG or PEG-C-DOMG at 40:10:40:10 molar ratios). When required, 0.2% SP-DiOC18 (Invitrogen, Burlington, Canada) may be incorporated to assess cellular uptake, intracellular delivery, and biodistribution. Encapsulation may be performed by dissolving lipid mixtures comprised of cationic lipid:DSPC:cholesterol:PEG-c-DOMG (40:10:40:10 molar ratio) in ethanol to a final lipid concentration of 10 mmol/1. This ethanol solution of lipid may be added drop-wise to 50 mmol/l citrate, pH 4.0 to form multilamellar vesicles to produce a final concentration of 30% ethanol vol/vol. Large unilamellar vesicles may be formed following extrusion of multilamellar vesicles through two stacked 80 nm Nuclepore polycarbonate filters using the Extruder (Northern Lipids, Vancouver, Canada). Encapsulation may be achieved by adding RNA dissolved at 2 mg/ml in 50

mmol/l citrate, pH 4.0 containing 30% ethanol vol/vol drop-wise to extruded preformed large unilamellar vesicles and incubation at 31 °C for 30 minutes with constant mixing to a final RNA/lipid weight ratio of 0.06/1 wt/wt. Removal of ethanol and neutralization of formulation buffer were performed by dialysis against phosphate-buffered saline (PBS), pH 7.4 for 16 hours using Spectra/Por 2 regenerated cellulose dialysis membranes. Nanoparticle size distribution may be determined by dynamic light scattering using a NICOMP 370 particle sizer, the vesicle/intensity modes, and Gaussian fitting (Nicomp Particle Sizing, Santa Barbara, CA). The particle size for all three LNP systems may be ~70 nm in diameter. siRNA encapsulation efficiency may be determined by removal of free siRNA using VivaPureD MiniH columns (Sartorius Stedim Biotech) from samples collected before and after dialysis. The encapsulated RNA may be extracted from the eluted nanoparticles and quantified at 260 nm. siRNA to lipid ratio was determined by measurement of cholesterol content in vesicles using the Cholesterol E enzymatic assay from Wako Chemicals USA (Richmond, VA). PEGylated liposomes (or LNPs) can also be used for delivery.

Preparation of large LNPs may be used/and or adapted from Rosin et al, Molecular Therapy, vol. 19, no. 12, pages 1286-2200, Dec. 2011. A lipid premix solution (20.4 mg/ml total lipid concentration) may be prepared in ethanol containing DLinKC2-DMA, DSPC, and cholesterol at 50:10:38.5 molar ratios. Sodium acetate may be added to the lipid premix at a molar ratio of 0.75:1 (sodium acetate:DLinKC2-DMA). The lipids may be subsequently hydrated by combining the mixture with 1.85 volumes of citrate buffer (10 mmol/l, pH 3.0) with vigorous stirring, resulting in spontaneous liposome formation in aqueous buffer containing 35% ethanol. The liposome solution may be incubated at 37 °C to allow for time-dependent increase in particle size. Aliquots may be removed at various times during incubation to investigate changes in liposome size by dynamic light scattering (Zetasizer Nano ZS, Malvern Instruments, Worcestershire, UK). Once the desired particle size is achieved, an aqueous PEG lipid solution (stock = 10 mg/ml PEG-DMG in 35% (vol/vol) ethanol) may be added to the liposome mixture to yield a final PEG molar concentration of 3.5% of total lipid. Upon addition of PEG-lipids, the liposomes should their size, effectively quenching further growth. RNA may then be added to the empty liposomes at an siRNA to total lipid ratio of approximately 1:10 (wt:wt), followed by incubation for 30 minutes at 37 °C to form loaded LNPs. The mixture may be subsequently dialyzed overnight in PBS and filtered with a 0.45-$\mu$m syringe filter.

Spherical Nucleic Acid (SNA™) constructs and other nanoparticles (particularly gold nanoparticles) are also contemplate as a means to delivery CRISPR/Cas system to intended targets. Significant data show that AuraSense Therapeutics' Spherical Nucleic Acid (SNA™) constructs, based upon nucleic acid-functionalized gold nanoparticles, are superior to alternative platforms based on multiple key success factors, such as:

High in vivo stability. Due to their dense loading, a majority of cargo (DNA or siRNA) remains bound to the constructs inside cells, conferring nucleic acid stability and resistance to enzymatic degradation.

Deliverability. For all cell types studied (e.g., neurons, tumor cell lines, etc.) the constructs demonstrate a transfection efficiency of 99% with no need for carriers or transfection agents.

Therapeutic targeting. The unique target binding affinity and specificity of the constructs allow exquisite specificity for matched target sequences (i.e., limited off-target effects).

Superior efficacy. The constructs significantly outperform leading conventional transfection reagents (Lipofectamine 2000 and Cytofectin).

Low toxicity. The constructs can enter a variety of cultured cells, primary cells, and tissues with no apparent toxicity.

No significant immune response. The constructs elicit minimal changes in global gene expression as measured by whole-genome microarray studies and cytokine-specific protein assays.

Chemical tailorability. Any number of single or combinatorial agents (e.g., proteins, peptides, small molecules) can be used to tailor the surface of the constructs.

This platform for nucleic acid-based therapeutics may be applicable to numerous disease states, including inflammation and infectious disease, cancer, skin disorders and cardiovascular disease.

Citable literature includes: Cutler et al.,. J. Am. Chem. Soc. 2011 133:9254-9257, Hao et al., Small. 2011 7:3158-3162, Zhang et al., ACS Nano. 2011 5:6962-6970, Cutler et al., J. Am. Chem. Soc. 2012 134:1376-1391, Young et al.,. Nano Lett. 2012 12:3867-71, Zheng et al., Proc. Natl. Acad. Sci. USA. 2012 109:11975-80, Mirkin, Nanomedicine 2012 7:635-638 Zhang et al., J. Am. Chem. Soc. 2012 134:16488-1691, Weintraub, Nature 2013 495:S14-S16, Choi et al., Proc. Natl. Acad. Sci. USA. 2013 110(19):7625-7630, Jensen et al., Sci. Transl. Med. 5, 209ra152 (2013) and Mirkin, et al., Small, doi.org/10.1002/smll.201302143.

Self-assembling nanoparticles with siRNA may be constructed with polyethyleneimine (PEI) that is PEGylated with an Arg-Gly-Asp (RGD) peptide ligand attached at the distal end of the polyethylene glycol (PEG), for example, as a means to target tumor neovasculature expressing integrins and used to deliver siRNA inhibiting vascular endothelial growth factor receptor-2 (VEGF R2) expression and thereby tumor angiogenesis (see, e.g., Schiffelers et al., Nucleic Acids Research, 2004, Vol. 32, No. 19). Nanoplexes may be prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid resulted in the formation of polyplexes with average particle size distribution of about 100 nm, hence referred to here as nanoplexes. A dosage of about 100 to 200 mg of CRISPR Cas is envisioned for delivery in the self-assembling nanoparticles of Schiffelers et al.

The nanoplexes of Bartlett et al. (PNAS, September 25, 2007,vol. 104, no. 39) may also be applied to the present invention. The nanoplexes of Bartlett et al. are prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid resulted in the formation of polyplexes with average particle size distribution of about 100 nm, hence referred to here as nanoplexes. The DOTA-siRNA of Bartlett et al. was synthesized as follows: 1,4,7,10-tetraazacyclododecane-1,4,7,10-tetraacetic acid mono(N-hydroxysuccinimide ester) (DOTA-NHSester) was ordered from Macrocyclics (Dallas, TX). The amine modified RNA sense strand with a 100-fold molar excess of DOTA-NHS-ester in carbonate buffer (pH 9) was added to a microcentrifuge tube. The contents were reacted by stirring for 4 h at room temperature. The DOTA-RNAsense conjugate was ethanol-precipitated, resuspended in water, and annealed to the unmodified antisense strand to yield DOTA-siRNA. All liquids were pretreated with Chelex-100 (Bio-Rad, Hercules, CA) to remove trace metal contaminants. Tf-targeted and nontargeted siRNA nanoparticles may be formed by using cyclodextrin-containing polycations. Typically, nanoparticles were formed in water at a charge ratio of 3 (+/-) and an siRNA concentration of 0.5 g/liter. One percent of the adamantane-PEG molecules on the surface of the targeted nanoparticles were modified with Tf (adamantane-PEG-Tf). The nanoparticles were suspended in a 5% (wt/vol) glucose carrier solution for injection.

Davis et al. (Nature, Vol 464, 15 April 2010) conducts a siRNA clinical trial that uses a targeted nanoparticle-delivery system (clinical trial registration number NCT00689065). Patients with solid cancers refractory to standard-of-care therapies are administered doses of targeted nanoparticles on days 1, 3, 8 and 10 of a 21-day cycle by a 30-min intravenous infusion. The nanoparticles consist of a synthetic delivery system containing: (1) a linear, cyclodextrin-based polymer (CDP), (2) a human transferrin protein (TF) targeting ligand displayed on the exterior of the nanoparticle to engage TF receptors (TFR) on the surface of the cancer cells, (3) a hydrophilic polymer (polyethylene glycol (PEG) used to promote nanoparticle stability in biological fluids), and (4) siRNA designed to reduce the expression of the RRM2 (sequence used in the clinic was previously denoted siR2B+5). The TFR has long been known to be upregulated in malignant cells, and RRM2 is an established anti-cancer target. These nanoparticles (clinical version denoted as CALAA-01) have been shown to be well tolerated in multi-dosing studies in non-human primates. Although a single patient with chronic myeloid leukaemia has been administered siRNAby liposomal delivery, Davis et al.'s clinical trial is the initial human trial to systemically deliver siRNA with a targeted delivery system and to treat patients with solid cancer. To ascertain whether the targeted delivery system can provide effective delivery of functional siRNA to human tumours, Davis et al. investigated biopsies from three patients from three different dosing cohorts; patients A, B and C, all of whom had metastatic melanoma and received CALAA-01 doses of 18, 24 and 30 mg m$^{-2}$ siRNA, respectively. Similar doses may also be contemplated for the CRISPR Cas system. The delivery
may be achieved with nanoparticles containing a linear, cyclodextrin-based polymer
(CDP), a human transferrin protein (TF) targeting ligand displayed on the exterior of the nanoparticle to engage TF receptors (TFR) on the surface of the cancer cells and/or a hydrophilic polymer (for example, polyethylene glycol (PEG) used to promote nanoparticle stability in biological fluids).

**Exosomes**

Exosomes are endogenous nano-vesicles that transport RNAs and proteins which can deliver short interfering (si)RNA to the brain in mice. To reduce immunogenicity, Alvarez-Erviti et al. (2011, Nat Biotechnol 29: 341) used self-derived dendritic cells for exosome production. Targeting was achieved by engineering the dendritic cells to express Lamp2b, an exosomal membrane protein, fused to the neuron-specific RVG peptide. Purified exosomes were loaded with exogenous siRNA by electroporation. Intravenously injected RVG-targeted exosomes delivered GAPDH siRNA specifically to neurons, microglia, oligodendrocytes in the brain, resulting in a specific gene knockdown. Pre-exposure to RVG exosomes did not attenuate knockdown, and nonspecific uptake in other tissues was not observed. The therapeutic potential of exosome-mediated siRNA delivery was demonstrated by the strong mRNA (60%) and protein (62%) knockdown of BACE1, a therapeutic target in Alzheimer's disease.

To obtain a pool of immunologically inert exosomes, Alvarez-Erviti et al. harvested bone marrow from inbred C57BL/6 mice with a homogenous major histocompatibility complex (MHC) haplotype. As immature dendritic cells produce large quantities of exosomes devoid of T-cell activators such as MHC-II and CD86, Alvarez-Erviti et al. selected for dendritic cells with granulocyte/macrophage-colony stimulating factor (GM-CSF) for 7 d. Exosomes were purified from the culture supernatant the following day using well-established ultracentrifugation protocols. The exosomes produced were physically homogenous, with a size distribution peaking at 80 nm in diameter as determined by nanoparticle tracking analysis (NTA) and electron microscopy. Alvarez-Erviti et al. obtained 6-12 $\mu$g of exosomes (measured based on protein concentration) per $10^6$ cells.

Next, Alvarez-Erviti et al. investigated the possibility of loading modified exosomes with exogenous cargoes using electroporation protocols adapted for nanoscale applications. As electroporation for membrane particles at the nanometer scale is not well-characterized, nonspecific Cy5-labeled siRNA was used for the empirical optimization of the electroporation protocol. The amount of encapsulated siRNA was assayed after ultracentrifugation and lysis of exosomes. Electroporation at 400 V and 125 $\mu$F resulted in the greatest retention of siRNA and was used for all subsequent experiments.

Alvarez-Erviti et al. administered 150 $\mu$g of each BACE1 siRNA encapsulated in 150 $\mu$g of RVG exosomes to normal C57BL/6 mice and compared the knockdown efficiency to four controls: untreated mice, mice injected with RVG exosomes only, mice injected with BACE1 siRNA complexed to an in vivo cationic liposome reagent and mice injected with BACE1 siRNA complexed to RVG-9R, the RVG pep tide conjugated to 9 D-arginines that electrostatically binds to the siRNA. Cortical tissue samples were analyzed 3 d after administration and a significant protein knockdown (45%, P < 0.05, versus 62%, P < 0.01) in both siRNA-RVG-9R-treated and siRNARVG exosome-treated mice was observed, resulting from a significant decrease in BACE1 mRNA levels (66% [+ or -] 15%, P < 0.001 and 61% [+ or -] 13% respectively, P < 0.01). Moreover, Applicants demonstrated a significant decrease (55%, P < 0.05) in the total [beta]-amyloid 1-42 levels, a main component of the amyloid plaques in Alzheimer's pathology, in the RVG-exosome-treated animals. The decrease observed was greater than the $\beta$-amyloid 1-40 decrease demonstrated in normal mice after intraventricular injection of BACE1 inhibitors. Alvarez-Erviti et al. carried out 5'-rapid amplification of cDNA ends (RACE) on BACE1 cleavage product, which provided evidence of RNAi-mediated knockdown by the siRNA.

Finally, Alvarez-Erviti et al. investigated whether siRNA-RVG exosomes induced immune responses in vivo by assessing IL-6, IP-10, TNF$\alpha$ and IFN-$\alpha$ serum concentrations. Following siRNA-RVG exosome treatment, nonsignificant changes in all cytokines were registered similar to siRNA-transfection reagent treatment in contrast to siRNA-RVG-9R, which potently stimulated IL-6 secretion, confirming the immunologically inert profile of the exosome treatment. Given that exosomes encapsulate only 20% of siRNA, delivery with RVG-exosome appears to be more efficient than RVG-9R delivery as comparable mRNA knockdown and greater protein knockdown was achieved with fivefold less siRNA without the corresponding level of immune stimulation. This experiment demonstrated the therapeutic potential of RVG-exosome technology, which is potentially suited for long-term silencing of genes related to neurodegenerative diseases. The exosome delivery system of Alvarez-Erviti et al. may be applied to deliver the CRISPR-Cas system to therapeutic targets, especially neurodegenerative diseases. A dosage of about 100 to 1000 mg of CRISPR Cas encapsulated in about 100 to 1000 mg of RVG exosomes may be contemplated for the present invention.

El-Andaloussi et al. (Nature Protocols 7,2112-2126(2012)) discloses how exosomes derived from cultured cells can be harnessed for delivery of siRNA in vitro and in vivo. This protocol first describes the generation of targeted exosomes through transfection of an expression vector, comprising an exosomal protein fused with a peptide ligand. Next, El-Andaloussi et al. explain how to purify and characterize exosomes from transfected cell supernatant. Next, El-Andaloussi et al. detail crucial steps for loading siRNA into exosomes. Finally, El-Andaloussi et al. outline how to use exosomes to efficiently deliver siRNA in vitro and in vivo in mouse brain. Examples of anticipated results in which exosome-mediated siRNA delivery is evaluated by functional assays and imaging are also provided. The entire protocol takes ~3 weeks. Delivery or administration according to the invention may be performed using exosomes produced from self-derived dendritic cells.

In another embodiment, the plasma exosomes of Wahlgren et al. (Nucleic Acids Research, 2012, Vol. 40, No. 17 e130) are contemplated. Exosomes are nano-sized vesicles (30-90nm in size) produced by many cell types, including dendritic cells (DC), B cells, T cells, mast cells, epithelial cells and tumor cells. These vesicles are formed by inward budding of late endosomes and are then released to the extracellular environment upon fusion with the plasma membrane. Because exosomes naturally carry RNA between cells, this property might be useful in gene therapy.

Exosomes from plasma are prepared by centrifugation of buffy coat at 900g for 20 min to isolate the plasma followed by harvesting cell supernatants, centrifuging at 300g for 10 min to eliminate cells and at 16 500g for 30 min followed by filtration through a 0.22 mm filter. Exosomes are pelleted by ultracentrifugation at 120 000g for70 min. Chemical transfection of siRNA into exosomes is carried out according to the manufacturer's instructions in RNAi Human/Mouse Starter Kit (Quiagen, Hilden, Germany). siRNA is added to 100 ml PBS at a final concentration of 2 mmol/ml. After adding HiPerFect transfection reagent, the mixture is incubated for 10 min at RT. In order to remove the excess of micelles, the exosomes are re-isolated using aldehyde/sulfate latex beads. The chemical transfection of CRISPR Cas into exosomes may be conducted similarly to siRNA. The exosomes may be co-cultured with monocytes and lymphocytes isolated from the peripheral blood of healthy donors. Therefore, it may be contemplated that exosomes containing CRISPR Cas may be introduced to monocytes and lymphocytes of and autologously reintroduced into a human. Accordingly, delivery or administration according to the invention may beperformed using plasma exosomes.

**Liposomes**

Delivery or administration according to the invention can be performed with liposomes. Liposomes are spherical vesicle structures composed of a uni- or multilamellar lipid bilayer surrounding internal aqueous compartments and a relatively impermeable outer lipophilic phospholipid bilayer. Liposomes have gained considerable attention as drug delivery carriers because they are biocompatible, nontoxic, can deliver both hydrophilic and lipophilic drug molecules, protect their cargo from degradation by plasma enzymes, and transport their load across biological membranes and the blood brain barrier (BBB) (see, e.g., Spuch and Navarro, Journal of Drug Delivery, vol. 2011, Article ID 469679, 12 pages, 2011. doi:10.1155/2011/469679 for review).

Liposomes can be made from several different types of lipids; however, phospholipids are most commonly used to generate liposomes as drug carriers. Although liposome formation is spontaneous when a lipid film is mixed with an aqueous solution, it can also be expedited by applying force in the form of shaking by using a homogenizer, sonicator, or an extrusion apparatus (see, e.g., Spuch and Navarro, Journal of Drug Delivery, vol. 2011, Article ID 469679, 12 pages, 2011. doi:10.1155/2011/469679 for review).

Several other additives may be added to liposomes in order to modify their structure and properties. For instance, either cholesterol or sphingomyelin may be added to the liposomal mixture in order to help stabilize the liposomal structure and to prevent the leakage of the liposomal inner cargo. Further, liposomes are prepared from hydrogenated egg phosphatidylcholine or egg phosphatidylcholine, cholesterol, and dicetyl phosphate, and their mean vesicle sizes were adjusted to about 50 and 100 nm. (see, e.g., Spuch and Navarro, Journal of Drug Delivery, vol. 2011, Article ID 469679, 12 pages, 2011. doi:10.1155/2011/469679 for review).

Conventional liposome formulation is mainly comprised of natural phospholipids and lipids such as 1,2-distearoryl-sn-glycero-3-phosphatidyl choline (DSPC), sphingomyelin, egg phosphatidylcholines and monosialoganglioside. Since this formulation is made up of phospholipids only, liposomal formulations have encountered many challenges, one of the ones being the instability in plasma. Several attempts to overcome these challenges have been made, specifically in the manipulation of the lipid membrane. One of these attempts focused on the manipulation of cholesterol. Addition of cholesterol to conventional formulations reduces rapid release of the encapsulated bioactive compound into the plasma or 1,2-dioleoyl-sn-glycero-3-phosphoethanolamine (DOPE) increases the stability (see, e.g., Spuch and Navarro, Journal of Drug Delivery, vol. 2011, Article ID 469679, 12 pages, 2011. doi:10.1155/2011/469679 for review).

In a particularly advantageous embodiment, Trojan Horse liposomes (also known as Molecular Trojan Horses) are desirable and protocols may be found at http://cshprotocols.cshlp.org/content/2010/4/pdb.prot5407.long. These particles allow delivery of a transgene to the entire brain after an intravascular injection. Without being bound by limitation, it is believed that neutral lipid particles with specific antibodies conjugated to surface allow crossing of the blood brain barrier via endocytosis. Applicant postulates utilizing Trojan Horse Liposomes to deliver the CRISPR family of nucleases to the brain via an intravascular injection, which would allow whole brain transgenic animals without the need for embryonic manipulation. About 1-5 g of nucleic acid molecule, e.g., DNA, RNA may be contemplated for in vivo administration in liposomes.

In another embodiment, the CRISPR Cas system may be administered in liposomes, such as a stable nucleic-acid-lipid particle (SNALP) (see, e.g., Morrissey et al., Nature Biotechnology, Vol. 23, No. 8, August 2005). Daily intravenous injections of about 1, 3 or 5 mg/kg/day of a specific CRISPR Cas targeted in a SNALP are contemplated. The daily treatment may be over about three days and then weekly for about five weeks. In another embodiment, a specific CRISPR Cas encapsulated SNALP) administered by intravenous injection to at doses of abpit 1 or 2.5 mg/kg are also contemplated (see, e.g., Zimmerman et al., Nature Letters, Vol. 441, 4 May 2006). The SNALP formulation may contain the lipids 3-N-[(wmethoxypoly(ethylene glycol) 2000) carbamoyl] -! ,2-dimyristyloxy-propylamine (PEG-C-DMA), 1,2-dilinoleyloxy-N,N-dimethyl-3-aminopropane (DLinDMA), 1,2-distearoyl-sn-glycero-3-phosphocholine (DSPC) and cholesterol, in a 2:40:10:48 molar per cent ratio (see, e.g., Zimmerman et al., Nature Letters, Vol. 441, 4 May 2006).

In another embodiment, stable nucleic-acid-lipid particles (SNALPs) have proven to be effective delivery molecules to highly vascularized HepG2-derived liver tumors but not in poorly vascularized HCT-116 derived liver tumors (see, e.g., Li, Gene Therapy (2012) 19, 775-780). The SNALP liposomes may be prepared by formulating D-Lin-DMA and PEG-C-DMA with distearoylphosphatidylcholine (DSPC), Cholesterol and siRNA using a 25:1 lipid/siRNA ratio and a 48/40/10/2 molar ratio of Cholesterol/D-Lin-DMA/DSPC/PEG-C-DMA. The resulted SNALP liposomes are about 80-100 nm in size.

In yet another embodiment, a SNALP may comprise synthetic cholesterol (Sigma-Aldrich, St Louis, MO, USA), dipalmitoylphosphatidylcholine (Avanti Polar Lipids, Alabaster, AL, USA), 3-N-[(w-methoxy poly(ethylene glycol)2000)carbamoyl]-1,2-dimyrestyloxypropylamine, and cationic 1,2-dilinoleyloxy-3-N,Ndimethylaminopropane (see, e.g., Geisbert et al., Lancet 2010; 375: 1896-905). A dosage of about 2 mg/kg total CRISPR Cas per dose administered as, for example, a bolus intravenous infusion may be contemplated.

In yet another embodiment, a SNALP may comprise synthetic cholesterol (Sigma-Aldrich), 1,2-distearoyl-sn-glycero-3-phosphocholine (DSPC; Avanti Polar Lipids Inc.), PEG-cDMA, and 1,2-dilinoleyloxy-3-(N;N-dimethyl)aminopropane (DLinDMA) (see, e.g., Judge, J. Clin. Invest. 119:661-673 (2009)). Formulations used for in vivo studies may comprise a final lipid/RNA mass ratio of about 9:1.

The safety profile of RNAi nanomedicines has been reviewed by Barros and Gollob of Alnylam Pharmaceuticals (see, e.g., Advanced Drug Delivery Reviews 64 (2012) 1730-1737). The stable nucleic acid lipid particle (SNALP) is comprised of four different lipids - an ionizable lipid (DLinDMA) that is cationic at low pH, a neutral helper lipid, cholesterol, and a diffusible polyethylene glycol (PEG)-lipid. The particle is approximately 80 nm in diameter and is charge-neutral at physiologic pH. During formulation, the ionizable lipid serves to condense lipid with the anionic siRNA during particle formation. When positively charged under increasingly acidic endosomal conditions, the ionizable lipid also mediates the fusion of SNALP with the endosomal membrane enabling release of siRNA into the cytoplasm. The PEG-lipid stabilizes the particle and reduces aggregation during formulation, and subsequently provides a neutral hydrophilic exterior that improves pharmacokinetic properties.

To date, two clinical programs have been initiated using SNALPsiRNA formulations. Tekmira Pharmaceuticals recently completed a phase I single-dose study of SNALP-ApoB in adult volunteers with elevated LDL cholesterol. ApoB is predominantly expressed in the liver and jejunum and is essential for the assembly and secretion of VLDL and LDL. ApoB is also succesfuly targeted by our CrISPR-Cas systems, see examples 38-39. Seventeen subjects received a single dose of SNALP-ApoB (dose escalation across 7 dose levels). There was no evidence of liver toxicity (anticipated as the potential dose-limiting toxicity based on preclinical studies). One (of two) subjects at the highest dose experienced flu-like symptoms consistent with immune system stimulation, and the decision was made to conclude the trial.

Alnylam Pharmaceuticals has similarly advanced ALN-TTR01, which employs the SNALP technology described above and targets hepatocyte production of both mutant and wild-type TTR to treat TTR amyloidosis (ATTR). Three ATTR syndromes have been described: familial amyloidotic polyneuropathy (FAP) and familial amyloidotic cardiomyopathy (FAC)-both caused by autosomal dominant mutations in TTR; and senile systemic amyloidosis (SSA) cause by wildtype TTR. A placebo-controlled, single dose-escalation phase I trial of ALN-TTR01 was recently completed in patients with ATTR. ALN-TTR01 was administered as a 15-minute IV infusion to 31 patients (23 with study drug and 8 with placebo) within a dose range of 0.01 to 1.0 mg/kg (based on siRNA). Treatment was well tolerated with no significant increases in liver function tests. Infusion-related reactions were noted in 3 of 23 patients at≥0.4 mg/kg; all responded to slowing of the infusion rate and all continued on study. Minimal and transient elevations of serum cytokines IL-6, IP-10 and IL-1ra were noted in two patients at the highest dose of 1 mg/kg (as anticipated from preclinical and NHP studies). Lowering of serum TTR, the expected pharmacodynamics effect of ALN-TTR01, was observed at 1 mg/kg.

In yet another embodiment, a SNALP may be made by solubilizing a cationic lipid, DSPC, cholesterol and PEG-lipid were solubilized in ethanol at a molar ratio of 40:10:40:10, respectively (see, Semple et al., Nature Niotechnology, Volume 28 Number 2 February 2010, pp. 172-177). The lipid mixture was added to an aqueous buffer (50 mM citrate, pH 4) with mixing to a final ethanol and lipid concentration of 30% (vol/vol) and 6.1 mg/ml, respectively, and allowed to equilibrate at 22 °C for 2 min before extrusion. The hydrated lipids were extruded through two stacked 80 nm pore-sized filters (Nuclepore) at 22 °C using a Lipex Extruder (Northern Lipids) until a vesicle diameter of 70-90 nm, as determined by dynamic light scattering analysis, was obtained. This generally required 1-3 passes. The siRNA (solubilized in a 50 mM citrate, pH 4 aqueous solution containing 30% ethanol) was added to the pre-equilibrated (35 °C) vesicles at a rate of ~5 ml/min with mixing. After a final target siRNA/lipid ratio of 0.06 (wt/wt) was reached, the mixture was incubated for a further 30 min at 35 °C to allow vesicle reorganization and encapsulation of the siRNA. The ethanol was then removed and the external buffer replaced with PBS (155 mM NaCl, 3 mM Na2HPO4, 1 mM KH2PO4, pH 7.5) by either dialysis or tangential flow diafiltration. siRNA were encapsulated in SNALP using a controlled step-wise dilution method process. The lipid constituents of KC2-SNALP were DLin-KC2-DMA (cationic lipid), dipalmitoylphosphatidylcholine (DPPC; Avanti Polar Lipids), synthetic cholesterol (Sigma) and PEG-C-DMA used at a molar ratio of 57.1:7.1:34.3:1.4. Upon formation of the loaded particles, SNALP were dialyzed against PBS and filter sterilized through a 0.2 $\mu$m filter before use. Mean particle sizes were 75-85 nm and 90-95% of the siRNA was encapsulated within the lipid particles. The final siRNA/lipid ratio in formulations used for in vivo testing was ~0.15 (wt/wt). LNP-siRNA systems containing Factor VII siRNA were diluted to the appropriate concentrations in sterile PBS immediately before use and the formulations were administered intravenously through the lateral tail vein in a total volume of 10 ml/kg. This method may be extrapolated to the CRISPR Cas system.

## Other Lipids

Other cationic lipids, such as amino lipid 2,2-dilinoleyl-4-dimethylaminoethyl-[1,3]-dioxolane (DLin-KC2-DMA) may be utilized to encapsulate CRISPR Cas similar to SiRNA (see, e.g., Jayaraman, Angew. Chem. Int. Ed. 2012, 51, 8529 -8533). A preformed vesicle with the following lipid composition may be contemplated: amino lipid, distearoylphosphatidylcholine (DSPC), cholesterol and (R)-2,3-bis(octadecyloxy) propyl-1-(methoxy poly(ethylene glycol)2000)propylcarbamate (PEG-lipid) in the molar ratio 40/10/40/10, respectively, and a FVII siRNA/total lipid ratio of approximately 0.05 (w/w). To ensure a narrow particle size distribution in the range of 70-90 nm and a low polydispersity index of 0.11_0.04 (n=56), the particles may be extruded up to three times through 80 nm membranes prior to adding the CRISPR Cas RNA. Particles containing the highly potent amino lipid 16 may be used, in which the molar ratio of the four lipid components 16, DSPC, cholesterol and PEG-lipid (50/10/38.5/1.5) which may be further optimized to enhance in vivo activity.

Michael S D Kormann et al. ("Expression of therapeutic proteins after delivery of chemically modified mRNA in mice: Nature Biotechnology, Volume:29, Pages: 154-157 (2011) Published online 09 January 2011) describes the use of lipid envelopes to deliver RNA. Use of lipid envelopes is also preferred in the present invention.

In another embodiment, lipids may be formulated with the CRISPR Cas system to form lipid nanoparticles (LNPs). Lipids include, but are not limited to, DLin-KC2-DMA4, C12-200 and colipids disteroylphosphatidyl choline, cholesterol, and PEG-DMG may be formulated with CRISPR Cas instead of siRNA (see, e.g., Novobrantseva, Molecular Therapy-Nucleic Acids (2012) 1, e4; doi:10.1038/mtna.2011.3) using a spontaneous vesicle formation procedure. The component molar ratio may be about 50/10/38.5/1.5 (DLin-KC2-DMA or C12-200/disteroylphosphatidyl choline/cholesterol/PEG-DMG). The final lipid:siRNA weight ratio may be ~12:1 and 9:1 in the case of DLin-KC2-DMA and C12-200 lipid nanoparticles (LNPs), respectively. The formulations may have mean particle diameters of ~80 nm with >90% entrapment efficiency. A 3 mg/kg dose may be contemplated.

Tekmira has a portfolio of approximately 95 patent families, in the U.S. and abroad, that are directed to various aspects of LNPs and LNP formulations (see, e.g., U.S. Pat. Nos. 7,982,027; 7,799,565; 8,058,069; 8,283,333; 7,901,708; 7,745,651; 7,803,397; 8,101,741; 8,188,263; 7,915,399; 8,236,943 and 7,838,658 and European Pat. Nos .1766035; 1519714; 1781593 and 1664316), all of which may be used/and or adapted to the present invention.

The CRISPR Cas system may be delivered encapsulated in PLGA Microspheres such as that further described in US published applications 20130252281 and 20130245107 and 20130244279 (assigned to Moderna Therapeutics) which relate to aspects of formulation of compositions comprising modified nucleic acid molecules which may encode a protein, a protein precursor, or a partially or fully processed form of the protein or a protein precursor. The formulation may have a molar ratio 50:10:38.5:1.5-3.0 (cationic lipid:fusogenic lipid:cholesterol:PEG lipid). The PEG lipid may be selected from, but is not limited to PEG-c-DOMG, PEG-DMG. The fusogenic lipid may be DSPC. See also, Schrum et al., Delivery and Formulation of Engineered Nucleic Acids, US published application 20120251618.

Nanomerics' technology addresses bioavailability challenges for a broad range of therapeutics, including low molecular weight hydrophobic drugs, peptides, and nucleic acid based therapeutics (plasmid, siRNA, miRNA). Specific administration routes for which the technology has demonstrated clear advantages include the oral route, transport across the blood-brain-barrier, delivery to solid tumours, as well as to the eye. See, e.g., Mazza et al., 2013, ACS Nano. 2013 Feb 26;7(2):1016-26; Uchegbu and Siew, 2013, J Pharm Sci. 102(2):305-10 and Lalatsa et al., 2012, J Control Release. 2012 Jul 20;161(2):523-36.

US Patent Publication No. 20050019923 describes cationic dendrimers for delivering bioactive molecules, such as polynucleotide molecules, peptides and polypeptides and/or pharmaceutical agents, to a mammalian body. The dendrimers are suitable for targeting the delivery of the bioactive molecules to, for example, the liver, spleen, lung, kidney or heart. Dendrimers are synthetic 3-dimensional macromolecules that are prepared in a step-wise fashion from simple branched monomer units, the nature and functionality of which can be easily controlled and varied. Dendrimers are synthesised from the repeated addition of building blocks to a multifunctional core (divergent approach to synthesis), or towards a multifunctional core

(convergent approach to synthesis) and each addition of a 3-dimensional shell of building blocks leads to the formation of a higher generation of the dendrimers. Polypropylenimine dendrimers start from a diaminobutane core to which is added twice the number of amino groups by a double Michael addition of acrylonitrile to the primary amines followed by the hydrogenation of the nitriles. This results in a doubling of the amino groups. Polypropylenimine dendrimers contain 100% protonable nitrogens and up to 64 terminal amino groups (generation 5, DAB 64). Protonable groups are usually amine groups which are able to accept protons at neutral pH. The use of dendrimers as gene delivery agents has largely focused on the use of the polyamidoamine. and phosphorous containing compounds with a mixture of amine/amide or N--P($O_2$)S as the conjugating units respectively with no work being reported on the use of the lower generation polypropylenimine dendrimers for gene delivery. Polypropylenimine dendrimers have also been studied as pH sensitive controlled release systems for drug delivery and for their encapsulation of guest molecules when chemically modified by peripheral amino acid groups. The cytotoxicity and interaction of polypropylenimine dendrimers with DNA as well as the transfection efficacy of DAB 64 has also been studied.

US Patent Publication No. 20050019923 is based upon the observation that, contrary to earlier reports, cationic dendrimers, such as polypropylenimine dendrimers, display suitable properties, such as specific targeting and low toxicity, for use in the targeted delivery of bioactive molecules, such as genetic material. In addition, derivatives of the cationic dendrimer also display suitable properties for the targeted delivery of bioactive molecules. See also, Bioactive Polymers, US published application 20080267903, which discloses "Various polymers, including cationic polyamine polymers and dendrimeric polymers, are shown to possess anti-proliferative activity, and may therefore be useful for treatment of disorders characterised by undesirable cellular proliferation such as neoplasms and tumours, inflammatory disorders (including autoimmune disorders), psoriasis and atherosclerosis. The polymers may be used alone as active agents, or as delivery vehicles for other therapeutic agents, such as drug molecules or nucleic acids for gene therapy. In such cases, the polymers' own intrinsic anti-tumour activity may complement the activity of the agent to be delivered."

## Supercharged proteins

Supercharged proteins are a class of engineered or naturally occurring proteins with unusually high positive or negative net theoretical charge. Both supernegatively and superpositively charged proteins exhibit a remarkable ability to withstand thermally or chemically induced aggregation. Superpositively charged proteins are also able to penetrate mammalian cells. Associating cargo with these proteins, such as plasmid DNA, siRNA, or other proteins, can enable the functional delivery of these macromolecules into mammalian cells both in vitro and in vivo. David Liu's lab reported the creation and characterization of supercharged proteins in 2007 (Lawrence et al., 2007, Journal of the American Chemical Society 129, 10110-10112).

The nonviral delivery of siRNA and plasmid DNA into mammalian cells are valuable both for research and therapeutic applications (Akinc et al., 2010, Nat. Biotech. 26, 561-569). Purified +36 GFP protein (or other superpositively charged protein) is mixed with siRNAs in the appropriate serum-free media and allowed to complex prior addition to cells. Inclusion of serum at this stage inhibits formation of the supercharged protein-siRNA complexes and reduces the effectiveness of the treatment. The following protocol has been found to be effective for a variety of cell lines (McNaughton et al., 2009, Proc. Natl. Acad. Sci. USA 106, 6111-6116). However, pilot experiments varying the dose of protein and siRNA should be performed to optimize the procedure for specific cell lines.

(1) One day before treatment, plate $1 \times 10^5$ cells per well in a 48-well plate.
(2) On the day of treatment, dilute purified +36 GFP protein in serumfree media to a final concentration 200nM. Add siRNA to a final concentration of 50nM. Vortex to mix and incubate at room temperature for 10min.
(3) During incubation, aspirate media from cells and wash once with PBS.
(4) Following incubation of +36 GFP and siRNA, add the protein-siRNA complexes to cells.
(5) Incubate cells with complexes at 37 C for 4h.
(6) Following incubation, aspirate the media and wash three times with 20 U/mL heparin PBS. Incubate cells with serum-containing media for a further 48h or longer depending upon the assay for knockdown.

(7) Analyze cells by immunoblot, qPCR, phenotypic assay, or other appropriate method.

It has been found +36 GFP to be an effective plasmid delivery reagent in a range of cells. As plasmid DNA is a larger cargo than siRNA, proportionately more +36 GFP protein is required to effectively complex plasmids. For effective plasmid delivery Applicants have developed a variant of +36 GFP bearing a C-terminal HA2 peptide tag, a known endosome-disrupting peptide derived from the influenza virus hemagglutinin protein. The following protocol has been effective in a variety of cells, but as above it is advised that plasmid DNA and supercharged protein doses be optimized for specific cell lines and delivery applications.

(1) One day before treatment, plate 1 x 10$^5$ per well in a 48-well plate.
(2) On the day of treatment, dilute purified Þ36 GFP protein in serumfree media to a final concentration 2 mM. Add 1mg of plasmid DNA. Vortex to mix and incubate at room temperature for 10min.
(3) During incubation, aspirate media from cells and wash once with PBS.
(4) Following incubation of Þ36 GFP and plasmid DNA, gently add the protein-DNA complexes to cells.
(5) Incubate cells with complexes at 37 C for 4h.
(6) Following incubation, aspirate the media and wash with PBS. Incubate cells in serum-containing media and incubate for a further 24-48h.
(7) Analyze plasmid delivery (e.g., by plasmid-driven gene expression) as appropriate.

See also, e.g., McNaughton et al., Proc. Natl. Acad. Sci. USA 106, 6111-6116 (2009); Cronican et al., ACS Chemical Biology 5, 747-752 (2010); Cronican et al., Chemistry & Biology 18, 833-838 (2011); Thompson et al., Methods in Enzymology 503, 293-319 (2012); Thompson, D.B., et al., Chemistry & Biology 19 (7), 831-843 (2012). The methods of the super charged proteins may be used and/or adapted for delivery of the CRISPR Cas system.

## Cell penetrating peptides

In yet another embodiment, cell penetrating peptides (CPPs) are contemplated for the delivery of the CRISPR Cas system. CPPs are short peptides that facilitate cellular uptake of various molecular cargo (from nanosize particles to small chemical molecules and large fragments of DNA). The term "cargo" as used herein includes but is not limited to the group consisting of therapeutic agents, diagnostic probes, peptides, nucleic acids, antisense oligonucleotides, plasmids, proteins, nanoparticles, liposomes, chromophores, small molecules and radioactive materials. In aspects of the invention, the cargo may also comprise any

component of the CRISPR Cas system or the entire functional CRISPR Cas system. Aspects of the present disclosure further provide methods for delivering a desired cargo into a subject

comprising: (a) preparing a complex comprising the cell penetrating peptide

and a desired cargo, and (b) orally, intraarticularly, intraperitoneally, intrathecally, intrarterially, intranasally, intraparenchymally, subcutaneously, intramuscularly, intravenously, dermally, intrarectally, or topically administering the complex to a subject. The cargo is associated with the peptides either through chemical linkage via covalent bonds or through non-covalent interactions.

The function of the CPPs are to deliver the cargo into cells, a process that commonly occurs through endocytosis with the cargo delivered to the endosomes of living mammalian cells. Cell-penetrating peptides are of different sizes, amino acid sequences, and charges but all CPPs have one distinct characteristic, which is the ability to translocate the plasma membrane and facilitate the delivery of various molecular cargoes to the cytoplasm or an organelle. CPP translocation may be classified into three main entry mechanisms: direct penetration in the membrane, endocytosis-mediated entry, and translocation through the formation of a transitory structure. CPPs have found numerous applications in medicine as drug delivery agents in the treatment of different diseases including cancer and virus inhibitors, as well as contrast agents for cell labeling. Examples of the latter include acting as a carrier for GFP, MRI contrast agents, or quantum dots. CPPs hold great potential as in vitro and in vivo delivery vectors for use in research and medicine. CPPs typically have an amino acid composition that either contains a high relative abundance of positively charged amino acids such as lysine or arginine or has sequences that contain an alternating pattern of polar/charged amino acids and non-polar, hydrophobic amino acids. These two types of structures are referred to as polycationic or amphipathic, respectively. A third class of CPPs are the hydrophobic peptides, containing only apolar residues, with low net charge or have hydrophobic amino acid groups that are crucial for cellular uptake. One of the initial CPPs discovered was the trans-activating transcriptional activator (Tat) from Human Immunodeficiency Virus 1 (HIV-1) which was found to be efficiently taken up from the surrounding media by numerous cell types in culture. Since then, the number of known CPPs has expanded considerably and small molecule synthetic analogues with more effective protein transduction properties have been generated. CPPs include but are not limited to Penetratin, Tat (48-60), Transportan, and (R-AhX-R4) (Ahx=aminohexanoyl).

U.S. patent 8,372,951provides a CPP derived from eosinophil cationic protein (ECP) which exhibits highly cell-penetrating efficiency and low toxicity. Aspects of delivering the CPP with its cargo into a vertebrate subject are also provided. Further aspects of CPPs and their delivery may be in U.S. patents 8,575,305; 8;614,194 and 8,044,019.

That CPPs can be employed to deliver the CRISPR-Cas system is also provided in the manuscript "Gene disruption by cell-penetrating peptide-mediated delivery of Cas9 protein and guide RNA", by Suresh Ramakrishna, Abu-Bonsrah Kwaku Dad, Jagadish Beloor, et al. Genome Res. 2014 Apr 2. [Epub ahead of print], incorporated by reference in its entirety, wherein it is demonstrated that treatment with CPP-conjugated recombinant Cas9 protein and CPP-complexed guide RNAs lead to endogenous gene disruptions in human cell lines. In the paper the Cas9 protein was conjugated to CPP via a thioether bond, whereas the guide RNA was complexed with CPP, forming condensed, positively charged nanoparticles. It was shown that simultaneous and sequential treatment of human cells, including embryonic stem cells, dermal fibroblasts, HEK293T cells, HeLa cells, and embryonic carcinoma cells, with the modified Cas9 and guide RNA led to efficient gene disruptions with reduced off-target mutations relative to plasmid transfections.

## Implantable devices

In another embodiment, implantable devices are also contemplated for delivery of the CRISPR Cas system. For example, US Patent Publication 20110195123 discloses an implantable medical device which elutes a drug locally and in prolonged period is provided, including several types of such a device, the treatment modes of implementation and methods of implantation. The device comprising of polymeric substrate, such as a matrix for example, that is used as the device body, and drugs, and in some cases additional scaffolding materials, such as metals or additional polymers, and materials to enhance visibility and imaging. The selection of drug is based on the advantageous of releasing drug locally and in prolonged period, where drug is released directly to the extracellular matrix (ECM) of the diseased area such as tumor, inflammation, degeneration or for symptomatic objectives, or to injured smooth muscle cells, or for prevention. One kind of drug is the gene silencing drugs based on RNA interference (RNAi), including but not limited to si RNA, sh RNA, or antisense RNA/DNA, ribozyme and nucleoside analogs. Therefore, this system may be used/and or adapted to the CRISPR Cas system. The

modes of implantation in some embodiments are existing implantation procedures that are developed and used today for other treatments, including brachytherapy and needle biopsy. In such cases the dimensions of the new implant described in this invention are similar to the original implant. Typically a few devices are implanted during the same treatment procedure.

As described in US Patent Publication 20110195123, there is provided a drug delivery implantable or insertable system, including systems applicable to a cavity such as the abdominal cavity and/or any other type of administration in which the drug delivery system is not anchored or attached, comprising a biostable and/or degradable and/or bioabsorbable polymeric substrate, which may for example optionally be a matrix. It should be noted that the term "insertion" also includes implantation. The drug delivery system is preferably implemented as a "Loder" as described in US Patent Publication 20110195123.

The polymer or plurality of polymers are biocompatible, incorporating an agent and/or plurality of agents, enabling the release of agent at a controlled rate, wherein the total volume of the polymeric substrate, such as a matrix for example, in some embodiments is optionally and preferably no greater than a maximum volume that permits a therapeutic level of the agent to be reached. As a non-limiting example, such a volume is preferably within the range of $0.1$ m$^3$ to 1000 mm$^3$, as required by the volume for the agent load. The Loder may optionally be larger, for example when incorporated with a device whose size is determined by functionality, for example and without limitation, a knee joint, an intra-uterine or cervical ring and the like.

The drug delivery system (for delivering the composition) is designed in some embodiments to preferably employ degradable polymers, wherein the main release mechanism is bulk erosion; or in some embodiments, non degradable, or slowly degraded polymers are used, wherein the main release mechanism is diffusion rather than bulk erosion, so that the outer part functions as membrane, and its internal part functions as a drug reservoir, which practically is not affected by the surroundings for an extended period (for example from about a week to about a few months). Combinations of different polymers with different release mechanisms may also optionally be used. The concentration gradient at the surface is preferably maintained effectively constant during a significant period of the total drug releasing period, and therefore the diffusion rate is effectively constant (termed "zero mode" diffusion). By the term "constant" it is meant a diffusion rate that is preferably maintained above the lower threshold of therapeutic effectiveness, but which may still optionally feature an initial burst and/or fluctuate, for example increasing and decreasing to a certain degree. The diffusion rate is preferably so maintained for a prolonged period, and it can be considered constant to a certain level to optimize the therapeutically effective period, for example the effective silencing period.

The drug delivery system optionally and preferably is designed to shield the nucleotide based therapeutic agent from degradation, whether chemical in nature or due to attack from enzymes and other factors in the body of the subject.

The drug delivery system as described in US Patent Publication 20110195123 is optionally associated with sensing and/or activation appliances that are operated at and/or after implantation of the device, by non and/or minimally invasive methods of activation and/or acceleration/deceleration, for example optionally including but not limited to thermal heating and cooling, laser beams, and ultrasonic, including focused ultrasound and/or RF (radiofrequency) methods or devices.

According to some embodiments of US Patent Publication 20110195123, the site for local delivery may optionally include target sites characterized by high abnormal proliferation of cells, and suppressed apoptosis, including tumors, active and or chronic inflammation and infection including autoimmune diseases states, degenerating tissue including muscle and nervous tissue, chronic pain, degenerative sites, and location of bone fractures and other wound locations for enhancement of regeneration of tissue, and injured cardiac, smooth and striated muscle. The site for local delivery also may optionally include sites enabling performing preventive activities including pregnancy, prevention of infection and aging.

The site for implantation of the composition, or target site, preferably features a radius, area and/or volume that is sufficiently small for targeted local delivery. For example, the target site optionally has a diameter in a range of from about 0.1 mm to about 5 cm.

The location of the target site is preferably selected for maximum therapeutic efficacy. For example, the composition of the drug delivery system (optionally with a device for implantation as described above) is optionally and preferably implanted within or in the proximity of a tumor environment, or the blood supply associated thereof.

For example the composition (optionally with the device) is optionally implanted within or in the proximity to pancreas, prostate, breast, liver, via the nipple, within the vascular system and so forth.

The target location is optionally selected from the group consisting of (as non-limiting examples only, as optionally any site within the body may be suitable for implanting a Loder): 1. brain at degenerative sites like in Parkinson or Alzheimer disease at the basal ganglia, white and gray matter; 2. spine as in the case of amyotrophic lateral sclerosis (ALS); 3. uterine cervix to prevent HPV infection; 4. active and chronic inflammatory joints; 5. dermis as in the case of psoriasis; 6. sympathetic and sensoric nervous sites for analgesic effect; 7. Intra osseous implantation; 8. acute and chronic infection sites; 9. Intra vaginal; 10. Inner ear--auditory system, labyrinth of the inner ear, vestibular system; 11. Intra tracheal; 12. Intra-cardiac; coronary, epicardiac; 13. urinary bladder; 14. biliary system; 15. parenchymal tissue including and not limited to the kidney, liver, spleen; 16. lymph nodes; 17. salivary glands; 18. dental gums; 19. Intra-articular (into joints); 20. Intra-ocular; 21. Brain tissue; 22. Brain ventricles; 23. Cavities, including abdominal cavity (for example but without limitation, for ovary cancer); 24. Intra esophageal and 25. Intra rectal.

Optionally insertion of the system (for example a device containing the composition) is associated with injection of material to the ECM at the target site and the vicinity of that site to affect local pH and/or temperature and/or other biological factors affecting the diffusion of the drug and/or drug kinetics in the ECM, of the target site and the vicinity of such a site.

Optionally, according to some embodiments, the release of said agent could be associated with sensing and/or activation appliances that are operated prior and/or at and/or after insertion, by non and/or minimally invasive and/or else methods of activation and/or acceleration/deceleration, including laser beam, radiation, thermal heating and cooling, and ultrasonic, including focused ultrasound and/or RF (radiofrequency) methods or devices, and chemical activators.

According to other embodiments of US Patent Publication 20110195123, the drug preferably comprises a gene silencing biological RNAi drug, for example for localized cancer cases in breast, pancreas, brain, kidney, bladder, lung, and prostate as described below. Moreover, many drugs other than siRNA are applicable to be encapsulated in Loder, and can be used in association with this invention, as long as such drugs can be encapsulated with the Loder substrate, such as a matrix for example. Such drugs include approved drugs that are delivered today by methods other than of this invention, including Amphotericin B for fungal infection; antibiotics such as in osteomyelitis; pain killers such as narcotics; anti degenerative such as in Alzheimer or Parkinson diseases in a Loder implanted in the vicinity of the spine in the case of back pain. Such a system may be used and/or adapted to deliver the CRISPR Cas .

For example, for specific applications such as prevention of growth or regrowth of smooth muscle cells (that are injured during a stenting procedure and as a result tend to proliferate), the drug may optionally be siRNA that silence smooth muscle cells, including H19 silencing, or a drug selected from the group consisting of taxol, rapamycin and rapamycin-analogs. In such cases the Loder is preferably either a Drug Eluting Stent (DES), with prolonged release at constant rate, or a dedicated device that is implanted separately, in association to the stent. All of this may be used/and or adapted to the CRISPR Cas system.

As another example of a specific application, neuro and muscular degenerative diseases develop due to abnormal gene expression. Local delivery of silencing RNAs may have therapeutic properties for interfering with such abnormal gene expression. Local delivery of anti apoptotic, anti inflammatory and anti degenerative drugs including small drugs and macromolecules may also optionally be therapeutic. In such cases the Loder is applied for prolonged release at constant rate and/or through a dedicated device that is implanted separately. All of this may be used and/or adapted to the CRISPR Cas system.

As yet another example of a specific application, psychiatric and cognitive disorders are treated with gene modifiers. Gene knockdown with silencing RNA is a treatment option. Loders locally delivering nucleotide based agents to central nervous system sites are therapeutic options for psychiatric and cognitive disorders including but not limited to psychosis, bi-polar diseases, neurotic disorders and behavioral maladies. The Loders could also deliver locally drugs including small drugs and macromolecules upon implantation at specific brain sites. All of this may be used and/or adapted to the CRISPR Cas system of the present invention.

As another example of a specific application, silencing of innate and/or adaptive immune mediators at local sites enables the prevention of organ transplant rejection. Local delivery of silencing RNAs and immunomodulating reagents with the Loder implanted into the transplanted organ and/or the implanted site renders local immune suppression by repelling immune cells such as CD8 activated against the transplanted organ. All of this may be used/and or adapted to the CRISPR Cas system.

As another example of a specific application, vascular growth factors including VEGFs and angiogenin and others are essential for neovascularization. Local delivery of the factors, peptides, peptidomimetics, or suppressing their repressors is an important therapeutic modality; silencing the repressors and local delivery of the factors, peptides, macromolecules and small drugs stimulating angiogenesis with the Loder is therapeutic for peripheral, systemic and cardiac vascular disease.

The method of insertion, such as implantation, may optionally already be used for other types of tissue implantation and/or for insertions and/or for sampling tissues, optionally without modifications, or alternatively optionally only with non-major modifications in such methods. Such methods optionally include but are not limited to brachytherapy methods, biopsy, endoscopy with and/or without ultrasound, such as ERCP, stereotactic methods into the brain tissue, Laparoscopy, including implantation with a laparoscope into joints, abdominal organs, the bladder wall and body cavities.

## CRISPR enzyme mRNA and guide RNA

CRISPR enzyme mRNA and guide RNA might also be delivered separately. CRISPR enzyme mRNA can be delivered prior to the guide RNA to give time for CRISPR enzyme to be expressed. CRISPR enzyme mRNA might be administered 1-12 hours (preferably around 2-6 hours) prior to the administration of guide RNA.

Alternatively, CRISPR enzyme mRNA and guide RNA can be administered together. Advantageously, a second booster dose of guide RNA can be administered 1-12 hours (preferably around 2-6 hours) after the initial administration of CRISPR enzyme mRNA + guide RNA.

Additional administrations of CRISPR enzyme mRNA and/or guide RNA might be useful to achieve the most efficient levels of genome modification.

For minimization of toxicity and off-target effect, it will be important to control the concentration of CRISPR enzyme mRNA and guide RNA delivered. Optimal concentrations of CRISPR enzyme mRNA and guide RNA can be determined by testing different concentrations in a cellular or animal model and using deep sequencing the analyze the extent of modification at potential off-target genomic loci. For example, for the guide sequence targeting 5'-GAGTCCGAGCAGAAGAAGAA-3' (SEQ ID NO: 5) in the EMX1 gene of the human genome, deep sequencing can be used to assess the level of modification at the following two off-target loci, 1: 5'-GAGTCCTAGCAGGAGAAGAA-3' (SEQ ID NO: 6) and 2: 5'-GAGTCTAAGCAGAAGAAGAA-3' (SEQ ID NO: 7). The concentration that gives the highest level of on-target modification while minimizing the level of off-target modification should be chosen for in vivo delivery.

Alternatively, to minimize the level of toxicity and off-target effect, CRISPR enzyme nickase mRNA (for example S. pyogenes Cas9 with the D10A mutation) can be delivered with a pair of guide RNAs targeting a site of interest. The two guide RNAs need to be spaced as follows. Guide sequences in red (single underline) and blue (double underline) respectively (these examples are based on the PAM requirement for *Streptococcus pyogenes* Cas9).

(SEQ ID NO: 8-61)

| Overhang length (bp) | Guide RNA design (guide sequence and PAM color coded) |
|---|---|
| 14 | 5'-<br>NNNNNNNNNNNNNNNNNNNNCCN<u>NNNNNNNNNNNNNNNNNNNN</u><br><u>NNN</u><u>GG</u>NNNNNNNNNNNNNNNN-3' |
| 13 | 3'-<br>NNNNNNNNNNNNNNNNNNNNN<u>GG</u><u>N</u><u>NNNNNNNNNNNNNNNNNNNN</u><br><u>NNN</u>NCCNNNNNNNNNNNNNNNN-5' |

(continued)

| Overhang length (bp) | Guide RNA design (guide sequence and PAM color coded) |
|---|---|
| 12 | 5'-NNNNNNNNNNNNNNNNNNNCCNNNNNNNNNNNNNNNNNNNNNNGGNNNNNNNNNNNNNNNN-3' |
| 11 | 3'-NNNNNNNNNNNNNNNNNNNNGGNNNNNNNNNNNNNNNNNNNNNNNCCNNNNNNNNNNNNNNNN-5' |
| 10 | 5'-NNNNNNNNNNNNNNNNNNNCCNNNNNNNNNNNNNNNNNNNNNGGNNNNNNNNNNNNNNNN-3' |
| 9 | 3'-NNNNNNNNNNNNNNNNNNNNGGNNNNNNNNNNNNNNNNNNNNNNNCCNNNNNNNNNNNNNNNN-5' |
| 8 | |
| 7 | 5'-NNNNNNNNNNNNNNNNNNNCCNNNNNNNNNNNNNNNNNNNNNGGNNNNNNNNNNNNNNNN-3' |
| 6 | 3'-NNNNNNNNNNNNNNNNNNNNGGNNNNNNNNNNNNNNNNNNNNNNNCCNNNNNNNNNNNNNNNN-5' |
| 5 | 5'-NNNNNNNNNNNNNNNNNNNNCCNNNNNNNNNNNNNNNNNNNNNGGNNNNNNNNNNNNNNNN-3' |
| 4 | 3'-NNNNNNNNNNNNNNNNNNNNGGNNNNNNNNNNNNNNNNNNNNNNNCCNNNNNNNNNNNNNNNN-5' |
| 3 | 5'-NNNNNNNNNNNNNNNNNNNNNNCCNNNNNNNNNNNNNNNNNNNNNGGNNNNNNNNNNNNNNNN-3' |
| 2 | 3'-NNNNNNNNNNNNNNNNNNNNNGGNNNNNNNNNNNNNNNNNNNNNCCNNNNNNNNNNNNNNNN-5' |
| 1 | 5'-NNNNNNNNNNNNNNNNNNNNNNNCCNNNNNNNNNNNNNNNNNNNNNGGNNNNNNNNNNNNNNNN-3' |

(continued)

| Overhang length (bp) | Guide RNA design (guide sequence and PAM color coded) |
|---|---|
| blunt | 3'-<br>NNNNNNNNNNNNNNNNNNNGGNNNNNNNNNNNNNNNNNNCCN<br>NNNNNNNNNNNNNNNNNN-5' |
| 1<br>2 | 5'-<br>NNNNNNNNNNNNNNNNNNNNNCCNNNNNNNNNNNNNNNGGNN<br>NNNNNNNNNNNNNNNNNN-3' |
| 3 | 3'-<br>NNNNNNNNNNNNNNNNNNNNGGNNNNNNNNNNNNNNNNCCNN<br>NNNNNNNNNNNNNNNNNN-5' |
| 4 | 5'-<br>NNNNNNNNNNNNNNNNNNNNNCCNNNNNNNNNNNNNNNGGNNN<br>NNNNNNNNNNNNNNNNNN-3' |
| 5 | 3'-<br>NNNNNNNNNNNNNNNNNNNNNGGNNNNNNNNNNNNNNCCNNN<br>NNNNNNNNNNNNNNNNNN-5' |
| 6 | 5'-<br>NNNNNNNNNNNNNNNNNNNNNNCCNNNNNNNNNNNNNGGNNNN<br>NNNNNNNNNNNNNNNNNN-3' |
| 7 | 3'-<br>NNNNNNNNNNNNNNNNNNNNNNGGNNNNNNNNNNNNNCCNNNN<br>NNNNNNNNNNNNNNNNNN-5' |
| 8 | 5'-<br>NNNNNNNNNNNNNNNNNNNNNNNCCNNNNNNNNNNNNNGGNNNN<br>NNNNNNNNNNNNNNNNNN-3' |
| 12 | 3'-<br>NNNNNNNNNNNNNNNNNNNNNNNGGNNNNNNNNNNNNCCNNNNN<br>NNNNNNNNNNNNNNNNNN-5' |
| 13<br>14 | 5'-<br><br>NNNNNNNNNNNNNNNNNNNNNNNNCCNNNNNNNNNNNNNGGNNNNN<br>NNNNNNNNNNNNNNNNNN-3' |

(continued)

| Overhang length (bp) | Guide RNA design (guide sequence and PAM color coded) |
|---|---|
| 15 | 3'- NNNNNNNNNNNNNNNNNNNGGNNNNNNNNNNNNNNCCNNNNNNN NNNNNNNNNNNNNNNNNNN-5' |
| 16 | 5'- NNNNNNNNNNNNNNNNNNNNCCNNNNNNNNNNNGGNNNNNNNN NNNNNNNNNNNNNNNNNNN-3' |
| 17 | 3'- NNNNNNNNNNNNNNNNNNNNGGNNNNNNNNNNNCCNNNNNNNN NNNNNNNNNNNNNNNNNNN-5' |
| | 5'- NNNNNNNNNNNNNNNNNNNNNCCNNNNNNNNNNGGNNNNNNNNN NNNNNNNNNNNNNNNNNNN-3' |
| | 3'- NNNNNNNNNNNNNNNNNNNNNGGNNNNNNNNNNCCNNNNNNNNN NNNNNNNNNNNNNNNNNNN-5' |
| | 5'- NNNNNNNNNNNNNNNNNNNNNNCCNNNNNNNNNGGNNNNNNNNNN NNNNNNNNNNNNNNNNNNN-3' |
| | 3'- NNNNNNNNNNNNNNNNNNNNNNGGNNNNNNNNNCCNNNNNNNNNN NNNNNNNNNNNNNNNNNNN-5' |
| | 5'- NNNNNNNNNNNNNNNNNNNNNNNCCNNNNNNNNGGNNNNNNNNNNN NNNNNNNNNNNNNNNNNNN-3' |
| | 3'- NNNNNNNNNNNNNNNNNNNNNNNGGNNNNNNCCNNNNNNNNNNNN NNNNNNNNNNNNNNNNNNN-5' |
| | 5'- NNNNNNNNNNNNNNNNNNNNNNNNCCNNNNNNNGGNNNNNNNNNNN NNNNNNNNNNNNNNNNNNN-3' |
| | 3'- NNNNNNNNNNNNNNNNNNNNNNNNGGNNNNNNCCNNNNNNNNNNNN NNNNNNNNNNNNNNNNNNN-5' |

(continued)

| Overhang length (bp) | Guide RNA design (guide sequence and PAM color coded) |
|---|---|
| | 5'- NNNNNNNNNNNNNNNNNNNNNNCCNNNNNGGNNNNNNNNNNNNNN NNNNNNNNNNNNNNNNNNN-3' <br> 3'- NNNNNNNNNNNNNNNNNNNNNGGNNNNNCCNNNNNNNNNNNNNN NNNNNNNNNNNNNNNNNNNN-5' <br> 5'- NNNNNNNNNNNNNNNNNNNNNNCCNNNNGGNNNNNNNNNNNNNN NNNNNNNNNNNNNNNNNNN-3' <br> 3'- NNNNNNNNNNNNNNNNNNNNNNNGGNNNNCCNNNNNNNNNNNNNN NNNNNNNNNNNNNNNNNNNN-5' <br> 5'- NNNNNNNNNNNNNNNNNNNNNNNNCCNNNGGNNNNNNNNNNNNNN NNNNNNNNNNNNNNNNNNN-3' <br> 3'- NNNNNNNNNNNNNNNNNNNNNNNNGGNNNCCNNNNNNNNNNNNNNNN <br><br> NNNNNNNNNNNNNNNNNNNN-5' |
| | 5'- NNNNNNNNNNNNNNNNNNNNNNNNCCNNGGNNNNNNNNNNNNNN NNNNNNNNNNNNNNNNNNN-3' <br> 3'- NNNNNNNNNNNNNNNNNNNNNNNGGNCCNNNNNNNNNNNNNNNNN NNNNNNNNNNNNNNNNNNNN-5' <br> 5'- NNNNNNNNNNNNNNNNNNNNNNNNCCNGGNNNNNNNNNNNNNN NNNNNNNNNNNNNNNNNNN-3' <br> 3'- NNNNNNNNNNNNNNNNNNNNNNNGGNCCNNNNNNNNNNNNNNNNN NNNNNNNNNNNNNNNNNNNN-5' |

(continued)

| Overhang length (bp) | Guide RNA design (guide sequence and PAM color coded) |
| --- | --- |
| | 5'-<br>NN<u>NNNNNNNNNNNNNNNNNNN</u>C<u>CGG</u>NNNNNNNNNNNNNNNN<br>NNNNNNNNNNNNNNNNNNN-3'<br>3'-<br>NNNNNNNNNNNNNNNNNNNN<u>GGC</u>C<u>NNNNNNNNNNNNNNNNNNN</u><br><u>NNN</u>NNNNNNNNNNNNNNNNN-5' |
| | 5'-<br>NN<u>NNNNNNNNNNNNNNNNNNNN</u><u>NGG</u>NNNNNNNNNNNNNNNN<br>NNNNNNNNNNNNNNNNNNN-3'<br>3'-<br>NNNNNNNNNNNNNNNNNNNNNCC<u>GG</u>N<u>NNNNNNNNNNNNNNN</u><br><u>NNNNNNN</u>NNNNNNNNNNNNN-5' |
| | 5'-<br>NN<u>NNNNNNNNNNNNNNNNNNNN</u><u>CGG</u>NNNNNNNNNNNNNNNN<br>NNNNNNNNNNNNNNNNNNN-3'<br>3'-<br>NNNNNNNNNNNNNNNNNNNNNCCN<u>GG</u>N<u>NNNNNNNNNNNNNN</u><br><u>NNNNNNNN</u>NNNNNNNNNNNN-5' |
| | 5'-<br>NN<u>NNNNNNNNNNNNNNNNNNNNN</u><u>CGG</u>NNNNNNNNNNNNNNNN<br>NNNNNNNNNNNNNNNNNNN-3'<br>3'-<br>NNNNNNNNNNNNNNNNNNNNNCCNN<u>GG</u>N<u>NNNNNNNNNNNNN</u><br><u>NNNNNNNNN</u>NNNNNNNNNNN-5' |
| | 5'-<br>NN<u>NNNNNNNNNNNNNNNNNNNNNN</u><u>CGG</u>NNNNNNNNNNNNNNNN<br>NNNNNNNNNNNNNNNNNNN-3'<br>3'-<br>NNNNNNNNNNNNNNNNNNNNNCCNNN<u>GG</u>N<u>NNNNNNNNNNNN</u><br><u>NNNNNNNNNN</u>NNNNNNNNNN-5' |
| | 5'-<br>NN<u>NNNNNNNNNNNNNNNNNNNNNNN</u><u>CGG</u>NNNNNNNNNNNNNNNN<br>NNNNNNNNNNNNNNNNNNN-3' |

(continued)

| Overhang length (bp) | Guide RNA design (guide sequence and PAM color coded) |
|---|---|
| | 3'-<br><br>NNNNNNNNNNNNNNNNNNNNCCNNNNGGNNNNNNNNNN<br><br>NNNNNNNNNNNNNNNNNNNN-5'<br><br>5'-<br><br>NNNNNNNNNNNNNNNNNNNNNNNNCGGNNNNNNNNNNNNNNNN<br><br>NNNNNNNNNNNNNNNNNNNNNNNN-3'<br><br>3'-<br>NNNNNNNNNNNNNNNNNNNNNNNNNCCNNNNNGGNNNNNNNNN<br><br>NNNNNNNNNNNNNNNNNNNNN-5' |

Further interrogation of the system have given Applicants evidence of the 5' overhang (see, e.g., Ran et al., Cell. 2013 Sep 12;154(6):1380-9

Applicants have further identified parameters that relate to efficient cleavage by the Cas9 nickase mutant when combined with two guide RNAs and these parameters include but are not limited to the length of the 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs or 1-34 base pairs. In other preferred methods of the invention the first guide sequence directing cleavage of one strand of the DNA duplex near the first target sequence and the second guide sequence directing cleavage of other strand near the second target sequence results in a blunt cut or a 3' overhang. In embodiments of the invention the 3' overhang is at most 150, 100 or 25 base pairs or at least 15, 10 or 1 base pairs. In preferred embodiments the 3' overhang is 1-100 basepairs.

Aspects of the dicslosure

relate to the expression of the gene product being decreased

or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein.

Only sgRNA pairs creating 5' overhangs with less than 8bp overlap between the guide sequences (offset greater than -8 bp) were able to mediate detectable indel formation. Importantly, each guide used in these assays is able to efficiently induce indels when paired with wildtype Cas9, indicating that the relative positions of the guide pairs are the most important parameters in predicting double nicking activity.

Since Cas9n and Cas9H840A nick opposite strands of DNA, substitution of Cas9n with Cas9H840A with a given sgRNA pair should result in the inversion of the overhang type. For example, a pair of sgRNAs that will generate a 5' overhang with Cas9n should in principle generate the corresponding 3' overhang instead. Therefore, sgRNA pairs that lead to the generation of a 3' overhang with Cas9n might be used with Cas9H840A to generate a 5' overhang. Unexpectedly, Applicants tested Cas9H840A with a set of sgRNA pairs designed to generate both 5' and 3' overhangs (offset range from -278 to +58 bp), but were unable to observe indel formation. Further work may be needed to identify the necessary design rules for sgRNA pairing to allow double nicking by Cas9H840A.

**Liver, proprotein convertase subtilisin kexin 9 (PCSK9)**

The data shows phenotypic conversion.

Proprotein convertase subtilisin kexin 9 (PCSK9) is a member of the subtilisin serine protease family. PCSK9 is primarily expressed by the liver and is critical for the down regulation of hepatocyte LDL receptor expression. LDL-C levels in plasma are highly elevated in humans with gain of function mutations in PCSK9, classifying them as having severe hypercholesterolemia. Therefore, PCSK9 is an attractive target for CRISPR. PCS9K-targeted CRISPR may be formulated in a lipid particle and for example administered at about 15, 45, 90, 150, 250 and 400 $\mu$g/kg intraveneously (see, e.g., http://www.alnylam.com/capella/wp-content/uploads/2013/08/ALN-PCS02-001-Protocol-Lancet.pdf).

Bailey et al. (J Mol Med (Berl). 1999 Jan;77(1):244-9) discloses insulin delivery by ex-vivo somatic cell gene therapy involves the removal of non-B-cell somatic cells (e.g. fibroblasts) from a diabetic patient, and genetically altering them in vitro to produce and secrete insulin. The cells can be grown in culture and returned to the donor as a source of insulin replacement. Cells modified in this way could be evaluated before implantation, and reserve stocks could be cryopreserved. By using the patient's own cells, the procedure should obviate the need for immunosuppression and overcome the problem of tissue supply, while avoiding a recurrence of cell destruction. Ex-vivo somatic cell gene therapy requires an accessible and robust cell type that is amenable to multiple transfections and subject to controlled proliferation. Special problems associated with the use of non-B-cell somatic cells include the processing of proinsulin to insulin, and the conferment of sensitivity to glucose-stimulated proinsulin biosynthesis and regulated insulin release. Preliminary studies using fibroblasts, pituitary cells, kidney (COS) cells and ovarian (CHO) cells suggest that these challenges could be met, and that ex-vivo somatic cell gene therapy offers a feasible approach to insulin replacement therapy. The system of Bailey et al. may be used/and or adapted to the CRISPR Cas system of the present invention for delivery to the liver.

The methods of Sato et al. (Nature Biotechnology Volume 26 Number 4 April 2008, Pages 431-442) may be applied to the CRISPR Cas system of the present invention for delivery to the liver. Sato et al. found that treatments with the siRNA-bearing vitamin A-coupled liposomes almost completely resolved liver fibrosis and prolonged survival in rats with otherwise lethal dimethylnitrosamine-induced liver cirrhosis in a dose- and duration-dependent manner. Cationic liposomes (Lipotrust) containing O,O'-ditetradecanoyl-N-(a-trimethylammonioacetyl) diethanolamine chloride (DC-6-14) as a cationic lipid, cholesterol and dioleoylphosphatidylethanolamine at a molar ratio of 4:3:3 (which has shown high transfection efficiency under serumcontaining conditions for in vitro and in vivo gene delivery) were purchased from Hokkaido System Science. The liposomes were manufactured using a freeze-dried empty liposomes method and prepared at a concentration of 1 mM (DC-16-4) by addition of double-distilled water (DDW) to the lyophilized lipid mixture under vortexing before use. To prepare VA-coupled liposomes, 200 nmol of vitamin A (retinol, Sigma) dissolved in DMSO was mixed with the liposome suspensions (100 nmol as DC-16-4) by vortexing in a 1.5 ml tube at 25 1C. To prepare VA-coupled liposomes carrying siRNAgp46 (VA-lip-siRNAgp46), a solution of siRNAgp46 (580 pmol/ml in DDW) was added to the retinol-coupled liposome solution with stirring at 25 C. The ratio of siRNA to DC-16-4 was 1:11.5 (mol/mol) and the siRNA to liposome ratio (wt/wt) was 1:1. Any free vitamin A or siRNA that was not taken up by liposomes were separated from liposomal preparations using a micropartition system (VIVASPIN 2 concentrator 30,000 MWCO PES, VIVASCIENCE). The liposomal suspension was added to the filters and centrifuged at 1,500g for 5 min 3 times at 25 1C. Fractions were collected and the material trapped in the filter was reconstituted with PBS to achieve the desired dose for in vitro or in vivo use. Three injections of 0.75 mg/kg siRNA were given every other day to rats. The system of Sato et al. may be used/and or adapted to the CRISPR Cas system for delivery to the liver by delivering about 0.5 to 1 mg/kg of CRISPR Cas RNA in the liposomes as described by Sato et al. to humans.

The methods of Rozema et al. (PNAS, August 7, 2007, vol. 104, no. 32) for a vehicle for the delivery of siRNA to hepatocytes both in vitro and in vivo, which Rozema et al. have named siRNA Dynamic PolyConjugates may also be applied to the present invention. Key features of the Dynamic Poly-Conjugate technology include a membrane-active polymer, the ability to reversibly mask the activity of this polymer until it reaches the acidic environment of endosomes, and the ability to target this modified polymer and its siRNA cargo specifically to hepatocytes in vivo after simple, low-pressure i.v. injection. SATA-modified siRNAs are synthesized by reaction of 5' aminemodified siRNA with 1 weight equivalents (wt eq) of Nsuccinimidyl-S-acetylthioacetate (SATA) reagent (Pierce) and 0.36 wt eq of $NaHCO_3$ in water at 4°C for 16 h. The modified siRNAs are then precipitated by the addition of 9 vol of ethanol and incubation at 80°C for 2 h. The precipitate is resuspended in 1X siRNA buffer (Dharmacon) and quantified by measuring absorbance at the 260-nm wavelength. PBAVE (30 mg/ml in 5mMTAPS, pH 9) is modified by addition of 1.5 wt % SMPT (Pierce). After a 1-h incubation, 0.8 mg of SMPT-PBAVE was added to 400 $\mu$l of isotonic glucose solution containing 5 mM TAPS (pH 9). To this solution was added 50 $\mu$g of SATA-modified siRNA. For the dose-response experiments where [PBAVE] was constant, different amounts of siRNA are added. The mixture is then incubated for 16 h. To the solution is then added 5.6 mg of Hepes free base followed by a mixture of 3.7 mg ofCDM-NAGand 1.9mg of CDM-PEG. The solution is then incubated for at least 1 h at room temperature before injection. CDM-PEG and CDM-NAG are synthesized from the acid chloride generated by using oxalyl chloride. To the acid chloride is added 1.1 molar equivalents polyethylene glycol monomethyl ether (molecular weight average of 450) to generate CDM-PEG or (aminoethoxy)ethoxy-2-(acetylamino)-2-deoxy-$\beta$-D-glucopyranoside to generate CDM-NAG. The final product is purified by using reverse-phase HPLC with a 0.1% TFA water/acetonitrile gradient. About 25 to 50 $\mu$g of siRNA was delivered to mice. The system of Rozema et al. may be applied to the CRISPR Cas system for delivery to the liver, for example by envisioning a dosage of about 50 to about 200 mg of CRISPR Cas for delivery to a human.

## Bone

Oakes and Lieberman (Clin Orthop Relat Res. 2000 Oct;(379 Suppl):S101-12) discusses delivery of genes to the bone. By transferring genes into cells at a specific anatomic site, the osteoinductive properties of growth factors can be used at physiologic doses for a sustained period to facilitate a more significant healing response. The specific anatomic site, the quality of the bone, and the soft-tissue envelope, influences the selection of the target cells for regional gene therapy. Gene therapy vectors delivered to a treatment site in osteoconductive carriers have yielded promising results. Several investigators have shown exciting results using ex vivo and in vivo regional gene therapy in animal models. Such a system may be used/and or adapted to the CRISPR Cas system for delivery to the bone.

## Brain

Delivery options for the brain include encapsulation of CRISPR enzyme and guide RNA in the form of either DNA or RNA into liposomes and conjugating to molecular Trojan horses for trans-blood brain barrier (BBB) delivery. Molecular Trojan horses have been shown to be effective for delivery of B-gal expression vectors into the brain of non-human primates. The same approach can be used to delivery vectors containing CRISPR enzyme and guide RNA. For instance, Xia CF and Boado RJ, Pardridge WM ("Antibody-mediated targeting of siRNA via the human insulin receptor using avidin-biotin technology." Mol Pharm. 2009 May-Jun;6(3):747-51. doi: 10.1021/mp800194) describes how delivery of short interfering RNA (siRNA) to cells in culture, and in vivo, is possible with combined use of a receptor-specific monoclonal antibody (mAb) and avidin-biotin technology. The authors also report that because the bond between the targeting mAb and the siRNA is stable with avidin-biotin technology, and RNAi effects at distant sites such as brain are observed in vivo following an intravenous administration of the targeted siRNA.

Zhang et al. (Mol Ther. 2003 Jan;7(1):11-8.)) describe how expression plasmids encoding reporters such as luciferase were encapsulated in the interior of an "artificial virus" comprised of an 85 nm pegylated immunoliposome, which was targeted to the rhesus monkey brain in vivo with a monoclonal antibody (MAb) to the human insulin receptor (HIR). The HIRMAb enables the liposome carrying the exogenous gene to undergo transcytosis across the blood-brain barrier and endocytosis across the neuronal plasma membrane following intravenous injection. The level of luciferase gene expression in the brain was 50-fold higher in the rhesus monkey as compared to the rat. Widespread neuronal expression of the beta-galactosidase gene in primate brain was demonstrated by both histochemistry and confocal microscopy. The authors indicate that this approach makes feasible reversible adult transgenics in 24 hours. Accordingly, the use of immunoliposome is preferred. These may be used in conjunction with antibodies to target specific tissues or cell surface proteins.

Other means of delivery or RNA are also preferred, such as via nanoparticles (Cho, S., Goldberg, M., Son, S., Xu, Q., Yang, F., Mei, Y., Bogatyrev, S., Langer, R. and Anderson, D., Lipid-like nanoparticles for small interfering RNA delivery to endothelial cells, Advanced Functional Materials, 19: 3112-3118, 2010) or exosomes (Schroeder, A., Levins, C., Cortez, C., Langer, R., and Anderson, D., Lipid-based nanotherapeutics for siRNA delivery, Journal of Internal Medicine, 267: 9-21, 2010, PMID: 20059641).

Indeed, exosomes have been shown to be particularly useful in delivery siRNA, a system with some parallels to the CRISPR system. For instance, El-Andaloussi S, et al. ("Exosome-mediated delivery of siRNA in vitro and in vivo." Nat Protoc. 2012 Dec;7(12):2112-26. doi: 10.1038/nprot.2012.131. Epub 2012 Nov 15.) describe how exosomes are promising tools for drug delivery across different biological barriers and can be harnessed for delivery of siRNA in vitro and in vivo. Their approach is to generate targeted exosomes through transfection of an expression vector, comprising an exosomal protein fused with a peptide ligand. The exosomes are then purify and characterized from transfected cell supernatant, then siRNA is loaded into the exosomes. Delivery or administration can be performed with exosomes, in particular but not limited to the brain.

Vitamin E (α-tocopherol) may be conjugated with CRISPR Cas and delivered to the brain along with high density lipoprotein (HDL), for example in a similar manner as was done by Uno et al. (HUMAN GENE THERAPY 22:711-719 (June 2011)) for delivering short-interfering RNA (siRNA) to the brain. Mice were infused via Osmotic minipumps (model 1007D; Alzet, Cupertino, CA) filled with phosphate-buffered saline (PBS) or free TocsiBACE or TocsiBACE/HDL and connected with Brain Infusion Kit 3 (Alzet). A brain-infusion cannula was placed about 0.5mm posterior to the bregma at midline for infusion into the dorsal third ventricle. Uno et al. found that as little as 3 nmol of Toc-siRNA with HDL could induce a target reduction in comparable degree by the same ICV infusion method. A similar dosage of CRISPR Cas conjugated to α-tocopherol and co-administered with HDL targeted to the brain may be contemplated for humans in the present invention, for example, about 3 nmol to about 3 μmol of CRISPR Cas targeted to the brain may becontemplated.

Zou et al. ((HUMAN GENE THERAPY 22:465-475 (April 2011)) describes a method of lentiviral-mediated delivery of short-hairpin RNAs targeting PKCγ for in vivo gene silencing in the spinal cord of rats. Zou et al. administered about 10 μl of a recombinant lentivirus having a titer of 1 x 10$^9$ transducing units (TU)/ml by an intrathecal catheter. A similar dosage of CRISPR Cas expressed in a lentiviral vector targeted to the brain may be contemplated for humans in the present invention, for example, about 10-50 ml of CRISPR Cas targeted to the brain in a lentivirus having a titer of 1 x $10^9$ transducing units (TU)/ml may becontemplated.

**Targeted deletion, therapeutic applications**

Targeted deletion of genes is preferred. Examples are exemplified in Example 18. Preferred are, therefore, genes involved in cholesterol biosynthesis, fatty acid biosynthesis, and other metabolic disorders, genes encoding misfolded proteins involved in amyloid and other diseases, oncogenes leading to cellular transformation, latent viral genes, and genes leading to dominant-negative disorders, amongst other disorders. As exemplified here, Applicants prefer gene delivery of a CRISPR-Cas system to the liver, brain, ocular, epithelial, hematopoetic, or another tissue of a subject or a patient in need thereof, suffering from metabolic disorders, amyloidosis and protein-aggregation related diseases, cellular transformation arising from genetic mutations and translocations, dominant negative effects of gene mutations, latent viral infections, and other related symptoms, using either viral or nanoparticle delivery system.

Therapeutic applications of the CRISPR-Cas system include Glaucoma, Amyloidosis, and Huntington's disease. These are exemplified in Example 20 and the features described therein are preferred alone or in combination.

Another example of a polyglutamine expansion disease that may be treated by the present invention includes spinocerebellar ataxia type 1 (SCA1). Upon intracerebellar injection, recombinant adenoassociated virus (AAV) vectors expressing short hairpin RNAs profoundly improve motor coordination, restored cerebellar morphology and resolved characteristic ataxin-1 inclusions in Purkinje cells of SCA1 mice (see, e.g., Xia et al., Nature Medicine, Vol. 10, No. 8, Aug. 2004). In particular, AAV1 and AAV5 vectors are preferred and AAV titers of about 1 x $10^{12}$ vector genomes/ml are desirable.

As an example, chronic infection by HIV-1 may be treated or prevented. In order to accomplish this, one may generate CRISPR-Cas guide RNAs that target the vast majority of the HIV-1 genome while taking into account HIV-1 strain variants for maximal coverage and effectiveness. One may accomplish delivery of the CRISPR-Cas system by conventional adenoviral or lentiviral-mediated infection of the host immune system. Depending on approach, host immune cells could be a) isolated, transduced with CRISPR-Cas, selected, and re-introduced in to the host or b) transduced in vivo by systemic delivery of the CRISPR-Cas system. The first approach allows for generation of a resistant immune population whereas the second is more likely to target latent viral reservoirs within the host. This is discussed in more detail in the Examples section.

In another example, US Patent Publication No. 20130171732 assigned to Sangamo BioSciences, Inc. relates to insertion of an anti-HIV transgene into the genome, methods of which may be applied to the CRISPR Cas system. In another

embodiment, the CXCR4 gene may be targeted and the TALE system of US Patent Publication No. 20100291048 assigned to Sangamo BioSciences, Inc. may be modified to the CRISPR Cas system.. The method of US Patent Publication Nos. 20130137104 and

20130122591 assigned to Sangamo BioSciences, Inc. and US Patent Publication No. 20100146651 assigned to Cellectis may be more generally applicable for transgene expression as it involves modifying a hypoxanthine-guanine phosphoribosyltransferase (HPRT) locus for increasing the frequency of gene modification.

It is also envisaged that the present invention generates a gene knockout cell library. Each cell may have a single gene knocked out. This is exemplified in Example 23.

One may make a library of ES cells where each cell has a single gene knocked out, and the entire library of ES cells will have every single gene knocked out. This library is useful for the screening of gene function in cellular processes as well as diseases. To make this cell library, one may integrate Cas9 driven by an inducible promoter (e.g. doxycycline inducible promoter) into the ES cell. In addition, one may integrate a single guide RNA targeting a specific gene in the ES cell. To make the ES cell library, one may simply mix ES cells with a library of genes encoding guide RNAs targeting each gene in the human genome. One may first introduce a single BxB1 attB site into the AAVS1 locus of the human ES cell. Then one may use the BxB1 integrase to facilitate the integration of individual guide RNA genes into the BxB1 attB site in

AAVS1 locus. To facilitate integration, each guide RNA gene may be contained on a plasmid that carries of a single attP site. This way BxB1 will recombine the attB site in the genome with the attP site on the guide RNA containing plasmid. To generate the cell library, one may take the library of cells that have single guide RNAs integrated and induce Cas9 expression. After induction, Cas9 mediates double strand break at sites specified by the guide RNA.

Chronic administration of protein therapeutics may elicit unacceptable immune responses to the specific protein. The immunogenicity of protein drugs can be ascribed to a few immunodominant helper T lymphocyte (HTL) epitopes. Reducing the MHC binding affinity of these HTL epitopes contained within these proteins can generate drugs with lower immunogenicity (Tangri S, et al. ("Rationally engineered therapeutic proteins with reduced immunogenicity" J Immunol. 2005 Mar 15;174(6):3187-96.) In the present invention, the immunogenicity of the CRISPR enzyme in particular may be reduced following the approach first set out in Tangri et al with respect to erythropoietin and subsequently developed.

Accordingly, directed evolution or rational design may be used to reduce the immunogenicity of the CRISPR enzyme (for instance a Cas9) in the host species (human or other species).

In Example 28, Applicants used 3 guideRNAs of interest and able to visualize efficient DNA cleavage *in vivo* occurring only in a small subset of cells. Essentially, what Applicants have shown here is targeted *in vivo* cleavage. In particular, this provides proof of concept that specific targeting in higher organisms such as mammals can also be achieved. It also highlights multiplex aspect in that multiple guide sequences (i.e. separate targets) can be used simultaneously (in the sense of co-delivery). In other words, Applicants used a multiple approach, with several different sequences targeted at the same time, but independently.

A suitable example of a protocol for producing AAV, a preferred vector is provided in Example 34.

Trinucleotide repeat disorders are preferred conditions to be treated. These are also exemplified herein.

For example, US Patent Publication No. 20110016540, describes use of zinc finger nucleases to genetically modify cells, animals and proteins associated with trinucleotide repeat expansion disorders. Trinucleotide repeat expansion disorders are complex, progressive disorders that involve developmental neurobiology and often affect cognition as well as sensori-motor functions.

Trinucleotide repeat expansion proteins are a diverse set of proteins associated with susceptibility for developing a trinucleotide repeat expansion disorder, the presence of a trinucleotide repeat expansion disorder, the severity of a trinucleotide repeat expansion disorder or any combination thereof. Trinucleotide repeat expansion disorders are divided into two categories determined by the type of repeat. The most common repeat is the triplet CAG, which, when present in the coding region of a gene, codes for the amino acid glutamine (Q). Therefore, these disorders are referred to as the polyglutamine (polyQ) disorders and comprise the following diseases: Huntington Disease (HD); Spinobulbar Muscular Atrophy (SBMA); Spinocerebellar Ataxias (SCA types 1, 2, 3, 6, 7, and 17); and Dentatorubro-Pallidoluysian Atrophy (DRPLA). The remaining trinucleotide repeat expansion disorders either do not involve the CAG triplet or the CAG triplet is not in the coding region of the gene and are, therefore, referred to as the non-polyglutamine disorders. The non-polyglutamine disorders comprise Fragile X Syndrome (FRAXA); Fragile XE Mental Retardation (FRAXE); Friedreich Ataxia (FRDA); Myotonic Dystrophy (DM); and Spinocerebellar Ataxias (SCA types 8, and 12).

The proteins associated with trinucleotide repeat expansion disorders are typically selected based on an experimental association of the protein associated with a trinucleotide repeat expansion disorder to a trinucleotide repeat expansion disorder. For example, the production rate or circulating concentration of a protein associated with a trinucleotide repeat expansion disorder may be elevated or depressed in a population having a trinucleotide repeat expansion disorder relative to a population lacking the trinucleotide repeat expansion disorder. Differences in protein levels may be assessed using proteomic techniques including but not limited to Western blot, immunohistochemical staining, enzyme linked immunosorbent assay (ELISA), and mass spectrometry. Alternatively, the proteins associated with trinucleotide repeat expansion disorders may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

Non-limiting examples of proteins associated with trinucleotide repeat expansion disorders include AR (androgen receptor), FMR1 (fragile X mental retardation 1), HTT (huntingtin), DMPK (dystrophia myotonica-protein kinase), FXN (frataxin), ATXN2 (ataxin 2), ATN1 (atrophin 1), FEN1 (flap structure-specific endonuclease 1), TNRC6A (trinucleotide repeat containing 6A), PABPN1 (poly(A) binding protein, nuclear 1), JPH3 (junctophilin 3), MED15 (mediator complex subunit 15), ATXN1 (ataxin 1), ATXN3 (ataxin 3), TBP (TATA box binding protein), CACNA1A (calcium channel, voltage-dependent, P/Q type, alpha 1A subunit), ATXN80S (ATXN8 opposite strand (non-protein coding)), PPP2R2B (protein phosphatase 2, regulatory subunit B, beta), ATXN7 (ataxin 7), TNRC6B (trinucleotide repeat containing 6B), TNRC6C (trinucleotide repeat containing 6C), CELF3 (CUGBP, Elav-like family member 3), MAB21L1 (mab-21-like 1 (C. elegans)), MSH2 (mutS homolog 2, colon cancer, nonpolyposis type 1 (E. coli)), TMEM185A (transmembrane protein 185A), SIX5 (SIX homeobox 5), CNPY3 (canopy 3 homolog (zebrafish)), FRAXE (fragile site, folic acid type, rare, fra(X)(q28) E), GNB2 (guanine nucleotide binding protein (G protein), beta polypeptide 2), RPL14 (ribosomal protein L14), ATXN8 (ataxin 8), INSR (insulin receptor), TTR (transthyretin), EP400 (E1A binding protein p400), GIGYF2 (GRB10 interacting GYF protein 2), OGG1 (8-oxoguanine DNA glycosylase), STC1 (stanniocalcin 1), CNDP1 (carnosine dipeptidase 1 (metallopeptidase M20 family)), C10orf2 (chromosome 10 open reading frame 2), MAML3 mastermind-like 3 (Drosophila), DKC1 (dyskeratosis congenita 1, dyskerin), PAXIP1 (PAX interacting (with transcription-activation domain) protein 1), CASK (calcium/calmodulin-dependent serine protein kinase (MAGUK family)), MAPT (microtubule-associated protein tau), SP1 (Sp1 transcription factor), POLG (polymerase (DNA directed), gamma), AFF2 (AF4/FMR2 family, member 2), THBS1 (thrombospondin 1), TP53 (tumor protein p53), ESR1 (estrogen receptor 1), CGGBP1 (CGG triplet repeat binding protein 1), ABT1 (activator of basal transcription 1), KLK3 (kallikrein-related peptidase 3), PRNP (prion protein), JUN (jun oncogene), KCNN3 (potassium intermediate/small conductance calcium-activated channel, subfamily N, member 3), BAX (BCL2-associated X protein), FRAXA (fragile site, folic acid type, rare, fra(X)(q27.3) A (macroorchidism, mental retardation)), KBTBD10 (kelch repeat and BTB (POZ) domain containing 10), MBNL1 (muscleblind-like (Drosophila)), RAD51 (RAD51 homolog (RecA homolog, E. coli) (S. cerevisiae)), NCOA3 (nuclear receptor coactivator 3), ERDA1 (expanded repeat domain, CAG/CTG 1), TSC1 (tuberous sclerosis 1), COMP (cartilage oligomeric matrix protein), GCLC (glutamate-cysteine ligase, catalytic subunit), RRAD (Ras-related associated with diabetes), MSH3 (mutS homolog 3 (E. coli)), DRD2 (dopamine receptor D2), CD44 (CD44 molecule (Indian blood group)), CTCF (CCCTC-binding factor (zinc finger protein)), CCND1 (cyclin D1), CLSPN (claspin homolog (Xenopus laevis)), MEF2A (myocyte enhancer factor 2A), PTPRU (protein tyrosine phosphatase, receptor type, U), GAPDH (glyceraldehyde-3-phosphate dehydrogenase), TRIM22 (tripartite motif-containing 22), WT1 (Wilms tumor 1), AHR (aryl hydrocarbon receptor), GPX1 (glutathione peroxidase 1), TPMT (thiopurine S-methyltransferase), NDP (Norrie disease (pseudoglioma)), ARX (aristaless related homeobox), MUS81 (MUS81 endonuclease homolog (S. cerevisiae)), TYR (tyrosinase (oculocutaneous albinism IA)), EGR1 (early growth response 1), UNG (uracil-DNA glycosylase), NUMBL (numb homolog (Drosophila)-like), FABP2 (fatty acid binding protein 2, intestinal), EN2 (engrailed homeobox 2), CRYGC (crystallin, gamma C), SRP14 (signal recognition particle 14 kDa (homologous Alu RNA binding protein)), CRYGB (crystallin, gamma B), PDCD1 (programmed cell death 1), HOXA1 (homeobox A1), ATXN2L (ataxin 2-like), PMS2 (PMS2 postmeiotic segregation increased 2 (S. cerevisiae)), GLA (galactosidase, alpha), CBL (Cas-Br-M (murine) ecotropic retroviral transforming sequence), FTH1 (ferritin, heavy polypeptide 1), IL12RB2 (interleukin 12 receptor, beta 2), OTX2 (orthodenticle homeobox 2), HOXA5 (homeobox A5), POLG2 (polymerase (DNA directed), gamma 2, accessory subunit), DLX2 (distal-less homeobox 2), SIRPA (signal-regulatory protein alpha), OTX1 (orthodenticle homeobox 1), AHRR (arylhydrocarbon receptor repressor), MANF (mesencephalic astrocyte-derived neurotrophic factor), TMEM158 (transmembrane protein 158 (gene/pseudogene)), and ENSG00000078687.

Preferred proteins associated with trinucleotide repeat expansion disorders include HTT (Huntingtin), AR (androgen receptor), FXN (frataxin), Atxn3 (ataxin), Atxn1 (ataxin), Atxn2 (ataxin), Atxn7 (ataxin), Atxn10 (ataxin), DMPK (dystrophia myotonica-protein kinase), Atn1 (atrophin 1), CBP (creb binding protein), VLDLR (very low density lipoprotein receptor), and any combination thereof.

According to another aspect, a method of gene therapy for the treatment of a subject having a mutation in the CFTR gene is provided and comprises administering a therapeutically effective amount of a CRISPR-Cas gene therapy particle, optionally via a biocompatible pharmaceutical carrier, to the cells of a subject. Preferably, the target DNA comprises the mutation deltaF508. In general, it is of preferred that the mutation is repaired to the wildtype. In this case, the mutation is a deletion of the three nucleotides that comprise the codon for phenylalanine (F) at position 508. Accordingly, repair in this instance requires reintroduction of the missing codon into the mutant.

To implement this Gene Repair Strategy, it is preferred that an adenovirus/AAV vector system is introduced into the host cell, cells or patient. Preferably, the system comprises a Cas9 (or Cas9 nickase) and the guide RNA along with a adenovirus/AAV vector system comprising the homology repair template containing the F508 residue. This may be introduced into the subject via one of the methods of delivery discussed earlier. The CRISPR-Cas system may be guided by the CFTRdelta 508 chimeric guide RNA. It targets a specific site of the CFTR genomic locus to be nicked or cleaved. After cleavage, the repair template is inserted into the cleavage site via homologous recombination correcting the deletion that results in cystic fibrosis or causes cystic fibrosis related symptoms. This strategy to direct delivery and provide systemic introduction of CRISPR systems with appropriate guide RNAs can be employed to target genetic mutations to edit or otherwise manipulate genes that cause metabolic, liver, kidney and protein diseases and disorders such as those in Table B.

## Genome editing

The CRISPR/Cas9 systems of the present invention can be used to correct genetic mutations that were previously attempted with limited success using TALEN and ZFN. For example, WO2013163628 A2, Genetic Correction of Mutated Genes, published application of Duke University describes efforts to correct, for example, a frameshift mutation which causes a premature stop codon and a truncated gene product that can be corrected via nuclease mediated non-homologous end joining such as those responsible for Duchenne Muscular Dystrophy, ("DMD") a recessive, fatal, X-linked disorder that results in muscle degeneration due to mutations in the dystrophin gene. The majority of dystrophin mutations that cause DMD are deletions of exons that disrupt the reading frame and cause premature translation termination in the dystrophin gene. Dystrophin is a cytoplasmic protein that provides structural stability to the dystroglycan complex of the cell membrane that is responsible for regulating muscle cell integrity and function. The dystrophin gene or "DMD gene" as used interchangeably herein is 2.2 megabases at locus Xp21. The primary transcription measures about 2,400 kb with the mature mRNA being about 14 kb. 79 exons code for the protein which is over 3500 amino acids. Exon 51 is frequently adjacent to frame-disrupting deletions in DMD patients and has been targeted in clinical trials for oligonucleotide-based exon skipping. A clinical trial for the exon 51 skipping compound eteplirsen recently reported a significant functional benefit across 48 weeks, with an average of 47% dystrophin positive fibers compared to baseline. Mutations in exon 51 are ideally suited for permanent correction by NHEJ-based genome editing.

The methods of US Patent Publication No. 20130145487 assigned to Cellectis, which relates to meganuclease variants to cleave a target sequence from the human dystrophin gene (DMD), may also be modified to for the CRISPR Cas system.

## Blood

The present disclosure also contemplates delivering the CRISPR-Cas system to the blood.

The plasma exosomes of Wahlgren et al. (Nucleic Acids Research, 2012, Vol. 40, No. 17 e130) were previously described and may be utilized to deliver the CRISPR Cas system to the blood.

The CRISPR Cas system is also contemplated to treat hemoglobinopathies, such as thalassemias and sickle cell disease. See, e.g., International Patent Publication No. WO 2013/126794 for potential targets that may be targeted by the CRISPR Cas system.

US Patent Publication Nos. 20110225664, 20110091441, 20100229252, 20090271881 and 20090222937 assigned to Cellectis, relates to CREI variants , wherein at least one of the two I-CreI monomers has at least two substitutions, one in each of the two functional subdomains of the LAGLIDADG core domain situated respectively from positions 26 to 40 and 44 to 77 of I-CreI, said variant being able to cleave a DNA target sequence from the human interleukin-2 receptor gamma chain (IL2RG) gene also named common cytokine receptor gamma chain gene or gamma C gene. The target sequences identified in US Patent Publication Nos. 20110225664, 20110091441, 20100229252, 20090271881 and 20090222937 may be utilized for the CRISPR Cas system.

Severe Combined Immune Deficiency (SCID) results from a defect in lymphocytes T maturation, always associated with a functional defect in lymphocytes B (Cavazzana-Calvo et al., Annu. Rev. Med., 2005, 56, 585-602; Fischer et al., Immunol. Rev., 2005, 203, 98-109). Overall incidence is estimated to 1 in 75 000 births. Patients with untreated SCID are subject to multiple opportunist micro-organism infections, and do generally not live beyond one year. SCID can be treated by allogenic hematopoietic stem cell transfer, from a familial donor. Histocompatibility with the donor can vary widely. In the case of Adenosine Deaminase (ADA) deficiency, one of the SCID forms, patients can be treated by injection of recombinant Adenosine Deaminase enzyme.

Since the ADA gene has been shown to be mutated in SCID patients (Giblett et al., Lancet, 1972, 2, 1067-1069), several other genes involved in SCID have been identified (Cavazzana-Calvo et al., Annu. Rev. Med., 2005, 56, 585-602; Fischer et al., Immunol. Rev., 2005, 203, 98-109). There are four major causes for SCID: (i) the most frequent form of SCID, SCID-X1 (X-linked SCID or X-SCID), is caused by mutation in the IL2RG gene, resulting in the absence of mature T lymphocytes and NK cells. IL2RG encodes the gamma C protein (Noguchi, et al., Cell, 1993, 73, 147-157), a common component of at least five interleukin receptor complexes. These receptors activate several targets through the JAK3 kinase (Macchi et al., Nature, 1995, 377, 65-68), which inactivation results in the same syndrome as gamma C inactivation; (ii) mutation in the ADA gene results in a defect in purine metabolism that is lethal for lymphocyte precursors, which in turn results in the quasi absence of B, T and NK cells; (iii) V(D)J recombination is an essential step in the maturation of immunoglobulins and T lymphocytes receptors (TCRs). Mutations in Recombination Activating Gene 1 and 2 (RAG1 and RAG2) and Artemis, three genes involved in this process, result in the absence of mature T and B lymphocytes; and (iv) Mutations in other genes such as CD45, involved in T cell specific signaling have also been reported, although they represent a minority of cases (Cavazzana-Calvo et al., Annu. Rev. Med., 2005, 56, 585-602; Fischer et al., Immunol. Rev., 2005, 203, 98-109).

Since when their genetic bases have been identified, the different SCID forms have become a paradigm for gene therapy approaches (Fischer et al., Immunol. Rev., 2005, 203, 98-109) for two major reasons. First, as in all blood diseases, an ex vivo treatment can be envisioned. Hematopoietic Stem Cells (HSCs) can be recovered from bone marrow, and keep their pluripotent properties for a few cell divisions. Therefore, they can be treated in vitro, and then reinjected into the patient, where they repopulate the bone marrow. Second, since the maturation of lymphocytes is impaired in SCID patients, corrected cells have a selective advantage. Therefore, a small number of corrected cells can restore a functional immune system. This hypothesis was validated several times by (i) the partial restoration of immune functions associated with the reversion of mutations in SCID patients (Hirschhorn et al., Nat. Genet., 1996, 13, 290-295; Stephan et al., N. Engl. J. Med., 1996, 335, 1563-1567; Bousso et al., Proc. Natl., Acad. Sci. USA, 2000, 97, 274-278; Wada et al., Proc. Natl. Acad. Sci. USA, 2001, 98, 8697-8702; Nishikomori et al., Blood, 2004, 103, 4565-4572), (ii) the correction of SCID-X1 deficiencies in vitro in hematopoietic cells (Candotti et al., Blood, 1996, 87, 3097-3102; Cavazzana-Calvo et al., Blood, 1996, Blood, 88, 3901-3909; Taylor et al., Blood, 1996, 87, 3103-3107; Hacein-Bey et al., Blood, 1998, 92, 4090-4097), (iii) the correction of SCID-X1 (Soudais et al., Blood, 2000, 95, 3071-3077; Tsai et al., Blood, 2002, 100, 72-79), JAK-3 (Bunting et al., Nat. Med., 1998, 4, 58-64; Bunting et al., Hum. Gene Ther., 2000, 11, 2353-2364) and RAG2 (Yates et al., Blood, 2002, 100, 3942-3949) deficiencies in vivo in animal models and (iv) by the result of gene therapy clinical trials (Cavazzana-Calvo et al., Science, 2000, 288, 669-672; Aiuti et al., Nat. Med., 2002; 8, 423-425; Gaspar et al., Lancet, 2004, 364, 2181-2187).

US Patent Publication No. 20110182867 assigned to the Children's Medical Center Corporation and the President and Fellows of Harvard College relates to methods and uses of modulating fetal hemoglobin expression (HbF) in a hematopoietic progenitor cells via inhibitors of BCL11A expression or activity, such as RNAi and antibodies. The targets disclosed in US Patent Publication No. 20110182867, such as BCL11A, may be targeted by the CRISPR Cas system for modulating fetal hemoglobin expression. See also Bauer et al.(Science 11 October 2013: Vol. 342 no. 6155 pp. 253-257) and Xu et al. (Science 18 November 2011: Vol. 334 no. 6058 pp. 993-996) for additional BCL11A targets.

## Ears

The present disclosure also contemplates delivering the CRISPR-Cas system to one or both ears.

Researchers are looking into whether gene therapy could be used to aid current deafness treatments-namely, cochlear implants. Deafness is often caused by lost or damaged hair cells that cannot relay signals to auditory neurons. In such cases, cochlear implants may be used to respond to sound and transmit electrical signals to the nerve cells. But these neurons often degenerate and retract from the cochlea as fewer growth factors are released by impaired hair cells.

US patent application 20120328580 describes injection of a pharmaceutical composition into the ear (e.g., auricular administration), such as into the luminae of the cochlea (e.g., the Scala media, Sc vestibulae, and Sc tympani), e.g., using a syringe, e.g., a single-dose syringe. For example, one or more of the compounds described herein can be administered by intratympanic injection (e.g., into the middle ear), and/or injections into the outer, middle, and/or inner ear. Such methods are routinely used in the art, for example, for the administration of steroids and antibiotics into human ears. Injection can be, for example, through the round window of the ear or through the cochlear capsule. Other inner ear administration methods are known in the art (see, e.g., Salt and Plontke, Drug Discovery Today, 10:1299-1306, 2005).

In another mode of administration, the pharmaceutical composition can be administered in situ, via a catheter or pump. A catheter or pump can, for example, direct a pharmaceutical composition into the cochlear luminae or the round window of the ear and/or the lumen of the colon. Exemplary drug delivery apparatus and methods suitable for administering one or more of the compounds described herein into an ear, e.g., a human ear, are described by McKenna et al., (U.S. Publication No. 2006/0030837) and Jacobsen et al., (U.S. Pat. No. 7,206,639). In some embodiments, a catheter or pump can be positioned, e.g., in the ear (e.g., the outer, middle, and/or inner ear) of a patient during a surgical procedure. In some embodiments, a catheter or pump can be positioned, e.g., in the ear (e.g., the outer, middle, and/or inner ear) of a patient without the need for a surgical procedure.

Alternatively or in addition, one or more of the compounds described herein can be administered in combination with a mechanical device such as a cochlear implant or a hearing aid, which is worn in the outer ear. An exemplary cochlear implant that is suitable for use with the present invention is described by Edge et al., (U.S. Publication No. 2007/0093878).

In some embodiments, the modes of administration described above may be combined in any order and can be simultaneous or interspersed.

Alternatively or in addition, the present invention may be administered according to any of the Food and Drug Administration approved methods, for example, as described in CDER Data Standards Manual, version number 004 (which is available at fda.give/cder/dsm/DRG/drg00301.htm).

In general, the cell therapy methods described in US patent application 20120328580 can be used to promote complete or partial differentiation of a cell to or towards a mature cell type of the inner ear (e.g., a hair cell) in vitro. Cells resulting from such methods can then be transplanted or implanted into a patient in need of such treatment. The cell culture methods required to practice these methods, including methods for identifying and selecting suitable cell types, methods for promoting complete or partial differentiation of selected cells, methods for identifying complete or partially differentiated cell types, and methods for implanting complete or partially differentiated cells are described below.

Cells suitable for use in the present invention include, but are not limited to, cells that are capable of differentiating completely or partially into a mature cell of the inner ear, e.g., a hair cell (e.g., an inner and/or outer hair cell), when contacted, e.g., in vitro, with one or more of the compounds described herein. Exemplary cells that are capable of differentiating into a hair cell include, but are not limited to stem cells (e.g., inner ear stem cells, adult stem cells, bone marrow derived stem cells, embryonic stem cells, mesenchymal stem cells, skin stem cells, iPS cells, and fat derived stem cells), progenitor cells (e.g., inner ear progenitor cells), support cells (e.g., Deiters' cells, pillar cells, inner phalangeal cells, tectal cells and Hensen's cells), and/or germ cells. The use of stem cells for the replacement of inner ear sensory cells is described in Li et al., (U.S. Publication No. 2005/0287127) and Li et al., (U.S. patent Ser. No. 11/953,797). The use of bone marrow derived stem cells for the replacement of inner ear sensory cells is described in Edge et al., PCT/US2007/084654. iPS cells are described, e.g., at Takahashi et al., Cell, Volume 131, Issue 5, Pages 861-872 (2007); Takahashi and Yamanaka, Cell 126, 663-76 (2006); Okita et al., Nature 448, 260-262 (2007); Yu, J. et al., Science 318(5858): 1917-1920 (2007); Nakagawa et al., Nat. Biotechnol. 26:101-106 (2008); and Zaehres and Scholer, Cell 131(5):834-835 (2007).

Such suitable cells can be identified by analyzing (e.g., qualitatively or quantitatively) the presence of one or more tissue specific genes. For example, gene expression can be detected by detecting the protein product of one or more tissue-specific genes. Protein detection techniques involve staining proteins (e.g., using cell extracts or whole cells) using antibodies against the appropriate antigen. In this case, the appropriate antigen is the protein product of the tissue-specific gene expression. Although, in principle, a first antibody (i.e., the antibody that binds the antigen) can be labeled, it is more common (and improves the visualization) to use a second antibody directed against the first (e.g., an anti-IgG). This second antibody is conjugated either with fluorochromes, or appropriate enzymes for colorimetric reactions, or gold beads (for electron microscopy), or with the biotin-avidin system, so that the location of the primary antibody, and thus the antigen, can be recognized.

The CRISPR Cas molecules may be delivered to the ear by direct application of pharmaceutical composition to the outer ear, with compositions modified from US Published application, 20110142917. In some embodiments the pharmaceutical composition is applied to the ear canal. Delivery to the ear may also be refered to as aural or otic delivery.

In some embodiments the RNA molecules are delivered in liposome or lipofectin formulations and the like and can be prepared by methods well known to those skilled in the art. Such methods are described, for example, in U.S. Pat. Nos. 5,593,972, 5,589,466, and 5,580,859, which are herein incorporated by reference.

Delivery systems aimed specifically at the enhanced and improved delivery of siRNA into mammalian cells have been developed, (see, for example, Shen et al FEBS Let. 2003, 539:111-114; Xia et al., Nat. Biotech. 2002, 20:1006-1010; Reich et al., Mol. Vision. 2003, 9: 210-216; Sorensen et al., J. Mol. Biol. 2003, 327: 761-766; Lewis et al., Nat. Gen. 2002, 32: 107-108 and Simeoni et al., NAR 2003, 31, 11: 2717-2724) and may be applied to the present invention. siRNA has recently been successfully used for inhibition of gene expression in primates (see for example. Tolentino et al., Retina 24(4):660 which may also be applied to the present invention.

Qi et al. discloses methods for efficient siRNA transfection to the inner ear through the intact round window by a novel proteidic delivery technology which may be applied to the CRISPR Cas system (see, e.g., Qi et al., Gene Therapy (2013), 1-9). In particular, a TAT double stranded RNA-binding domains (TAT-DRBDs), which can transfect Cy3-labeled siRNA into cells of the inner ear, including the inner and outer hair cells, crista ampullaris, macula utriculi and macula sacculi, through intact round-window permeation was successful for delivering double stranded siRNAs in vivo for treating various inner ear ailments and preservation of hearing function. About 40 $\mu$l of 10mM RNA may be contemplated as the dosage for administration to the ear.

According to Rejali et al. (Hear Res. 2007 Jun;228(1-2):180-7), cochlear implant function can be improved by good preservation of the spiral ganglion neurons, which are the target of electrical stimulation by the implant and brain derived neurotrophic factor (BDNF) has previously been shown to enhance spiral ganglion survival in experimentally deafened ears. Rejali et al. tested a modified design of the cochlear implant electrode that includes a coating of fibroblast cells transduced by a viral vector with a BDNF gene insert. To accomplish this type of ex vivo gene transfer, Rejali et al. transduced guinea pig fibroblasts with an adenovirus with a BDNF gene cassette insert, and determined that these cells secreted BDNF and then attached BDNF-secreting cells to the cochlear implant electrode via an agarose gel, and implanted the electrode in the scala tympani. Rejali et al. determined that the BDNF expressing electrodes were able to preserve significantly more spiral ganglion neurons in the basal turns of the cochlea after 48 days of implantation when compared to control electrodes and demonstrated the feasibility of combining cochlear implant therapy with ex vivo gene transfer for enhancing spiral ganglion neuron survival. Such a system may be applied to the CRISPR Cas system for delivery to the ear.

Mukherjea et al. (Antioxidants & Redox Signaling, Volume 13, Number 5, 2010) document that knockdown of NOX3 using short interfering (si) RNA abrogated cisplatin ototoxicity, as evidenced by protection of OHCs from damage and reduced threshold shifts in auditory brainstem responses (ABRs). Different doses of siNOX3 (0.3, 0.6, and 0.9 $\mu$g) were administered to rats and NOX3 expression was evaluated by real time RT-PCR. The lowest dose of NOX3 siRNA used (0.3 $\mu$g) did not show any inhibition of NOX3 mRNA when compared to transtympanic administration of scrambled siRNA or untreated cochleae. However, administration of the higher doses of NOX3 siRNA (0.6 and 0.9 $\mu$g) reduced NOX3 expression compared to control scrambled siRNA. Such a system may be applied to the CRISPR Cas system for transtympanic administration with a dosage of about 2 mg to about 4 mg of CRISPR Cas for administration to a human.

Jung et al. (Molecular Therapy, vol. 21 no. 4, 834-841 apr. 2013) demonstrate that Hes5 levels in the utricle decreased after the application of siRNA and that the number of hair cells in these utricles was significantly larger than following control treatment. The data suggest that siRNA technology may be useful for inducing repair and regeneration in the inner ear and that the Notch signaling pathway is a potentially useful target for specific gene expression inhibition. Jung et al. injected 8 $\mu$g of Hes5 siRNA in 2 $\mu$l volume, prepared by adding sterile normal saline to the lyophilized siRNA to a vestibular epithelium of the ear. Such a system may be applied to the CRISPR Cas system for administration to the vestibular epithelium of the ear with a dosage of about 1 to about 30 mg of CRISPR Cas for administration to a human.

## Eyes

The present disclosure also contemplates delivering the CRISPR-Cas system to one or both eyes.

In yet another aspect of the invention, the CRISPR-Cas system may be used to correct ocular defects that arise from several genetic mutations further described in Genetic Diseases of the Eye, Second Edition, edited by Elias I. Traboulsi, Oxford University Press, 2012.

For administration to the eye, lentiviral vectors, in particular equine infectious anemia viruses (EIAV) are particularly preferred.

In another embodiment, minimal non-primate lentiviral vectors based on the equine infectious anemia virus (EIAV) are also contemplated, especially for ocular gene therapy (see, e.g., Balagaan, J Gene Med 2006; 8: 275 - 285, Published online 21 November 2005 in Wiley InterScience (www.interscience.wiley.com). DOI: 10.1002/jgm.845). The vectors are contemplated to have cytomegalovirus (CMV) promoter driving expression of the target gene. Intracameral, subretinal, intraocular and intravitreal injections are all contemplated (see, e.g., Balagaan, J Gene Med 2006; 8: 275 - 285, Published online 21 November 2005 in Wiley InterScience (www.interscience.wiley.com). DOI: 10.1002/jgm.845). Intraocular injections may be performed with the aid of an operating microscope. For subretinal and intravitreal injections, eyes may be prolapsed by gentle digital pressure and fundi visualised using a contact lens system consisting of a drop of a coupling medium solution on the cornea covered with a glass microscope slide coverslip. For subretinal injections, the tip of a 10-mm 34-gauge needle, mounted on a 5-$\mu$l Hamilton syringe may be advanced under direct visualisation through the superior equatorial sclera tangentially towards the posterior pole until the aperture of the needle was visible in the subretinal space. Then, 2 $\mu$l of vector suspension may be injected to produce a superior bullous retinal detachment, thus confirming subretinal vector administration. This approach creates a self-sealing sclerotomy allowing the vector suspension to be retained in the subretinal space until it is absorbed by the RPE, usually within 48 h of the procedure. This procedure may be repeated in the inferior hemisphere to produce an inferior retinal detachment. This technique results in the exposure of approximately 70% of neurosensory retina and RPE to the vector suspension. For intravitreal injections, the needle tip may be advanced through the sclera 1 mm posterior to the corneoscleral limbus and 2 $\mu$l of vector suspension injected into the vitreous cavity. For intracameral injections, the needle tip may be advanced through a corneoscleral limbal paracentesis, directed towards the central cornea, and 2 $\mu$l of vector suspension may be injected. For intracameral injections, the needle tip may be advanced through a corneoscleral limbal paracentesis, directed towards the central cornea, and 2 $\mu$l of vector suspension may be injected. These vectors may be injected at titres of either 1.0-1.4 $\times$ 10$^{10}$ or 1.0-1.4 $\times$ 10$^9$ transducing units (TU)/ml.

In another embodiment, RetinoStat®, an equine infectious anemia virus-based lentiviral gene therapy vector that expresses angiostatic proteins endostain and angiostatin that is delivered via a subretinal injection for the treatment of the web form of age-related macular degeneration is also contemplated (see, e.g., Binley et al., HUMAN GENE THERAPY 23:980-991 (September 2012)). Such a vector may be modified for the CRISPR-Cas system. Each eye may be treated with either RetinoStat® at a dose of 1.1 x 10$^5$ transducing units per eye (TU/eye) in a total volume of 100 $\mu$l.

In another embodiment, an E1-, partial E3-, E4-deleted adenoviral vector may be contemplated for delivery to the eye. Twenty-eight patients with advanced neovascular age-related macular degeneration (AMD) were given a single intravitreous injection of an E1-, partial E3-, E4-deleted adenoviral vector expressing human pigment ep- ithelium-derived factor (AdPEDF.ll) (see, e.g., Campochiaro et al., Human Gene Therapy 17:167-176 (February 2006)). Doses ranging from 10$^6$ to 10$^{9.5}$ particle units (PU) were investigated and there were no serious adverse events related to AdPEDF.ll and no dose-limiting toxicities (see, e.g., Campochiaro et al., Human Gene Therapy 17:167-176 (February 2006)). Adenoviral vector-mediated ocular gene transfer appears to be a viable approach for the treatment of ocular disorders and could be applied to the CRISPR Cas system.

In another embodiment, the sd-rxRNA® system of RXi Pharmaceuticals may be used/and or adapted for delivering CRISPR Cas to the eye. In this system, a single intravitreal administration of 3 $\mu$g of sd-rxRNA results in sequence-specific reduction of PPIB mRNA levels for 14 days. The the sd-rxRNA® system may be applied to the CRISPR Cas system, contemplating a dose of about 3 to 20 mg of CRISPR administered to a human.

Millington-Ward et al. (Molecular Therapy, vol. 19 no. 4, 642-649 apr. 2011) describes adeno-associated virus (AAV) vectors to deliver an RNA interference (RNAi)-based rhodopsin suppressor and a codon-modified rhodopsin replacement gene resistant to suppression due to nucleotide alterations at degenerate positions over the RNAi target site. An injection of either 6.0 x 10$^8$ vp or 1.8 x 10$^{10}$ vp AAV were subretinally injected into the eyes by Millington-Ward et al. The AAV vectors of Millington-Ward et al. may be applied to the CRISPR Cas system, contemplating a dose of about $2 \times 10^{11}$ to about $6 \times 10^{13}$ vp administered to a human.

Dalkara et al. (Sci Transl Med 5, 189ra76 (2013)) also relates to in vivo directed evolution to fashion an AAV vector that delivers wild-type versions of defective genes throughout the retina after noninjurious injection into the eyes' vitreous humor. Dalkara describes a a 7mer peptide display library and an AAV library constructed by DNA shuffling of cap genes from AAV1, 2, 4, 5, 6, 8, and 9. The rcAAV libraries and rAAV vectors expressing GFP under a CAG or Rho promoter were packaged and and deoxyribonuclease-resistant genomic titers were obtained through quantitative PCR. The libraries were pooled, and two rounds of evolution were performed, each consisting of initial library diversification followed by three in vivo selection steps. In each such step, P30 rho-GFP mice were intravitreally injected with 2 ml of iodixanol-purified, phosphate-buffered saline (PBS)-dialyzed library with a genomic titer of about $1 \times 10^{12}$ vg/ml. The AAV vectors of Dalkara et al. may be applied to the CRISPR Cas system, contemplating a dose of about $1 \times 10^{15}$ to about $1 \times 10^{16}$ vg/ml administered to a human.

In another embodiment, the rhodopsin gene may be targeted for the treatment of retinitis pigmentosa (RP), wherein the system of US Patent Publication No. 20120204282 assigned to Sangamo BioSciences, Inc. may be modified in accordance of the CRISPR Cas system.

In another embodiment, the methods of US Patent Publication No. 20130183282 assigned to Cellectis, which is directed to methods of cleaving a target sequence from the human rhodopsin gene, may also be modified to the CRISPR Cas system.

US Patent Publication No. 20130202678 assigned to Academia Sinica relates to methods for treating retinopathies and sight-threatening ophthalmologic disorders relating to delivering of the Puf-A gene (which is expressed in retinal ganglion and pigmented cells of eye tissues and displays a unique anti-apoptotic activity) to the sub-retinal or intravitreal space in the eye. In particular, desirable targets are zgc: 193933, prdmla, spata2, tex10, rbb4, ddx3, zp2.2, Blimp-1 and HtrA2, all of which may be targeted by the CRISPR Cas system.

Wu (Cell Stem Cell,13:659-62, 2013) designed a guide RNA that led Cas9 to a single base pair mutation that causes cataracts in mice, where it induced DNA cleavage. Then using either the other wild-type allele or oligos given to the zygotes repair mechanisms corrected the sequence of the broken allele and corrected the cataract-causing genetic defect in mutant mouse.

US Patent Publication No. 20120159653, describes use of zinc finger nucleases to genetically modify cells, animals and proteins associated with macular degeration (MD). Macular degeneration (MD) is the primary cause of visual impairment in the elderly, but is also a hallmark symptom of childhood diseases such as Stargardt disease, Sorsby fundus, and fatal childhood neurodegenerative diseases, with an age of onset as young as infancy. Macular degeneration results in a loss of vision in the center of the visual field (the macula) because of damage to the retina. Currently existing animal models do not recapitulate major hallmarks of the disease as it is observed in humans. The available animal models comprising mutant genes encoding proteins associated with MD also produce highly variable phenotypes, making translations to human disease and therapy development problematic.

One aspect of US Patent Publication No. 20120159653 relates to editing of any chromosomal sequences that encode proteins associated with MD which may be applied to the CRISPR Cas system. The proteins associated with MD are typically selected based on an experimental association of the protein associated with MD to an MD disorder. For example, the production rate or circulating concentration of a protein associated with MD may be elevated or depressed in a population having an MD disorder relative to a population lacking the MD disorder. Differences in protein levels may be assessed using proteomic techniques including but not limited to Western blot, immunohistochemical staining, enzyme linked immunosorbent assay (ELISA), and mass spectrometry. Alternatively, the proteins associated with MD may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

By way of non-limiting example, proteins associated with MD include but are not limited to the following proteins: (ABCA4) ATP-binding cassette, sub-family A (ABC1), member 4 ACHM1 achromatopsia (rod monochromacy) 1 ApoE Apolipoprotein E (ApoE) C1QTNF5 (CTRP5) C1q and tumor necrosis factor related protein 5 (C1QTNF5) C2 Complement component 2 (C2) C3 Complement components (C3) CCL2 Chemokine (C-C motif) Ligand 2 (CCL2) CCR2 Chemokine (C-C motif) receptor 2 (CCR2) CD36 Cluster of Differentiation 36 CFB Complement factor B CFH Complement factor CFH H CFHR1 complement factor H-related 1 CFHR3 complement factor H-related 3 CNGB3 cyclic nucleotide gated channel beta 3 CP ceruloplasmin (CP) CRP C reactive protein (CRP) CST3 cystatin C or cystatin 3 (CST3) CTSD Cathepsin D (CTSD) CX3CR1 chemokine (C-X3-C motif) receptor 1 ELOVL4 Elongation of very long chain fatty acids 4 ERCC6 excision repair cross- complementing rodent repair deficiency, complementation group 6 FBLN5 Fibulin-5 FBLN5 Fibulin 5 FBLN6 Fibulin 6 FSCN2 fascin (FSCN2) HMCN1 Hemicentrin 1 HMCN1 hemicentin 1 HTRA1 HtrA serine peptidase 1 (HTRA1) HTRA1 HtrA serine peptidase 1 IL-6 Interleukin 6 IL-8 Interleukin 8 LOC387715 Hypothetical protein PLEKHA1 Pleckstrin homology domain-containing family A member 1 (PLEKHA1) PROM1 Prominin 1(PROM1 or CD133) PRPH2 Peripherin-2 RPGR retinitis pigmentosa GTPase regulator SERPING1 serpin peptidase inhibitor, clade G, member 1 (C1- inhibitor) TCOF1 Treacle TIMP3 Metalloproteinase inhibitor 3 (TIMP3) TLR3 Toll-like receptor 3

The identity of the protein associated with MD whose chromosomal sequence is edited can and will vary. In preferred embodiments, the proteins associated with MD whose chromosomal sequence is edited may be the ATP-binding cassette, sub-family A (ABC1) member 4 protein (ABCA4) encoded by the ABCR gene, the apolipoprotein E protein (APOE) encoded by the APOE gene, the chemokine (C-C motif) Ligand 2 protein (CCL2) encoded by the CCL2 gene, the chemokine (C-C motif) receptor 2 protein (CCR2) encoded by the CCR2 gene, the ceruloplasmin protein (CP) encoded by the CP gene, the cathepsin D protein (CTSD) encoded by the CTSD gene, or the metalloproteinase inhibitor 3 protein (TIMP3) encoded by the TIMP3 gene. In an exemplary embodiment, the genetically modified animal is a rat, and the edited chromosomal sequence encoding the protein associated with MD may be: (ABCA4) ATP-binding cassette, NM_000350 sub-family A (ABC1), member 4 APOE Apolipoprotein E NM_138828 (APOE) CCL2 Chemokine (C-C NM_031530 motif) Ligand 2 (CCL2) CCR2 Chemokine (C-C NM_021866 motif) receptor 2 (CCR2) CP ceruloplasmin (CP) NM_012532 CTSD Cathepsin D (CTSD) NM_134334 TIMP3 Metalloproteinase NM_012886 inhibitor 3 (TIMP3) The animal or cell may comprise 1, 2, 3, 4, 5, 6, 7 or more disrupted chromosomal sequences encoding a protein associated with MD and zero, 1, 2, 3, 4, 5, 6, 7 or more chromosomally integrated sequences encoding the disrupted protein associated with MD.

The edited or integrated chromosomal sequence may be modified to encode an altered protein associated with MD. Several mutations in MD-related chromosomal sequences have been associated with MD. Non-limiting examples of mutations in chromosomal sequences associated with MD include those that may cause MD including in the ABCR protein, E471K (i.e. glutamate at position 471 is changed to lysine), R1129L (i.e. arginine at position 1129 is changed to leucine), T1428M (i.e. threonine at position 1428 is changed to methionine), R1517S (i.e. arginine at position 1517 is changed to serine), I1562T (i.e. isoleucine at position 1562 is changed to threonine), and G1578R (i.e. glycine at position 1578 is changed to arginine); in the CCR2 protein, V64I (i.e. valine at position 192 is changed to isoleucine); in CP protein, G969B (i.e. glycine at position 969 is changed to asparagine or aspartate); in TIMP3 protein, S156C (i.e. serine at position 156 is changed to cysteine), G166C (i.e. glycine at position 166 is changed to cysteine), G167C (i.e. glycine at position 167 is changed to cysteine), Y168C (i.e. tyrosine at position 168 is changed to cysteine), S170C (i.e. serine at position 170 is changed to cysteine), Y172C (i.e. tyrosine at position 172 is changed to cysteine) and S181C (i.e. serine at position 181 is changed to cysteine). Other associations of genetic variants in MD-associated genes and disease are known in the art.

### Heart

The present disclosure also contemplates delivering the CRISPR-Cas system to the heart. For the heart, a myocardium tropic adena-associated virus (AAVM) is preferred, in particular AAVM41 which showed preferential gene transfer in the heart (see, e.g., Lin-Yanga et al., PNAS, March 10, 2009, vol. 106, no. 10). Administration may be systemic or local. A dosage of about 1-10 x 10$^{14}$ vector genomes are contemplated for systemic administration. See also, e.g., Eulalio et al. (2012) Nature 492: 376 and Somasuntharam et al. (2013) Biomaterials 34: 7790.

For example, US Patent Publication No. 20110023139, describes use of zinc finger nucleases to genetically modify cells, animals and proteins associated with cardiovascular disease. Cardiovascular diseases generally include high blood pressure, heart attacks, heart failure, and stroke and TIA. Any chromosomal sequence involved in cardiovascular disease or the protein encoded by any chromosomal sequence involved in cardiovascular disease may be utilized in the methods described in this disclosure. The cardiovascular-related proteins are typically selected based on an experimental association of the cardiovascular-related protein to the development of cardiovascular disease. For example, the production rate or circulating concentration of a cardiovascular-related protein may be elevated or depressed in a population having a cardiovascular disorder relative to a population lacking the cardiovascular disorder. Differences in protein levels may be assessed using proteomic techniques including but not limited to Western blot, immunohistochemical staining, enzyme linked immunosorbent assay (ELISA), and mass spectrometry. Alternatively, the cardiovascular-related proteins may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

By way of example, the chromosomal sequence may comprise, but is not limited to, IL1B (interleukin 1, beta), XDH (xanthine dehydrogenase), TP53 (tumor protein p53), PTGIS (prostaglandin 12 (prostacyclin) synthase), MB (myoglobin), IL4 (interleukin 4), ANGPT1 (angiopoietin 1), ABCG8 (ATP-binding cassette, sub-family G (WHITE), member 8), CTSK (cathepsin K), PTGIR (prostaglandin 12 (prostacyclin) receptor (IP)), KCNJ11 (potassium inwardly-rectifying channel, subfamily J, member 11), INS (insulin), CRP (C-reactive protein, pentraxin-related), PDGFRB (platelet-derived growth factor receptor, beta polypeptide), CCNA2 (cyclin A2), PDGFB (platelet-derived growth factor beta polypeptide (simian sarcoma viral (v-sis) oncogene homolog)), KCNJ5 (potassium inwardly-rectifying channel, subfamily J, member 5), KCNN3 (potassium intermediate/small conductance calcium-activated channel, subfamily N, member 3), CAPN10 (calpain 10), PTGES (prostaglandin E synthase), ADRA2B (adrenergic, alpha-2B-, receptor), ABCG5 (ATP-binding cassette, sub-family G (WHITE), member 5), PRDX2 (peroxiredoxin 2), CAPN5 (calpain 5), PARP14 (poly (ADP-ribose) polymerase family, member 14), MEX3C (mex-3 homolog C (C. elegans)), ACE angiotensin I converting enzyme (peptidyl-dipeptidase A) 1), TNF (tumor necrosis factor (TNF superfamily, member 2)), IL6 (interleukin 6 (interferon, beta 2)), STN (statin), SERPINE1 (serpin peptidase inhibitor, clade E (nexin, plasminogen activator inhibitor type 1), member 1), ALB (albumin), ADIPOQ (adiponectin, C1Q and collagen domain containing), APOB (apolipoprotein B (including Ag(x) antigen)), APOE (apolipoprotein E), LEP (leptin), MTHFR (5,10-methylenetetrahydrofolate reductase (NADPH)), APOA1 (apolipoprotein A-I), EDN1 (endothelin 1), NPPB (natriuretic peptide precursor B), NOS3 (nitric oxide synthase 3 (endothelial cell)), PPARG (peroxisome proliferator-activated receptor gamma), PLAT (plasminogen activator, tissue), PTGS2 (prostaglandin-endoperoxide synthase 2 (prostaglandin G/H synthase and cyclooxygenase)), CETP (cholesteryl ester transfer protein, plasma), AGTR1 (angiotensin II receptor, type 1), HMGCR (3-hydroxy-3-methylglutaryl-Coenzyme A reductase), IGF1 (insulin-like growth factor 1 (somatomedin C)), SELE (selectin E), REN (renin), PPARA (peroxisome proliferator-activated receptor alpha), PON1 (paraoxonase 1), KNG1 (kininogen 1), CCL2 (chemokine (C-C motif) ligand 2), LPL (lipoprotein lipase), VWF (von Willebrand factor), F2 (coagulation factor II (thrombin)), ICAM1 (intercellular adhesion molecule 1), TGFB1 (transforming growth factor, beta 1), NPPA (natriuretic peptide precursor A), IL10 (interleukin 10), EPO (erythropoietin), SOD1 (superoxide dismutase 1, soluble), VCAM1 (vascular cell adhesion molecule 1), IFNG (interferon, gamma), LPA (lipoprotein, Lp(a)), MPO (myeloperoxidase), ESR1 (estrogen receptor 1), MAPK1 (mitogen-activated protein kinase 1), HP (haptoglobin), F3 (coagulation factor III (thromboplastin, tissue factor)), CST3 (cystatin C), COG2 (component of oligomeric golgi complex 2), MMP9 (matrix metallopeptidase 9 (gelatinase B, 92 kDa gelatinase, 92 kDa type IV collagenase)), SERPINC1 (serpin peptidase inhibitor, clade C (antithrombin), member 1), F8 (coagulation factor VIII, procoagulant component), HMOX1 (heme oxygenase (decycling) 1), APOC3 (apolipoprotein C-III), IL8 (interleukin 8), PROK1 (prokineticin 1), CBS (cystathionine-beta-synthase), NOS2 (nitric oxide synthase 2, inducible), TLR4 (toll-like receptor 4), SELP (selectin P (granule membrane protein 140 kDa, antigen CD62)), ABCA1 (ATP-binding cassette, sub-family A (ABC1), member 1), AGT (angiotensinogen (serpin peptidase inhibitor, clade A, member 8)), LDLR (low density lipoprotein receptor), GPT (glutamic-pyruvate transaminase (alanine aminotransferase)), VEGFA (vascular endothelial growth factor A), NR3C2 (nuclear receptor subfamily 3, group C, member 2), IL18 (interleukin 18 (interferon-gamma-inducing factor)), NOS1 (nitric oxide synthase 1 (neuronal)), NR3C1 (nuclear receptor subfamily 3, group C, member 1 (glucocorticoid receptor)), FGB (fibrinogen beta chain), HGF (hepatocyte growth factor (hepapoietin A; scatter factor)), IL1A (interleukin 1, alpha), RETN (resistin), AKT1 (v-akt murine thymoma viral oncogene homolog 1), LIPC (lipase, hepatic), HSPD1 (heat shock 60 kDa protein 1 (chaperonin)), MAPK14 (mitogen-activated protein kinase 14), SPP1 (secreted phosphoprotein 1), ITGB3 (integrin, beta 3 (platelet glycoprotein 111a, antigen CD61)), CAT (catalase), UTS2 (urotensin 2), THBD (thrombomodulin), F10 (coagulation factor X), CP (ceruloplasmin (ferroxidase)), TNFRSF11B (tumor necrosis factor receptor superfamily, member 11b), EDNRA (endothelin receptor type A), EGFR (epidermal growth factor receptor (erythroblastic leukemia viral (v-erb-b) oncogene homolog, avian)), MMP2 (matrix metallopeptidase 2 (gelatinase A, 72 kDa gelatinase, 72 kDa type IV collagenase)), PLG (plasminogen), NPY (neuropeptide Y), RHOD (ras homolog gene family, member D), MAPK8 (mitogen-activated protein kinase 8), MYC (v-myc myelocytomatosis viral oncogene homolog (avian)), FN1 (fibronectin 1), CMA1 (chymase 1, mast cell), PLAU (plasminogen activator, urokinase), GNB3 (guanine nucleotide binding protein (G protein), beta polypeptide 3), ADRB2 (adrenergic, beta-2-, receptor, surface), APOA5 (apolipoprotein A-V), SOD2 (superoxide dismutase 2, mitochondrial), F5 (coagulation factor V (proaccelerin, labile factor)), VDR (vitamin D (1,25-dihydroxyvitamin D3) receptor), ALOX5 (arachidonate 5-lipoxygenase), HLA-DRB1 (major histocompatibility complex, class II, DR beta 1), PARP1 (poly (ADP-ribose) polymerase 1), CD40LG (CD40 ligand), PON2 (paraoxonase 2), AGER (advanced glycosylation end product-specific receptor), IRS1 (insulin receptor substrate 1), PTGS1 (prostaglandin-endoperoxide synthase 1 (prostaglandin G/H synthase and cyclooxygenase)), ECE1 (endothelin converting enzyme 1), F7 (coagulation factor VII (serum prothrombin conversion accelerator)), URN (interleukin 1 receptor antagonist), EPHX2 (epoxide hydrolase 2, cytoplasmic), IGFBP1 (insulin-like growth factor binding protein 1), MAPK10 (mitogen-activated protein kinase 10), FAS (Fas (TNF receptor superfamily, member 6)), ABCB1 (ATP-binding cassette, sub-family B (MDR/TAP), member 1), JUN (jun oncogene), IGFBP3 (insulin-like growth factor binding protein 3), CD14 (CD14 molecule), PDE5A (phosphodiesterase 5A, cGMP-specific), AGTR2 (angiotensin II receptor, type 2), CD40 (CD40 molecule, TNF receptor superfamily member 5), LCAT (lecithin-cholesterol acyltransferase), CCR5 (chemokine (C-C motif) receptor 5), MMP1 (matrix metallopeptidase 1 (interstitial collagenase)), TIMP1 (TIMP metallopeptidase inhibitor 1), ADM (adrenomedullin), DYT10 (dystonia 10), STAT3 (signal transducer and activator of transcription 3 (acute-phase response factor)), MMP3 (matrix metallopeptidase 3 (stromelysin 1, progelatinase)), ELN (elastin), USF1 (upstream transcription factor 1), CFH (complement factor H), HSPA4 (heat shock 70 kDa protein 4), MMP12 (matrix metallopeptidase 12 (macrophage elastase)), MME (membrane metallo-endopeptidase), F2R (coagulation factor II (thrombin) receptor), SELL (selectin L), CTSB (cathepsin B), ANXA5 (annexin A5), ADRB1 (adrenergic, beta-1-, receptor), CYBA (cytochrome b-245, alpha polypeptide), FGA (fibrinogen alpha chain), GGT1 (gamma-glutamyltransferase 1), LIPG (lipase, endothelial), HIF1A (hypoxia inducible factor 1, alpha subunit (basic helix-loop-helix transcription factor)), CXCR4 (chemokine (C-X-C motif) receptor 4), PROC (protein C (inactivator of coagulation factors Va and VIIIa)), SCARB1 (scavenger receptor class B, member 1), CD79A (CD79a molecule, immunoglobulin-associated alpha), PLTP (phospholipid transfer protein), ADD1 (adducin 1 (alpha)), FGG (fibrinogen gamma chain), SAA1 (serum amyloid A1), KCNH2 (potassium voltage-gated channel, subfamily H (eag-related), member 2), DPP4 (dipeptidyl-peptidase 4), G6PD (glucose-6-phosphate dehydrogenase), NPR1 (natriuretic peptide receptor A/guanylate cyclase A (atrionatriuretic peptide receptor A)), VTN (vitronectin), KIAA0101 (KIAA0101), FOS (FBJ murine osteosarcoma viral oncogene homolog), TLR2 (toll-like receptor 2), PPIG (peptidylprolyl isomerase G (cyclophilin G)), IL1R1 (interleukin 1 receptor, type I), AR (androgen receptor), CYP1A1 (cytochrome P450, family 1, subfamily A, polypeptide 1), SERPINA1 (serpin peptidase inhibitor, clade A (alpha-1 antiproteinase, antitrypsin), member 1), MTR (5-methyltetrahydrofolate-homocysteine methyltransferase), RBP4 (retinol binding protein 4, plasma), APOA4 (apolipoprotein A-IV), CDKN2A (cyclin-dependent kinase inhibitor 2A (melanoma, p16, inhibits CDK4)), FGF2 (fibroblast growth factor 2 (basic)), EDNRB (endothelin receptor type B), ITGA2 (integrin, alpha 2 (CD49B, alpha 2 subunit of VLA-2 receptor)), CABIN1 (calcineurin binding protein 1), SHBG (sex hormone-binding globulin), HMGB1 (high-mobility group box 1), HSP90B2P (heat shock protein 90 kDa beta (Grp94), member 2 (pseudogene)), CYP3A4 (cytochrome P450, family 3, subfamily A, polypeptide 4), GJA1 (gap junction protein, alpha 1, 43 kDa), CAV1 (caveolin 1, caveolae protein, 22 kDa), ESR2 (estrogen receptor 2 (ER beta)), LTA (lymphotoxin alpha (TNF superfamily, member 1)), GDF15 (growth differentiation factor 15), BDNF (brain-derived neurotrophic factor), CYP2D6 (cytochrome P450, family 2, subfamily D, polypeptide 6), NGF (nerve growth factor (beta polypeptide)), SP1 (Sp1 transcription factor), TGIF1 (TGFB-induced factor homeobox 1), SRC (v-src sarcoma (Schmidt-Ruppin A-2) viral oncogene homolog (avian)), EGF (epidermal growth factor (beta-urogastrone)), PIK3CG (phosphoinositide-3-kinase, catalytic, gamma polypeptide), HLA-A (major histocompatibility complex, class I, A), KCNQ1 (potassium voltage-gated channel, KQT-like subfamily, member 1), CNR1 (cannabinoid receptor 1 (brain)), FBN1 (fibrillin 1), CHKA (choline kinase alpha), BEST1 (bestrophin 1), APP (amyloid beta (A4) precursor protein), CTNNB1 (catenin (cadherin-associated protein), beta 1, 88 kDa), IL2 (interleukin 2), CD36 (CD36 molecule (thrombospondin receptor)), PRKAB1 (protein kinase, AMP-activated, beta 1 non-catalytic subunit), TPO (thyroid peroxidase), ALDH7A1 (aldehyde dehydrogenase 7 family, member A1), CX3CR1 (chemokine (C-X3-C motif) receptor 1), TH (tyrosine hydroxylase), F9 (coagulation factor IX), GH1 (growth hormone 1), TF (transferrin), HFE (hemochromatosis), IL17A (interleukin 17A), PTEN (phosphatase and tensin homolog), GSTM1 (glutathione S-transferase mu 1), DMD (dystrophin), GATA4 (GATA binding protein 4), F13A1 (coagulation factor XIII, A1 polypeptide), TTR (transthyretin), FABP4 (fatty acid binding protein 4, adipocyte), PON3 (paraoxonase 3), APOC1 (apolipoprotein C-I), INSR (insulin receptor), TNFRSF1B (tumor necrosis factor receptor superfamily, member 1B), HTR2A (5-hydroxytryptamine (serotonin) receptor 2A), CSF3 (colony stimulating factor 3 (granulocyte)), CYP2C9 (cytochrome P450, family 2, subfamily C, polypeptide 9), TXN (thioredoxin), CYP11B2 (cytochrome P450, family 11, subfamily B, polypeptide 2), PTH (parathyroid hormone), CSF2 (colony stimulating factor 2 (granulocyte-macrophage)), KDR (kinase insert domain receptor (a type III receptor tyrosine kinase)), PLA2G2A (phospholipase A2, group IIA (platelets, synovial fluid)), B2M (beta-2-microglobulin), THBS1 (thrombospondin 1), GCG (glucagon), RHOA (ras homolog gene family, member A), ALDH2 (aldehyde dehydrogenase 2 family (mitochondrial)), TCF7L2 (transcription factor 7-like 2 (T-cell specific, HMG-box)), BDKRB2 (bradykinin receptor B2), NFE2L2 (nuclear factor (erythroid-derived 2)-like 2), NOTCH1 (Notch homolog 1, translocation-associated (Drosophila)), UGT1A1 (UDP glucuronosyltransferase 1 family, polypeptide A1), IFNA1 (interferon, alpha 1), PPARD (peroxisome proliferator-activated receptor delta), SIRT1 (sirtuin (silent mating type information regulation 2 homolog) 1 (S. cerevisiae)), GNRH1 (gonadotropin-releasing hormone 1 (luteinizing-releasing hormone)), PAPPA (pregnancy-associated plasma protein A, pappalysin 1), ARR3 (arrestin 3, retinal (X-arrestin)), NPPC (natriuretic peptide precursor C), AHSP (alpha hemoglobin stabilizing protein), PTK2 (PTK2 protein tyrosine kinase 2), IL13 (interleukin 13), MTOR (mechanistic target of rapamycin (serine/threonine kinase)), ITGB2 (integrin, beta 2 (complement component 3 receptor 3 and 4 subunit)), GSTT1 (glutathione S-transferase theta 1), IL6ST (interleukin 6 signal transducer (gp130, oncostatin M receptor)), CPB2 (carboxypeptidase B2 (plasma)), CYP1A2 (cytochrome P450, family 1, subfamily A, polypeptide 2), HNF4A (hepatocyte nuclear factor 4, alpha), SLC6A4 (solute carrier family 6 (neurotransmitter transporter, serotonin), member 4), PLA2G6 (phospholipase A2, group VI (cytosolic, calcium-independent)), TNFSF11 (tumor necrosis factor (ligand) superfamily, member 11), SLC8A1 (solute carrier family 8 (sodium/calcium exchanger), member 1), F2RL1 (coagulation factor II (thrombin) receptor-like 1), AKR1A1 (aldo-keto reductase family 1, member A1 (aldehyde reductase)), ALDH9A1 (aldehyde dehydrogenase 9 family, member A1), BGLAP (bone gamma-carboxyglutamate (gla) protein), MTTP (microsomal triglyceride transfer protein), MTRR (5-methyltetrahydrofolate-homocysteine methyltransferase reductase), SULT1A3 (sulfotransferase family, cytosolic, 1A, phenol-preferring, member 3), RAGE (renal tumor antigen), C4B (complement component 4B (Chido blood group), P2RY12 (purinergic receptor P2Y, G-protein coupled, 12), RNLS (renalase, FAD-dependent amine oxidase), CREB1 (cAMP responsive element binding protein 1), POMC (proopiomelanocortin), RAC1 (ras-related C3 botulinum toxin substrate 1 (rho family, small GTP binding protein Rac1)), LMNA (lamin NC), CD59 (CD59 molecule, complement regulatory protein), SCN5A (sodium channel, voltage-gated, type V, alpha subunit), CYP1B1 (cytochrome P450, family 1, subfamily B, polypeptide 1), MIF (macrophage migration inhibitory factor (glycosylation-inhibiting factor)), MMP13 (matrix metallopeptidase 13 (collagenase 3)), TIMP2 (TIMP metallopeptidase inhibitor 2), CYP19A1 (cytochrome P450, family 19, subfamily A, polypeptide 1), CYP21A2 (cytochrome P450, family 21, subfamily A, polypeptide 2), PTPN22 (protein tyrosine phosphatase, non-receptor type 22 (lymphoid)), MYH14 (myosin, heavy chain 14, non-muscle), MBL2 (mannose-binding lectin (protein C) 2, soluble (opsonic defect)), SELPLG (selectin P ligand), AOC3 (amine oxidase, copper containing 3 (vascular adhesion protein 1)), CTSL1 (cathepsin L1), PCNA (proliferating cell nuclear antigen), IGF2 (insulin-like growth factor 2 (somatomedin A)), ITGB1 (integrin, beta 1 (fibronectin receptor, beta polypeptide, antigen CD29 includes MDF2, MSK12)), CAST (calpastatin), CXCL12 (chemokine (C-X-C motif) ligand 12 (stromal cell-derived factor 1)), IGHE (immunoglobulin heavy constant epsilon), KCNE1 (potassium voltage-gated channel, Isk-related family, member 1), TFRC (transferrin receptor (p90, CD71)), COL1A1 (collagen, type I, alpha 1), COL1A2 (collagen, type I, alpha 2), IL2RB (interleukin 2 receptor, beta), PLA2G10 (phospholipase A2, group X), ANGPT2 (angiopoietin 2), PROCR (protein C receptor, endothelial (EPCR)), NOX4 (NADPH oxidase 4), HAMP (hepcidin antimicrobial peptide), PTPN11 (protein tyrosine phosphatase, non-receptor type 11), SLC2A1 (solute carrier family 2 (facilitated glucose transporter), member 1), IL2RA (interleukin 2 receptor, alpha), CCL5 (chemokine (C-C motif) ligand 5), IRF1 (interferon regulatory factor 1), CFLAR (CASP8 and

FADD-like apoptosis regulator), CALCA (calcitonin-related polypeptide alpha), EIF4E (eukaryotic translation initiation factor 4E), GSTP1 (glutathione S-transferase pi 1), JAK2 (Janus kinase 2), CYP3A5 (cytochrome P450, family 3, sub-family A, polypeptide 5), HSPG2 (heparan sulfate proteoglycan 2), CCL3 (chemokine (C-C motif) ligand 3), MYD88 (myeloid differentiation primary response gene (88)), VIP (vasoactive intestinal peptide), SOAT1 (sterol O-acyltransferase 1), ADRBK1 (adrenergic, beta, receptor kinase 1), NR4A2 (nuclear receptor subfamily 4, group A, member 2), MMP8 (matrix metallopeptidase 8 (neutrophil collagenase)), NPR2 (natriuretic peptide receptor B/guanylate cyclase B (atrionatriuretic peptide receptor B)), GCH1 (GTP cyclohydrolase 1), EPRS (glutamyl-prolyl-tRNA synthetase), PPARGC1A (peroxisome proliferator-activated receptor gamma, coactivator 1 alpha), F12 (coagulation factor XII (Hageman factor)), PECAM1 (platelet/endothelial cell adhesion molecule), CCL4 (chemokine (C-C motif) ligand 4), SERPINA3 (serpin peptidase inhibitor, clade A (alpha-1 antiproteinase, antitrypsin), member 3), CASR (calcium-sensing receptor), GJA5 (gap junction protein, alpha 5, 40 kDa), FABP2 (fatty acid binding protein 2, intestinal), TTF2 (transcription termination factor, RNA polymerase II), PROS1 (protein S (alpha)), CTF1 (cardiotrophin 1), SGCB (sarcoglycan, beta (43 kDa dystrophin-associated glycoprotein)), YME1L1 (YME1-like 1 (S. cerevisiae)), CAMP (cathelicidin antimicrobial peptide), ZC3H12A (zinc finger CCCH-type containing 12A), AKR1B1 (aldo-keto reductase family 1, member B1 (aldose reductase)), DES (desmin), MMP7 (matrix metallopeptidase 7 (matrilysin, uterine)), AHR (aryl hydrocarbon receptor), CSF1 (colony stimulating factor 1 (macrophage)), HDAC9 (histone deacetylase 9), CTGF (connective tissue growth factor), KCNMA1 (potassium large conductance calcium-activated channel, subfamily M, alpha member 1), UGT1A (UDP glucuronosyltransferase 1 family, polypeptide A complex locus), PRKCA (protein kinase C, alpha), COMT (catechol-.beta.-methyltransferase), S100B (S100 calcium binding protein B), EGR1 (early growth response 1), PRL (prolactin), IL15 (interleukin 15), DRD4 (dopamine receptor D4), CAMK2G (calcium/calmodulin-dependent protein kinase II gamma), SLC22A2 (solute carrier family 22 (organic cation transporter), member 2), CCL11 (chemokine (C-C motif) ligand 11), PGF (B321 placental growth factor), THPO (thrombopoietin), GP6 (glycoprotein VI (platelet)), TACR1 (tachykinin receptor 1), NTS (neurotensin), HNF1A (HNF1 homeobox A), SST (somatostatin), KCND1 (potassium voltage-gated channel, Shal-related subfamily, member 1), LOC646627 (phospholipase inhibitor), TBXAS1 (thromboxane A synthase 1 (platelet)), CYP2J2 (cytochrome P450, family 2, subfamily J, polypeptide 2), TBXA2R (thromboxane A2 receptor), ADH1C (alcohol dehydrogenase 1C (class I), gamma polypeptide), ALOX12 (arachidonate 12-lipoxygenase), AHSG (alpha-2-HS-glycoprotein), BHMT (betaine-homocysteine methyltransferase), GJA4 (gap junction protein, alpha 4, 37 kDa), SLC25A4 (solute carrier family 25 (mitochondrial carrier; adenine nucleotide translocator), member 4), ACLY (ATP citrate lyase), ALOX5AP (arachidonate 5-lipoxygenase-activating protein), NUMA1 (nuclear mitotic apparatus protein 1), CYP27B1 (cytochrome P450, family 27, subfamily B, polypeptide 1), CYSLTR2 (cysteinyl leukotriene receptor 2), SOD3 (superoxide dismutase 3, extracellular), LTC4S (leukotriene C4 synthase), UCN (urocortin), GHRL (ghrelin/obestatin prepropeptide), APOC2 (apolipoprotein C-II), CLEC4A (C-type lectin domain family 4, member A), KBTBD10 (kelch repeat and BTB (POZ) domain containing 10), TNC (tenascin C), TYMS (thymidylate synthetase), SHC1 (SHC (Src homology 2 domain containing) transforming protein 1), LRP1 (low density lipoprotein receptor-related protein 1), SOCS3 (suppressor of cytokine signaling 3), ADH1B (alcohol dehydrogenase 1B (class I), beta polypeptide), KLK3 (kallikrein-related peptidase 3), HSD11B1 (hydroxysteroid (11-beta) dehydrogenase 1), VKORC1 (vitamin K epoxide reductase complex, subunit 1), SERPINB2 (serpin peptidase inhibitor, clade B (ovalbumin), member 2), TNS1 (tensin 1), RNF19A (ring finger protein 19A), EPOR (erythropoietin receptor), ITGAM (integrin, alpha M (complement component 3 receptor 3 subunit)), PITX2 (paired-like homeodomain 2), MAPK7 (mitogen-activated protein kinase 7), FCGR3A (Fc fragment of IgG, low affinity 111a, receptor (CD16a)), LEPR (leptin receptor), ENG (endoglin), GPX1 (glutathione peroxidase 1), GOT2 (glutamic-oxaloacetic transaminase 2, mitochondrial (aspartate aminotransferase 2)), HRH1 (histamine receptor H1), NR112 (nuclear receptor subfamily 1, group I, member 2), CRH (corticotropin releasing hormone), HTR1A (5-hydroxytryptamine (serotonin) receptor 1A), VDAC1 (voltage-dependent anion channel 1), HPSE (heparanase), SFTPD (surfactant protein D), TAP2 (transporter 2, ATP-binding cassette, sub-family B (MDR/TAP)), RNF123 (ring finger protein 123), PTK2B (PTK2B protein tyrosine kinase 2 beta), NTRK2 (neurotrophic tyrosine kinase, receptor, type 2), IL6R (interleukin 6 receptor), ACHE (acetylcholinesterase (Yt blood group)), GLP1R (glucagon-like peptide 1 receptor), GHR (growth hormone receptor), GSR (glutathione reductase), NQO1 (NAD(P)H dehydrogenase, quinone 1), NR5A1 (nuclear receptor subfamily 5, group A, member 1), GJB2 (gap junction protein, beta 2, 26 kDa), SLC9A1 (solute carrier family 9 (sodium/hydrogen exchanger), member 1), MAOA (monoamine oxidase A), PCSK9 (proprotein convertase subtilisin/kexin type 9), FCGR2A (Fc fragment of IgG, low affinity IIa, receptor (CD32)), SERPINF1 (serpin peptidase inhibitor, clade F (alpha-2 antiplasmin, pigment epithelium derived factor), member 1), EDN3 (endothelin 3), DHFR (dihydrofolate reductase), GAS6 (growth arrest-specific 6), SMPD1 (sphingomyelin phosphodiesterase 1, acid lysosomal), UCP2 (uncoupling protein 2 (mitochondrial, proton carrier)), TFAP2A (transcription factor AP-2 alpha (activating enhancer binding protein 2 alpha)), C4BPA (complement component 4 binding protein, alpha), SERPINF2 (serpin peptidase inhibitor, clade F (alpha-2 antiplasmin, pigment epithelium derived factor), member 2), TYMP (thymidine phosphorylase), ALPP (alkaline phosphatase, placental (Regan isozyme)), CXCR2 (chemokine (C-X-C motif) receptor 2), SLC39A3 (solute carrier family 39 (zinc transporter), member 3), ABCG2 (ATP-binding cassette, sub-family G (WHITE), member 2), ADA (adenosine deaminase), JAK3 (Janus kinase 3), HSPA1A (heat shock 70 kDa protein 1A), FASN (fatty acid synthase), FGF1 (fibroblast growth factor 1 (acidic)), F11 (coagulation factor XI), ATP7A (ATPase, Cu++ transporting, alpha polypeptide), CR1 (complement component (3b/4b) receptor 1 (Knops blood group)), GFAP (glial fibrillary acidic protein), ROCK1 (Rho-associated, coiled-coil containing protein kinase 1), MECP2 (methyl CpG binding protein 2 (Rett syndrome)), MYLK (myosin light chain kinase), BCHE (butyrylcholinesterase), LIPE (lipase, hormone-sensitive), PRDX5 (peroxiredoxin 5), ADORA1 (adenosine A1 receptor), WRN (Werner syndrome, RecQ helicase-like), CXCR3 (chemokine (C-X-C motif) receptor 3), CD81 (CD81 molecule), SMAD7 (SMAD family member 7), LAMC2 (laminin, gamma 2), MAP3K5 (mitogen-activated protein kinase kinase kinase 5), CHGA (chromogranin A (parathyroid secretory protein 1)), IAPP (islet amyloid polypeptide), RHO (rhodopsin), ENPP1 (ectonucleotide pyrophosphatase/phosphodiesterase 1), PTHLH (parathyroid hormone-like hormone), NRG1 (neuregulin 1), VEGFC (vascular endothelial growth factor C), ENPEP (glutamyl aminopeptidase (aminopeptidase A)), CEBPB (CCAAT/enhancer binding protein (C/EBP), beta), NAGLU (N-acetylglucosaminidase, alpha-), F2RL3 (coagulation factor II (thrombin) receptor-like 3), CX3CL1 (chemokine (C-X3-C motif) ligand 1), BDKRB1 (bradykinin receptor B1), ADAMTS13 (ADAM metallopeptidase with thrombospondin type 1 motif, 13), ELANE (elastase, neutrophil expressed), ENPP2 (ectonucleotide pyrophosphatase/phosphodiesterase 2), CISH (cytokine inducible SH2-containing protein), GAST (gastrin), MYOC (myocilin, trabecular meshwork inducible glucocorticoid response), ATP1A2 (ATPase, Na+/K+ transporting, alpha 2 polypeptide), NF1 (neurofibromin 1), GJB1 (gap junction protein, beta 1, 32 kDa), MEF2A (myocyte enhancer factor 2A), VCL (vinculin), BMPR2 (bone morphogenetic protein receptor, type II (serine/threonine kinase)), TUBB (tubulin, beta), CDC42 (cell division cycle 42 (GTP binding protein, 25 kDa)), KRT18 (keratin 18), HSF1 (heat shock transcription factor 1), MYB (v-myb myeloblastosis viral oncogene homolog (avian)), PRKAA2 (protein kinase, AMP-activated, alpha 2 catalytic subunit), ROCK2 (Rho-associated, coiled-coil containing protein kinase 2), TFPI (tissue factor pathway inhibitor (lipoprotein-associated coagulation inhibitor)), PRKG1 (protein kinase, cGMP-dependent, type I), BMP2 (bone morphogenetic protein 2), CTNND1 (catenin (cadherin-associated protein), delta 1), CTH (cystathionase (cystathionine gamma-lyase)), CTSS (cathepsin S), VAV2 (vav 2 guanine nucleotide exchange factor), NPY2R (neuropeptide Y receptor Y2), IGFBP2 (insulin-like growth factor binding protein 2, 36 kDa), CD28 (CD28 molecule), GSTA1 (glutathione S-transferase alpha 1), PPIA (peptidylprolyl isomerase A (cyclophilin A)), APOH (apolipoprotein H (beta-2-glycoprotein I)), S100A8 (S100 calcium binding protein A8), IL11 (interleukin 11), ALOX15 (arachidonate 15-lipoxygenase), FBLN1 (fibulin 1), NR1H3 (nuclear receptor subfamily 1, group H, member 3), SCD (stearoyl-CoA desaturase (delta-9-desaturase)), GIP (gastric inhibitory polypeptide), CHGB (chromogranin B (secretogranin 1)), PRKCB (protein kinase C, beta), SRD5A1 (steroid-5-alpha-reductase, alpha polypeptide 1 (3-oxo-5 alpha-steroid delta 4-dehydrogenase alpha 1)), HSD11B2 (hydroxysteroid (11-beta) dehydrogenase 2), CALCRL (calcitonin receptor-like), GALNT2 (UDP-N-acetyl-alpha-D-galactosamine:polypeptide N-acetylgalactosaminyltransferase 2 (GalNAc-T2)), ANGPTL4 (angiopoietin-like 4), KCNN4 (potassium intermediate/small conductance calcium-activated channel, subfamily N, member 4), PIK3C2A (phosphoinositide-3-kinase, class 2, alpha polypeptide), HBEGF (heparin-binding EGF-like growth factor), CYP7A1 (cytochrome P450, family 7, subfamily A, polypeptide 1), HLA-DRB5 (major histocompatibility complex, class II, DR beta 5), BNIP3 (BCL2/adenovirus E1B 19 kDa interacting protein 3), GCKR (glucokinase (hexokinase 4) regulator), S100A12 (S100 calcium binding protein A12), PADI4 (peptidyl arginine deiminase, type IV), HSPA14 (heat shock 70 kDa protein 14), CXCR1 (chemokine (C-X-C motif) receptor 1), H19 (H19, imprinted maternally expressed transcript (non-protein coding)), KRTAP19-3 (keratin associated protein 19-3), IDDM2 (insulin-dependent diabetes mellitus 2), RAC2 (ras-related C3 botulinum toxin substrate 2 (rho family, small GTP binding protein Rac2)), RYR1 (ryanodine receptor 1 (skeletal)), CLOCK (clock homolog (mouse)), NGFR (nerve growth factor receptor (TNFR superfamily, member 16)), DBH (dopamine beta-hydroxylase (dopamine beta-monooxygenase)), CHRNA4 (cholinergic receptor, nicotinic, alpha 4), CACNA1C (calcium channel, voltage-dependent, L type, alpha 1C subunit), PRKAG2 (protein kinase, AMP-activated, gamma 2 non-catalytic subunit), CHAT (choline acetyltransferase), PTGDS (prostaglandin D2 synthase 21 kDa (brain)), NR1H2 (nuclear receptor sub-

family 1, group H, member 2), TEK (TEK tyrosine kinase, endothelial), VEGFB (vascular endothelial growth factor B), MEF2C (myocyte enhancer factor 2C), MAPKAPK2 (mitogen-activated protein kinase-activated protein kinase 2), TNFRSF11A (tumor necrosis factor receptor superfamily, member 11a, NFKB activator), HSPA9 (heat shock 70 kDa protein 9 (mortalin)), CYSLTR1 (cysteinyl leukotriene receptor 1), MAT1A (methionine adenosyltransferase I, alpha), OPRL1 (opiate receptor-like 1), IMPA1 (inositol(myo)-1(or 4)-monophosphatase 1), CLCN2 (chloride channel 2), DLD (dihydrolipoamide dehydrogenase), PSMA6 (proteasome (prosome, macropain) subunit, alpha type, 6), PSMB8 (proteasome (prosome, macropain) subunit, beta type, 8 (large multifunctional peptidase 7)), CHI3L1 (chitinase 3-like 1 (cartilage glycoprotein-39)), ALDH1B1 (aldehyde dehydrogenase 1 family, member B1), PARP2 (poly (ADP-ribose) polymerase 2), STAR (steroidogenic acute regulatory protein), LBP (lipopolysaccharide binding protein), ABCC6 (ATP-binding cassette, sub-family C(CFTR/MRP), member 6), RGS2 (regulator of G-protein signaling 2, 24 kDa), EFNB2 (ephrin-B2), GJB6 (gap junction protein, beta 6, 30 kDa), APOA2 (apolipoprotein A-II), AMPD1 (adenosine monophosphate deaminase 1), DYSF (dysferlin, limb girdle muscular dystrophy 2B (autosomal recessive)), FDFT1 (farnesyl-diphosphate farnesyltransferase 1), EDN2 (endothelin 2), CCR6 (chemokine (C-C motif) receptor 6), GJB3 (gap junction protein, beta 3, 31 kDa), IL1RL1 (interleukin 1 receptor-like 1), ENTPD1 (ectonucleoside triphosphate diphosphohydrolase 1), BBS4 (Bardet-Biedl syndrome 4), CELSR2 (cadherin, EGF LAG seven-pass G-type receptor 2 (flamingo homolog, Drosophila)), F11R (F11 receptor), RAPGEF3 (Rap guanine nucleotide exchange factor (GEF) 3), HYAL1 (hyaluronoglucosaminidase 1), ZNF259 (zinc finger protein 259), ATOX1 (ATX1 antioxidant protein 1 homolog (yeast)), ATF6 (activating transcription factor 6), KHK (ketohexokinase (fructokinase)), SAT1 (spermidine/spermine N1-acetyltransferase 1), GGH (gamma-glutamyl hydrolase (conjugase, folylpolygammaglutamyl hydrolase)), TIMP4 (TIMP metallopeptidase inhibitor 4), SLC4A4
(solute carrier family 4, sodium bicarbonate cotransporter, member 4), PDE2A (phosphodiesterase 2A, cGMP-stimulated), PDE3B (phosphodiesterase 3B, cGMP-inhibited), FADS1 (fatty acid desaturase 1), FADS2 (fatty acid desaturase 2), TMSB4X (thymosin beta 4, X-linked), TXNIP (thioredoxin interacting protein), LIMS1 (LIM and senescent cell antigen-like domains 1), RHOB (ras homolog gene family, member B), LY96 (lymphocyte antigen 96), FOXO1 (forkhead box O1), PNPLA2 (patatin-like phospholipase domain containing 2), TRH (thyrotropin-releasing hormone), GJC1 (gap junction protein, gamma 1, 45 kDa), SLC17A5 (solute carrier family 17 (anion/sugar transporter), member 5), FTO (fat mass and obesity associated), GJD2 (gap junction protein, delta 2, 36 kDa), PSRC1 (proline/serine-rich coiled-coil 1), CASP12 (caspase 12 (gene/pseudogene)), GPBAR1 (G protein-coupled bile acid receptor 1), PXK (PX domain containing serine/threonine kinase), IL33 (interleukin 33), TRIB1 (tribbles homolog 1 (Drosophila)), PBX4 (pre-B-cell leukemia homeobox 4), NUPR1 (nuclear protein, transcriptional regulator, 1), 15-Sep(15 kDa selenoprotein), CILP2 (cartilage intermediate layer protein 2), TERC (telomerase RNA component), GGT2 (gamma-glutamyltransferase 2), MT-CO1 (mitochondrially encoded cytochrome c oxidase I), and UOX (urate oxidase, pseudogene).

In an additional embodiment, the chromosomal sequence may further be selected from Pon1 (paraoxonase 1), LDLR (LDL receptor), ApoE (Apolipoprotein E), Apo B-100 (Apolipoprotein B-100), ApoA (Apolipoprotein(a)), ApoA1 (Apolipoprotein A1), CBS (Cystathione B-synthase), Glycoprotein IIb/IIb, MTHRF (5,10-methylenetetrahydrofolate reductase (NADPH)), and combinations thereof. In one iteration, the chromosomal sequences and proteins encoded by chromosomal sequences involved in cardiovascular disease may be chosen from Cacna1C, Sod1, Pten, Ppar(alpha), Apo E, Leptin, and combinations thereof.

## Kidneys

The present disclosure
also contemplates delivering the CRISPR-Cas system to the
kidney. Delivery strategies to induce cellular uptake of the therapeutic nucleic acid include physical force or vector systems such as viral-, lipid- or complex- based delivery, or nanocarriers. From the initial applications with less possible clinical relevance, when nucleic acids were addressed to renal cells with hydrodynamic high pressure injection systemically, a wide range of gene therapeutic viral and non-viral carriers have been applied already to target posttranscriptional events in different animal kidney disease models in vivo (Csaba Révész and Peter Hamar (2011). Delivery Methods to Target RNAs in the Kidney, Gene TherapyApplications, Prof. Chunsheng Kang (Ed.), ISBN: 978-953-307-541-9, InTech, Available from: http://www.intechopen.com/books/gene-therapy-applications/delivery-methods-to-target-rnas-in-the-kidney). Delivery methods to the kidney are summarized as follows:

| Delivery method | Carrier | Target RNA | Disease | Model | Functional assays | Author |
|---|---|---|---|---|---|---|
| Hydrodynamic/Lipid | TransIT In Vivo Gene Delivery System, DOTAP | p85$\alpha$ | Acute renal injury | Ischemia-reperfusion | Uptake, biodistribution | Larson et al., Surgery, (Aug 2007), Vol. 142, No. 2, pp. (262-269) |
| Hydrodynamic/Lipid | Lipofectamine 2000 | Fas | Acute renal injury | Ischemia-reperfusion | Blood urea nitrogen, Fas Immunohisto chemistry, apoptosis, histological scoring | Hamar et al., Proc Natl Acad Sci, (Oct 2004), Vol. 101, No. 41, pp. (14883-14888) |
| Hydrodynamic | n.a. | Apoptosis cascade elements | Acute renal injury | Ischemia-reperfusion | n.a. | Zheng et al., Am J Pathol, (Oct 2008), Vol. 173, No. 4, pp. (973-980) |
| Hydrodynamic | n.a. | Nuclear factor kappa-b (NFkB) | Acute renal injury | Ischemia-reperfusion | n.a. | Feng et al., Transplantation, (May 2009), Vol. 87, No. 9, pp. (1283-1289) |
| Hydrodynamic/Viral | Lipofectamine 2000 | Apoptosis antagonizing transcription factor (AATF) | Acute renal injury | Ischemia-reperfusion | Apoptosis, oxidative stress, caspase activation, membrane lipid peroxidation | Xie & Guo, Am Soc Nephrol, (Dec 2006), Vol. 17, No. 12, pp. (3336-3346) |
| Hydrodynamic | pBAsi mU6 Neo/TransIT-EE Hydrodynamic Delivery System | Gremlin | Diabetic nephropathy | Streptozotozin-induced diabetes | Proteinuria, serum creatinine, glomerular and tubular diameter, collagen type IV/BMP7 expression | Q.Zhang et al., PloS ONE, (Jul 2010), Vol. 5, No. 7, e1171709, pp. (1-13) |
| Viral/Lipid | pSUPER vector/Lipofectamine | TGF-$\beta$ type II receptor | Interstitial renal fibrosis | Unilateral urethral obstruction | $\alpha$-SMA expression, collagen content, | Kushibikia et al., J Controlled Release, (Jul 2005), Vol. 105, No. 3, pp. (318-331) |
| Viral | Adeno-associated virus-2 | Mineral corticoid receptor | Hypertension caused renal damage | Cold-induced hypertension | blood pressure, serum albumin, serum urea nitrogen, serum creatinine, kidney weight, urinary sodium | Wang et al., Gene Therapy, (Jul 2006), Vol. 13, No. 14, pp. (1097-1103) |
| Hydrodynamic/Viral | pU6 vector | Luciferase | n.a. | n.a. | uptake | Kobayashi et al., Journal of Pharmacology and Experimental Therapeutics, (Feb 2004), Vol. 308, No. 2, pp. (688-693) |

| Delivery method | Carrier | Target RNA | Disease | Model | Functional assays | Author |
|---|---|---|---|---|---|---|
| Lipid | Lipoproteins, albumin | apoB1, apoM | n.a. | n.a. | Uptake, binding affinity to lipoproteins and albumin | Wolfrum et al., Nature Biotechnology, (Sep 2007), Vol. 25, No. 10, pp. (1149-1157) |
| Lipid | Lipofectamine 2000 | p53 | Acute renal injury | Ischemic and cisplatin-induced acute injury | Histological scoring, apoptosis | Molitoris et al., J Am Soc Nephrol, (Aug 2009), Vol. 20, No. 8, pp. (1754-1764) |
| Lipid | DOTAP/DOPE, DOTAP/DOPE/ DOPE-PEG2000 | COX-2 | Breast adenocarcinoma | MDA-MB-231 breast cancer xenograft-bearing mouse | Cell viability, uptake | Mikhaylova et al., Cancer Gene Therapy, (Mar 2011), Vol. 16, No. 3, pp. (217-226) |
| Lipid | Cholesterol | 12/15-lipoxygenase | Diabetic nephropathy | Streptozot ocin-induced diabetes | Albuminuria, urinary creatinine, histology, type I and IV collagen, fibronectin, plasminogen activator inhibitor 1 | Yuan et al., Am J Physiol Renal Physiol, (Jun 2008), Vol. 295, pp. (F605-F617) |
| Lipid | Lipofectamine 2000 | Mitochondrial membrane 44 (TIM44) | Diabetic nephropathy | Streptozotocin-induced diabetes | Cell proliferation and apoptosis, histology, ROS, mitochondrial import of Mn-SOD and glutathione peroxidase, cellular membrane polarization | Y. Zhang et al., J Am Soc Nephrol, (Apr 2006), Vol. 17, No. 4, pp. (1090-1101) |
| Hydrodynamic/ Lipid | Proteolipo-some | RLIP76 | Renal carcinoma | Caki-2 kidney cancer xenograft-bearing mouse | uptake | Singhal et al., Cancer Res, (May 2009), Vol. 69, No. 10, pp. (4244-4251) |
| Polymer | PEGylated PEI | Luciferase pGL3 | n.a. | n.a. | Uptake, biodistribution, erythrocyte aggregation | Malek et al., Toxicology and Applied Pharmacology, (Apr 2009), Vol. 236, No. 1, pp. (97-108) |
| Polymer | PEGylated poly-L-lysine | MAPK1 | Lupus glomerulo-nephritis | Glomerul O-nephritis | Proteinuria, glomerulosclerosis, TGF-$\beta$, fibronectin, plasminogen activator inhibitor 1 | Shimizu et al., J Am Soc Nephrology, (Apr 2010), Vol. 21, No. 4, pp. (622-633) |

EP 3 011 032 B1

(continued)

| Delivery method | Carrier | Target RNA | Disease | Model | Functional assays | Author |
|---|---|---|---|---|---|---|
| Polymer/N ano particle | Hyaluronic acid/ Quantum dot/ PEI | VEGF | Kidney cancer/ melanoma | B16F1 melanoma tumor-bearing mouse | Biodistributio citotoxicity, tumor volume, endocytosis | Jiang et al., Molecular Pharmaceutics , (May-Jun 2009), Vol. 6, No. 3, pp. (727-737) |
| Polymer/Nano particle | PEGylated polycaprolactone nanofiber | GAPDH | n.a. | n.a. | cell viability, uptake | Cao et al, J Controlled Release, (Jun 2010), Vol. 144, No. 2, pp. (203-212) |
| Aptamer | Spiegelmer mNOX-E36 | CC chemokine ligand 2 | Glomerulo sclerosis | Uninephrecto-mized mouse | urinary albumin, urinary creatinine, histopathology, glomerular filtration rate, macrophage count, serum Ccl2, Mac-2+, Ki-67+ | Ninichuk et al., Am J Pathol, (Mar 2008), Vol. 172, No. 3 , pp. (628-637) |
| Aptamer | Aptamer NOX-F37 | vasopressin (AVP) | Congestive heart failure | n.a. | Binding affinity to D-AVP, Inhibition of AVP Signaling, Urine osmolality and sodium concentration, | Purschke et al., Proc Natl Acad Sci, (Mar 2006), Vol. 103, No. 13, pp. (5173-5178) |

Yuan et al. (Am J Physiol Renal Physiol 295: F605-F617, 2008) investigated whether in vivo delivery of small interfering RNAs (siRNAs) targeting the 12/15-lipoxygenase (12/15-LO) pathway of arachidonate acid metabolism can ameliorate renal injury and diabetic nephropathy (DN) in a streptozotocininjected mouse model of type 1 diabetes. To achieve greater in vivo access and siRNA expression in the kidney, Yuan et al. used double-stranded 12/15-LO siRNA oligonucleotides conjugated with cholesterol. About 400 µg of siRNA was injected subcutaneously into mice. The method of Yuang et al. may be applied to the CRISPR Cas system contemplating a 1-2 g subcutaneous injection of CRISPR Cas conjugated with cholesterol to a human for delivery to the kidneys.

Molitoris et al. (J Am Soc Nephrol 20: 1754-1764, 2009) exploited proximal tubule cells (PTCs), as the site of oligonucleotide reabsorption within the kidney to test the efficacy of siRNA targeted to p53, a pivotal protein in the apoptotic pathway, to prevent kidney injury. Naked synthetic siRNA to p53 injected intravenously 4 h after ischemic injury maximally protected both PTCs and kidney function. Molitoris et al.'s data indicates that rapid delivery of siRNA to proximal tubule cells follows intravenous administration. For dose-response analysis, rats were injected with doses of siP53, 0.33; 1, 3, or 5mg/kg, given at the same four time points, resulting in cumulative doses of 1.32; 4, 12, and 20 mg/kg, respectively. All siRNA doses tested produced a SCr reducing effect on day one with higher doses being effective over approximately five days compared with PBS-treated ischemic control rats. The 12 and 20 mg/kg cumulative doses provided the best protective effect. The method of Molitoris et al. may be applied to the CRISPR Cas system contemplating 12 and 20 mg/kg cumulative doses
to a human for delivery to the kidneys.

Thompson et al. (Nucleic Acid Therapeutics, Volume 22, Number 4, 2012) reports the toxicological and pharmacokinetic properties of the synthetic, small interfering RNA I5NP following intravenous administration in rodents and nonhuman primates. I5NP is designed to act via the RNA interference (RNAi) pathway to temporarily inhibit expression of the pro-apoptotic protein p53 and is being developed to protect cells from acute ischemia/reperfusion injuries such as acute kidney injury that can occur during major cardiac surgery and delayed graft function that can occur following renal transplantation. Doses of 800mg/kg I5NP in rodents, and 1,000 mg/kg I5NP in nonhuman primates, were required to elicit adverse effects, which in the monkey were isolated to direct effects on the blood that included a sub-clinical activation of complement and slightly increased clotting times. In the rat, no additional adverse effects were observed with a rat analogue of I5NP, indicating that the effects likely represent class effects of synthetic RNA duplexes rather than toxicity related to the intended pharmacologic activity of I5NP. Taken together, these data support clinical testing of intravenous administration of I5NP for the preservation of renal function following acute ischemia/reperfusion injury. The no observed adverse effect level (NOAEL) in the monkey was 500 mg/kg. No effects on cardiovascular, respiratory, and neurologic parameters were observed in monkeys following i.v. administration at dose levels up to 25 mg/kg. Therefore, a similar dosage may be contemplated for intravenous administration of CRISPR Cas to the kidneys of a human.

Shimizu et al. (J Am Soc Nephrol 21: 622-633, 2010) developed a system to target delivery of siRNAs to glomeruli via poly(ethylene glycol)-poly(L-lysine)-based vehicles. The siRNA/nanocarrier complex was approximately 10 to 20 nm in diameter, a size that would allow it to move across the fenestrated endothelium to access to the mesangium. After intraperitoneal injection of fluorescence-labeled siRNA/nanocarrier complexes, Shimizu et al. detected siRNAs in the blood circulation for a prolonged time. Repeated intraperitoneal administration of a mitogen-activated protein kinase 1 (MAPK1) siRNA/nanocarrier complex suppressed glomerular MAPK1 mRNA and protein expression in a mouse model of glomerulonephritis. For the investigation of siRNA accumulation, Cy5-labeled siRNAs complexed with PIC nanocarriers (0.5 ml, 5 nmol of siRNA content), naked Cy5-labeled siRNAs (0.5 ml, 5 nmol), or Cy5-labeled siRNAs encapsulated in HVJ-E (0.5 ml, 5 nmol of siRNA content) were administrated to BALB-c mice. The method of Shimizu et al. may be applied to the CRISPR Cas system
contemplating a dose of about of 10-20 µmol CRISPR Cas complexed with
nanocarriers in about 1-2 liters to a human for intraperitoneal administration and delivery to the kidneys.

## Lungs

The present disclosure
also contemplates delivering the CRISPR-Cas system to oneor
both lungs.

Although AAV-2-based vectors were originally proposed for CFTR delivery to CF airways, other serotypes such as AAV-1, AAV-5, AAV-6, and AAV-9 exhibit improved gene transfer efficiency in a variety of models of the lung epithelium (see, e.g., Li et al., Molecular Therapy, vol. 17 no. 12, 2067-2077 Dec 2009). AAV-1 was demonstrated to be ~100-fold more efficient than AAV-2 and AAV-5 at transducing human airway epithelial cells in vitro,5 although AAV-1 transduced murine tracheal airway epithelia in vivo with an efficiency equal to that of AAV-5. Other studies have shown that AAV-5 is 50-fold more efficient than AAV-2 at gene delivery to human airway epithelium (HAE) in vitro and significantly more efficient in the mouse lung airway epithelium in vivo. AAV-6 has also been shown to be more efficient than AAV-2 in human airway epithelial cells in vitro and murine airways in vivo.8 The more recent isolate, AAV-9, was shown to display greater gene transfer efficiency than AAV-5 in murine nasal and alveolar epithelia in vivo with gene expression detected for over 9 months suggesting AAV may enable long-term gene expression in vivo, a desirable property for a CFTR gene delivery vector. Furthermore, it was demonstrated that AAV-9 could be readministered to the murine lung with no loss of CFTR expression and minimal immune consequences. CF and non-CF HAE cultures may be inoculated on the apical surface with 100 μl of AAV vectors for hours (see, e.g., Li et al., Molecular Therapy, vol. 17 no. 12, 2067-2077 Dec 2009). The MOI may vary from $1 \times 10^3$ to $4 \times 10^5$ vector genomes/cell, depending on virus concentration and purposes of the experiments. The above cited vectors are contemplated for the delivery and/or administration.

Zamora et al. (Am J Respir Crit Care Med Vol 183. pp 531-538, 2011) reported an example of the application of an RNA interference therapeutic to the treatment of human infectious disease and also a randomized trial of an antiviral drug in respiratory syncytial virus (RSV)-infected lung transplant recipients. Zamora et al. performed a randomized, double-blind, placebocontrolled trial in LTX recipients with RSV respiratory tract infection. Patients were permitted to receive standard of care for RSV. Aerosolized ALN-RSV01 (0.6 mg/kg) or placebo was administered daily for 3 days. This study demonstrates that an RNAi therapeutic targeting RSV can be safely administered to LTX recipients with RSV infection. Three daily doses of ALN-RSV01 did not result in any exacerbation of respiratory tract symptoms or impairment of lung function and did not exhibit any systemic proinflammatory effects, such as induction of cytokines or CRP. Pharmacokinetics showed only low, transient systemic exposure after inhalation, consistent with preclinical animal data showing that ALN-RSV01, administered intravenously or by inhalation, is rapidly cleared from the circulation through exonucleasemediated digestion and renal excretion. The method of Zamora et al. may be applied to the CRISPR Cas system and an aerosolized CRISPR Cas, for example with a dosage of 0.6 mg/kg, may be contemplated for the present invention.

For an example of CFTRdelta508 chimeric guide RNA, see Example 22 which demonstrates gene transfer or gene delivery of a CRISPR-Cas system in airways of subject or a patient in need thereof, suffering from cystic fibrosis or from cystic fibrosis (CF) related symptoms, using adeno-associated virus (AAV) particles. In particular, they exemplify a repair strategy for Cystic Fibrosis delta F508 mutation. This type of strategy should apply across all organisms. With particular reference to CF, suitable patients may include: Human, non-primate human, canine, feline, bovine, equine and other domestic animals. In this instance, Applicants utilized a CRISPR-Cas system comprising a Cas9 enzyme to target deltaF508 or other CFTR-inducing mutations.

The treated subjects in this instance receive pharmaceutically effective amount of aerosolized AAV vector system per lung endobronchially delivered while spontaneously breathing. As such, aerosolized delivery is preferred for AAV delivery in general. An adenovirus or an AAV particle may be used for delivery. Suitable gene constructs, each operably linked to one or more regulatory sequences, may be cloned into the delivery vector. In this instance, the following constructs are provided as examples: Cbh or EF1a promoter for Cas9, U6 or H1 promoter for chimeric guide RNA),: A preferred arrangement is to use a CFTRdelta508 targeting chimeric guide, a repair template for deltaF508 mutation and a codon optimized Cas9 enzyme (preferred Cas9s are those with nuclease or nickase activity) with optionally one or more nuclear localization signal or sequence(s) (NLS(s)), e.g., two (2) NLSs. Constructs without NLS are also envisaged.

In order to identify the Cas9 target site, Applicants analyzed the human CFTR genomic locus and identified the Cas9 target site. Preferably, in general and in this CF case, the PAM may contain a NGG or a NNAGAAW motif.

Accordingly, in the case of CF, the present method comprises manipulation of a target sequence in a genomic locus of interest comprising

delivering a non-naturally occurring or engineered composition comprising a viral vector system comprising one or more viral vectors operably encoding a composition for expression thereof, wherein the composition comprises:

a non-naturally occurring or engineered composition comprising a vector system comprising one or more vectors comprising

I. a first regulatory element operably linked to a CRISPR-Cas system chimeric RNA (chiRNA) polynucleotide sequence, wherein the polynucleotide sequence comprises

(a) a guide sequence capable of hybridizing to the CF target sequence in a suitable mammalian cell,
(b) a tracr mate sequence, and
(c) a tracr sequence, and

II. a second regulatory element operably linked to an enzyme-coding sequence encoding a CRISPR enzyme comprising at least one or more nuclear localization sequences,

wherein (a), (b) and (c) are arranged in a 5' to 3' orientation,
wherein components I and II are located on the same or different vectors of the system,
wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridized to the target sequence, and (2) the tracr mate sequence that is hybridized to the tracr sequence. In respect of CF, preferred target DNA sequences comprise the CFTRdelta508 mutation. A preferred PAM is described above. A preferred CRISPR enzyme is any Cas (described herein, but particularly that described in Example 22).

Alternatives to CF include any genetic disorder and examples of these are well known. Another preferred method or use is for correcting defects in the EMP2A

and EMP2B genes that have been identified to be associated with Lafora disease.

In some embodiments, a "guide sequence" may be distinct from "guide RNA". A guide sequence may refer to an approx. 20bp sequence, within the guide RNA, that specifies the target site.

In some embodiments, the Cas9 is (or is derived from) SpCas9. In such embodiments, preferred mutations are at any or all or positions 10, 762, 840, 854, 863 and/or 986 of SpCas9 or corresponding positions in other Cas9s (which may be ascertained for instance by standard sequence comparison tools. In particular, any or all of the following mutations are preferred in SpCas9: D10A, E762A, H840A, N854A, N863A and/or D986A; as well as conservative substitution for any of the replacement amino acids is also envisaged. The same (or conservative substitutions of these mutations) at corresponding positions in other Cas9s are also preferred. Particularly preferred are D10 and H840 in SpCas9. However, in other Cas9s, residues corresponding to SpCas9 D10 and H840 are also preferred. These are advantageous as they provide nickase activity. Such mutations may be applied to all aspects of the present invention, not only treatment of CF.

Schwank et al. (Cell Stem Cell, 13:653-58, 2013) used CRISPR/Cas9 to correct a defect associated with cystic fibrosis in human stem cells. The team's target was the gene for an ion channel, cystic fibrosis transmembrane conductor receptor (CFTR). A deletion in CFTR causes the protein to misfold in cystic fibrosis patients. Using cultured intestinal stem cells developed from cell samples from two children with cystic fibrosis, Schwank et al. were able to correct the defect using CRISPR along with a donor plasmid containing the reparative sequence to be inserted. The researchers then grew the cells into intestinal "organoids," or miniature guts, and showed that they functioned normally. In this case, about half of clonal organoids underwent the proper genetic correction.

### Muscles

The present disclosure

also contemplates delivering the CRISPR-Cas system to

muscle(s).

Bortolanza et al. (Molecular Therapy vol. 19 no. 11, 2055-2064 Nov. 2011) shows that systemic delivery of RNA interference expression cassettes in the FRG1 mouse, after the onset of facioscapulohumeral muscular dystrophy (FSHD), led to a dose-dependent long-term FRG1 knockdown without signs of toxicity. Bortolanza et al. found that a single intravenous injection of $5 \times 10^{12}$ vg of rAAV6-sh1FRG1 rescues muscle histopathology and muscle function of FRG1 mice. In detail, 200 $\mu$l containing $2 \times 10^{12}$ or $5 \times 10^{12}$ vg of vector in physiological solution were injected into the tail vein using a 25-gauge Terumo syringe. The method of Bortolanza et al. may be applied to an AAV expressing CRISPR Cas and injected into humans at a dosage of about $2 \times 10^{15}$ or $2 \times 10^{16}$ vg of vector.

Dumonceaux et al. (Molecular Therapy vol. 18 no. 5, 881-887 May 2010) inhibit the myostatin pathway using the technique of RNA interference directed against the myostatin receptor AcvRIIb mRNA (sh-AcvRIIb). The restoration of a quasi-dystrophin was mediated by the vectorized U7 exon-skipping technique (U7-DYS). Adeno-associated vectors carrying either the sh-AcvrIIb construct alone, the U7-DYS construct alone, or a combination of both constructs were injected in the tibialis anterior (TA) muscle of dystrophic mdx mice. The injections were performed with $10^{11}$ AAV viral genomes. The method of Dumonceaux et al. may be applied to an AAV expressing CRISPR Cas and injected into humans, for example, at a dosage of about $10^{14}$ to about $10^{15}$ vg of vector.

Kinouchi et al. (Gene Therapy (2008) 15, 1126-1130) report the effectiveness of in vivo siRNA delivery into skeletal muscles of normal or diseased mice through nanoparticle formation of chemically unmodified siRNAs with atelocollagen (ATCOL). ATCOL-mediated local application of siRNA targeting myostatin, a negative regulator of skeletal muscle growth, in mouse skeletal muscles or intravenously, caused a marked increase in the muscle mass within a few weeks after application. These results imply that ATCOL-mediated application of siRNAs is a powerful tool for future therapeutic use for diseases including muscular atrophy. Mst-siRNAs (final concentration, 10 mM) were mixed with ATCOL (final concentration for local administration, 0.5%) (AteloGene, Kohken, Tokyo, Japan) according to the manufacturer's instructions. After anesthesia of mice (20-week-old male C57BL/6) by Nembutal (25 mg/kg, i.p.), the Mst-siRNA/ATCOL complex was injected into the masseter and biceps femoris muscles. The method of Kinouchi et al. may be applied to CRISPR Cas and injected into a human, for example, at a dosage of about 500 to 1000 ml of a 40 $\mu$M solution into the muscle.

Hagstrom et al. (Molecular Therapy Vol. 10, No. 2, August 2004) describe an intravascular, nonviral methodology that enables efficient and repeatable delivery of nucleic acids to muscle cells (myofibers) throughout the limb muscles of mammals. The procedure involves the injection of naked plasmid DNA or siRNA into a distal vein of a limb that is transiently isolated by a tourniquet or blood pressure cuff. Nucleic acid delivery to myofibers is facilitated by its rapid injection in sufficient volume to enable extravasation of the nucleic acid solution into muscle tissue. High levels of transgene expression in skeletal muscle were achieved in both small and large animals with minimal toxicity. Evidence of siRNA delivery to limb muscle was also obtained. For plasmid DNA intravenous injection into a rhesus monkey, a threeway stopcock was connected to two syringe pumps (Model PHD 2000; Harvard Instruments), each loaded with a single syringe. Five minutes after a papaverine injection, pDNA (15.5 to 25.7 mg in 40 -100 ml saline) was injected at a rate of 1.7 or 2.0 ml/s. This could be scaled up for plasmid DNA expressing CRISPR Cas with an injection of about 300 to 500 mg in 800 to 2000 ml saline for a human. For adenoviral vector injections into a rat, $2 \times 10^9$ infectious particles were injected in 3 ml of normal saline solution (NSS). This could be scaled up for an adenoviral vector expressing CRISPR Cas with

an injection of about $1 \times 10^{13}$ infectious particles were injected in 10 liters of NSS for a human. For siRNA, a rat was injected into the great saphenous vein with 12.5 $\mu$g of a siRNA and a primate was injected injected into the great saphenous vein with 750 $\mu$g of a siRNA. This could be scaled up for a CRISPR Cas, for example, with an injection of about 15 to about 50 mg into the great saphenous vein of a human.

### Skin

The present disclosure
also contemplates delivering the CRISPR-Cas system to the
skin.

Hickerson et al. (Molecular Therapy-Nucleic Acids (2013) 2, e129) relates to a motorized microneedle array skin delivery device for delivering self-delivery (sd)-siRNA to human and murine skin. The primary challenge to translating siRNA-based skin therapeutics to the clinic is the development of effective delivery systems. Substantial effort has been invested in a variety of skin delivery technologies with limited success. In a clinical study in which skin was treated with siRNA, the exquisite pain associated with the hypodermic needle injection precluded enrollment of additional patients in the trial, highlighting the need for improved, more "patient-friendly" (i.e., little or no pain) delivery approaches. Microneedles represent an efficient way to deliver large charged cargos including siRNAs across the primary barrier, the stratum corneum, and are generally regarded as less painful than conventional hypodermic needles. Motorized "stamp type" microneedle devices, including the motorized microneedle array (MMNA) device used by Hickerson et al., have been shown to be safe in hairless mice studies and cause little or no pain as evidenced by (i) widespread use in the cosmetic industry and (ii) limited testing in which nearly all volunteers found use of the device to be much less painful than a flushot, suggesting siRNA delivery using this device will result in much less pain than was experienced in the previous clinical trial using hypodermic needle injections. The MMNA device (marketed as Triple-M or Tri-M by Bomtech Electronic Co, Seoul, South Korea) was adapted for delivery of siRNA to mouse and human skin. sd-siRNA solution (up to 300 $\mu$l of 0.1 mg/ml RNA) was introduced into the chamber of the disposable Tri-M needle cartridge (Bomtech), which was set to a depth of 0.1 mm. For treating human skin, deidentified skin (obtained immediately following surgical procedures) was manually stretched and pinned to a cork platform before treatment. All intradermal injections were performed using an insulin syringe with a 28-gauge 0.5-inch needle. The MMNA device and method of Hickerson et al. could be used and/or adapted to deliver the CRISPR Cas, for example, at a dosage
of up to 300 $\mu$l of 0.1 mg/ml CRISPR Cas to the skin.

Leachman et al. (Molecular Therapy, vol. 18 no. 2, 442-446 Feb. 2010) relates to a phase Ib clinical trial for treatment of a rare skin disorder pachyonychia congenita (PC), an autosomal dominant syndrome that includes a disabling plantar keratoderma, utilizing the first short-interfering RNA (siRNA)-based therapeutic for skin. This siRNA, called TD101, specifically and potently targets the keratin 6a (K6a) N171K mutant mRNA without affecting wild-type K6a mRNA. The dose-escalation schedule is presented below:

| Week | Dose no. | Days | Volume (ml) | Concentration of TD101 (mg/ml) | Total dose TD101 (mg) |
|------|----------|------|-------------|--------------------------------|-----------------------|
| 1 | 1-2 | 1-7 | 0.1 | 1.0 | 0.10 |
| 2 | 3-4 | 8-14 | 0.25 | 1.0 | 0.25 |
| 3 | 5-6 | 15-21 | 0.50 | 1.0 | 0.50 |
| 4 | 7-8 | 22-28 | 1.0 | 1.0 | 1.0 |
| 5 | 9-10 | 29-35 | 1.5 | 1.0 | 1.5 |
| 6 | 11-12 | 36-42 | 2.0 | 1.0 | 2.0 |
| 7 | 13-14 | 43-49 | 2.0 | 1.5 | 3.0 |

(continued)

| Week | Dose no. | Days | Volume (ml) | Concentration of TD101 (mg/ml) | Total dose TD101 (mg) |
|---|---|---|---|---|---|
| 8 | 15-16 | 50-56 | 2.0 | 2.0 | 4.0 |
| 9 | 17-18 | 57-63 | 2.0 | 2.5 | 5.0 |
| 10 | 19-20 | 64-70 | 2.0 | 3.0 | 6.0 |
| 11 | 21-22 | 71-77 | 2.0 | 3.5 | 7.0 |
| 12 | 23-24 | 78-84 | 2.0 | 4.0 | 8.0 |
| 13 | 25-26 | 85-91 | 2.0 | 4.5 | 9.0 |
| 14 | 27-28 | 92-98 | 2.0 | 5.0 | 10.0 |
| 15 | 29-30 | 99-105 | 2.0 | 6.0 | 12.0 |
| 16 | 31-32 | 106-112 | 2.0 | 7.0 | 14.0 |
| 17 | 33 | 113-119 | 2.0 | 8.5 | 17.0 |

Initially, 0.1 ml of a 1.0 mg/ml solution of TD101 or vehicle alone (Dulbecco's phosphate-buffered saline without calcium or magnesium) was administered to symmetric calluses. Six rising dose-volumes were completed without an adverse reaction to the increases: 0.1, 0.25, 0.5, 1.0, 1.5, and 2.0 ml of a 1.0 mg/ml solution of TD101 solution per injection. As the highest planned volume (2.0 ml) was well tolerated, the concentration of TD101 was then increased each week from 1 mg/ml up to a final concentration of 8.5 mg/ml. Similar dosages are contemplated for the administration of a CRISPR Cas that specifically and potently targets the keratin 6a (K6a) N171K mutant mRNA.

Zheng et al. (PNAS, July 24, 2012, vol. 109, no. 30, 11975-11980) show that spherical nucleic acid nanoparticle conjugates (SNA-NCs), gold cores surrounded by a dense shell of highly oriented, covalently immobilized siRNA, freely penetrate almost 100% of keratinocytes in vitro, mouse skin, and human epidermis within hours after application. Zheng et al. demonstrated that a single application of 25 nM epidermal growth factor receptor (EGFR) SNA-NCs for 60 h demonstrate effective gene knockdown in human skin. A similar dosage may be contemplated for CRISPR Cas immobilized in SNA-NCs for administration to the skin.

## Hepatitis viruses

The present invention may also be applied to treat hepatitis B virus (HBV). However, the CRISPR Cas system must be adapted to avoid the shortcomings of RNAi, such as the risk of oversatring endogenous small RNA pathways, by for example, optimizing dose and sequence (see, e.g., Grimm et al., Nature vol. 441, 26 May 2006). For example, low doses, such as about 1-10 x $10^{14}$ particles per humane are contemplated.

In another embodiment, the CRISPR Cas system directed against HBV may be administered in liposomes, such as a stable nucleic-acid-lipid particle (SNALP) (see, e.g., Morrissey et al., Nature Biotechnology, Vol. 23, No. 8, August 2005). Daily intravenous injections of about 1, 3 or 5 mg/kg/day of CRISPR Cas targeted to HBV RNA in a SNALP are contemplated. The daily treatment may be over about three days and then weekly for about five weeks.

In another embodiment, the system of Chen et al. (Gene Therapy (2007) 14, 11-19) may be used/and or adapted for the CRISPR Cas system. Chen et al. use
a double-stranded adenoassociated virus 8-pseudotyped vector (dsAAV2/8) to deliver shRNA. A single administration of dsAAV2/8 vector (1 x $10^{12}$ vector genomes per mouse), carrying HBV-specific shRNA, effectively suppressed the steady level of HBV protein, mRNA and replicative DNA in liver of HBV transgenic mice, leading to up to 2-3 $\log_{10}$ decrease in HBV load in the circulation. Significant HBV suppression sustained for at least 120 days after vector administration. The therapeutic effect of shRNA was target sequence dependent and did not involve activation of interferon. For the present invention, a CRISPR Cas system directed to HBV may be cloned into an AAV vector, such as a dsAAV2/8 vector and administered to a human, for example, at a dosage of about 1 x $10^{15}$ vector genomes to about 1 x $10^{16}$ vector genomes per human.

In another embodiment, the method of Wooddell et al. (Molecular Therapy vol. 21 no. 5, 973-985 May 2013) may be used/and or adapted to the CRISPR Cas system.
Wooddell et al. show that simple coinjection of a hepatocyte-targeted, N-acetylgalactosamine-conjugated melittin-like peptide (NAG-MLP) with a liver-tropic cholesterol-conjugated siRNA (chol-siRNA) targeting coagulation factor VII (F7) results in efficient F7 knockdown in mice and nonhuman primates without changes in clinical chemistry or induction of cytokines. Using transient and transgenic mouse models of HBV infection, Wooddell et al. show that a single coinjection of NAG-MLP with potent chol-siRNAs targeting conserved HBV sequences resulted in multilog repression of viral RNA, proteins, and viral DNA with long duration of effect. Intraveinous coinjections, for example, of about 6 mg/kg of NAG-MLP and 6 mg/kg of HBV specific CRISPR Cas may be envisioned for the present invention. In the alternative, about 3 mg/kg of NAG-MLP and 3 mg/kg of HBV specific CRISPR Cas may be delivered on day one, followed by administration of about about 2-3 mg/kg of NAG-MLP and 2-3 mg/kg of HBV specific CRISPR Cas two weeks later.

The present invention may also be applied to treat hepatitis C virus (HCV). The methods of Roelvinki et al. (Molecular Therapy vol. 20 no. 9, 1737-1749 Sep 2012) may be applied to the CRISPR Cas system. For example, an AAV vector such as AAV8 may be a contemplated vector and for example a dosage of about $1.25 \times 10^{11}$ to $1.25 \times 10^{13}$ vector genomes per kilogram body weight (vg/kg) may be contemplated.

In yet another embodiment, CRISPR-Cas9-mediated genome editing can be used to correct a disease mutation and/or phenotype. That CRISPR-Cas9-mediated genome editing can be used to correct a disease mutation and/or phenotype in the liver and or hepatocytes is illustrated in the manuscript entitled "Genome editing with Cas9 in adult mice corrects a disease mutation and phenotype" by Hao Yin et al. published at Nature Biotechnology published online 30 March 2014; corrected online 31 March 2014, available at the website nature.com/doifinder/10.1038/nbt.2884, incorporated herein by reference in its entirety. The paper relates to CRISPR-Cas9-mediated correction of a Fah mutation in hepatocytes in a mouse model of the human disease hereditary tyrosinemia. It was shown that delivery of components of the CRISPR-Cas9 system by hydrodynamic injection resulted in initial expression of the wild-type Fah protein in ~1/250 liver cells. It was further shown that expansion of Fah-positive hepatocytes rescued the body weight loss phenotype.

It will be readily apparent that a host of other diseases can be treated in a similar fashion. Some examples of genetic diseases caused by mutations are provided herein, but many more are known. The above strategy can be applied to these diseases.

## Huntington's Disease (HD)

RNA interference (RNAi) offers therapeutic potential for this disorder by reducing the expression of *HTT,* the disease-causing gene of Huntington's disease (see, e.g., McBride et al., Molecular Therapy vol. 19 no. 12 Dec. 2011, pp. 2152-2162), therefore Applicant postulates that it may be used/and or adapted to the CRISPR-Cas system. The CRISPR-Cas system may be generated using an algorithm to reduce the off-targeting potential of antisense sequences. The CRISPR-Cas sequences may target either a sequence in exon 52 of mouse, rhesus or human huntingtin and expressed in a viral vector, such as AAV. Animals, including humans, may be injected with about three microinjections per hemisphere (six injections total): the first 1 mm rostral to the anterior commissure (12 $\mu$l) and the two remaining injections (12 $\mu$l and 10 $\mu$l, respectively) spaced 3 and 6 mm caudal to the first injection with 1e12 vg/ml of AAV at a rate of about 1 $\mu$l/minute, and the needle was left in place for an additional 5 minutes to allow the injectate to diffuse from the needle tip.

DiFiglia et al. (PNAS, October 23, 2007, vol. 104, no. 43, 17204-17209) observed that single administration into the adult striatum of an siRNA targeting Htt can silence mutant Htt, attenuate neuronal pathology, and delay the abnormal behavioral phenotype observed in a rapid-onset, viral transgenic mouse model of HD. DiFiglia injected mice intrastriatally with 2 $\mu$l of Cy3-labeled cc-siRNA-Htt or unconjugated siRNA-Htt at 10 $\mu$M. A similar dosage of CRISPR Cas targeted to Htt may be contemplated for humans in the present invention, for example, about 5-10 ml of 10 $\mu$M CRISPR Cas targeted to Htt may be injected intrastriatally.

In another example, Boudreau et al. (Molecular Therapy vol. 17 no. 6 june 2009) injects 5 $\mu$l of recombinant AAV serotype 2/1 vectors expressing htt-specific RNAi virus (at $4 \times 10^{12}$ viral genomes/ml) into the straiatum. A similar dosage of CRISPR Cas targeted to Htt may be contemplated for humans in the present invention, for example, about 10-20 ml of $4 \times 10^{12}$ viral genomes/ml) CRISPR Cas targeted to Htt may be injected intrastriatally.

In another example, a CRISPR Cas targetd to HTT may be administered continuously (see, e.g., Yu et al., Cell 150, 895-908, August 31, 2012). Yu et al. utilizes osmotic pumps delivering 0.25 ml/hr (Model 2004) to deliver 300 mg/day of ss-siRNA or phosphate-buffered saline (PBS) (Sigma Aldrich) for 28 days, and pumps designed to deliver 0.5 $\mu$l/hr (Model 2002) were used to deliver 75 mg/day of the positive control MOE ASO for 14 days. Pumps (Durect Corporation) were filled with ss-siRNA or MOE diluted in sterile PBS and then incubated at 37 C for 24 or 48 (Model 2004) hours prior to implantation. Mice were anesthetized with 2.5% isofluorane, and a midline incision was made at the base of the skull. Using stereotaxic guides, a cannula was implanted into the right lateral ventricle and secured with Loctite adhesive. A catheter attached to an Alzet osmotic mini pump was attached to the cannula, and the pump was placed subcutaneously in the midscapular area. The incision was closed with 5.0 nylon sutures. A similar dosage of CRISPR Cas targeted to Htt may be contemplated for humans in the present invention, for example, about 500 to 1000 g/day CRISPR Cas targeted to Htt may be administered.

In another example of continuous infusion, Stiles et al. (Experimental Neurology 233 (2012) 463-471) implanted an intraparenchymal catheter with a titanium needle tip into the right putamen. The catheter was connected to a SynchroMed® II Pump (Medtronic Neurological, Minneapolis, MN) subcutaneously implanted in the abdomen. After a 7 day infusion of phosphate buffered saline at 6 $\mu$L/day, pumps were re-filled with test article and programmed for continuous delivery for 7 days. About 2.3 to 11.52 mg/d of siRNA were infused at varying infusion rates of about 0.1 to 0.5 $\mu$L/min.

A similar dosage of CRISPR Cas targeted to Htt may be contemplated for humans in the present invention, for example, about 20 to 200 mg/day CRISPR Cas targeted to Htt may be administered.

In another example, the methods of US Patent Publication No. 20130253040 assigned to Sangamo may also be also be adapted from TALES to the CRISPR Cas system for treating Huntington's Disease.

## Nucleic acids, amino acids and proteins

The invention uses nucleic acids to bind target DNA sequences. This is advantageous as nucleic acids are much easier and cheaper to produce than proteins, and the specificity can be varied according to the length of the stretch where homology is sought. Complex 3-D positioning of multiple fingers, for example is not required.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. The term also encompasses nucleic-acid-like structures with synthetic backbones, see, e.g., WO 97/03211; WO 96/39154. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

"Complementarity" refers to the ability of a nucleic acid to form hydrogen bond(s) with another nucleic acid sequence by either traditional Watson-Crick base pairing or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y. Where reference is made to a polynucleotide sequence, then complementary or partially complementary sequences are also envisaged. These are preferably capable of hybridising to the reference sequence under highly stringent conditions. Generally, in order to maximize the hybridization rate, relatively low-stringency hybridization conditions are selected: about 20 to 25° C lower than the thermal melting point ($T_m$). The $T_m$ is the temperature at which 50% of specific target sequence hybridizes to a perfectly complementary probe in solution at a defined ionic strength and pH. Generally, in order to require at least about 85% nucleotide complementarity of hybridized sequences, highly stringent washing conditions are selected to be about 5 to 15° C lower than the $T_m$. In order to require at least about 70% nucleotide complementarity of hybridized sequences, moderately-stringent washing conditions are selected to be about 15 to 30° C lower than the $T_m$. Highly permissive (very low stringency) washing conditions may be as low as 50° C below the $T_m$, allowing a high level of mismatching between hybridized sequences. Those skilled in the art will recognize that other physical and chemical parameters in the hybridization and wash stages can also be altered to affect the outcome of a detectable hybridization signal from a specific level of homology between target and probe sequences. Preferred highly stringent conditions comprise incubation in 50% formamide, 5×SSC, and 1% SDS at 42° C, or incubation in 5×SSC and 1% SDS at 65° C, with wash in 0.2×SSC and 0.1% SDS at 65° C.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, the term "genomic locus" or "locus" (plural loci) is the specific location of a gene or DNA sequence on a chromosome. A "gene" refers to stretches of DNA or RNA that encode a polypeptide or an RNA chain that has functional role to play in an organism and hence is the molecular unit of heredity in living organisms. For the purpose of this invention it may be considered that genes include regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

As used herein, "expression of a genomic locus" or "gene expression" is the process by which information from a gene is used in the synthesis of a functional gene product. The products of gene expression are often proteins, but in non-protein coding genes such as rRNA genes or tRNA genes, the product is functional RNA. The process of gene expression is used by all known life - eukaryotes (including multicellular organisms), prokaryotes (bacteria and archaea) and viruses to generate functional products to survive. As used herein "expression" of a gene or nucleic acid encompasses not only cellular gene expression, but also the transcription and translation of nucleic acid(s) in cloning systems and in any other context. As used herein, "expression" also refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

As used herein, the term "domain" or "protein domain" refers to a part of a protein sequence that may exist and function independently of the rest of the protein chain.

As described in aspects of the invention, sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. In some preferred embodiments, the capping region of the dTALEs described herein have sequences that are at least 95% identical or share identity to the capping region amino acid sequences provided herein.

Sequence homologies may be generated by any of a number of computer programs known in the art, for example BLAST or FASTA, etc. A suitable computer program for carrying out such an alignment is the GCG Wisconsin Bestfit package (University of Wisconsin, U.S.A; Devereux et al., 1984, Nucleic Acids Research 12:387). Examples of other software than may perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

Percentage (%) sequence homology may be calculated over contiguous sequences, i.e., one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion may cause the following amino acid residues to be put out of alignment, thus potentially resulting in a large reduction in % homology when a global alignment is performed. Consequently, most sequence comparison methods are designed to produce optimal alignments that take into consideration possible insertions and deletions without unduly penalizing the overall homology or identity score. This is achieved by inserting "gaps" in the sequence alignment to try to maximize local homology or identity.

However, these more complex methods assign "gap penalties" to each gap that occurs in the alignment so that, for the same number of identical amino acids, a sequence alignment with as few gaps as possible - reflecting higher relatedness between the two compared sequences - may achieve a higher score than one with many gaps. "Affinity gap costs" are typically used that charge a relatively high cost for the existence of a gap and a smaller penalty for each subsequent residue in the gap. This is the most commonly used gap scoring system. High gap penalties may, of course, produce optimized alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore first requires the production of an optimal alignment, taking into consideration gap penalties. A suitable computer program for carrying out such an alignment is the GCG Wisconsin Bestfit package (Devereux et al., 1984 Nuc. Acids Research 12 p387). Examples of other software than may perform sequence comparisons include, but are not limited to, the BLAST package (see Ausubel et al., 1999 Short Protocols in Molecular Biology, 4th Ed. - Chapter 18), FASTA (Altschul et al., 1990 J. Mol. Biol. 403-410) and the GENEWORKS suite of comparison tools. Both BLAST and FASTA are available for offline and online searching (see Ausubel et al., 1999, Short Protocols in Molecular Biology, pages 7-58 to 7-60). However, for some applications, it is preferred to use the GCG Bestfit program. A new tool, called BLAST 2 Sequences is also available for comparing protein and nucleotide sequences (see FEMS Microbiol Lett. 1999 174(2): 247-50; FEMS Microbiol Lett. 1999 177(1): 187-8 and the website of the National Center for Biotechnology information at the website of the National Institutes for Health).

Although the final % homology may be measured in terms of identity, the alignment process itself is typically not based on an all-or-nothing pair comparison. Instead, a scaled similarity score matrix is generally used that assigns scores to each pair-wise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table, if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244). Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in amino acid properties (such as polarity, charge, solubility, hydrophobicity, hydrophilicity, and/or the amphipathic nature of the residues) and it is therefore useful to group amino acids together in functional groups. Amino acids may be grouped together based on the properties of their side chains alone. However, it is more useful to include mutation data as well. The sets of amino acids thus derived are likely to be conserved for structural reasons. These sets may be described in the form of a Venn diagram (Livingstone C.D. and Barton G.J. (1993) "Protein sequence alignments: a strategy for the hierarchical analysis of residue conservation" Comput. Appl. Biosci. 9: 745-756) (Taylor W.R. (1986) "The classification of amino acid conservation" J. Theor. Biol. 119; 205-218). Conservative substitutions may be made, for example according to the table below which describes a generally accepted Venn diagram grouping of amino acids.

| Set | | Sub-set | |
|---|---|---|---|
| Hydrophobic | F W Y H K M I L V A G C | Aromatic | F W Y H |
| | | Aliphatic | I L V |
| Polar | W Y H K R E D C S T N Q | Charged | H K R E D |
| | | Positively charged | H K R |
| | | Negatively charged | E D |
| Small | V C A G S P T N D | Tiny | A G S |

Embodiments of the disclosure

include sequences (both polynucleotide or

polypeptide) which may comprise homologous substitution (substitution and replacement are both used herein to mean the interchange of an existing amino acid residue or nucleotide, with an alternative residue or nucleotide) that may occur i.e., like-for-like substitution in the case of amino acids such as basic for basic, acidic for acidic, polar for polar, etc. Non-homologous substitution may also occur i.e., from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, which involves the presence of one or more amino acid residues in peptoid form, may be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The practice of the present invention employs, unless otherwise indicated, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

## Vectors

In one aspect, the disclosure

provides for vectors that are used in the engineering and

optimization of CRISPR-Cas systems.

A used herein, a "vector" is a tool that allows or facilitates the transfer of an entity from one environment to another. It is a replicon, such as a plasmid, phage, or cosmid, into which another DNA segment may be inserted so as to bring about the replication of the inserted segment. Generally, a vector is capable of replication when associated with the proper control elements. In general, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses (AAVs)). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors can comprise a nucleic acid in a

form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell). With regards to recombination and cloning methods, mention is made of U.S. patent application 10/815,730, published September 2, 2004 as US 2004-0171156 A1, the contents of which are herein incorporated by reference in their entirety.

Aspects of the disclosure

relate to vectors for chimeric RNA and Cas9. Bicistronic

expression vectors for chimeric RNA and Cas9 are preferred. In general and particularly in this embodiment Cas9 is preferably driven by the CBh promoter. The chimeric RNA may preferably be driven by a U6 promoter. Ideally the two are combined. The chimeric guide RNA typically consists of a 20bp guide sequence (Ns) and this may be joined to the tracr sequence (running from the first "U" of the lower strand to the end of the transcript). The tracr sequence may be truncated at various positions as indicated. The guide and tracr sequences are separated by the tracr-mate sequence, which may be GUUUUAGAGCUA (SEQ ID NO: 63). This may be followed by the loop sequence GAAA as shown. Both of these are preferred examples. Applicants have demonstrated Cas9-mediated indels at the human *EMX1* and *PVALB* loci by SURVEYOR assays. ChiRNAs are indicated by their "+n" designation, and crRNA refers to a hybrid RNA where guide and tracr sequences are expressed as separate transcripts. Throughout this application, chimeric RNA may also be called single guide, or synthetic guide RNA (sgRNA). The loop is preferably GAAA, but it is not limited to this sequence or indeed to being only 4bp in length. Indeed, preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG.

The term "regulatory element" is intended to include promoters, enhancers, internal ribosomal entry sites (IRES), and other expression control elements (e.g. transcription termination signals, such as polyadenylation signals and poly-U sequences). Such regulatory elements are described, for example, in Goeddel, GENE EXPRESSION TECHNOLOGY: METHODS IN ENZYMOLOGY 185, Academic Press, San Diego, Calif. (1990). Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest, such as muscle, neuron, bone, skin, blood, specific organs (e.g. liver, pancreas), or particular cell types (e.g. lymphocytes). Regulatory elements may also direct expression in a temporal-dependent manner, such as in a cell-cycle dependent or developmental stage-dependent manner, which may or may not also be tissue or cell-type specific. In some embodiments, a vector comprises one or more pol III promoter (e.g. 1, 2, 3, 4, 5, or more pol III promoters), one or more pol II promoters (e.g. 1, 2, 3, 4, 5, or more pol II promoters), one or more pol I promoters (e.g. 1, 2, 3, 4, 5, or more pol I promoters), or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1α promoter. Also encompassed by the term "regulatory element" are enhancer elements, such as WPRE; CMV enhancers; the R-U5' segment in LTR of HTLV-I (Mol. Cell. Biol., Vol. 8(1), p. 466-472, 1988); SV40 enhancer; and the intron sequence between exons 2 and 3 of rabbit β-globin (Proc. Natl. Acad. Sci. USA., Vol. 78(3), p. 1527-31, 1981). It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., clustered regularly interspersed short palindromic repeats (CRISPR) transcripts, proteins, enzymes, mutant forms thereof, fusion proteins thereof, etc.). With regards to regulatory sequences, mention is made of U.S. patent application 10/491,026. With regards to promoters, mention is made of PCT publication WO 2011/028929 and U.S. application 12/511,940.

Vectors can be designed for expression of CRISPR transcripts (e.g. nucleic acid transcripts, proteins, or enzymes) in prokaryotic or eukaryotic cells. For example, CRISPR transcripts can be expressed in bacterial cells such as Escherichia coli, insect cells (using baculovirus expression vectors), yeast cells, or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase.

Vectors may be introduced and propagated in a prokaryote or prokaryotic cell. In some embodiments, a prokaryote is used to amplify copies of a vector to be introduced into a eukaryotic cell or as an intermediate vector in the production of a vector to be introduced into a eukaryotic cell (e.g. amplifying a plasmid as part of a viral vector packaging system). In some embodiments, a prokaryote is used to amplify copies of a vector and express one or more nucleic acids, such as to provide a source of one or more proteins for delivery to a host cell or host organism. Expression of proteins in prokaryotes is most often carried out in Escherichia coli with vectors containing constitutive or inducible promoters directing the expression of either fusion or non-fusion proteins. Fusion vectors add a number of amino acids to a protein encoded therein, such as to the amino terminus of the recombinant protein. Such fusion vectors may serve one or more purposes, such as: (i) to increase expression of recombinant protein; (ii) to increase the solubility of the recombinant protein; and (iii) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Example fusion expression vectors

include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion E. coli expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

In some embodiments, a vector is a yeast expression vector. Examples of vectors for expression in yeast Saccharomyces cerivisae include pYepSec1 (Baldari, et al., 1987. EMBO J. 6: 229-234), pMFa (Kuijan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

In some embodiments, a vector drives protein expression in insect cells using baculovirus expression vectors. Baculovirus vectors available for expression of proteins in cultured insect cells (e.g., SF9 cells) include the pAc series (Smith, et al., 1983. Mol. Cell. Biol. 3: 2156-2165) and the pVL series (Lucklow and Summers, 1989. Virology 170: 31-39).

In some embodiments, a vector is capable of driving expression of one or more sequences in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are typically provided by one or more regulatory elements. For example, commonly used promoters are derived from polyoma, adenovirus 2, cytomegalovirus, simian virus 40, and others disclosed herein and known in the art. For other suitable expression systems for both prokaryotic and eukaryotic cells see, e.g., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In some embodiments, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Baneiji, et al., 1983. Cell 33: 729-740; Queen and Baltimore, 1983. Cell 33: 741-748), neuron-specific promoters (e.g., the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. Science 249: 374-379) and the $\alpha$-fetoprotein promoter (Campes and Tilghman, 1989. Genes Dev. 3: 537-546). With regards to these prokaryotic and eukaryotic vectors, mention is made of U.S. Patent 6,750,059. Other embodiments of the dsclosure may relate to the use of viral vectors, with regards to which mention is made of U.S. Patent application 13/092,085. Tissue-specific regulatory elements are known in the art and in this regard, mention is made of U.S. Patent 7,776,321.

**Regulatory elements**

In some embodiments, a regulatory element is operably linked to one or more elements of a CRISPR system so as to drive expression of the one or more elements of the CRISPR system. In general, CRISPRs (Clustered Regularly Interspaced Short Palindromic Repeats), also known as SPIDRs (SPacer Interspersed Direct Repeats), constitute a family of DNA loci that are usually specific to a particular bacterial species. The CRISPR locus comprises a distinct class of interspersed short sequence repeats (SSRs) that were recognized in E. coli (Ishino et al., J. Bacteriol., 169:5429-5433 [1987]; and Nakata et al., J. Bacteriol., 171:3553-3556 [1989]), and associated genes. Similar interspersed SSRs have been identified in Haloferax mediterranei, Streptococcus pyogenes, Anabaena, and Mycobacterium tuberculosis (See, Groenen et al., Mol. Microbiol., 10:1057-1065 [1993]; Hoe et al., Emerg. Infect. Dis., 5:254-263 [1999]; Masepohl et al., Biochim. Biophys. Acta 1307:26-30 [1996]; and Mojica et al., Mol. Microbiol., 17:85-93 [1995]). The CRISPR loci typically differ from other SSRs by the structure of the repeats, which have been termed short regularly spaced repeats (SRSRs) (Janssen et al.,OMICS J. Integ. Biol., 6:23-33 [2002]; and Mojica et al., Mol. Microbiol., 36:244-246 [2000]). In general, the repeats are short elements that occur in clusters that are regularly spaced by unique intervening sequences with a substantially constant length (Mojica et al., [2000], supra). Although the repeat sequences are highly conserved between strains, the number of interspersed repeats and the sequences of the spacer regions typically differ from strain to strain (van Embden et al., J. Bacteriol., 182:2393-2401 [2000]). CRISPR loci have been identified in more than 40 prokaryotes (See e.g., Jansen et al., Mol. Microbiol., 43:1565-1575 [2002]; and Mojica et al., [2005]) including, but not limited to Aeropyrum, Pyrobaculum, Sulfolobus, Archaeoglobus, Halocarcula, Methanobacterium, Methanococcus, Methanosarcina, Methanopyrus, Pyrococcus, Picrophilus, Thermoplasma, Corynebacterium, Mycobacterium, Streptomyces, Aquifex, Porphyromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myxococcus, Campylobacter, Wolinella, Acinetobacter, Erwinia, Escherichia, Legionella, Methylococcus, Pasteurella, Photobacterium, Salmonella, Xanthomonas, Yersinia, Treponema, and Thermotoga.

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In embodiments of the invention the terms guide sequence and guide RNA are used interchangeably. In some embodiments, one or more elements of a CRISPR system is derived from a type I, type II, or type III CRISPR system. In some embodiments, one or more elements of a CRISPR system is derived from a particular organism comprising an endogenous CRISPR system, such as Streptococcus pyogenes. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides. In some embodiments, a target sequence is located in the nucleus or cytoplasm of a cell.

In some embodiments, direct repeats may be identified *in silico* by searching for repetitive motifs that fulfill any or all of the following criteria:

1. found in a 2Kb window of genomic sequence flanking the type II CRISPR locus;
2. span from 20 to 50 bp; and
3. interspaced by 20 to 50 bp.

In some embodiments, 2 of these criteria may be used, for instance 1 and 2, 2 and 3, or 1 and 3. In some embodiments, all 3 criteria may be used.

In some embodiments, candidate tracrRNA may be subsequently predicted by sequences that fulfill any or all of the following criteria:

1. sequence homology to direct repeats (motif search in Geneious with up to 18-bp mismatches);
2. presence of a predicted Rho-independent transcriptional terminator in direction of transcription; and
3. stable hairpin secondary structure between tracrRNA and direct repeat.

In some embodiments, 2 of these criteria may be used, for instance 1 and 2, 2 and 3, or 1 and 3. In some embodiments, all 3 criteria may be used.

In some embodiments, chimeric synthetic guide RNAs (sgRNAs) designs may incorporate at least 12 bp of duplex structure between the direct repeat and tracrRNA.

In preferred embodiments of the invention, the CRISPR system is a type II CRISPR system and the Cas enzyme is Cas9, which catalyzes DNA cleavage. Enzymatic action by Cas9 derived from Streptococcus pyogenes or any closely related Cas9 generates double stranded breaks at target site sequences which hybridize to 20 nucleotides of the guide sequence and that have a protospacer-adjacent motif (PAM) sequence (examples include NGG/NRG or a PAM that can be determined as described herein) following the 20 nucleotides of the target sequence. CRISPR activity through Cas9 for site-specific DNA recognition and cleavage is defined by the guide sequence, the tracr sequence that hybridizes in part to the guide sequence and the PAM sequence. More aspects of the CRISPR system are described in Karginov and Hannon, TheCRISPR system: small RNA-guided defence in bacteria and archaea, Mole Cell 2010, January 15; 37(1): 7.

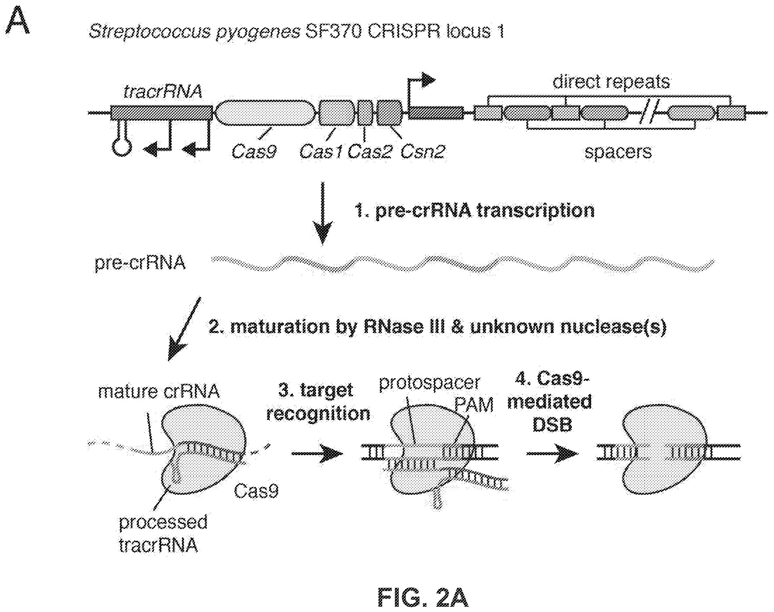

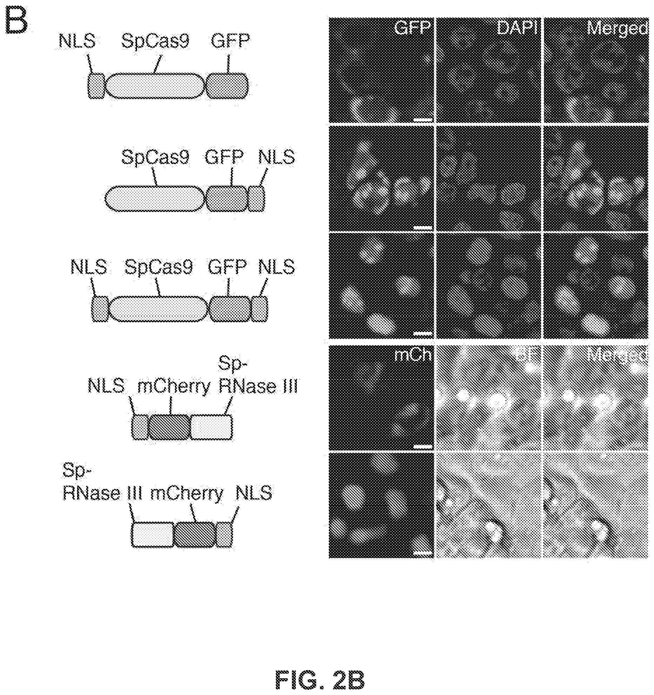

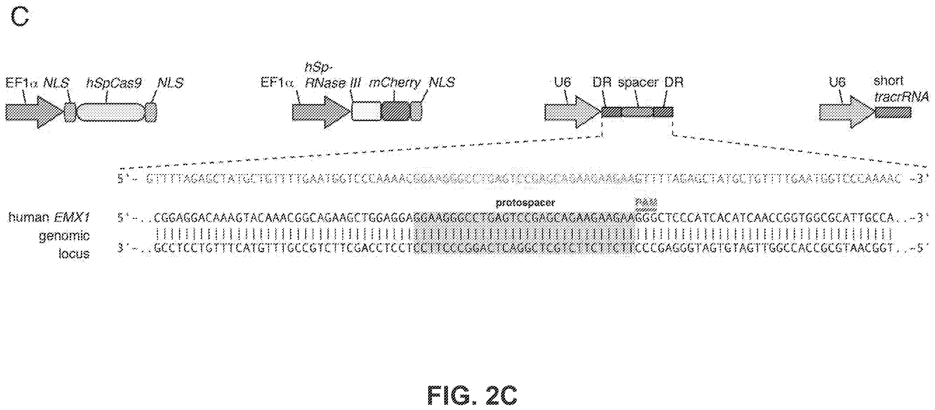

The type II CRISPR locus from Streptococcus pyogenes SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Fig. 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Fig. 2A). Fig. 2B demonstrates the nuclear localization of the codon optimized Cas9. To promote precise transcriptional initiation, the RNA polymerase III-based U6 promoter was selected to drive the expression of tracrRNA (Fig. 2C). Similarly, a U6 promoter-based construct was developed to express a pre-crRNA array consisting of a single spacer flanked by two direct repeats (DRs, also encompassed by the term "tracr-mate sequences"; Fig. 2C). The initial spacer was designed to target a 33-base-pair (bp) target site (30-bp protospacer plus a 3-bp CRISPR motif (PAM) sequence satisfying the NGG recognition motif of Cas9) in the human EMX1 locus (Fig. 2C), a key gene in the development of the cerebral cortex.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, the tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence. In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments, the CRISPR enzyme, guide sequence, tracr mate sequence, and tracr sequence are operably linked to and expressed from the same promoter.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments, a vector comprises a regulatory element operably linked to an enzyme-coding sequence encoding a CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In some embodiments, the unmodified CRISPR enzyme has DNA cleavage activity, such as Cas9. In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from S. pyogenes converts Cas9 from a nuclease that cleaves both strands to a nickase (cleaves a single strand). Other examples of mutations that render Cas9 a nickase include, without limitation, H840A, N854A, and N863A. As a further example, two or more catalytic domains of Cas9 (RuvC I, RuvC II, and RuvC III or the HNH domain) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form. Where the enzyme is not SpCas9, mutations may be made at any or all residues corresponding to positions 10, 762, 840, 854, 863 and/or 986 of SpCas9

(which may be ascertained for instance by standard sequence comparison tools . In particular, any or all of the following mutations are preferred in SpCas9: D10A, E762A, H840A, N854A, N863A and/or D986A; as well as conservative substitution for any of the replacement amino acids is also envisaged. The same (or conservative substitutions of these mutations) at corresponding positions in other Cas9s are also preferred. Particularly preferred are D10 and H840 in SpCas9 . However, in other Cas9s, residues corresponding to SpCas9 D10 and H840 are also preferred.

An aspartate-to-alanine substitution (D10A) in the RuvC I catalytic domain of SpCas9 was engineered to convert the nuclease into a nickase (SpCas9n) (see e.g. Sapranauskas et al., 2011, Nucleic Acis Research, 39: 9275; Gasiunas et al., 2012, Proc. Natl. Acad. Sci. USA, 109:E2579), such that nicked genomic DNA undergoes the high-fidelity homology-directed repair (HDR). Surveyor assay confirmed that SpCas9n does not generate indels at the EMX1 protospacer target. Co-expression of EMX1-targeting chimeric crRNA (having the tracrRNA component as well) with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting EMX1, hSpCas9 or hSpCas9n, as well as a HR template to introduce a pair of restriction sites (HindIII and NheI) near the protospacer.

Preferred orthologs are described herein. A Cas enzyme may be identified Cas9 as this can refer to the general class of enzymes that share homology to the biggest nuclease with multiple nuclease domains from the type II CRISPR system. Most preferably, the Cas9 enzyme is from, or is derived from, spCas9 or saCas9. By derived, Applicants mean that the derived enzyme is largely based, in the sense of having a high degree of sequence homology with, a wildtype enzyme, but that it has been mutated (modified) in some way as described herein.

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes.* However, it will be appreciated that this invention includes many more Cas9s from other species of microbes, such as SpCas9, SaCa9, St1Cas9 and so forth.

## Codon optimization

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species is known.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human mammal or primate. In some embodiments, processes for modifying the germ line genetic identity of human beings and/or processes for modifying the genetic identity of animals which are likely to cause them suffering without any substantial medical benefit to man or animal, and also animals resulting from such processes, may be excluded.

In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ (visited Jul. 9, 2002), and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

## Nuclear localization sequences (NLSs)

In some embodiments, a vector encodes a CRISPR enzyme comprising one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In a preferred embodiment of the invention, the CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Non-limiting examples of NLSs include an NLS sequence derived from: the NLS of the SV40 virus large T-antigen, having the amino acid sequence PKKKRKV (SEQ ID NO: 64); the NLS from nucleoplasmin (e.g. the nucleoplasmin bipartite NLS with the sequence KRPAATKKAGQAKKKK (SEQ ID NO: 65)); the c-myc NLS having the amino acid sequence PAAKRVKLD (SEQ ID NO: 66) or RQRRNELKRSP (SEQ ID NO: 67), the hRNPA1 M9 NLS having the sequence NQSSNFGPMKGGNFGGRSSGPYGGGGQYFAKPRNQGGY (SEQ ID NO: 68); the sequence RMRIZFKNKGKDTAELRRRRVEVSVELRKAKKDEQILKRRNV (SEQ ID NO: 69) of the IBB domain from importin-alpha; the sequences VSRKRPRP (SEQ ID NO: 70) and PPKKARED (SEQ ID NO: 71) of the myoma T protein; the sequence POPKKKPL(SEQ ID NO: 72) of human p53; the sequence SALIKKKKKMAP (SEQ ID NO: 73) of mouse c-abl IV; the sequences DRLRR (SEQ ID NO: 74) and PKQKKRK (SEQ ID NO: 75) of the influenza virus NS1; the sequence RKLKKKIKKL (SEQ ID NO: 76) of the Hepatitis virus delta antigen; the sequence REKKKFLKRR (SEQ ID NO: 77) of the mouse Mx1 protein; the sequence KRKGDEVDGVDEVAKKKSKK (SEQ ID NO: 78) of the human poly(ADP-ribose) polymerase; and the sequence RKCLQAGMNLEARKTKK (SEQ ID NO: 79) of the steroid hormone receptors (human) glucocorticoid.

In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

## Guide sequence

Particualrly preferred guides are in the range of 20-22 nucleotides (nt), as described herein and see Example 39. In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

A guide sequence may be selected to target any target sequence. In some embodiments, the target sequence is a sequence within a genome of a cell. Exemplary target sequences include those that are unique in the target genome. For example, for the *S. pyogenes* Cas9, a unique target sequence in a genome may include a Cas9 target site of the form MMMMMMMMNNNNNNNNNNNNNXGG where NNNNNNNNNNNNNXGG (N is A, G, T, or C; and X can be anything) has a single occurrence in the genome. A unique target sequence in a genome may include an *S. pyogenes* Cas9 target site of the form MMMMMMMMMNNNNNNNNNNNNXGG where NNNNNNNNNNNNXGG (N is A, G, T, or C; and X can be anything) has a single occurrence in the genome. For the *S. thermophilus* CRISPR1 Cas9, a unique target sequence in a genome may include a Cas9 target site of the form MMMMMMMMMNNNNNNNNNNNNNNNNXXAGAAW (SEQ ID NO: 80) where NNNNNNNNNNNNNXXAGAAW (SEQ ID NO: 81) (N is A, G, T, or C; X can be anything; and W is A or T) has a single occurrence in the genome. A unique target sequence in a genome may include an *S. thermophilus* CRISPR1 Cas9 target site of the form MMMMMMMMMNNNNNNNNNNNNNNNXXAGAAW (SEQ ID NO: 82 where NNNNNNNNNNNNNXX-AGAAW (SEQ ID NO: 83) (N is A, G, T, or C; X can be anything; and W is A or T) has a single occurrence in the genome. For the *S. pyogenes* Cas9, a unique target sequence in a genome may include a Cas9 target site of the form MMMMMMMMNNNNNNNNNNNNNXGGXG where NNNNNNNNNNNNNXGGXG (N is A, G, T, or C; and X can be anything) has a single occurrence in the genome. A unique target sequence in a genome may include an *S. pyogenes* Cas9 target site of the form MMMMMMMMMNNNNNNNNNNNNXGGXG where NNNNNNNNNNNNXGGXG (N is A, G, T, or C; and X can be anything) has a single occurrence in the genome. In each of these sequences "M" may be A, G, T, or C, and need not be considered in identifying a sequence as unique.

In some embodiments, a guide sequence is selected to reduce the degree secondary structure within the guide sequence. In some embodiments, about or less than about 75%, 50%, 40%, 30%, 25%, 20%, 15%, 10%, 5%, 1%, or fewer of the nucleotides of the guide sequence participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

### Tracr mate sequence

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In a hairpin structure the portion of the sequence 5' of the final "N" and upstream of the loop corresponds to the tracr mate sequence, and the portion of the sequence 3' of the loop corresponds to the tracr sequence Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator:        (1)        NNNNNNNNNNNNNNNNNNNNNNgttttgtactctcaagatttaGAAAtaaatcttgcagaagctacaaagataa ggcttcatgccgaaatcaacaccctgtcattttatggcagggtgttttcgttatttaaTTTTTT        (SEQ ID NO: 84);        (2)

NNNNNNNNNNNNNNNNNNNNNgtttttgtactctcaGAAAtgcagaagctacaaagataaggcttcatgccg aaatcaacaccctgtcattttatggcagggtgtttcgttatttaaTTTTTT (SEQ ID NO: 85); (3) NNNNNNNNNNNNNNNNNNNNNgtttttgtactctcaGAAAtgcagaagctacaaagataaggcttcatgccg aaatcaacaccctgtcattttatggcagggtgtTTTTTT (SEQ ID NO: 86); (4) NNNNNNNNNNNNNNNNNNNNNgttttagagctaGAAAtagcaagttaaaataaggctagtccgttatcaactt gaaaaagtggcaccgagtcggtgcTTTTTT (SEQ ID NO: 87); (5) NNNNNNNNNNNNNNNNNNNNNgttttagagctaGAAATAGcaagttaaaataaggctagtccgttatcaac ttgaaaaagtgTTTTTTT (SEQ ID NO: 88); and (6) NNNNNNNNNNNNNNNNNNNNNgttttagagctagAAATAGcaagttaaaataaggctagtccgttatcaTT TTTTTT (SEQ ID NO: 89). In some embodiments, sequences (1) to (3) are used in combination with Cas9 from *S. thermophilus* CRISPR1. In some embodiments, sequences (4) to (6) are used in combination with Cas9 from *S. pyogenes.* In some embodiments, the tracr sequence is a separate transcript from a transcript comprising the tracr mate sequence.

## Recombination template

In some embodiments, a recombination template is also provided. A recombination template may be a component of another vector as described herein, contained in a separate vector, or provided as a separate polynucleotide. In some embodiments, a recombination template is designed to serve as a template in homologous recombination, such as within or near a target sequence nicked or cleaved by a CRISPR enzyme as a part of a CRISPR complex. A template polynucleotide may be of any suitable length, such as about or more than about 10, 15, 20, 25, 50, 75, 100, 150, 200, 500, 1000, or more nucleotides in length. In some embodiments, the template polynucleotide is complementary to a portion of a polynucleotide comprising the target sequence. When optimally aligned, a template polynucleotide might overlap with one or more nucleotides of a target sequences (e.g. about or more than about 1, 5, 10, 15, 20, or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence. Additional discussion about the HDR pathway is herein provided; for instance, as to 'CRISPR Complexes.'

## Fusion protein

In some embodiments, the CRISPR enzyme is part of a fusion protein comprising one or more heterologous protein domains (e.g. about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP).
A CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions. Additional domains that may form part of a fusion protein comprising a CRISPR enzyme are described in US20110059502. In some embodiments, a tagged CRISPR enzyme is used to identify the location of a target sequence.

## Inducible system

In some embodiments, a CRISPR enzyme may form a component of an inducible system. The inducible nature of the system would allow for spatiotemporal control of gene editing or gene expression using a form of energy. The form of energy may include but is not limited to electromagnetic radiation, sound energy, chemical energy and thermal energy. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochrome). In one embodiment, the CRISPR enzyme may be a part of a Light Inducible Transcriptional Effector (LITE) to direct changes in transcriptional activity in a sequence-specific manner. The components of a light may include a CRISPR enzyme, a light-responsive cryptochrome heterodimer (e.g. from Arabidopsis thaliana), and a transcriptional activation/repression domain. Further examples of inducible DNA binding proteins and methods for their use are provided in US 61/736465 and US 61/721,283.

**Delivery**

In some aspects, the disclosure provides methods comprising delivering one or more polynucleotides, such as or one or more vectors as described herein, one or more transcripts thereof, and/or one or proteins transcribed therefrom, to a host cell. In some aspects, the disclosure further provides cells produced by such methods, and animals comprising or produced from such cells. In some embodiments, a CRISPR enzyme in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10): 1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds) (1995); and Yu et al., Gene Therapy 1:13-26 (1994).

Methods of non-viral delivery of nucleic acids include lipofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam™ and Lipofectin™). Cationic and neutral lipids that are suitable for efficient receptor-recognition lipofection of polynucleotides include those of Felgner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in the body and trafficking the viral payload to the nucleus. Viral vectors can be administered directly to patients (in vivo) or they can be used to treat cells in vitro, and the modified cells may optionally be administered to patients (ex vivo). Conventional viral based systems could include retroviral, lentivirus, adenoviral, adeno-associated and herpes simplex virus vectors for gene transfer.

Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommnerfelt et al., Virol. 176:58-59 (1990); Wilson et al., J. Virol. 63:2374-2378 (1989); Miller et al., J. Virol. 65:2220-2224 (1991); PCT/US94/05700).

In another embodiment, Cocal vesiculovirus envelope pseudotyped retroviral vector particles are contemplated (see, e.g., US Patent Publication No. 20120164118 assigned to the Fred Hutchinson Cancer Research Center). Cocal virus is in the Vesiculovirus genus, and is a causative agent of vesicular stomatitis in mammals. Cocal virus was originally isolated from mites in Trinidad (Jonkers et al., Am. J. Vet. Res. 25:236-242 (1964)), and infections have been identified in Trinidad, Brazil, and Argentina from insects, cattle, and horses. Many of the vesiculoviruses that infect mammals have been isolated from naturally infected arthropods, suggesting that they are vector-borne. Antibodies to vesiculoviruses are common among people living in rural areas where the viruses are endemic and laboratory-acquired; infections in humans usually result in influenza-like symptoms. The Cocal virus envelope glycoprotein shares 71.5% identity at the amino acid level with VSV-G Indiana, and phylogenetic comparison of the envelope gene of vesiculoviruses shows that Cocal virus is serologically distinct from, but most closely related to, VSV-G Indiana strains among the vesiculoviruses. Jonkers et al., Am. J. Vet. Res. 25:236-242 (1964) and Travassos da Rosa et al., Am. J. Tropical Med. & Hygiene 33:999-1006 (1984). The Cocal vesiculovirus envelope pseudotyped retroviral vector particles may include for example, lentiviral, alpharetroviral, betaretroviral, gammaretroviral, deltaretroviral, and epsilonretroviral vector particles that may comprise retroviral Gag, Pol, and/or one or more accessory protein(s) and a Cocal vesiculovirus envelope protein. Within certain aspects of these embodiments, the Gag, Pol, and accessory proteins are lentiviral and/or gammaretroviral.

In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system.

Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producer a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV.

Accordingly, AAV is considered an ideal candidate for use as a transducing vector. Such AAV transducing vectors can comprise sufficient cis-acting functions to replicate in the presence of adenovirus or herpesvirus or poxvirus (e.g., vaccinia virus) helper functions provided in trans. Recombinant AAV (rAAV) can be used to carry exogenous genes into cells of a variety of lineages. In these vectors, the AAV cap and/or rep genes are deleted from the viral genome and replaced with a DNA segment of choice. Current AAV vectors may accommodate up to 4300 bases of inserted DNA.

There are a number of ways to produce rAAV, and the disclosure provides rAAV and methods for preparing rAAV. For example, plasmid(s) containing or consisting essentially of the desired construct are transfected into AAV-infected cells. In addition, a second or additional helper plasmid is cotransfected into these cells to provide the AAV rep and/or cap genes which are obligatory for replication and packaging of the recombinant viral construct. Under these conditions, the rep and/or cap proteins of AAV act in trans to stimulate replication and packaging of the rAAV construct. Two to Three days after transfection, rAAV is harvested. Traditionally rAAV is harvested from the cells along with adenovirus. The contaminating adenovirus is then inactivated by heat treatment. In the instant invention, rAAV is advantageously harvested not from the cells themselves, but from cell supernatant. Accordingly, in an initial aspect the disclosure

provides for preparing rAAV, and in addition to the foregoing, rAAV can be prepared

by a method that comprises or consists essentially of: infecting susceptible cells with a rAAV containing exogenous DNA including DNA for expression, and helper virus (e.g., adenovirus, herpesvirus, poxvirus such as vaccinia virus) wherein the rAAV lacks functioning cap and/or rep (and the helper virus (e.g., adenovirus, herpesvirus, poxvirus such as vaccinia virus) provides the cap and/or rev function that the rAAV lacks); or infecting susceptible cells with a rAAV containing exogenous DNA including DNA for expression, wherein the recombinant lacks functioning cap and/or rep, and transfecting said cells with a plasmid supplying cap and/or rep function that the rAAV lacks; or infecting susceptible cells with a rAAV containing exogenous DNA including DNA for expression, wherein the recombinant lacks functioning cap and/or rep, wherein said cells supply cap and/or rep function that the recombinant lacks; or transfecting the susceptible cells with an AAV lacking functioning cap and/or rep and plasmids for inserting

exogenous DNA into the recombinant so that the exogenous DNA is expressed by the recombinant and for supplying rep and/or cap functions whereby transfection results in an rAAV containing the exogenous DNA including DNA for expression that lacks functioning cap and/or rep.

The rAAV can be from an AAV as herein described, and advantageously can be an rAAV1, rAAV2, AAV5 or rAAV having hybrid capsid which may comprise AAV1, AAV2, AAV5 or any combination thereof. One can select the AAV of the rAAV with regard to the cells to be targeted by the rAAV; e.g., one can select AAV serotypes 1, 2, 5 or a hybrid capsid AAV1, AAV2, AAV5 or any combination thereof for targeting brain or neuronal cells; and one can select AAV4 for targeting cardiac tissue.

In addition to 293 cells, other cells that can be used in the practice of the invention and the relative infectivity of certain AAV serotypes *in vitro* as to these cells (see Grimm, D. et al, J. Virol. 82: 5887-5911 (2008)) are as follows:

| Cell Line | AAV-1 | AAV-2 | AAV-3 | AAV-4 | AAV-5 | AAV-6 | AAV-8 | AAV-9 |
|---|---|---|---|---|---|---|---|---|
| Huh-7 | 13 | 100 | 2.5 | 0.0 | 0.1 | 10 | 0.7 | 0.0 |
| HEK293 | 25 | 100 | 2.5 | 0.1 | 0.1 | 5 | 0.7 | 0.1 |
| HeLa | 3 | 100 | 2.0 | 0.1 | 6.7 | 1 | 0.2 | 0.1 |
| HepG2 | 3 | 100 | 16.7 | 0.3 | 1.7 | 5 | 0.3 | ND |
| HepIA | 20 | 100 | 0.2 | 1.0 | 0.1 | 1 | 0.2 | 0.0 |
| 911 | 17 | 100 | 11 | 0.2 | 0.1 | 17 | 0.1 | ND |
| CHO | 100 | 100 | 14 | 1.4 | 333 | 50 | 10 | 1.0 |
| cos | 33 | 100 | 33 | 3.3 | 5.0 | 14 | 2.0 | 0.5 |
| MeWo | 10 | 100 | 20 | 0.3 | 6.7 | 10 | 1.0 | 0.2 |
| NIH3T3 | 10 | 100 | 2.9 | 2.9 | 0.3 | 10 | 0.3 | ND |
| A549 | 14 | 100 | 20 | ND | 0.5 | 10 | 0.5 | 0.1 |
| HT1180 | 20 | 100 | 10 | 0.1 | 0.3 | 33 | 0.5 | 0.1 |
| Monocytes | 1111 | 100 | ND | ND | 125 | 1429 | ND | ND |
| Immature DC | 2500 | 100 | ND | ND | 222 | 2857 | ND | ND |
| Mature DC | 2222 | 100 | ND | ND | 333 | 3333 | ND | ND |

The disclosure provides rAAV that contains or consists essentially of an exogenous nucleic acid molecule encoding a CRISPR (Clustered Regularly Interspaced Short Palindromic Repeats) system, e.g., a plurality of cassettes comprising or consisting a first cassette comprising or consisting essentially of a promoter, a nucleic acid molecule encoding a CRISPR-associated (Cas) protein (putative nuclease or helicase proteins), e.g., Cas9 and a terminator, and a two, or more, advantageously up to the packaging size limit of the vector, e.g., in total (including the first cassette) five, cassettes comprising or consisting essentially of a promoter, nucleic acid molecule encoding guide RNA (gRNA) and a terminator (e.g., each cassette schematically represented as Promoter-gRNAl-terminator, Promoter-gRNA2-terminator ... Promoter-gRNA(N)-terminator (where N is a number that can be inserted that is at an upper limit of the packaging size limit of the vector), or two or more individual rAAVs, each containing one or more than one cassette of a CRISPR system, e.g., a first rAAV containing the first cassette comprising or consisting essentially of a promoter, a nucleic acid molecule encoding Cas, e.g., Cas9 and a terminator, and a second rAAV containing a plurality, four, cassettes comprising or consisting essentially of a promoter, nucleic acid molecule encoding guide RNA (gRNA) and a terminator (e.g., each cassette schematically represented as Promoter-gRNAl-terminator, Promoter-gRNA2-terminator ... Promoter-gRNA(N)-terminator (where N is a number that can be inserted that is at an upper limit of the packaging size limit of the vector). As rAAV is a DNA virus, the nucleic acid molecules in the herein discussion concerning AAV or rAAV are advantageously DNA. The promoter is in some embodiments advantageously human Synapsin I promoter (hSyn).

Additional methods for the delivery of nucleic acids to cells are known to those skilled in the art. See, for example, US20030087817. See also the Kanasty.

In some embodiments, a host cell is transiently or non-transiently transfected with one or more vectors described herein. In some embodiments, a cell is transfected as it naturally occurs in a subject. In some embodiments, a cell that is transfected is taken from a subject. In some embodiments, the cell is derived from cells taken from a subject, such as a cell line. A wide variety of cell lines for tissue culture are known in the art. Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepalclc7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, a cell transfected with one or more vectors described herein is used to establish a new cell line comprising one or more vector-derived sequences. In some embodiments, a cell transiently transfected with the components of a CRISPR system as described herein (such as by transient transfection of one or more vectors, or transfection with RNA), and modified through the activity of a CRISPR complex, is used to establish a new cell line comprising cells containing the modification but lacking any other exogenous sequence. In some embodiments, cells transiently or non-transiently transfected with one or more vectors described herein, or cell lines derived from such cells are used in assessing one or more test compounds.

In some embodiments, one or more vectors described herein are used to produce a non-human transgenic animal or transgenic plant. In some embodiments, the transgenic animal is a mammal, such as a mouse, rat, or rabbit. Methods for producing transgenic animals and plants are known in the art, and generally begin with a method of cell transfection, such as described herein.

In another embodiment, a fluid delivery device with an array of needles (see, e.g., US Patent Publication No. 20110230839 assigned to the Fred Hutchinson Cancer Research Center) may be contemplated for delivery of CRISPR Cas to solid tissue. A device of US Patent Publication No. 20110230839 for delivery of a fluid to a solid tissue may comprise a plurality of needles arranged in an array; a plurality of reservoirs, each in fluid communication with a respective one of the plurality of needles; and a plurality of actuators operatively coupled to respective ones of the plurality of reservoirs and configured to control a fluid pressure within the reservoir. In certain embodiments each of the plurality of actuators may comprise one of a plurality of plungers, a first end of each of the plurality of plungers being received in a respective one of the plurality of reservoirs, and in certain further embodiments the plungers of the plurality of plungers are operatively coupled together at respective second ends so as to be simultaneously depressable. Certain still further embodiments may comprise a plunger driver configured to depress all of the plurality of plungers at a selectively variable rate. In other embodiments each of the plurality of actuators may comprise one of a plurality of fluid transmission lines having first and second ends, a first end of each of the plurality of fluid transmission lines being coupled to a respective one of the plurality of reservoirs. In other embodiments the device may comprise a fluid pressure source, and each of the plurality of actuators comprises a fluid coupling between the fluid pressure source and a respective one of the plurality of reservoirs. In further embodiments the fluid pressure source may comprise at least one of a compressor, a vacuum accumulator, a peristaltic pump, a master cylinder, a microfluidic pump, and a valve. In another embodiment, each of the plurality of needles may comprise a plurality of ports distributed along its length.

### Modifying a target

In one aspect, the disclosure provides for methods of modifying a target polynucleotide in a eukaryotic cell, which may be *in vivo, ex vivo* or *in vitro.* In some embodiments, the method comprises sampling or biopsying a cell or population of cells from a human or non-human animal, and modifying the cell or cells. Culturing may occur at any stage *ex vivo.* The cell or cells may even be re-introduced into the non-human animal. For re-introduced cells it is particularly preferred that the cells are stem cells.

In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In one aspect, the disclosure
provides a method of modifying expression of a
polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. Similar considerations and conditions apply as above for methods of modifying a target polynucleotide. In fact, these sampling, culturing and re-introduction options apply across the aspects of the present invention.

Indeed, in any aspect of the invention, the CRISPR complex may comprise a CRISPR enzyme complexed with a guide sequence hybridized to a target sequence, wherein said guide sequence may be linked to a tracr mate sequence which in turn may hybridize to a tracr sequence. Similar considerations and conditions apply as above for methods of modifying a target polynucleotide.

## Kits

In one aspect, the disclosure
provides kits containing any one or more of the elements
disclosed in the above methods and compositions. Elements may be provided individually or in combinations, and may be provided in any suitable container, such as a vial, a bottle, or a tube. In some embodiments, the kit includes instructions in one or more languages, for example in more than one language.

In some embodiments, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g. in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide. In some embodiments, the kit comprises one or more of the vectors and/or one or more of the polynucleotides described herein. The kit may advantageously allows to provide all elements of the systems of the invention.

## CRISPR complex

In one aspect, the disclosure
provides methods for using one or more elements of a
CRISPR system. The CRISPR complex provides an effective means for
modifying a target polynucleotide. The CRISPR complex of the invention has a wide variety of utility including modifying (e.g., deleting, inserting, translocating, inactivating, activating) a target polynucleotide in a multiplicity of cell types. As such the CRISPR complex has a broad spectrum of applications in, e.g., gene therapy, drug screening, disease diagnosis, and prognosis. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

In one embodiment, this disclosure provides a method of cleaving a target polynucleotide. The method comprises modifying a target polynucleotide using a CRISPR complex that binds to the target polynucleotide and effect cleavage of said target polynucleotide. Typically, the CRISPR complex of the disclosure,
when introduced into a cell, creates a break
(e.g., a single or a double strand break) in the genome sequence. For example, the method can be used to cleave a disease gene in a cell.

The break created by the CRISPR complex can be repaired by a repair processes such as the error prone non-homologous end joining (NHEJ) pathway or the high fidelity homology-directed repair (HDR) (Fig. 29). During these repair process, an exogenous polynucleotide template can be introduced into the genome sequence. In some methods, the HDR process is used modify genome sequence. For example, an exogenous polynucleotide template comprising a sequence to be integrated flanked by an upstream sequence and a downstream sequence is introduced into a cell. The upstream and downstream sequences share sequence similarity with either side of the site of integration in the chromosome.

Where desired, a donor polynucleotide can be DNA, e.g., a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer.

The exogenous polynucleotide template comprises a sequence to be integrated (e.g., a mutated gene). The sequence for integration may be a sequence endogenous or exogenous to the cell. Examples of a sequence to be integrated include polynucleotides encoding a protein or a non-coding RNA (e.g., a microRNA). Thus, the sequence for integration may be operably linked to an appropriate control sequence or sequences. Alternatively, the sequence to be integrated may provide a regulatory function.

The upstream and downstream sequences in the exogenous polynucleotide template are selected to promote recombination between the chromosomal sequence of interest and the donor polynucleotide. The upstream sequence is a nucleic acid sequence that shares sequence similarity with the genome sequence upstream of the targeted site for integration. Similarly, the downstream sequence is a nucleic acid sequence that shares sequence similarity with the chromosomal sequence downstream of the targeted site of integration. The upstream and downstream sequences in the exogenous polynucleotide template can have 75%, 80%, 85%, 90%, 95%, or 100% sequence identity with the targeted genome sequence. Preferably, the upstream and downstream sequences in the exogenous polynucleotide template have about 95%, 96%, 97%, 98%, 99%, or 100% sequence identity with the targeted genome sequence. In some methods, the upstream and downstream sequences in the exogenous polynucleotide template have about 99% or 100% sequence identity with the targeted genome sequence.

An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence have about 200 bp to about 2000 bp, about 600 bp to about 1000 bp, or more particularly about 700 bp to about 1000 bp.

In some methods, the exogenous polynucleotide template may further comprise a marker. Such a marker may make it easy to screen for targeted integrations. Examples of suitable markers include restriction sites, fluorescent proteins, or selectable markers. The exogenous polynucleotide template can be constructed using recombinant techniques (see, for example, Sambrook et al., 2001 and Ausubel et al., 1996).

In an exemplary method for modifying a target polynucleotide by integrating an exogenous polynucleotide template, a double stranded break is introduced into the genome sequence by the CRISPR complex, the break is repaired via homologous recombination by an exogenous polynucleotide template such that the template is integrated into the genome. The presence of a double-stranded break facilitates integration of the template.

In other embodiments, this disclosure provides a method of modifying expression of a polynucleotide in a eukaryotic cell. The method comprises increasing or decreasing expression of a target polynucleotide by using a CRISPR complex that binds to the polynucleotide.

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein is not produced.

In some methods, a control sequence can be inactivated such that it no longer functions as a control sequence. As used herein, "control sequence" refers to any nucleic acid sequence that effects the transcription, translation, or accessibility of a nucleic acid sequence. Examples of a control sequence include, a promoter, a transcription terminator, and an enhancer are control sequences.

The inactivated target sequence may include a deletion mutation (i.e., deletion of one or more nucleotides), an insertion mutation (i.e., insertion of one or more nucleotides), or a nonsense mutation (i.e., substitution of a single nucleotide for another nucleotide such that a stop codon is introduced). In some methods, the inactivation of a target sequence results in "knock-out" of the target sequence.

**Disease models**

A method of the invention may be used to create, an animal or cell that may be used as a disease model. As used herein, "disease" refers to a disease, disorder, or indication in a subject. For example, a method of the invention may be used to create an animal or cell that comprises a modification in one or more nucleic acid sequences associated with a disease, or a plant, animal or cell in which the expression of one or more nucleic acid sequences associated with a disease are altered. Such a nucleic acid sequence may encode a disease associated protein sequence or may be a disease associated control sequence. Accordingly, it is understood that in embodiments of the invention, a plant, subject, patient, organism or cell can be a non-human subject, patient, organism or cell. Thus, the disclosure provides a plant, animal or cell, produced by the present methods, or a progeny thereof. The progeny may be a clone of the produced or animal, or may result from sexual reproduction by crossing with other individuals of the same species to introgress further desirable traits into their offspring. The cell may be *in vivo* or *ex vivo* in the cases of multicellular organisms, particularly animals. In the instance where the cell is in cultured, a cell line may be established if appropriate culturing conditions are met and preferably if the cell is suitably adapted for this purpose (for instance a stem cell). Hence, cell lines are also envisaged.

In some methods, the disease model can be used to study the effects of mutations on the animal or cell and development and/or progression of the disease using measures commonly used in the study of the disease. Alternatively, such a disease model is useful for studying the effect of a pharmaceutically active compound on the disease.

In some methods, the disease model can be used to assess the efficacy of a potential gene therapy strategy. That is, a disease-associated gene or polynucleotide can be modified such that the disease development and/or progression is inhibited or reduced. In particular, the method comprises modifying a disease-associated gene or polynucleotide such that an altered protein is produced and, as a result, the animal or cell has an altered response. Accordingly, in some methods, a genetically modified animal may be compared with an animal predisposed to development of the disease such that the effect of the gene therapy event may be assessed.

In another embodiment, this disclosure

provides a method of developing a biologically

active agent that modulates a cell signaling event associated with a disease gene. The method comprises contacting a test compound with a cell comprising one or more vectors that drive expression of one or more of a CRISPR enzyme, a guide sequence linked to a tracr mate sequence, and a tracr sequence; and detecting a change in a readout that is indicative of a reduction or an augmentation of a cell signaling event associated with, e.g., a mutation in a disease gene contained in the cell.

A cell model , including an organoid or cell collection as described herein, or animal model can be constructed in combination with the method for screening a cellular function change. Such a model may be used to study the effects of a genome sequence

modified by the CRISPR complex on a cellular function of interest. For

example, a cellular function model may be used to study the effect of a modified genome sequence on intracellular signaling or extracellular signaling. Alternatively, a cellular function model may be used to study the effects of a modified genome sequence on sensory perception. In some such models, one or more genome sequences associated with a signaling biochemical pathway in the model are modified.

Several disease models have been specifically investigated. These include *de novo* autism risk genes CHD8, KATNAL2, and SCN2A; and the syndromic autism (Angelman Syndrome) gene UBE3A. These genes and resulting autism models are of course preferred, but serve to show the broad applicability of the invention across genes and corresponding models.

An altered expression of one or more genome sequences associated with a signaling biochemical pathway can be determined by assaying for a difference in the mRNA levels of the corresponding genes between the test model cell and a control cell, when they are contacted with a candidate agent. Alternatively, the differential expression of the sequences associated with a signaling biochemical pathway is determined by detecting a difference in the level of the encoded polypeptide or gene product.

To assay for an agent-induced alteration in the level of mRNA transcripts or corresponding polynucleotides, nucleic acid contained in a sample is first extracted according to standard methods in the art. For instance, mRNA can be isolated using various lytic enzymes or chemical solutions according to the procedures set forth in Sambrook et al. (1989), or extracted by nucleic-acid-binding resins following the accompanying instructions provided by the manufacturers. The mRNA contained in the extracted nucleic acid sample is then detected by amplification procedures or conventional hybridization assays (e.g. Northern blot analysis) according to methods widely known in the art or based on the methods exemplified herein.

For purpose of this invention, amplification means any method employing a primer and a polymerase capable of replicating a target sequence with reasonable fidelity. Amplification may be carried out by natural or recombinant DNA polymerases such as TaqGold™, T7 DNA polymerase, Klenow fragment of E.coli DNA polymerase, and reverse transcriptase. A preferred amplification method is PCR. In particular, the isolated RNA can be subjected to a reverse transcription assay that is coupled with a quantitative polymerase chain reaction (RT-PCR) in order to quantify the expression level of a sequence associated with a signaling biochemical pathway.

Detection of the gene expression level can be conducted in real time in an amplification assay. In one aspect, the amplified products can be directly visualized with fluorescent DNA-binding agents including but not limited to DNA intercalators and DNA groove binders. Because the amount of the intercalators incorporated into the double-stranded DNA molecules is typically proportional to the amount of the amplified DNA products, one can conveniently determine the amount of the amplified products by quantifying the fluorescence of the intercalated dye using conventional optical systems in the art. DNA-binding dye suitable for this application include SYBR green, SYBR blue, DAPI, propidium iodine, Hoeste, SYBR gold, ethidium bromide, acridines, proflavine, acridine orange, acriflavine, fluorcoumanin, ellipticine, daunomycin, chloroquine, distamycin D, chromomycin, homidium, mithramycin, ruthenium polypyridyls, anthramycin, and the like.

In another aspect, other fluorescent labels such as sequence specific probes can be employed in the amplification reaction to facilitate the detection and quantification of the amplified products. Probe-based quantitative amplification relies on the sequence-specific detection of a desired amplified product. It utilizes fluorescent, target-specific probes (e.g., TaqMan® probes) resulting in increased specificity and sensitivity. Methods for performing probe-based quantitative amplification are well established in the art and are taught in U.S. Patent No. 5,210,015.

In yet another aspect, conventional hybridization assays using hybridization probes that share sequence homology with sequences associated with a signaling biochemical pathway can be performed. Typically, probes are allowed to form stable complexes with the sequences associated with a signaling biochemical pathway contained within the biological sample derived from the test subject in a hybridization reaction. It will be appreciated by one of skill in the art that where antisense is used as the probe nucleic acid, the target polynucleotides provided in the sample are chosen to be complementary to sequences of the antisense nucleic acids. Conversely, where the nucleotide probe is a sense nucleic acid, the target polynucleotide is selected to be complementary to sequences of the sense nucleic acid.

Hybridization can be performed under conditions of various stringency. Suitable hybridization conditions for the practice of the present invention are such that the recognition interaction between the probe and sequences associated with a signaling biochemical pathway is both sufficiently specific and sufficiently stable. Conditions that increase the stringency of a hybridization reaction are widely known and published in the art. See, for example, (Sambrook, et al., (1989); Nonradioactive In Situ Hybridization Application Manual, Boehringer Mannheim, second edition). The hybridization assay can be formed using probes immobilized on any solid support, including but are not limited to nitrocellulose, glass, silicon, and a variety of gene arrays. A preferred hybridization assay is conducted on high-density gene chips as described in U.S. Patent No. 5,445,934.

For a convenient detection of the probe-target complexes formed during the hybridization assay, the nucleotide probes are conjugated to a detectable label. Detectable labels suitable for use in the present invention include any composition detectable by photochemical, biochemical, spectroscopic, immunochemical, electrical, optical or chemical means. A wide variety of appropriate detectable labels are known in the art, which include fluorescent or chemiluminescent labels, radioactive isotope labels, enzymatic or other ligands. In preferred embodiments, one will likely desire to employ a fluorescent label or an enzyme tag, such as digoxigenin, β-galactosidase, urease, alkaline phosphatase or peroxidase, avidin/biotin complex.

The detection methods used to detect or quantify the hybridization intensity will typically depend upon the label selected above. For example, radiolabels may be detected using photographic film or a phosphoimager. Fluorescent markers may be detected and quantified using a photodetector to detect emitted light. Enzymatic labels are typically detected by providing the enzyme with a substrate and measuring the reaction product produced by the action of the enzyme on the substrate; and finally colorimetric labels are detected by simply visualizing the colored label.

An agent-induced change in expression of sequences associated with a signaling biochemical pathway can also be determined by examining the corresponding gene products. Determining the protein level typically involves a) contacting the protein contained in a biological sample with an agent that specifically bind to a protein associated with a signaling biochemical pathway; and (b) identifying any agent:protein complex so formed. In one aspect of this embodiment, the agent that specifically binds a protein associated with a signaling biochemical pathway is an antibody, preferably a monoclonal antibody.

The reaction is performed by contacting the agent with a sample of the proteins associated with a signaling biochemical pathway derived from the test samples under conditions that will allow a complex to form between the agent and the proteins associated with a signaling biochemical pathway. The formation of the complex can be detected directly or indirectly according to standard procedures in the art. In the direct detection method, the agents are supplied with a detectable label and unreacted agents may be removed from the complex; the amount of remaining label thereby indicating the amount of complex formed. For such method, it is preferable to select labels that remain attached to the agents even during stringent washing conditions. It is preferable that the label does not interfere with the binding reaction. In the alternative, an indirect detection procedure may use an agent that contains a label introduced either chemically or enzymatically. A desirable label generally does not interfere with binding or the stability of the resulting agent:polypeptide complex. However, the label is typically designed to be accessible to an antibody for an effective binding and hence generating a detectable signal.

A wide variety of labels suitable for detecting protein levels are known in the art. Nonlimiting examples include radioisotopes, enzymes, colloidal metals, fluorescent compounds, bioluminescent compounds, and chemiluminescent compounds.

The amount of agent:polypeptide complexes formed during the binding reaction can be quantified by standard quantitative assays. As illustrated above, the formation of agent:polypeptide complex can be measured directly by the amount of label remained at the site of binding. In an alternative, the protein associated with a signaling biochemical pathway is tested for its ability to compete with a labeled analog for binding sites on the specific agent. In this competitive assay, the amount of label captured is inversely proportional to the amount of protein sequences associated with a signaling biochemical pathway present in a test sample.

A number of techniques for protein analysis based on the general principles outlined above are available in the art. They include but are not limited to radioimmunoassays, ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunofluorescent assays, and SDS-PAGE.

Antibodies that specifically recognize or bind to proteins associated with a signaling biochemical pathway are preferable for conducting the aforementioned protein analyses. Where desired, antibodies that recognize a specific type of post-translational modifications (e.g., signaling biochemical pathway inducible modifications) can be used. Post-translational modifications include but are not limited to glycosylation, lipidation, acetylation, and phosphorylation. These antibodies may be purchased from commercial vendors. For example, anti-phosphotyrosine antibodies that specifically recognize tyrosine-phosphorylated proteins are available from a number of vendors including Invitrogen and Perkin Elmer. Anti-phosphotyrosine antibodies are particularly useful in detecting proteins that are differentially phosphorylated on their tyrosine residues in response to an ER stress. Such proteins include but are not limited to eukaryotic translation initiation factor 2 alpha (eIF-2$\alpha$). Alternatively, these antibodies can be generated using conventional polyclonal or monoclonal antibody technologies by immunizing a host animal or an antibody-producing cell with a target protein that exhibits the desired post-translational modification.

In practicing the subject method, it may be desirable to discern the expression pattern of an protein associated with a signaling biochemical pathway in different bodily tissue, in different cell types, and/or in different subcellular structures. These studies can be performed with the use of tissue-specific, cell-specific or subcellular structure specific antibodies capable of binding to protein markers that are preferentially expressed in certain tissues, cell types, or subcellular structures.

An altered expression of a gene associated with a signaling biochemical pathway can also be determined by examining a change in activity of the gene product relative to a control cell. The assay for an agent-induced change in the activity of a protein associated with a signaling biochemical pathway will dependent on the biological activity and/or the signal transduction pathway that is under investigation. For example, where the protein is a kinase, a change in its ability to phosphorylate the downstream substrate(s) can be determined by a variety of assays known in the art. Representative assays include but are not limited to immunoblotting and immunoprecipitation with antibodies such as anti-phosphotyrosine antibodies that recognize phosphorylated proteins. In addition, kinase activity can be detected by high throughput chemiluminescent assays such as AlphaScreen™ (available from Perkin Elmer) and eTag™ assay (Chan-Hui, et al. (2003) Clinical Immunology 111: 162-174).

Where the protein associated with a signaling biochemical pathway is part of a signaling cascade leading to a fluctuation of intracellular pH condition, pH sensitive molecules such as fluorescent pH dyes can be used as the reporter molecules. In another example where the protein associated with a signaling biochemical pathway is an ion channel, fluctuations in membrane potential and/or intracellular ion concentration can be monitored. A number of commercial kits and high-throughput devices are particularly suited for a rapid and robust screening for modulators of ion channels. Representative instruments include FLIPRTM (Molecular Devices, Inc.) and VIPR (Aurora Biosciences). These instruments are capable of detecting reactions in over 1000 sample wells of a microplate simultaneously, and providing real-time measurement and functional data within a second or even a minisecond.

In practicing any of the methods disclosed herein, a suitable vector can be introduced to a cell or an embryo via one or more methods known in the art, including without limitation, microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In some methods, the vector is introduced into an embryo by microinjection. The vector or vectors may be microinjected into the nucleus or the cytoplasm of the embryo. In some methods, the vector or vectors may be introduced into a cell by nucleofection.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA). Without wishing to be bound by theory, it is believed that the target sequence should be associated with a PAM (protospacer adjacent motif); that is, a short sequence recognized by the CRISPR complex. The precise sequence and length requirements for the PAM differ depending on the CRISPR enzyme used, but PAMs are typically 2-5 base pair sequences adjacent the protospacer (that is, the target sequence) Examples of PAM sequences are given in the examples section below, and the skilled person will be able to identify further PAM sequences for use with a given CRISPR enzyme.

The target polynucleotide of a CRISPR complex may include a number of disease-associated genes and polynucleotides as well as signaling biochemical pathway-associated genes and polynucleotides as listed in US provisional patent applications 61/736,527 and 61/748,427 having Broad reference BI-2011/008/WSGR Docket No. 44063-701.101 and BI-2011/008/WSGR Docket No. 44063-701.102 respectively, both entitled SYSTEMS METHODS AND COMPOSITIONS FOR SEQUENCE MANIPULATION filed on December 12, 2012 and January 2, 2013, respectively, the contents of all of which are herein incorporated by reference in their entirety.

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

Examples of disease-associated genes and polynucleotides are listed in Tables A and B. Disease specific information is available from McKusick-Nathans Institute of Genetic Medicine, Johns Hopkins University (Baltimore, Md.) and National Center for Biotechnology Information, National Library of Medicine (Bethesda, Md.), available on the World Wide Web. Examples of signaling biochemical pathway-associated genes and polynucleotides are listed in Table C.

Mutations in these genes and pathways can result in production of improper proteins or proteins in improper amounts which affect function. Further examples of genes, diseases and proteins are hereby incorporated by reference from US Provisional application 61/736,527 filed December 12, 2012. Such genes, proteins and pathways may be the target polynucleotide of a CRISPR complex.

**Table A**

| DISEASE/DISORDERS | GENE(S) |
|---|---|
| Neoplasia | PTEN; ATM; ATR; EGFR; ERBB2; ERBB3; ERBB4; |
| | Notch1; Notch2; Notch3; Notch4; AKT; AKT2; AKT3; HIF; |
| | HIF1a; HIF3a; Met; HRG; Bcl2; PPAR alpha; PPAR |
| | gamma; WT1 (Wilms Tumor); FGF Receptor Family |
| | members (5 members: 1, 2, 3, 4, 5); CDKN2a; APC; RB |
| | (retinoblastoma); MEN1; VHL; BRCA1; BRCA2; AR |
| | (Androgen Receptor); TSG101; IGF; IGF Receptor; Igf1 (4 |
| | variants); Igf2 (3 variants); Igf 1 Receptor; Igf 2 Receptor; |
| | Bax; Bcl2; caspases family (9 members: |
| | 1, 2, 3, 4, 6, 7, 8, 9, 12); Kras; Apc |
| Age-related Macular | Abcr; Ccl2; Cc2; cp (ceruloplasmin); Timp3; cathepsinD; |
| Degeneration | Vldlr; Ccr2 |
| Schizophrenia | Neuregulin1 (Nrg1); Erb4 (receptor for Neuregulin); |

(continued)

| DISEASE/DISORDERS | GENE(S) |
|---|---|
| | Complexin1 (Cplx1); Tph1 Tryptophan hydroxylase; Tph2 Tryptophan hydroxylase 2; Neurexin 1; GSK3; GSK3a; GSK3b |
| Disorders | 5-HTT (Slc6a4); COMT; DRD (Drd1a); SLC6A3; DAOA; DTNBP1; Dao (Dao1) |
| Trinucleotide Repeat Disorders | HTT (Huntington's Dx); SBMA/SMAX1/AR (Kennedy's Dx); FXN/X25 (Friedrich's Ataxia); ATX3 (Machado-Joseph's Dx); ATXN1 and ATXN2 (spinocerebellar ataxias); DMPK (myotonic dystrophy); Atrophin-1 and Atn1 (DRPLA Dx); CBP (Creb-BP - global instability); VLDLR (Alzheimer's); Atxn7; AtxnlO |
| Fragile X Syndrome | FMR2; FXR1; FXR2; mGLUR5 |
| Secretase Related Disorders | APH-1 (alpha and beta); Presenilin (Psen1); nicastrin (Ncstn); PEN-2 |
| Others | Nos1; Parp1; Nat1; Nat2 |
| Prion - related disorders | Prp |
| ALS | SOD1; ALS2; STEX; FUS; TARDBP; VEGF (VEGF-a; VEGF-b; VEGF-c) |
| Drug addiction | Prkce (alcohol); Drd2; Drd4; ABAT (alcohol); GRIA2; Grm5; Grin1; Htr1b; Grin2a; Drd3; Pdyn; Gria1 (alcohol) |
| Autism | Mecp2; BZRAP1; MDGA2; Sema5A; Neurexin 1; Fragile X (FMR2 (AFF2); FXR1; FXR2; Mglur5) |
| Alzheimer's Disease | E1; CHIP; UCH; UBB; Tau; LRP; PICALM; Clusterin; PS1; SORL1; CR1; Vldlr; Uba1; Uba3; CHIP28 (Aqp1, Aquaporin 1); Uch11; Uchl3; APP |
| Inflammation | IL-10; IL-1 (IL-1a; IL-1b); IL-13; IL-17 (IL-17a (CTLA8); IL-17b; IL-17c; IL-17d; IL-17f); II-23; Cx3cr1; ptpn22; TNFa; NOD2/CARD15 for IBD; IL-6; IL-12 (IL-12a; IL-12b); CTLA4; Cx3cl1 |
| Parkinson's Disease | x-Synuclein; DJ-1; LRRK2; Parkin; PINK1 |

**Table B:**

| | |
|---|---|
| Blood and coagulation diseases and disorders | Anemia (CDAN1, CDA1, RPS19, DBA, PKLR, PK1, NT5C3, UMPH1, PSN1, RHAG, RH50A, NRAMP2, SPTB, ALAS2, ANH1, ASB, ABCB7, ABC7, ASAT); Bare lymphocyte syndrome (TAPBP, TPSN, TAP2, ABCB3, PSF2, RING11, MHC2TA, C2TA, RFX5, RFXAP, RFX5), Bleeding disorders (TBXA2R, P2RX1, P2X1); Factor H and factor H-like 1 (HF1, CFH, HUS); Factor V and factor VIII (MCFD2); Factor VII deficiency (F7); Factor X deficiency (F10); Factor XI deficiency (F11); Factor XII deficiency (F12, HAF); Factor XIIIA deficiency (F13A1, F13A); Factor XIIIB deficiency (F13B); Fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FANCE, FACE, FANCF, XRCC9, FANCG, BRIP1, BACH1, FANCJ, PHF9, FANCL, FANCM, KIAA1596); Hemophagocytic lymphohistiocytosis disorders (PRF1, HPLH2, UNC13D, MUNC13-4, HPLH3, HLH3, FHL3); Hemophilia A (F8, F8C, HEMA); Hemophilia B (F9, HEMB), Hemorrhagic disorders (PI, ATT, F5); Leukocyde deficiencies and disorders (ITGB2, CD18, LCAMB, LAD, EIF2B1, EIF2BA, EIF2B2, EIF2B3, EIF2B5, LVWM, CACH, CLE, EIF2B4); Sickle cell anemia (HBB); Thalassemia (HBA2, HBB, HBD, LCRB, HBA1). |
| | |
| Cell dysregulation and oncology diseases and disorders | B-cell non-Hodgkin lymphoma (BCL7A, BCL7); Leukemia (TAL1, TCL5, SCL, TAL2, FLT3, NBS1, NBS, ZNFN1A1, IK1, LYF1, HOXD4, HOX4B, BCR, CML, PHL, ALL, ARNT, KRAS2, RASK2, GMPS, AF10, ARHGEF12, LARG, KIAA0382, CALM, CLTH, CEBPA, CEBP, CHIC2, BTL, FLT3, KIT, PBT, LPP, NPM1, NUP214, D9S46E, CAN, CAIN, RUNX1, CBFA2, AML1, WHSC1L1, NSD3, FLT3, AF1Q, NPM1, NUMA1, ZNF145, PLZF, PML, MYL, STAT5B, AF10, CALM, CLTH, ARL11, ARLTS1, P2RX7, P2X7, BCR, CML, PHL, ALL, GRAF, NF1, VRNF, WSS, NFNS, PTPN11, PTP2C, SHP2, NS1, BCL2, CCND1, PRAD1, BCL1, TCRA, GATA1, GF1, ERYF1, NFE1, ABL1, NQO1, DIA4, NMOR1, NUP214, D9S46E, CAN, CAIN). |
| | |
| Inflammation and immune related diseases and disorders | AIDS (KIR3DL1, NKAT3, NKB1, AMB11, KIR3DS1, IFNG, CXCL12, SDF1); Autoimmune lymphoproliferative syndrome (TNFRSF6, APT1, FAS, CD95, ALPS1A); Combined immunodeficiency, (IL2RG, SCIDX1, SCIDX, IMD4); HIV-1 (CCL5, SCYA5, D17S136E, TCP228), HIV susceptibility or infection (IL10, CSIF, CMKBR2, CCR2, CMKBR5, CCCKR5 (CCR5)); Immunodeficiencies (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, TACI); Inflammation (IL-10, IL-1 (IL-1a, IL-1b), IL-13, IL-17 (IL-17a (CTLA8), IL-17b, IL-17c, IL-17d, IL-17f), Il-23, Cx3cr1, ptpn22, TNFa, NOD2/CARD15 for IBD, IL-6, IL-12 (IL-12a, IL-12b), CTLA4, Cx3cl1); Severe combined immunodeficiencies (SCIDs)(JAK3, JAKL, DCLRE1C, ARTEMIS, SCIDA, RAG1, RAG2, ADA, PTPRC, CD45, LCA, IL7R, CD3D, T3D, IL2RG, SCIDX1, SCIDX, IMD4). |
| | |
| Metabolic, liver, kidney and protein diseases and disorders | Amyloid neuropathy (TTR, PALB); Amyloidosis (APOA1, APP, AAA, CVAP, AD1, GSN, FGA, LYZ, TTR, PALB); Cirrhosis (KRT18, KRT8, CIRH1A, NAIC, TEX292, KIAA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD1, PKD2, PKD4, PKDTS, PRKCSH, G19P1, PCLD, SEC63). |
| | |

(continued)

| Muscular / Skeletal diseases and disorders | Becker muscular dystrophy (DMD, BMD, MYF6), Duchenne Muscular Dystrophy (DMD, BMD); Emery-Dreifuss muscular dystrophy (LMNA, LMN1, EMD2, FPLD, CMD1A, HGPS, LGMD1B, LMNA, LMN1, EMD2, FPLD, CMD1A); Facioscapulohumeral muscular dystrophy (FSHMD1A, FSHD1A); Muscular dystrophy (FKRP, MDC1C, LGMD2I, LAMA2, LAMM, LARGE, KIAA0609, MDC1D, FCMD, TTID, MYOT, CAPN3, CANP3, DYSF, LGMD2B, SGCG, LGMD2C, DMDA1, SCG3, SGCA, ADL, DAG2, LGMD2D, DMDA2, SGCB, LGMD2E, SGCD, SGD, LGMD2F, CMD1L, TCAP, LGMD2G, CMD1N, TRIM32, HT2A, LGMD2H, FKRP, MDC1C, LGMD2I, TTN, CMD1G, TMD, LGMD2J, POMT1, CAV3, LGMD1C, SEPN1, SELN, RSMD1, PLEC1, PLTN, EBS1); Osteopetrosis (LRP5, BMND1, LRP7, LR3, OPPG, VBCH2, CLCN7, CLC7, OPTA2, OSTM1, GL, TCIRG1, TIRC7, OC116, OPTB1); Muscular atrophy (VAPB, VAPC, ALS8, SMN1, SMA1, SMA2, SMA3, SMA4, BSCL2, SPG17, GARS, SMAD1, CMT2D, HEXB, IGHMBP2, SMUBP2, CATF1, SMARD1). |
|---|---|
| Neurological and neuronal diseases and disorders | ALS (SOD1, ALS2, STEX, FUS, TARDBP, VEGF (VEGF-a, VEGF-b, VEGF-c); Alzheimer disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, AD3); Autism (Mecp2, BZRAP1, MDGA2, Sema5A, Neurexin 1, GLO1, MECP2, RTT, PPMX, MRX16, MRX79, NLGN3, NLGN4, KIAA1260, AUTSX2); Fragile X Syndrome (FMR2, FXR1, FXR2, mGLUR5); Huntington's disease and disease like disorders (HD, IT15, PRNP, PRIP, JPH3, JP3, HDL2, TBP, SCA17); Parkinson disease (NR4A2, NURR1, NOT, TINUR, SNCAIP, TBP, SCA17, SNCA, NACP, PARK1, PARK4, DJ1, PARK7, LRRK2, PARK8, PINK1, PARK6, UCHL1, PARK5, SNCA, NACP, PARK1, PARK4, PRKN, PARK2, PDJ, DBH, NDUFV2); Rett syndrome (MECP2, RTT, PPMX, MRX16, MRX79, CDKL5, STK9, MECP2, RTT, PPMX, MRX16, MRX79, x-Synuclein, DJ-1); Schizophrenia (Neuregulin1 (Nrg1), Erb4 (receptor for Neuregulin), Complexin1 (Cplx1), Tph1 Tryptophan hydroxylase, Tph2, Tryptophan hydroxylase 2, Neurexin 1, GSK3, GSK3a, GSK3b, 5-HTT (Slc6a4), COMT, DRD (Drd1a), SLC6A3, DAOA, DTNBP1, Dao (Dao1)); Secretase Related Disorders (APH-1 (alpha and beta), Presenilin (Psen1), nicastrin, (Ncstn), PEN-2, Nos1, Parp1, Natl, Nat2); Trinucleotide Repeat Disorders (HTT (Huntington's Dx), SBMA/SMAX1/AR (Kennedy's Dx), FXN/X25 (Friedrich's Ataxia), ATX3 (Machado-Joseph's Dx), ATXN1 and ATXN2 (spinocerebellar ataxias), DMPK (myotonic dystrophy), Atrophin-1 and Atn1 (DRPLA Dx), CBP (Creb-BP - global instability), VLDLR (Alzheimer's), Atxn7, Atxn10). |
| Occular diseases and disorders | Age-related macular degeneration (Abcr, Ccl2, Cc2, cp (ceruloplasmin), Timp3, cathepsinD, Vldlr, Ccr2); Cataract (CRYAA, CRYA1, CRYBB2, CRYB2, PITX3, BFSP2, CP49, CP47, CRYAA, CRYA1, PAX6, AN2, MGDA, CRYBA1, CRYB1, CRYGC, CRYG3, CCL, LIM2, MP19, CRYGD, CRYG4, BFSP2, CP49, CP47, HSF4, CTM, HSF4, CTM, MIP, AQP0, CRYAB, CRYA2, CTPP2, CRYBB1, CRYGD, CRYG4, CRYBB2, CRYB2, CRYGC, CRYG3, CCL, CRYAA, CRYA1, GJA8, CX50, CAE1, GJA3, CX46, CZP3, CAE3, CCM1, CAM, KRIT1); Corneal clouding and dystrophy (APOA1, TGFBI, CSD2, CDGG1, CSD, BIGH3, CDG2, TACSTD2, TROP2, M1S1, VSX1, RINX, PPCD, PPD, KTCN, COL8A2, FECD, PPCD2, PIP5K3, CFD); Cornea plana congenital (KERA, CNA2); Glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, GLC3A); Leber congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, LCA3); Macular dystrophy (ELOVL4, ADMD, STGD2, STGD3, RDS, RP7, PRPH2, PRPH, AVMD, AOFMD, VMD2). |

**Table C:**

| CELLULAR FUNCTION | GENES |
|---|---|
| PI3K/AKT Signaling | PRKCE; ITGAM; ITGA5; IRAK1; PRKAA2; EIF2AK2; |
| | PTEN; EIF4E; PRKCZ; GRK6; MAPK1; TSC1; PLK1; |

(continued)

| CELLULAR FUNCTION | GENES |
|---|---|
| | AKT2; IKBKB; PIK3CA; CDK8; CDKN1B; NFKB2; BCL2; |
| | PIK3CB; PPP2R1A; MAPK8; BCL2L1; MAPK3; TSC2; |
| | ITGA1; KRAS; EIF4EBP1; RELA; PRKCD; NOS3; |
| | PRKAA1; MAPK9; CDK2; PPP2CA; PIM1; ITGB7; |
| | YWHAZ; ILK; TP53; RAF1; IKBKG; RELB; DYRK1A; |
| | CDKN1A; ITGB1; MAP2K2; JAK1; AKT1; JAK2; PIK3R1; |
| | CHUK; PDPK1; PPP2R5C; CTNNB1; MAP2K1; NFKB1; |
| | PAK3; ITGB3; CCND1; GSK3A; FRAP1; SFN; ITGA2; |
| | TTK; CSNK1A1; BRAF; GSK3B; AKT3; FOXO1; SGK; |
| | HSP90AA1; RPS6KB1 |
| ERK/MAPK Signaling | PRKCE; ITGAM; ITGA5; HSPB1; IRAK1; PRKAA2; |
| | EIF2AK2; RAC1; RAP1A; TLN1; EIF4E; ELK1; GRK6; |
| | MAPK1; RAC2; PLK1; AKT2; PIK3CA; CDK8; CREB1; |
| | PRKCI; PTK2; FOS; RPS6KA4; PIK3CB; PPP2R1A; |
| | PIK3C3; MAPK8; MAPK3; ITGA1; ETS1; KRAS; MYCN; |
| | EIF4EBP1; PPARG; PRKCD; PRKAA1; MAPK9; SRC; |
| | CDK2; PPP2CA; PIM1; PIK3C2A; ITGB7; YWHAZ; |
| | PPP1CC; KSR1; PXN; RAF1; FYN; DYRK1A; ITGB1; |
| | MAP2K2; PAK4; PIK3R1; STAT3; PPP2R5C; MAP2K1; |
| | PAK3; ITGB3; ESR1; ITGA2; MYC; TTK; CSNK1A1; |
| | CRKL; BRAF; ATF4; PRKCA; SRF; STAT1; SGK |
| Glucocorticoid Receptor | RAC1; TAF4B; EP300; SMAD2; TRAF6; PCAF; ELK1; |
| Signaling | MAPK1; SMAD3; AKT2; IKBKB; NCOR2; UBE2I; |
| | PIK3CA; CREB1; FOS; HSPA5; NFKB2; BCL2; |
| | MAP3K14; STAT5B; PIK3CB; PIK3C3; MAPK8; BCL2L1; |
| | MAPK3; TSC22D3; MAPK10; NRIP1; KRAS; MAPK13; |
| | RELA; STAT5A; MAPK9; NOS2A; PBX1; NR3C1; |
| | PIK3C2A; CDKN1C; TRAF2; SERPINE1; NCOA3; |
| | MAPK14; TNF; RAF1; IKBKG; MAP3K7; CREBBP; |
| | CDKN1A; MAP2K2; JAK1; IL8; NCOA2; AKT1; JAK2; |
| | PIK3R1; CHUK; STAT3; MAP2K1; NFKB1; TGFBR1; |
| | ESR1; SMAD4; CEBPB; JUN; AR; AKT3; CCL2; MMP1; |
| | STAT1; IL6; HSP90AA1 |
| Axonal Guidance Signaling | PRKCE; ITGAM; ROCK1; ITGA5; CXCR4; ADAM12; |
| | IGF1; RAC1; RAP1A; EIF4E; PRKCZ; NRP1; NTRK2; |
| | ARHGEF7; SMO; ROCK2; MAPK1; PGF; RAC2; |
| | PTPN11; GNAS; AKT2; PIK3CA; ERBB2; PRKCI; PTK2; |
| | CFL1; GNAQ; PIK3CB; CXCL12; PIK3C3; WNT11; |

(continued)

| CELLULAR FUNCTION | GENES |
|---|---|
| | PRKD1; GNB2L1; ABL1; MAPK3; ITGA1; KRAS; RHOA; |
| | PRKCD; PIK3C2A; ITGB7; GLI2; PXN; VASP; RAF1; |
| | FYN; ITGB1; MAP2K2; PAK4; ADAM17; AKT1; PIK3R1; |
| | GLI1; WNT5A; ADAM10; MAP2K1; PAK3; ITGB3; |
| | CDC42; VEGFA; ITGA2; EPHA8; CRKL; RND1; GSK3B; |
| | AKT3; PRKCA |
| Ephrin Receptor Signaling | PRKCE; ITGAM; ROCK1; ITGA5; CXCR4; IRAK1; |
| | PRKAA2; EIF2AK2; RAC1; RAP1A; GRK6; ROCK2; |
| | MAPK1; PGF; RAC2; PTPN11; GNAS; PLK1; AKT2; |
| | DOK1; CDK8; CREB1; PTK2; CFL1; GNAQ; MAP3K14; |
| | CXCL12; MAPK8; GNB2L1; ABL1; MAPK3; ITGA1; |
| | KRAS; RHOA; PRKCD; PRKAA1; MAPK9; SRC; CDK2; |
| | PIM1; ITGB7; PXN; RAF1; FYN; DYRK1A; ITGB1; |
| | MAP2K2; PAK4; AKT1; JAK2; STAT3; ADAM10; |
| | MAP2K1; PAK3; ITGB3; CDC42; VEGFA; ITGA2; |
| | EPHA8; TTK; CSNK1A1; CRKL; BRAF; PTPN13; ATF4; |
| | AKT3; SGK |
| Actin Cytoskeleton | ACTN4; PRKCE; ITGAM; ROCK1; ITGA5; IRAK1; |
| Signaling | PRKAA2; EIF2AK2; RAC1; INS; ARHGEF7; GRK6; |
| | ROCK2; MAPK1; RAC2; PLK1; AKT2; PIK3CA; CDK8; |
| | PTK2; CFL1; PIK3CB; MYH9; DIAPH1; PIK3C3; MAPK8; |
| | F2R; MAPK3; SLC9A1; ITGA1; KRAS; RHOA; PRKCD; |
| | PRKAA1; MAPK9; CDK2; PIM1; PIK3C2A; ITGB7; |
| | PPP1CC; PXN; VIL2; RAF1; GSN; DYRK1A; ITGB1; |
| | MAP2K2; PAK4; PIP5K1A; PIK3R1; MAP2K1; PAK3; |
| | ITGB3; CDC42; APC; ITGA2; TTK; CSNK1A1; CRKL; |
| | BRAF; VAV3; SGK |
| Huntington's Disease | PRKCE; IGF1; EP300; RCOR1; PRKCZ; HDAC4; TGM2; |
| Signaling | MAPK1; CAPNS1; AKT2; EGFR; NCOR2; SP1; CAPN2; |
| | PIK3CA; HDAC5; CREB1; PRKCI; HSPA5; REST; |
| | GNAQ; PIK3CB; PIK3C3; MAPK8; IGF1R; PRKD1; |
| | GNB2L1; BCL2L1; CAPN1; MAPK3; CASP8; HDAC2; |
| | HDAC7A; PRKCD; HDAC11; MAPK9; HDAC9; PIK3C2A; |
| | HDAC3; TP53; CASP9; CREBBP; AKT1; PIK3R1; |
| | PDPK1; CASP1; APAF1; FRAP1; CASP2; JUN; BAX; |
| | ATF4; AKT3; PRKCA; CLTC; SGK; HDAC6; CASP3 |
| Apoptosis Signaling | PRKCE; ROCK1; BID; IRAK1; PRKAA2; EIF2AK2; BAK1; |
| | BIRC4; GRK6; MAPK1; CAPNS1; PLK1; AKT2; IKBKB; |

(continued)

| CELLULAR FUNCTION | GENES |
|---|---|
| | CAPN2; CDK8; FAS; NFKB2; BCL2; MAP3K14; MAPK8; |
| | BCL2L1; CAPN1; MAPK3; CASP8; KRAS; RELA; |
| | PRKCD; PRKAA1; MAPK9; CDK2; PIM1; TP53; TNF; |
| | RAF1; IKBKG; RELB; CASP9; DYRK1A; MAP2K2; |
| | CHUK; APAF1; MAP2K1; NFKB1; PAK3; LMNA; CASP2; |
| | BIRC2; TTK; CSNK1A1; BRAF; BAX; PRKCA; SGK; |
| | CASP3; BIRC3; PARP1 |
| B Cell Receptor Signaling | RAC1; PTEN; LYN; ELK1; MAPK1; RAC2; PTPN11; |
| | AKT2; IKBKB; PIK3CA; CREB1; SYK; NFKB2; CAMK2A; |
| | MAP3K14; PIK3CB; PIK3C3; MAPK8; BCL2L1; ABL1; |
| | MAPK3; ETS1; KRAS; MAPK13; RELA; PTPN6; MAPK9; |
| | EGR1; PIK3C2A; BTK; MAPK14; RAF1; IKBKG; RELB; |
| | MAP3K7; MAP2K2; AKT1; PIK3R1; CHUK; MAP2K1; |
| | NFKB1; CDC42; GSK3A; FRAP1; BCL6; BCL10; JUN; |
| | GSK3B; ATF4; AKT3; VAV3; RPS6KB1 |
| Leukocyte Extravasation | ACTN4; CD44; PRKCE; ITGAM; ROCK1; CXCR4; CYBA; |
| Signaling | RAC1; RAP1A; PRKCZ; ROCK2; RAC2; PTPN11; |
| | MMP14; PIK3CA; PRKCI; PTK2; PIK3CB; CXCL12; |
| | PIK3C3; MAPK8; PRKD1; ABL1; MAPK10; CYBB; |
| | MAPK13; RHOA; PRKCD; MAPK9; SRC; PIK3C2A; BTK; |
| | MAPK14; NOX1; PXN; VIL2; VASP; ITGB1; MAP2K2; |
| | CTNND1; PIK3R1; CTNNB1; CLDN1; CDC42; F11R; ITK; |
| | CRKL; VAV3; CTTN; PRKCA; MMP1; MMP9 |
| Integrin Signaling | ACTN4; ITGAM; ROCK1; ITGA5; RAC1; PTEN; RAP1A; |
| | TLN1; ARHGEF7; MAPK1; RAC2; CAPNS1; AKT2; |
| | CAPN2; PIK3CA; PTK2; PIK3CB; PIK3C3; MAPK8; |
| | CAV1; CAPN1; ABL1; MAPK3; ITGA1; KRAS; RHOA; |
| | SRC; PIK3C2A; ITGB7; PPP1CC; ILK; PXN; VASP; |
| | RAF1; FYN; ITGB1; MAP2K2; PAK4; AKT1; PIK3R1; |
| | TNK2; MAP2K1; PAK3; ITGB3; CDC42; RND3; ITGA2; |
| | CRKL; BRAF; GSK3B; AKT3 |
| Acute Phase Response | IRAK1; SOD2; MYD88; TRAF6; ELK1; MAPK1; PTPN11; |
| Signaling | AKT2; IKBKB; PIK3CA; FOS; NFKB2; MAP3K14; |
| | PIK3CB; MAPK8; RIPK1; MAPK3; IL6ST; KRAS; |
| | MAPK13; IL6R; RELA; SOCS1; MAPK9; FTL; NR3C1; |
| | TRAF2; SERPINE1; MAPK14; TNF; RAF1; PDK1; |
| | IKBKG; RELB; MAP3K7; MAP2K2; AKT1; JAK2; PIK3R1; |
| | CHUK; STAT3; MAP2K1; NFKB1; FRAP1; CEBPB; JUN; |

(continued)

| CELLULAR FUNCTION | GENES |
|---|---|
| | AKT3; IL1R1; IL6 |
| PTEN Signaling | ITGAM; ITGA5; RAC1; PTEN; PRKCZ; BCL2L11; |
| | MAPK1; RAC2; AKT2; EGFR; IKBKB; CBL; PIK3CA; |
| | CDKN1B; PTK2; NFKB2; BCL2; PIK3CB; BCL2L1; |
| | MAPK3; ITGA1; KRAS; ITGB7; ILK; PDGFRB; INSR; |
| | RAF1; IKBKG; CASP9; CDKN1A; ITGB1; MAP2K2; |
| | AKT1; PIK3R1; CHUK; PDGFRA; PDPK1; MAP2K1; |
| | NFKB1; ITGB3; CDC42; CCND1; GSK3A; ITGA2; |
| | GSK3B; AKT3; FOXO1; CASP3; RPS6KB1 |
| p53 Signaling | PTEN; EP300; BBC3; PCAF; FASN; BRCA1; GADD45A; |
| | BIRC5; AKT2; PIK3CA; CHEK1; TP53INP1; BCL2; |
| | PIK3CB; PIK3C3; MAPK8; THBS1; ATR; BCL2L1; E2F1; |
| | PMAIP1; CHEK2; TNFRSF10B; TP73; RB1; HDAC9; |
| | CDK2; PIK3C2A; MAPK14; TP53; LRDD; CDKN1A; |
| | HIPK2; AKT1; PIK3R1; RRM2B; APAF1; CTNNB1; |
| | SIRT1; CCND1; PRKDC; ATM; SFN; CDKN2A; JUN; |
| | SNAI2; GSK3B; BAX; AKT3 |
| Aryl Hydrocarbon Receptor | HSPB1; EP300; FASN; TGM2; RXRA; MAPK1; NQO1; |
| Signaling | NCOR2; SP1; ARNT; CDKN1B; FOS; CHEK1; |
| | SMARCA4; NFKB2; MAPK8; ALDH1A1; ATR; E2F1; |
| | MAPK3; NRIP1; CHEK2; RELA; TP73; GSTP1; RB1; |
| | SRC; CDK2; AHR; NFE2L2; NCOA3; TP53; TNF; |
| | CDKN1A; NCOA2; APAF1; NFKB1; CCND1; ATM; ESR1; |
| | CDKN2A; MYC; JUN; ESR2; BAX; IL6; CYP1B1; |
| | HSP90AA1 |
| Xenobiotic Metabolism | PRKCE; EP300; PRKCZ; RXRA; MAPK1; NQO1; |
| Signaling | NCOR2; PIK3CA; ARNT; PRKCI; NFKB2; CAMK2A; |
| | PIK3CB; PPP2R1A; PIK3C3; MAPK8; PRKD1; |
| | ALDH1A1; MAPK3; NRIP1; KRAS; MAPK13; PRKCD; |
| | GSTP1; MAPK9; NOS2A; ABCB1; AHR; PPP2CA; FTL; |
| | NFE2L2; PIK3C2A; PPARGC1A; MAPK14; TNF; RAF1; |
| | CREBBP; MAP2K2; PIK3R1; PPP2R5C; MAP2K1; |
| | NFKB1; KEAP1; PRKCA; EIF2AK3; IL6; CYP1B1; |
| | HSP90AA1 |
| SAPK/JNK Signaling | PRKCE; IRAK1; PRKAA2; EIF2AK2; RAC1; ELK1; |
| | GRK6; MAPK1; GADD45A; RAC2; PLK1; AKT2; PIK3CA; |
| | FADD; CDK8; PIK3CB; PIK3C3; MAPK8; RIPK1; |
| | GNB2L1; IRS1; MAPK3; MAPK10; DAXX; KRAS; |

(continued)

| CELLULAR FUNCTION | GENES |
|---|---|
| | PRKCD; PRKAA1; MAPK9; CDK2; PIM1; PIK3C2A; |
| | TRAF2; TP53; LCK; MAP3K7; DYRK1A; MAP2K2; |
| | PIK3R1; MAP2K1; PAK3; CDC42; JUN; TTK; CSNK1A1; |
| | CRKL; BRAF; SGK |
| PPAr/RXR Signaling | PRKAA2; EP300; INS; SMAD2; TRAF6; PPARA; FASN; |
| | RXRA; MAPK1; SMAD3; GNAS; IKBKB; NCOR2; |
| | ABCA1; GNAQ; NFKB2; MAP3K14; STAT5B; MAPK8; |
| | IRS1; MAPK3; KRAS; RELA; PRKAA1; PPARGC1A; |
| | NCOA3; MAPK14; INSR; RAF1; IKBKG; RELB; MAP3K7; |
| | CREBBP; MAP2K2; JAK2; CHUK; MAP2K1; NFKB1; |
| | TGFBR1; SMAD4; JUN; IL1R1; PRKCA; IL6; HSP90AA1; |
| | ADIPOQ |
| NF-KB Signaling | IRAKI; EIF2AK2; EP300; INS; MYD88; PRKCZ; TRAF6; |
| | TBK1; AKT2; EGFR; IKBKB; PIK3CA; BTRC; NFKB2; |
| | MAP3K14; PIK3CB; PIK3C3; MAPK8; RIPK1; HDAC2; |
| | KRAS; RELA; PIK3C2A; TRAF2; TLR4; PDGFRB; TNF; |
| | INSR; LCK; IKBKG; RELB; MAP3K7; CREBBP; AKT1; |
| | PIK3R1; CHUK; PDGFRA; NFKB1; TLR2; BCL10; |
| | GSK3B; AKT3; TNFAIP3; IL1R1 |
| Neuregulin Signaling | ERBB4; PRKCE; ITGAM; ITGA5; PTEN; PRKCZ; ELK1; |
| | MAPK1; PTPN11; AKT2; EGFR; ERBB2; PRKCI; |
| | CDKN1B; STAT5B; PRKD1; MAPK3; ITGA1; KRAS; |
| | PRKCD; STAT5A; SRC; ITGB7; RAF1; ITGB1; MAP2K2; |
| | ADAM17; AKT1; PIK3R1; PDPK1; MAP2K1; ITGB3; |
| | EREG; FRAP1; PSEN1; ITGA2; MYC; NRG1; CRKL; |
| | AKT3; PRKCA; HSP90AA1; RPS6KB1 |
| Wnt & Beta catenin | CD44; EP300; LRP6; DVL3; CSNK1E; GJA1; SMO; |
| Signaling | AKT2; PIN1; CDH1; BTRC; GNAQ; MARK2; PPP2R1A; |
| | WNT11; SRC; DKK1; PPP2CA; SOX6; SFRP2; ILK; |
| | LEF1; SOX9; TP53; MAP3K7; CREBBP; TCF7L2; AKT1; |
| | PPP2R5C; WNT5A; LRP5; CTNNB1; TGFBR1; CCND1; |
| | GSK3A; DVL1; APC; CDKN2A; MYC; CSNK1A1; GSK3B; |
| | AKT3; SOX2 |
| Insulin Receptor Signaling | PTEN; INS; EIF4E; PTPN1; PRKCZ; MAPK1; TSC1; |
| | PTPN11; AKT2; CBL; PIK3CA; PRKCI; PIK3CB; PIK3C3 |
| | MAPK8; IRS1; MAPK3; TSC2; KRAS; EIF4EBP1; |
| | SLC2A4; PIK3C2A; PPP1CC; INSR; RAF1; FYN; |
| | MAP2K2; JAK1; AKT1; JAK2; PIK3R1; PDPK1; MAP2K1; |

(continued)

| CELLULAR FUNCTION | GENES |
|---|---|
| | GSK3A; FRAP1; CRKL; GSK3B; AKT3; FOX01; SGK; |
| | RPS6KB 1 |
| IL-6 Signaling | HSPB1; TRAF6; MAPKAPK2; ELK1; MAPK1; PTPN11; |
| | IKBKB; FOS; NFKB2; MAP3K14; MAPK8; MAPK3; |
| | MAPK10; IL6ST; KRAS; MAPK13; IL6R; RELA; SOCS1; |
| | MAPK9; ABCB1; TRAF2; MAPK14; TNF; RAF1; IKBKG; |
| | RELB; MAP3K7; MAP2K2; IL8; JAK2; CHUK; STAT3; |
| | MAP2K1; NFKB1; CEBPB; JUN; IL1R1; SRF; IL6 |
| Hepatic Cholestasis | PRKCE; IRAKI; INS; MYD88; PRKCZ; TRAF6; PPARA; |
| | RXRA; IKBKB; PRKCI; NFKB2; MAP3K14; MAPK8; |
| | PRKD1; MAPK10; RELA; PRKCD; MAPK9; ABCB1; |
| | TRAF2; TLR4; TNF; INSR; IKBKG; RELB; MAP3K7; IL8; |
| | CHUK; NR1H2; TJP2; NFKB1; ESR1; SREBF1; FGFR4; |
| | JUN; IL1R1; PRKCA; IL6 |
| IGF-1 Signaling | IGF1; PRKCZ; ELK1; MAPK1; PTPN11; NEDD4; AKT2; |
| | PIK3CA; PRKCI; PTK2; FOS; PIK3CB; PIK3C3; MAPK8; |
| | IGF1R; IRS1; MAPK3; IGFBP7; KRAS; PIK3C2A; |
| | YWHAZ; PXN; RAF1; CASP9; MAP2K2; AKT1; PIK3R1; |
| | PDPK1; MAP2K1; IGFBP2; SFN; JUN; CYR61; AKT3; |
| | FOX01; SRF; CTGF; RPS6KB1 |
| NRF2-mediated Oxidative | PRKCE; EP300; SOD2; PRKCZ; MAPK1; SQSTM1; |
| Stress Response | NQO1; PIK3CA; PRKCI; FOS; PIK3CB; PIK3C3; MAPK8; |
| | PRKD1; MAPK3; KRAS; PRKCD; GSTP1; MAPK9; FTL; |
| | NFE2L2; PIK3C2A; MAPK14; RAF1; MAP3K7; CREBBP; |
| | MAP2K2; AKT1; PIK3R1; MAP2K1; PPIB; JUN; KEAP1; |
| | GSK3B; ATF4; PRKCA; EIF2AK3; HSP90AA1 |
| Hepatic Fibrosis/Hepatic | EDN1; IGF1; KDR; FLT1; SMAD2; FGFR1; MET; PGF; |
| Stellate Cell Activation | SMAD3; EGFR; FAS; CSF1; NFKB2; BCL2; MYH9; |
| | IGF1R; IL6R; RELA; TLR4; PDGFRB; TNF; RELB; IL8; |
| | PDGFRA; NFKB1; TGFBR1; SMAD4; VEGFA; BAX; |
| | IL1R1; CCL2; HGF; MMP1; STAT1; IL6; CTGF; MMP9 |
| PPAR Signaling | EP300; INS; TRAF6; PPARA; RXRA; MAPK1; IKBKB; |
| | NCOR2; FOS; NFKB2; MAP3K14; STAT5B; MAPK3; |
| | NRIP1; KRAS; PPARG; RELA; STAT5A; TRAF2; |
| | PPARGC1A; PDGFRB; TNF; INSR; RAF1; IKBKG; |
| | RELB; MAP3K7; CREBBP; MAP2K2; CHUK; PDGFRA; |
| | MAP2K1; NFKB1; JUN; IL1R1; HSP90AA1 |
| Fc Epsilon RI Signaling | PRKCE; RAC1; PRKCZ; LYN; MAPK1; RAC2; PTPN11; |

(continued)

| CELLULAR FUNCTION | GENES |
|---|---|
| | AKT2; PIK3CA; SYK; PRKCI; PIK3CB; PIK3C3; MAPK8; |
| | PRKD1; MAPK3; MAPK10; KRAS; MAPK13; PRKCD; |
| | MAPK9; PIK3C2A; BTK; MAPK14; TNF; RAF1; FYN; |
| | MAP2K2; AKT1; PIK3R1; PDPK1; MAP2K1; AKT3; |
| | VAV3; PRKCA |
| G-Protein Coupled | PRKCE; RAP1A; RGS16; MAPK1; GNAS; AKT2; IKBKB; |
| Receptor Signaling | PIK3CA; CREB1; GNAQ; NFKB2; CAMK2A; PIK3CB; |
| | PIK3C3; MAPK3; KRAS; RELA; SRC; PIK3C2A; RAF1; |
| | IKBKG; RELB; FYN; MAP2K2; AKT1; PIK3R1; CHUK; |
| | PDPK1; STAT3; MAP2K1; NFKB1; BRAF; ATF; AKT3; PRKCA |
| Inositol Phosphate | PRKCE; IRAKI; PRKAA2; EIF2AK2; PTEN; GRK6; |
| Metabolism | MAPK1; PLK1; AKT2; PIK3CA; CDK8; PIK3CB; PIK3C3; |
| | MAPK8; MAPK3; PRKCD; PRKAA1; MAPK9; CDK2; |
| | PIM1; PIK3C2A; DYRK1A; MAP2K2; PIP5K1A; PIK3R1; |
| | MAP2K1; PAK3; ATM; TTK; CSNK1A1; BRAF; SGK |
| PDGF Signaling | EIF2AK2; ELK1; ABL2; MAPK1; PIK3CA; FOS; PIK3CB; |
| | PIK3C3; MAPK8; CAV1; ABL1; MAPK3; KRAS; SRC; |
| | PIK3C2A; PDGFRB; RAF1; MAP2K2; JAK1; JAK2; |
| | PIK3R1; PDGFRA; STAT3; SPHK1; MAP2K1; MYC; |
| | JUN; CRKL; PRKCA; SRF; STAT1; SPHK2 |
| VEGF Signaling | ACTN4; ROCK1; KDR; FLT1; ROCK2; MAPK1; PGF; |
| | AKT2; PIK3CA; ARNT; PTK2; BCL2; PIK3CB; PIK3C3; |
| | BCL2L1; MAPK3; KRAS; HIF1A; NOS3; PIK3C2A; PXN; |
| | RAF1; MAP2K2; ELAVL1; AKT1; PIK3R1; MAP2K1; SFN; |
| | VEGFA; AKT3; FOX01; PRKCA |
| Natural Killer Cell Signaling | PRKCE; RAC1; PRKCZ; MAPK1; RAC2; PTPN11; |
| | KIR2DL3; AKT2; PIK3CA; SYK; PRKCI; PIK3CB; |
| | PIK3C3; PRKD1; MAPK3; KRAS; PRKCD; PTPN6; |
| | PIK3C2A; LCK; RAF1; FYN; MAP2K2; PAK4; AKT1; |
| | PIK3R1; MAP2K1; PAK3; AKT3; VAV3; PRKCA |
| Cell Cycle: GI/S | HDAC4; SMAD3; SUV39H1; HDAC5; CDKN1B; BTRC; |
| Checkpoint Regulation | ATR; ABL1; E2F1; HDAC2; HDAC7A; RB1; HDAC11; |
| | HDAC9; CDK2; E2F2; HDAC3; TP53; CDKN1A; CCND1; |
| | E2F4; ATM; RBL2; SMAD4; CDKN2A; MYC; NRG1; |
| | GSK3B; RBL1; HDAC6 |
| T Cell Receptor Signaling | RAC1; ELK1; MAPK1; IKBKB; CBL; PIK3CA; FOS; |
| | NFKB2; PIK3CB; PIK3C3; MAPK8; MAPK3; KRAS; |
| | RELA; PIK3C2A; BTK; LCK; RAF1; IKBKG; RELB; FYN; |

(continued)

| CELLULAR FUNCTION | GENES |
|---|---|
| | MAP2K2; PIK3R1; CHUK; MAP2K1; NFKB1; ITK; BCL10; |
| | JUN; VAV3 |
| Death Receptor Signaling | CRADD; HSPB1; BID; BIRC4; TBK1; IKBKB; FADD; |
| | FAS; NFKB2; BCL2; MAP3K14; MAPK8; RIPK1; CASP8; |
| | DAXX; TNFRSF10B; RELA; TRAF2; TNF; IKBKG; RELB; |
| | CASP9; CHUK; APAF1; NFKB1; CASP2; BIRC2; CASP3; |
| | BIRC3 |
| FGF Signaling | RAC1; FGFR1; MET; MAPKAPK2; MAPK1; PTPN11; |
| | AKT2; PIK3CA; CREB1; PIK3CB; PIK3C3; MAPK8; |
| | MAPK3; MAPK13; PTPN6; PIK3C2A; MAPK14; RAF1; |
| | AKT1; PIK3R1; STAT3; MAP2K1; FGFR4; CRKL; ATF4; |
| | AKT3; PRKCA; HGF |
| GM-CSF Signaling | LYN; ELK1; MAPK1; PTPN11; AKT2; PIK3CA; CAMK2A; |
| | STAT5B; PIK3CB; PIK3C3; GNB2L1; BCL2L1; MAPK3; |
| | ETS1; KRAS; RUNX1; PIM1; PIK3C2A; RAF1; MAP2K2; |
| | AKT1; JAK2; PIK3R1; STAT3; MAP2K1; CCND1; AKT3; |
| | STAT 1 |
| Amyotrophic Lateral | BID; IGF1; RAC1; BIRC4; PGF; CAPNS1; CAPN2; |
| Sclerosis Signaling | PIK3CA; BCL2; PIK3CB; PIK3C3; BCL2L1; CAPN1; |
| | PIK3C2A; TP53; CASP9; PIK3R1; RAB5A; CASP1; |
| | APAF1; VEGFA; BIRC2; BAX; AKT3; CASP3; BIRC3 |
| JAK/Stat Signaling | PTPN1; MAPK1; PTPN11; AKT2; PIK3CA; STAT5B; |
| | PIK3CB; PIK3C3; MAPK3; KRAS; SOCS1; STAT5A; |
| | PTPN6; PIK3C2A; RAF1; CDKN1A; MAP2K2; JAK1; |
| | AKT1; JAK2; PIK3R1; STAT3; MAP2K1; FRAP1; AKT3; |
| | STAT 1 |
| Nicotinate and Nicotinamide | PRKCE; IRAKI; PRKAA2; EIF2AK2; GRK6; MAPK1; |
| Metabolism | PLK1; AKT2; CDK8; MAPK8; MAPK3; PRKCD; PRKAA1; |
| | PBEF1; MAPK9; CDK2; PIM1; DYRK1A; MAP2K2; |
| | MAP2K1; PAK3; NT5E; TTK; CSNK1A1; BRAF; SGK |
| Chemokine Signaling | CXCR4; ROCK2; MAPK1; PTK2; FOS; CFL1; GNAQ; |
| | CAMK2A; CXCL12; MAPK8; MAPK3; KRAS; MAPK13; |
| | RHOA; CCR3; SRC; PPP1CC; MAPK14; NOX1; RAF1; |
| | MAP2K2; MAP2K1; JUN; CCL2; PRKCA |
| IL-2 Signaling | ELK1; MAPK1; PTPN11; AKT2; PIK3CA; SYK; FOS; |
| | STAT5B; PIK3CB; PIK3C3; MAPK8; MAPK3; KRAS; |
| | SOCS1; STAT5A; PIK3C2A; LCK; RAF1; MAP2K2; |
| | JAK1; AKT1; PIK3R1; MAP2K1; JUN; AKT3 |

(continued)

| CELLULAR FUNCTION | GENES |
|---|---|
| Synaptic Long Term | PRKCE; IGF1; PRKCZ; PRDX6; LYN; MAPK1; GNAS; |
| Depression | PRKCI; GNAQ; PPP2R1A; IGF1R; PRKD1; MAPK3; |
| | KRAS; GRN; PRKCD; NOS3; NOS2A; PPP2CA; |
| | YWHAZ; RAF1; MAP2K2; PPP2R5C; MAP2K1; PRKCA |
| Estrogen Receptor | TAF4B; EP300; CARM1; PCAF; MAPK1; NCOR2; |
| Signaling | SMARCA4; MAPK3; NRIP1; KRAS; SRC; NR3C1; |
| | HDAC3; PPARGC1A; RBM9; NCOA3; RAF1; CREBBP; |
| | MAP2K2; NCOA2; MAP2K1; PRKDC; ESR1; ESR2 |
| Protein Ubiquitination | TRAF6; SMURF1; BIRC4; BRCA1; UCHL1; NEDD4; |
| Pathway | CBL; UBE2I; BTRC; HSPA5; USP7; USP10; FBXW7; |
| | USP9X; STUB1; USP22; B2M; BIRC2; PARK2; USP8; |
| | USP1; VHL; HSP90AA1; BIRC3 |
| IL-10 Signaling | TRAF6; CCR1; ELK1; IKBKB; SP1; FOS; NFKB2; |
| | MAP3K14; MAPK8; MAPK13; RELA; MAPK14; TNF; |
| | IKBKG; RELB; MAP3K7; JAK1; CHUK; STAT3; NFKB1; |
| | JUN; IL1R1; IL6 |
| VDR/RXR Activation | PRKCE; EP300; PRKCZ; RXRA; GADD45A; HES1; |
| | NCOR2; SP1; PRKCI; CDKN1B; PRKD1; PRKCD; |
| | RUNX2; KLF4; YY1; NCOA3; CDKN1A; NCOA2; SPP1; |
| | LRP5; CEBPB; FOX01; PRKCA |
| TGF-beta Signaling | EP300; SMAD2; SMURF1; MAPK1; SMAD3; SMAD1; |
| | FOS; MAPK8; MAPK3; KRAS; MAPK9; RUNX2; |
| | SERPINE1; RAF1; MAP3K7; CREBBP; MAP2K2; |
| | MAP2K1; TGFBR1; SMAD4; JUN; SMAD5 |
| Toll-like Receptor Signaling | IRAKI; EIF2AK2; MYD88; TRAF6; PPARA; ELK1; |
| | IKBKB; FOS; NFKB2; MAP3K14; MAPK8; MAPK13; |
| | RELA; TLR4; MAPK14; IKBKG; RELB; MAP3K7; CHUK; |
| | NFKB1; TLR2; JUN |
| p38 MAPK Signaling | HSPB1; IRAKI; TRAF6; MAPKAPK2; ELK1; FADD; FAS; |
| | CREB1; DDIT3; RPS6KA4; DAXX; MAPK13; TRAF2; |
| | MAPK14; TNF; MAP3K7; TGFBR1; MYC; ATF4; IL1R1; |
| | SRF; STAT1 |
| Neurotrophin/TRK Signaling | NTRK2; MAPK1; PTPN11; PIK3CA; CREB1; FOS; |
| | PIK3CB; PIK3C3; MAPK8; MAPK3; KRAS; PIK3C2A; |
| | RAF1; MAP2K2; AKT1; PIK3R1; PDPK1; MAP2K1; |
| | CDC42; JUN; ATF4 |
| FXR/RXR Activation | INS; PPARA; FASN; RXRA; AKT2; SDC1; MAPK8; |
| | APOB; MAPK10; PPARG; MTTP; MAPK9; PPARGC1A; |

(continued)

| CELLULAR FUNCTION | GENES |
|---|---|
| | TNF; CREBBP; AKT1; SREBF1; FGFR4; AKT3; F0X01 |
| Synaptic Long Term | PRKCE; RAP1A; EP300; PRKCZ; MAPK1; CREB1; |
| Potentiation | PRKCI; GNAQ; CAMK2A; PRKD1; MAPK3; KRAS; |
| | PRKCD; PPP1CC; RAF1; CREBBP; MAP2K2; MAP2K1; |
| | ATF4; PRKCA |
| Calcium Signaling | RAP1A; EP300; HDAC4; MAPK1; HDAC5; CREB1; |
| | CAMK2A; MYH9; MAPK3; HDAC2; HDAC7A; HDAC11; |
| | HDAC9; HDAC3; CREBBP; CALR; CAMKK2; ATF4; |
| | HDAC6 |
| EGF Signaling | ELK1; MAPK1; EGFR; PIK3CA; FOS; PIK3CB; PIK3C3; |
| | MAPK8; MAPK3; PIK3C2A; RAF1; JAK1; PIK3R1; |
| | STAT3; MAP2K1; JUN; PRKCA; SRF; STAT1 |
| Hypoxia Signaling in the | EDN1; PTEN; EP300; NQO1; UBE2I; CREB1; ARNT; |
| Cardiovascular System | HIF1A; SLC2A4; NOS3; TP53; LDHA; AKT1; ATM; |
| | VEGFA; JUN; ATF4; VHL; HSP90AA1 |
| LPS/IL-1 Mediated Inhibition | IRAKI; MYD88; TRAF6; PPARA; RXRA; ABCA1; |
| of RXR Function | MAPK8; ALDH1A1; GSTP1; MAPK9; ABCB1; TRAF2; |
| | TLR4; TNF; MAP3K7; NR1H2; SREBF1; JUN; IL1R1 |
| LXR/RXR Activation | FASN; RXRA; NCOR2; ABCA1; NFKB2; IRF3; RELA; |
| | NOS2A; TLR4; TNF; RELB; LDLR; NR1H2; NFKB1; |
| | SREBF1; IL1R1; CCL2; IL6; MMP9 |
| Amyloid Processing | PRKCE; CSNK1E; MAPK1; CAPNS1; AKT2; CAPN2; |
| | CAPN1; MAPK3; MAPK13; MAPT; MAPK14; AKT1; |
| | PSEN1; CSNK1A1; GSK3B; AKT3; APP |
| IL-4 Signaling | AKT2; PIK3CA; PIK3CB; PIK3C3; IRS1; KRAS; SOCS1; |
| | PTPN6; NR3C1; PIK3C2A; JAK1; AKT1; JAK2; PIK3R1; |
| | FRAP1; AKT3; RPS6KB1 |
| Cell Cycle: G2/M DNA | EP300; PCAF; BRCA1; GADD45A; PLK1; BTRC; |
| Damage Checkpoint | CHEK1; ATR; CHEK2; YWHAZ; TP53; CDKN1A; |
| Regulation | PRKDC; ATM; SFN; CDKN2A |
| Nitric Oxide Signaling in the | KDR; FLT1; PGF; AKT2; PIK3CA; PIK3CB; PIK3C3; |
| Cardiovascular System | CAV1; PRKCD; NOS3; PIK3C2A; AKT1; PIK3R1; |
| | VEGFA; AKT3; HSP90AA1 |
| Purine Metabolism | NME2; SMARCA4; MYH9; RRM2; ADAR; EIF2AK4; |
| | PKM2; ENTPD1; RAD51; RRM2B; TJP2; RAD51C; |
| | NT5E; POLD1; NME1 |
| cAMP-mediated Signaling | RAP1A; MAPK1; GNAS; CREB1; CAMK2A; MAPK3; |
| | SRC; RAF1; MAP2K2; STAT3; MAP2K1; BRAF; ATF4 |

(continued)

| CELLULAR FUNCTION | GENES |
|---|---|
| Mitochondrial Dysfunction | SOD2; MAPK8; CASP8; MAPK10; MAPK9; CASP9; |
| | PARK7; PSEN1; PARK2; APP; CASP3 |
| Notch Signaling | HES1; JAG1; NUMB; NOTCH4; ADAM17; NOTCH2; |
| | PSEN1; NOTCH3; NOTCH1; DLL4 |
| Endoplasmic Reticulum | HSPA5; MAPK8; XBP1; TRAF2; ATF6; CASP9; ATF4; |
| Stress Pathway | EIF2AK3; CASP3 |
| Pyrimidine Metabolism | NME2; AICDA; RRM2; EIF2AK4; ENTPD 1; RRM2B; |
| | NT5E; POLD1; NME1 |
| Parkinson's Signaling | UCHL1; MAPK8; MAPK13; MAPK14; CASP9; PARK7; |
| | PARK2; CASP3 |
| Cardiac & Beta Adrenergic | GNAS; GNAQ; PPP2R1A; GNB2L1; PPP2CA; PPP1CC; |
| Signaling | PPP2R5C |
| Glycolysis/Gluconeogene sis | HK2; GCK; GPI; ALDH1A1; PKM2; LDHA; HK1 |
| Interferon Signaling | IRF1; SOCS1; JAK1; JAK2; IFITM1; STAT1; IFIT3 |
| Sonic Hedgehog Signaling | ARRB2; SMO; GLI2; DYRK1A; GLI1; GSK3B; DYRK1B |
| Glycerophospholipid | PLD1; GRN; GPAM; YWHAZ; SPHK1; SPHK2 |
| Metabolism | |
| Phospholipid Degradation | PRDX6; PLD1; GRN; YWHAZ; SPHK1; SPHK2 |
| Tryptophan Metabolism | SIAH2; PRMT5; NEDD4; ALDH1A1; CYP1B1; SIAH1 |
| Lysine Degradation | SUV39H1; EHMT2; NSD1; SETD7; PPP2R5C |
| Nucleotide Excision Repair | ERCC5; ERCC4; XPA; XPC; ERCC1 |
| Pathway | |
| Starch and Sucrose | UCHL1; HK2; GCK; GPI; HK1 |
| Metabolism | |
| Aminosugars Metabolism | NQO1; HK2; GCK; HK1 |
| Arachidonic Acid | PRDX6; GRN; YWHAZ; CYP1B1 |
| Metabolism | |
| Circadian Rhythm Signaling | CSNK1E; CREB1; ATF4; NR1D1 |
| Coagulation System | BDKRB1; F2R; SERPINE1; F3 |
| Dopamine Receptor | PPP2R1A; PPP2CA; PPP1CC; PPP2R5C |
| Signaling | |
| Glutathione Metabolism | IDH2; GSTP1; ANPEP; IDH 1 |
| Glycerolipid Metabolism | ALDH1A1; GPAM; SPHK1; SPHK2 |
| Linoleic Acid Metabolism | PRDX6; GRN; YWHAZ; CYP1B1 |
| Methionine Metabolism | DNMT1; DNMT3B; AHCY; DNMT3A |
| Pyruvate Metabolism | GLO1; ALDH1A1; PKM2; LDHA |
| Arginine and Proline | ALDH1A1; NOS3; NOS2A |
| Metabolism | |

(continued)

| CELLULAR FUNCTION | GENES |
|---|---|
| Eicosanoid Signaling | PRDX6; GRN; YWHAZ |
| Fructose and Mannose Metabolism | HK2; GCK; HK1 |
| Galactose Metabolism | HK2; GCK; HK1 |
| Stilbene, Coumarine and Lignin Biosynthesis | PRDX6; PRDX1; TYR |
| Antigen Presentation Pathway | CALR; B2M |
| Biosynthesis of Steroids | NQO1; DHCR7 |
| Butanoate Metabolism | ALDH1A1; NLGN1 |
| Citrate Cycle | IDH2; IDH1 |
| Fatty Acid Metabolism | ALDH1A1; CYP1B1 |
| Glycerophospholipid Metabolism | PRDX6; CHKA |
| Histidine Metabolism | PRMT5; ALDH1A1 |
| Inositol Metabolism | EROIL; APEX1 |
| Metabolism of Xenobiotics by Cytochrome p450 | GSTP1; CYP1B1 |
| Methane Metabolism | PRDX6; PRDX1 |
| Phenylalanine Metabolism | PRDX6; PRDX1 |
| Propanoate Metabolism | ALDH1A1; LDHA |
| Selenoamino Acid Metabolism | PRMT5; AHCY |
| Sphingolipid Metabolism | SPHK1; SPHK2 |
| Aminophosphonate Metabolism | PRMT5 |
| Androgen and Estrogen Metabolism | PRMT5 |
| Ascorbate and Aldarate Metabolism | ALDH1A1 |
| Bile Acid Biosynthesis | ALDH1A1 |
| Cysteine Metabolism | LDHA |
| Fatty Acid Biosynthesis | FASN |
| Glutamate Receptor Signaling | GNB2L1 |
| NRF2-mediated Oxidative Stress Response | PRDX1 |
| Pentose Phosphate | GPI |

(continued)

| CELLULAR FUNCTION | GENES |
|---|---|
| Pathway | |
| Pentose and Glucuronate | UCHL1 |
| Interconversions | |
| Retinol Metabolism | ALDH1A1 |
| Riboflavin Metabolism | TYR |
| Tyrosine Metabolism | PRMT5, TYR |
| Ubiquinone Biosynthesis | PRMT5 |
| Valine, Leucine and | ALDH1A1 |
| Isoleucine Degradation | |
| Glycine, Serine and | CHKA |
| Threonine Metabolism | |
| Lysine Degradation | ALDH1A1 |
| Pain/Taste | TRPM5; TRPA1 |
| Pain | TRPM7; TRPC5; TRPC6; TRPC1; Cnr1; cnr2; Grk2; |
| | Trpa1; Pomc; Cgrp; Crf; Pka; Era; Nr2b; TRPM5; Prkaca; |
| | Prkacb; Prkar1a; Prkar2a |
| Mitochondrial Function | AIF; CytC; SMAC (Diablo); Aifin-1; Aifin-2 |
| Developmental Neurology | BMP-4; Chordin (Chrd); Noggin (Nog); WNT (Wnt2; |
| | Wnt2b; Wnt3a; Wnt4; Wnt5a; Wnt6; Wnt7b; Wnt8b; |
| | Wnt9a; Wnt9b; Wnt10a; Wnt10b; Wnt16); beta-catenin; |
| | Dkk-1; Frizzled related proteins; Otx-2; Gbx2; FGF-8; |
| | Reelin; Dab1; unc-86 (Pou4f1 or Brn3a); Numb; Reln |

Embodiments of the invention also relate to methods and compositions related to knocking out genes, amplifying genes and repairing particular mutations associated with DNA repeat instability and neurological disorders (Robert D. Wells, Tetsuo Ashizawa, Genetic Instabilities and Neurological Diseases, Second Edition, Academic Press, Oct 13, 2011 - Medical). Specific aspects of tandem repeat sequences have been found to be responsible for more than twenty human diseases (New insights into repeat instability: role of RNA•DNA hybrids. McIvor EI, Polak U, Napierala M. RNA Biol. 2010 Sep-Oct;7(5):551-8). The CRISPR-Cas system may be harnessed to correct these defects of genomic instability.

A further aspect relates to utilizing the CRISPR-Cas system for correcting defects in the EMP2A and EMP2B genes that have been identified to be associated with Lafora disease. Lafora disease is an autosomal recessive condition which is characterized by progressive myoclonus epilepsy which may start as epileptic seizures in adolescence. A few cases of the disease may be caused by mutations in genes yet to be identified. The disease causes seizures, muscle spasms, difficulty walking, dementia, and eventually death. There is currently no therapy that has proven effective against disease progression. Other genetic abnormalities associated with epilepsy may also be targeted by the CRISPR-Cas system and the underlying genetics is further described in Genetics of Epilepsy and Genetic Epilepsies, edited by Giuliano Avanzini, Jeffrey L. Noebels, Mariani Foundation Paediatric Neurology:20; 2009).

The methods of US Patent Publication No. 20110158957 assigned to Sangamo BioSciences, Inc. involved in inactivating T cell receptor (TCR) genes may also be modified to the CRISPR Cas system. In another example, the methods of US PatentPublication No. 20100311124 assigned to Sangamo BioSciences, Inc. and US Patent Publication No. 20110225664 assigned to Cellectis, which are both involved in inactivating glutamine synthetase gene expression genes may also be modified to the CRISPR Cas system.

Several further aspects of the invention relate to correcting defects associated with a wide range of genetic diseases which are further described on the website of the National Institutes of Health under the topic subsection Genetic Disorders (website at health.nih.gov/topic/GeneticDisorders). The genetic brain diseases may include but are not limited to Adrenoleukodystrophy, Agenesis of the Corpus Callosum, Aicardi Syndrome, Alpers' Disease, Alzheimer's Disease, Barth Syndrome, Batten Disease, CADASIL, Cerebellar Degeneration, Fabry's Disease, Gerstmann-Straussler-Scheinker Disease, Huntington's Disease and other Triplet Repeat Disorders, Leigh's Disease, Lesch-Nyhan Syndrome, Menkes Disease,

Mitochondrial Myopathies and NINDS Colpocephaly. These diseases are further described on the website of the National Institutes of Health under the subsection Genetic Brain Disorders.

In some embodiments, the condition may be neoplasia. In some embodiments, where the condition is neoplasia, the genes to be targeted are any of those listed in Table A (in this case PTEN and so forth). In some embodiments, the condition may be Age-related Macular Degeneration. In some embodiments, the condition may be a Schizophrenic Disorder. In some embodiments, the condition may be a Trinucleotide Repeat Disorder. In some embodiments, the condition may be Fragile X Syndrome. In some embodiments, the condition may be a Secretase Related Disorder. In some embodiments, the condition may be a Prion - related disorder. In some embodiments, the condition may be ALS. In some embodiments, the condition may be a drug addiction. In some embodiments, the condition may be Autism. In some embodiments, the condition may be Alzheimer's Disease. In some embodiments, the condition may be inflammation. In some embodiments, the condition may be Parkinson's Disease.

For example, US Patent Publication No. 20110023145, describes use of zinc finger nucleases to genetically modify cells, animals and proteins associated with autism spectrum disorders (ASD). Autism spectrum disorders (ASDs) are a group of disorders characterized by qualitative impairment in social interaction and communication, and restricted repetitive and stereotyped patterns of behavior, interests, and activities. The three disorders, autism, Asperger syndrome (AS) and pervasive developmental disorder-not otherwise specified (PDD-NOS) are a continuum of the same disorder with varying degrees of severity, associated intellectual functioning and medical conditions. ASDs are predominantly genetically determined disorders with a heritability of around 90%.

US Patent Publication No. 20110023145 comprises editing of any chromosomal sequences that encode proteins associated with ASD which may be applied to the CRISPR Cas system. The proteins associated with ASD are typically selected based

on an experimental association of the protein associated with ASD to an incidence or indication of an ASD. For example, the production rate or circulating concentration of a protein associated with ASD may be elevated or depressed in a population having an ASD relative to a population lacking the ASD. Differences in protein levels may be assessed using proteomic techniques including but not limited to Western blot, immunohistochemical staining, enzyme linked immunosorbent assay (ELISA), and mass spectrometry. Alternatively, the proteins associated

with ASD may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

Non limiting examples of disease states or disorders that may be associated with proteins associated with ASD include autism, Asperger syndrome (AS), pervasive developmental disorder-not otherwise specified (PDD-NOS), Rett's syndrome, tuberous sclerosis, phenylketonuria, Smith-Lemli-Opitz syndrome and fragile X syndrome. By way of non-limiting example, proteins associated with ASD include but are not limited to the following proteins: ATP10C aminophospholipid- MET MET receptor transporting ATPase tyrosine kinase (ATP10C) BZRAP1 MGLUR5 (GRM5) Metabotropic glutamate receptor 5 (MGLUR5) CDH10 Cadherin-10 MGLUR6 (GRM6) Metabotropic glutamate receptor 6 (MGLUR6) CDH9 Cadherin-9 NLGN1 Neuroligin-1 CNTN4 Contactin-4 NLGN2 Neuroligin-2 CNTNAP2 Contactin-associated SEMA5A Neuroligin-3 protein-like 2 (CNTNAP2) DHCR7 7-dehydrocholesterol NLGN4X Neuroligin-4 X- reductase (DHCR7) linked DOC2A Double C2-like domain- NLGN4Y Neuroligin-4 Y- containing protein alpha linked DPP6 Dipeptidyl NLGN5 Neuroligin-5 aminopeptidase-like protein 6 EN2 engrailed 2 (EN2) NRCAM Neuronal cell adhesion molecule (NRCAM) MDGA2 fragile X mental retardation NRXN1 Neurexin-1 1 (MDGA2) FMR2 (AFF2) AF4/FMR2 family member 2 OR4M2 Olfactory receptor (AFF2) 4M2 FOXP2 Forkhead box protein P2 OR4N4 Olfactory receptor (FOXP2) 4N4 FXR1 Fragile X mental OXTR oxytocin receptor retardation, autosomal (OXTR) homolog 1 (FXR1) FXR2 Fragile X mental PAH phenylalanine retardation, autosomal hydroxylase (PAH) homolog 2 (FXR2) GABRA1 Gamma-aminobutyric acid PTEN Phosphatase and receptor subunit alpha-1 tensin homologue (GABRA1) (PTEN) GABRA5 GABAA (.gamma.-aminobutyric PTPRZ1 Receptor-type acid) receptor alpha 5 tyrosine-protein subunit (GABRA5) phosphatase zeta (PTPRZ1) GABRB1 Gamma-aminobutyric acid RELN Reelin receptor subunit beta-1 (GABRB1) GABRB3 GABAA (.gamma.-aminobutyric RPL10 60S ribosomal acid) receptor .beta.3 subunit protein L10 (GABRB3) GABRG1 Gamma-aminobutyric acid SEMA5A Semaphorin-5A receptor subunit gamma-1 (SEMA5A) (GABRG1) HIRIP3 HIRA-interacting protein 3 SEZ6L2 seizure related 6 homolog (mouse)- like 2 HOXA1 Homeobox protein Hox-A1 SHANK3 SH3 and multiple (HOXA1) ankyrin repeat domains 3 (SHANK3) IL6 Interleukin-6 SHBZRAP1 SH3 and multiple ankyrin repeat domains 3 (SHBZRAP1) LAMB1 Laminin subunit beta-1 SLC6A4 Serotonin (LAMB1) transporter (SERT) MAPK3 Mitogen-activated protein TAS2R1 Taste receptor kinase 3 type 2 member 1 TAS2R1 MAZ Myc-associated zinc finger TSC1 Tuberous sclerosis protein protein 1 MDGA2 MAM domain containing TSC2 Tuberous sclerosis glycosylphosphatidylinositol protein 2 anchor 2 (MDGA2) MECP2 Methyl CpG binding UBE3A Ubiquitin protein protein 2 (MECP2) ligase E3A (UBE3A) MECP2 methyl CpG binding WNT2 Wingless-type protein 2 (MECP2) MMTV integration site family, member 2 (WNT2)

The identity of the protein associated with ASD whose chromosomal sequence is edited can and will vary. In preferred embodiments, the proteins associated with ASD whose chromosomal sequence is edited may be the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, the fragile X mental retardation autosomal homolog 2 protein (FXR2) encoded by the FXR2 gene, the MAM domain containing glycosylphosphatidylinositol anchor 2 protein (MDGA2) encoded by the MDGA2 gene, the methyl CpG binding protein 2 (MECP2) encoded by the MECP2 gene, the metabotropic glutamate receptor 5 (MGLUR5) encoded by the MGLUR5-1 gene (also termed GRM5), the neurexin 1 protein encoded by the NRXN1 gene, or the semaphorin-5A protein (SEMA5A) encoded by the SEMA5A gene. In an exemplary embodiment, the genetically modified animal is a rat, and the edited chromosomal sequence encoding the protein associated with ASD is as listed below: BZRAP1 benzodiazapine receptor XM_002727789, (peripheral) associated XM_213427, protein 1 (BZRAP1) XM_002724533, XM_001081125 AFF2 (FMR2) AF4/FMR2 family member 2 XM_219832, (AFF2) XM_001054673 FXR1 Fragile X mental NM_001012179 retardation, autosomal homolog 1 (FXR1) FXR2 Fragile X mental NM 001100647 retardation, autosomal homolog 2 (FXR2) MDGA2 MAM domain containing NM_199269 glycosylphosphatidylinositol anchor 2 (MDGA2) MECP2 Methyl CpG binding NM_022673 protein 2 (MECP2) MGLUR5 Metabotropic glutamate NM_017012 (GRM5) receptor 5 (MGLUR5) NRXN1 Neurexin-1 NM_021767 SEMA5A Semaphorin-5A (SEMA5A) NM_001107659.

Exemplary animals or cells may comprise one, two, three, four, five, six, seven, eight, or nine or more inactivated chromosomal sequences encoding a protein associated with ASD, and zero, one, two, three, four, five, six, seven, eight, nine or more chromosomally integrated sequences encoding proteins associated with ASD. The edited or integrated chromosomal sequence may be modified to encode an altered protein associated with ASD. Non-limiting examples of mutations in proteins associated with ASD include the L18Q mutation in neurexin 1 where the leucine at position 18 is replaced with a glutamine, the R451C mutation in neuroligin 3 where the arginine at position 451 is replaced with a cysteine, the R87W mutation in neuroligin 4 where the arginine at position 87 is replaced with a tryptophan, and the I425V mutation in serotonin transporter where the isoleucine at position 425 is replaced with a valine. A number of other mutations and chromosomal rearrangements in ASD-related chromosomal sequences have been associated with ASD and are known in the art. See, for example, Freitag et al. (2010) Eur. Child. Adolesc. Psychiatry 19:169-178, and Bucan et al. (2009) PLoS Genetics 5: e1000536, the disclosure of which is incorporated by reference herein in its entirety.

Examples of proteins associated with Parkinson's disease include but are not limited to α-synuclein, DJ-1, LRRK2, PINK1, Parkin, UCHL1, Synphilin-1, and NURR1.

Examples of addiction-related proteins may include ABAT for example.

Examples of inflammation-related proteins may include the monocyte chemoattractant protein-1 (MCP1) encoded by the Ccr2 gene, the C-C chemokine receptor type 5 (CCR5) encoded by the Ccr5 gene, the IgG receptor IIB (FCGR2b, also termed CD32) encoded by the Fcgr2b gene, or the Fc epsilon R1g (FCER1g) protein encoded by the Fcer1g gene, for example.

Examples of cardiovascular diseases associated proteins may include IL1B (interleukin 1, beta), XDH (xanthine dehydrogenase), TP53 (tumor protein p53), PTGIS (prostaglandin I2 (prostacyclin) synthase), MB (myoglobin), IL4 (interleukin 4), ANGPT1 (angiopoietin 1), ABCG8 (ATP-binding cassette, sub-family G (WHITE), member 8), or CTSK (cathepsin K), for example.

For example, US Patent Publication No. 20110023153, describes use of zinc finger nucleases to genetically modify cells, animals and proteins associated with Alzheimer's Disease. Once modified cells and animals may be further tested using known methods to study the effects of the targeted mutations on the development and/or progression of AD using measures commonly used in the study of AD - such as, without limitation, learning and memory, anxiety, depression, addiction, and sensory motor functions as well as assays that measure behavioral, functional, pathological, metaboloic and biochemical function.

The present disclosure comprises editing of any chromosomal sequences that encode proteins associated with AD. The AD-related proteins are typically selected based on an experimental association of the AD-related protein to an AD disorder. For example, the production rate or circulating concentration of an AD-related protein may be elevated or depressed in a population having an AD disorder relative to a population lacking the AD disorder. Differences in protein levels may be assessed using proteomic techniques including but not limited to Western blot, immunohistochemical staining, enzyme linked immunosorbent assay (ELISA), and mass spectrometry. Alternatively, the AD-related proteins may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

Examples of Alzheimer's disease associated proteins may include the very low density lipoprotein receptor protein (VLDLR) encoded by the VLDLR gene, the ubiquitin-like modifier activating enzyme 1 (UBA1) encoded by the UBA1 gene, or the NEDD8-activating enzyme E1 catalytic subunit protein (UBE1C) encoded by the UBA3 gene, for example.

By way of non-limiting example, proteins associated with AD include but are not limited to the proteins listed as follows: Chromosomal Sequence Encoded Protein ALAS2 Delta-aminolevulinate synthase 2 (ALAS2) ABCA1 ATP-binding cassette transporter (ABCA1) ACE Angiotensin I-converting enzyme (ACE) APOE Apolipoprotein E precursor (APOE) APP amyloid precursor protein (APP) AQP1 aquaporin 1 protein (AQP1) BIN1 Myc box-dependent-interacting protein 1 or bridging integrator 1 protein (BIN1) BDNF brain-derived neurotrophic factor (BDNF) BTNL8 Butyrophilin-like protein 8 (BTNL8) C1ORF49 chromosome 1 open reading frame 49 CDH4 Cadherin-4 CHRNB2 Neuronal acetylcholine receptor subunit beta-2 CKLFSF2 CKLF-like MARVEL transmembrane domain- containing protein 2 (CKLFSF2) CLEC4E C-type lectin domain family 4, member e (CLEC4E) CLU clusterin protein (also known as apoplipoprotein J) CR1 Erythrocyte complement receptor 1 (CR1, also known as CD35, C3b/C4b receptor and immune adherence receptor) CR1L Erythrocyte complement receptor 1 (CR1L) CSF3R granulocyte colony-stimulating factor 3 receptor (CSF3R) CST3 Cystatin C or cystatin 3 CYP2C Cytochrome P450 2C DAPK1 Death-associated protein kinase 1 (DAPK1) ESR1 Estrogen receptor 1 FCAR Fc fragment of IgA receptor (FCAR, also known as CD89) FCGR3B Fc fragment of IgG, low affinity IIIb, receptor (FCGR3B or CD16b) FFA2 Free fatty acid receptor 2 (FFA2) FGA Fibrinogen (Factor I) GAB2 GRB2-associated-binding protein 2 (GAB2) GAB2 GRB2-associated-binding protein 2 (GAB2) GALP Galanin-like peptide GAPDHS Glyceraldehyde-3-phosphate dehydrogenase, spermatogenic (GAPDHS) GMPB GMBP HP Haptoglobin (HP) HTR7 5-hydroxytryptamine (serotonin) receptor 7 (adenylate cyclase-coupled) IDE Insulin degrading enzyme IF127 IF127 IFI6 Interferon, alpha-inducible protein 6 (IFI6) IFIT2 Interferon-induced protein with tetratricopeptide repeats 2 (IFIT2) IL1RN interleukin-1 receptor antagonist (IL-1RA) IL8RA Interleukin 8 receptor, alpha (IL8RA or CD181) IL8RB Interleukin 8 receptor, beta (IL8RB) JAG1 Jagged 1 (JAG1) KCNJ15 Potassium inwardly-rectifying channel, subfamily J, member 15 (KCNJ15) LRP6 Low-density lipoprotein receptor-related protein 6 (LRP6) MAPT microtubule-associated protein tau (MAPT) MARK4 MAP/microtubule affinity-regulating kinase 4 (MARK4) MPHOSPH1 M-phase phosphoprotein 1 MTHFR 5,10-methylenetetrahydrofolate reductase MX2 Interferon-induced GTP-binding protein Mx2 NBN Nibrin, also known as NBN NCSTN Nicastrin NIACR2 Niacin receptor 2 (NIACR2, also known as GPR109B) NMNAT3 nicotinamide nucleotide adenylyltransferase 3 NTM Neurotrimin (or HNT) ORM1 Orosmucoid 1 (ORM1) or Alpha-1-acid glycoprotein 1 P2RY13 P2Y purinoceptor 13 (P2RY13) PBEF1 Nicotinamide phosphoribosyltransferase (NAmPRTase or Nampt) also known as pre-B-cell colony-enhancing factor 1 (PBEF1) or visfatin PCK1 Phosphoenolpyruvate carboxykinase PICALM phosphatidylinositol binding clathrin assembly protein (PICALM) PLAU Urokinase-type plasminogen activator (PLAU) PLXNC1 Plexin C1 (PLXNC1) PRNP Prion protein PSEN1 presenilin 1 protein (PSEN1) PSEN2 presenilin 2 protein (PSEN2) PTPRA protein tyrosine phosphatase receptor type A protein (PTPRA) RALGPS2 Ral GEF with PH domain and SH3 binding motif 2 (RALGPS2) RGSL2 regulator of G-protein signaling like 2 (RGSL2) SELENBP1 Selenium binding protein 1 (SELNBP1) SLC25A37 Mitoferrin-1 SORL1 sortilin-related receptor L(DLR class) A repeats-containing protein (SORL1) TF Transferrin TFAM Mitochondrial transcription factor A TNF Tumor necrosis factor TNFRSF10C Tumor necrosis factor receptor superfamily member 10C (TNFRSF10C) TNFSF10 Tumor necrosis factor receptor superfamily, (TRAIL) member 10a (TNFSF10) UBA1 ubiquitin-like modifier activating enzyme 1 (UBA1) UBA3 NEDD8-activating enzyme E1 catalytic subunit protein (UBE1C) UBB ubiquitin B protein (UBB) UBQLN1 Ubiquilin-1 UCHL1 ubiquitin carboxyl-terminal esterase L1 protein (UCHL1) UCHL3 ubiquitin carboxyl-terminal hydrolase isozyme L3 protein (UCHL3) VLDLR very low density lipoprotein receptor protein (VLDLR)

In exemplary embodiments, the proteins associated with AD whose chromosomal sequence is edited may be the very low density lipoprotein receptor protein (VLDLR) encoded by the VLDLR gene, the ubiquitin-like modifier activating enzyme 1 (UBA1) encoded by the UBA1 gene, the NEDD8-activating enzyme E1 catalytic subunit protein (UBE1C) encoded by the UBA3 gene, the aquaporin 1 protein (AQP1) encoded by the AQP1 gene, the ubiquitin carboxyl-terminal esterase L1 protein (UCHL1) encoded by the UCHL1 gene, the ubiquitin carboxyl-terminal hydrolase isozyme L3 protein (UCHL3) encoded by the UCHL3 gene, the ubiquitin B protein (UBB) encoded by the UBB gene, the microtubule-associated protein tau (MAPT) encoded by the MAPT gene, the protein tyrosine phosphatase receptor type A protein (PTPRA) encoded by the PTPRA gene, the phosphatidylinositol binding clathrin assembly protein (PICALM) encoded by the PICALM gene, the clusterin protein (also known as apolipoprotein J) encoded by the CLU gene, the presenilin 1 protein encoded by the PSEN1 gene, the presenilin 2 protein encoded by the PSEN2 gene, the sortilin-related receptor L(DLR class) A repeats-containing protein (SORL1) protein encoded by the SORL1 gene, the amyloid precursor protein (APP) encoded by the APP gene, the Apolipoprotein E precursor (APOE) encoded by the APOE gene, or the brain-derived neurotrophic factor (BDNF) encoded by the BDNF gene. In an exemplary embodiment, the genetically modified animal is a rat, and the edited chromosomal sequence encoding the protein associated with AD is as as follows: APP amyloid precursor protein (APP) NM_019288 AQP1 aquaporin 1 protein (AQP1) NM_012778 BDNF Brain-derived neurotrophic factor NM_012513 CLU clusterin protein (also known as NM_053021 apoplipoprotein J) MAPT microtubule-associated protein NM_017212 tau (MAPT) PICALM phosphatidylinositol binding NM_053554 clathrin assembly protein (PICALM) PSEN1 presenilin 1 protein (PSEN1) NM_019163 PSEN2 presenilin 2 protein (PSEN2) NM_031087 PTPRA protein tyrosine phosphatase NM_012763 receptor type A protein (PTPRA) SORL1 sortilin-related receptor L(DLR NM_053519, class) A repeats-containing XM_001065506, protein (SORL1) XM217115 UBA1 ubiquitin-like modifier activating NM_001014080 enzyme 1 (UBA1) UBA3 NEDD8-activating enzyme E1_NM_057205 catalytic subunit protein (UBE1C) UBB ubiquitin B protein (UBB) NM_138895 UCHL1 ubiquitin carboxyl-terminal NM_017237 esterase L1 protein (UCHL1) UCHL3 ubiquitin carboxyl-terminal NM_001110165 hydrolase isozyme L3 protein (UCHL3) VLDLR very low density lipoprotein NM_013155 receptor protein (VLDLR)

The animal or cell may comprise 1, 2, 3, 4, 5, 6, 7, 8, 9,10, 11, 12, 13, 14, 15 or more disrupted chromosomal sequences encoding a protein associated with AD and zero, 1, 2, 3, 4, 5, 6, 7, 8, 9, 10, 11, 12, 13, 14, 15 or more chromosomally integrated sequences encoding a protein associated with AD.

The edited or integrated chromosomal sequence may be modified to encode an altered protein associated with AD. A number of mutations in AD-related chromosomal sequences have been associated with AD. For instance, the V7171 (i.e. valine at position 717 is changed to isoleucine) missense mutation in APP causes familial AD. Multiple mutations in the presenilin-1 protein, such as H163R (i.e. histidine at position 163 is changed to arginine), A246E (i.e. alanine at position 246 is changed to glutamate), L286V (i.e. leucine at position 286 is changed to valine) and C410Y (i.e. cysteine at position 410 is changed to tyrosine) cause familial Alzheimer's type 3. Mutations in the presenilin-2 protein, such as N141 I (i.e. asparagine at position 141 is changed to isoleucine), M239V (i.e. methionine at position 239 is changed to valine), and D439A (i.e. aspartate at position 439 is changed to alanine) cause familial Alzheimer's type 4. Other associations of genetic variants in AD-associated genes and disease are known in the art. See, for example, Waring et al. (2008) Arch. Neurol. 65:329-334, the disclosure of which is incorporated by reference herein in its entirety.

Examples of proteins associated Autism Spectrum Disorder may include the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, or the fragile X mental retardation autosomal homolog 2 protein (FXR2) encoded by the FXR2 gene, for example.

Examples of proteins associated Macular Degeneration may include the ATP-binding cassette, sub-family A (ABC1) member 4 protein (ABCA4) encoded by the ABCR gene, the apolipoprotein E protein (APOE) encoded by the APOE gene, or the chemokine (C-C motif) Ligand 2 protein (CCL2) encoded by the CCL2 gene, for example.

Examples of proteins associated Schizophrenia may include NRG1, ErbB4, CPLX1, TPH1, TPH2, NRXN1, GSK3A, BDNF, DISC1, GSK3B, and combinations thereof.

Examples of proteins involved in tumor suppression may include ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4, for example.

Examples of proteins associated with a secretase disorder may include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), or BACE1 (beta-site APP-cleaving enzyme 1), for example.

For example, US Patent Publication No. 20110023146, describes use of zinc finger nucleases to genetically modify cells, animals and proteins associated with secretase-associated disorders. Secretases are essential for processing pre-proteins into their biologically active forms. Defects in various components of the secretase pathways contribute to many disorders, particularly those with hallmark amyloidogenesis or amyloid plaques, such as Alzheimer's disease (AD).

A secretase disorder and the proteins associated with these disorders are a diverse set of proteins that effect susceptibility for numerous disorders, the presence of the disorder, the severity of the disorder, or any combination thereof. The present disclosure comprises editing of any chromosomal sequences that encode proteins associated with a secretase disorder. The proteins associated with a secretase disorder are typically selected based on an experimental association of the secretase--related proteins with the development of a secretase disorder. For example, the production rate or circulating concentration of a protein associated with a secretase disorder may be elevated or depressed in a population with a secretase disorder relative to a population without a secretase disorder. Differences in protein levels may be assessed using proteomic techniques including but not limited to Western blot, immunohistochemical staining, enzyme linked immunosorbent assay (ELISA), and mass spectrometry. Alternatively, the protein associated with a secretase disorder may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

By way of non-limiting example, proteins associated with a secretase disorder include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), BACE1 (beta-site APP-cleaving enzyme 1), ITM2B (integral membrane protein 2B), CTSD (cathepsin D), NOTCH1 (Notch homolog 1, translocation-associated (Drosophila)), TNF (tumor necrosis factor (TNF superfamily, member 2)), INS (insulin), DYT10 (dystonia 10), ADAM17 (ADAM metallopeptidase domain 17), APOE (apolipoprotein E), ACE (angiotensin I converting enzyme (peptidyl-dipeptidase A) 1), STN (statin), TP53 (tumor protein p53), IL6 (interleukin 6 (interferon, beta 2)), NGFR (nerve growth factor receptor (TNFR superfamily, member 16)), IL1B (interleukin 1, beta), ACHE (acetylcholinesterase (Yt blood group)), CTNNB1 (catenin (cadherin-associated protein), beta 1, 88kDa), IGF1 (insulin-like growth factor 1 (somatomedin C)), IFNG (interferon, gamma), NRG1 (neuregulin 1), CASP3 (caspase 3, apoptosis-related cysteine peptidase), MAPK1 (mitogen-activated protein kinase 1), CDH1 (cadherin 1, type 1, E-cadherin (epithelial)), APBB1 (amyloid beta (A4) precursor protein-binding, family B, member 1 (Fe65)), HMGCR (3-hydroxy-3-methylglutaryl-Coenzyme A reductase), CREB1 (cAMP responsive element binding protein 1), PTGS2 (prostaglandin-endoperoxide synthase 2 (prostaglandin G/H synthase and cyclooxygenase)), HES1 (hairy and enhancer of split 1, (Drosophila)), CAT (catalase), TGFB1 (transforming growth factor, beta 1), ENO2 (enolase 2 (gamma, neuronal)), ERBB4 (v-erb-a erythroblastic leukemia viral oncogene homolog 4 (avian)), TRAPPC10 (trafficking protein particle complex 10), MAOB (monoamine oxidase B), NGF (nerve growth factor (beta polypeptide)), MMP12 (matrix metallopeptidase 12 (macrophage elastase)), JAG1 (jagged 1 (Alagille syndrome)), CD40LG (CD40 ligand), PPARG (peroxisome proliferator-activated receptor gamma), FGF2 (fibroblast growth factor 2 (basic)), IL3 (interleukin 3 (colony-stimulating factor, multiple)), LRP1 (low density lipoprotein receptor-related protein 1), NOTCH4 (Notch homolog 4 (Drosophila)), MAPK8 (mitogen-activated protein kinase 8), PREP (prolyl endopeptidase), NOTCH3 (Notch homolog 3 (Drosophila)), PRNP (prion protein), CTSG (cathepsin G), EGF (epidermal growth factor (beta-urogastrone)), REN (renin), CD44 (CD44 molecule (Indian blood group)), SELP (selectin P (granule membrane protein 140 kDa, antigen CD62)), GHR (growth hormone receptor), ADCYAP1 (adenylate cyclase activating polypeptide 1 (pituitary)), INSR (insulin receptor), GFAP (glial fibrillary acidic protein), MMP3 (matrix metallopeptidase 3 (stromelysin 1, progelatinase)), MAPK10 (mitogen-activated protein kinase 10), SP1 (Sp1 transcription factor), MYC (v-myc myelocytomatosis viral oncogene homolog (avian)), CTSE (cathepsin E), PPARA (peroxisome proliferator-activated receptor alpha), JUN (jun oncogene), TIMP1 (TIMP metallopeptidase inhibitor 1), IL5 (interleukin 5 (colony-stimulating factor, eosinophil)), ILIA (interleukin 1, alpha), MMP9 (matrix metallopeptidase 9 (gelatinase B, 92 kDa gelatinase, 92 kDa type IV collagenase)), HTR4 (5-hydroxytryptamine (serotonin) receptor 4), HSPG2 (heparan sulfate proteoglycan 2), KRAS (v-Ki-ras2 Kirsten rat sarcoma viral oncogene homolog), CYCS (cytochrome c, somatic), SMG1 (SMG1 homolog, phosphatidylinositol 3-kinase-related kinase (C. elegans)), IL1R1 (interleukin 1 receptor, type I), PROK1 (prokineticin 1), MAPK3 (mitogen-activated protein kinase 3), NTRK1 (neurotrophic tyrosine kinase, receptor, type 1), IL13 (interleukin 13), MME (membrane metallo-endopeptidase), TKT (transketolase), CXCR2 (chemokine (C-X-C motif) receptor 2), IGF1R (insulin-like growth factor 1 receptor), RARA (retinoic acid receptor, alpha), CREBBP (CREB binding protein), PTGS1 (prostaglandin-endoperoxide synthase 1 (prostaglandin G/H synthase and cyclooxygenase)), GALT (galactose-1-phosphate uridylyltransferase), CHRM1 (cholinergic receptor, muscarinic 1), ATXN1 (ataxin 1), PAWR (PRKC, apoptosis, WT1, regulator), NOTCH2 (Notch homolog 2 (Drosophila)), M6PR (mannose-6-phosphate receptor (cation dependent)), CYP46A1 (cytochrome P450, family 46, subfamily A, polypeptide 1), CSNK1 D (casein kinase 1, delta), MAPK14 (mitogen-activated protein kinase 14), PRG2 (proteoglycan 2, bone marrow (natural killer cell activator, eosinophil granule major basic protein)), PRKCA (protein kinase C, alpha), L1 CAM (L1 cell adhesion molecule), CD40 (CD40 molecule, TNF receptor superfamily member 5), NR1I2 (nuclear receptor subfamily 1, group I, member 2), JAG2 (jagged 2), CTNND1 (catenin (cadherin-associated protein), delta 1), CDH2 (cadherin 2, type 1, N-cadherin (neuronal)), CMA1 (chymase 1, mast cell), SORT1 (sortilin 1), DLK1 (delta-like 1 homolog (Drosophila)), THEM4 (thioesterase superfamily member 4), JUP (junction plakoglobin), CD46 (CD46 molecule, complement regulatory protein), CCL11 (chemokine (C-C motif) ligand 11), CAV3 (caveolin 3), RNASE3 (ribonuclease, RNase A family, 3 (eosinophil cationic protein)), HSPA8 (heat shock 70kDa protein 8), CASP9 (caspase 9, apoptosis-related cysteine peptidase), CYP3A4 (cytochrome P450, family 3, subfamily A, polypeptide 4), CCR3 (chemokine (C-C motif) receptor 3), TFAP2A (transcription factor AP-2 alpha (activating enhancer binding protein 2 alpha)), SCP2 (sterol carrier protein 2), CDK4 (cyclin-dependent kinase 4), HIF1A (hypoxia inducible factor 1, alpha subunit (basic helix-loop-helix transcription factor)), TCF7L2 (transcription factor 7-like 2 (T-cell specific, HMG-box)), IL1R2 (interleukin 1 receptor, type II), B3GALTL (beta 1,3-galactosyltransferase-like), MDM2 (Mdm2 p53 binding protein homolog (mouse)), RELA (v-rel reticuloendotheliosis viral oncogene homolog A (avian)), CASP7 (caspase 7, apoptosis-related cysteine peptidase), IDE (insulin-degrading enzyme), FABP4 (fatty acid binding protein 4, adipocyte), CASK (calcium/calmodulin-dependent serine protein kinase (MAGUK family)), ADCYAP1R1 (adenylate cyclase activating polypeptide 1 (pituitary) receptor type I), ATF4 (activating transcription factor 4 (tax-responsive enhancer element B67)), PDGFA (platelet-derived growth factor alpha polypeptide), C21 or f33 (chromosome 21 open reading frame 33), SCG5 (secretogranin V (7B2 protein)), RNF123 (ring finger protein 123), NFKB1 (nuclear factor of kappa light polypeptide gene enhancer in B-cells 1), ERBB2 (v-erb-b2 erythroblastic leukemia viral oncogene homolog 2, neuro/glioblastoma derived oncogene homolog (avian)), CAV1 (caveolin 1, caveolae protein, 22 kDa), MMP7 (matrix metallopeptidase 7 (matrilysin, uterine)), TGFA (transforming growth factor, alpha), RXRA (retinoid X receptor, alpha), STX1A (syntaxin 1A (brain)), PSMC4 (proteasome (prosome, macropain) 26S subunit, ATPase, 4), P2RY2 (purinergic receptor P2Y, G-protein coupled, 2), TNFRSF21 (tumor necrosis factor receptor superfamily, member 21), DLG1 (discs, large homolog 1 (Drosophila)), NUMBL (numb homolog (Drosophila)-like), SPN (sialophorin), PLSCR1 (phospholipid scramblase 1), UBQLN2 (ubiquilin 2), UBQLN1 (ubiquilin 1), PCSK7 (proprotein convertase subtilisin/kexin type 7), SPON1 (spondin 1, extracellular matrix protein), SILV (silver homolog (mouse)), QPCT (glutaminyl-peptide cyclotransferase), HESS (hairy and enhancer of split 5 (Drosophila)), GCC1 (GRIP and coiled-coil domain containing 1), and any combination thereof.

The genetically modified animal or cell may comprise 1, 2, 3, 4, 5, 6, 7, 8, 9, 10 or more disrupted chromosomal sequences encoding a protein associated with a secretase disorder and zero, 1, 2, 3, 4, 5, 6, 7, 8, 9, 10 or more chromosomally integrated sequences encoding a disrupted protein associated with a secretase disorder.

Examples of proteins associated with Amyotrophic Lateral Sclerosis may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

For example, US Patent Publication No. 20110023144, describes use of zinc finger nucleases to genetically modify cells, animals and proteins associated with amyotrophyic lateral sclerosis (ALS) disease. ALS is characterized by the gradual steady degeneration of certain nerve cells in the brain cortex, brain stem, and spinal cord involved in voluntary movement.

Motor neuron disorders and the proteins associated with these disorders are a diverse set of proteins that effect susceptibility for developing a motor neuron disorder, the presence of the motor neuron disorder, the severity of the motor neuron disorder or any combination thereof. The present disclosure comprises editing of any chromosomal sequences that encode proteins associated with ALS disease, a specific motor neuron disorder. The proteins associated with ALS are typically selected based on an experimental association of ALS--related proteins to ALS. For example, the production rate or circulating concentration of a protein associated with ALS may be elevated or depressed in a population with ALS relative to a population without ALS. Differences in protein levels may be assessed using proteomic techniques including but not limited to Western blot, immunohistochemical staining, enzyme linked immunosorbent assay (ELISA), and mass spectrometry. Alternatively, the proteins associated with ALS may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

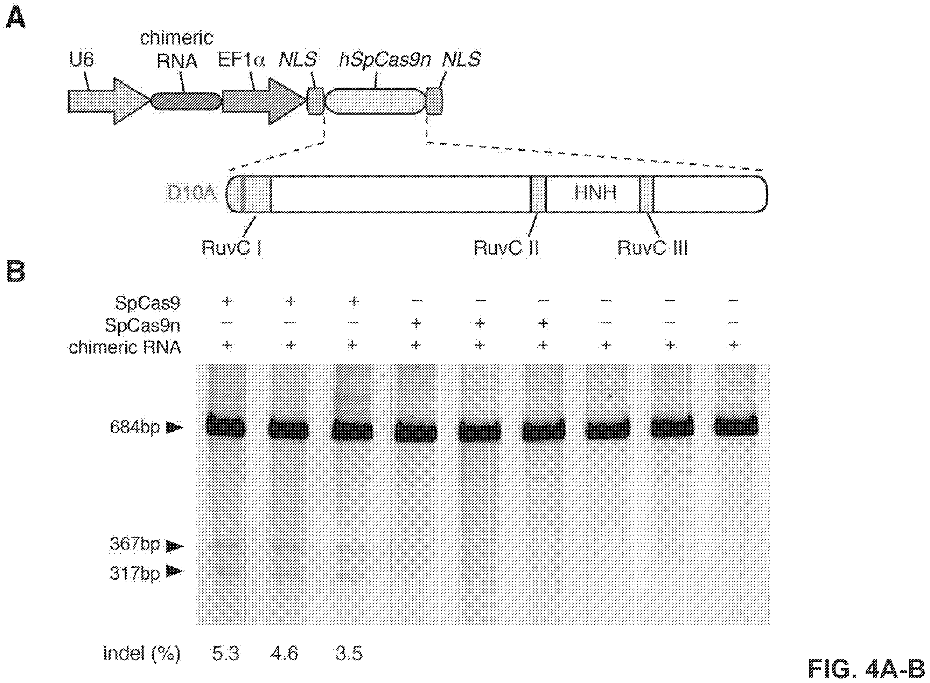

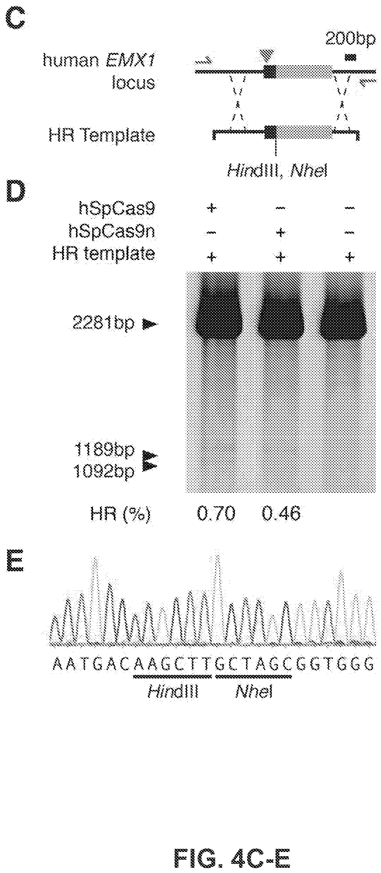

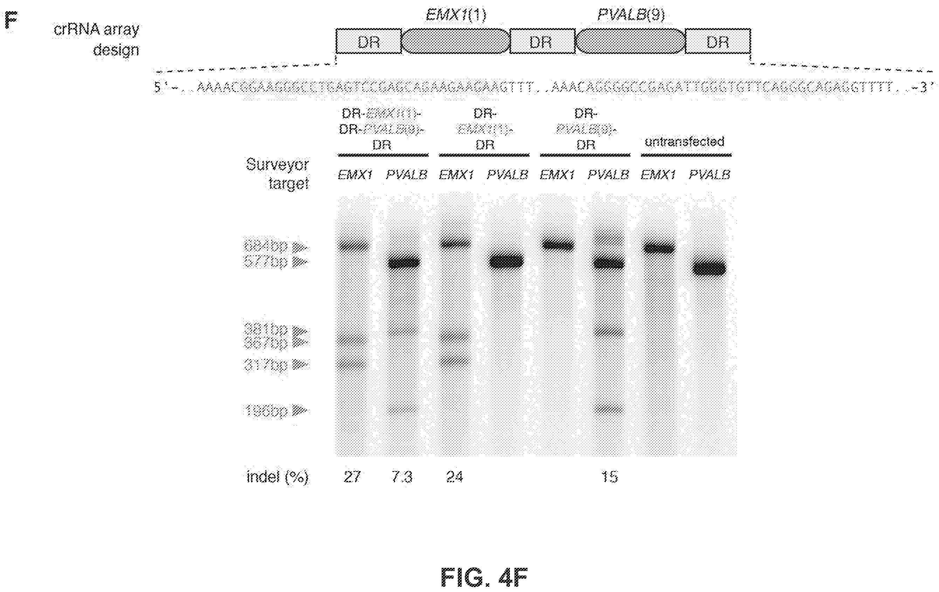

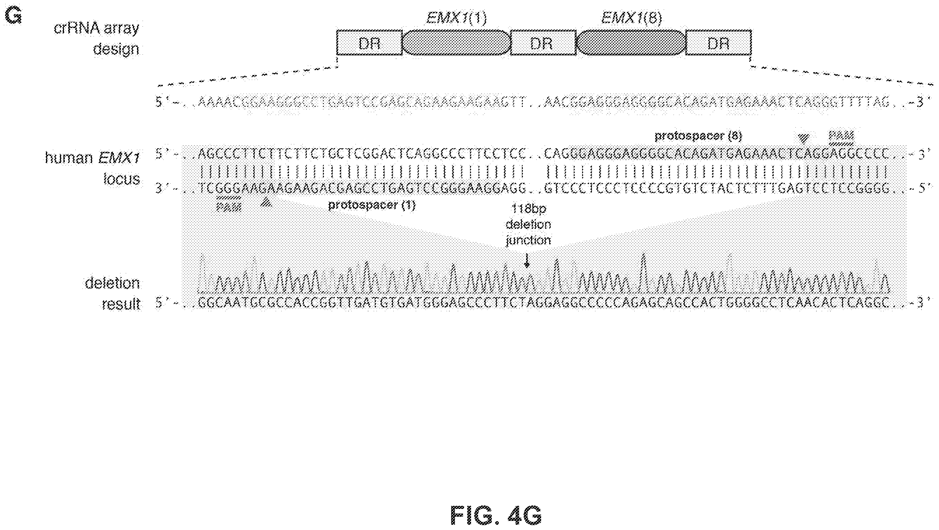

By way of non-limiting example, proteins associated with ALS include but are not limited to the following proteins: SOD1 superoxide dismutase 1, ALS3 amyotrophic lateral soluble sclerosis 3 SETX senataxin ALS5 amyotrophic lateral sclerosis 5 FUS fused in sarcoma ALS7 amyotrophic lateral sclerosis 7 ALS2 amyotrophic lateral DPP6 Dipeptidyl-peptidase 6 sclerosis 2 NEFH neurofilament, heavy PTGS1 prostaglandin- polypeptide endoperoxide synthase 1 SLC1A2 solute carrier family 1 TNFRSF10B tumor necrosis factor (glial high affinity receptor superfamily, glutamate transporter), member 10b member 2 PRPH peripherin HSP90AA1 heat shock protein 90 kDa alpha (cytosolic), class A member 1 GRIA2 glutamate receptor, IFNG interferon, gamma ionotropic, AMPA 2 S100B S100 calcium binding FGF2 fibroblast growth factor 2 protein B AOX1 aldehyde oxidase 1 CS citrate synthase TARDBP TAR DNA binding protein TXN thioredoxin RAPH1 Ras association MAP3K5 mitogen-activated protein (RalGDS/AF-6) and kinase 5 pleckstrin homology domains 1 NBEAL1 neurobeachin-like 1 GPX1 glutathione peroxidase 1 ICA1L islet cell autoantigen RAC1 ras-related C3 botulinum 1.69 kDa-like toxin substrate 1 MAPT microtubule-associated ITPR2 inositol 1,4,5- protein tau triphosphate receptor, type 2 ALS2CR4 amyotrophic lateral GLS glutaminase sclerosis 2 (juvenile) chromosome region, candidate 4 ALS2CR8 amyotrophic lateral CNTFR ciliary neurotrophic factor sclerosis 2 (juvenile) receptor chromosome region, candidate 8 ALS2CR11 amyotrophic lateral FOLH1 folate hydrolase 1 sclerosis 2 (juvenile) chromosome region, candidate 11 FAM117B family with sequence P4HB prolyl 4-hydroxylase, similarity 117, member B beta polypeptide CNTF ciliary neurotrophic factor SQSTM1 sequestosome 1 STRADB STE20-related kinase NAIP NLR family, apoptosis adaptor beta inhibitory protein YWHAQ tyrosine 3- SLC33A1 solute carrier family 33 monooxygenase/tryptoph (acetyl-CoA transporter), an 5-monooxygenase member 1 activation protein, theta polypeptide TRAK2 trafficking protein, FIG. 4 FIG. 4 homolog, SAC1 kinesin binding 2 lipid phosphatase domain containing NIF3L1 NIF3 NGG1 interacting INA internexin neuronal factor 3-like 1 intermediate filament protein, alpha PARD3B par-3 partitioning COX8A cytochrome c oxidase defective 3 homolog B subunit VIIIA CDK15 cyclin-dependent kinase HECW1 HECT, C2 and WW 15 domain containing E3 ubiquitin protein ligase 1 NOS1 nitric oxide synthase 1 MET met proto-oncogene SOD2 superoxide dismutase 2, HSPB1 heat shock 27 kDa mitochondrial protein 1 NEFL neurofilament, light CTSB cathepsin B polypeptide ANG angiogenin, HSPA8 heat shock 70 kDa ribonuclease, RNase A protein 8 family, 5 VAPB VAMP (vesicle- ESR1 estrogen receptor 1 associated membrane protein)-associated protein B and C SNCA synuclein, alpha HGF hepatocyte growth factor CAT catalase ACTB actin, beta NEFM neurofilament, medium TH tyrosine hydroxylase polypeptide BCL2 B-cell CLL/lymphoma 2 FAS Fas (TNF receptor superfamily, member 6) CASP3 caspase 3, apoptosis- CLU clusterin related cysteine peptidase SMN1 survival of motor neuron G6PD glucose-6-phosphate 1, telomeric dehydrogenase BAX BCL2-associated X HSF1 heat shock transcription protein factor 1 RNF19A ring finger protein 19A JUN jun oncogene ALS2CR12 amyotrophic lateral HSPA5 heat shock 70 kDa sclerosis 2 (juvenile) protein 5 chromosome region, candidate 12 MAPK14 mitogen-activated protein IL10 interleukin 10 kinase 14 APEX1 APEX nuclease TXNRD1 thioredoxin reductase 1 (multifunctional DNA repair enzyme) 1 NOS2 nitric oxide synthase 2, TIMP1 TIMP metallopeptidase inducible inhibitor 1 CASP9 caspase 9, apoptosis- XIAP X-linked inhibitor of related cysteine apoptosis peptidase GLG1 golgi glycoprotein 1 EPO erythropoietin VEGFA vascular endothelial ELN elastin growth factor A GDNF glial cell derived NFE2L2 nuclear factor (erythroid- neurotrophic factor derived 2)-like 2 SLC6A3 solute carrier family 6 HSPA4 heat shock 70 kDa (neurotransmitter protein 4 transporter, dopamine), member 3 APOE apolipoprotein E PSMB8 proteasome (prosome, macropain) subunit, beta type, 8 DCTN1 dynactin 1 TIMP3 TIMP metallopeptidase inhibitor 3 KIFAP3 kinesin-associated SLC1A1 solute carrier family 1 protein 3 (neuronal/epithelial high affinity glutamate transporter, system Xag), member 1 SMN2 survival of motor neuron CCNC cyclin C 2, centromeric MPP4 membrane protein, STUB1 STIP1 homology and U- palmitoylated 4 box containing protein 1 ALS2 amyloid beta (A4) PRDX6 peroxiredoxin 6 precursor protein SYP synaptophysin CABIN 1 calcineurin binding protein 1 CASP1 caspase 1, apoptosis- GART phosphoribosylglycinami related cysteine de formyltransferase, peptidase phosphoribosylglycinami de synthetase, phosphoribosylaminoimi dazole synthetase CDK5 cyclin-dependent kinase 5 ATXN3 ataxin 3 RTN4 reticulon 4 C1QB complement component 1, q subcomponent, B chain VEGFC nerve growth factor HTT huntingtin receptor PARK7 Parkinson disease 7 XDH xanthine dehydrogenase GFAP glial fibrillary acidic MAP2 microtubule-associated protein protein 2 CYCS cytochrome c, somatic FCGR3B Fc fragment of IgG, low affinity IIIb, CCS copper chaperone for UBL5 ubiquitin-like 5 superoxide dismutase MMP9 matrix metallopeptidase SLC18A3 solute carrier family 18 9 ((vesicular acetylcholine), member 3 TRPM7 transient receptor HSPB2 heat shock 27 kDa potential cation channel, protein 2 subfamily M, member 7 AKT1 v-akt murine thymoma DERL1 Der1-like domain family, viral oncogene homolog 1 member 1 CCL2 chemokine (C--C motif) NGRN neugrin, neurite ligand 2 outgrowth associated GSR glutathione reductase TPPP3 tubulin polymerization- promoting protein family member 3 APAF1 apoptotic peptidase BTBD10 BTB (POZ) domain activating factor 1 containing 10 GLUD1 glutamate CXCR4 chemokine (C--X--C motif) dehydrogenase 1 receptor 4 SLC1A3 solute carrier family 1 FLT1 fins-related tyrosine (glial high affinity glutamate transporter), member 3 kinase 1 PON1 paraoxonase 1 AR androgen receptor LIF leukemia inhibitory factor ERBB3 v-erb-b2 erythroblastic leukemia viral oncogene homolog 3 LGALS1 lectin, galactoside- CD44 CD44 molecule binding, soluble, 1 TP53 tumor protein p53 TLR3 toll-like receptor 3 GRIA1 glutamate receptor, GAPDH glyceraldehyde-3- ionotropic, AMPA 1 phosphate dehydrogenase GRIK1 glutamate receptor, DES desmin ionotropic, kainate 1 CHAT choline acetyltransferase FLT4 fins-related tyrosine kinase 4 CHMP2B chromatin modifying BAG1 BCL2-associated protein 2B athanogene MT3 metallothionein 3 CHRNA4 cholinergic receptor, nicotinic, alpha 4 GSS glutathione synthetase BAK1 BCL2-antagonist/killer 1 KDR kinase insert domain GSTP1 glutathione S-transferase receptor (a type III pi 1 receptor tyrosine kinase) OGG1 8-oxoguanine DNA IL6 interleukin 6 (interferon, glycosylase beta 2).

The animal or cell may comprise 1, 2, 3, 4, 5, 6, 7, 8, 9, 10 or more disrupted chromosomal sequences encoding a protein associated with ALS and zero, 1, 2, 3, 4, 5, 6, 7, 8, 9, 10 or more chromosomally integrated sequences encoding the disrupted protein associated with ALS. Preferred proteins associated with ALS include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins associated with prion diseases may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins related to neurodegenerative conditions in prion disorders may include A2M (Alpha-2-Macroglobulin), AATF (Apoptosis antagonizing transcription factor), ACPP (Acid phosphatase prostate), ACTA2 (Actin alpha 2 smooth muscle aorta), ADAM22 (ADAM metallopeptidase domain), ADORA3 (Adenosine A3 receptor), or ADRA1D (Alpha-1D adrenergic receptor for Alpha-ID adrenoreceptor), for example.

Examples of proteins associated with Immunodeficiency may include A2M [alpha-2-macroglobulin]; AANAT [arylalkylamine N-acetyltransferase]; ABCA1 [ATP-binding cassette, sub-family A (ABC1), member 1]; ABCA2 [ATP-binding cassette, sub-family A (ABC1), member 2]; or ABCA3 [ATP-binding cassette, sub-family A (ABC1), member 3]; for example.

Examples of proteins associated with Trinucleotide Repeat Disorders include AR (androgen receptor), FMR1 (fragile X mental retardation 1), HTT (huntingtin), or DMPK (dystrophia myotonica-protein kinase), FXN (frataxin), ATXN2 (ataxin 2), for example.

Examples of proteins associated with Neurotransmission Disorders include SST (somatostatin), NOS1 (nitric oxide synthase 1 (neuronal)), ADRA2A (adrenergic, alpha-2A-, receptor), ADRA2C (adrenergic, alpha-2C-, receptor), TACR1 (tachykinin receptor 1), or HTR2c (5-hydroxytryptamine (serotonin) receptor 2C), for example.

Examples of neurodevelopmental-associated sequences include A2BP1 [ataxin 2-binding protein 1], AADAT [aminoadipate aminotransferase], AANAT [arylalkylamine N-acetyltransferase], ABAT [4-aminobutyrate aminotransferase], ABCA1 [ATP-binding cassette, sub-family A (ABC1), member 1], or ABCA13 [ATP-binding cassette, sub-family A (ABC1), member 13], for example.

Further examples of preferred conditions treatable with the present system include may be selected from: Aicardi-Goutieres Syndrome; Alexander Disease; Allan-Herndon-Dudley Syndrome; POLG-Related Disorders; Alpha-Mannosidosis (Type II and III); Alström Syndrome; Angelman; Syndrome; Ataxia-Telangiectasia; Neuronal Ceroid-Lipofuscinoses; Beta-Thalassemia; Bilateral Optic Atrophy and (Infantile) Optic Atrophy Type 1; Retinoblastoma (bilateral); Canavan Disease; Cerebrooculofacioskeletal Syndrome 1 [COFS1]; Cerebrotendinous Xanthomatosis; Cornelia de Lange Syndrome; MAPT-Related Disorders; Genetic Prion Diseases; Dravet Syndrome; Early-Onset Familial Alzheimer Disease; Friedreich Ataxia [FRDA]; Fryns Syndrome; Fucosidosis; Fukuyama Congenital Muscular Dystrophy; Galactosialidosis; Gaucher Disease; Organic Acidemias; Hemophagocytic Lymphohistiocytosis; Hutchinson-Gilford Progeria Syndrome; Mucolipidosis II; Infantile Free Sialic Acid Storage Disease; PLA2G6-Associated Neurodegeneration; Jervell and Lange-Nielsen Syndrome; Junctional Epidermolysis Bullosa; Huntington Disease; Krabbe Disease (Infantile); Mitochondrial DNA-Associated Leigh Syndrome and NARP; Lesch-Nyhan Syndrome; LIS1-Associated Lissencephaly; Lowe Syndrome; Maple Syrup Urine Disease; MECP2 Duplication Syndrome; ATP7A-Related Copper Transport Disorders; LAMA2-Related Muscular Dystrophy; Arylsulfatase A Deficiency; Mucopolysaccharidosis Types I, II or III; Peroxisome Biogenesis Disorders, Zellweger Syndrome Spectrum; Neurodegeneration with Brain Iron Accumulation Disorders; Acid Sphingomyelinase Deficiency; Niemann-Pick Disease Type C; Glycine Encephalopathy; ARX-Related Disorders; Urea Cycle Disorders; COL1A1/2-Related Osteogenesis Imperfecta; Mitochondrial DNA Deletion Syndromes; PLP1-Related Disorders; Perry Syndrome; Phelan-McDermid Syndrome; Glycogen Storage Disease Type II (Pompe Disease) (Infantile); MAPT-Related Disorders; MECP2-Related Disorders; Rhizomelic Chondrodysplasia Punctata Type 1; Roberts Syndrome; Sandhoff Disease; Schindler Disease - Type 1; Adenosine Deaminase Deficiency; Smith-Lemli-Opitz Syndrome; Spinal Muscular Atrophy; Infantile-Onset Spinocerebellar Ataxia; Hexosaminidase A Deficiency; Thanatophoric Dysplasia Type 1; Collagen Type VI-Related Disorders; Usher Syndrome Type I; Congenital Muscular Dystrophy; Wolf-Hirschhorn Syndrome; Lysosomal Acid Lipase Deficiency; and Xeroderma Pigmentosum.

As will be apparent, it is envisaged that the present system can be used to target any polynucleotide sequence of interest. Some examples of conditions or diseases that might be usefully treated using the present system are included in the Tables above and examples of genes currently associated with those conditions are also provided there. However, the genes exemplified are not exhaustive.

For example, "wild type StCas9" refers to wild type Cas9 from S thermophilus, the protein sequence of which is given in the SwissProt database under accession number G3ECR1. Similarly, S pyogenes Cas9 is included in SwissProt under accession number Q99ZW2.

## EXAMPLES

*Example 1: CRISPR Complex Activity in the Nucleus of a Eukaryotic Cell*

An example type II CRISPR system is the type II CRISPR locus from *Streptococcus pyogenes* SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Fig. 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Fig. 2A). This example describes an example process for adapting this RNA-programmable nuclease system to direct CRISPR complex activity in the nuclei of eukaryotic cells.

*Cell culture and transfection*

Human embryonic kidney (HEK) cell line HEK 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and $100\mu$g/mL streptomycin at 37°C with 5% $CO_2$ incubation. Mouse neuro2A (N2A) cell line (ATCC) was maintained with DMEM supplemented with 5% fetal bovine serum (HyClone), 2mM GlutaMAX (Life Technologies), 100U/mL penicillin, and $100\mu$g/mL streptomycin at 37°C with 5% $CO_2$.

HEK 293FT or N2A cells were seeded into 24-well plates (Corning) one day prior to transfection at a density of 200,000 cells per well. Cells were transfected using Lipofectamine 2000 (Life Technologies) following the manufacturer's recommended protocol. For each well of a 24-well plate a total of 800ng of plasmids were used.

*Surveyor assay and sequencing analysis for genome modification*

HEK 293FT or N2A cells were transfected with plasmid DNA as described above. After transfection, the cells were incubated at 37°C for 72 hours before genomic DNA extraction. Genomic DNA was extracted using the QuickExtract DNA extraction kit (Epicentre) following the manufacturer's protocol. Briefly, cells were resuspended in QuickExtract solution and incubated at 65°C for 15 minutes and 98°C for 10 minutes. Extracted genomic DNA was immediately processed or stored at -20°C.

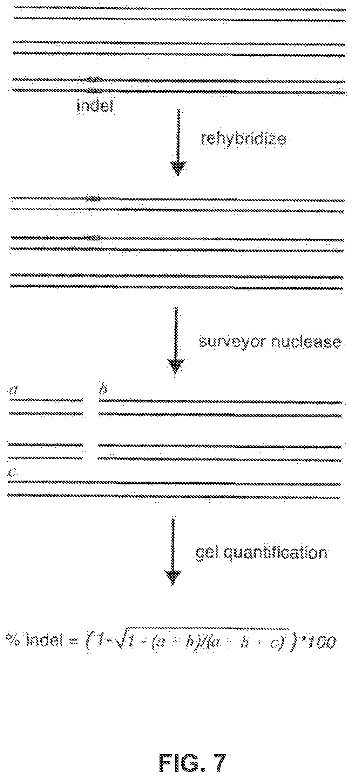

The genomic region surrounding a CRISPR target site for each gene was PCR amplified, and products were purified using QiaQuick Spin Column (Qiagen) following manufacturer's protocol. A total of 400ng of the purified PCR products were mixed with 2μl 10X Taq polymerase PCR buffer (Enzymatics) and ultrapure water to a final volume of 20μl, and subjected to a re-annealing process to enable heteroduplex formation: 95°C for 10min, 95°C to 85°C ramping at - 2°C/s, 85°C to 25°C at - 0.25°C/s, and 25°C hold for 1 minute. After re-annealing, products were treated with Surveyor nuclease and Surveyor enhancer S (Transgenomics) following the manufacturer's recommended protocol, and analyzed on 4-20% Novex TBE poly-acrylamide gels (Life Technologies). Gels were stained with SYBR Gold DNA stain (Life Technologies) for 30 minutes and imaged with a Gel Doc gel imaging system (Bio-rad). Quantification was based on relative band intensities, as a measure of the fraction of cleaved DNA. Fig. 7 provides a schematic illustration of this Surveyor assay.

Restriction fragment length polymorphism assay for detection of homologous recombination.

HEK 293FT and N2A cells were transfected with plasmid DNA, and incubated at 37°C for 72 hours before genomic DNA extraction as described above. The target genomic region was PCR amplified using primers outside the homology arms of the homologous recombination (HR) template. PCR products were separated on a 1% agarose gel and extracted with MinElute GelExtraction Kit (Qiagen). Purified products were digested with HindIII (Fermentas) and analyzed on a 6% Novex TBE poly-acrylamide gel (Life Technologies).

RNA secondary structure prediction and analysis

RNA secondary structure prediction was performed using the online webserver RNAfold developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

RNA purification

HEK 293FT cells were maintained and transfected as stated above. Cells were harvested by trypsinization followed by washing in phosphate buffered saline (PBS). Total cell RNA was extracted with TRI reagent (Sigma) following manufacturer's protocol. Extracted total RNA was quantified using Naonodrop (Thermo Scientific) and normalized to same concentration.

*Northern blot analysis of crRNA and tracrRNA expression in mammalian cells*

RNAs were mixed with equal volumes of 2X loading buffer (Ambion), heated to 95°C for 5 min, chilled on ice for 1 min, and then loaded onto 8% denaturing polyacrylamide gels (SequaGel, National Diagnostics) after pre-running the gel for at least 30 minutes. The samples were electrophoresed for 1.5 hours at 40W limit. Afterwards, the RNA was transferred to Hybond N+ membrane (GE Healthcare) at 300 mA in a semi-dry transfer apparatus (Bio-rad) at room temperature for 1.5 hours. The RNA was crosslinked to the membrane using autocrosslink button on Stratagene UV Crosslinker the Stratalinker (Stratagene). The membrane was pre-hybridized in ULTRAhyb-Oligo Hybridization Buffer (Ambion) for 30 min with rotation at 42°C, and probes were then added and hybridized overnight. Probes were ordered from IDT and labeled with [gamma-$^{32}$P] ATP (Perkin Elmer) with T4 polynucleotide kinase (New England Biolabs). The membrane was washed once with pre-warmed (42°C) 2xSSC, 0.5% SDS for 1 min followed by two 30 minute washes at 42°C. The membrane was exposed to a phosphor screen for one hour or overnight at room temperature and then scanned with a phosphorimager (Typhoon).

*Bacterial CRISPR system construction and evaluation*

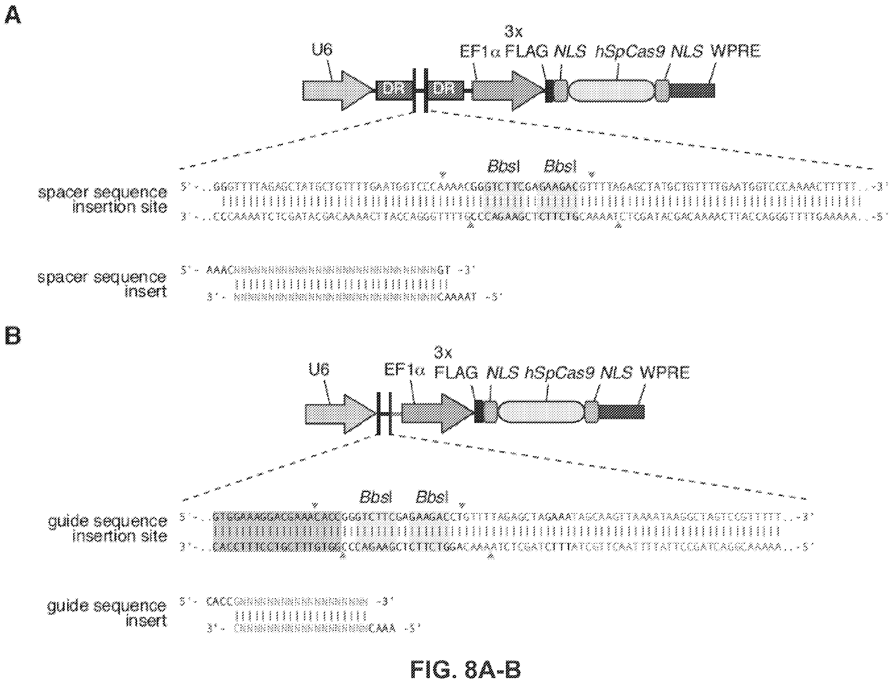

CRISPR locus elements, including tracrRNA, *Cas9,* and leader were PCR amplified from *Streptococcus pyogenes* SF370 genomic DNA with flanking homology arms for Gibson Assembly. Two *Bsa*I type IIS sites were introduced in between two direct repeats to facilitate easy insertion of spacers (Fig. 8). PCR products were cloned into EcoRV-digested pACYC184 downstream of the tet promoter using Gibson Assembly Master Mix (NEB). Other endogenous CRISPR system elements were omitted, with the exception of the last 50bp of Csn2. Oligos (Integrated DNA Technology) encoding spacers with complimentary overhangs were cloned into the *Bsa*I-digested vector pDC000 (NEB) and then ligated with T7 ligase (Enzymatics) to generate pCRISPR plasmids. Challenge plasmids containing spacers with PAM

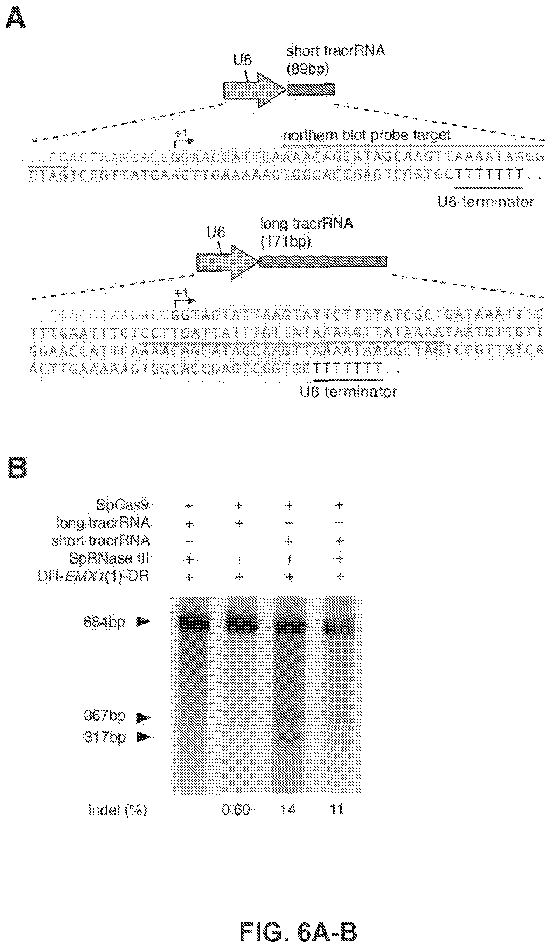

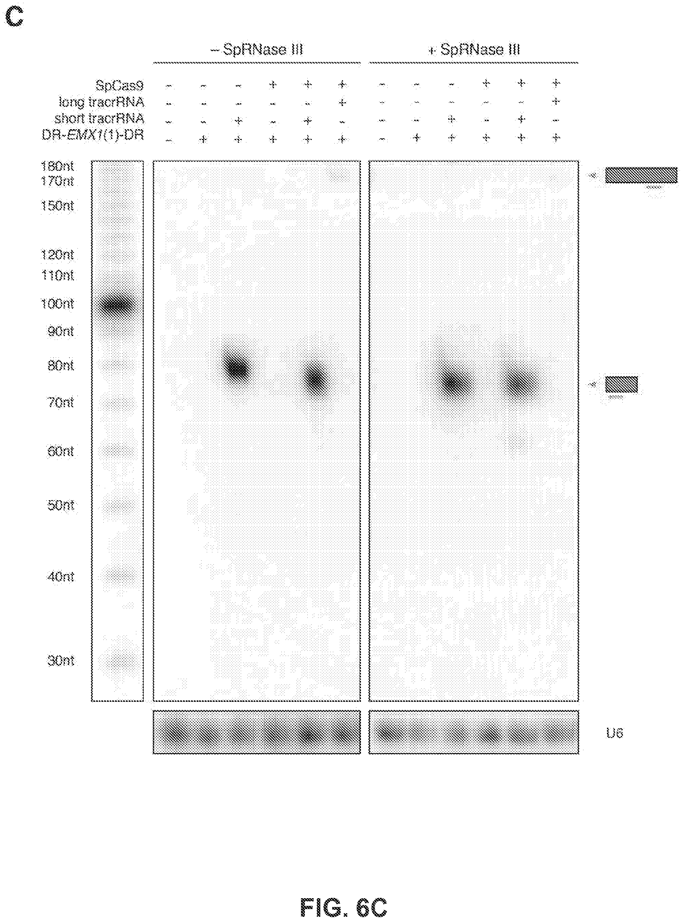

expression in mammalian cells (expression constructs illustrated in Fig. 6A, with functionality as determined by results of the Surveyor assay shown in Fig. 6B). Transcription start sites are marked as +1, and transcription terminator and the sequence probed by northern blot are also indicated. Expression of processed tracrRNA was also confirmed by Northern blot. Fig. 6C shows results of a Northern blot analysis of total RNA extracted from 293FT cells transfected with U6 expression constructs carrying long or short tracrRNA, as well as SpCas9 and DR-EMX1(1)-DR. Left and right panels are from 293FT cells transfected without or with

SpRNase III, respectively. U6 indicate loading control blotted with a probe targeting human U6 snRNA. Transfection of the short tracrRNA expression construct led to abundant levels of the processed form of tracrRNA (~75bp). Very low amounts of long tracrRNA are detected on the Northern blot.

To promote precise transcriptional initiation, the RNA polymerase III-based U6 promoter was selected to drive the expression of tracrRNA (Fig. 2C). Similarly, a U6 promoter-based construct was developed to express a pre-crRNA array consisting of a single spacer flanked by two direct repeats (DRs, also encompassed by the term "tracr-mate sequences"; Fig. 2C). The initial spacer was designed to target a 33-base-pair (bp) target site (30-bp protospacer plus a 3-bp CRISPR motif (PAM) sequence satisfying the NGG recognition motif of Cas9) in the human *EMX1* locus (Fig. 2C), a key gene in the development of the cerebral cortex.

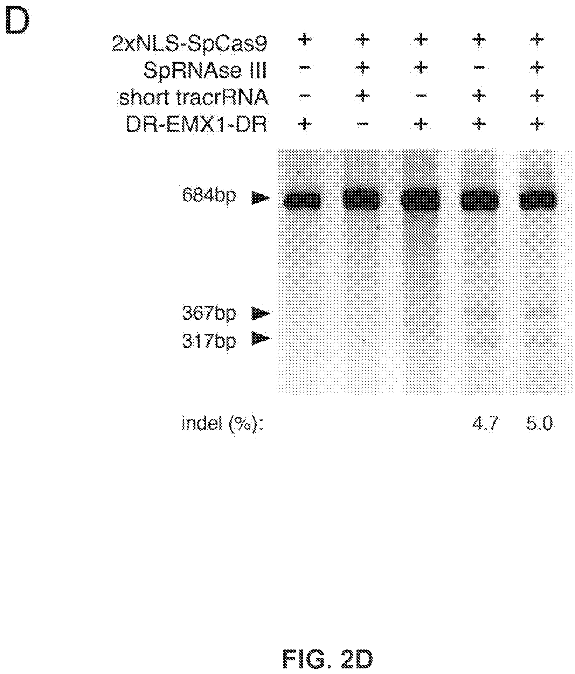

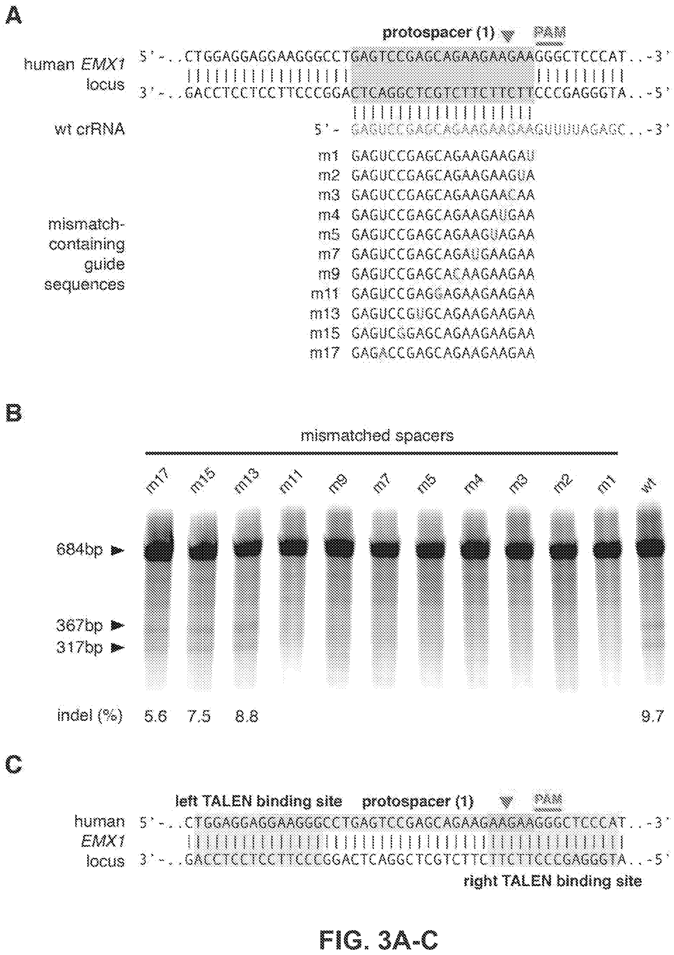

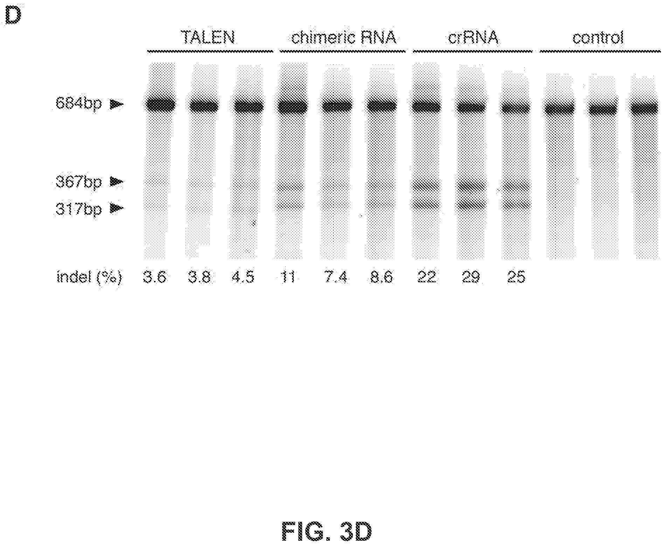

To test whether heterologous expression of the CRISPR system (SpCas9, SpRNase III, tracrRNA, and pre-crRNA) in mammalian cells can achieve targeted cleavage of mammalian chromosomes, HEK 293FT cells were transfected with combinations of CRISPR components. Since DSBs in mammalian nuclei are partially repaired by the non-homologous end joining (NHEJ) pathway, which leads to the formation of indels, the Surveyor assay was used to detect potential cleavage activity at the target *EMX1* locus (Fig. 7) (see e.g. Guschin et al., 2010, Methods Mol Biol 649: 247). Co-transfection of all four CRISPR components was able to induce up to 5.0% cleavage in the protospacer (see Fig. 2D). Co-transfection of all CRISPR components minus SpRNase III also induced up to 4.7% indel in the protospacer, suggesting that there may be endogenous mammalian RNases that are capable of assisting with crRNA maturation, such as for example the related Dicer and Drosha enzymes. Removing any of the remaining three components abolished the genome cleavage activity of the CRISPR system (Fig. 2D). Sanger sequencing of amplicons containing the target locus verified the cleavage activity: in 43 sequenced clones, 5 mutated alleles (11.6%) were found. Similar experiments using a variety of guide sequences produced indel percentages as high as 29% (see Figs. 3-6, 10, and 11). These results define a three-component system for efficient CRISPR-mediated genome modification in mammalian cells. To optimize the cleavage efficiency, Applicants also tested whether different isoforms of tracrRNA affected the cleavage efficiency and found that, in this example system, only the short (89-bp) transcript form was able to mediate cleavage of the human *EMX1* genomic locus (Fig. 6B).

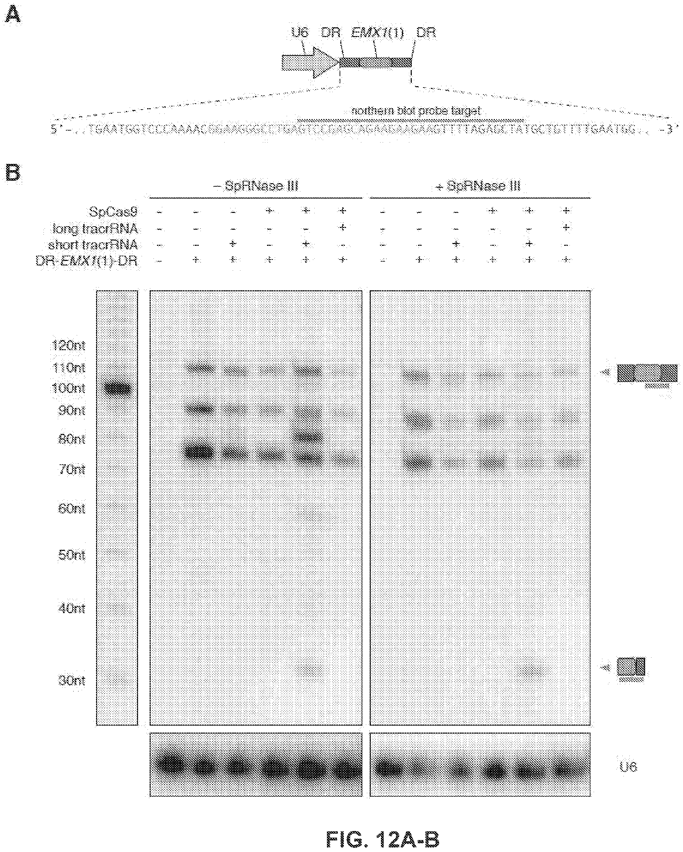

Fig. 12 provides an additional Northern blot analysis of crRNA processing in mammalian cells. Fig. 12A illustrates a schematic showing the expression vector for a single spacer flanked by two direct repeats (DR-EMX1(1)-DR). The 30bp spacer targeting the human EMX1 locus protospacer 1 (see Fig. 6) and the direct repeat sequences are shown in the sequence beneath Fig. 12A. The line indicates the region whose reverse-complement sequence was used to generate Northern blot probes for EMX1(1) crRNA detection. Fig. 12B shows a Northern blot analysis of total RNA extracted from 293FT cells transfected with U6 expression constructs carrying DR-EMX1(1)-DR. Left and right panels are from 293FT cells transfected without or with SpRNase III respectively. DR-EMX1(1)-DR was processed into mature crRNAs only in the presence of SpCas9 and short tracrRNA and was not dependent on the presence of SpRNase III. The mature crRNA detected from transfected 293FT total RNA is ~33bp and is shorter than the 39-42bp mature crRNA from *S. pyogenes*. These results demonstrate that a CRISPR system can be transplanted into eukaryotic cells and reprogrammed to facilitate cleavage of endogenous mammalian target polynucleotides.

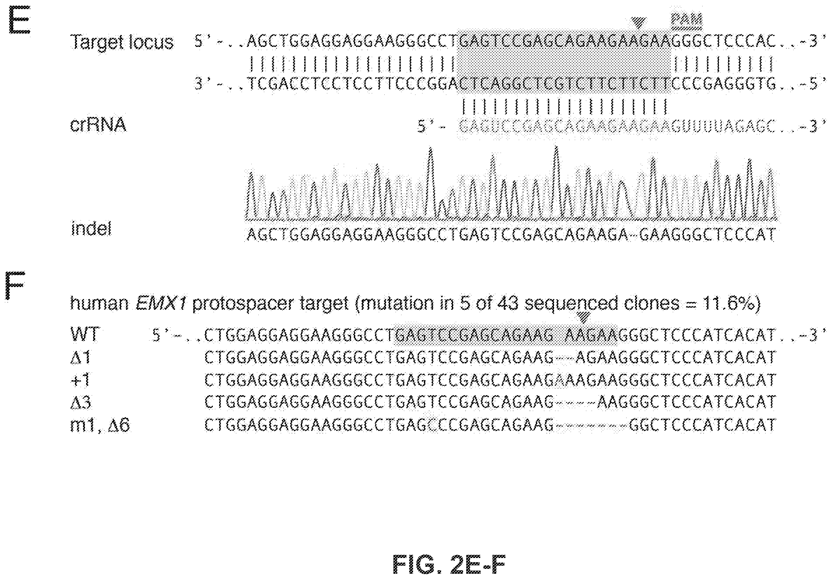

Fig. 2 illustrates the bacterial CRISPR system described in this example. Fig. 2A illustrates a schematic showing the CRISPR locus 1 from *Streptococcus pyogenes* SF370 and a proposed mechanism of CRISPR-mediated DNA cleavage by this system. Mature crRNA processed from the direct repeat-spacer array directs Cas9 to genomic targets consisting of complimentary protospacers and a protospacer-adjacent motif (PAM). Upon target-spacer base pairing, Cas9 mediates a double-strand break in the target DNA. Fig. 2B illustrates engineering of *S. pyogenes* Cas9 (SpCas9) and RNase III (SpRNase III) with nuclear localization signals (NLSs) to enable import into the mammalian nucleus. Fig. 2C illustrates mammalian expression of SpCas9 and SpRNase III driven by the constitutive EF1a promoter and tracrRNA and pre-crRNA array (DR-Spacer-DR) driven by the RNA Pol3 promoter U6 to promote precise transcription initiation and termination. A protospacer from the human *EMX1* locus with a satisfactory PAM sequence is used as the spacer in the pre-crRNA array. Fig. 2D illustrates surveyor nuclease assay for SpCas9-mediated minor insertions and deletions. SpCas9 was expressed with and without SpRNase III, tracrRNA, and a pre-crRNA array carrying the *EMX1*-target spacer. Fig. 2E illustrates a schematic representation of base pairing between target locus and *EMX1*-targeting crRNA, as well as an example chromatogram showing a micro deletion adjacent to the SpCas9 cleavage site. Fig. 2F illustrates mutated alleles identified from sequencing analysis of 43 clonal amplicons showing a variety of micro insertions and deletions. Dashes indicate deleted bases, and non-aligned or mismatched bases indicate insertions or mutations. Scale bar = 101μm.

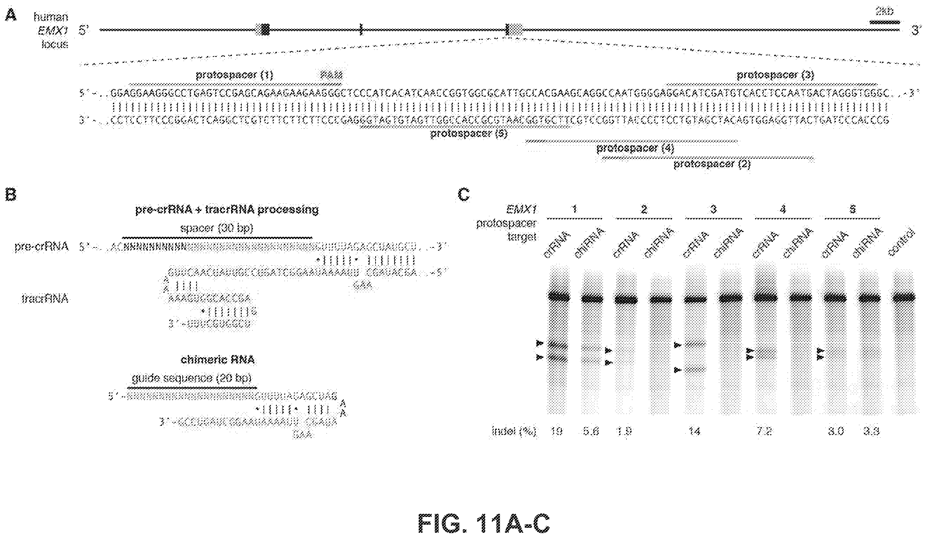

To further simplify the three-component system, a chimeric crRNA-tracrRNA hybrid design was adapted, where a mature crRNA (comprising a guide sequence) may be fused to a partial tracrRNA via a stem-loop to mimic the natural crRNA:tracrRNA duplex. To increase co-delivery efficiency, a bicistronic expression vector was created to drive co-expression of a chimeric RNA and SpCas9 in transfected cells. In parallel, the bicistronic vectors were used to express a pre-crRNA (DR-guide sequence-DR) with SpCas9, to induce processing into crRNA with a separately expressed tracrRNA (compare Fig. 11B top and bottom). Fig. 8 provides schematic illustrations of bicistronic expression vectors for pre-crRNA array (Figure 8A) or chimeric crRNA (represented by the short line downstream of the guide sequence insertion site and upstream of the EF1α promoter in Fig. 8B) with hSpCas9, showing location of various elements and the point of guide sequence insertion. The expanded sequence around the location of the guide sequence insertion site in Fig. 8B also shows a partial DR sequence (GTTTAGAGCTA (SEQ ID NO: 90)) and a partial tracrRNA sequence (TAGCAAGTTAAAATAAGGCTAGTCCGTTTTT (SEQ ID NO: 91)). Guide sequences can be inserted between BbsI sites using annealed oligonucleotides. Sequence design for the oligonucleotides are shown below the schematic illustrations in Fig. 8, with appropriate ligation adapters indicated. WPRE represents the Woodchuck hepatitis virus posttranscriptional regulatory element. The efficiency of chimeric RNA-mediated cleavage was tested by targeting the same *EMX1* locus described above. Using both Surveyor assay and Sanger sequencing of amplicons, Applicants confirmed that the chimeric RNA design facilitates cleavage of human *EMX1* locus with approximately a 4.7% modification rate (Fig. 3).

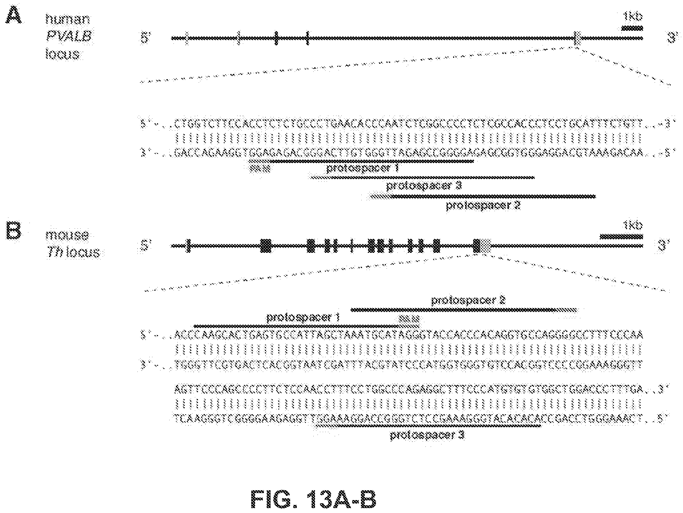

Generalizability of CRISPR-mediated cleavage in eukaryotic cells was tested by targeting additional genomic loci in both human and mouse cells by designing chimeric RNA targeting multiple sites in the human *EMX1* and *PVALB,* as well as the mouse *Th* loci. Fig. 13 illustrates the selection of some additional targeted protospacers in human *PVALB* (Fig. 13A) and mouse *Th* (Fig. 13B) loci. Schematics of the gene loci and the location of three protospacers within the last exon of each are provided. The underlined sequences include 30bp of protospacer sequence and 3bp at the 3' end corresponding to the PAM sequences. Protospacers on the sense and anti-sense strands are indicated above and below the DNA sequences, respectively. A modification rate of 6.3% and 0.75% was achieved for the human *PVALB* and mouse *Th* loci respectively, demonstrating the broad applicability of the CRISPR system in modifying different loci across multiple organisms (Fig. 5). While cleavage was only detected with one out of three spacers for each locus using the chimeric constructs, all target sequences were cleaved with efficiency of indel production reaching 27% when using the co-expressed pre-crRNA arrangement (Figs. 6 and 13).

Fig. 11 provides a further illustration that SpCas9 can be reprogrammed to target multiple genomic loci in mammalian cells. Fig. 11A provides a schematic of the human *EMX1* locus showing the location of five protospacers, indicated by the underlined sequences. Fig. 11B provides a schematic of the pre-crRNA/trcrRNA complex showing hybridization between the direct repeat region of the pre-crRNA and tracrRNA (top), and a schematic of a chimeric RNA design comprising a 20bp guide sequence, and tracr mate and tracr sequences consisting of partial direct repeat and tracrRNA sequences hybridized in a hairpin structure (bottom). Results of a Surveyor assay comparing the efficacy of Cas9-mediated cleavage at five protospacers in the human *EMX1* locus is illustrated in Fig. 11C. Each protospacer is targeted using either processed pre-crRNA/tracrRNA complex (crRNA) or chimeric RNA (chiRNA).

Since the secondary structure of RNA can be crucial for intermolecular interactions, a structure prediction algorithm based on minimum free energy and Boltzmann-weighted structure ensemble was used to compare the putative secondary structure of all guide sequences used in the genome targeting experiment (see e.g. Gruber et al., 2008, Nucleic Acids Research, 36: W70). Analysis revealed that in most cases, the effective guide sequences in the chimeric crRNA context were substantially free of secondary structure motifs, whereas the ineffective guide sequences were more likely to form internal secondary structures that could prevent base pairing with the target protospacer DNA. It is thus possible that variability in the spacer secondary structure might impact the efficiency of CRISPR-mediated interference when using a chimeric crRNA.

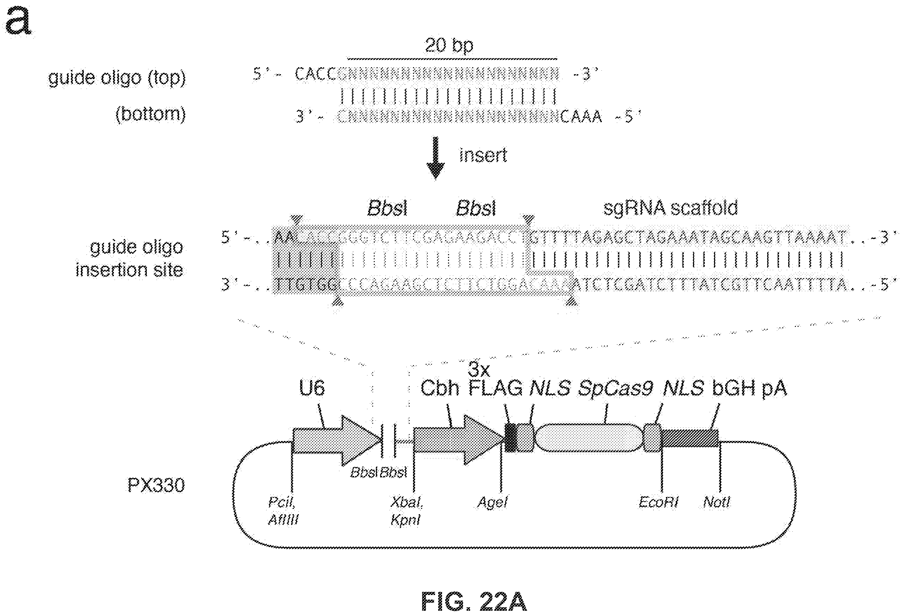

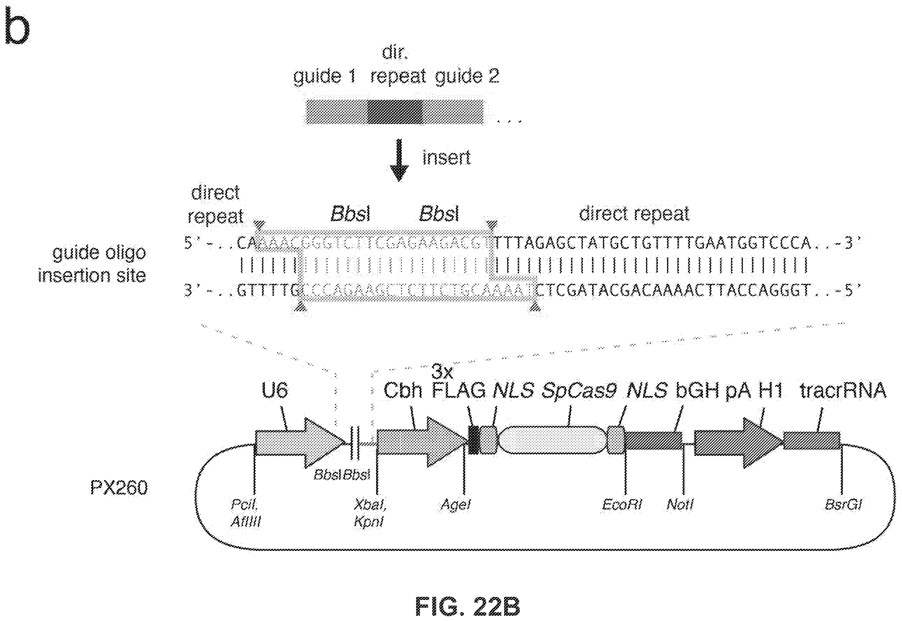

Further vector designs for SpCas9 are shown in Fig. 22, which illustrates single expression vectors incorporating a U6 promoter linked to an insertion site for a guide oligo, and a Cbh promoter linked to SpCas9 coding sequence. The vector shown in Fig. 22b includes a tracrRNA coding sequence linked to an H1 promoter.

In the bacterial assay, all spacers facilitated efficient CRISPR interference (Fig. 3C). These results suggest that there may be additional factors affecting the efficiency of CRISPR activity in mammalian cells.

To investigate the specificity of CRISPR-mediated cleavage, the effect of single-nucleotide mutations in the guide sequence on protospacer cleavage in the mammalian genome was analyzed using a series of *EMX1*-targeting chimeric crRNAs with single point mutations (Fig. 3A). Fig. 3B illustrates results of a Surveyor nuclease assay comparing the cleavage efficiency of Cas9 when paired with different mutant chimeric RNAs. Single-base mismatch up to 12-bp 5' of the PAM substantially abrogated genomic cleavage by SpCas9, whereas spacers with mutations at farther upstream positions retained activity against the original protospacer target (Fig. 3B). In addition to the PAM, SpCas9 has single-base specificity within the last 12-bp of the spacer. Furthermore, CRISPR is able to mediate genomic cleavage as efficiently as a pair of TALE nucleases (TALEN) targeting the same *EMX1* protospacer. Fig. 3C provides a schematic showing the design of TALENs targeting *EMX1,* and Fig. 3D shows a Surveyor gel comparing the efficiency of TALEN and Cas9 (n=3).

Having established a set of components for achieving CRISPR-mediated gene editing in mammalian cells through the error-prone NHEJ mechanism, the ability of CRISPR to stimulate homologous recombination (HR), a high fidelity gene repair pathway for making precise edits in the genome, was tested. The wild type SpCas9 is able to mediate site-specific DSBs, which can be repaired through both NHEJ and HR. In addition, an aspartate-to-alanine substitution (D10A) in the RuvC I catalytic domain of SpCas9 was engineered to convert the nuclease into a nickase (SpCas9n; illustrated in Fig. 4A) (see e.g. Sapranausaks et al., 2011, Nucleic Acids Resch, 39: 9275; Gasiunas et al., 2012, Proc. Natl. Acad. Sci. USA, 109:E2579), such that nicked genomic DNA undergoes the high-fidelity homology-directed repair (HDR). Surveyor assay confirmed that SpCas9n does not generate indels at the *EMX1* protospacer target. As illustrated in Fig. 4B, co-expression of *EMX1*-targeting chimeric crRNA with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting *EMX1,* hSpCas9 or hSpCas9n, as well as a HR template to introduce a pair of restriction sites (*Hind*III and *Nhe*I) near the protospacer. Fig. 4C provides a schematic illustration of the HR strategy, with relative locations of recombination points and primer annealing sequences (arrows). SpCas9 and SpCas9n indeed catalyzed integration of the HR template into the *EMX1* locus. PCR amplification of the target region followed by restriction digest with *Hind*III revealed cleavage products corresponding to expected fragment sizes (arrows in restriction fragment length polymorphism gel analysis shown in Fig. 4D), with SpCas9 and SpCas9n mediating similar levels of HR efficiencies. Applicants further verified HR using Sanger sequencing of genomic amplicons (Fig. 4E). These results demonstrate the utility of CRISPR for facilitating targeted gene insertion in the mammalian genome. Given the 14-bp (12-bp from the spacer and 2-bp from the PAM) target specificity of the wild type SpCas9, the availability of a nickase can significantly reduce the likelihood of off-target modifications, since single strand breaks are not substrates for the error-prone NHEJ pathway.

Expression constructs mimicking the natural architecture of CRISPR loci with arrayed spacers (Fig. 2A) were constructed to test the possibility of multiplexed sequence targeting. Using a single CRISPR array encoding a pair of *EMX1-* and *PVALB*-targeting spacers, efficient cleavage at both loci was detected (Fig. 4F, showing both a schematic design of the crRNA array and a Surveyor blot showing efficient mediation of cleavage). Targeted deletion of larger genomic regions through concurrent DSBs using spacers against two targets within *EMX1* spaced by 119bp was also tested, and a 1.6% deletion efficacy (3 out of 182 amplicons; Fig. 4G) was detected. This demonstrates that the CRISPR system can mediate multiplexed editing within a single genome.

*Example 2: CRISPR system modifications and alternatives*

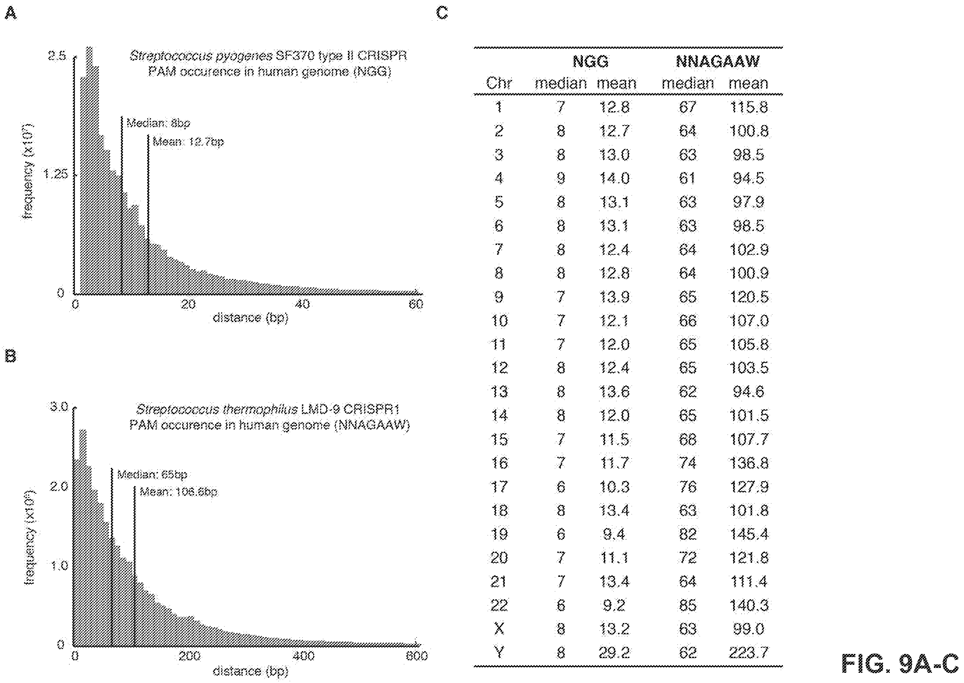

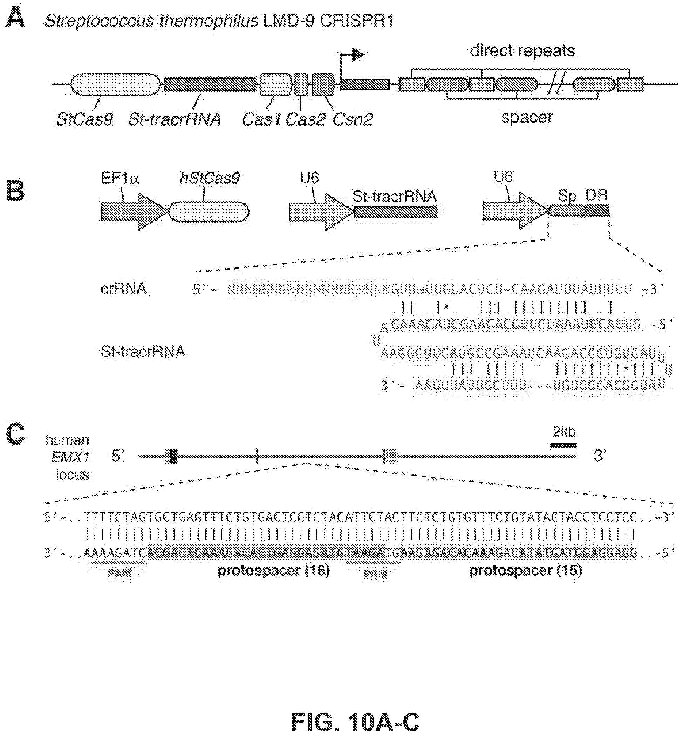

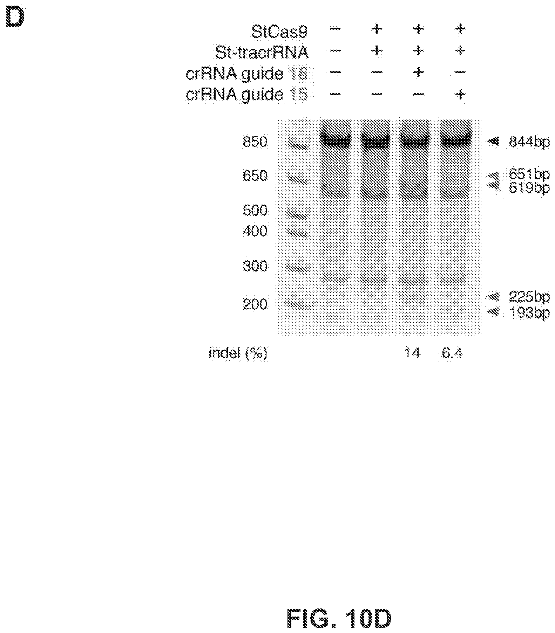

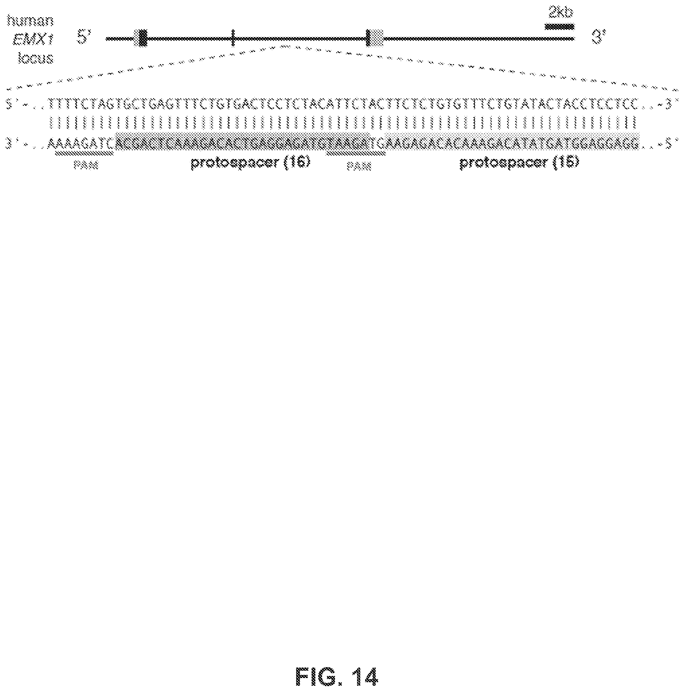

The ability to use RNA to program sequence-specific DNA cleavage defines a new class of genome engineering tools for a variety of research and industrial applications. Several aspects of the CRISPR system can be further improved to increase the efficiency and versatility of CRISPR targeting. Optimal Cas9 activity may depend on the availability of free $Mg^{2+}$ at levels higher than that present in the mammalian nucleus (see e.g. Jinek et al., 2012, Science, 337:816), and the preference for an NGG motif immediately downstream of the protospacer restricts the ability to target on average every 12-bp in the human genome (Fig. 9, evaluating both plus and minus strands of human chromosomal sequences). Some of these constraints can be overcome by exploring the diversity of CRISPR loci across the microbial metagenome (see e.g. Makarova et al., 2011, Nat Rev Microbiol, 9:467). Other CRISPR loci may be transplanted into the mammalian cellular milieu by a process similar to that described in Example 1. For example, Fig. 10 illustrates adaptation of the Type II CRISPR system from CRISPR 1 of *Streptococcus thermophilus* LMD-9 for heterologous expression in mammalian cells to achieve CRISPR-mediated genome editing. Fig. 10A provides a Schematic illustration of CRISPR 1 from *S. thermophilus* LMD-9. Figure 10B illustrates the design of an expression system for the *S. thermophilus* CRISPR system. Human codon-optimized *hStCas9* is expressed using a constitutive EF1α promoter. Mature versions of tracrRNA and crRNA are expressed using the U6 promoter to promote precise transcription initiation. Sequences from the mature crRNA and tracrRNA are illustrated. A single base indicated by the lower case "a" in the crRNA sequence is used to remove the polyU sequence, which serves as a RNA polIII transcriptional terminator. Fig. 10C provides a schematic showing guide sequences targeting the human *EMX1* locus. Fig. 10D shows the results of hStCas9-mediated cleavage in the target locus using the Surveyor assay. RNA guide spacers 1 and 2 induced 14% and 6.4%, respectively. Statistical analysis of cleavage activity across biological replica at these two protospacer sites is also provided in Fig. 5. Fig. 14 provides a schematic of additional protospacer and corresponding PAM sequence targets of the *S. thermophilus* CRISPR system in the human *EMX1* locus. Two protospacer sequences are highlighted and their corresponding PAM sequences satisfying NNAGAAW motif are indicated by underlining 3' with respect to the corresponding highlighted sequence. Both protospacers target the anti-sense strand.

*Example 3: Sample target sequence selection algorithm*

A software program is designed to identify candidate CRISPR target sequences on both strands of an input DNA sequence based on desired guide sequence length and a CRISPR motif sequence (PAM) for a specified CRISPR enzyme. For example, target sites for Cas9 from S. *pyogenes,* with PAM sequences NGG, may be identified by searching for 5'-$N_x$-NGG-3' both on the input sequence and on the reverse-complement of the input. Likewise, target sites for Cas9 of S. *thermophilus* CRISPR1, with PAM sequence NNAGAAW, may be identified by searching for 5'-$N_x$-NNAGAAW-3' both on the input sequence and on the reverse-complement of the input. Likewise, target sites for Cas9 of *S. thermophilus* CRISPR3, with PAM sequence NGGNG, may be identified by searching for 5'-$N_x$-NGGNG-3' both on the input sequence and on the reverse-complement of the input. The value "x" in $N_x$ may be fixed by the program or specified by the user, such as 20.

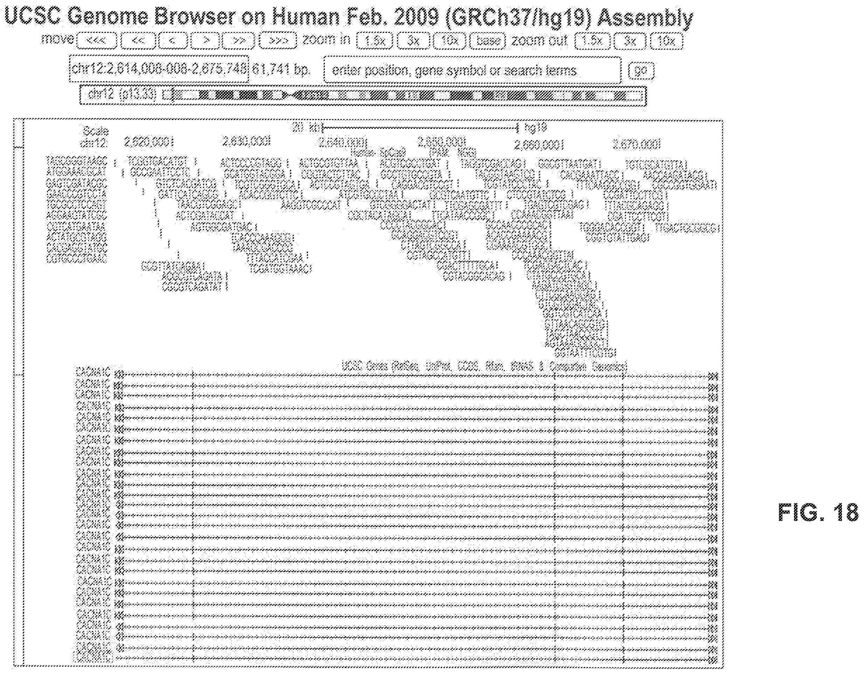

Since multiple occurrences in the genome of the DNA target site may lead to nonspecific genome editing, after identifying all potential sites, the program filters out sequences based on the number of times they appear in the relevant reference genome. For those CRISPR enzymes for which sequence specificity is determined by a 'seed' sequence, such as the 11-12bp 5' from the PAM sequence, including the PAM sequence itself, the filtering step may be based on the seed sequence. Thus, to avoid editing at additional genomic loci, results are filtered based on the number of occurrences of the seed:PAM sequence in the relevant genome. The user may be allowed to choose the length of the seed sequence. The user may also be allowed to specify the number of occurrences of the seed:PAM sequence in a genome for purposes of passing the filter. The default is to screen for unique sequences. Filtration level is altered by changing both the length of the seed sequence and the number of occurrences of the sequence in the genome. The program may in addition or alternatively provide the sequence of a guide sequence complementary to the reported target sequence(s) by providing the reverse complement of the identified target sequence(s). An example visualization of some target sites in the human genome is provided in Fig. 18.

Further details of methods and algorithms to optimize sequence selection can be found in U.S. application Serial No. 61/064,798 (Attorney docket 44790.11.2022; Broad Reference BI-2012/084).

*Example 4: Evaluation of multiple chimeric crRNA-tracrRNA hybrids*

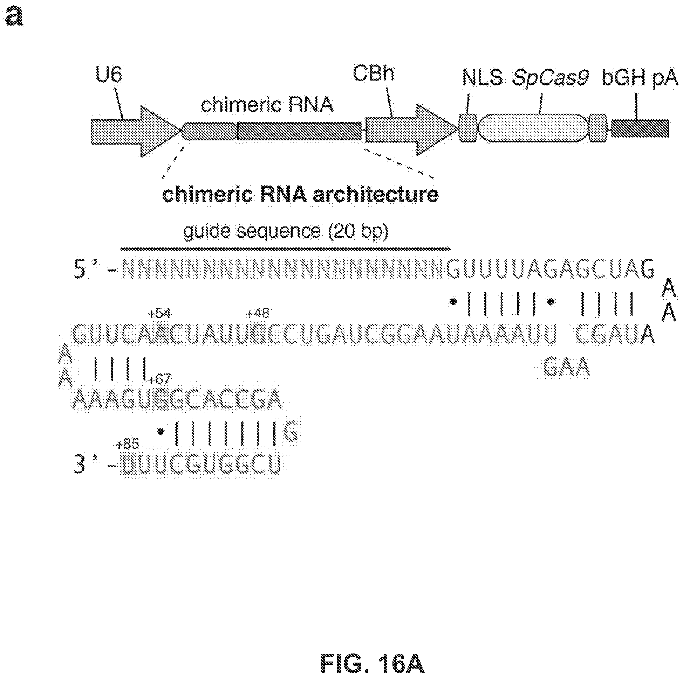

This example describes results obtained for chimeric RNAs (chiRNAs; comprising a guide sequence, a tracr mate sequence, and a tracr sequence in a single transcript) having tracr sequences that incorporate different lengths of wild-type tracrRNA sequence. Fig.16a illustrates a schematic of a bicistronic expression vector for chimeric RNA and Cas9. Cas9 is driven by the CBh promoter and the chimeric RNA is driven by a U6 promoter. The chimeric guide RNA consists of a 20bp guide sequence (Ns) joined to the tracr sequence (running from the first "U" of the lower strand to the end of the transcript), which is truncated at various positions as indicated. The guide and tracr sequences are separated by the tracr-mate sequence GUUUUAGAGCUA (SEQ ID NO: 63) followed by the loop sequence GAAA. Results of

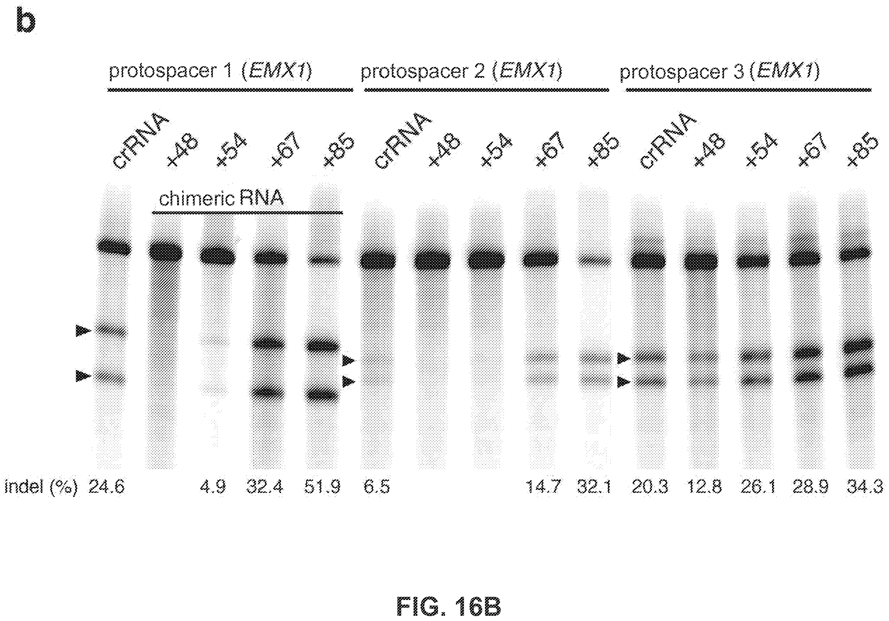

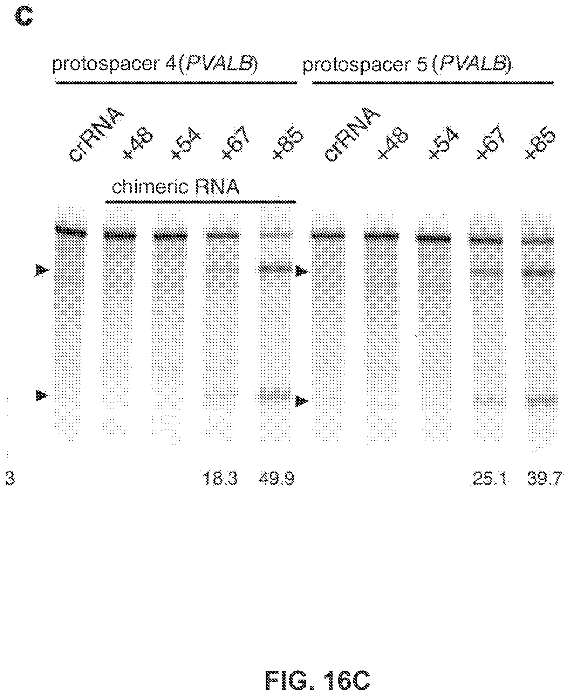

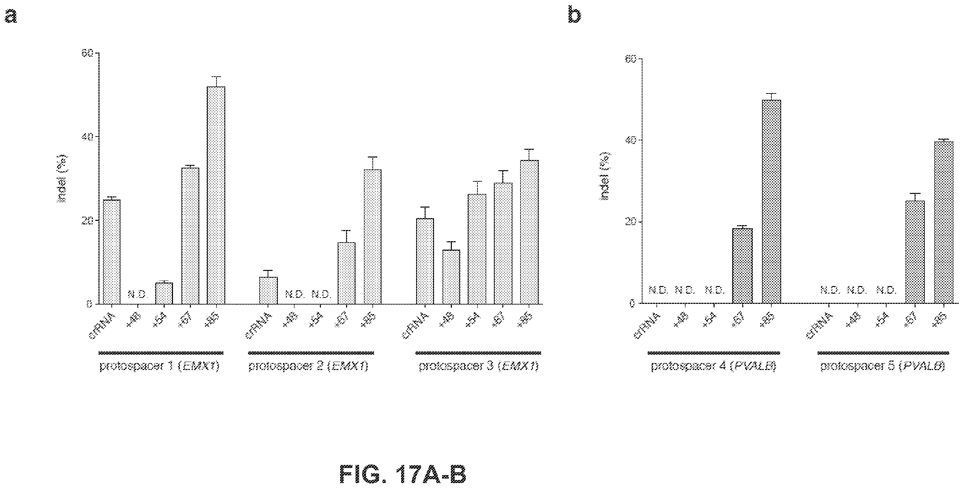

SURVEYOR assays for Cas9-mediated indels at the human *EMX1* and *PVALB* loci are illustrated in Figs. 16b and 16c, respectively. Arrows indicate the expected SURVEYOR fragments. ChiRNAs are indicated by their "+n" designation, and crRNA refers to a hybrid RNA where guide and tracr sequences are expressed as separate transcripts. Quantification of these results, performed in triplicate, are illustrated by histogram in Figs. 17a and 17b, corresponding to Figs. 16b and 16c, respectively ("N.D." indicates no indels detected). Protospacer IDs and their corresponding genomic target, protospacer sequence, PAM sequence, and strand location are provided in Table D. Guide sequences were designed to be complementary to the entire protospacer sequence in the case of separate transcripts in the hybrid system, or only to the underlined portion in the case of chimeric RNAs.

**Table D:**

| protospacer ID | genomic target | protospacer sequence (5' to 3') | PAM | strand |
|:---:|:---:|:---:|:---:|:---:|
| 1 | *EMX1* | GGACATCGATGTCACCTCCAATGACTAGGG | TGG | + |
| 2 | *EMX1* | CATTGGAGGTGACATCGATGTCCTCCCCAT | TGG | - |
| 3 | *EMX1* | GGAAGGGCCTGAGTCCGAGCAGAAGAAGAA | GGG | + |
| 4 | *PVALB* | GGTGGCGAGAGGGGCCGAGATTGGGTGTTC | AGG | + |
| 5 | *PVALB* | ATGCAGGAGGGTGGCGAGAGGGGCCGAGAT | TGG | + |

(SEQ ID NOS: 93 to 97_ respectively.)

Further details to optimize guide sequences can be found in U.S. application Serial No. 61/836,127 (Attorney docket 44790.08.2022; Broad Reference BI-2013/004G).

Initially, three sites within the EMX1 locus in human HEK 293FT cells were targeted. Genome modification efficiency of each chiRNA was assessed using the SURVEYOR nuclease assay, which detects mutations resulting from DNA double-strand breaks (DSBs) and their subsequent repair by the non-homologous end joining (NHEJ) DNA damage repair pathway. Constructs designated chiRNA(+n) indicate that up to the +n nucleotide of wild-type tracrRNA is included in the chimeric RNA construct, with values of 48, 54, 67, and 85 used for n. Chimeric RNAs containing longer fragments of wild-type tracrRNA (chiRNA(+67) and chiRNA(+85)) mediated DNA cleavage at all three EMX1 target sites, with chiRNA(+85) in particular demonstrating significantly higher levels of DNA cleavage than the corresponding crRNA/tracrRNA hybrids that expressed guide and tracr sequences in separate transcripts (Figs. 16b and 17a). Two sites in the PVALB locus that yielded no detectable cleavage using the hybrid system (guide sequence and tracr sequence expressed as separate transcripts) were also targeted using chiRNAs. chiRNA(+67) and chiRNA(+85) were able to mediate significant cleavage at the two PVALB protospacers (Figs. 16c and 17b).

For all five targets in the EMX1 and PVALB loci, a consistent increase in genome modification efficiency with increasing tracr sequence length was observed. Without wishing to be bound by any theory, the secondary structure formed by the 3' end of the tracrRNA may play a role in enhancing the rate of CRISPR complex formation.

*Example 5: Cas9 diversity*

The CRISPR-Cas system is an adaptive immune mechanism against invading exogenous DNA employed by diverse species across bacteria and archaea. The type II CRISPR-Cas9 system consists of a set of genes encoding proteins responsible for the "acquisition" of foreign DNA into the CRISPR locus, as well as a set of genes encoding the "execution" of the DNA cleavage mechanism; these include the DNA nuclease (Cas9), a non-coding transactivating crRNA (tracrRNA), and an array of foreign DNA-derived spacers flanked by direct repeats (crRNAs). Upon maturation by Cas9, the tracRNA and crRNA duplex guide the Cas9 nuclease to a target DNA sequence specified by the spacer guide sequences, and mediates double-stranded breaks in the DNA near a short sequence motif in the target DNA that is required for cleavage and specific to each CRISPR-Cas system. The type II CRISPR-Cas systems are found throughout the bacterial kingdom and highly diverse in in Cas9 protein sequence and size, tracrRNA and crRNA direct repeat sequence, genome organization of these elements, and the motif requirement for target cleavage. One species may have multiple distinct CRISPR-Cas systems.

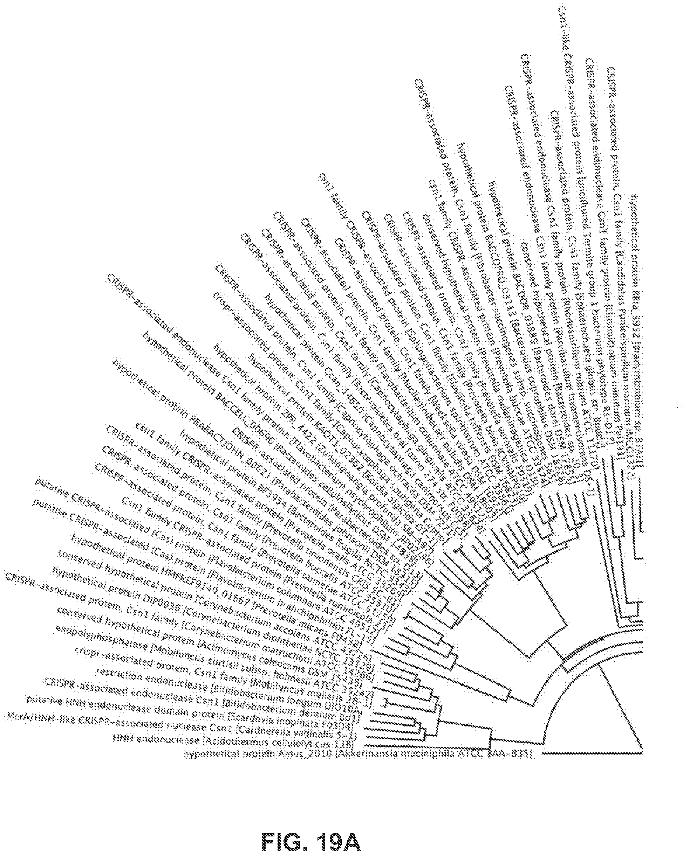

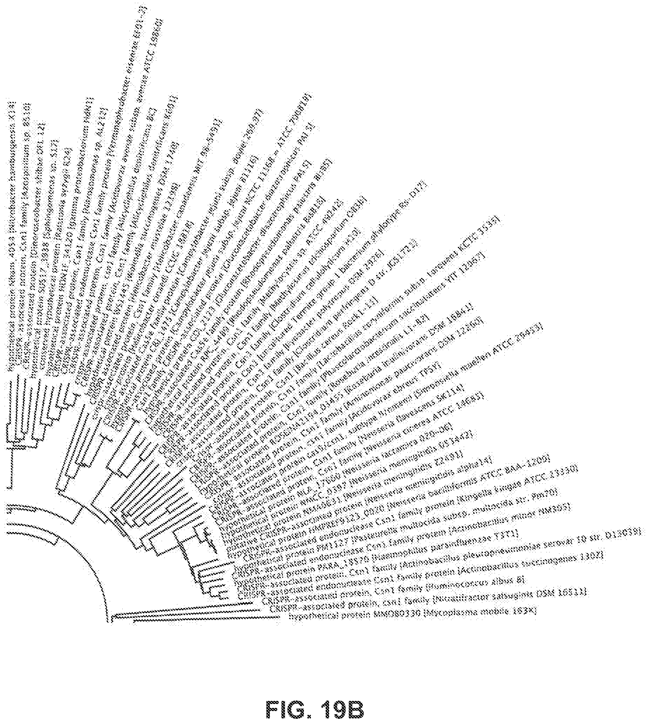

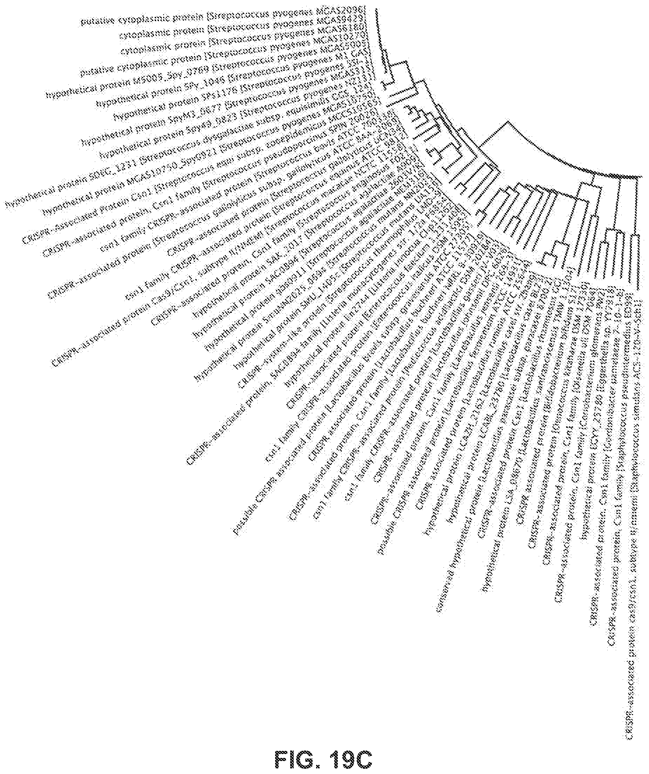

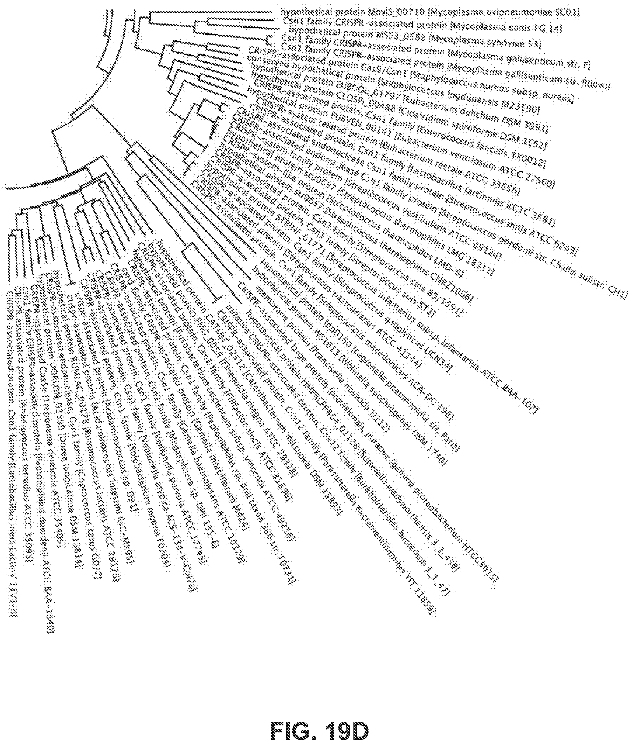

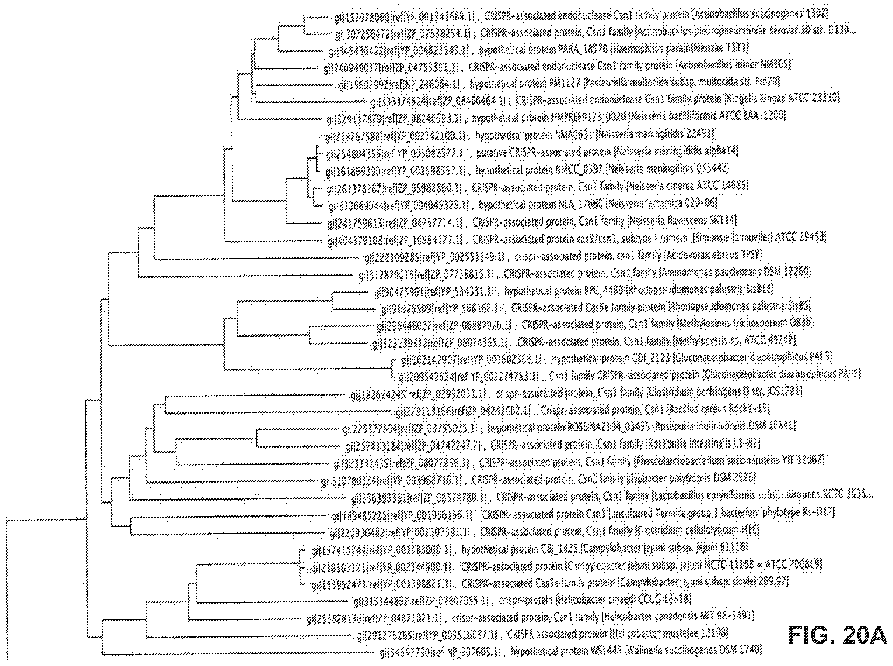

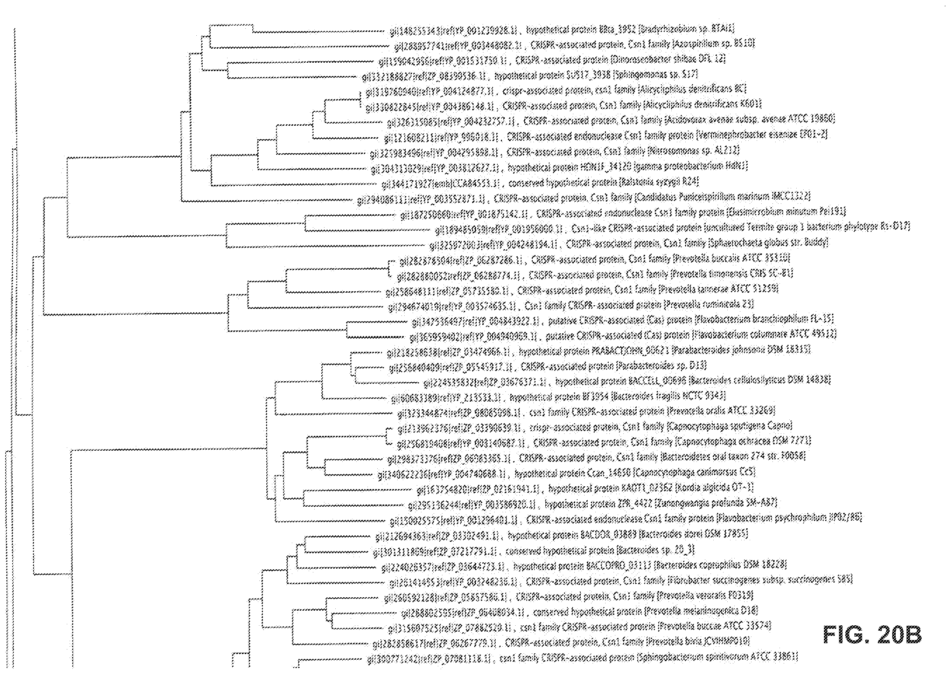

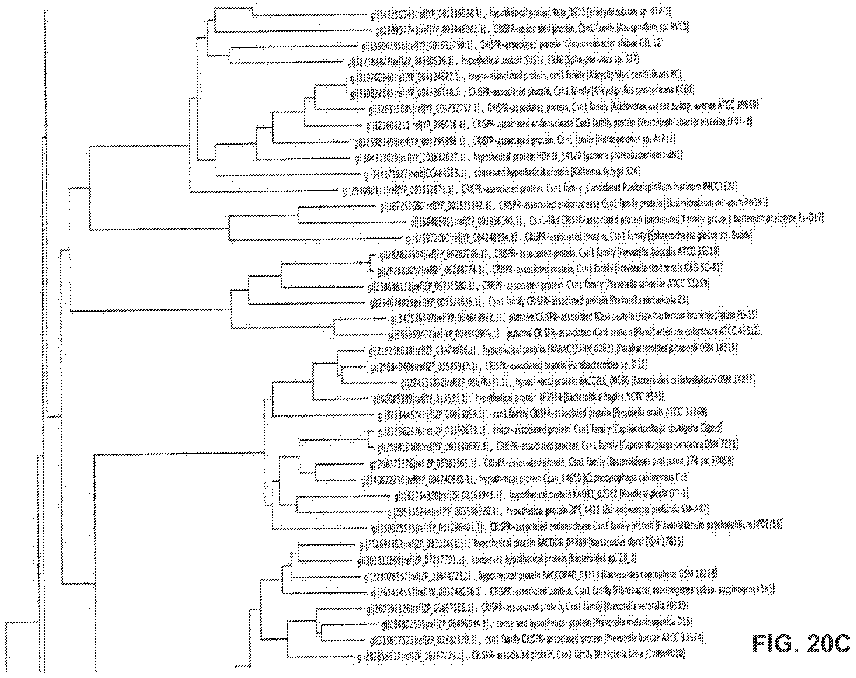

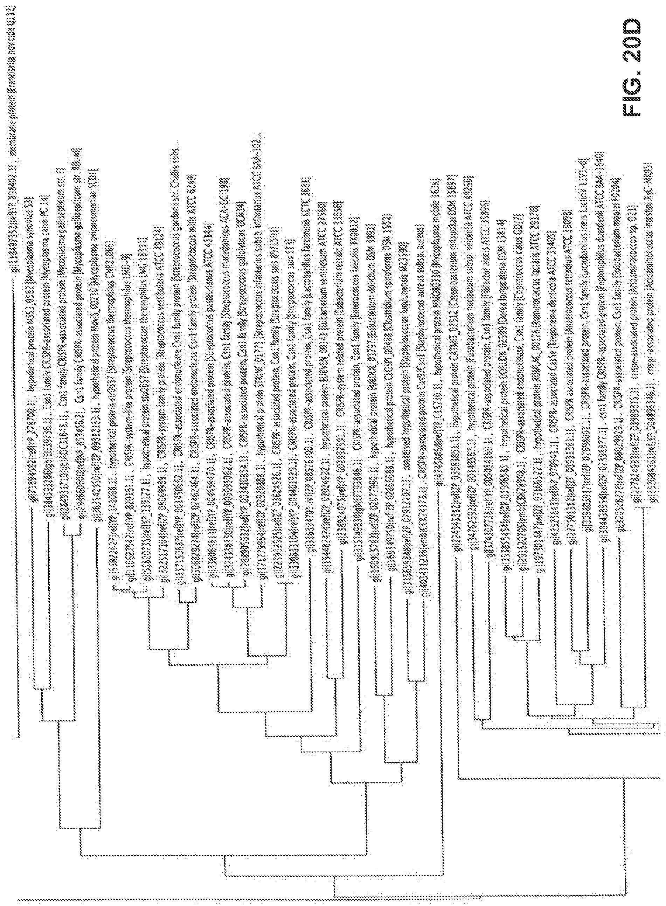

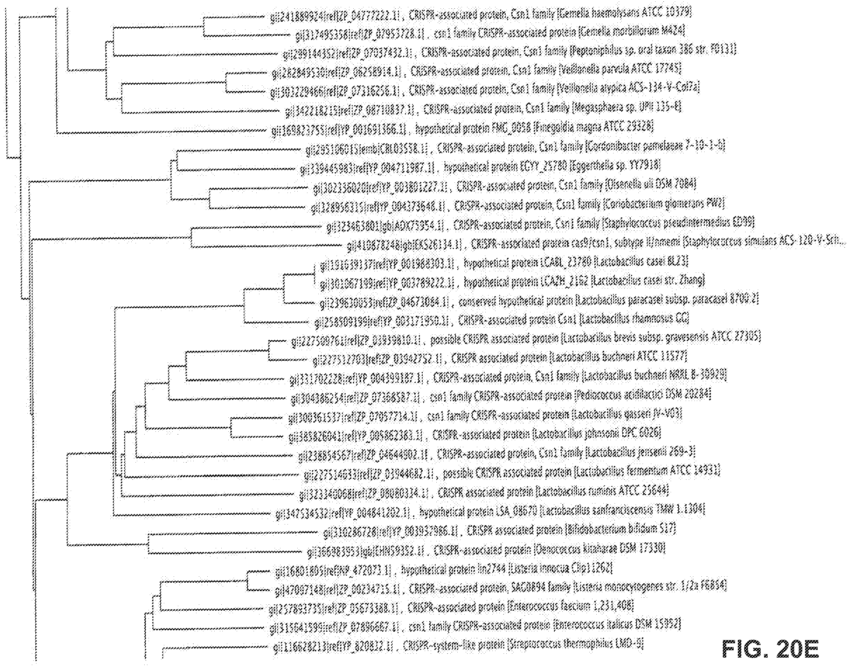

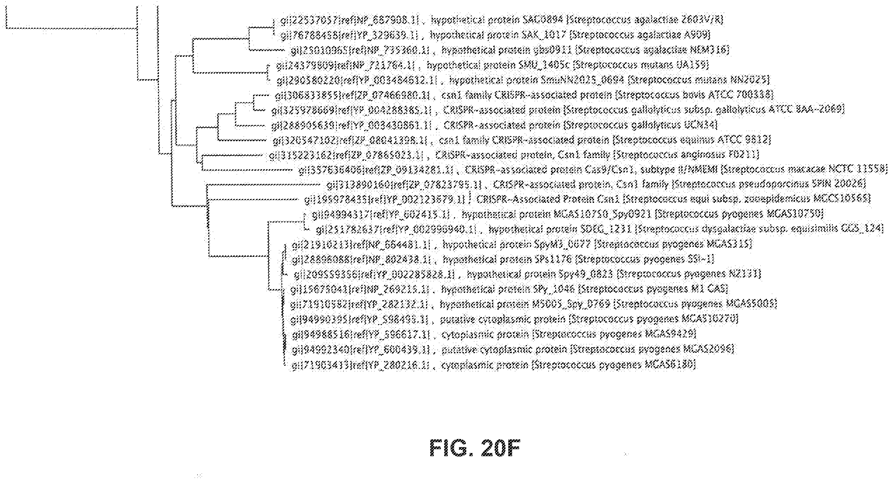

Applicants evaluated 207 putative Cas9s from bacterial species identified based on sequence homology to known Cas9s and structures orthologous to known subdomains, including the HNH endonuclease domain and the RuvC endonuclease domains [information from the Eugene Koonin and Kira Makarova]. Phylogenetic analysis based on the protein sequence conservation of this set revealed five families of Cas9s, including three groups of large Cas9s (~1400 amino acids) and two of small Cas9s (~1100 amino acids) (see Figs. 19 and 20A-F).

Further details of Cas9s and mutations of the Cas9 enzyme to convert into a nickase or DNA binding protein and use of same with altered functionality can be found in U.S. application Serial Nos 61/836,101 and 61/835,936 (Attorney docket 44790.09.2022 and 4790.07.2022 and Broad Reference BI-2013/004E and BI-2013/004F respectively).

*Example 6: Cas9 orthologs*

Applicants analyzed Cas9 orthologs to identify the relevant PAM sequences and the corresponding chimeric guide RNA. Having an expanded set of PAMs provides broader targeting across the genome and also significantly increases the number of unique target sites and provides potential for identifying novel Cas9s with increased levels of specificity in the genome.

The specificity of Cas9 orthologs can be evaluated by testing the ability of each Cas9 to tolerate mismatches between the guide RNA and its DNA target. For example, the specificity of SpCas9 has been characterized by testing the effect of mutations in the guide RNA on cleavage efficiency. Libraries of guide RNAs were made with single or multiple mismatches between the guide sequence and the target DNA. Based on these findings, target sites for SpCas9 can be selected based on the following guidelines:

To maximize SpCas9 specificity for editing a particular gene, one should choose a target site within the locus of interest such that potential 'off-target' genomic sequences abide by the following four constraints: First and foremost, they should not be followed by a PAM with either 5'-NGG or NAG sequences. Second, their global sequence similarity to the target sequence should be minimized. Third, a maximal number of mismatches should lie within the PAM-proximal region of the off-target site. Finally, a maximal number of mismatches should be consecutive or spaced less than four bases apart.

Similar methods can be used to evaluate the specificity of other Cas9 orthologs and to establish criteria for the selection of specific target sites within the genomes of target species. As mentioned previously phylogenetic analysis based on the protein sequence conservation of this set revealed five families of Cas9s, including three groups of large Cas9 (~1400 amino acids) and two of small Cas9s (~1100 amino acids) (see Figs. 19 and 20A-F). Further details on Cas orthologs can be found in U.S. application Serial Nos 61/836,101 and 61/835,936 (Attorney docket 44790.09.2022 and 4790.07.2022 and Broad Reference BI-2013/004E and BI-2013/004F respectively).

*Example 7: Methodological improvement to simplify cloning and delivery.*

Rather than encoding the U6-promoter and guide RNA on a plasmid, Applicants amplified the U6 promoter with a DNA oligo to add on the guide RNA. The resulting PCR product may be transfected into cells to drive expression of the guide RNA.

Example primer pair that allows the generation a PCR product consisting of U6-promoter::guideRNA targeting human Emx1 locus:

Forward Primer: AAACTCTAGAgagggcctatttcccatgattc (SEQ ID NO:)

Reverse Primer (carrying the guide RNA, which is underlined):

acctctag<u>AAAAAAAGCACCGACTCGGTGCCACTTTTTCAAGTTGATAACGGACTAGC</u>
<u>CTTATTTTAACTTGCTATGCTGTTTTGTTTCCAAAACAGCATAGCTCTAAAACCCC</u>
<u>TAGTCATTGGA</u>GGTGACGGTGTTTCGTCCTTTCCACaag (SEQ ID NO: 99 )

*Example 8: Methodological improvement to improve activity:*

Rather than use pol3 promoters, in particular RNA polymerase III (e.g. U6 or H1 promoters), to express guide RNAs in eukaryotic cells, Applicants express the T7 polymerase in eukaryotic cells to drive expression of guide RNAs using the T7 promoter.

One example of this system may involve introduction of three pieces of DNA:

1. expression vector for Cas9
2. expression vector for T7 polymerase
3. expression vector containing guideRNA fused to the T7 promoter

*Example 9: Methodological improvement to reduce toxicity of Cas9: Delivery of Cas9 in the form of mRNA.*

Delivery of Cas9 in the form of mRNA enables transient expression of Cas9 in cells, to reduce toxicity. For example, humanized SpCas9 may be amplified using the following primer pair:

Forward Primer (to add on T7 promoter for in vitro transcription): TAATACGACTCACTATAGGAAGTGCGCCACCATGGCCCCAAAGAAGAAGCGG (SEQ ID NO: 100)

Reverse Primer (to add on polyA tail): GGTTTTTTTTTTTTTTTTTTTTTTTTTTTTTTTTTttcttaCTTTTTCTTTTTTGCCT-GGCCG (SEQ ID NO: 101)

Applicants transfect the Cas9 mRNA into cells with either guide RNA in the form of RNA or DNA cassettes to drive guide RNA expression in eukaryotic cells.

*Example 10: Methodological improvement to reduce toxicity of Cas9: Use of an inducible promoter*

Applicants transiently turn on Cas9 expression only when it is needed for carrying out genome modification. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochrome).

*Example 11: Improvement of the Cas9 system for in vivo application*

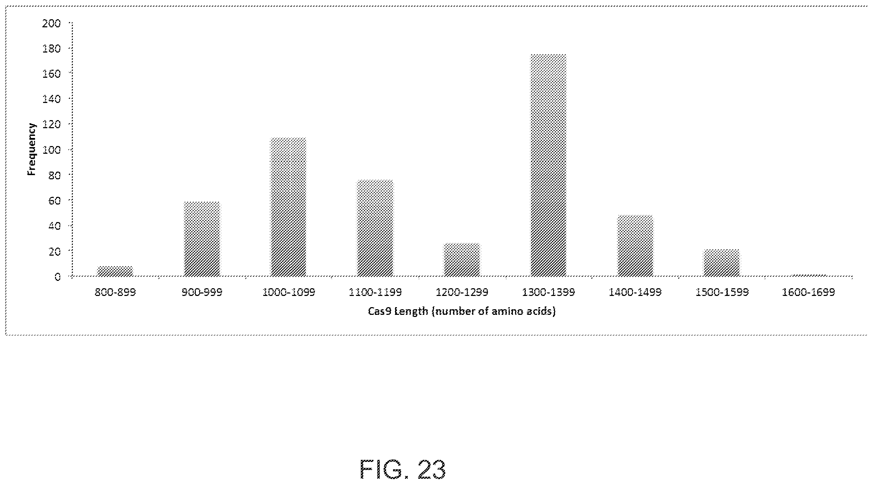

Applicants conducted a Metagenomic search for a Cas9 with small molecular weight. Most Cas9 homologs are fairly large. For example the SpCas9 is around 1368aa long, which is too large to be easily packaged into viral vectors for delivery. A graph representing the length distribution of Cas9 homologs is generated from sequences deposited in GenBank (Fig. 23). Some of the sequences may have been mis-annotated and therefore the exact frequency for each length may not necessarily be accurate. Nevertheless it provides a glimpse at distribution of Cas9 proteins and suggest that there are shorter Cas9 homologs.

Through computational analysis, Applicants found that in the bacterial strain *Campylobacter,* there are two Cas9 proteins with less than 1000 amino acids. The sequence for one Cas9 from *Campylobacter jejuni* is presented below. At this length, CjCas9 can be easily packaged into AAV, lentiviruses, Adenoviruses, and other viral vectors for robust delivery into primary cells and *in vivo* in animal models. In a preferred embodiment of disclosure, the Cas9 protein from *S. aureus* is used.

>Campylobacter jejuni Cas9 (CjCas9)

MARILAFDIGISSIGWAFSENDELKDCGVRIFTKVENPKTGESLALPRRLAR

SARKRLARRKARLNHLKHLIANEFKLNYEDYQSFDESLAKAYKGSLISPYELRFRALN

ELLSKQDFARVILHIAKRRGYDDIKNSDDKEKGAILKAIKQNEEKLANYQSVGEYLYK

EYFQKFKENSKEFTNVRNKKESYERCIAQSFLKDELKLIFKKQREFGFSFSKKFEEEVL

SVAFYKRALKDFSHLVGNCSFFTDEKRAPKNSPLAFMFVALTRIINLLNNLKNTEGIL

YTKDDLNALLNEVLKNGTLTYKQTKKLLGLSDDYEFKGEKGTYFIEFKKYKEFIKAL

GEHNLSQDDLNEIAKDITLIKDEIKLKKALAKYDLNQNQIDSLSKLEFKDHLNISFKAL

KLVTPLMLEGKKYDEACNELNLKVAINEDKKDFLPAFNETYYKDEVTNPVVLRAIKE

YRKVLNALLKKYGKVHKINIELAREVGKNHSQRAKIEKEQNENYKAKKDAELECEK

LGLKINSKNILKLRLFKEQKEFCAYSGEKIKISDLQDEKMLEIDHIYPYSRSFDDSYMN

KVLVFTKQNQEKLNQTPFEAFGNDSAKWQKIEVLAKNLPTKKQKRILDKNYKDKEQ

KNFKDRNLNDTRYIARLVLNYTKDYLDFLPLSDDENTKLNDTQKGSKVHVEAKSGM

LTSALRHTWGFSAKDRNNHLHHAIDAVIIAYANNSIVKAFSDFKKEQESNSAELYAK

KISELDYKNRKFFEPFSGFRQKVLDKIDEIFVSKPERKKPSGALHEETFRKEEEFYQS

YGGKEGVLKALELGKIRKVNGKIVKNGDMFRVDIFKHKKTNKFYAVPIYTMDFALK

VLPNKAVARSKKGEIKDWILMDENYEFCFSLYKDSLILIQTKDMQEPEFVYYNAFTSS

TVSLIVSKHDNKFETLSKNQKILFKNANEKEVIAKSIGIQNLKVFEKYIVSALGEVTKA

EFRQREDFKK. (SEQ ID NO:102   )

The putative tracrRNA element for this CjCas9 is:

TATAATCTCATAAGAAATTTAAAAAGGGACTAAAATAAAGAGTTTGCG

GGACTCTGCGGGGTTACAATCCCCTAAAACCGCTTTTAAAATT (SEQ ID NO: 103   )

The Direct Repeat sequence is:
ATTTTACCATAAAGAAATTTAAAAAGGGACTAAAAC (SEQ ID NO: 104)

An example of a chimeric guideRNA for CjCas9 is:

NNNNNNNNNNNNNNNNNNNNNNGUUUUAGUCCCGAAAGGGACUAAAAU AAAGAGUUUGCGGGACUCUGCGGGGUUACAAUCCCCUAAAACCGCUUUU (SEQ ID NO: 105 )

*Example 12: Cas9 optimization*

For enhanced function or to develop new functions, Applicants generate chimeric Cas9 proteins by combining fragments from different Cas9 homologs. For example, two example chimeric Cas9 proteins:
For example, Applicants fused the N-term of St1Cas9 (fragment from this protein is in bold) with C-term of SpCas9 (fragment from this protein is underlined).

>St1(N)Sp(C)Cas9

**MSDLVLGLDIGIGSVGVGILNKVTGEIIHKNSRIFPAAQAENNLVRRTN RQGRRLARRKKHRRVRLNRLFEESGLITDFTKISINLNPYQLRVKGLTDELSNEE LFIALKNMVKHRGISYLDDASDDGNSSVGDYAQIVKENSKQLETKTPGQIQLERY QTYGQLRGDFTVEKDGKKHRLINVFPTSAYRSEALRILQTQQEFNPQITDEFINR YLEILTGKRKYYHGPGNEKSRTDYGRYRTSGETLDNIFGILIGKCTFYPDEFRAAK ASYTAQEFNLLNDLNNLTVPTETKKLSKEQKNQIINYVKNEKAMGPAKLFKYIAK LLSCDVADIKGYRIDKSGKAEIHTFEAYRKMKTLETLDIEQMDRETLDKLAYVLT**

**LNTEREGIQEALEHEFADGSFSQKQVDELVQFRKANSSIFGKGWHNFSVKLMME LIPELYETSEEQMTILTRLGKQKTTSSSNKTKYIDEKLLTEEIYNPVVAKSVRQAIK IVNAAIKEYGDFDNIVIEMARE**<u>NQTTQKGQKNSRERMKRIEEGIKELGSQILKEHPVE NTQLQNEKLYLYYLQNGRDMYVDQELDINRLSDYDVDHIVPQSFLKDDSIDNKVLT RSDKNRGKSDNVPSEEVVKKMKNYWRQLLNAKLITQRKFDNLTKAERGGLSELDK AGFIKRQLVETRQITKHVAQILDSRMNTKYDENDKLIREVKVITLKSKLVSDFRKDFQ FYKVREINNYHHAHDAYLNAVVGTALIKKYPKLESEFVYGDYKVYDVRKMIAKSEQ EIGKATAKYFFYSNIMNFFKTEITLANGEIRKRPLIETNGETGEIVWDKGRDFATVRKV LSMPQVNIVKKTEVQTGGFSKESILPKRNSDKLIARKKDWDPKKYGGFDSPTVAYSV LVVAKVEKGKSKKLKSVKELLGITIMERSSFEKNPIDFLEAKGYKEVKKDLIIKLPKYS LFELENGRKRMLASAGELQKGNELALPSKYVNFLYLASHYEKLKGSPEDNEQKQLF VEQHKHYLDEIIEQISEFSKRVILADANLDKVLSAYNKHRDKPIREQAENIIHLFTLTNL GAPAAFKYFDTTIDRKRYTSTKEVLDATLIHQSITGLYETRIDLSQLGGD </u>(SEQ ID NO: 106 )

>Sp(N)St1(C)Cas9

MDKKYSIGLDIGTNSVGWAVITDEYKVPSKKFKVLGNTDRHSIKKNLIGA
LLFDSGETAEATRLKRTARRRYTRRKNRICYLQEIFSNEMAKVDDSFFHRLEESFLVE
EDKKHERHPIFGNIVDEVAYHEKYPTIYHLRKKLVDSTDKADLRLIYLALAHMIKFRG
HFLIEGDLNPDNSDVDKLFIQLVQTYNQLFEENPINASGVDAKAILSARLSKSRRLENL
IAQLPGEKKNGLFGNLIALSLGLTPNFKSNFDLAEDAKLQLSKDTYDDDLDNLLAQIG
DQYADLFLAAKNLSDAILLSDILRVNTEITKAPLSASMIKRYDEHHQDLTLLKALVRQ
QLPEKYKEIFFDQSKNGYAGYIDGGASQEEFYKFIKPILEKMDGTEELLVKLNREDLL
RKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYE
YFTVYNELTKVKYVTEGMRKPAFLSGEQKKAIVDLLFKTNRKVTVKQLKEDYFKKIE
CFDSVEISGVEDRFNASLGTYHDLLKIIKDKDFLDNEENEDILEDIVLTLTLFEDREMIE
ERLKTYAHLFDDKVMKQLKRRRYTGWGRLSRKLINGIRDKQSGKTILDFLKSDGFAN
RNFMQLIHDDSLTFKEDIQKAQVSGQGDSLHEHIANLAGSPAIKKGILQTVKVVDELV
KVMGRHKPENIVIEMARE**TNEDDEKKAIQKIQKANKDEKDAAMLKAANQYNGKA
ELPHSVFHGHKQLATKIRLWHQQGERCLYTGKTISIHDLINNSNQFEVDHILPLSI
TFDDSLANKVLVYATANQEKGQRTPYQALDSMDDAWSFRELKAFVRESKTLSNK
KKEYLLTEEDISKFDVRKKFIERNLVDTRYASRVVLNALQEHFRAHKIDTKVSVVR
GQFTSQLRRHWGIEKTRDTYHHHAVDALIIAASSQLNLWKKQKNTLVSYSEDQL
LDIETGELISDDEYKESVFKAPYQHFVDTLKSKEFEDSILFSYQVDSKFNRKISDATI
YATRQAKVGKDKADETYVLGKIKDIYTQDGYDAFMKIYKKDKSKFLMYRHDPQT
FEKVIEPILENYPNKQINEKGKEVPCNPFLKYKEEHGYIRKYSKKGNGPEIKSLKYY
DSKLGNHIDITPKDSNNKVVLQSVSPWRADVYFNKTTGKYEILGLKYADLQFEKG
TGTYKISQEKYNDIKKKEGVDSDSEFKFTLYKNDLLLVKDTETKEQQLFRFLSRT
MPKQKHYVELKPYDKQKFEGGEALIKVLGNVANSGQCKKGLGKSNISIYKVRTD
VLGNQHIIKNEGDKPKLDF** (SEQ ID NO: 107 )

The benefit of making chimeric Cas9 include:

> reduce toxicity
> improve expression in eukaryotic cells
> enhance specificity
> reduce molecular weight of protein, make protein smaller by combining the smallest domains from different Cas9
> homologs.

Altering the PAM sequence requirement

*Example 13: Utilization of Cas9 as a generic DNA binding protein*

Applicants used Cas9 as a generic DNA binding protein by mutating the two catalytic domains (D10 and H840) responsible for cleaving both strands of the DNA target. In order to upregulate gene transcription at a target locus Applicants fused the transcriptional activation domain (VP64) to Cas9. Applicants hypothesized that it would be important to see strong nuclear localization of the Cas9-VP64 fusion protein because transcription factor activation strength is a function of time spent at the target. Therefore, Applicants cloned a set of Cas9-VP64-GFP constructs, transfected them into 293 cells and assessed their localization under a fluorescent microscope 12 hours post-transfection.

The same constructs were cloned as a 2A-GFP rather than a direct fusion in order to functionally test the constructs without a bulky GFP present to interfere. Applicants elected to target the Sox2 locus with the Cas9 transactivator because it could be useful for cellular reprogram and the locus has already been validated as a target for TALE-TF mediated transcriptional activation. For the Sox2 locus Applicants chose eight targets near the transcriptional start site (TSS). Each target was 20bp long with a neighboring NGG protospacer adjacent motif (PAM). Each Cas9-VP64 construct was co-transfected with each PCR generated chimeric crispr RNA (chiRNA) in 293 cells. 72 hours post transfection the transcriptional activation was assessed using RT-qPCR.

To further optimize the transcriptional activator, Applicants titrated the ratio of chiRNA (Sox2.1 and Sox2.5) to Cas9 (NLS-VP64-NLS-hSpCas9-NLS-VP64-NLS), transfected into 293 cells, and quantified using RT-qPCR. These results indicate that Cas9 can be used as a generic DNA binding domain to upregulate gene transcription at a target locus.

Applicants designed a second generation of constructs. (Table below).

| pLenti-EF1a-GFP-2A-6xHis-NLS-VP64-NLS-hSpCsn1(D10A, H840A)-NLS |
| pLenti-EF1a-GFP-2A-6xHis-NLS-VP64-NLS-hSpCsn1(D10A, H840A) |
| pLenti-EF1a-GFP-2A-6xHis-NLS-VP64-NLS-NLS-hSpCsn1(D10A, H840A) |
| pLenti-EF1a-GFP-2A-6xHis-NLS-hSpCsn1(D10A, H840A)-NLS |
| pLenti-EF1a-GFP-2A-6xHis-NLS-hSpCsn1(D10A, H840A) |
| pLenti-EF1a-GFP-2A-6xHis-NLS-NLS-hSpCsn1(D10A, H840A) |

Applicants use these constructs to assess transcriptional activation (VP64 fused constructs) and repression (Cas9 only) by RT-qPCR. Applicants assess the cellular localization of each construct using anti-His antibody, nuclease activity using a Surveyor nuclease assay, and DNA binding affinity using a gel shift assay. In a preferred embodiment of the disclosure,

the gel shift assay is an EMSA gel shift assay.

*Example 14: Cas9 transgenic and knock in mice*

To generate a mouse that expresses the Cas9 nuclease Applicants submit two general strategies, transgenic and knock in. These strategies may be applied to generate any other model organism of interest, for e.g. Rat. For each of the general strategies Applicants made a constitutively active Cas9 and a Cas9 that is conditionally expressed (Cre recombinase dependent). The constitutively active Cas9 nuclease is expressed in the following context: pCAG-NLS-Cas9-NLS-P2A-EGFP-WPRE-bGHpolyA. pCAG is the promoter, NLS is a nuclear localization signal, P2A is the peptide cleavage sequence, EGFP is enhanced green fluorescent protein, WPRE is the woodchuck hepatitis virus posttranscriptional regulatory element, and bGHpolyA is the bovine growth hormone poly-A signal sequence (Figs. 25A-B). The conditional version has one additional stop cassette element, loxP-SV40 polyA x3-loxP, after the promoter and before NLS-Cas9-NLS (i.e. pCAG-loxP-SV40polyAx3-loxP-NLS-Cas9-NLS-P2A-EGFP-WPRE-bGHpolyA). The important expression elements can be visualized as in Fig. 26. The constitutive construct should be expressed in all cell types throughout development, whereas, the conditional construct will only allow Cas9 expression when the same cell is expressing the Cre recombinase. This latter version will allow for tissue specific expression of Cas9 when Cre is under the expression of a tissue specific promoter. Moreover, Cas9 expression could be induced in adult mice by putting Cre under the expression of an inducible promoter such as the TET on or off system.

Validation of Cas9 constructs: Each plasmid was functionally validated in three ways: 1) transient transfection in 293 cells followed by confirmation of GFP expression; 2) transient transfection in 293 cells followed by immunofluorescence using an antibody recognizing the P2A sequence; and 3) transient transfection followed by Surveyor nuclease assay. The 293 cells may be 293FT or 293 T cells depending on the cells that are of interest. In a preferred embodiment the cells are 293FT cells. The results of the Surveyor were run out on the top and bottom row of the gel for the conditional and constitutive constructs, respectively. Each was tested in the presence and absence of chimeric RNA targeted to the hEMX1 locus (chimeric RNA hEMX1.1). The results indicate that the construct can successfully target the hEMX1 locus only in the presence of chimeric RNA (and Cre in the conditional case). The gel was quantified and the results are presented as average cutting efficiency and standard deviation for three samples.

Transgenic Cas9 mouse: To generate transgenic mice with constructs, Applicants inject pure, linear DNA into the pronucleus of a zygote from a pseudo pregnant CB56 female. Founders are identified, genotyped, and backcrossed to CB57 mice. The constructs were successfully cloned and verified by Sanger sequencing.

Knock in Cas9 mouse: To generate Cas9 knock in mice Applicants target the same constitutive and conditional constructs to the Rosa26 locus. Applicants did this by cloning each into a Rosa26 targeting vector with the following elements: Rosa26 short homology arm - constitutive/conditional Cas9 expression cassette - pPGK-Neo-Rosa26 long homology arm - pPGK-DTA. pPGK is the promoter for the positive selection marker Neo, which confers resistance to neomycin, a 1 kb short arm, a 4.3 kb long arm, and a negative selection diphtheria toxin (DTA) driven by PGK.

The two constructs were electroporated into R1 mESCs and allowed to grow for 2 days before neomycin selection was applied. Individual colonies that had survived by days 5-7 were picked and grown in individual wells. 5-7 days later the colonies were harvested, half were frozen and the other half were used for genotyping. Genotyping was done by genomic PCR, where one primer annealed within the donor plasmid (AttpF) and the other outside of the short homology arm (Rosa26-R) Of the 22 colonies harvested for the conditional case, 7 were positive (Left). Of the 27 colonies harvested for the constitutive case, zero were positive (Right). It is likely that Cas9 causes some level of toxicity in the mESC and for this reason there were no positive clones. To test this Applicants introduced a Cre expression plasmid into correctly targeted conditional Cas9 cells and found very low toxicity after many days in culture. The reduced copy number of Cas9 in correctly targeted conditional Cas9 cells (1-2 copies per cell) is enough to allow stable expression and relatively no cytotoxicity. Moreover, this data indicates that the Cas9 copy number determines toxicity. After electroporation each cell should get several copies of Cas9 and this is likely why no positive colonies were found in the case of the constitutive Cas9 construct. This provides strong evidence that utilizing a conditional, Cre-dependent strategy should show reduced toxicity. Applicants inject correctly targeted cells into a blastocyst and implant into a female mouse. Chimerics are identified and backcrossed. Founders are identified and genotyped.

Utility of the conditional Cas9 mouse: Applicants have shown in 293 cells that the Cas9 conditional expression construct can be activated by co-expression with Cre. Applicants also show that the correctly targeted R1 mESCs can have active Cas9 when Cre is expressed. Because Cas9 is followed by the P2A peptide cleavage sequence and then EGFP Applicants identify successful expression by observing EGFP. This same concept is what makes the conditional Cas9 mouse so useful. Applicants may cross their conditional Cas9 mouse with a mouse that ubiquitously expresses Cre (ACTB-Cre line) and may arrive at a mouse that expresses Cas9 in every cell. It should only take the delivery of chimeric RNA to induce genome editing in embryonic or adult mice. Interestingly, if the conditional Cas9 mouse is crossed with a mouse expressing Cre under a tissue specific promoter, there should only be Cas9 in the tissues that also express Cre. This approach may be used to edit the genome in only precise tissues by delivering chimeric RNA to the same tissue.

*Example 15: Cas9 diversity and chimeric RNAs*

The CRISPR-Cas system is an adaptive immune mechanism against invading exogenous DNA employed by diverse species across bacteria and archaea. The type II CRISPR-Cas system consists of a set of genes encoding proteins responsible for the "acquisition" of foreign DNA into the CRISPR locus, as well as a set of genes encoding the "execution" of the DNA cleavage mechanism; these include the DNA nuclease (Cas9), a non-coding transactivating crRNA (tracrRNA), and an array of foreign DNA-derived spacers flanked by direct repeats (crRNAs). Upon maturation by Cas9, the tracrRNA and crRNA duplex guide the Cas9 nuclease to a target DNA sequence specified by the spacer guide sequences, and mediates double-stranded breaks in the DNA near a short sequence motif in the target DNA that is required for cleavage and specific to each CRISPR-Cas system. The type II CRISPR-Cas systems are found throughout the bacterial kingdom and highly diverse in in Cas9 protein sequence and size, tracrRNA and crRNA direct repeat sequence, genome organization of these elements, and the motif requirement for target cleavage. One species may have multiple distinct CRISPR-Cas systems.

Applicants evaluated 207 putative Cas9s from bacterial species identified based on sequence homology to known Cas9s and structures orthologous to known subdomains, including the HNH endonuclease domain and the RuvC endonuclease domains [information from the Eugene Koonin and Kira Makarova]. Phylogenetic analysis based on the protein sequence conservation of this set revealed five families of Cas9s, including three groups of large Cas9s (~1400 amino acids) and two of small Cas9s (~1100 amino acids) (Figs.19A-D and 20A-F).

Applicants have also optimized Cas9 guide RNA using *in vitro* methods.

*Example 16: Cas9 mutations*

In this example, Applicants show that the following mutations can convert SpCas9 into a nicking enzyme: D10A, E762A, H840A, N854A, N863A, D986A.

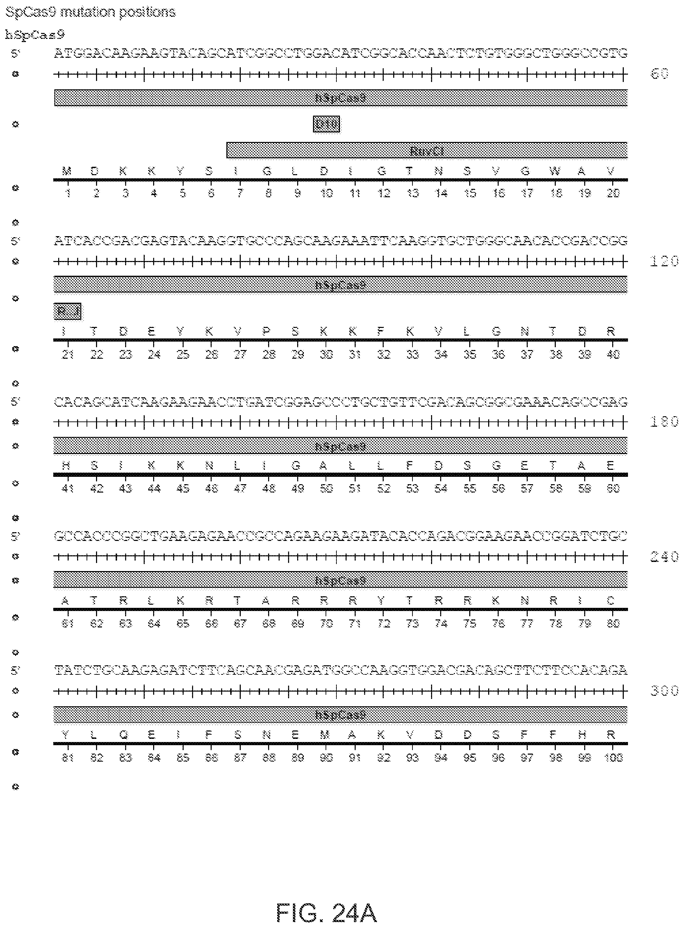

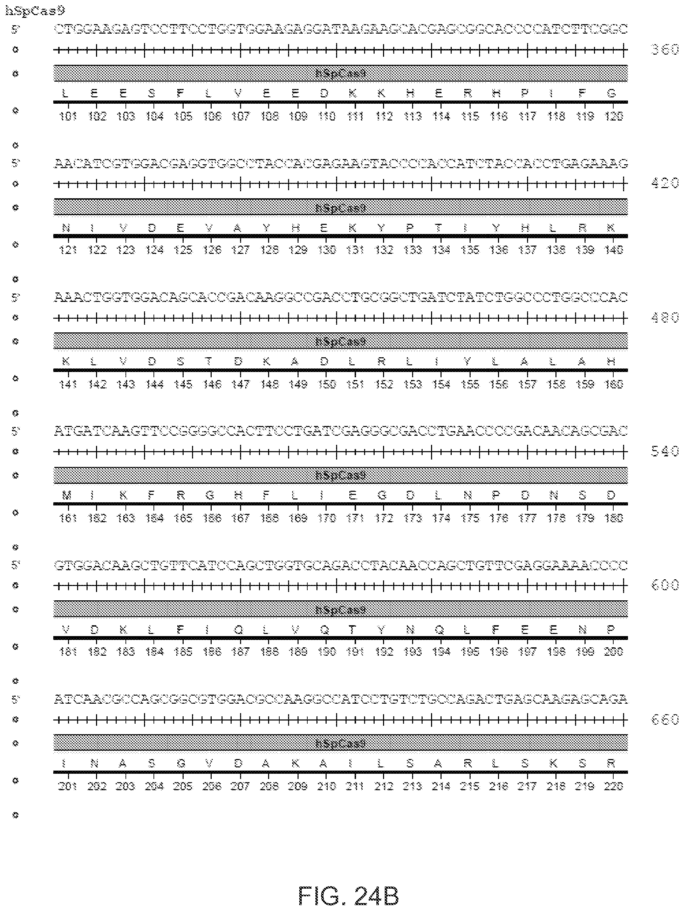

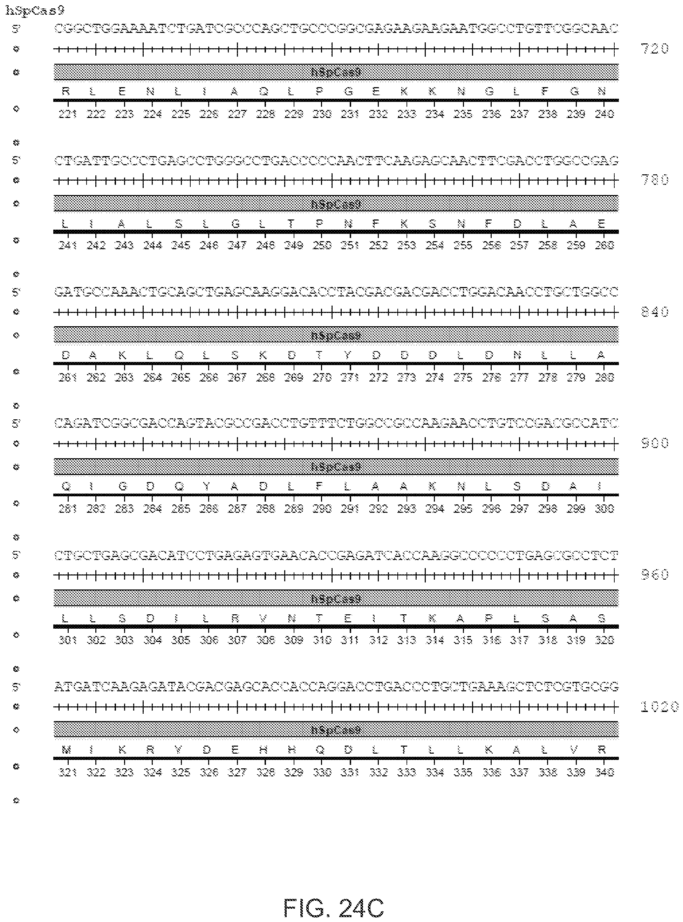

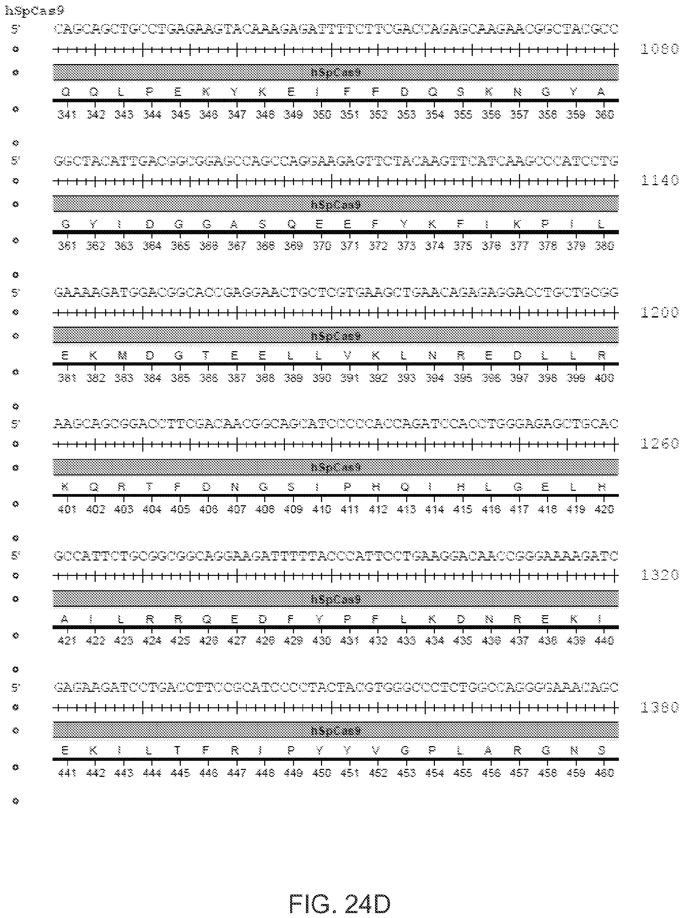

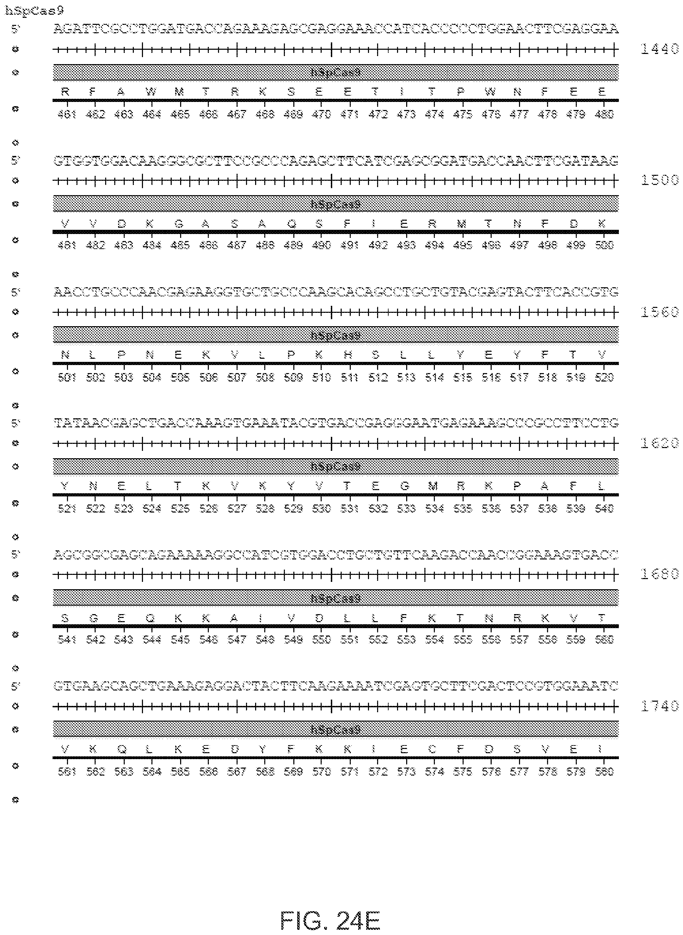

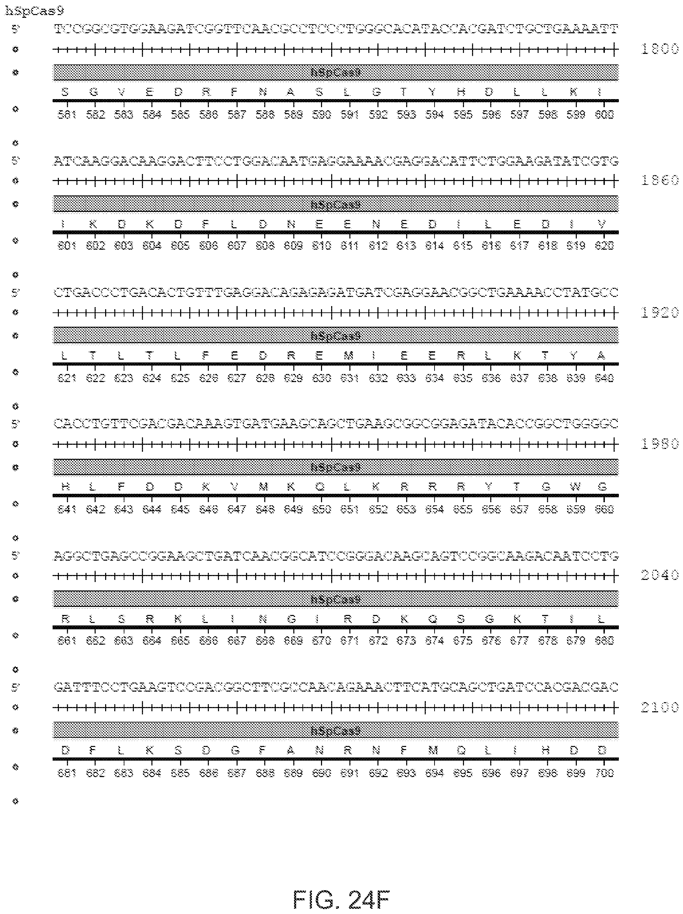

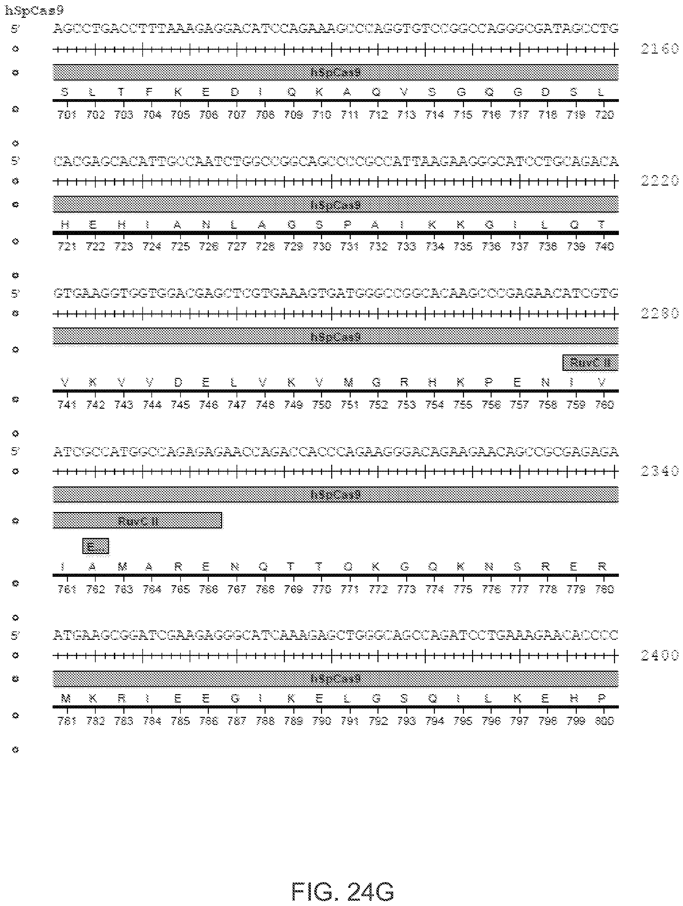

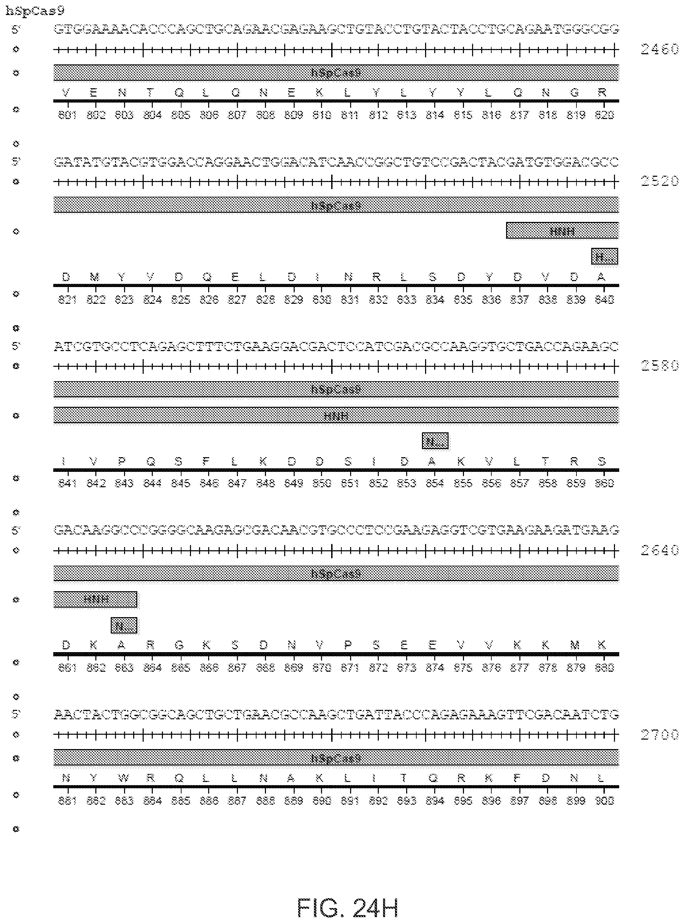

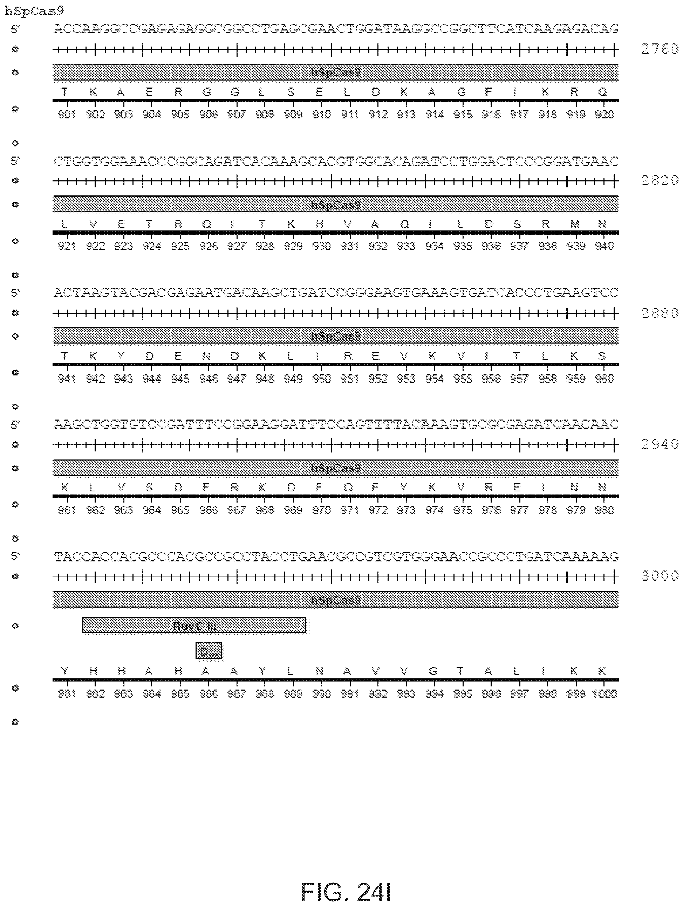

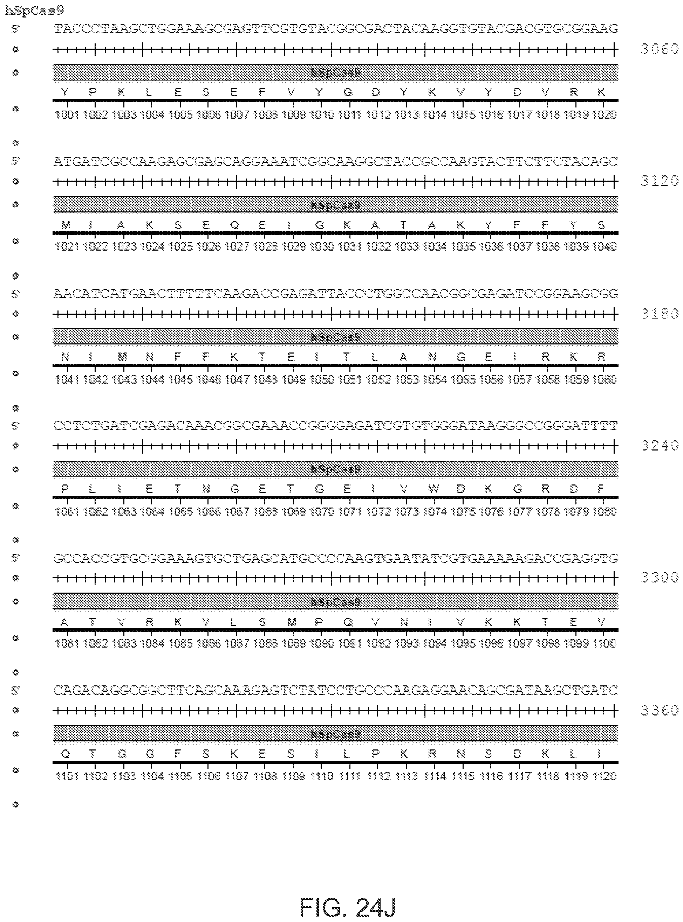

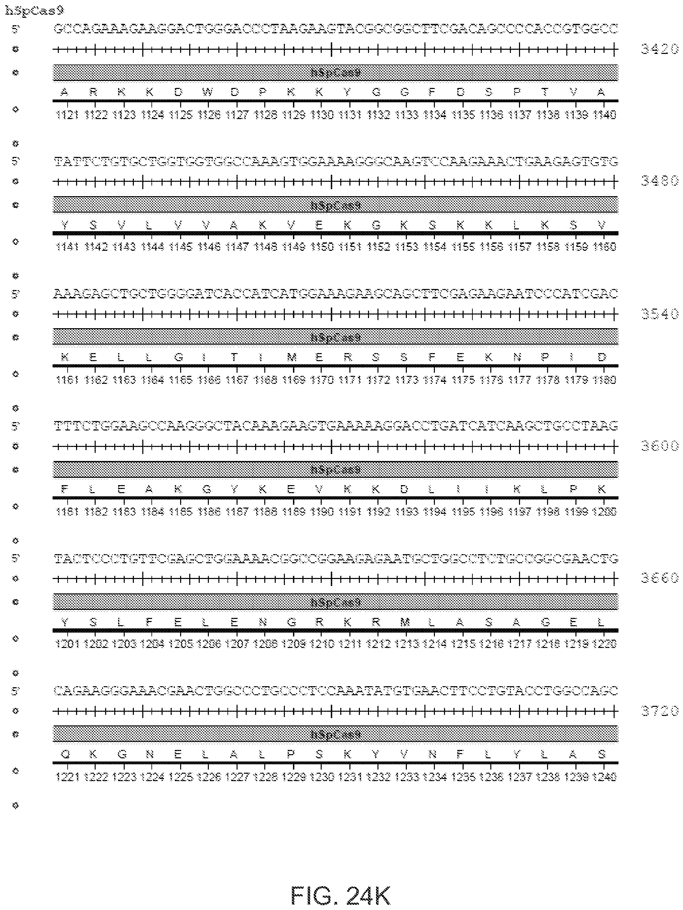

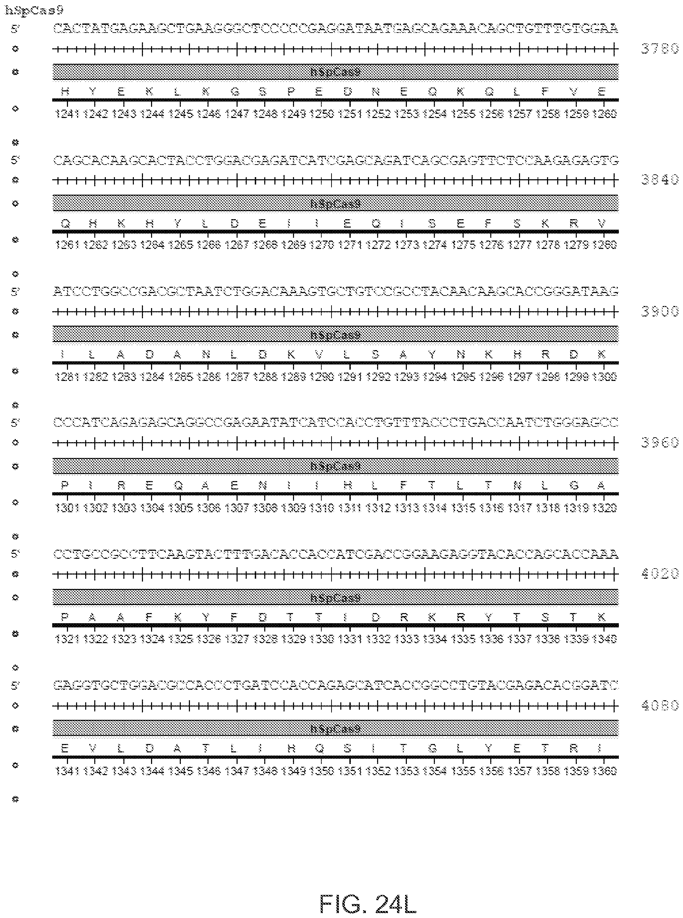

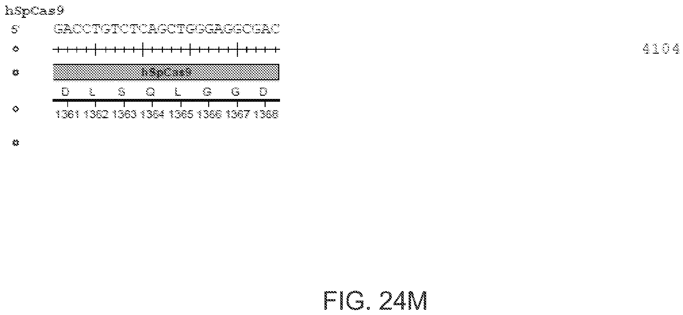

Applicants provide sequences showing where the mutation points are located within the SpCas9 gene (Fig. 24A-M). Applicants also show that the nickases are still able to mediate homologous recombination. Furthermore, Applicants show that SpCas9 with these mutations (individually) do not induce double strand break.

Cas9 orthologs all share the general organization of 3-4 RuvC domains and a HNH domain. The 5' most RuvC domain cleaves the non-complementary strand, and the HNH domain cleaves the complementary strand. All notations are in reference to the guide sequence.

The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue is mutated to alanine to convert Cas9 into a complementary-strand nicking enzyme. Similarly, the conserved His and Asn residues in the HNH domains are mutated to Alanine to convert Cas9 into a non-complementary-strand nicking enzyme.

*Example 17: Cas9 Transcriptional Activation and Cas9 Repressor*

Cas9 Transcriptional Activation

A second generation of constructs were designed and tested (Table 1). These constructs are used to assess transcriptional activation (VP64 fused constructs) and repression (Cas9 only) by RT-qPCR. Applicants assess the cellular localization of each construct using anti-His antibody, nuclease activity using a Surveyor nuclease assay, and DNA binding affinity using a gel shift assay.

Cas Repressor

It has been shown previously that dCas9 can be used as a generic DNA binding domain to repress gene expression. Applicants report an improved dCas9 design as well as dCas9 fusions to the repressor domains KRAB and SID4x. From the plasmid library created for modulating transcription using Cas9 in Table 1, the following repressor plasmids were functionally characterized by qPCR: pXRP27, pXRP28, pXRP29, pXRP48, pXRP49, pXRP50, pXRP51, pXRP52, pXRP53, pXRP56, pXRP58, pXRP59, pXRP61, and pXRP62.

Each dCas9 repressor plasmid was co-transfected with two guide RNAs targeted to the coding strand of the beta-catenin gene. RNA was isolated 72 hours after transfection and gene expression was quantified by RT-qPCR. The endogenous control gene was GAPDH. Two validated shRNAs were used as positive controls. Negative controls were certain plasmids transfected without gRNA, these are denoted as "pXRP## control". The plasmids pXRP28, pXRP29, pXRP48, and pXRP49 could repress the beta-catenin gene when using the specified targeting strategy. These plasmids correspond to dCas9 without a functional domain (pXRP28 and pXRP28) and dCas9 fused to SID4x (pXRP48 and pXRP49).

Further work investigates: repeating the above experiment, targeting different genes, utilizing other gRNAs to determine the optimal targeting position, and multiplexed repression.

Table 1

| |
|---|
| pXRP024-pLenti2-EF1a-VP64-NLS-FLAG-Linker-dCas9-NLS-gLuc-2A-GFP-WPRE |
| pXRP025-pLenti2-EF1a-VP64-NLS-GGGGS$_3$Linker-dCas9-NLS-gLuc-2A-GFP-WPRE |
| pXRP026-pLenti2-EF1a-VP64-NLS-EAAAK$_3$Linker-dCas9-NLS-gLuc-2A-GFP-WPRE |
| pXRP027-pLenti2-EF1a-NLS-FLAG-Linker-dCas9-NLS-gLuc-2A-GFP-WPRE |
| pXRP028-pLenti2-EF1a-NLS-GGGGS$_3$Linker-dCas9-NLS-gLuc-2A-GFP-WPRE |
| pXRP029-pLenti2-EF1a-NLS-EAAAK$_3$Linker-dCas9-NLS-gLuc-2A-GFP-WPRE |
| pXRP030-pLenti2-pSV40-VP64-NLS-FLAG-Linker-dCas9-NLS-gLuc-2A-GFP-WPRE |
| pXRP031-pLenti2-pPGK-VP64-NLS-FLAG-Linker-dCas9-NLS-gLuc-2A-GFP-WPRE |
| pXRP032-pLenti2-LTR-VP64-NLS-FLAG-Linker-dCas9-NLS-gLuc-2A-GFP-WPRE |
| pXRP033-pLenti2-pSV40-VP64-NLS-GGGGS$_3$Linker-dCas9-NLS-gLuc-2A-GFP-WPRE |
| pXRP034-pLenti2-pPGK-VP64-NLS-GGGGS$_3$Linker-dCas9-NLS-gLuc-2A-GFP-WPRE |
| pXRP035-pLenti2-LTR-VP64-NLS-GGGGS$_3$Linker-dCas9-NLS-gLuc-2A-GFP-WPRE |
| pXRP036-pLenti2-pSV40-VP64-NLS-EAAAK$_3$Linker-dCas9-NLS-gLuc-2A-GFP-WPRE |
| pXRP037-pLenti2-pPGK-VP64-NLS-EAAAK$_3$Linker-dCas9-NLS-gLuc-2A-GFP-WPRE |

(continued)

| |
|---|
| pXRP038-pLenti2-LTR-VP64-NLS-EAAAK$_3$Linker-dCas9-NLS-gLuc-2A-GFP-WPRE |
| pXRP048-pLenti2-EFla-SID4x-NLS-FLAG-Linker-dCas9-NLS-gLuc-2A-GFP-WPRE |
| pXRP049-pLenti2-EF1a-SID4X-NLS-GGGGS$_3$Linker-dCas9-NLS-gLuc-2A-GFP-WPRE |
| pXRP050-pLenti2-EF1a-SID4X-NLS-EAAAK$_3$Linker-dCas9-NLS-gLuc-2A-GFP-WPRE |
| pXRP051-pLenti2-EF1a-KRAB-NLS-FLAG-Linker-dCas9-NLS-gLuc-2 A-GFP-WPRE |
| pXRP052-pLenti2-EF1a-KRAB-NLS-GGGGS$_3$Linker-dCas9-NLS-gLuc-2A-GFP-WPRE |
| pXRP053-pLenti2-EF1a-KRAB-NLS-EAAAK$_3$Linker-dCas9-NLS-gLuc-2A-GFP-WPRE |
| pXRP054-pLenti2-EF1a-dCas9-Linker-FLAG-NLS-VP64-gLuc-2A-GFP-WPRE |
| pXRP055-pLenti2-EF1a-dCas9-Linker-FLAG-NLS-SID4X-gLuc-2A-GFP-WPRE |
| pXRP056-pLenti2-EF1a-dCas9-Linker-FLAG-NLS-KRAB-gLuc-2A-GFP-WPRE |
| pXRP057-pLenti2-EF1a-dCas9-GGGGGS$_3$-NLS-VP64-gLuc-2A-GFP-WPRE |
| pXRP058-pLenti2-EF1a-dCas9-GGGGGS$_3$-NLS-SID4X-gLuc-2A-GFP-WPRE |
| pXRP059-pLenti2-EF1a-dCas9-GGGGGS$_3$-NLS-KRAB-gLuc-2A-GFP-WPRE |
| pXRP060-pLenti2-EF1a-dCas9-EAAAK$_3$-NLS-VP64-gLuc-2A-GFP-WPRE |
| pXRP061-pLenti2-EF 1a-dCas9-EAAAK$_3$-NLS-SID4X-gLuc-2A-GFP-WPRE |
| pXRP062-pLenti2-EF1a-dCas9-EAAAK$_3$-NLS-KRAB-gLuc-2A-GFP-WPRE |
| pXRP024-pLenti2-EF1a-VP64-NLS-FLAG-Linker-Cas9-NLS-gLuc-2A-GFP-WPRE |
| pXRP025-pLenti2-EF1a-VP64-NLS-GGGGS$_3$Linker-Cas9-NLS-gLuc-2A-GFP-WPRE |
| pXRP026-pLenti2-EF1a-VP64-NLS-EAAAK$_3$Linker-Cas9-NLS-gLuc-2A-GFP-WPRE |
| pXRP027-pLenti2-EF1a-NLS-FLAG-Linker-Cas9-NLS-gLuc-2A-GFP-WPRE |
| pXRP028-pLenti2-EF1a-NLS-GGGGS$_3$Linker-Cas9-NLS-gLuc-2A-GFP-WPRE |
| pXRP029-pLenti2-EF1a-NLS-EAAAK$_3$Linker-Cas9-NLS-gLuc-2A-GFP-WPRE |
| pXRP030-pLenti2-pSV40-VP64-NLS-FLAG-Linker-Cas9-NLS-gLuc-2A-GFP-WPRE |
| pXRP031-pLenti2-pPGK-VP64-NLS-FLAG-Linker-Cas9-NLS-gLuc-2A-GFP-WPRE |
| pXRP032-pLenti2-LTR-VP64-NLS-FLAG-Linker-Cas9-NLS-gLuc-2A-GFP-WPRE |
| pXRP033-pLenti2-pSV40-VP64-NLS-GGGGS$_3$Linker-Cas9-NLS-gLuc-2A-GFP-WPRE |
| pXRP034-pLenti2-pPGK-VP64-NLS-GGGGS$_3$Linker-Cas9-NLS-gLuc-2A-GFP-WPRE |
| pXRP035-pLenti2-LTR-VP64-NLS-GGGGS$_3$Linker-Cas9-NLS-gLuc-2A-GFP-WPRE |
| pXRP036-pLenti2-pSV40-VP64-NLS-EAAAK$_3$Linker-Cas9-NLS-gLuc-2A-GFP-WPRE |
| pXRP037-pLenti2-pPGK-VP64-NLS-EAAAK$_3$Linker-Cas9-NLS-gLuc-2A-GFP-WPRE |
| pXRP038-pLenti2-LTR-VP64-NLS-EAAAK$_3$Linker-Cas9-NLS-gLuc-2A-GFP-WPRE |
| pXRP048-pLenti2-EF1a-SID4x-NLS-FLAG-Linker-Cas9-NLS-gLuc-2A-GFP-WPRE |
| pXRP049-pLenti2-EF1a-SID4X-NLS-GGGGS$_3$Linker-Cas9-NLS-gLuc-2A-GFP-WPRE |
| pXRP050-pLenti2-EF1a-SID4X-NLS-EAAAK$_3$Linker-Cas9-NLS-gLuc-2A-GFP-WPRE |
| pXRP051-pLenti2-EF1a-KRAB-NLS-FLAG-Linker-Cas9-NLS-gLuc-2 A-GFP-WPRE |
| pXRP052-pLenti2-EF1a-KRAB-NLS-GGGGS$_3$Linker-Cas9-NLS-gLuc-2A-GFP-WPRE |
| pXRP053-pLenti2-EF1a-KRAB-NLS-EAAAK$_3$Linker-Cas9-NLS-gLuc-2A-GFP-WPRE |
| pXRP054-pLenti2-EF1a-Cas9-Linker-FLAG-NLS-VP64-gLuc-2A-GFP-WPRE |
| pXRP055-pLenti2-EF1a-Cas9-Linker-FLAG-NLS-SID4X-gLuc-2A-GFP-WPRE |

(continued)

| pXRP056-pLenti2-EF1a-Cas9-Linker-FLAG-NLS-KRAB-gLuc-2A-GFP-WPRE |
| pXRP057-pLenti2-EF1a-Cas9-GGGGGS$_3$-NLS-VP64-gLuc-2A-GFP-WPRE |
| pXRP058-pLenti2-EF1a-Cas9-GGGGGS$_3$-NLS-SID4X-gLuc-2A-GFP-WPRE |
| pXRP059-pLenti2-EF1a-Cas9-GGGGGS$_3$-NLS-KRAB-gLuc-2A-GFP-WPRE |
| pXRP060-pLenti2-EF1a-Cas9-EAAAK$_3$-NLS-VP64-gLuc-2A-GFP-WPRE |
| pXRP061-pLenti2-EF 1a-Cas9-EAAAK$_3$-NLS-SID4X-gLuc-2A-GFP-WPRE |
| pXRP062-pLenti2-EF1a-Cas9-EAAAK$_3$-NLS-KRAB-gLuc-2A-GFP-WPRE |

*Example 18: Targeted deletion of genes involved in cholesterol biosynthesis, fatty acid biosynthesis, and other metabolic disorders, genes encoding mis-folded proteins involved in amyloid and other diseases, oncogenes leading to cellular transformation, latent viral genes, and genes leading to dominant-negative disorders, amongst other disorders.*

Applicants demonstrate gene delivery of a CRISPR-Cas system in the liver, brain, ocular, epithelial, hematopoetic, or another tissue of a subject or a patient in need thereof, suffering from metabolic disorders, amyloidosis and protein-aggregation related diseases, cellular transformation arising from genetic mutations and translocations, dominant negative effects of gene mutations, latent viral infections, and other related symptoms, using either viral or nanoparticle delivery system.

**Study Design:** Subjects or patients in need thereof suffering from metabolic disorders, amyloidosis and protein aggregation related disease which include but are not limited to human, non-primate human, canine, feline, bovine, equine, other domestic animals and related mammals. The CRISPR-Cas system is guided by a chimeric guide RNA and targets a specific site of the human genomic loci to be cleaved. After cleavage and non-homologous end-joining mediated repair, frame-shift mutation results in knock out of genes.

Applicants select guide-RNAs targeting genes involved in above-mentioned disorders to be specific to endogenous loci with minimal off-target activity. Two or more guide RNAs may be encoded into a single CRISPR array to induce simultaneous double-stranded breaks in DNA leading to micro-deletions of affected genes or chromosomal regions.

Identification and design of gene targets

For each candidate disease gene, Applicants select DNA sequences of interest include protein-coding exons, sequences including and flanking known dominant negative mutation sites, sequences including and flanking pathological repetitive sequences. For gene-knockout approaches, early coding exons closest to the start codon offer best options for achieving complete knockout and minimize possibility of truncated protein products retaining partial function.

Applicants analyze sequences of interest for all possible targetable 20-bp sequences immediately 5' to a NGG motif (for SpCas9 system) or a NNAGAAW (for St1Cas9 system). Applicants choose sequences for unique, single RNA-guided Cas9 recognition in the genome to minimize off-target effects based on computational algorithm to determine specificity.

Cloning of guide sequences into a delivery system

Guide sequences are synthesized as double-stranded 20-24 bp oligonucleotides. After 5'-phosphorylation treatment of oligos and annealing to form duplexes, oligos are ligated into suitable vector depending on the delivery method:

Virus-based delivery methods

AAV-based vectors (PX260, 330, 334, 335) have been described elsewhere
Lentiviral-based vectors use a similar cloning strategy of directly ligating guide sequences into a single vector carrying a U6 promoter-driven chimeric RNA scaffold and a EF1a promoter-driven Cas9 or Cas9 nickase.

Virus production is described elsewhere.

Nanoparticle-based RNA delivery methods

1. Guide sequences are synthesized as an oligonucleotide duplex encoding T7 promoter-guide sequence-chimeric RNA. A T7 promoter is added 5' of Cas9 by PCR method.

2. T7-driven Cas9 and guide-chimeric RNAs are transcribed *in vitro,* and Cas9 mRNA is further capped and A-tailed using commercial kits. RNA products are purified per kit instructions.

Hydrodynamic tail vein delivery methods (for mouse)

Guide sequences are cloned into AAV plasmids as described above and elsewhere in this application.

*In vitro* validation on cell lines

Transfection

1. DNA plasmid transfection

Plasmids carrying guide sequences are transfected into human embryonic kidney (HEK293T) or human embryonic stem (hES) cells, other relevant cell types using lipid-, chemical-, or electroporation-based methods. For a 24-well transfection of HEK293T cells (~260,000 cells), 500ng of total DNA is transfected into each single well using Lipofectamine 2000. For a 12-well transfection of hES cells, 1ug of total DNA is transfected into a single well using Fugene HD.

2. RNA transfection

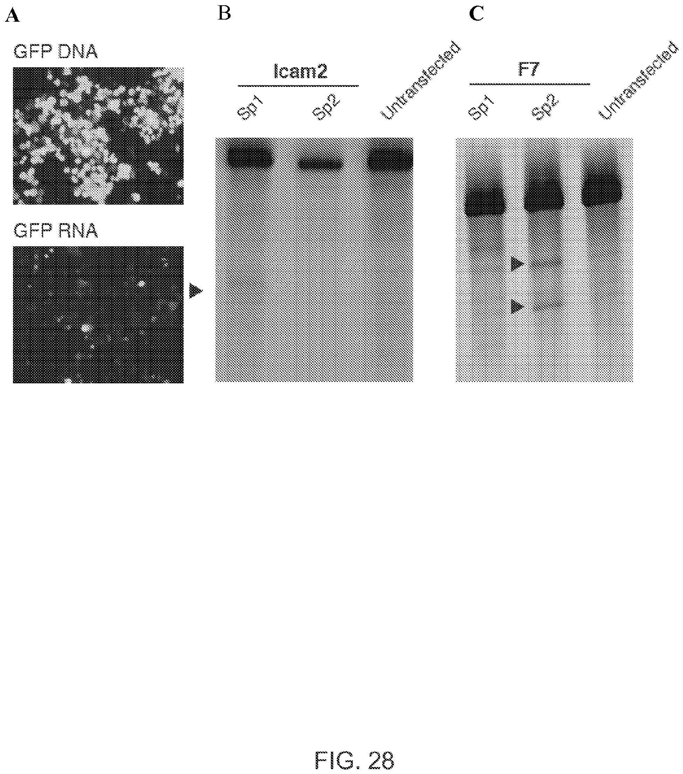

Purified RNA described above is used for transfection into HEK293T cells. 1-2ug of RNA may be transfected into ~260,000 using Lipofectamine 2000 per manufacturer's instruction. RNA delivery of Cas9 and chimeric RNA is shown in Fig. 28.

Assay of indel formation *in vitro*

Cells are harvested 72-hours post-transfection and assayed for indel formation as an indication of double-stranded breaks.

Briefly, genomic region around target sequence is PCR amplified (~400-600 bp amplicon size) using high-fidelity polymerase. Products are purified, normalized to equal concentration, and slowly annealed from 95°C to 4°C to allow formation of DNA heteroduplexes. Post annealing, the Cel-I enzyme is used to cleave heteroduplexes, and resulting products are separated on a polyacrylamide gel and indel efficiency calculated.

In vivo proof of principle in animal

Delivery mechanisms

AAV or Lentivirus production is described elsewhere.

Nanoparticle formulation: RNA mixed into nanoparticle formulation

Hydrodynamic tail vein injections with DNA plasmids in mice are conducted using a commercial kit

Cas9 and guide sequences are delivered as virus, nanoparticle-coated RNA mixture, or DNA plasmids, and injected into subject animals. A parallel set of control animals is injected with sterile saline, Cas9 and GFP, or guide sequence and GFP alone.

Three weeks after injection, animals are tested for amelioration of symptoms and sacrificed. Relevant organ systems analyzed for indel formation. Phenotypic assays include blood levels of HDL, LDL, lipids,

Assay for indel formation

DNA is extracted from tissue using commercial kits; indel assay will be performed as described for *in vitro* demonstration.

Therapeutic applications of the CRISPR-Cas system are amenable for achieving tissue-specific and temporally controlled targeted deletion of candidate disease genes. Examples include genes involved in cholesterol and fatty acid metabolism, amyloid diseases, dominant negative diseases, latent viral infections, among other disorders.

Examples of a single guide-RNA to introduce targeted indels at a gene locus

| Disease | GENE | SPACER | PAM | Mechanism | References |
|---|---|---|---|---|---|
| Hypercholesterolemia | HMGCR | GCCAAATTGGACGACCCTCG | CGG | Knockout | Fluvastatin: a review of its pharmacology and use in the management of hypercholesterolaemia.(Plosker GL et al. Drugs 1996, 51(3):433-459) |
| Hypercholesterolemia | SQLE | CGAGGAGACCCCCGTTTCGG | TGG | Knockout | Potential role of nonstatin cholesterol lowering agents (Trapani et al. IUBMB Life, Volume 63, Issue 11, pages 964-971, November 2011) |
| Hyperlipidemia | DGAT1 | CCCGCCGCCGCCGTGGCTCG | AGG | Knockout | DGAT1 inhibitors as anti-obesity and anti-diabetic agents. (Birch AM et al. Current Opinion in Drug Discovery & Development [2010, 13(4):489-496) |
| Leukemia | BCR-ABL | TGAGCTCTACGAGATCCACA | AGG | Knockout | Killing of leukemic cells with a BCR/ABL fusion gene by RNA interference (RNAi).( Fuchs et al. Oncogene 2002, 21(37):5716-5724) |

(SEQ ID NOS: _111_ to _114_, respectively)

Examples of a pair of guide-RNA to introduce chromosomal microdeletion at a gene locus

| Disease | GENE | SPACER | PAM | Mechanis m | References |
|---|---|---|---|---|---|
| Hyperlipidemia | PLIN2 guide1 | CTCAAAATTCATACCGGTTG | TGG | Microdeletion | Perilipin-2 Null Mice are Protected Against Diet-Induced Obesity, Adipose Inflammation and Fatty Liver Disease (McManaman JL et al. The Journal of Lipid Research, jlr.M035063. First Published on February 12, 2013) |
| Hyperlipidemia | PLIN2 guide2 | CGTTAAACAACAACCGGACT | TGG | Microdeletion | |
| Hyperlipidemia | SREBP guide1 | TTCACCCCGCGGCGCTGAAT | ggg | Microdeletion | Inhibition of SREBP by a Small Molecule, Betulin, Improves Hyperlipidemia and Insulin Resistance and Reduces Atherosclerotic Plaques (Tang J et al. Cell Metabolism, Volume 13, Issue 1, 44-56, 5 January 2011) |
| Hyperlipidemia | SREBP guide2 | ACCACTACCAGTCCGTCCAC | agg | Microdeletion | |

(SEQ ID NOS: _115_to_118_, respectively)

*Example 19: Targeted integration of repair for genes carrying disease-causing mutations; reconstitution of enzyme deficiencies and other related diseases.*

Study design

I. Identification and design of gene targets

• Described in Example 22

II. Cloning of guide sequences and repair templates into a delivery system

• Described above in Example 22
• Applicants clone DNA repair templates to include homology arms with diseased allele as well a wild-type repair template

III. *In vitro* validation on cell lines

a. Transfection is described above in Example 22; Cas9, guide RNAs, and repair template are co-transfected into relevant cell types.

b. Assay for repair *in vitro*

i. Applicants harvest cells 72-hours post-transfection and assay for repair

ii. Briefly, Applicants amplify genomic region around repair template PCR using high-fidelity polymerase. Applicants sequence products for decreased incidence of mutant allele.

IV. *In vivo* proof of principle in animal

a. Delivery mechanisms are described above Examples 22 and 34.

b. Assay for repair *in vivo*

i. Applicants perform the repair assay as described in the *in vitro* demonstration.

V. Therapeutic applications

The CRISPR-Cas system is amenable for achieving tissue-specific and temporally controlled targeted deletion of candidate disease genes. Examples include genes involved in cholesterol and fatty acid metabolism, amyloid diseases, dominant negative diseases, latent viral infections, among other disorders.

Example of one single missense mutation with repair template:

| Disease | GENE | SPACER | PAM |
|---|---|---|---|
| Familial amyloid polyneuropathy | TTR | AGCCTTTCTGAACACATGCA | CGG |

| Mechanism | References |
|---|---|
| | Transthyretin mutations in health and disease (Joao et al. Human Mutation, Volume 5, Issue 3, pages 191-196, 1995) |
| V30M repair | |
| V30M allele | CCTGCCATCAATGTGGCCATGCATGTGTTCAGAAAGGCT (SEQ ID NO: 120) |
| WT allele | CCTGCCATCAATGTGGCCGTGCATGTGTTCAGAAAGGCT (SEQ ID NO: 121) |

*Example 20: Therapeutic application of the CRISPR-Cas system in Glaucoma, Amyloidosis, and Huntington's disease*

Glaucoma: Applicants design guide RNAs to target the first exon of the mycilin (MYOC) gene. Applicants use adenovirus vectors (Ad5) to package both Cas9 as well as a guide RNA targeting the MYOC gene. Applicants inject adenoviral vectors into the trabecular meshwork where cells have been implicated in the pathophysiology of glaucoma. Applicants initially test this out in mouse models carrying the mutated MYOC gene to see whether they improve visual acuity and decrease pressure in the eyes. Therapeutic application in humans employ a similar strategy.

Amyloidosis: Applicants design guide RNAs to target the first exon of the transthyretin (TTR) gene in the liver. Applicants use AAV8 to package Cas9 as well as guide RNA targeting the first exon of the TTR gene. AAV8 has been shown to have efficient targeting of the liver and will be administered intravenously. Cas9 can be driven either using liver specific promoters such as the albumin promoter, or using a constitutive promoter. A pol3 promoter drives the guide RNA.

Alternatively, Applicants utilize hydrodynamic delivery of plasmid DNA to knockout the TTR gene. Applicants deliver a plasmid encoding Cas9 and the guideRNA targeting Exon1 of TTR.

As a further alternative approach, Applicants administer a combination of RNA (mRNA for Cas9, and guide RNA). RNA can be packaged using liposomes such as Invivofectamine from Life Technologies and delivered intravenously. To reduce RNA-induced immunogenicity, increase the level of Cas9 expression and guide RNA stability, Applicants modify the Cas9 mRNA using 5' capping. Applicants also incorporate modified RNA nucleotides into Cas9 mRNA and guide RNA to increase their stability and reduce immunogenicity (e.g. activation of TLR). To increase efficiency, Applicants administer multiple doses of the virus, DNA, or RNA.

Huntington's Disease: Applicants design guide RNA based on allele specific mutations in the HTT gene of patients. For example, in a patient who is heterozygous for HTT with expanded CAG repeat, Applicants identify nucleotide sequences unique to the mutant HTT allele and use it to design guideRNA. Applicants ensure that the mutant base is located within the last 9 bp of the guide RNA (which Applicants have ascertained has the ability to discriminate between single DNA base mismatches between the target size and the guide RNA).

Applicants package the mutant HTT allele specific guide RNA and Cas9 into AAV9 and deliver into the striatum of Huntington's patients. Virus is injected into the striatum stereotactically via a craniotomy. AAV9 is known to transduce neurons efficiently. Applicants drive Cas9 using a neuron specific promoter such as human Synapsin I.

*Example 21: Therapeutic application of the CRISPR-Cas system in HIV*

Chronic viral infection is a source of significant morbidity and mortality. While there exists for many of these viruses conventional antiviral therapies that effectively target various aspects of viral replication, current therapeutic modalities are usually non-curative in nature due to "viral latency." By its nature, viral latency is characterized by a dormant phase in the viral life cycle without active viral production. During this period, the virus is largely able to evade both immune surveillance and conventional therapeutics allowing for it to establish long-standing viral reservoirs within the host from which subsequent re-activation can permit continued propagation and transmission of virus. Key to viral latency is the ability to stably maintain the viral genome, accomplished either through episomal or proviral latency, which stores the viral genome in the cytoplasm or integrates it into the host genome, respectively. In the absence of effective vaccinations which would prevent primary infection, chronic viral infections characterized by latent reservoirs and episodes of lytic activity can have significant consequences: human papilloma virus (HPV) can result in cervical cancer, hepatitis C virus (HCV) predisposes to hepatocellular carcinoma, and human immunodeficiency virus eventually destroys the host immune system resulting in susceptibility to opportunistic infections. As such, these infections require life-long use of currently available antiviral therapeutics. Further complicating matters is the high mutability of many of these viral genomes which lead to the evolution of resistant strains for which there exists no effective therapy.

The CRISPR-Cas system is a bacterial adaptive immune system able to induce double-stranded DNA breaks (DSB) in a multiplex-able, sequence-specific manner and has been recently re-constituted within mammalian cell systems. It has been shown that targeting DNA with one or numerous guide-RNAs can result in both indels and deletions of the intervening sequences, respectively. As such, this new technology represents a means by which targeted and multiplexed DNA mutagenesis can be accomplished within a single cell with high efficiency and specificity. Consequently, delivery of the CRISPR-Cas system directed against viral DNA sequences could allow for targeted disruption and deletion of latent viral genomes even in the absence of ongoing viral production.

As an example, chronic infection by HIV-1 represents a global health issue with 33 million individuals infected and an annual incidence of 2.6 million infections. The use of the multimodal highly active antiretroviral therapy (HAART), which simultaneously targets multiple aspects of viral replication, has allowed HIV infection to be largely managed as a chronic, not terminal, illness. Without treatment, progression of HIV to AIDS occurs usually within 9-10 years resulting in depletion of the host immune system and occurrence of opportunistic infections usually leading to death soon thereafter. Secondary to viral latency, discontinuation of HAART invariably leads to viral rebound. Moreover, even temporary disruptions in therapy can select for resistant strains of HIV uncontrollable by available means. Additionally, the costs of HAART therapy are significant: within the US $10,000-15,0000 per person per year. As such, treatment approaches directly targeting the HIV genome rather than the process of viral replication represents a means by which eradication of latent reservoirs could allow for a curative therapeutic option.

Development and delivery of an HIV-1 targeted CRISPR-Cas system represents a unique approach differentiable from existing means of targeted DNA mutagenesis, i.e. ZFN and TALENs, with numerous therapeutic implications. Targeted disruption and deletion of the HIV-1 genome by CRISPR-mediated DSB and indels in conjunction with HAART could allow for simultaneous prevention of active viral production as well as depletion of latent viral reservoirs within the host.

Once integrated within the host immune system, the CRISPR-Cas system allows for generation of a HIV-1 resistant sub-population that, even in the absence of complete viral eradication, could allow for maintenance and reconstitution of host immune activity. This could potentially prevent primary infection by disruption of the viral genome preventing viral production and integration, representing a means to "vaccination". Multiplexed nature of the CRISPR-Cas system allows targeting of multiple aspects of the genome simultaneously within individual cells.

As in HAART, viral escape by mutagenesis is minimized by requiring acquisition of multiple adaptive mutations concurrently. Multiple strains of HIV-1 can be targeted simultaneously which minimizes the chance of super-infection and prevents subsequent creation of new recombinants strains. Nucleotide, rather than protein, mediated sequence-specificity of the CRISPR-Cas system allows for rapid generation of therapeutics without need for significantly altering delivery mechanism.

In order to accomplish this, Applicants generate CRISPR-Cas guide RNAs that target the vast majority of the HIV-1 genome while taking into account HIV-1 strain variants for maximal coverage and effectiveness. Sequence analyses of genomic conservation between HIV-1 subtypes and variants should allow for targeting of flanking conserved regions of the genome with the aims of deleting intervening viral sequences or induction of frame-shift mutations which would disrupt viral gene functions.

Applicants accomplish delivery of the CRISPR-Cas system by conventional adenoviral or lentiviral-mediated infection of the host immune system. Depending on approach, host immune cells could be a) isolated, transduced with CRISPR-Cas, selected, and re-introduced in to the host or b) transduced in vivo by systemic delivery of the CRISPR-Cas system. The first approach allows for generation of a resistant immune population whereas the second is more likely to target latent viral reservoirs within the host.

| Examples of potential HIV-1 targeted spacers adapted from Mcintyre *et al,* which generated shRNAs against HIV-1 optimized for maximal coverage of HIV-1 variants. |
|---|
| CACTGCTTAAGCCTCGCTCGAGG (SEQ ID NO: 122)<br>TCACCAGCAATATTCGCTCGAGG (SEQ ID NO: 123)<br>CACCAGCAATATTCCGCTCGAGG (SEQ ID NO: 124)<br>TAGCAACAGACATACGCTCGAGG (SEQ ID NO: 125)<br>GGGCAGTAGTAATACGCTCGAGG (SEQ ID NO: 126)<br>CCAATTCCCATACATTATTGTAC (SEQ ID NO: 127) |

*Example 22: Targeted correction of deltaF508 or other mutations in cystic fibrosis*

An aspect of the disclosure provides for a pharmaceutical composition that may comprise an CRISPR-Cas gene therapy particle and a biocompatible pharmaceutical carrier. According to another aspect, a method of gene therapy for the treatment of a subject having a mutation in the CFTR gene comprises administering a therapeutically effective amount of a CRISPR-Cas gene therapy particle to the cells of a subject.

This Example demonstrates gene transfer or gene delivery of a CRISPR-Cas system in airways of subject or a patient in need thereof, suffering from cystic fibrosis or from cystic fibrosis related symptoms, using adeno-associated virus (AAV) particles.

Study Design: Subjects or patients in need there of: Human, non-primate human, canine, feline, bovine, equine and other domestic animals, related. This study tests efficacy of gene transfer of a CRISPR-Cas system by a AAV vector. Applicants determine transgene levels sufficient for gene expression and utilize a CRISPR-Cas system comprising a Cas9 enzyme to target deltaF508 or other CFTR-inducing mutations.

The treated subjects receive pharmaceutically effective amount of aerosolized AAV vector system per lung endobronchially delivered while spontaneously breathing. The control subjects receive equivalent amount of a pseudotyped AAV vector system with an internal control gene. The vector system may be delivered along with a pharmaceutically

**EP 3 011 032 B1**

acceptable or biocompatible pharmaceutical carrier. Three weeks or an appropriate time interval following vector administration, treated subjects are tested for amelioration of cystic fibrosis related symptoms.

Applicants use an adenovirus or an AAV particle.

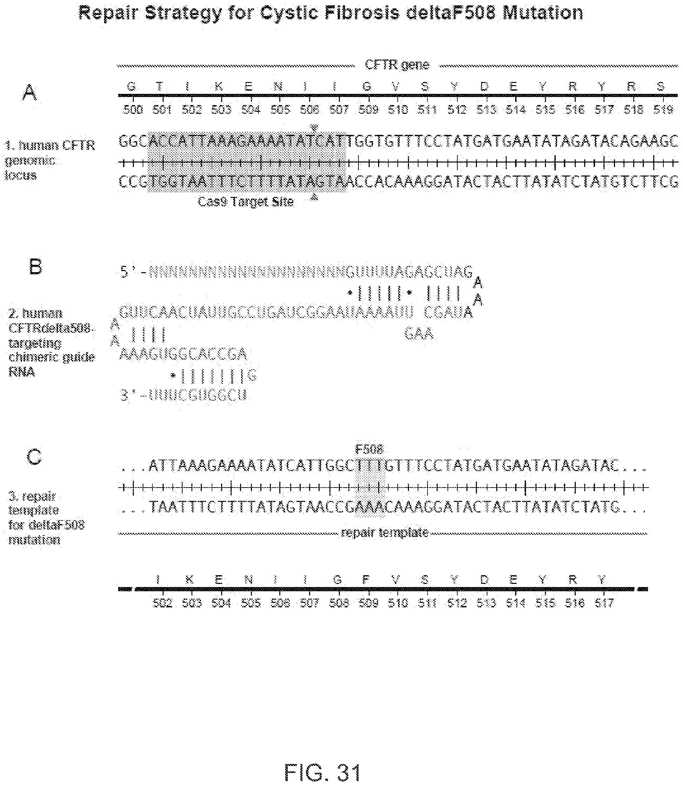

Applicants clone the following gene constructs, each operably linked to one or more regulatory sequences (Cbh or EF1a promoter for Cas9, U6 or HI promoter for chimeric guide RNA), into one or more adenovirus or AAV vectors or any other compatible vector: A CFTRdelta508 targeting chimeric guide RNA (Fig. 31B), a repair template for deltaF508 mutation (Fig. 31C) and a codon optimized Cas9 enzyme with optionally one or more nuclear localization signal or sequence(s) (NLS(s)), e.g., two (2) NLSs.

Identification of Cas9 target site

Applicants analyzed the human CFTR genomic locus and identified the Cas9 target site (Fig. 31A). (PAM may contain a NGG or a NNAGAAW motif).

Gene Repair Strategy

Applicants introduce an adenovirus/AAV vector system comprising a Cas9 (or Cas9 nickase) and the guide RNA along with a adenovirus/AAV vector system comprising the homology repair template containing the F508 residue into the subject via one of the methods of delivery discussed earlier. The CRISPR-Cas system is guided by the CFTRdelta 508 chimeric guide RNA and targets a specific site of the CFTR genomic locus to be nicked or cleaved. After cleavage, the repair template is inserted into the cleavage site via homologous recombination correcting the deletion that results in cystic fibrosis or causes cystic fibrosis related symptoms. This strategy to direct delivery and provide systemic introduction of CRISPR systems with appropriate guide RNAs can be employed to target genetic mutations to edit or otherwise manipulate genes that cause metabolic, liver, kidney and protein diseases and disorders such as those in Table B.

*Example 23: Generation of Gene Knockout Cell Library*

This example demonstrates how to generate a library of cells where each cell has a single gene knocked out: Applicants make a library of ES cells where each cell has a single gene knocked out, and the entire library of ES cells will have every single gene knocked out. This library is useful for the screening of gene function in cellular processes as well as diseases.

To make this cell library, Applicants integrate Cas9 driven by an inducible promoter (e.g. doxycycline inducible promoter) into the ES cell. In addition, Applicants integrate a single guide RNA targeting a specific gene in the ES cell. To make the ES cell library, Applicants simply mix ES cells with a library of genes encoding guide RNAs targeting each gene in the human genome. Applicants first introduce a single BxB1 attB site into the AAVS1 locus of the human ES cell. Then Applicants use the BxB1 integrase to facilitate the integration of individual guide RNA genes into the BxB1 attB site in AAVS1 locus. To facilitate integration, each guide RNA gene is contained on a plasmid that carries of a single attP site. This way BxB1 will recombine the attB site in the genome with the attP site on the guide RNA containing plasmid.

To generate the cell library, Applicants take the library of cells that have single guide RNAs integrated and induce Cas9 expression. After induction, Cas9 mediates double strand break at sites specified by the guide RNA. To verify the diversity of this cell library, Applicants carry out whole exome sequencing to ensure that Applicants are able to observe mutations in every single targeted gene. This cell library can be used for a variety of applications, including who library-based screens, or can be sorted into individual cell clones to facilitate rapid generation of clonal cell lines with individual human genes knocked out.

*Example 24: Engineering of Microalgae using Cas9*

Methods of delivering Cas9

Method 1: Applicants deliver Cas9 and guide RNA using a vector that expresses Cas9 under the control of a constitutive promoter such as Hsp70A-Rbc S2 or Beta2-tubulin.

Method 2: Applicants deliver Cas9 and T7 polymerase using vectors that expresses Cas9 and T7 polymerase under the control of a constitutive promoter such as Hsp70A-Rbc S2 or Beta2-tubulin. Guide RNA will be delivered using a vector containing T7 promoter driving the guide RNA.

Method 3: Applicants deliver Cas9 mRNA and *in vitro* transcribed guide RNA to algae cells. RNA can be *in vitro* transcribed. Cas9 mRNA will consist of the coding region for Cas9 as well as 3'UTR from Cop1 to ensure stabilization

**149**

of the Cas9 mRNA.

For Homologous recombination, Applicants provide an additional homology directed repair template.

Sequence for a cassette driving the expression of Cas9 under the control of beta-2 tubulin promoter, followed by the 3' UTR of Cop1.

TCTTTCTTGCGCTATGACACTTCCAGCAAAAGGTAGGGCGGGCTGCGA

GACGGCTTCCCGGCGCTGCATGCAACACCGATGATGCTTCGACCCCCCGAAGCTC

CTTCGGGGCTGCATGGGCGCTCCGATGCCGCTCCAGGGCGAGCGCTGTTTAAATA

GCCAGGCCCCCGATTGCAAAGACATTATAGCGAGCTACCAAAGCCATATTCAAAC

ACCTAGATCACTACCACTTCTACACAGGCCACTCGAGCTTGTGATCGCACTCCGC

TAAGGGGGCGCCTCTTCCTCTTCGTTTCAGTCACAACCCGCAAACATGTACCCATA

CGATGTTCCAGATTACGCTTCGCCGAAGAAAAAGCGCAAGGTCGAAGCGTCCGA

CAAGAAGTACAGCATCGGCCTGGACATCGGCACCAACTCTGTGGGCTGGGCCGTG

ATCACCGACGAGTACAAGGTGCCCAGCAAGAAATTCAAGGTGCTGGGCAACACC

GACCGGCACAGCATCAAGAAGAACCTGATCGGAGCCCTGCTGTTCGACAGCGGC

GAAACAGCCGAGGCCACCCGGCTGAAGAGAACCGCCAGAAGAAGATACACCAG

ACGGAAGAACCGGATCTGCTATCTGCAAGAGATCTTCAGCAACGAGATGGCCAA

GGTGGACGACAGCTTCTTCCACAGACTGGAAGAGTCCTTCCTGGTGGAAGAGGAT

AAGAAGCACGAGCGGCACCCCATCTTCGGCAACATCGTGGACGAGGTGGCCTAC

CACGAGAAGTACCCCACCATCTACCACCTGAGAAAGAAACTGGTGGACAGCACC

GACAAGGCCGACCTGCGGCTGATCTATCTGGCCCTGGCCCACATGATCAAGTTCC

GGGGCCACTTCCTGATCGAGGGCGACCTGAACCCCGACAACAGCGACGTGGACA

AGCTGTTCATCCAGCTGGTGCAGACCTACAACCAGCTGTTCGAGGAAAACCCCAT

CAACGCCAGCGGCGTGGACGCCAAGGCCATCCTGTCTGCCAGACTGAGCAAGAG

CAGACGGCTGGAAAATCTGATCGCCCAGCTGCCCGGCGAGAAGAAGAATGGCCT

GTTCGGCAACCTGATTGCCCTGAGCCTGGGCCTGACCCCCAACTTCAAGAGCAAC

TTCGACCTGGCCGAGGATGCCAAACTGCAGCTGAGCAAGGACACCTACGACGAC

GACCTGGACAACCTGCTGGCCCAGATCGGCGACCAGTACGCCGACCTGTTTCTGG
CCGCCAAGAACCTGTCCGACGCCATCCTGCTGAGCGACATCCTGAGAGTGAACAC
CGAGATCACCAAGGCCCCCCTGAGCGCCTCTATGATCAAGAGATACGACGAGCA
CCACCAGGACCTGACCCTGCTGAAAGCTCTCGTGCGGCAGCAGCTGCCTGAGAAG
TACAAAGAGATTTTCTTCGACCAGAGCAAGAACGGCTACGCCGGCTACATTGACG
GCGGAGCCAGCCAGGAAGAGTTCTACAAGTTCATCAAGCCCATCCTGGAAAAGA
TGGACGGCACCGAGGAACTGCTCGTGAAGCTGAACAGAGAGGACCTGCTGCGGA
AGCAGCGGACCTTCGACAACGGCAGCATCCCCACCAGATCCACCTGGGAGAGC
TGCACGCCATTCTGCGGCGGCAGGAAGATTTTTACCCATTCCTGAAGGACAACCG
GGAAAAGATCGAGAAGATCCTGACCTTCCGCATCCCCTACTACGTGGGCCCTCTG
GCCAGGGGAAACAGCAGATTCGCCTGGATGACCAGAAAGAGCGAGGAAACCATC
ACCCCCTGGAACTTCGAGGAAGTGGTGGACAAGGGCGCTTCCGCCCAGAGCTTCA
TCGAGCGGATGACCAACTTCGATAAGAACCTGCCCAACGAGAAGGTGCTGCCCA
AGCACAGCCTGCTGTACGAGTACTTCACCGTGTATAACGAGCTGACCAAAGTGAA
ATACGTGACCGAGGGAATGAGAAAGCCCGCCTTCCTGAGCGGCGAGCAGAAAAA
GGCCATCGTGGACCTGCTGTTCAAGACCAACCGGAAAGTGACCGTGAAGCAGCT
GAAAGAGGACTACTTCAAGAAAATCGAGTGCTTCGACTCCGTGGAAATCTCCGGC
GTGGAAGATCGGTTCAACGCCTCCCTGGGCACATACCACGATCTGCTGAAAATTA
TCAAGGACAAGGACTTCCTGGACAATGAGGAAAACGAGGACATTCTGGAAGATA
TCGTGCTGACCCTGACACTGTTTGAGGACAGAGAGATGATCGAGGAACGGCTGA
AAACCTATGCCCACCTGTTCGACGACAAAGTGATGAAGCAGCTGAAGCGGCGGA
GATACACCGGCTGGGGCAGGCTGAGCCGGAAGCTGATCAACGGCATCCGGGACA
AGCAGTCCGGCAAGACAATCCTGGATTTCCTGAAGTCCGACGGCTTCGCCAACAG
AAACTTCATGCAGCTGATCCACGACGACAGCCTGACCTTTAAAGAGGACATCCAG
AAAGCCCAGGTGTCCGGCCAGGGCGATAGCCTGCACGAGCACATTGCCAATCTG
GCCGGCAGCCCCGCCATTAAGAAGGGCATCCTGCAGACAGTGAAGGTGGTGGAC
GAGCTCGTGAAAGTGATGGGCCGGCACAAGCCCGAGAACATCGTGATCGAAATG
GCCAGAGAGAACCAGACCACCCAGAAGGGACAGAAGAACAGCCGCGAGAGAAT
GAAGCGGATCGAAGAGGGCATCAAAGAGCTGGGCAGCCAGATCCTGAAAGAACA
CCCCGTGGAAAACACCCAGCTGCAGAACGAGAAGCTGTACCTGTACTACCTGCAG
AATGGGCGGGATATGTACGTGGACCAGGAACTGGACATCAACCGGCTGTCCGAC

TACGATGTGGACCATATCGTGCCTCAGAGCTTTCTGAAGGACGACTCCATCGACA
ACAAGGTGCTGACCAGAAGCGACAAGAACCGGGGCAAGAGCGACAACGTGCCCT
CCGAAGAGGTCGTGAAGAAGATGAAGAACTACTGGCGGCAGCTGCTGAACGCCA
AGCTGATTACCCAGAGAAAGTTCGACAATCTGACCAAGGCCGAGAGAGGCGGCC
TGAGCGAACTGGATAAGGCCGGCTTCATCAAGAGACAGCTGGTGGAAACCCGGC
AGATCACAAAGCACGTGGCACAGATCCTGGACTCCCGGATGAACACTAAGTACG
ACGAGAATGACAAGCTGATCCGGGAAGTGAAAGTGATCACCCTGAAGTCCAAGC
TGGTGTCCGATTTCCGGAAGGATTTCCAGTTTTACAAAGTGCGCGAGATCAACAA
CTACCACCACGCCCACGACGCCTACCTGAACGCCGTCGTGGGAACCGCCCTGATC
AAAAAGTACCCTAAGCTGGAAAGCGAGTTCGTGTACGGCGACTACAAGGTGTAC
GACGTGCGGAAGATGATCGCCAAGAGCGAGCAGGAAATCGGCAAGGCTACCGCC
AAGTACTTCTTCTACAGCAACATCATGAACTTTTTCAAGACCGAGATTACCCTGG
CCAACGGCGAGATCCGGAAGCGGCCTCTGATCGAGACAAACGGCGAAACCGGGG
AGATCGTGTGGGATAAGGGCCGGGATTTTGCCACCGTGCGGAAAGTGCTGAGCA
TGCCCCAAGTGAATATCGTGAAAAAGACCGAGGTGCAGACAGGCGGCTTCAGCA
AAGAGTCTATCCTGCCCAAGAGGAACAGCGATAAGCTGATCGCCAGAAAGAAGG
ACTGGGACCCTAAGAAGTACGGCGGCTTCGACAGCCCCACCGTGGCCTATTCTGT
GCTGGTGGTGGCCAAAGTGGAAAAGGGCAAGTCCAAGAAACTGAAGAGTGTGAA
AGAGCTGCTGGGGATCACCATCATGGAAAGAAGCAGCTTCGAGAAGAATCCCAT
CGACTTTCTGGAAGCCAAGGGCTACAAAGAAGTGAAAAAGGACCTGATCATCAA
GCTGCCTAAGTACTCCCTGTTCGAGCTGGAAAACGGCCGGAAGAGAATGCTGGC
CTCTGCCGGCGAACTGCAGAAGGGAAACGAACTGGCCCTGCCCTCCAAATATGT
GAACTTCCTGTACCTGGCCAGCCACTATGAGAAGCTGAAGGGCTCCCCCGAGGAT
AATGAGCAGAAACAGCTGTTTGTGGAACAGCACAAGCACTACCTGGACGAGATC
ATCGAGCAGATCAGCGAGTTCTCCAAGAGAGTGATCCTGGCCGACGCTAATCTGG
ACAAAGTGCTGTCCGCCTACAACAAGCACCGGGATAAGCCCATCAGAGAGCAGG
CCGAGAATATCATCCACCTGTTTACCCTGACCAATCTGGGAGCCCCTGCCGCCTT
CAAGTACTTTGACACCACCATCGACCGGAAGAGGTACACCAGCACCAAAGAGGT
GCTGGACGCCACCCTGATCCACCAGAGCATCACCGGCCTGTACGAGACACGGAT
CGACCTGTCTCAGCTGGGAGGCGACAGCCCCAAGAAGAAGAGAAAGGTGGAGGC
CAGCTAAGGATCCGGCAAGACTGGCCCCGCTTGGCAACGCAACAGTGAGCCCCTC

CCTAGTGTGTTTGGGGATGTGACTATGTATTCGTGTGTTGGCCAACGGGTCAACC
CGAACAGATTGATACCCGCCTTGGCATTTCCTGTCAGAATGTAACGTCAGTTGAT
GGTACT  (SEQ ID NO: 128  )

Sequence for a cassette driving the expression of T7 polymerase under the control of beta-2 tubulin promoter, followed by the 3' UTR of Cop1:

TCTTTCTTGCGCTATGACACTTCCAGCAAAAGGTAGGGCGGGCTGCGA
GACGGCTTCCCGGCGCTGCATGCAACACCGATGATGCTTCGACCCCCCGAAGCTC
CTTCGGGGCTGCATGGGCGCTCCGATGCCGCTCCAGGGCGAGCGCTGTTTAAATA
GCCAGGCCCCCGATTGCAAAGACATTATAGCGAGCTACCAAAGCCATATTCAAAC
ACCTAGATCACTACCACTTCTACACAGGCCACTCGAGCTTGTGATCGCACTCCGC
TAAGGGGGCGCCTCTTCCTCTTCGTTTCAGTCACAACCCGCAAACatgcctaagaagaaga
ggaaggttaacacgattaacatcgctaagaacgacttctctgacatcgaactggctgctatcccgttcaacactctggctgaccatt
acggtgagcgtttagctcgcgaacagttggcccttgagcatgagtcttacgagatgggtgaagcacgcttccgcaagatgtttga
gcgtcaacttaaagctggtgaggttgcggataacgctgccgccaagcctctcatcactaccctactccctaagatgattgcacgc
atcaacgactggtttgaggaagtgaaagctaagcgcggcaagcgcccgacagccttccagttcctgcaagaaatcaagccgga
agccgtagcgtacatcaccattaagaccactctggcttgcctaaccagtgctgacaatacaaccgttcaggctgtagcaagcgca
atcggtcgggccattgaggacgaggctcgcttcggtcgtatccgtgaccttgaagctaagcacttcaagaaaaacgttgaggaa
caactcaacaagcgcgtagggcacgtctacaagaaagcatttatgcaagttgtcgaggctgacatgctctctaagggtctactcg
gtggcgaggcgtggtcttcgtggcataaggaagactctattcatgtaggagtacgctgcatcgagatgctcattgagtcaaccgg
aatggttagcttacaccgccaaaatgctggcgtagtaggtcaagactctgagactatcgaactcgcacctgaatacgctgaggct
atcgcaacccgtgcaggtgcgctggctggcatctctccgatgttccaaccttgcgtagttcctcctaagccgtggactggcattac
tggtggtggctattgggctaacggtcgtcgtcctctggcgctggtgcgtactcacagtaagaaagcactgatgcgctacgaagac
gtttacatgcctgaggtgtacaaagcgattaacattgcgcaaaacaccgcatggaaaatcaacaagaaagtcctagcggtcgcc
aacgtaatcaccaagtggaagcattgtccggtcgaggacatccctgcgattgagcgtgaagaactcccgatgaaaccggaaga
catcgacatgaatcctgaggctctcaccgcgtggaaacgtgctgccgctgctgtgtaccgcaaggacaaggctcgcaagtctcg
ccgtatcagccttgagttcatgcttgagcaagccaataagtttgctaaccataaggccatctggttcccttacaacatggactggcg
cggtcgtgtttacgctgtgtcaatgttcaacccgcaaggtaacgatatgaccaaaggactgcttacgctggcgaaaggtaaacca
atcggtaaggaaggttactactggctgaaaatccacggtgcaaactgtgcgggtgtcgacaaggttccgttccctgagcgcatca
agttcattgaggaaaaccacgagaacatcatggcttgcgctaagtctccactggagaacacttggtgggctgagcaagattctcc
gttctgcttccttgcgttctgctttgagtacgctggggtacagcaccacggcctgagctataactgctcccttccgctggcgtttgac
gggtcttgctctggcatccagcacttctccgcgatgctccgagatgaggtaggtggtcgcgcggttaacttgcttcctagtgaaac

cgttcaggacatctacgggattgttgctaagaaagtcaacgagattctacaagcagacgcaatcaatgggaccgataacgaagta

gttaccgtgaccgatgagaacactggtgaaatctctgagaaagtcaagctgggcactaaggcactggctggtcaatggctggct

tacggtgttactcgcagtgtgactaagcgttcagtcatgacgctggcttacgggtccaaagagttcggcttccgtcaacaagtgct

ggaagataccattcagccagctattgattccggcaagggtctgatgttcactcagccgaatcaggctgctggatacatggctaag

ctgatttgggaatctgtgagcgtgacggtggtagctgcggttgaagcaatgaactggcttaagtctgctgctaagctgctggctgc

tgaggtcaaagataagaagactggagagattcttcgcaagcgttgcgctgtgcattgggtaactcctgatggtttccctgtgtggc

aggaatacaagaagcctattcagacgcgcttgaacctgatgttcctcggtcagttccgcttacagcctaccattaacaccaacaa

agatagcgagattgatgcacacaaacaggagtctggtatcgctcctaactttgtacacagccaagacggtagccaccttcgtaag

actgtagtgtgggcacacgagaagtacggaatcgaatcttttgcactgattcacgactccttcggtacgattccggctgacgctgc

gaacctgttcaaagcagtgcgcgaaactatggttgacacatatgagtcttgtgatgtactggctgatttctacgaccagttcgctga

ccagttgcacgagtctcaattggacaaaatgccagcacttccggctaaaggtaacttgaacctccgtgacatcttagagtcggact

tcgcgttcgcgtaaGGATCCGGCAAGACTGGCCCCGCTTGGCAACGCAACAGTGAGCCC
CTCCCTAGTGTGTTTGGGGATGTGACTATGTATTCGTGTGTTGGCCAACGGGTCA
ACCCGAACAGATTGATACCCGCCTTGGCATTTCCTGTCAGAATGTAACGTCAGTT
GATGGTACT (SEQ ID NO: 129 )

Sequence of guide RNA driven by the T7 promoter (T7 promoter, Ns represent targeting sequence):

gaaat**TAATACGACTCACTATA**<u>NNNNNNNNNNNNNNNNNNNNNNNN</u>gttttagagc

ta**GAAA**tagcaagttaaaataaggctagtccgttatcaacttgaaaaagtggcaccgagtcggtgcttttttt (SEQ ID NO: 130 )

Gene delivery:

Chlamydomonas reinhardtii strain CC-124 and CC-125 from the Chlamydomonas Resource Center will be used for electroporation. Electroporation protocol follows standard recommended protocol from the GeneArt Chlamydomonas Engineering kit.

Also, Applicants generate a line of Chlamydomonas reinhardtii that expresses Cas9 constitutively. This can be done by using pChlamy1 (linearized using PvuI) and selecting for hygromycin resistant colonies. Sequence for pChlamy1 containing Cas9 is below. In this way to achieve gene knockout one simply needs to deliver RNA for the guideRNA. For homologous recombination Applicants deliver guideRNA as well as a linearized homologous recombination template.

pChlamy1-Cas9:

TGCGGTATTTCACACCGCATCAGGTGGCACTTTTCGGGGAAATGTGC
GCGGAACCCCTATTTGTTTATTTTTCTAAATACATTCAAATATGTATCCGCTCATGA
GATTATCAAAAAGGATCTTCACCTAGATCCTTTTAAATTAAAAATGAAGTTTTAA
ATCAATCTAAAGTATATATGAGTAAACTTGGTCTGACAGTTACCAATGCTTAATC
AGTGAGGCACCTATCTCAGCGATCTGTCTATTTCGTTCATCCATAGTTGCCTGACT
CCCCGTCGTGTAGATAACTACGATACGGGAGGGCTTACCATCTGGCCCCAGTGCT
GCAATGATACCGCGAGACCCACGCTCACCGGCTCCAGATTTATCAGCAATAAACC
AGCCAGCCGGAAGGGCCGAGCGCAGAAGTGGTCCTGCAACTTTATCCGCCTCCAT
CCAGTCTATTAATTGTTGCCGGGAAGCTAGAGTAAGTAGTTCGCCAGTTAATAGT
TTGCGCAACGTTGTTGCCATTGCTACAGGCATCGTGGTGTCACGCTCGTCGTTTGG
TATGGCTTCATTCAGCTCCGGTTCCCAACGATCAAGGCGAGTTACATGATCCCCC
ATGTTGTGCAAAAAAGCGGTTAGCTCCTTCGGTCCTCCGATCGTTGTCAGAAGTA
AGTTGGCCGCAGTGTTATCACTCATGGTTATGGCAGCACTGCATAATTCTCTTACT
GTCATGCCATCCGTAAGATGCTTTTCTGTGACTGGTGAGTACTCAACCAAGTCATT
CTGAGAATAGTGTATGCGGCGACCGAGTTGCTCTTGCCCGGCGTCAATACGGGAT
AATACCGCGCCACATAGCAGAACTTTAAAAGTGCTCATCATTGGAAAACGTTCTT
CGGGGCGAAAACTCTCAAGGATCTTACCGCTGTTGAGATCCAGTTCGATGTAACC
CACTCGTGCACCCAACTGATCTTCAGCATCTTTTACTTTCACCAGCGTTTCTGGGT
GAGCAAAAACAGGAAGGCAAAATGCCGCAAAAAAGGGAATAAGGGCGACACGG
AAATGTTGAATACTCATACTCTTCCTTTTTCAATATTATTGAAGCATTTATCAGGG
TTATTGTCTCATGACCAAAATCCCTTAACGTGAGTTTTCGTTCCACTGAGCGTCAG
ACCCCGTAGAAAAGATCAAAGGATCTTCTTGAGATCCTTTTTTTCTGCGCGTAATC
TGCTGCTTGCAAACAAAAAAACCACCGCTACCAGCGGTGGTTTGTTTGCCGGATC
AAGAGCTACCAACTCTTTTTCCGAAGGTAACTGGCTTCAGCAGAGCGCAGATACC
AAATACTGTTCTTCTAGTGTAGCCGTAGTTAGGCCACCACTTCAAGAACTCTGTA
GCACCGCCTACATACCTCGCTCTGCTAATCCTGTTACCAGTGGCTGTTGCCAGTGG
CGATAAGTCGTGTCTTACCGGGTTGGACTCAAGACGATAGTTACCGGATAAGGCG
CAGCGGTCGGGCTGAACGGGGGGTTCGTGCACACAGCCCAGCTTGGAGCGAACG
ACCTACACCGAACTGAGATACCTACAGCGTGAGCTATGAGAAAGCGCCACGCTTC
CCGAAGGGAGAAAGGCGGACAGGTATCCGGTAAGCGGCAGGGTCGGAACAGGA
GAGCGCACGAGGGAGCTTCCAGGGGGAAACGCCTGGTATCTTTATAGTCCTGTCG

GGTTTCGCCACCTCTGACTTGAGCGTCGATTTTTGTGATGCTCGTCAGGGGGGCGG
AGCCTATGGAAAAACGCCAGCAACGCGGCCTTTTTACGGTTCCTGGCCTTTTGCTG
GCCTTTTGCTCACATGTTCTTTCCTGCGTTATCCCCTGATTCTGTGGATAACCGTA
TTACCGCCTTTGAGTGAGCTGATACCGCTCGCCGCAGCCGAACGACCGAGCGCAG
CGAGTCAGTGAGCGAGGAAGCGGTCGCTGAGGCTTGACATGATTGGTGCGTATGT
TTGTATGAAGCTACAGGACTGATTTGGCGGGCTATGAGGGCGGGGGAAGCTCTGG
AAGGGCCGCGATGGGGCGCGCGGCGTCCAGAAGGCGCCATACGGCCCGCTGGCG
GCACCCATCCGGTATAAAAGCCCGCGACCCCGAACGGTGACCTCCACTTTCAGCG
ACAAACGAGCACTTATACATACGCGACTATTCTGCCGCTATACATAACCACTCAG
CTAGCTTAAGATCCCATCAAGCTTGCATGCCGGGCGCGCCAGAAGGAGCGCAGC
CAAACCAGGATGATGTTTGATGGGGTATTTGAGCACTTGCAACCCTTATCCGGAA
GCCCCCTGGCCCACAAAGGCTAGGCGCCAATGCAAGCAGTTCGCATGCAGCCCCT
GGAGCGGTGCCCTCCTGATAAACCGGCCAGGGGGCCTATGTTCTTTACTTTTTTAC
AAGAGAAGTCACTCAACATCTTAAAATGGCCAGGTGAGTCGACGAGCAAGCCCG
GCGGATCAGGCAGCGTGCTTGCAGATTTGACTTGCAACGCCCGCATTGTGTCGAC
GAAGGCTTTTGGCTCCTCTGTCGCTGTCTCAAGCAGCATCTAACCCTGCGTCGCCG
TTTCCATTTGCAGGAGATTCGAGGTACCATGTACCCATACGATGTTCCAGATTACG
CTTCGCCGAAGAAAAAGCGCAAGGTCGAAGCGTCCGACAAGAAGTACAGCATCG
GCCTGGACATCGGCACCAACTCTGTGGGCTGGGCCGTGATCACCGACGAGTACAA
GGTGCCCAGCAAGAAATTCAAGGTGCTGGGCAACACCGACCGGCACAGCATCAA
GAAGAACCTGATCGGAGCCCTGCTGTTCGACAGCGGCGAAACAGCCGAGGCCAC
CCGGCTGAAGAGAACCGCCAGAAGAAGATACACCAGACGGAAGAACCGGATCTG
CTATCTGCAAGAGATCTTCAGCAACGAGATGGCCAAGGTGGACGACAGCTTCTTC
CACAGACTGGAAGAGTCCTTCCTGGTGGAAGAGGATAAGAAGCACGAGCGGCAC
CCCATCTTCGGCAACATCGTGGACGAGGTGGCCTACCACGAGAAGTACCCCACCA
TCTACCACCTGAGAAAGAAACTGGTGGACAGCACCGACAAGGCCGACCTGCGGC
TGATCTATCTGGCCCTGGCCCACATGATCAAGTTCCGGGGCCACTTCCTGATCGA
GGGCGACCTGAACCCCGACAACAGCGACGTGGACAAGCTGTTCATCCAGCTGGT
GCAGACCTACAACCAGCTGTTCGAGGAAAACCCCATCAACGCCAGCGGCGTGGA
CGCCAAGGCCATCCTGTCTGCCAGACTGAGCAAGAGCAGACGGCTGGAAAATCT
GATCGCCCAGCTGCCCGGCGAGAAGAAGAATGGCCTGTTCGGCAACCTGATTGC

CCTGAGCCTGGGCCTGACCCCCAACTTCAAGAGCAACTTCGACCTGGCCGAGGAT
GCCAAACTGCAGCTGAGCAAGGACACCTACGACGACGACCTGGACAACCTGCTG
GCCCAGATCGGCGACCAGTACGCCGACCTGTTTCTGGCCGCCAAGAACCTGTCCG
ACGCCATCCTGCTGAGCGACATCCTGAGAGTGAACACCGAGATCACCAAGGCCCC
CCTGAGCGCCTCTATGATCAAGAGATACGACGAGCACCACCAGGACCTGACCCTG
CTGAAAGCTCTCGTGCGGCAGCAGCTGCCTGAGAAGTACAAAGAGATTTTCTTCG
ACCAGAGCAAGAACGGCTACGCCGGCTACATTGACGGCGGAGCCAGCCAGGAAG
AGTTCTACAAGTTCATCAAGCCCATCCTGGAAAAGATGGACGGCACCGAGGAAC
TGCTCGTGAAGCTGAACAGAGAGGACCTGCTGCGGAAGCAGCGGACCTTCGACA
ACGGCAGCATCCCCCACCAGATCCACCTGGGAGAGCTGCACGCCATTCTGCGGCG
GCAGGAAGATTTTTACCCATTCCTGAAGGACAACCGGGAAAAGATCGAGAAGAT
CCTGACCTTCCGCATCCCCTACTACGTGGGCCCTCTGGCCAGGGGAAACAGCAGA
TTCGCCTGGATGACCAGAAAGAGCGAGGAAACCATCACCCCCTGGAACTTCGAG
GAAGTGGTGGACAAGGGCGCTTCCGCCCAGAGCTTCATCGAGCGGATGACCAAC
TTCGATAAGAACCTGCCCAACGAGAAGGTGCTGCCCAAGCACAGCCTGCTGTAC
GAGTACTTCACCGTGTATAACGAGCTGACCAAAGTGAAATACGTGACCGAGGGA
ATGAGAAAGCCCGCCTTCCTGAGCGGCGAGCAGAAAAAGGCCATCGTGGACCT
GCTGTTCAAGACCAACCGGAAAGTGACCGTGAAGCAGCTGAAAGAGGACTACTT
CAAGAAAATCGAGTGCTTCGACTCCGTGGAAATCTCCGGCGTGGAAGATCGGTTC
AACGCCTCCCTGGGCACATACCACGATCTGCTGAAAATTATCAAGGACAAGGACT
TCCTGGACAATGAGGAAAACGAGGACATTCTGGAAGATATCGTGCTGACCCTGA
CACTGTTTGAGGACAGAGAGATGATCGAGGAACGGCTGAAAACCTATGCCCACC
TGTTCGACGACAAAGTGATGAAGCAGCTGAAGCGGCGGAGATACACCGGCTGGG
GCAGGCTGAGCCGGAAGCTGATCAACGGCATCCGGGACAAGCAGTCCGGCAAGA
CAATCCTGGATTTCCTGAAGTCCGACGGCTTCGCCAACAGAAACTTCATGCAGCT
GATCCACGACGACAGCCTGACCTTTAAAGAGGACATCCAGAAAGCCCAGGTGTC
CGGCCAGGGCGATAGCCTGCACGAGCACATTGCCAATCTGGCCGGCAGCCCCGC
CATTAAGAAGGGCATCCTGCAGACAGTGAAGGTGGTGGACGAGCTCGTGAAAGT
GATGGGCCGGCACAAGCCCGAGAACATCGTGATCGAAATGGCCAGAGAGAACCA
GACCACCCAGAAGGGACAGAAGAACAGCCGCGAGAGAATGAAGCGGATCGAAG
AGGGCATCAAAGAGCTGGGCAGCCAGATCCTGAAAGAACACCCCGTGGAAAACA

CCCAGCTGCAGAACGAGAAGCTGTACCTGTACTACCTGCAGAATGGGCGGGATA

TGTACGTGGACCAGGAACTGGACATCAACCGGCTGTCCGACTACGATGTGGACC

ATATCGTGCCTCAGAGCTTTCTGAAGGACGACTCCATCGACAACAAGGTGCTGAC

CAGAAGCGACAAGAACCGGGGCAAGAGCGACAACGTGCCCTCCGAAGAGGTCG

TGAAGAAGATGAAGAACTACTGGCGGCAGCTGCTGAACGCCAAGCTGATTACCC

AGAGAAAGTTCGACAATCTGACCAAGGCCGAGAGAGGCGGCCTGAGCGAACTGG

ATAAGGCCGGCTTCATCAAGAGACAGCTGGTGGAAACCCGGCAGATCACAAAGC

ACGTGGCACAGATCCTGGACTCCGGATGAACACTAAGTACGACGAGAATGACA

AGCTGATCCGGGAAGTGAAAGTGATCACCCTGAAGTCCAAGCTGGTGTCCGATTT

CCGGAAGGATTTCCAGTTTTACAAAGTGCGCGAGATCAACAACTACCACCACGCC

CACGACGCCTACCTGAACGCCGTCGTGGGAACCGCCCTGATCAAAAAGTACCCTA

AGCTGGAAAGCGAGTTCGTGTACGGCGACTACAAGGTGTACGACGTGCGGAAGA

TGATCGCCAAGAGCGAGCAGGAAATCGGCAAGGCTACCGCCAAGTACTTCTTCTA

CAGCAACATCATGAACTTTTTCAAGACCGAGATTACCCTGGCCAACGGCGAGATC

CGGAAGCGGCCTCTGATCGAGACAAACGGCGAAACCGGGGAGATCGTGTGGGAT

AAGGGCCGGGATTTTGCCACCGTGCGGAAAGTGCTGAGCATGCCCCAAGTGAAT

ATCGTGAAAAAGACCGAGGTGCAGACAGGCGGCTTCAGCAAAGAGTCTATCCTG

CCCAAGAGGAACAGCGATAAGCTGATCGCCAGAAAGAAGGACTGGGACCCTAAG

AAGTACGGCGGCTTCGACAGCCCCACCGTGGCCTATTCTGTGCTGGTGGTGGCCA

AAGTGGAAAAGGGCAAGTCCAAGAAACTGAAGAGTGTGAAAGAGCTGCTGGGG

ATCACCATCATGGAAAGAAGCAGCTTCGAGAAGAATCCCATCGACTTTCTGGAAG

CCAAGGGCTACAAAGAAGTGAAAAAGGACCTGATCATCAAGCTGCCTAAGTACT

CCCTGTTCGAGCTGGAAAACGGCCGGAAGAGAATGCTGGCCTCTGCCGGCGAAC

TGCAGAAGGGAAACGAACTGGCCCTGCCCTCCAAATATGTGAACTTCCTGTACC

TGGCCAGCCACTATGAGAAGCTGAAGGGCTCCCCCGAGGATAATGAGCAGAAAC

AGCTGTTTGTGGAACAGCACAAGCACTACCTGGACGAGATCATCGAGCAGATCA

GCGAGTTCTCCAAGAGAGTGATCCTGGCCGACGCTAATCTGGACAAAGTGCTGTC

CGCCTACAACAAGCACCGGGATAAGCCCATCAGAGAGCAGGCCGAGAATATCAT

CCACCTGTTTACCCTGACCAATCTGGGAGCCCCTGCCGCCTTCAAGTACTTTGACA

CCACCATCGACCGGAAGAGGTACACCAGCACCAAAGAGGTGCTGGACGCCACCC

TGATCCACCAGAGCATCACCGGCCTGTACGAGACACGGATCGACCTGTCTCAGCT

GGGAGGCGACAGCCCCAAGAAGAAGAGAAAGGTGGAGGCCAGCTAACATATGATTCG

AATGTCTTTCTTGCGCTATGACACTTCCAGCAAAAGGTAGGGCG

GGCTGCGAGACGGCTTCCCGGCGCTGCATGCAACACCGATGATGCTTCGACCCCC

CGAAGCTCCTTCGGGGCTGCATGGGCGCTCCGATGCCGCTCCAGGGCGAGCGCTG

TTTAAATAGCCAGGCCCCCGATTGCAAAGACATTATAGCGAGCTACCAAAGCCAT

ATTCAAACACCTAGATCACTACCACTTCTACACAGGCCACTCGAGCTTGTGATCG

CACTCCGCTAAGGGGGCGCCTCTTCCTCTTCGTTTCAGTCACAACCCGCAAACAT

GACACAAGAATCCCTGTTACTTCTCGACCGTATTGATTCGGATGATTCCTACGCG

AGCCTGCGGAACGACCAGGAATTCTGGGAGGTGAGTCGACGAGCAAGCCCGGCG

GATCAGGCAGCGTGCTTGCAGATTTGACTTGCAACGCCCGCATTGTGTCGACGAA

GGCTTTTGGCTCCTCTGTCGCTGTCTCAAGCAGCATCTAACCCTGCGTCGCCGTTT

CCATTTGCAGCCGCTGGCCCGCCGAGCCCTGGAGGAGCTCGGGCTGCCGGTGCCG

CCGGTGCTGCGGGTGCCCGGCGAGAGCACCAACCCCGTACTGGTCGGCGAGCCC

GGCCCGGTGATCAAGCTGTTCGGCGAGCACTGGTGCGGTCCGGAGAGCCTCGCG

TCGGAGTCGGAGGCGTACGCGGTCCTGGCGGACGCCCCGGTGCCGGTGCCCCGC

CTCCTCGGCCGCGGCGAGCTGCGGCCCGGCACCGGAGCCTGGCCGTGGCCCTACC

TGGTGATGAGCCGGATGACCGGCACCACCTGGCGGTCCGCGATGGACGGCACGA

CCGACCGGAACGCGCTGCTCGCCCTGGCCCGCGAACTCGGCCGGGTGCTCGGCCG

GCTGCACAGGGTGCCGCTGACCGGGAACACCGTGCTCACCCCCCATTCCGAGGTC

TTCCCGGAACTGCTGCGGGAACGCCGCGCGGCGACCGTCGAGGACCACCGCGGG

TGGGGCTACCTCTCGCCCCGGCTGCTGGACCGCCTGGAGGACTGGCTGCCGGACG

TGGACACGCTGCTGGCCGGCCGCGAACCCCGGTTCGTCCACGGCGACCTGCACGG

GACCAACATCTTCGTGGACCTGGCCGCGACCGAGGTCACCGGGATCGTCGACTTC

ACCGACGTCTATGCGGGAGACTCCCGCTACAGCCTGGTGCAACTGCATCTCAACG

CCTTCCGGGGCGACCGCGAGATCCTGGCCGCGCTGCTCGACGGGGCGCAGTGGA

AGCGGACCGAGGACTTCGCCCGCGAACTGCTCGCCTTCACCTTCCTGCACGACTT

CGAGGTGTTCGAGGAGACCCCGCTGGATCTCTCCGGCTTCACCGATCCGGAGGAA

CTGGCGCAGTTCCTCTGGGGGCCGCCGGACACCGCCCCCGGCGCCTGATAAGGAT

CCGGCAAGACTGGCCCCGCTTGGCAACGCAACAGTGAGCCCCTCCCTAGTGTGTT

TGGGGATGTGACTATGTATTCGTGTGTTGGCCAACGGGTCAACCCGAACAGATTG

ATACCCGCCTTGGCATTTCCTGTCAGAATGTAACGTCAGTTGATGGTACT   (SEQ ID NO: 131 )

For all modified Chlamydomonas reinhardtii cells, Applicants use PCR, SURVEYOR nuclease assay, and DNA sequencing to verify successful modification.

*Example 25: Use of Cas9 to target a variety of disease types*

Diseases that involve mutations in protein coding sequence:
Dominant disorders may be targeted by inactivating the dominant negative allele. Applicants use Cas9 to target a unique sequence in the dominant negative allele and introduce a mutation via NHEJ. The NHEJ-induced indel may be able to introduce a frame-shift mutation in the dominant negative allele and eliminate the dominant negative protein. This may work if the gene is haplo-sufficient (e.g. MYOC mutation induced glaucoma and Huntington's disease).

Recessive disorders may be targeted by repairing the disease mutation in both alleles. For dividing cells, Applicants use Cas9 to introduce double strand breaks near the mutation site and increase the rate of homologous recombination using an exogenous recombination template. For dividing cells, this may be achieved using multiplexed nickase activity to catalyze the replacement of the mutant sequence in both alleles via NHEJ-mediated ligation of an exogenous DNA fragment carrying complementary overhangs.

Applicants also use Cas9 to introduce protective mutations (e.g. inactivation of CCR5 to prevent HIV infection, inactivation of PCSK9 for cholesterol reduction, or introduction of the A673T into APP to reduce the likelihood of Alzheimer's disease).

**Diseases that involve non-coding sequences**

Applicants use Cas9 to disrupt non-coding sequences in the promoter region, to alter transcription factor binding sites and alter enhancer or repressor elements. For example, Cas9 may be used to excise out the Klf1 enhancer EHS1 in hematopoietic stem cells to reduce BCL11a levels and reactivate fetal globin gene expression in differentiated erythrocytes

Applicants also use Cas9 to disrupt functional motifs in the 5' or 3' untranslated regions. For example, for the treatment of myotonic dystrophy, Cas9 may be used to remove CTG repeat expansions in the DMPK gene.

*Example 26: Multiplexed Nickase*

Aspects of optimization and the teachings of Cas9 detailed in this application may also be used to generate Cas9 nickases. Applicants use Cas9 nickases in combination with pairs of guide RNAs to generate DNA double strand breaks with defined overhangs. When two pairs of guide RNAs are used, it is possible to excise an intervening DNA fragment. If an exogenous piece of DNA is cleaved by the two pairs of guide RNAs to generate compatible overhangs with the genomic DNA, then the exogenous DNA fragment may be ligated into the genomic DNA to replace the excised fragment. For example, this may be used to remove trinucleotide repeat expansion in the huntintin (HTT) gene to treat Huntington's Disease.

If an exogenous DNA that bears fewer number of CAG repeats is provided, then it may be able to generate a fragment of DNA that bears the same overhangs and can be ligated into the HTT genomic locus and replace the excised fragment.

HTT locus with fragment excised by Cas9 nickase and two pairs of guide RNAs

```
. . . CCGTGCCGGGCGGGAGACCGCCATGG          GGCCCGGCTGTGGCTGAGGAGC. . .
. . . GGCACGGCCCGCCCTCTGGC               TGGGCCGGGCCGACACCGACTCCTCG. . .
```

**+**

exogenous DNA fragment with fewer number of CAG repeats also cleaved by Cas9 nicakse and the two pairs of guide RNAs

```
            CGACCCTGGAAA.....reduced number of CAG repeats.....CCCCGCCGCCACCC
            GGTACCGCTGGGACCTTT.....                          .....GGGGCGGCGG
```

(SEQ ID NO: 132 to SEQ ID NO: 139 )

The ligation of the exogenous DNA fragment into the genome does not require homologous recombination machineries and therefore this method may be used in post-mitotic cells such as neurons.

*Example 27: Delivery of CRISPR System*

Cas9 and its chimeric guide RNA, or combination of tracrRNA and crRNA, can be delivered either as DNA or RNA. Delivery of Cas9 and guide RNA both as RNA (normal or containing base or backbone modifications) molecules can be used to reduce the amount of time that Cas9 protein persist in the cell. This may reduce the level of off-target cleavage activity in the target cell. Since delivery of Cas9 as mRNA takes time to be translated into protein, it might be advantageous to deliver the guide RNA several hours following the delivery of Cas9 mRNA, to maximize the level of guide RNA available for interaction with Cas9 protein.

In situations where guide RNA amount is limiting, it may be desirable to introduce Cas9 as mRNA and guide RNA in the form of a DNA expression cassette with a promoter driving the expression of the guide RNA. This way the amount of guide RNA available will be amplified via transcription.

A variety of delivery systems can be introduced to introduce Cas9 (DNA or RNA) and guide RNA (DNA or RNA) into the host cell. These include the use of liposomes, viral vectors, electroporation, nanoparticles , nanowires (Shalek et al., Nano Letters, 2012), exosomes. Molecular trojan horses liposomes (Pardridge et al., Cold Spring Harb Protoc; 2010; doi:10.1101/pdb.prot5407) may be used to deliver Cas9 and guide RNA across the blood brain barrier.

*Example 28: Therapeutic strategies for Trinucleotide repeat disorders*

As previously mentioned in the application, the target polynucleotide of a CRISPR complex may include a number of disease-associated genes and polynucleotides and some of these disease associated gene may belong to a set of genetic disorders referred to as Trinucleotide repeat disorders (referred to as also trinucleotide repeat expansion disorders, triplet repeat expansion disorders or codon reiteration disorders).

These diseases are caused by mutations in which the trinucleotide repeats of certain genes exceed the normal, stable threshold which may usually differ in a gene. The discovery of more repeat expansion disorders has allowed for the classification of these disorders into a number of categories based on underlying similar characteristics. Huntington's disease (HD) and the spinocerebellar ataxias that are caused by a CAG repeat expansion in protein-coding portions of specific genes are included in Category I. Diseases or disorders with expansions that tend to make them phenotypically diverse and include expansions are usually small in magnitude and also found in exons of genes are included in Category II. Category III includes disorders or diseases which are characterized by much larger repeat expansions than either Category I or II and are generally located outside protein coding regions. Examples of Category III diseases or disorders include but are not limited to Fragile X syndrome, myotonic dystrophy, two of the spinocerebellar ataxias, juvenile myoclonic epilepsy, and Friedreich's ataxia.

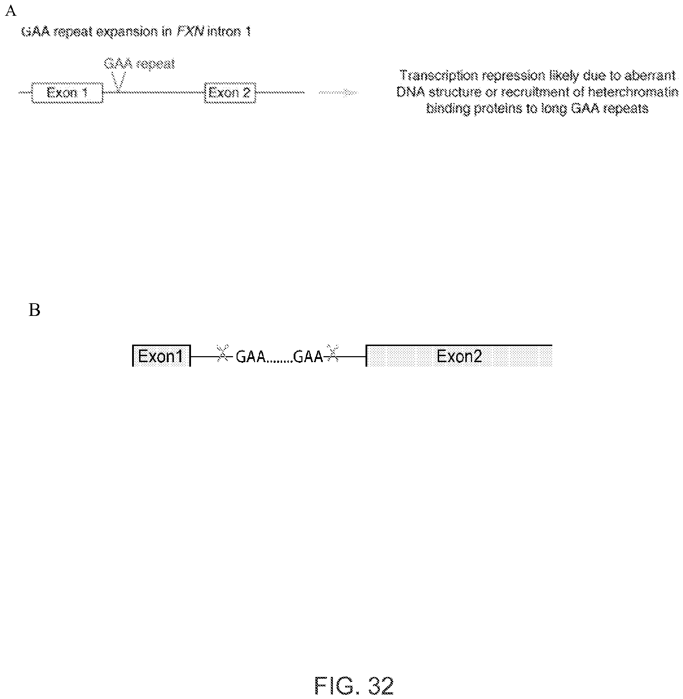

Similar therapeutic strategies, like the one mentioned for Friedreich's ataxia below may be adopted to address other trinucleotide repeat or expansion disorders as well. For example, another triple repeat disease that can be treated using almost identical strategy is dystrophia myotonica 1 (DM1), where there is an expanded CTG motif in the 3' UTR. In Friedreich's ataxia, the disease results from expansion of GAA trinucleotides in the first intron of frataxin (FXN). One therapeutic strategy using CRISPR is to excise the GAA repeat from the first intron. The expanded GAA repeat is thought to affect the DNA structure and leads to recruit the formation of heterochromatin which turn off the frataxin gene (Fig. 32A).

Competitive Advantage over other therapeutic strategies are listed below:
siRNA knockdown is not applicable in this case, as disease is due to reduced expression of frataxin. Viral gene therapy is currently being explored. HSV-1 based vectors were used to deliver the frataxin gene in animal models and have shown therapeutic effect. However, long term efficacy of virus-based frataxin delivery suffer from several problems: First, it is difficult to regulate the expression of frataxin to match natural levels in health individuals, and second, long term over expression of frataxin leads to cell death.

Nucleases may be used to excise the GAA repeat to restore healthy genotype, but Zinc Finger Nuclease and TALEN strategies require delivery of two pairs of high efficacy nucleases, which is difficult for both delivery as well as nuclease engineering (efficient excision of genomic DNA by ZFN or TALEN is difficult to achieve).

In contrast to above strategies, the CRISPR-Cas system has clear advantages. The Cas9 enzyme is more efficient and more multiplexible, by which it is meant that one or more targets can be set at the same time. So far, efficient excision of genomic DNA > 30% by Cas9 in human cells and may be as high as 30%, and may be improved in the future. Furthermore, with regard to certain trinucleotide repeat disorders like Huntington's disease (HD), trinucleotide repeats in the coding region may be addressed if there are differences between the two alleles. Specifically, if a HD patient is heterozygous for mutant HTT and there are nucleotide differences such as SNPs between the wt and mutant HTT alleles, then Cas9 may be used to specifically target the mutant HTT allele. ZFN or TALENs will not have the ability to distinguish two alleles based on single base differences.

In adopting a strategy using the CRISPR-Cas 9 enzyme to address Friedreich's ataxia, Applicants design a number of guide RNAs targeting sites flanking the GAA expansion and the most efficient and specific ones are chosen (Fig. 32B).

Applicants deliver a combination of guide RNAs targeting the intron 1 of FXN along with Cas9 to mediate excision of the GAA expansion region. AAV9 may be used to mediate efficient delivery of Cas9 and in the spinal cord.

If the Alu element adjacent to the GAA expansion is considered important, there may be constraints to the number of sites that can be targeted but Applicants may adopt strategies to avoid disrupting it.

Alternative Strategies:

Rather than modifying the genome using Cas9, Applicants may also directly activate the FXN gene using Cas9 (nuclease activity deficient)-based DNA binding domain to target a transcription activation domain to the FXN gene.

*Example 29: Strategies for minimizing off-target cleavage using Cas9 nickase*

As previously mentioned in the application, Cas9 may be mutated to mediate single strand cleavage via one or more of the following mutations: D10A, E762A, and H840A.

To mediate gene knockout via NHEJ, Applicants use a nickase version of Cas9 along with two guide RNAs. Off-target nicking by each individual guide RNA may be primarily repaired without mutation, double strand breaks (which can lead to mutations via NHEJ) only occur when the target sites are adjacent to each other. Since double strand breaks introduced by double nicking are not blunt, co-expression of end-processing enzymes such as TREX1 will increase the level of NHEJ activity.

The following list of targets in tabular form are for genes involved in the following diseases:

Lafora's Disease - target GSY1 or PPP1R3C (PTG) to reduce glycogen in neurons.

Hypercholesterolemia - target PCSK9

Target sequences are listed in pairs (L and R) with different number of nucleotides in the spacer (0 to 3bp). Each spacer may also be used by itself with the wild type Cas9 to introduce double strand break at the target locus.

| | | | (SEQ ID NO: ) |
|---|---|---|---|
| GYS1 (human) | GGCC-L | ACCCTTGTTAGCCACCTCCC | 140 |
| | GGCC-R | GAACGCAGTGCTCTTCGAAG | 141 |
| | GGNCC-L | CTCACGCCCTGCTCCGTGTA | 142 |
| | GGNCC-R | GGCGACAACTACTTCCTGGT | 143 |
| | GGNNCC-L | CTCACGCCCTGCTCCGTGTA | 144 |
| | GGNNCC-R | GGGCGACAACTACTTCCTGG | 145 |
| | GGNNNCC-L | CCTCTTCAGGGCCGGGGTGG | 146 |
| | GGNNNCC-R | GAGGACCCAGGTGGAACTGC | 147 |
| PCSK9 (human) | GGCC-L | TCAGCTCCAGGCGGTCCTGG | 148 |
| | GGCC-R | AGCAGCAGCAGCAGTGGCAG | 149 |
| | GGNCC-L | TGGGCACCGTCAGCTCCAGG | 150 |
| | GGNCC-R | CAGCAGTGGCAGCGGCCACC | 151 |
| | GGNNCC-L | ACCTCTCCCCTGGCCCTCAT | 152 |
| | GGNNCC-R | CCAGGACCGCCTGGAGCTGA | 153 |
| | GGNNNCC-L | CCGTCAGCTCCAGGCGGTCC | 154 |
| | GGNNNCC-R | AGCAGCAGCAGCAGTGGCAG | 155 |
| PPP1R3C (PTG) (human) | | | |
| | GGCC-L | ATGTGCCAAGCAAAGCCTCA | 156 |
| | GGCC-R | TTCGGTCATGCCCGTGGATG | 157 |
| | GGNCC-L | GTCGTTGAAATTCATCGTAC | 158 |
| | GGNCC-R | ACCACCTGTGAAGAGTTTCC | 159 |
| | GGNNCC-L | CGTCGTTGAAATTCATCGTA | 160 |
| | GGNNCC-R | ACCACCTGTGAAGAGTTTCC | 161 |
| Gys1 (mouse) | GGCC-L | GAACGCAGTGCTTTTCGAGG | 162 |
| | GGCC-R | ACCCTTGTTGGCCACCTCCC | 163 |
| | GGNCC-L | GGTGACAACTACTATCTGGT | 164 |
| | GGNCC-R | CTCACACCCTGCTCCGTGTA | 165 |
| | GGNNCC-L | GGGTGACAACTACTATCTGG | 166 |
| | GGNNCC-R | CTCACACCCTGCTCCGTGTA | 167 |

(continued)

| | | | (SEQ ID NO: ) |
|---|---|---|---|
| | GGNNNCC-L | CGAGAACGCAGTGCTTTTCG | 168 |
| | GGNNNCC-R | ACCCTTGTTGGCCACCTCCC | 169 |
| PPP1R3C (PTG) (mouse) | | | |
| | GGCC-L | ATGAGCCAAGCAAATCCTCA | 170 |
| | GGCC-R | TTCCGTCATGCCCGTGGACA | 171 |
| | GGNCC-L | CTTCGTTGAAAACCATTGTA | 172 |
| | GGNCC-R | CCACCTCTGAAGAGTTTCCT | 173 |
| | GGNNCC-L | CTTCGTTGAAAACCATTGTA | 174 |
| | GGNNCC-R | ACCACCTCTGAAGAGTTTCC | 175 |
| | GGNNNCC-L | CTTCCACTCACTCTGCGATT | 176 |
| | GGNNNCC-R | ACCATGTCTCAGTGTCAAGC | 177 |
| PCSK9 (mouse) | GGCC-L | GGCGGCAACAGCGGCAACAG | 178 |
| | GGCC-R | ACTGCTCTGCGTGGCTGCGG | 179 |
| | GGNNCC-L | CCGCAGCCACGCAGAGCAGT | 180 |
| | GGNNCC-R | GCACCTCTCCTCGCCCCGAT | 181 |

Alternative strategies for improving stability of guide RNA and increasing specificity

1. Nucleotides in the 5' of the guide RNA may be linked via thiolester linkages rather than phosphoester linkage like in natural RNA. Thiolester linkage may prevent the guide RNA from being digested by endogenous RNA degradation machinery.

2. Nucleotides in the guide sequence (5' 20bp) of the guide RNA can use bridged nucleic acids (BNA) as the bases to improve the binding specificity.

*Example 30: CRISPR-Cas for rapid, multiplex genome editing*

Aspects of the disclosure
relate to protocols and methods by which efficiency and
specificity of gene modification may be tested within 3-4 days after target design, and modified clonal cell lines may be derived within 2-3 weeks.
Programmable nucleases are powerful technologies for mediating genome alteration with high precision. The RNA-guided Cas9 nuclease from the microbial CRISPR adaptive immune system can be used to facilitate efficient genome editing in eukaryotic cells by simply specifying a 20-nt targeting sequence in its guide RNA. Applicants describe a set of protocols for applying Cas9 to facilitate efficient genome editing in mammalian cells and generate cell lines for downstream functional studies. Beginning with target design, efficient and specific gene modification can be achieved within 3-4 days, and modified clonal cell lines can be derived within 2-3 weeks.
The ability to engineer biological systems and organisms holds enormous potential for applications across basic science, medicine, and biotechnology. Programmable sequence-specific endonucleases that facilitate precise editing of endogenous genomic loci are now enabling systematic interrogation of genetic elements and causal genetic variations in a broad range of species, including those that have not been genetically tractable previously. A number of genome editing technologies have emerged in recent years, including zinc finger nucleases (ZFNs), transcription activator-like effector nucleases (TALENs), and the RNA-guided CRISPR-Cas nuclease system. The first two technologies use a common strategy of tethering endonuclease catalytic domains to modular DNA-binding proteins for inducing targeted DNA double stranded breaks (DSB) at specific genomic loci. By contrast, Cas9 is a nuclease guided by small RNAs through Watson-Crick base-pairing with target DNA, presenting a system that is easy to design, efficient, and well-suited for high-throughput and multiplexed gene editing for a variety of cell types and organisms. Here Applicants describe a set of protocols for applying the recently developed Cas9 nuclease to facilitate efficient genome editing in mammalian cells and generate cell lines for downstream functional studies.
Like ZFNs and TALENs, Cas9 promotes genome editing by stimulating DSB at the target genomic loci. Upon cleavage by Cas9, the target locus undergoes one of two major pathways for DNA damage repair, the error-prone non-homologous end joining (NHEJ) or the high-fidelity homology directed repair (HDR) pathway. Both pathways may be utilized to achieve the desired editing outcome.

NHEJ: In the absence of a repair template, the NHEJ process re-ligates DSBs, which may leave a scar in the form of indel mutations. This process can be harnessed to achieve gene knockouts, as indels occurring within a coding exon may lead to frameshift mutations and a premature stop codon. Multiple DSBs may also be exploited to mediate larger deletions in the genome.

HDR: Homology directed repair is an alternate major DNA repair pathway to NHEJ. Although HDR typically occurs at lower frequencies than NHEJ, it may be harnessed to generate precise, defined modifications at a target locus in the presence of an exogenously introduced repair template. The repair template may be either in the form of double stranded DNA, designed similarly to conventional DNA targeting constructs with homology arms flanking the insertion sequence, or single-stranded DNA oligonucleotides (ssODNs). The latter provides an effective and simple method for making small edits in the genome, such as the introduction of single nucleotide mutations for probing causal genetic variations. Unlike NHEJ, HDR is generally active only in dividing cells and its efficiency varies depending on the cell type and state.

Overview of CRISPR: The CRISPR-Cas system, by contrast, is at minimum a two-component system consisting of the Cas9 nuclease and a short guide RNA. Re-targeting of Cas9 to different loci or simultaneous editing of multiple genes simply requires cloning a different 20-bp oligonucleotide. Although specificity of the Cas9 nuclease has yet to be thoroughly elucidated, the simple Watson-Crick base-pairing of the CRISPR-Cas system is likely more predictable than that of ZFN or TALEN domains.

The type II CRISPR-Cas (clustered regularly interspaced short palindromic repeats) is a bacterial adaptive immune system that uses Cas9, to cleave foreign genetic elements. Cas9 is guided by a pair of non-coding RNAs, a variable crRNA and a required auxiliary tracrRNA. The crRNA contains a 20-nt guide sequence determines specificity by locating the target DNA via Watson-Crick base-pairing. In the native bacterial system, multiple crRNAs are co-transcribed to direct Cas9 against various targets. In the CRISPR-Cas system derived from *Streptococcus pyogenes,* the target DNA must immediately precede a 5'-NGG/NRG protospacer adjacent motif (PAM), which can vary for other CRISPR systems.

CRISPR-Cas is reconstituted in mammalian cells through the heterologous expression of human codon-optimized Cas9 and the requisite RNA components. Furthermore, the crRNA and tracrRNA can be fused to create a chimeric, synthetic guide RNA (sgRNA). Cas9 can thus be redirected toward any target of interest by altering the 20-nt guide sequence within the sgRNA.

Given its ease of implementation and multiplex capability, Cas9 has been used to generate engineered eukaryotic cells carrying specific mutations via both NHEJ and HDR. In addition, direct injection of sgRNA and mRNA encoding Cas9 into embryos has enabled the rapid generation of transgenic mice with multiple modified alleles; these results hold promise for editing organisms that are otherwise genetically intractable.

A mutant Cas9 carrying a disruption in one of its catalytic domains has been engineered to nick rather than cleave DNA, allowing for single-stranded breaks and preferential repair through HDR, potentially ameliorating unwanted indel mutations from off-target DSBs. Additionally, a Cas9 mutant with both DNA-cleaving catalytic residues mutated has been adapted to enable transcriptional regulation in *E. coli,* demonstrating the potential of functionalizing Cas9 for diverse applications. Certain aspects relate to the construction and application of Cas9 for multiplexed editing of human cells.

Applicants have provided a human codon-optimized, nuclear localization sequence-flanked Cas9 to facilitate eukaryotic gene editing. Applicants describe considerations for designing the 20-nt guide sequence, protocols for rapid construction and functional validation of sgRNAs, and finally use of the Cas9 nuclease to mediate both NHEJ- and HDR-based genome modifications in human embryonic kidney (HEK-293FT) and human stem cell (HUES9) lines. This protocol can likewise be applied to other cell types and organisms.

Target selection for sgRNA: There are two main considerations in the selection of the 20-nt guide sequence for gene targeting: 1) the target sequence should precede the 5'-NGG PAM for S. pyogenes Cas9, and 2) guide sequences should be chosen to minimize off-target activity. Applicants provided an online Cas9 targeting design tool that takes an input sequence of interest and identifies suitable target sites. To experimentally assess off-target modifications for each sgRNA, Applicants also provide computationally predicted off-target sites for each intended target, ranked according to Applicants' quantitative specificity analysis on the effects of base-pairing mismatch identity, position, and distribution.

The detailed information on computationally predicted off-target sites is as follows:
Considerations for Off-target Cleavage Activities: Similar to other nucleases, Cas9 can cleave off-target DNA targets in the genome at reduced frequencies. The extent to which a given guide sequence exhibit off-target activity depends on a combination of factors including enzyme concentration, thermodynamics of the specific guide sequence employed, and the abundance of similar sequences in the target genome. For routine application of Cas9, it is important to consider ways to minimize the degree of off-target cleavage and also to be able to detect the presence of off-target cleavage.

Minimizing off-target activity: For application in cell lines, Applicants recommend following two steps to reduce the degree of off-target genome modification. First, using our online CRISPR target selection tool, it is possible to computationally assess the likelihood of a given guide sequence to have off-target sites. These analyses are performed through an exhaustive search in the genome for off-target sequences that are similar sequences as the guide sequence. Comprehensive experimental investigation of the effect of mismatching bases between the sgRNA and its target DNA revealed that mismatch tolerance is 1) position dependent - the 8-14 bp on the 3' end of the guide sequence are less tolerant of mismatches than the 5' bases, 2) quantity dependent - in general more than 3 mismatches are not tolerated, 3) guide sequence dependent - some guide sequences are less tolerant of mismatches than others, and 4) concentration dependent - off-target cleavage is highly sensitive to the amount of transfected DNA. The Applicants' target site analysis web tool (available at the website genome-engineering.org/tools) integrates these criteria to provide predictions for likely off-target sites in the target genome. Second, Applicants recommend titrating the amount of Cas9 and sgRNA expression plasmid to minimize off-target activity.

Detection of off-target activities: Using Applicants' CRISPR targeting web tool, it is possible to generate a list of most likely off-target sites as well as primers performing SURVEYOR or sequencing analysis of those sites. For isogenic clones generated using Cas9, Applicants strongly recommend sequencing these candidate off-target sites to check for any undesired mutations. It is worth noting that there may be off target modifications in sites that are not included in the predicted candidate list and full genome sequence should be performed to completely verify the absence of off-target sites. Furthermore, in multiplex assays where several

DSBs are induced within the same genome, there may be low rates of translocation events and can be evaluated using a variety of techniques such as deep sequencing.

The online tool provides the sequences for all oligos and primers necessary for 1) preparing the sgRNA constructs, 2) assaying target modification efficiency, and 3) assessing cleavage at potential off-target sites. It is worth noting that because the U6 RNA polymerase III promoter used to express the sgRNA prefers a guanine (G) nucleotide as the first base of its transcript, an extra G is appended at the 5' of the sgRNA where the 20-nt guide sequence does not begin with G.

Approaches for sgRNA construction and delivery: Depending on the desired application, sgRNAs may be delivered as either 1) PCR amplicons containing an expression cassette or 2) sgRNA-expressing plasmids. PCR-based sgRNA delivery appends the custom sgRNA sequence onto the reverse PCR primer used to amplify a U6 promoter template. The resulting amplicon may be co-transfected with a plasmid containing Cas9 (PX165). This method is optimal for rapid screening of multiple candidate sgRNAs, as cell transfections for functional testing can be performed mere hours after obtaining the sgRNA-encoding primers. Because this simple method obviates the need for plasmid-based cloning and sequence verification, it is well suited for testing or co-transfecting a large number of sgRNAs for generating large knockout libraries or other scale-sensitive applications. Note that the sgRNA-encoding primers are over 100-bp, compared to the ~20-bp oligos required for plasmid-based sgRNA delivery.

Construction of an expression plasmid for sgRNA is also simple and rapid, involving a single cloning step with a pair of partially complementary oligonucleotides. After annealing the oligo pairs, the resulting guide sequences may be inserted into a plasmid bearing both Cas9 and an invariant scaffold bearing the remainder of the sgRNA sequence (PX330). The transfection plasmids may also be modified to enable virus production for *in vivo* delivery.

In addition to PCR and plasmid-based delivery methods, both Cas9 and sgRNA can be introduced into cells as RNA.

Design of repair template: Traditionally, targeted DNA modifications have required use of plasmid-based donor repair templates that contain homology arms flanking the site of alteration. The homology arms on each side can vary in length, but are typically longer than 500 bp. This method can be used to generate large modifications, including insertion of reporter genes such as fluorescent proteins or antibiotic resistance markers. The design and construction of targeting plasmids has been described elsewhere.

More recently, single-stranded DNA oligonucleotides (ssODNs) have been used in place of targeting plasmids for short modifications within a defined locus without cloning. To achieve high HDR efficiencies, ssODNs contain flanking sequences of at least 40 bp on each side that are homologous to the target region, and can be oriented in either the sense or antisense direction relative to the target locus.

Functional testing

SURVEYOR nuclease assay: Applicants detected indel mutations either by the SURVEYOR nuclease assay (or PCR amplicon sequencing. Applicants online CRISPR target design tool provides recommended primers for both approaches. However, SURVEYOR or sequencing primers may also be designed manually to amplify the region of interest from genomic DNA and to avoid non-specific amplicons using NCBI Primer-BLAST. SURVEYOR primers should be designed to amplify 300-400 bp (for a 600-800 bp total amplicon) on either side of the Cas9 target for allowing clear visualization of cleavage bands by gel electrophoresis. To prevent excessive primer dimer formation, SURVEYOR primers should be designed to be typically under 25-nt long with melting temperatures of ~60°C. Applicants recommend testing each pair of candidate primers for specific PCR amplicons as well as for the absence of non-specific cleavage during the SURVEYOR nuclease digestion process.

Plasmid- or ssODN-mediated HDR: HDR can be detected via PCR-amplification and sequencing of the modified region. PCR primers for this purpose should anneal outside the region spanned by the homology arms to avoid false detection of residual repair template (HDR Fwd and Rev, Fig. 30). For ssODN-mediated HDR, SURVEYOR PCR primers can be used.

Detection of indels or HDR by sequencing: Applicants detected targeted genome modifications by either Sanger or next-generation deep sequencing (NGS). For the former, genomic DNA from modified region can be amplified using either SURVEYOR or HDR primers. Amplicons should be subcloned into a plasmid such as pUC19 for transformation; individual colonies can be sequenced to reveal clonal genotype.

Applicants designed next-generation sequencing (NGS) primers for shorter amplicons, typically in the 100-200 bp size range. For detecting NHEJ mutations, it is important to design primers with at least 10-20 bp between the priming regions and the Cas9 target site to allow detection of longer indels. Applicants provide guidelines for a two-step PCR method to attach barcoded adapters for multiplex deep sequencing. Applicants recommend the Illumina platform, due to its generally low levels of false positive indels. Off-target analysis (described previously) can then be performed through read alignment programs such as ClustalW, Geneious, or simple sequence analysis scripts.

Materials and Reagents

### sgRNA preparation:

UltraPure DNaseRNase-free distilled water (Life Technologies, cat. no. 10977-023)
Herculase II fusion polymerase (Agilent Technologies, cat. no. 600679)
CRITICAL. Standard Taq polymerase, which lacks 3'-5' exonuclease proofreading activity, has lower fidelity and can lead to amplification errors. Herculase II is a high-fidelity polymerase (equivalent fidelity to Pfu) that produces high yields of PCR product with minimal optimization. Other high-fidelity polymerases may be substituted.
Herculase II reaction buffer (5x; Agilent Technologies, included with polymerase) dNTP solution mix (25 mM each; Enzymatics, cat. no. N205L)
MgCl2 (25mM; ThermoScientific, cat. no. R0971)
QIAquick gel extraction kit (Qiagen, cat. no. 28704)
QIAprep spin miniprep kit (Qiagen, cat. no. 27106)
UltraPure TBE buffer (10X; Life Technologies, cat. no. 15581-028)
SeaKem LE agarose (Lonza, cat. no. 50004)
SYBR Safe DNA stain (10,000x; Life Technologies, cat. no. S33102)
1-kb Plus DNA ladder (Life Technologies, cat. no. 10787-018)
TrackIt CyanOrange loading buffer (Life Technologies, cat. no. 10482-028)
FastDigest BbsI (BpiI) (Fermentas/ThermoScientific, cat. no. FD1014)
Fermentas Tango Buffer (Fermentas/ThermoScientific, cat. no. BY5)
DL-dithiothreitol (DTT; Fermentas/ThermoScientific, cat. no. R0862)
T7 DNA ligase (Enzymatics, cat. no. L602L)
Critical:Do not substitute the more commonly used T4 ligase. T7 ligase has 1,000-fold higher activity on the sticky ends than on the blunt ends and higher overall activity than commercially available concentrated T4 ligases.
T7 2X Rapid Ligation Buffer (included with T7 DNA ligase, Enzymatics, cat. no. L602L)
T4 Polynucleotide Kinase (New England Biolabs, cat. no M0201S)
T4 DNA Ligase Reaction Buffer (10X; New England Biolabs, cat. no B0202S)
Adenosine 5'-triphosphate (10 mM; New England Biolabs, cat. no. P0756S)
PlasmidSafe ATP-dependent DNase (Epicentre, cat. no. E3101K)
One Shot Stbl3 chemically competent Escherichia coli (E. coli) (Life Technologies, cat. no. C7373-03)
SOC medium (New England Biolabs, cat. no. B9020S)
LB medium (Sigma, cat. no. L3022)
LB agar medium (Sigma, cat. no. L2897)
Ampicillin, sterile filtered (100 mg ml-1; Sigma, cat. no. A5354)

### Mammalian cell culture:

HEK293FT cells (Life Technologies, cat. no. R700-07)
Dulbecco's minimum Eagle's medium (DMEM, IX, high glucose; Life Technologies, cat. no. 10313-039)
Dulbecco's minimum Eagle's medium (DMEM, IX, high glucose, no phenol red; Life Technologies, cat. no. 31053-028)
Dulbecco's phosphate-buffered saline (DPBS, 1X; Life Technologies, cat. no. 14190-250)
Fetal bovine serum, qualified and heat inactivated (Life Technologies, cat. no. 10438-034)

Opti-MEM I reduced-serum medium (FBS; Life Technologies, cat. no. 11058-021)
Penicillin-streptomycin (100x; Life Technologies, cat. no. 15140-163)
TrypLE™ Express (IX, no Phenol Red; Life Technologies, cat. no. 12604-013)
Lipofectamine 2000 transfection reagent (Life Technologies, cat. no. 11668027)
Amaxa SF Cell Line 4D-Nucleofector® X Kit S (32 RCT; Lonza, cat. no V4XC-2032)
HUES 9 cell line (HARVARD STEM CELL SCIENCE)
Geltrex LDEV-Free Reduced Growth Factor Basement Membrane Matrix (Life Technologies, cat. no. A1413201)
mTeSR1 medium (Stemcell Technologies, cat. no. 05850)
Accutase cell detachment solution (Stemcell Technologies, cat. no. 07920)
ROCK Inhibitor (Y-27632; Millipore, cat. no. SCM075)
Amaxa P3 Primary Cell 4D-Nucleofector® X Kit S (32 RCT; Lonza cat. no. V4XP-3032)

**Genotyping analysis:**

QuickExtract DNA extraction solution (Epicentre, cat. no. QE09050)
PCR primers for SURVEYOR, RFLP analysis, or sequencing (see Primer table)
Herculase II fusion polymerase (Agilent Technologies, cat. no. 600679)
CRITICAL. As Surveyor assay is sensitive to single-base mismatches, it is particularly important to use a high-fidelity polymerase. Other high-fidelity polymerases may be substituted.
Herculase II reaction buffer (5x; Agilent Technologies, included with polymerase)
dNTP solution mix (25 mM each; Enzymatics, cat. no. N205L)
QIAquick gel extraction kit (Qiagen, cat. no. 28704)
Taq Buffer (10x; Genscript, cat. no. B0005)
SURVEYOR mutation detection kit for standard gel electrophoresis (Transgenomic, cat. no. 706025)
UltraPure TBE buffer (10x; Life Technologies, cat. no. 15581-028)
SeaKem LE agarose (Lonza, cat. no. 50004)
4-20% TBE Gels 1.0 mm, 15 Well (Life Technologies, cat. no. EC62255BOX)
Novex® Hi-Density TBE Sample Buffer (5X; Life Technologies, cat. no. LC6678)
SYBR Gold Nucleic Acid Gel Stain (10,000X; Life Technologies, cat. no. S-11494)
1-kb Plus DNA ladder (Life Technologies, cat. no. 10787-018)
TrackIt CyanOrange loading buffer (Life Technologies, cat. no. 10482-028)
FastDigest HindIII (Fermentas/ThermoScientific, cat. no. FD0504)

**Equipment**

Filtered sterile pipette tips (Corning)
Standard 1.5ml microcentrifuge tubes (Eppendorf, cat. no. 0030 125.150)
Axygen 96-well PCR plates (VWR, cat. no. PCR-96M2-HSC)
Axygen 8-Strip PCR tubes (Fischer Scientific, cat. no. 14-222-250)
Falcon tubes, polypropylene, 15 ml (BD Falcon, cat. no. 352097)
Falcon tubes, polypropylene, 50 ml (BD Falcon, cat. no. 352070)
Round-bottom Tube with cell strainer cap, 5ml (BD Falcon, cat. no. 352235)
Petri dishes (60 mm × 15 mm; BD Biosciences, cat. no. 351007)
Tissue culture plate (24 well; BD Falcon, cat. no. 353047)
Tissue culture plate (96 well, flat bottom; BD Falcon, cat. no. 353075)
Tissue culture dish (100 mm; BD Falcon, 353003)
96-well thermocycler with programmable temperature stepping functionality (Applied Biosystems Veriti, cat. no. 4375786).
Desktop microcentrifuges 5424, 5804 (Eppendorf)
Gel electrophoresis system (PowerPac basic power supply, Bio-Rad, cat. no. 164-5050, and Sub-Cell GT System gel tray, Bio-Rad, cat. no. 170-4401)
Novex XCell SureLock Mini-Cell (Life Technologies, cat. no. EI0001)
Digital gel imaging system (GelDoc EZ, Bio-Rad, cat. no. 170-8270, and blue sample tray, Bio-Rad, cat. no. 170-8273)
Blue light transilluminator and orange filter goggles (SafeImager 2.0; Invitrogen, cat. no. G6600)
Gel quantification software (Bio-Rad, ImageLab, included with GelDoc EZ,or open-source ImageJ from the National Institutes of Health, available at the website rsbweb.nih.gov/ij/) UV spectrophotometer (NanoDrop 2000c, Thermo Scientific)

Reagent Setup

Tris-borate EDTA (TBE) electrophoresis solution Dilute TBE buffer in distilled water to IX working solution for casting agarose gels and for use as a buffer for gel electrophoresis. Buffer may be stored at room temperature (18 - 22 °C) for at least 1 year.

- ATP, 10 mM Divide 10 mM ATP into 50-μl aliquots and store at - 20 °C for up to 1 year; avoid repeated freeze-thaw cycles.
- DTT, 10 mM Prepare 10 mM DTT solution in distilled water and store in 20-μl aliquots at - 70 °C for up to 2 years; for each reaction, use a new aliquot, as DTT is easily oxidized.
- D10 culture medium For culture of HEK293FT cells, prepare D10 culture medium by supplementing DMEM with 1X GlutaMAX and 10% (vol/vol) fetal bovine serum. As indicated in the protocol, this medium can also be supplemented with 1X penicillin-streptomycin . D10 medium can be made in advance and stored at 4 °C for up to 1 month.
- mTeSR1 culture medium For culture of human embryonic stem cells, prepare mTeSR1 medium by supplementing the 5X supplement (included with mTeSR1 basal medium), and 100 ug/ml Normocin.

**Procedure**

Design of targeting components and use of the online tool • Timing 1 d

1| Input target genomic DNA sequence. Applicants provide an online Cas9 targeting design tool that takes an input sequence of interest, identifies and ranks suitable target sites, and computationally predicts off-target sites for each intended target. Alternatively, one can manually select guide sequence by identifying the 20-bp sequence directly upstream of any 5'-NGG.

2| Order necessary oligos and primers as specified by the online tool. If the site is chosen manually, the oligos and primers should be designed.

Preparation of sgRNA expression construct

3| To generate the sgRNA expression construct, either the PCR- or plasmid-based protocol can be used.

(A) via PCR amplification • Timing 2 h

(i) Applicants prepare diluted U6 PCR template. Applicants recommend using PX330 as a PCR template, but any U6-containing plasmid may likewise be used as the PCR template. Applicants diluted template with ddH$_2$O to a concentration of 10 ng/ul. Note that if a plasmid or cassette already containing an U6-driven sgRNA is used as a template, a gel extraction needs to be performed to ensure that the product contains only the intended sgRNA and no trace sgRNA carryover from template.

(ii) Applicants prepared diluted PCR oligos. U6-Fwd and U6-sgRNA-Rev primers are diluted to a final concentration of 10 uM in ddH$_2$O (add 10 ul of 100 uM primer to 90 ul ddH$_2$O).

(iii) U6-sgRNA PCR reaction. Applicants set up the following reaction for each U6-sgRNA-Rev primer and mastermix as needed:

| Component: | Amount (ul) | Final concentration |
|---|---|---|
| Herculase II PCR buffer, 5X | 10 | 1X |
| dNTP, 100mM (25mM each) | 0.5 | 1 mM |
| U6 template (PX330) | 1 | 0.2 ng/ul |
| U6-Fwd primer | 1 | 0.2 uM |
| U6-sgRNA-Rev primer (variable) | 1 | 0.2 uM |
| Herculase II Fusion polymerase | 0.5 | |
| Distilled water | 36 | |
| Total | 50 | |

(iv) Applicants performed PCR reaction on the reactions from step (iii) using the following cycling conditions:

| Cycle number | Denature | Anneal | Extend |
|---|---|---|---|
| 1 | 95°C, 2 m | | |
| 2-31 | 95°C, 20 s | 60°C, 20 s | 72°C, 20 s |
| 32 | | | 72°C, 3 m |

(v) After the reaction is completed, Applicants ran the product on a gel to verify successful, single-band amplification. Cast a 2% (wt/vol) agarose gel in 1X TBE buffer with 1X SYBR Safe dye. Run 5 ul of the PCR product in the gel at 15 V cm-1 for 20-30 min. Successful amplicons should yield one single 370-bp product and the template should be invisible. It should not be necessary to gel extract the PCR amplicon.

(vi) Applicants purified the PCR product using the QIAquick PCR purification kit according to the manufacturer's directions. Elute the DNA in 35 ul of Buffer EB or water. Purified PCR products may be stored at 4°C or -20°C.

## (B) Cloning sgRNA into Cas9-containing bicistronic expression vector • Timing 3 d

(i) *Prepare the sgRNA oligo inserts.* Applicants resuspended the top and bottom strands of oligos for each sgRNA design to a final concentration of 100 uM. Phosphorylate and anneal the

| oligo as follows: | |
|---|---|
| Oligo 1 (100 uM) | 1 ul |
| Oligo 2 (100 uM) | 1 ul |
| T4 Ligation Buffer, 10X | 1 ul |
| T4 PNK | 1 ul |
| ddH$_2$O | 6 ul |
| Total | 10 ul |

(ii) Anneal in a thermocycler using the following parameters:

37°C for 30 m

95°C for 5 m

Ramp down to 25°C at 5°C per m

(iii) Applicants diluted phosphorylated and annealed oligos 1:200 by add 1ul of oligo to 199 ul room temperature ddH$_2$O.

(iv) *Clone sgRNA oligo into PX330.* Applicants set up Golden Gate reaction for each sgRNA. Applicants recommend also setting up a no-insert, PX330 only negative control.

| PX330 (100 ng) | x ul |
|---|---|
| Diluted oligo duplex from step (iii) | 2 ul |
| Tango Buffer, 10X | 2 ul |
| DTT, 10mM | 1 ul |
| ATP, 10mM | 1 ul |
| FastDigest BbsI | 1 ul |
| T7 Ligase | 0.5 ul |
| ddH$_2$O | x ul |
| Total | 20 ul |

(v) Incubate the Golden Gate reaction for a total of 1 h:

Cycle number Condition

1-6 37°C for 5 m, 21°C for 5 m

(vi) Applicants treated Golden Gate reaction with PlasmidSafe exonuclease to digest any residual linearized DNA. This step is optional but highly recommended.

| Golden Gate reaction from step 4 | 11 ul |
|---|---|
| 10X PlasmidSafe Buffer | 1.5 ul |
| ATP, 10 mM | 1.5 ul |
| PlasmidSafe exonuclease | 1 ul |
| Total | 15 ul |

(vii) Applicants incubated the PlasmidSafe reaction at 37°C for 30 min, followed by inactivation at 70°C for 30 min. **Pause point:** after completion, the reaction may be frozen and continued later. The circular DNA should be stable for at least 1 week.

(viii) *Transformation.* Applicants transformed the PlasmidSafe-treated plasmid into a competent *E. coli* strain, according to the protocol supplied with the cells. Applicants recommend Stbl3 for quick transformation. Briefly, Applicants added 5ul of the product from step (vii) into 20ul of ice-cold chemically competent Stbl3 cells. This is then incubated on ice for 10 m, heat shocked at 42°C for 30 s, returned immediately to ice for 2 m, 100 ul of SOC medium is added, and this is plated onto an LB plate containing 100 ug/ml ampicillin with incubation overnight at 37°C.

(ix) Day 2: Applicants inspected plates for colony growth. Typically, there are no colonies on the negative control plates (ligation of BbsI-digested PX330 only, no annealed sgRNA oligo), and tens to hundreds of colonies on the PX330-sgRNA cloning plates.

(x) From each plate, Applicants picked 2-3 colonies to check correct insertion of sgRNA. Applicants used a sterile pipette tip to inoculate a single colony into a 3 ml culture of LB medium with 100 ug/ml ampicillin. Incubate and shake at 37°C overnight.

(xi) Day 3: Applicants isolated plasmid DNA from overnight cultures using a QiAprep Spin miniprep kit according to the manufacturer's instructions.

(xii) *Sequence validate CRISPR plasmid.* Applicants verified the sequence of each colony by sequencing from the U6 promoter using the U6-Fwd primer. Optional: sequence the Cas9 gene using primers listed in the following Primer table.

| Primer | Sequence (5' to 3') | (SEQ ID NO: ) | Purpose |
|---|---|---|---|
| U6-For | GAGGGCCTATTTCCCATGATTCC | 182 | Amplify U6-sgRNA |
| U6-Rev | AAAAAAAGCACCGACTCGGTGCCACTTTTT CAAGTTGATAACGGACTAGCCTTATTTTAA CTTGCTATTTCTAG CTCTAAAACNNNNNNNNNNNNNNNNNNNC CGGTGTTTCGTCCTTTCCACAAG | 183 | Amplify U6-sgRNA; N is reverse complement of target |
| sgRNA-top | CACCGNNNNNNNNNNNNNNNNNNN | 184 | Clone sgRNA into PX330 |
| sgRNA - bottom | AAACNNNNNNNNNNNNNNNNNNNC | 185 | Clone sgRNA into PX330 |
| U6-*EMX1*-Rev | AAAAAAAGCACCGACTCGGTGCCACTTTTT CAAGTTGATAACGGACTAGCCTTATTTTAA CTTGCTATTTCTAG CTCTAAAACCCCTAGTCATTGGAGGTGACC GGTGTTTCGTCCTTTCCACAAG | 186 | Amplify U6-EMX1 sgRNA |

(continued)

| Primer | Sequence (5' to 3') | (SEQ ID NO: ) | Purpose |
|---|---|---|---|
| *EMX1*-top | CACCGTCACCTCCAATGACTAGGG | 187 | Clone EMX1 sgRNA into PX330 |
| *EMX1*-bottom | AAACCCCTAGTCATTGGAGGTGAC | 188 | Clone EMX1 sgRNA into PX330 |
| ssODN-sense | CAGAAGAAGAAGGGCTCCCATCACATCAA CCGGTGGCGCATTGCCACGAAGCAGGCCA ATGGGGAGGACATC GATGTCACCTCCAATGAC<u>AAGCTTGCTAGC</u> GGTGGGCAACCACAAACCCACGAGGGCAG AGTGCTGCTTGCTG CTGGCCAGGCCCCTGCGTGGGCCCAAGCTG GACTCTGGCCACTCCCT | 189 | EMX1HDR (sense; insertion underlined) |
| ssODN-antisense | AGGGAGTGGCCAGAGTCCAGCTTGGGCCC ACGCAGGGGCCTGGCCAGCAGCAAGCAGC ACTCTGCCCTCGTG GGTTTGTGGTTGCCCACC<u>GCTAGCAAGCTT</u> GTCATTGGAGGTGACATCGATGTCCTCCCC ATTGGCCTGCTTCG TGGCAATGCGCCACCGGTTGATGTGATGGG AGCCCTTCTTCTTCTG | 190 | EMX1HDR (antisense; insertion underlined) |
| EMX1-SURV-F | CCATCCCCTTCTGTGAATGT | 191 | EMX1 SURVEYOR assay PCR, sequencing |
| EMX1-SURV-R | GGAGATTGGAGACACGGAGA | 192 | EMX1 SURVEYOR assay PCR, sequencing |
| EMX1-HDR-F | GGCTCCCTGGGTTCAAAGTA | 193 | EMX1 RFLP analysis PCR, sequencing |
| EMX1-HDR-R | AGAGGGGTCTGGATGTCGTAA | 194 | EMX1 RFLP analysis PCR, sequencing |
| pUC19-F | CGCCAGGGTTTTCCCAGTCACGAC | 195 | pUC19 multiple cloning site F primer, for Sanger sequencing |

Applicants referenced the sequencing results against the PX330 cloning vector sequence to check that the 20 bp guide sequence was inserted between the U6 promoter and the remainder of the sgRNA scaffold. Details and sequence of the PX330 map in GenBank vector map format (*.gb file) can be found at the website crispr.genome-engineering.org.

**(Optional) Design of ssODN template • Timing 3 d planning ahead**

3| *Design and order ssODN.* Either the sense or antisense ssODN can be purchased directly from supplier. Applicants recommend designing homology arms of at least 40 bp on either side and 90 bp for optimal HDR efficiency. In

171

Applicants' experience, antisense oligos have slightly higher modification efficiencies.

4| Applicants resuspended and diluted ssODN ultramers to a final concentration of 10 uM. Do not combine or anneal the sense and antisense ssODNs. Store at -20°C.

5| Note for HDR applications, Applicants recommend cloning sgRNA into the PX330 plasmid.

## Functional validation of sgRNAs: cell culture and transfections • Timing 3-4 d

The CRISPR-Cas system has been used in a number of mammalian cell lines. Conditions may vary for each cell line. The protocols below details transfection conditions for HEK239FT cells. Note for ssODN-mediated HDR transfections, the Amaxa SF Cell Line Nucleofector Kit is used for optimal delivery of ssODNs. This is described in the next section.

7| *HEK293FT maintenance.* Cells are maintained according to the manufacturer's recommendations. Briefly, Applicants cultured cells in D10 medium (GlutaMax DMEM supplemented with 10% Fetal Bovine Serum), at 37°C and 5% $CO_2$.

8| To passage, Applicants removed medium and rinsed once by gently adding DPBS to side of vessel, so as not to dislodge cells. Applicants added 2 ml of TrypLE to a T75 flask and incubated for 5 m at 37°C. 10 ml of warm D10 medium is added to inactivate and transferred to a 50 ml Falcon tube. Applicants dissociated cells by triturating gently, and re-seeded new flasks as necessary. Applicants typically passage cells every 2-3 d at a split ratio of 1:4 or 1:8, never allowing cells to reach more than 70% confluency. Cell lines are restarted upon reaching passage number 15.

9| *Prepare cells for transfection.* Applicants plated well-dissociated cells onto 24-well plates in D10 medium without antibiotics 16-24 h before transfection at a seeding density of $1.3 \times 10^5$ cells per well and a seeding volume of 500 ul. Scale up or down according to the manufacturer's manual as needed. It is suggested to not plate more cells than recommended density as doing so may reduce transfection efficiency.

10| On the day of transfection, cells are optimal at 70-90% confluency. Cells may be transfected with Lipofectamine 2000 or Amaxa SF Cell Line Nucleofector Kit according to the manufacturers' protocols.

(A) For sgRNAs cloned into PX330, Applicants transfected 500 ng of sequence-verified CRISPR plasmid; if transfecting more than one plasmid, mix at equimolar ratio and no more than 500 ng total.

(B) For sgRNA amplified by PCR, Applicants mixed the following:

| | |
|---|---|
| PX165 (Cas9 only) | 200 ng |
| sgRNA amplicon (each) | 40 ng |
| pUC19 | fill up total DNA to 500 ng |

Applicants recommend transfecting in technical triplicates for reliable quantification and including transfection controls (e.g. GFP plasmid) to monitor transfection efficiency. In addition, PX330 cloning plasmid and/or sgRNA amplicon may be transfected alone as a negative control for downstream functional assays.

111 Applicants added Lipofectamine complex to cells gently as HEK293FT cells may detach easily from plate easily and result in lower transfection efficiency.

12| Applicants checked cells 24 h after transfection for efficiency by estimating the fraction of fluorescent cells in the control (e.g., GFP) transfection using a fluorescence microscope. Typically cells are more than 70% transfected.

13| Applicants supplemented the culture medium with an additional 500 ul of warm D10 medium. Add D10 very slowly to the side of the well and do not use cold medium, as cells can detach easily.

14| Cells are incubated for a total of 48-72 h post-transfection before harvested for indel analysis. Indel efficiency does not increase noticeably after 48 h.

## (Optional) Co-transfection of CRISPR plasmids and ssODNs or targeting plasmids for HR • Timing 3-4 d

15| *Linearize targeting plasmid.* Targeting vector is linearized if possible by cutting once at a restriction site in the vector backbone near one of the homology arms or at the distal end of either homology arm.

16| Applicants ran a small amount of the linearized plasmid alongside uncut plasmid on a 0.8-1% agarose gel to check successful linearization. Linearized plasmid should run above the supercoiled plasmid.

17| Applicants purified linearized plasmid with the QIAQuick PCR Purification kit.

18| Prepare cells for transfection. Applicants cultured HEK293FT in T75 or T225 flasks. Sufficient cell count before day of transfection is planned for. For the Amaxa strip-cuvette format, $2 \times 10^6$ cells are used per transfection.

19| Prepare plates for transfection. Applicants added 1 ml of warm D10 medium into each well of a 12 well plate. Plates are placed into the incubator to keep medium warm.

20| Nucleofection. Applicants transfected HEK293FT cells according to the Amaxa SF Cell Line Nucleofector 4D Kit manufacturer's instructions, adapted in the steps below.
a. For ssODN and CRISPR cotransfection, pre-mix the following DNA in PCR tubes:

| | |
|---|---|
| pCRISPR plasmid (Cas9 + sgRNA) | 500 ng |
| ssODN template (10uM) | 1 ul |

b. For HDR targeting plasmid and CRISPR cotransfection, pre-mix the following DNA in PCR tubes:

| | |
|---|---|
| CRISPR plasmid (Cas9 + sgRNA) | 500 ng |
| Linearized targeting plasmid | 500 ng |

For transfection controls, see previous section. In addition, Applicants recommend transfecting ssODN or targeting plasmid alone as a negative control.

21| Dissociate to single cells. Applicants removed medium and rinsed once gently with DPBS, taking care not to dislodge cells. 2 ml of TrypLE is added to a T75 flask and incubated for 5 m at 37°C. 10 ml of warm D10 medium is added to inactivate and triturated gently in a 50 ml Falcon tube. It is recommended that cells are triturated gently and dissociated to single cells. Large clumps will reduce transfection efficiency. Applicants took a 10 ul aliquot from the suspension and diluted into 90 ul of D10 medium for counting. Applicants counted cells and calculated the number of cells and volume of suspension needed for transfection. Applicants typically transfected $2 \times 10^5$ cells per condition using the Amaxa Nucleocuvette strips, and recommend calculating for 20% more cells than required to adjust for volume loss in subsequent pipetting steps. The volume needed is transferred into a new Falcon tube.

23| Applicants spun down the new tube at 200 x g for 5 m.

Applicants prepared the transfection solution by mixing the SF solution and S1 supplement as recommended by Amaxa. For Amaxa strip-cuvettes, a total of 20 ul of supplemented SF solution is needed per transfection. Likewise, Applicants recommend calculating for 20% more volume than required.

25| Applicants removed medium completely from pelleted cells from step 23 and gently resuspended in appropriate volume (20 ul per $2 \times 10^5$ cells) of S1-supplemented SF solution. Do not leave cells in SF solution for extended period of time.

26| 20 ul of resuspended cells is pipetted into each DNA pre-mix from step 20. Pipette gently to mix and transfer to Nucleocuvette strip chamber. This is repeated for each transfection condition.

Electroporate cells using the Nucleofector 4D program recommended by Amaxa, CM-130.

28| Applicants gently and slowly pipetted 100 ul of warm D10 medium into each Nucleocuvette strip chamber, and transferred all volume into the pre-warmed plate from step 19. CRITICAL. Cells are very fragile at this stage and harsh pipetting can cause cell death. Incubate for 24 h. At this point, transfection efficiency can be estimated from fraction of fluorescent cells in positive transfection control. Nucleofection typically results in greater than 70-80% transfection efficiency. Applicants slowly added 1 ml warm D10 medium to each well without dislodging the cells. Incubate cells for a total of 72 h.

**Human embryonic stem cell (HUES 9) culture and transfection • Timing 3-4 d**

Maintaining hESC (HUES9) line. Applicants routinely maintain HUES9 cell line in feeder-free conditions with mTesR1 medium. Applicants prepared mTeSR1 medium by adding the 5X supplement included with basal medium and 100 ug/ml Normocin. Applicants prepared a 10 ml aliquot of mTeSR1 medium supplemented further with 10 uM Rock Inhibitor. Coat tissue culture plate. Dilute cold GelTrex 1:100 in cold DMEM and coat the entire surface of a 100 mm tissue culture plate.

Place plate in incubator for at least 30 m at 37°C. Thaw out a vial of cells at 37°C in a 15 ml Falcon tube, add 5 ml of mTeSR1 medium, and pellet at 200 x g for 5 m. Aspirate off GelTrex coating and seed ~1 x 106 cells with 10 ml mTeSR1 medium containing Rock Inhibitor. Change to normal mTeSR1 medium 24 h after transfection and re-feed daily. Passaging cells. Re-feed cells with fresh mTeSR1 medium daily and passage before reaching 70% confluency. Aspirate off mTeSR1 medium and wash cells once with DPBS. Dissociate cells by adding 2 ml Accutase and incubating at 37°C for 3 - 5 m. Add 10 ml mTeSR1 medium to detached cells, transfer to 15 ml Falcon tube and resuspend gently. Re-plate onto GelTrex-coated plates in mTeSR1 medium with 10 uM Rock Inhibitor. Change to normal mTeSR1 medium 24 h after plating.

Transfection. Applicants recommend culturing cells for at least 1 week post-thaw before transfecting using the Amaxa P3 Primary Cell 4-D Nucleofector Kit (Lonza). Re-feed log-phase growing cells with fresh medium 2 h before transfection. Dissociate to single cells or small clusters of no more than 10 cells with accutase and gentle resuspension.

Count the number of cells needed for nucleofection and spin down at 200 x g for 5 m. Remove medium completely and resuspend in recommended volume of S1-supplemented P3 nucleofection solution. Gently plate electroporated cells into coated plates in presence of 1X Rock Inhibitor.

Check transfection success and re-feed daily with regular mTeSR1 medium beginning 24 h after nucleofection. Typically, Applicants observe greater than 70% transfection efficiency with Amaxa Nucleofection. Harvest DNA. 48-72 h post transfection, dissociate cells using accutase and inactivate by adding 5 x volume of mTeSR1. Spin cells down at 200 x g for 5 m. Pelleted cells can be directed processed for DNA extraction with QuickExtract solution. It is recommended to not mechanically dissociate cells without accutase. It is recommended to not spin cells down without inactivating accutase or above the recommended speed; doing so may cause cells to lyse.

**Isolation of clonal cell lines by FACS. Timing • 2-3 h hands-on; 2-3 weeks expansion**

Clonal isolation may be performed 24 h post-transfection by FACS or by serial dilution.

54| *Prepare FACS buffer.* Cells that do not need sorting using colored fluorescence may be sorted in regular D10 medium supplemented with 1X penicillin/streptinomycin. If colored fluorescence sorting is also required, a phenol-free DMEM or DPBS is substituted for normal DMEM. Supplement with 1X penicillin/streptinomycin and filter through a .22 um Steriflip filter.

55| *Prepare 96 well plates.* Applicants added 100 ul of D10 media supplemented with 1X penicillin/streptinomycin per well and prepared the number of plates as needed for the desired number of clones.

56| *Prepare cells for FACS.* Applicants dissociated cells by aspirating the medium completely and adding 100 ul TrypLE per well of a 24-well plate. Incubate for 5 m and add 400 ul warm D10 media.

57| Resuspended cells are transferred into a 15 ml Falcon tube and gently triturated 20 times. Recommended to check under the microscope to ensure dissociation to single cells.

58| Spin down cells at 200 x g for 5 minutes.

59| Applicants aspirated the media, and resuspended the cells in 200 ul of FACS media.

60| Cells are filtered through a 35 um mesh filter into labeled FACS tubes. Applicants recommend using the BD Falcon 12 x 75 mm Tube with Cell Strainer cap. Place cells on ice until sorting.

61| Applicants sorted single cells into 96-well plates prepared from step 55. Applicants recommend that in one single designated well on each plate, sort 100 cells as a positive control.

NOTE. The remainder of the cells may be kept and used for genotyping at the population level to gauge overall modification efficiency.

62| Applicants returned cells into the incubator and allowed them to expand for 2-3 weeks. 100 ul of warm D10 medium is added 5 d post sorting. Change 100 ul of medium every 3-5 d as necessary.

63| Colonies are inspected for "clonal" appearance 1 week post sorting: rounded colonies radiating from a central point. Mark off wells that are empty or may have been seeded with doublets or multiplets.

64| When cells are more than 60% confluent, Applicants prepared a set of replica plates for passaging. 100 ul of D10 medium is added to each well in the replica plates. Applicants dissociated cells directly by pipetting up and down vigorously 20 times. 20% of the resuspended volume was plated into the prepared replica plates to keep the clonal lines. Change the medium every 2-3 d thereafter and passage accordingly.

65| Use the remainder 80% of cells for DNA isolation and genotyping.

**Optional: Isolation of clonal cell lines by dilution. Timing • 2-3 h hands-on; 2-3 weeks expansion**

66| Applicants dissociated cells from 24-well plates as described above. Make sure to dissociate to single cells. A cell strainer can be used to prevent clumping of cells.

67| The number of cells are counted in each condition. Serially dilute each condition in D10 medium to a final concentration of 0.5 cells per 100 ul. For each 96 well plate, Applicants recommend diluting to a final count of 60 cells in 12 ml of D10. Accurate count of cell number is recommended for appropriate clonal dilution. Cells may be recounted at an intermediate serial dilution stage to ensure accuracy.

68| Multichannel pipette was used to pipette 100 ul of diluted cells to each well of a 96 well plate.

NOTE. The remainder of the cells may be kept and used for genotyping at the population level to gauge overall modification efficiency.

69| Applicants inspected colonies for "clonal" appearance ~1 week post plating: rounded colonies radiating from a central point. Mark off wells that may have seeded with doublets or multiplets.

70| Applicants returned cells to the incubator and allowed them to expand for 2-3 weeks. Re-feed cells as needed as detailed in previous section.

**SURVEYOR assay for CRISPR cleavage efficiency. Timing • 5-6 h**

Before assaying cleavage efficiency of transfected cells, Applicants recommend testing each new SURVEYOR primer on negative (untransfected) control samples through the step of SURVEYOR nuclease digestion using the protocol described below. Occasionally, even single-band clean SURVEYOR PCR products can yield non-specific SURVEYOR nuclease cleavage bands and potentially interfere with accurate indel analysis.

71| *Harvest cells for DNA.* Dissociate cells and spin down at 200 x g for 5 m. NOTE. Replica plate at this stage as needed to keep transfected cell lines.

72| Aspirate the supernatant completely.

73| Applicants used QuickExtract DNA extraction solution according to the manufacturer's instructions. Applicants typically used 50 ul of the solution for each well of a 24 well plate and 10 ul for a 96 well plate.

74| Applicants normalized extracted DNA to a final concentration of 100-200 ng/ul with ddH$_2$O. **Pause point:** Extracted DNA may be stored at -20°C for several months.

75| *Set up the SURVEYOR PCR.* Master mix the following using SURVEYOR primers provided by Applicants online/computer algorithm tool:

| Component: | Amount (ul) | Final concentration |
|---|---|---|
| Herculase II PCR buffer, 5X | 10 | 1X |
| dNTP, 100mM (25mM each) | 1 | 1 mM |
| SURVEYOR Fwd primer (10uM) | 1 | 0.2 uM |
| SURVEYOR Rev primer (10uM) | 1 | 0.2 uM |
| Herculase II Fusion polymerase | 1 | |
| MgCl$_2$ (25mM) | 2 | 1 mM |
| Distilled water | 33 | |
| Total | 49 (for each reaction) | |

76| Applicants added 100-200 ng of normalized genomic DNA template from step 74 for each reaction.

77| PCR reaction was performed using the following cycling conditions, for no more than 30 amplification cycles:

| Cycle number | Denature | Anneal | Extend |
|---|---|---|---|
| 1 | 95°C, 2 min | | |
| 2-31 | 95°C, 20 s | 60°C, 20 s | 72°C, 30 s |
| 32 | | | 72°C, 3 min |

78| Applicants ran 2-5 ul of PCR product on a 1% gel to check for single-band product. Although these PCR conditions are designed to work with most pairs of SURVEYOR primers, some primers may need additional optimization by adjusting the template concentration, MgCl$_2$ concentration, and/or the annealing temperature.

79| Applicants purified the PCR reactions using the QIAQuick PCR purification kit and normalized eluant to 20 ng/ul. **Pause point:** Purified PCR product may be stored at -20°C.

80| *DNA heteroduplex formation.* The annealing reaction was set up as follows:

| Taq PCR buffer, 10X | 2 ul |
|---|---|
| Normalized DNA (20 ng/ul) | 18 ul |
| Total volume | 20 ul |

81| Anneal the reaction using the following conditions:

| Cycle number | Condition |
|---|---|
| 1 | 95°C, 10 mn |
| 2 | 95°C-85°C, -2°C/s |
| 3 | 85°C, 1 min |
| 4 | 85°C-75°C, -0.3°C/s |

**175**

(continued)

| Cycle number | Condition |
|---|---|
| 5 | 75°C, 1 min |
| 6 | 75°C-65°C, -0.3°C/s |
| 7 | 65°C, 1 min |
| 8 | 65°C-55°C, -0.3°C/s |
| 9 | 55°C, 1 min |
| 10 | 55°C-45°C, -0.3°C/s |
| 11 | 45°C, 1 min |
| 12 | 45°C-35°C, -0.3°C/s |
| 13 | 35°C, 1 min |
| 14 | 35°C-25°C, -0.3°C/s |
| 15 | 25°C, 1 min |

82| *SURVEYOR nuclease S digestion.* Applicants prepared master-mix and added the following components on ice to annealed heteroduplexes from step 81 for a total final volume of 25 ul:

| Component | Amount (ul) | Final Concentration |
|---|---|---|
| MgCl$_2$ solution, 0.15M | 2.5 | 15mM |
| ddH$_2$O | 0.5 | |
| SURVEYOR nuclease S | 1 | 1X |
| SURVEYOR enhancer S | 1 | 1X |
| Total | 5 | |

83| Vortex well and spin down. Incubate the reaction at 42°C for 1 h.

84| Optional: 2 ul of the Stop Solution from the SURVEYOR kit may be added. **Pause point.** The digested product may be stored at -20°C for analysis at a later time.

85| *Visualize the SURVEYOR reaction.* SURVEYOR nuclease digestion products may be visualized on a 2% agarose gel. For better resolution, products may be run on a 4-20% gradient Polyacrylamide TBE gel. Applicants loaded 10 ul of product with the recommended loading buffer and ran the gel according to manufacturer's instructions. Typically, Applicants run until the bromophenol blue dye has migrated to the bottom of the gel. Include DNA ladder and negative controls on the same gel.

86| Applicants stained the gel with 1X SYBR Gold dye diluted in TBE. The gel was gently rocked for 15 m.

87| Applicants imaged the gel using a quantitative imaging system without overexposing the bands. The negative controls should have only one band corresponding to the size of the PCR product, but may have occasionally non-specific cleavage bands of other sizes. These will not interfere with analysis if they are different in size from target cleavage bands. The sum of target cleavage band sizes, provided by Applicants online/computer algorithm tool, should be equal to the size of the PCR product.

88| *Estimate the cleavage intensity.* Applicants quantified the integrated intensity of each band using ImageJ or other gel quantification software.

89| For each lane, Applicants calculated the fraction of the PCR product cleaved ($f_{cut}$) using the following formula: $f_{cut}$ = *(b + c) / (a + b + c)*, where a is the integrated intensity of the undigested PCR product and b and c are the integrated intensities of each cleavage product. 90| Cleavage efficiency may be estimated using the following formula, based on the binomial probability distribution of duplex formation:

$$91| indel\ (\%) = 100 \times \left(1 - \sqrt{(1 - f_{cut})}\right)$$

**Sanger sequencing for assessing CRISPR cleavage efficiency. Timing • 3 d**

Initial steps are identical to Steps 71-79 of the SURVEYOR assay. Note: SURVEYOR primers may be used for Sanger sequencing if appropriate restriction sites are
appended to the Forward and Reverse primers. For cloning into the recommended pUC19 backbone, EcoRI may be used for the Fwd primer and HindIII for the Rev primer.

92| *Amplicon digestion.* Set up the digestion reaction as follows:

| Component | Amount (ul) |
|---|---|
| Fast Digest buffer, 10X | 3 |
| FastDigest EcoRI | 1 |
| FastDigest HindIII | 1 |
| Normalized DNA (20 ng/ul) | 10 |
| ddH$_2$O | 15 |
| Total volume | 30 |

93| pUC19 backbone digestion. Set up the digestion reaction as follows:

| Component | Amount (ul) |
|---|---|
| Fast Digest buffer, 10X | 3 |
| FastDigest EcoRI | 1 |
| FastDigest HindIII | 1 |
| FastAP Alkaline Phosphatase | 1 |
| pUC19 vector (200 ng/ul) | 5 |
| ddH$_2$O | 20 |
| Total volume | 30 |

94| Applicants purified the digestion reactions using the QIAQuick PCR purification kit. **Pause point:** Purified PCR product may be stored at -20°C.

95| Applicants ligated the digested pUC19 backbone and Sanger amplicons at a 1:3 vector: insert ratio as follows:

| Component | Amount (ul) |
|---|---|
| Digested pUC19 | x (50 ng) |
| Digested insert | x (1:3 vector:insert molar ratio) |
| T7 ligase | 1 |
| 2X Rapid Ligation Buffer | 10 |
| ddH$_2$O | x |
| Total volume | 20 |

96| *Transformation.* Applicants transformed the PlasmidSafe-treated plasmid into a competent *E. coli* strain, according to the protocol supplied with the cells. Applicants recommend Stbl3 for quick transformation. Briefly, 5ul of the product from step 95 is added into 20ul of ice-cold chemically competent Stbl3 cells, incubated on ice for 10 m, heat shocked at 42°C for 30 s, returned immediately to ice for 2 m, 100 ul of SOC medium is added, and plated onto an LB plate containing 100 ug/ml ampicillin. This is incubated overnight at 37°C.

97| Day 2: Applicants inspected plates for colony growth. Typically, there are no colonies on the negative control plates (ligation of EcoRI-HindIII digested pUC19 only, no Sanger amplicon insert), and tens to hundreds of colonies on the pUC19-Sanger amplicon cloning plates.

98| Day 3: Applicants isolated plasmid DNA from overnight cultures using a QIAprep Spin miniprep kit according to the manufacturer's instructions.

99| *Sanger sequencing.* Applicants verified the sequence of each colony by sequencing from the pUC19 backbone using the pUC19-For primer. Applicants referenced the sequencing results against the expected genomic DNA sequence to check for the presence of Cas9-induced NHEJ mutations. % editing efficiency = (# modified clones)/(# total clones). It is important to pick a reasonable number of clones (>24) to generate accurate modification efficiencies.

**Genotyping for microdeletion. Timing • 2-3 d hands on; 2-3 weeks expansion**

100| Cells were transfected as described above with a pair of sgRNAs targeting the region to be deleted.

101| 24 h post-transfection, clonal lines are isolated by FACS or serial dilution as described above.

102| Cells are expanded for 2-3 weeks.

103| Applicants harvested DNA from clonal lines as described above using 10 ul QuickExtract solution and normalized genomic DNA with ddH$_2$O to a final concentration of 50-100 ng/ul.

104| *PCR Amplify the modified region.* The PCR reaction is set up as follows:

| Component: | Amount (ul) | Final concentration |
|---|---|---|
| Herculase II PCR buffer, 5X | 10 | 1X |
| dNTP, 100mM (25mM each) | 1 | 1 mM |
| Out Fwd primer (10uM) | 1 | 0.2 uM |
| Out Rev primer (10uM) | 1 | 0.2 uM |
| Herculase II Fusion polymerase | 1 | |
| MgCl$_2$ (25mM) | 2 | 1 mM |
| ddH$_2$O | 32 | |
| Total | 48 (for each reaction) | |

**[001183]** Note: if deletion size is more than 1 kb, set up a parallel set of PCR reactions with In-Fwd and In-Rev primers to screen for the presence of the wt allele.

105| To screen for inversions, a PCR reaction is set up as follows:

| Component: | Amount (ul) | Final concentration |
|---|---|---|
| Herculase II PCR buffer, 5X | 10 | 1X |
| dNTP, 100mM (25mM each) | 1 | 1 mM |
| Out Fwd *or* Out-Rev primer (10uM) | 1 | 0.2 uM |
| In Fwd *or* In-Rev primer (10uM) | 1 | 0.2 uM |
| Herculase II Fusion polymerase | 1 | |
| MgCl$_2$ (25mM) | 2 | 1 mM |
| ddH$_2$O | 32 | |
| Total | 48 (for each reaction) | |

**[001185]** Note: primers are paired either as Out-Fwd + In Fwd, or Out-Rev + In-Rev.

106| Applicants added 100-200 ng of normalized genomic DNA template from step 103 for each reaction.

107| PCR reaction was performed using the following cycling conditions:

| Cycle number | Denature | Anneal | Extend |
|---|---|---|---|
| 1 | 95°C, 2 min | | |
| 2-31 | 95°C, 20 s | 60°C, 20 s | 72°C, 30 s |
| 32 | | | 72°C, 3 m |

108| Applicants run 2-5 ul of PCR product on a 1-2% gel to check for product. Although these PCR conditions are designed to work with most primers, some primers may need additional optimization by adjusting the template concentration, MgCl$_2$ concentration, and/or the annealing temperature.

**Genotyping for targeted modifications via HDR. Timing • 2-3 d, 2-3 h hands on**

109| Applicants harvested DNA as described above using QuickExtract solution and normalized genomic DNA with TE to a final concentration of 100-200 ng/ul.

110| *PCR Amplify the modified region.* The PCR reaction is set up as follows:

| Component: | Amount (ul) | Final concentration |
|---|---|---|
| Herculase II PCR buffer, 5X | 10 | 1X |

(continued)

| Component: | Amount (ul) | Final concentration |
|---|---|---|
| dNTP, 100 mM (25 mM each) | 1 | 1 mM |
| HDR Fwd primer (10 uM) | 1 | 0.2 uM |
| HDR Rev primer (10 uM) | 1 | 0.2 uM |
| Herculase II Fusion polymerase | 1 | |
| MgCl$_2$ (25mM) | 2 | 1 mM |
| ddH$_2$O | 33 | |
| Total | 49 (for each reaction) | |

111| Applicants added 100-200 ng of genomic DNA template from step 109 for each reaction and run the following program.

| Cycle number | Denature | Anneal | Extend |
|---|---|---|---|
| 1 | 95°C, 2 min | | |
| 2-31 | 95°C, 20 s | 60°C, 20 s | 72°C, *30-60 s per kb* |
| 32 | | | 72°C, 3 min |

112| Applicants ran 5 ul of PCR product on a 0.8-1% gel to check for single-band product. Primers may need additional optimization by adjusting the template concentration, MgCl$_2$ concentration, and/or the annealing temperature.

113| Applicants purified the PCR reactions using the QIAQuick PCR purification kit.

114| In the HDR example, a *Hind*III restriction site is inserted into the EMX1 gene. These are detected by a restriction digest of the PCR amplicon:

| Component | Amount (ul) |
|---|---|
| Purified PCR amplicon (200-300ng) | x |
| F.D. buffer, Green | 1 |
| HindIII | 0.5 |
| ddH2O | x |
| Total | 10 |

i. The DNA is digested for 10 m at 37°C:

ii. Applicants ran 10 ul of the digested product with loading dye on a 4-20% gradient polyacrylamide TBE gel until the xylene cyanol band had migrated to the bottom of the gel.

iii. Applicants stained the gel with 1X SYBR Gold dye while rocking for 15 m.

iv. The cleavage products are imaged and quantified as described above in the SURVEYOR assay section. HDR efficiency is estimated by the formula: $(b + c)/(a + b + c)$, where a is the integrated intensity for the undigested HDR PCR product, and b and c are the integrated intensities for the HindIII-cut fragments.

115| Alternatively, purified PCR amplicons from step 113 may be cloned and genotyped using Sanger sequencing or NGS.

### Deep sequencing and off-target analysis • Timing 1 - 2 d

The online CRISPR target design tool generates candidate genomic off-target sites for each identified target site. Off-target analysis at these sites can be performed by SURVEYOR nuclease assay, Sanger sequencing, or next-generation deep sequencing. Given the likelihood of low or undetectable modification rates at many of these sites, Applicants recommend deep sequencing with the Illumina Miseq platform for high sensitivity and accuracy. Protocols will vary with sequencing platform; here, Applicants briefly describe a fusion PCR method for attaching sequencing adapters.

116| *Design deep sequencing primers.* Next-generation sequencing (NGS) primers are designed for shorter amplicons, typically in the 100-200 bp size range. Primers may be manually designed using NCBI Primer-Blast or generated with online CRISPR target design tools (website at genome-engineering.org/tools).

117| Harvest genomic DNA from Cas9-targeted cells. Normalize QuickExtract genomic DNA to 100-200 ng/ul with ddH$_2$O.

118| *Initial library preparation PCR.* Using the NGS primers from step 116, prepare the initial library preparation PCR

| Component: | Amount (ul) | Final concentration |
|---|---|---|
| Herculase II PCR buffer, 5X | 10 | 1X |
| dNTP, 100mM (25mM each) | 1 | 1 mM |
| NGS Fwd primer (10uM) | 1 | 0.2 uM |
| NGS Rev primer (10uM) | 1 | 0.2 uM |
| Herculase II Fusion polymerase | 1 | |
| $MgCl_2$ (25mM) | 2 | 1 mM |
| ddH2O | 33 | |
| Total | 49 (for each reaction) | |

119| Add 100-200 ng of normalized genomic DNA template for each reaction.

120| Perform PCR reaction using the following cycling conditions, for no more than 20 amplification cycles:

| Cycle number | Denature | Anneal | Extend |
|---|---|---|---|
| 1 | 95°C, 2 min | | |
| 2-21 | 95°C, 20 s | 60°C, 20 s | 72°C, 15 s |
| 22 | | | 72°C, 3 min |

121| Run 2-5 ul of PCR product on a 1% gel to check for single-band product. As with all genomic DNA PCRs, NGS primers may require additional optimization by adjusting the template concentration, $MgCl_2$ concentration, and/or the annealing temperature.

122| Purify the PCR reactions using the QIAQuick PCR purification kit and normalize eluant to 20 ng/ul. **Pause point:** Purified PCR product may be stored at -20°C.

123| *Nextera XT DNA Sample Preparation Kit.* Following the manufacturer's protocol, generate Miseq sequencing-ready libraries with unique barcodes for each sample.

124| *Analyze sequencing data.* Off-target analysis may be performed through read alignment programs such as ClustalW, Geneious, or simple sequence analysis scripts.

**Timing**

Steps 1 - 2 Design and synthesis of sgRNA oligos and ssODNs: 1-5 d, variable depending on supplier
Steps 3 - 5 Construction of CRISPR plasmid or PCR expression cassette: 2 h to 3 d
Steps 6 - 53 Transfection into cell lines: 3 d (1 h hands-on time)
Steps 54 - 70 Optional derivation of clonal lines: 1-3 weeks, variable depending on cell type
Steps 71 - 91 Functional validation of NHEJ via SURVEYOR: 5-6 h
Steps 92 - 124 Genotyping via Sanger or next-gen deep sequencing: 2-3 d (3-4 h hands on time)

**Addressing Situations Concerning Herein Examples**

| Situation | Solution |
|---|---|
| No amplification of sgRNA | Titrate U6-template concentration |
| SURVEYOR or HDR PCR dirty or no amplification | Titrate MgCl2; normalize and titrate template concentration; annealing temp gradient; redesign primers |
| Unequal amplification of alleles in microdeletion PCRs | Set up separate PCRs to detect wildtype and deletion alleles; Redesign primers with similar sized amplicons |
| Colonies on negative control plate | Increase *Bbs*I; increase Golden Gate reaction cycle number, cut PX330 separately with Antarctic Phosphate treatment |
| No sgRNA sequences or wrong sequences | Screen additional colonies |

(continued)

| Situation | Solution |
|---|---|
| Low lipofectamine transfection efficiency | Check cell health and density; titrate DNA; add GFP transfection control |
| Low nucleofection transfection efficiency | Check cell health and density; titrate DNA; suspend to single cell |
| Clumps or no cells after FACS | Filter cells before FACS; dissociate to single cells; resuspend in appropriate density |
| Clumps or no cells in serial dilution | Recount cells; dissociate to single cells and filter through strainer; check serial dilution |
| High SURVEYOR background on negative sample | Redesign primers to prime from different locations |
| Dirty SURVEYOR result on gel | Purify PCR product; reduce input DNA; reduce 42°C incubation to 30 m |
| No SURVEYOR cleavage | Purify and normalize PCR product; re-anneal with TaqB buffer; Redesign sgRNAs; sequence verify Cas9 on px330 backbone |
| Samples do not sink in TBE acrylamide gel | Supplement with MgCl2 to a final concentration of 15mM or add loading buffer containing glycerol |

## Discussion

CRISPR-Cas may be easily multiplexed to facilitate simultaneous modification of several genes and mediate chromosomal microdeletions at high efficiencies. Applicants used two sgRNAs to demonstrate simultaneous targeting of the human *GRIN2B* and *DYRK1A* loci at efficiencies of up to 68% in HEK293FT cells. Likewise, a pair of sgRNAs may be used to mediate microdeletions, such as excision of an exon, which can be genotyped by PCR on a clonal level. Note that the precise location of exon junctions can vary. Applicants also demonstrated the use of ssODNs and targeting vector to mediate HDR with both wildtype and nickase mutant of Cas9 in HEK 293FT and HUES9 cells (Fig. 30). Note that Applicants have not been able to detect HDR in HUES9 cells using the Cas9 nickase, which may be due to low efficiency or a potential difference in repair activities in HUES9 cells. Although these values are typical, there is some variability in the cleavage efficiency of a given sgRNA, and on rare occasions certain sgRNAs may not work for reasons yet unknown. Applicants recommend designing two sgRNAs for each locus, and testing their efficiencies in the intended cell type.

*Example 31: NLSs*

Cas9 Transcriptional Modulator: Applicants set out to turn the Cas9/gRNA CRISPR system into a generalized DNA binding system in which functions beyond DNA cleavage can be executed. For instance, by fusing functional domain(s) onto a catalytically inactive Cas9 Applicants have imparted novel functions, such as transcriptional activation/repression, methylation/demethylation, or chromatin modifications. To accomplish this goal Applicants made a catalytically inactive Cas9 mutant by changing two residues essential for nuclease activity, D10 and H840, to alanine. By mutating these two residues the nuclease activity of Cas9 is abolished while maintaining the ability to bind target DNA. The functional domains Applicants decided to focus on to test Applicants' hypothesis are the transcriptional activator VP64 and the transcriptional repressors SID and KRAB.

Cas9 Nuclear localization: Applicants hypothesized that the most effective Cas9 transcriptional modulator would be strongly localized to the nucleus where it would have its greatest influence on transcription. Moreover, any residual Cas9 in the cytoplasm could have unwanted effects. Applicants determined that wild-type Cas9 does not localize into the nucleus without including multiple nuclear localization signals (NLSs) (although a CRISPR system need not have one or more NLSs but advantageously has at least one or more NLS(s)). Because multiple NLS sequences were required it was reasoned that it is difficult to get Cas9 into the nucleus and any additional domain that is fused to Cas9 could disrupt the nuclear localization. Therefore, Applicants made four Cas9-VP64-GFP fusion constructs with different NLS sequences (pXRP02- pLenti2-EF1a-NLS-hSpCsn1(10A,840A)-NLS-VP64-EGFP, pXRP04- pLenti2-EF1a-NLS-hSpCsn1(10A,840A)-NLS-VP64-2A-EGFP-NLS, pXRP06- pLenti2-EF1a-EGFP-VP64-NLS-hSpCsn1(10A,840A)-NLS, pXRP08- pLenti2-EF1a-NLS-VP64-NLS-hSpCsn1(10A,840A)-NLS-VP64-EGFP-NLS). These constructs were cloned into a lenti backbone under the expression of the human EF1a promoter. The WPRE element was also added for more robust protein expression. Each construct was transfected into HEK 293FT cells using Lipofectame 2000 and imaged 24 hours post-transfection. The best nuclear localization is obtained when the fusion proteins have NLS sequences on both the N- and C-term of the fusion protein. The highest observed nuclear localization occurred in the construct with four NLS elements.

To more robustly understand the influence of NLS elements on Cas9 Applicants made 16 Cas9-GFP fusions by adding the same alpha importin NLS sequence on either the N- or C-term looking at zero to three tandem repeats. Each construct was transfected into HEK 293FT cells using Lipofectame 2000 and imaged 24 hours post-transfection. Notably, the number of NLS elements does not directly correlate with the extent of nuclear localization. Adding an NLS on the C-term has a greater influence on nuclear localization than adding on the N-term.

Cas9 Transcriptional Activator: Applicants functionally tested the Cas9-VP64 protein by targeting the Sox2 locus and quantifying transcriptional activation by RT-qPCR. Eight DNA target sites were chosen to span the promoter of Sox2. Each construct was transfected into HEK 293FT cells using Lipofectame 2000 and 72 hours post-transfection total RNA was extracted from the cells. 1 ug of RNA was reverse transcribed into cDNA (qScript Supermix) in a 40 ul reaction. 2 ul of reaction product was added into a single 20 ul TaqMan assay qPCR reaction. Each experiment was performed in biological and technical triplicates. No RT control and no template control reactions showed no amplification. Constructs that do not show strong nuclear localization, pXRP02 and pXRP04, result in no activation. For the construct that did show strong nuclear localization, pXRP08, moderate activation was observed. Statistically significant activation was observed in the case of guide RNAs Sox2.4 and Sox2.5.

*Example 32: In Vivo Mouse Data*

**Material and reagents**

Herculase II fusion polymerase (Agilent Technologies, cat. no. 600679)
10x NEBuffer 4 (NEB, cat. No. B7004S)
BsaI HF (NEB, cat. No. R3535S)
T7 DNA ligase (Enzymatics, cat. no. L602L)
Fast Digest buffer, 10X (ThermoScientific, cat. No. B64)
FastDigest NotI (ThermoScientific, cat. No. FD0594)
FastAP Alkaline Phosphatase (ThermoScientific, cat. No. EF0651)
Lipofectamine2000 (Life Technologies, cat. No. 11668-019)
Trypsin (Life Technologies, cat. No. 15400054)
Forceps #4 (Sigma, cat. No. Z168777-1EA)
Forceps #5 (Sigma, cat. No. F6521-1EA)
10x Hank's Balanced Salt Solution (Sigma, cat. No. H4641-500ML)
Penicillin/Streptomycin solution (Life Technologies, cat. No. P4333)
Neurobasal (Life Technologies, cat. No. 21103049)
B27 Supplement (Life Technologies, cat. No. 17504044)
L-glutamine (Life Technologies, cat. No. 25030081)
Glutamate (Sigma, cat. No. RES5063G-A7)
$\beta$-mercaptoethanol (Sigma, cat. No. M6250-100ML)
HA rabbit antibody (Cell Signaling, cat. No. 3724S)
LIVE/DEAD® Cell Imaging Kit (Life Technologies, cat. No. R37601)
30G World Precision Instrument syringe (World Precision Instruments, cat. No. NANOFIL)
Stereotaxic apparatus (Kopf Instruments)
UltraMicroPump3 (World Precision Instruments, cat. No. UMP3-4)
Sucrose (Sigma, cat. No. S7903)
Calcium chloride (Sigma, cat. No. C1016)
Magnesium acetate (Sigma, cat. No. M0631)
Tris-HCl (Sigma, cat. no T5941)
EDTA (Sigma, cat. No. E6758)
NP-40 (Sigma, cat. No. NP40)
Phenylmethanesulfonyl fluoride (Sigma, cat. No. 78830)
Magnesium chloride (Sigma, cat. No. M8266)
Potassium chloride (Sigma, cat. No. P9333)
$\beta$-glycerophosphate (Sigma, cat. No. G9422)
Glycerol (Sigma, cat. No. G9012)
Vybrant® DyeCycle™ Ruby Stain (Life technologies, cat. No. S4942)
FACS Aria Flu-act-cell sorter (Koch Institute of MIT, Cambridge US)

DNAeasy Blood & Tissue Kit (Qiagen, cat. No. 69504)

**Procedure**

***Constructing gRNA multiplexes for using in vivo in the brain***

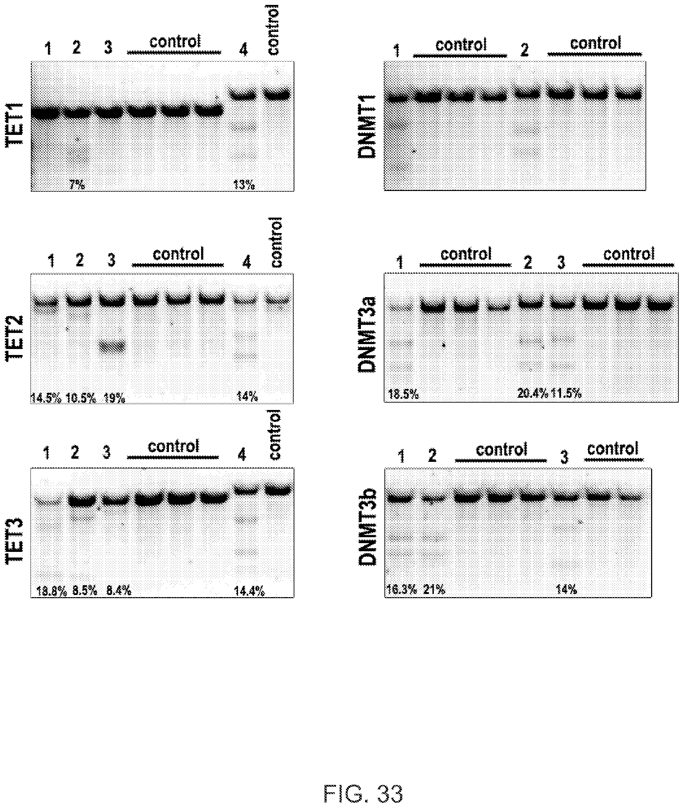

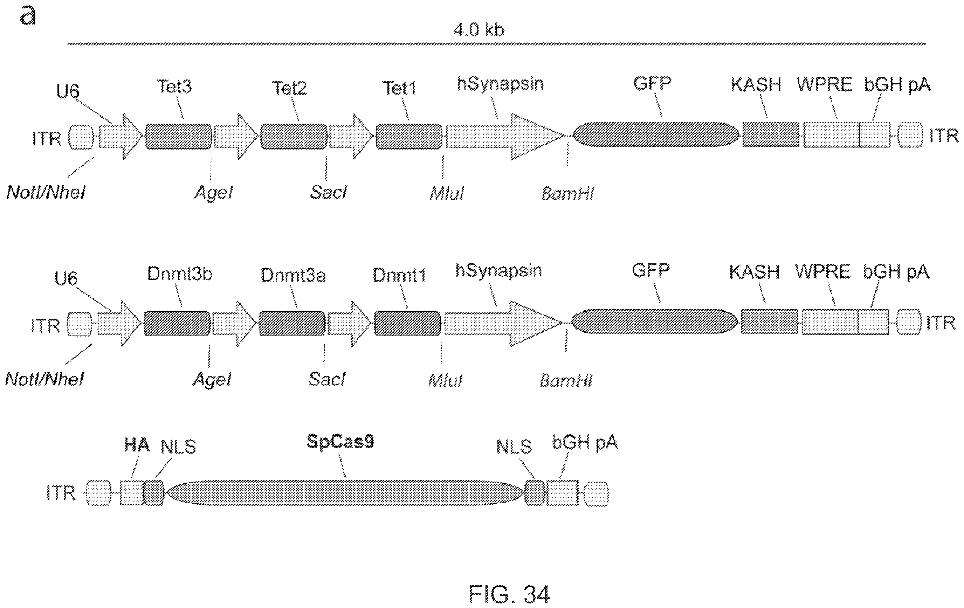

Applicants designed and PCR amplified single gRNAs targeting mouse TET and DNMT family members (as described herein) Targeting efficiency was assessed in N2a cell line (Fig. 33). To obtain simultaneous modification of several genes in vivo, efficient gRNA was multiplexed in AAV-packaging vector (Fig. 34). To facilitate further analysis of system efficiency applicants added to the system expression cassette consistent of GFP-KASH domain fusion protein under control of human Synapsin I promoter (Fig. 34). This modification allows for further analysis of system efficiency in neuronal population (more detail procedure in section ***Sorting nuclei and in vivo results***).

All 4 parts of the system were PCR amplified using Herculase II Fusion polymerase using following primers:

1st U6 Fw: gagggtctcgtccttgcggccgcgctagcgagggcctatttcccatgattc (SEQ ID NO: 196)

1st gRNA Rv: ctcggtctcggtAAAAAAgcaccgactcggtgccacttttcaagttgataacggactagccttattttaacttgctaTTTCtagctctaaaacNNNNNNNNNNNNNNNNNNNNGGTGTTTC GTCCTTTCCAC (SEQ ID NO: 197)

2nd U6 Fw:

gagggtctcTTTaccggtgagggcctatttcccatgattcc (SEQ ID NO: 198)

2nd gRNA Rv: ctcggtctcctcAAAAAAgcaccgactcggtgccacttttcaagttgataacggactagccttattttaacttgctaTTTCtagctctaaaacNNNNNNNNNNNNNNNNNNNNGGTGTTTC GTCCTTTCCAC (SEQ ID NO: 199)

3rd U6 Fw:

gagggtctcTTTgagctcgagggcctatttcccatgattc (SEQ ID NO: 200)

3rd gRNA Rv: ctcggtctcgcgtAAAAAAgcaccgactcggtgccacttttcaagttgataacggactagccttattttaacttgctaTTTCtagctctaaaacNNNNNNNNNNNNNNNNNNNNGGTGTTT CGTCCTTTCCA (SEQ ID NO: 201)

hSyn_GFP-kash Fw: gagggtctcTTacgcgtgtgtctagac (SEQ ID NO: 202)

hSynGFP-kash Rv:

ctcggtctcAaggaCAGGGAAGGGAGCAGTGGTTCACGCCTGTAATCCCAGCAATTTGG GA GGCCAAGGTGGGTAGATCACCTGAGATTAGGAGTTGC  (SEQ ID NO: 203   )

Applicants used Golden Gate strategy to assemble all parts (1:1 molecular ratio) of the system in a single step reaction:

| | |
|---|---|
| 1st U6_gRNA | 18 ng |
| 2nd U6_gRNA | 18 ng |
| 3rd U6_gRNA | 18 ng |
| Syn_GFP-kash | 100 ng |
| 10x NEBuffer 4 | 1.0 μl |
| 10x BSA | 1.0 μl |
| 10 mM ATP | 1.0 μl |
| BsaI HF | 0.75 μl |
| T7 ligase | 0.25 μl |
| ddH$_2$O | 10 μl |
| Cycle number | Condition |
| 1-50 | 37°C for 5 m, 21°C for 5 m |

Golden Gate reaction product was PCR amplified using Herculase II fusion polymerase and following primers:

Fw 5' cctgtccttgcggccgcgctagcgagggcc (SEQ ID NO: 204)

Rv 5' cacgcggccgcaaggacagggaagggagcag (SEQ ID NO: 205)

PCR product was cloned into AAV backbone, between ITR sequences using NotI restriction sites:

PCR product digestion:

| | |
|---|---|
| Fast Digest buffer, 10X | 3 µl |
| FastDigest NotI | 1 µl |
| DNA | 1 µg |
| ddH$_2$O | up to 30 µl |

AAV backbone digestion:

| | |
|---|---|
| Fast Digest buffer, 10X | 3 µl |
| FastDigest NotI | 1 µl |
| FastAP Alkaline Phosphatase | 1 µl |
| AAV backbone | 1 µg |
| ddH$_2$O | up to 30 µl |

After 20 min incubation in 37°C samples were purified using QIAQuick PCR purification kit. Standardized samples were ligated at a 1:3 vector:insert ratio as follows:

| | |
|---|---|
| Digested pUC19 | 50 ng |
| Digested insert | 1:3 vector:insert molar ratio |
| T7 ligase | 1 µl |
| 2X Rapid Ligation Buffer | 5 µl |
| ddH$_2$O | up to 10 ul |

After transformation of bacteria with ligation reaction product, applicants confirmed obtained clones with Sanger sequencing.

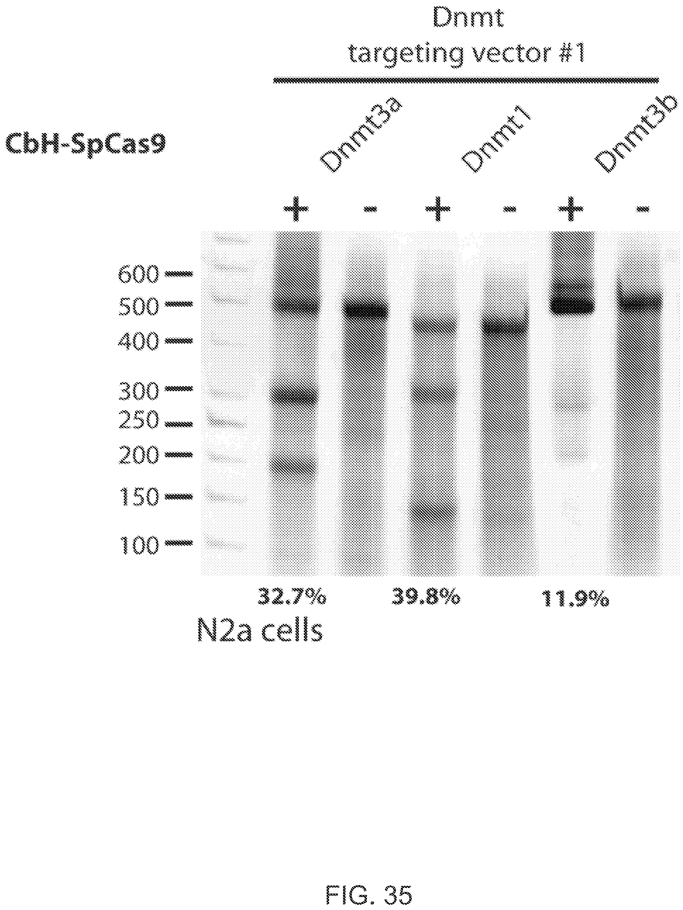

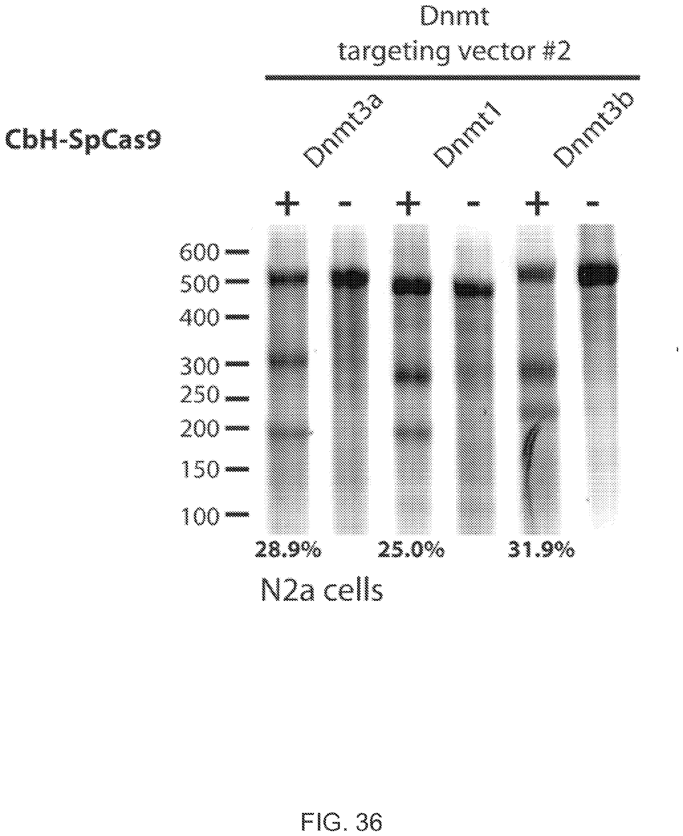

Positive DNA clones were tested in N2a cells after co-transfection with Cas9 construct (Figs. 35 and 36).

***Design of new Cas9 constructs for AAV delivery***

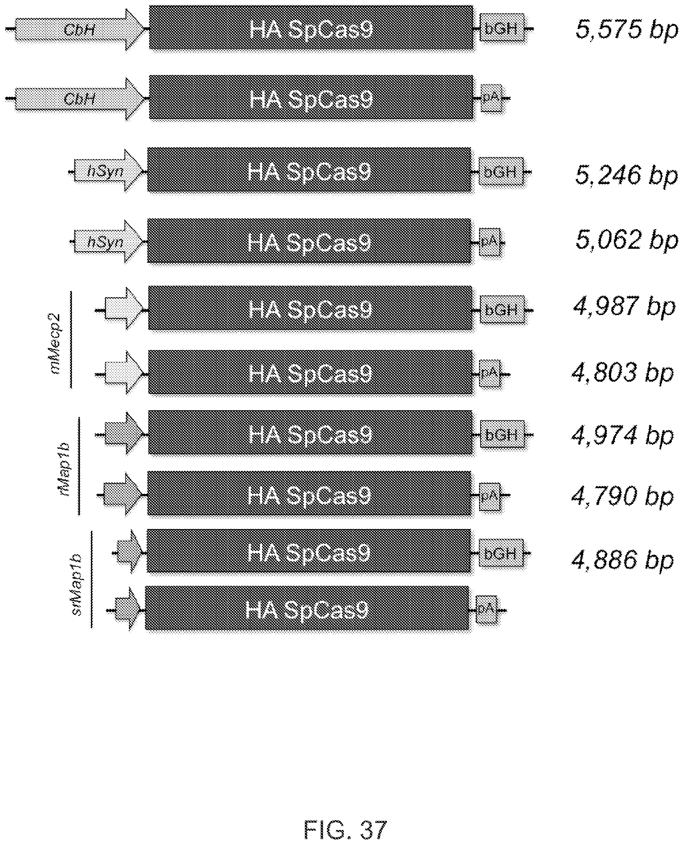

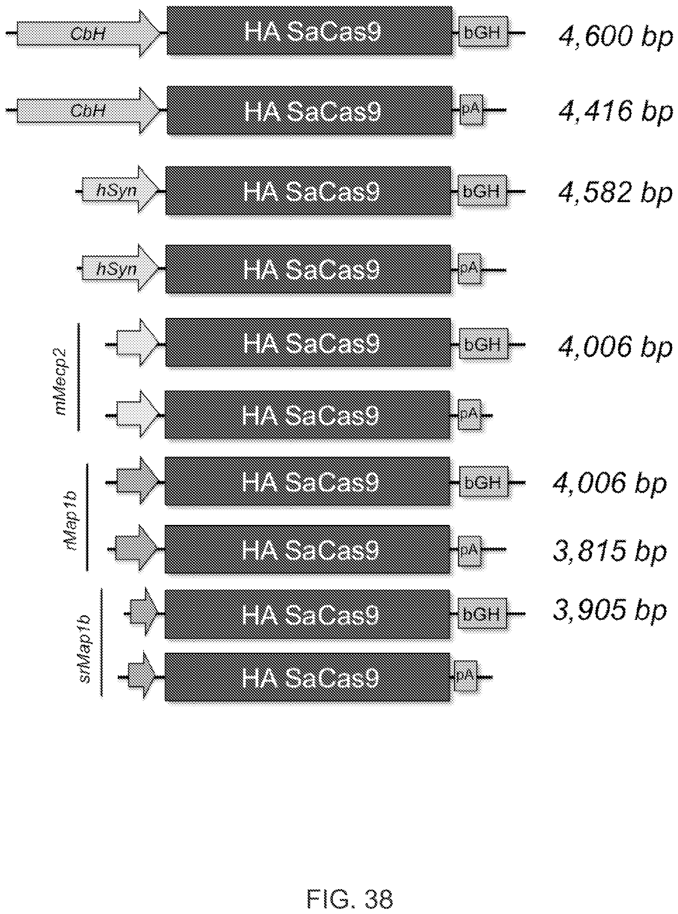

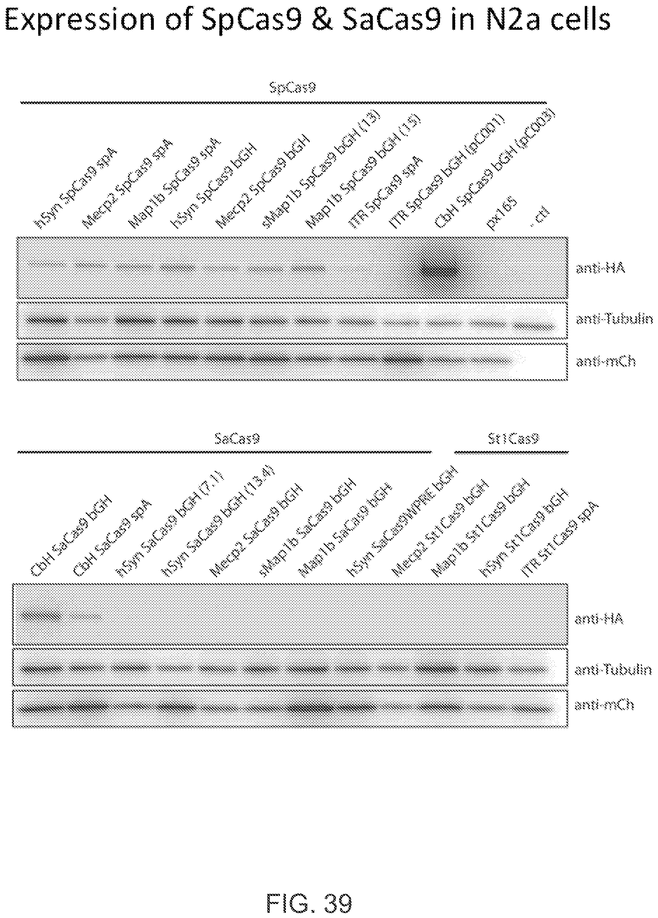

AAV delivery system despite its unique features has packing limitation - to successfully deliver expressing cassette in vivo it has to be in size < then 4.7 kb. To decrease the size of SpCas9 expressing cassette and facilitate delivery applicants tested several alteration: different promoters, shorter polyA signal and finally a smaller version of Cas9 from *Staphylococcus aureus* (SaCas9) (Figs. 37 and 38). All tested promoters were previously tested and published to be active in neurons, including mouse *Mecp2* (Gray et al., 2011), rat *Map1b* and truncated rat *Map1b* (Liu and Fischer, 1996). Alternative synthetic polyA sequence was previously shown to be functional as well (Levitt et al., 1989; Gray et al., 2011). All cloned constructs were expressed in N2a cells after transfection with Lipofectamine 2000, and tested with Western blotting method (Fig. 39).

***Testing AAV multiplex system in primary neurons***

To confirm functionality of developed system in neurons, Applicants use primary neuronal cultures *in vitro.* Mouse cortical neurons was prepared according to the protocol published previously by Banker and Goslin (Banker and Goslin, 1988).

Neuronal cells are obtained from embryonic day 16. Embryos are extracted from the euthanized pregnant female and decapitated, and the heads are placed in ice-cold HBSS. The brains are then extracted from the skulls with forceps (#4 and #5) and transferred to another change of ice-cold HBSS. Further steps are performed with the aid of a stereoscopic microscope in a Petri dish filled with ice-cold HBSS and #5 forceps. The hemispheres are separated from each other and the brainstem and cleared of meninges. The hippocampi are then very carefully dissected and placed in a 15 ml conical tube filled with ice-cold HBSS. Cortices that remain after hippocampal dissection can be used for further cell isolation using an analogous protocol after removing the brain steam residuals and olfactory bulbs. Isolated hippocampi are washed three times with 10 ml ice-cold HBSS and dissociated by 15 min incubation with trypsin in HBSS (4 ml HBSS with the addition of 10 μl 2.5% trypsin per hippocampus) at 37°C. After trypsinization, the hippocampi are very carefully washed three times to remove any traces of trypsin with HBSS preheated to 37°C and dissociated in warm HBSS. Applicants usually dissociate cells obtained from 10-12 embryos in 1 ml HBSS using 1 ml pipette tips and dilute dissociated cells up to 4 ml. Cells are plated at a density of 250 cells/mm2 and cultured at 37°C and 5% CO2 for up to 3 week

**HBSS**

435 ml H2O
50 ml 10x Hank's Balanced Salt Solution
16.5 ml 0.3M HEPES pH 7.3
5 ml penicillin-streptomycin solution
Filter (0.2 μm) and store 4°C

**Neuron Plating Medium** (100 ml)

97 ml Neurobasal
2 ml B27 Supplement
1 ml penicillin-streptomycin solution
250 μl glutamine
125 glutamate

Neurons are transduced with concentrated AAV1/2 virus or AAV1 virus from filtered medium of HEK293FT cells, between 4-7 days in culture and keep for at least one week in culture after transduction to allow for delivered gene expression.

*AAV-driven expression of the system*

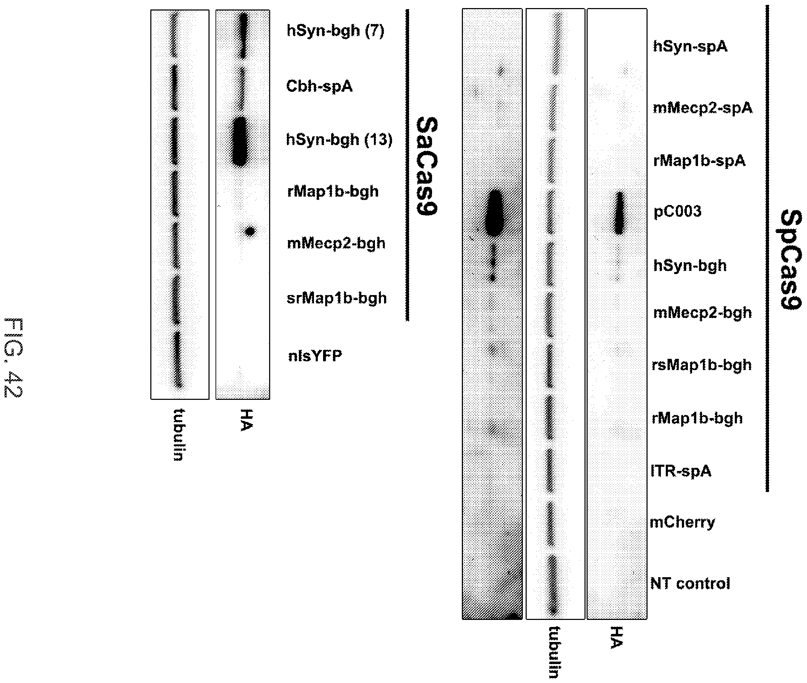

Applicants confirmed expression of SpCas9 and SaCas9 in neuronal cultures after AAV delivery using Western blot method (Fig. 42). One week after transduction neurons were collected in NuPage SDS loading buffer with β-mercaptoethanol to denaturate proteins in 95°C for 5 min. Samples were separated on SDS PAGE gel and transferred on PVDF membrane for WB protein detection. Cas9 proteins were detected with HA antibody.

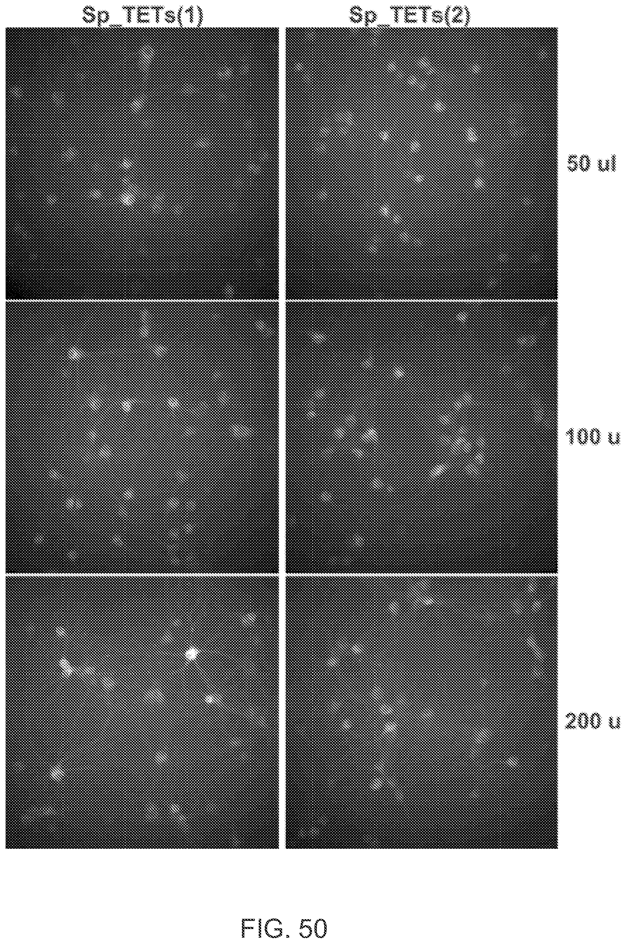

Expression of *Syn*-GFP-kash from gRNA multiplex AAV was confirmed with fluorescent microscopy (Fig. 50).

*Toxicity*

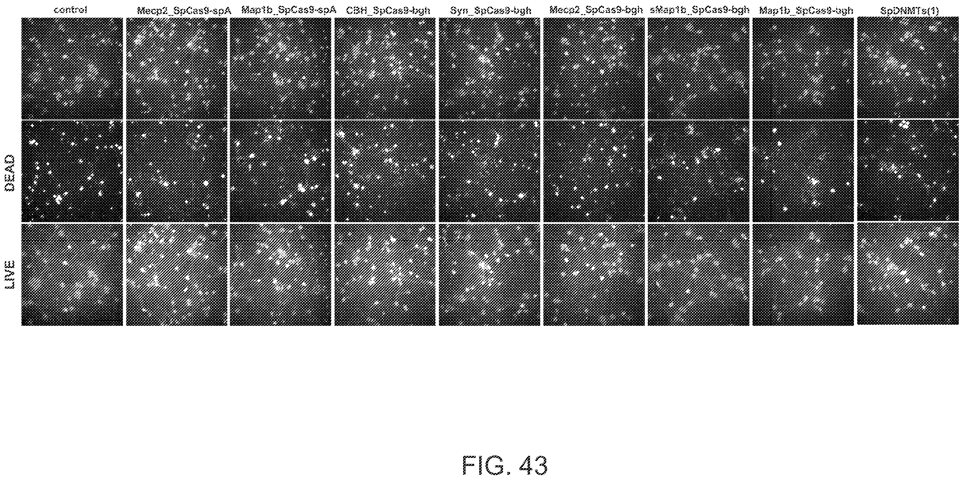

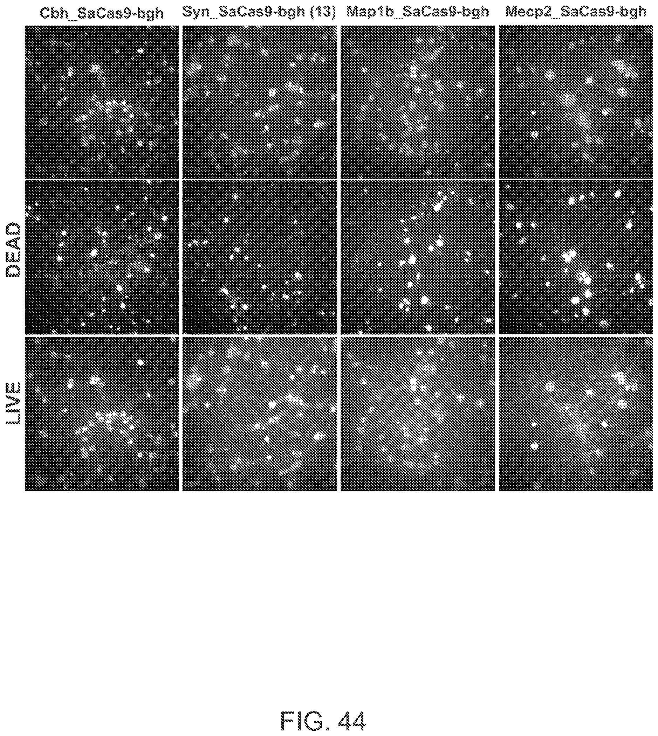

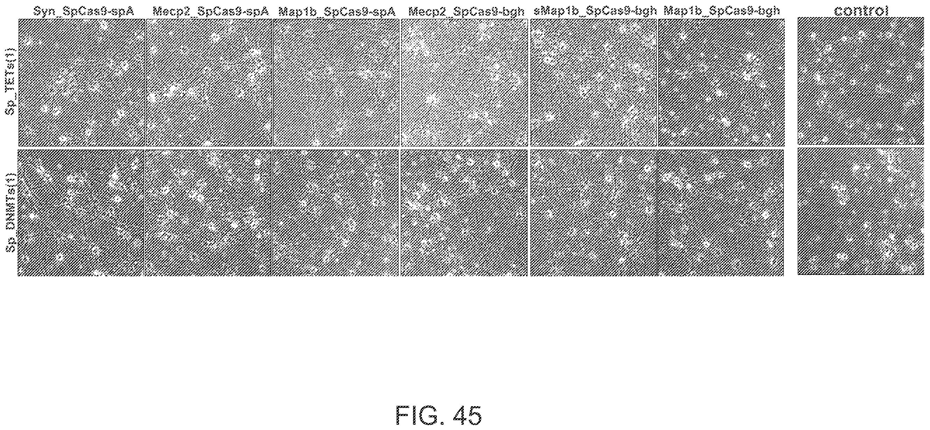

To assess the toxicity of AAV with CRISPR system Applicants tested overall morphology of neurons one week after virus transduction (Fig. 45). Additionally, Applicants tested potential toxicity of designed system with the LIVE/DEAD® Cell Imaging Kit, which allows to distinguish live and dead cells in culture. It is based on the presence of intracellular esterase activity (as determined by the enzymatic conversion of the non-fluorescent calcein AM to the intensely green fluorescent calcein). On the other hand, the red, cell-impermeant component of the Kit enters cells with damaged membranes only and bind to DNA generating fluorescence in dead cells. Both flourophores can be easily visualized in living cells with fluorescent microscopy. AAV-driven expression of Cas9 proteins and multiplex gRNA constructs in the primary cortical neurons was well tolerated and not toxic (Figs. 43 and 44), what indicates that designed AAV system is suitable for in vivo tests.

**Virus production**

Concentrated virus was produced according to the methods described in McClure et al., 2011. Supernatant virus production occurred in HEK293FT cells.

**Brain surgeries**

For viral vector injections 10-15 week old male C57BL/6N mice were anesthetized with a Ketamine/Xylazine cocktail (Ketamine dose of 100 mg/kg and Xylazine dose of 10 mg/kg) by intraperitoneal injection. Intraperitonial administration of Buprenex was used as a pre-emptive analgesic (1 mg/kg). Animals were immobilized in a Kopf stereotaxic apparatus using intra-aural positioning studs and tooth bar to maintain an immobile skull. Using a hand-held drill, a hole (1-2mm) at -3.0 mm posterior to Bregma and 3.5 mm lateral for injection in the CA1 region of the hippocampus was made. Using 30G World Precision Instrument syringe at a depth of 2.5 mm, the solution of AAV viral particles in a total volume of 1 ul was injected. The injection was monitored by a 'World Precision Instruments UltraMicroPump3' injection pump at a flow rate of 0.5 ul/min to prevent tissue damage. When the injection was complete, the injection needle was removed slowly, at a rate of 0.5 mm/min. After injection, the skin was sealed with 6-0 Ethilon sutures. Animals were postoperatively hydrated with 1 mL lactated Ringer's (subcutaneous) and housed in a temperature controlled (37°C) environment until achieving an ambulatory recovery. 3 weeks after surgery animals were euthanized by deep anesthesia followed by tissue removal for nuclei sorting or with 4% paraformaldehyde perfusion for immunochemistry.

### Sorting nuclei and in vivo results

Applicants designed a method to specifically genetically tag the gRNA targeted neuronal cell nuclei with GFP for Fluorescent Activated Cell Sorting (FACS) of the labeled cell nuclei and downstream processing of DNA, RNA and nuclear proteins. To that purpose the applicants' multiplex targeting vector was designed to express both a fusion protein between GFP and the mouse nuclear membrane protein domain KASH (Starr DA, 2011, Current biology) and the 3 gRNAs to target specific gene loci of interest (Fig. 34). GFP-KASH was expressed under the control of the human Synapsin promoter to specifically label neurons. The amino acid of the fusion protein GFP-KASH was:

MVSKGEELFTGVVPILVELDGDVNGHKFSVSGEGEGDATYGKLTLKFICT

TGKLPVPWPTLVTTLTYGVQCFSRYPDHMKQHDFFKSAMPEGYVQERTIFFKDDGN

YKTRAEVKFEGDTLVNRIELKGIDFKEDGNILGHKLEYNYNSHNVYIMADKQKNGIK

VNFKIRHNIEDGSVQLADHYQQNTPIGDGPVLLPDNHYLSTQSALSKDPNEKRDHMV

LLEFVTAAGITLGMDELYKSGLRSREEEEETDSRMPHLDSPGSSQPRRSFLSRVIRAAL

PLQLLLLLLLLLLACLLPASEDDYSCTQANNFARSFYPMLRYTNGPPPT  (SEQ ID NO: 206 )

One week after AAV1/2 mediated delivery into the brain a robust expression of GFP-KASH was observed. For FACS and downstream processing of labeled nuclei, the hippocampi were dissected 3 weeks after surgery and processed for cell nuclei purification using a gradient centrifugation step. For that purpose the tissue was homogenized in 320 mM Sucrose, 5 mM CaCl, 3 mM Mg(Ac)2, 10 mM Tris pH 7.8, 0.1 mM EDTA, 0.1% NP40, 0.1 mM Phenylmethanesulfonyl fluoride (PMSF), 1 mM β-mercaptoethanol using 2ml Dounce homogenizer (Sigma) The homogenisate was centrifuged on a 25% to 29% Optiprep® gradient according to the manufacture's protocol for 30 min at 3.500 rpm at 4 °C. The nuclear pellet was resuspended in 340 mM Sucrose, 2 mM MgCl2, 25 mM KCl, 65 mM glycerophosphate, 5% glycerol, 0.1 mM PMSF, 1 mM β-mercaptoethanol and Vybrant® DyeCycle™ Ruby Stain (Life technologies) was added to label cell nuclei (offers near-infrared emission for DNA). The labeled and purified nuclei were sorted by FACS using an Aria Flu-act-cell sorter and BDFACS Diva software. The sorted GFP+ and GFP- nuclei were finally used to purify genomic DNA using DNAeasy Blood & Tissue Kit (Qiagen) for Surveyor assay analysis of the targeted genomic regions. The same approach can be easily used to purify nuclear RNA or protein from targeted cells for downstream processing. Due to the 2-vector system (Fig. 34) the applicants using in this approach efficient Cas9 mediated DNA cleavage was expected to occur only in a small subset of cells in the brain (cells which were co-infected with both the multiplex targeting vector and the Cas9 encoding vector). The method described here enables the applicants to specifically purify DNA, RNA and nuclear proteins from the cell population expressing the 3 gRNAs of interest and therefore are supposed to undergo Cas9 mediated DNA cleavage. By using this method the applicants were able to visualize efficient DNA cleavage *in vivo* occurring only in a small subset of cells.

Essentially, what Applicants have shown here is targeted *in vivo* cleavage. Furthermore, Applicants used a multiple approach, with several different sequences targeted at the same time, but independently. Presented system can be applied for studying brain pathologic conditions (gene knock out, e.g. Parkinson disease) and also open a field for further development of genome editing tools in the brain. By replacing nuclease activity with gene transcription regulators or epigenetic regulators it will be possible to answer whole spectrum of scientific question about role of gene regulation and epigenetic changes in the brain in not only in the pathologic conditions but also in physiological process as learning and memory formation. Finally, presented technology can be applied in more complex mammalian system as primates, what allows to overcome current technology limitations.

### Example 33: Model Data

Several disease models have been specifically investigated. These include *de novo* autism risk genes CHD8, KATNAL2, and SCN2A; and the syndromic autism (Angelman Syndrome) gene UBE3A. These genes and resulting autism models are of course preferred, but show that the invention may be applied to any gene and therefore any model is possible.

Applicants have made these cells lines using Cas9 nuclease in human embryonic stem cells (hESCs). The lines were created by transient transfection of hESCs with Cbh-Cas9-2A-EGFP and pU6-sgRNA. Two sgRNAs are designed for each gene targeting most often the same exons in which patient nonsense (knock-out) mutations have been recently described from whole exome sequencing studies of autistic patients. The Cas9-2A-EGFP and pU6 plasmids were created specifically for this project.

*Example 34: AAV production system or protocol*

An AAV production system or protocol that was developed for, and works particularly well with, high through put screening uses is provided herein, but it has broader applicability in the present invention as well. Manipulating endogenous gene expression presents various challenges, as the rate of expression depends on many factors, including regulatory elements, mRNA processing, and transcript stability. To overcome this challenge, Applicants developed an adeno-associated virus (AAV)-based vector for the delivery. AAV has an ssDNA-based genome and is therefore less susceptible to recombination.

AAV1/2 (serotype AAV1/2, i.e., hybrid or mosaic AAV1 / AAV2 capsid AAV) heparin purified concentrated virus protocol

**Media: D10 + HEPES**

500ml bottle DMEM high glucose + Glutamax (GIBCO)
50ml Hyclone FBS (heat-inactivated) (Thermo Fischer)
5.5ml HEPES solution (1M, GIBCO)
Cells: low passage HEK293FT (passage <10 at time of virus production, thaw new cells of passage
2-4 for virus production, grow up for 3-5 passages)

**Transfection reagent: Polyethylenimine (PEI) "Max"**

Dissolve 50mg PEI "Max" in 50ml sterile Ultrapure H20
Adjust pH to 7.1
Filter with 0.22um fliptop filter
Seal tube and wrap with parafilm
Freeze aliquots at -20°C (for storage, can also be used immediately)

**Cell Culture**

Culture low passage HEK293FT in D10 + HEPES
Passage everyday between 1:2 and 1:2.5
Advantageously do not allow cells to reach more than 85% confluency

For T75

- Warm 10ml HBSS (-Mg2+, -Ca2+, GIBCO) + 1ml TrypLE Express (GIBCO) per flask to 37°C (Waterbath)

Aspirate media fully

- Add 10ml warm HBSS gently (to wash out media completely)
- Add 1ml TrypLE per Flask
- Place flask in incubator (37°C) for 1min
- Rock flask to detach cells
- Add 9ml D10 + HEPES media (37°C)
- Pipette up and down 5 times to generate single cell suspension
- Split at 1:2 - 1:2.5 (12ml media for T75) ratio (if cells are growing more slowly, discard and thaw a new batch, they are not in optimal growth)

- transfer to T225 as soon as enough cells are present (for ease of handling large amounts of cells)

**AAV production (5*15cm dish scale per construct):**

Plate 10 million cells in 21.5 ml media into a 15cm dish
Incubate for 18-22 hours at 37°C
Transfection is ideal at 80% confluence

**Per plate**

Prewarm 22ml media (D10 + HEPES)

Prepare tube with DNA mixture (use endofree maxiprep DNA):

5.2 ug vector of interest plasmid
4.35 ug AAV 1 serotype plasmid
4.35 ug AAV 2 serotype plasmid
10.4 ug pDF6 plasmid (adenovirus helper genes) □ Vortex to mix
Add 434 uL DMEM (no serum!)
Add 130 ul PEI solution
Vortex 5-10 seconds
Add DNA/DMEM/PEI mixture to prewarmed media

Vortex briefly to mix
Replace media in 15cm dish with DNA/DMEM/PEI mixture
Return to 37°C incubator
Incubate 48h before harvesting (make sure medium isn't turning too acidic)

**Virus harvest:**

1. aspirate media carefully from 15cm dish dishes (advantageously do not dislodge cells)
2. Add 25 ml RT DPBS (Invitrogen) to each plate and gently remove cells with a cell scraper. Collect suspension in 50 ml tubes.
3. Pellet cells at 800x g for 10 minutes.
4. Discard supernatant

**pause point: freeze cell pellet at -80C if desired**

5. resuspend pellet in 150 mM NaCl, 20 mM Tris pH 8.0, use 10 ml per tissue culture plate.
6. Prepare a fresh solution of 10% sodium deoxycholate in dH2O. Add 1.25 ml of this per tissue culture plate for a final concentration of 0.5%. Add benzonase nuclease to a final concentration of 50 units per ml. Mix tube thoroughly.
7. Incubate at 37°C for 1 hour (Waterbath).
8. Remove cellular debris by centrifuging at 3000 x g for 15 mins. Transfer to fresh 50 ml tube and ensure all cell debris has been removed to prevent blocking of heparin columns.

**Heparin column purification of AAV1/2:**

1. Set up HiTrap heparin columns using a peristaltic pump so that solutions flow through the column at 1 ml per minute. It is important to ensure no air bubbles are introduced into the heparin column.
2. Equilibrate the column with 10 ml 150 mM NaCl, 20 mM Tris, pH 8.0 using the peristaltic pump.
3. Binding of virus: Apply 50 ml virus solution to column and allow to flow through.
4. Wash step 1: column with 20 ml 100 mM NaCl, 20 mM Tris, pH 8.0. (using the peristaltic pump)

5. Wash step 2: Using a 3 ml or 5 ml syringe continue to wash the column with 1 ml 200 mM NaCl, 20 mM Tris, pH 8.0, followed by 1 ml 300 mM NaCl, 20 mM Tris, pH 8.0.

Discard the flow-through.

(prepare the syringes with different buffers during the 50min flow through of virus solution above)

6. Elution Using 5 ml syringes and gentle pressure (flow rate of < 1ml/min) elute the virus from the column by applying:

1.5 ml 400 mM NaCl, 20 mM Tris, pH 8.0
3.0 ml 450 mM NaCl, 20 mM Tris, pH 8.0
1.5 ml 500 mM NaCl, 20 mM Tris, pH 8.0
Collect these in a 15 ml centrifuge tube.

**Concentration of AAV1/2:**

1. Concentration step 1: Concentrate the eluted virus using Amicon ultra 15ml centrifugal filter units with a 100,000 molecular weight cutoff. Load column eluate into the concentrator and centrifuge at 2000x g for 2 minutes (at room temperature. Check concentrated volume - it should be approximately 500 $\mu$l. If necessary, centrifuge in 1min intervals until correct volume is reached.
2. buffer exchange: Add 1ml sterile DPBS to filter unit, centrifuge in 1min intervals until correct volume (500ul) is reached.
3. Concentration step 2: Add 500ul concentrate to an Amicon Ultra 0.5ml 100K filter unit. Centrifuge at 6000g for 2min. Check concentrated volume - it should be approximately 100 $\mu$l. If necessary, centrifuge in 1min intervals until correct volume is reached.
4. Recovery: Invert filter insert and insert into fresh collection tube. Centrifuge at 1000g for 2min.

Aliquot and freeze at -80°C

1ul is typically required per injection site, small aliquots (e.g. 5ul) are therefore recommended (avoid freeze-thaw of virus).

determine DNaseI-resistant GC particle titer using qPCR (see separate protocol)

**Materials**

Amicon Ultra, 0.5ml, 100K; MILLIPORE; UFC510024
Amicon Ultra, 15ml, 100K; MILLIPORE; UFC910024
Benzonase nuclease; Sigma-Aldrich, E1014
HiTrap Heparin cartridge; Sigma-Aldrich; 54836
Sodium deoxycholate; Sigma-Aldrich; D5670

AAV1 supernatant production protocol

Media: D10 + HEPES
500ml bottle DMEM high glucose + Glutamax (Invitrogen)
50ml Hyclone FBS (heat-inactivated) (Thermo Fischer)
5.5ml HEPES solution (1M, GIBCO)
Cells: low passage HEK293FT (passage <10 at time of virus production)
Thaw new cells of passage 2-4 for virus production, grow up for 2-5 passages
Transfection reagent: Polyethylenimine (PEI) "Max"
Dissolve 50mg PEI "Max" in 50ml sterile Ultrapure H20
Adjust pH to 7.1
Filter with 0.22um fliptop filter
Seal tube and wrap with parafilm
Freeze aliquots at -20°C (for storage, can also be used immediately)

Cell Culture

Culture low passage HEK293FT in D10 + HEPES Passage everyday between 1:2 and 1:2.5 Advantageously do let cells reach more than 85% confluency

For T75

- Warm 10ml HBSS (-Mg2+, -Ca2+, GIBCO) + 1ml TrypLE Express (GIBCO) per flask to 37°C (Waterbath)
- Aspirate media fully
- Add 10ml warm HBSS gently (to wash out media completely)
- Add 1ml TrypLE per Flask
- Place flask in incubator (37°C) for 1min
- Rock flask to detach cells
- Add 9ml D10 + HEPES media (37°C)
- Pipette up and down 5 times to generate single cell suspension
- Split at 1:2 - 1:2.5 (12ml media for T75) ratio (if cells are growing more slowly, discard and thaw a new batch, they are not in optimal growth)
- transfer to T225 as soon as enough cells are present (for ease of handling large amounts of cells) AAV production (single 15cm dish scale)

Plate 10 million cells in 21.5 ml media into a 15cm dish
Incubate for 18-22 hours at 37°C
Transfection is ideal at 80% confluence per plate
Prewarm 22ml media (D10 + HEPES)
Prepare tube with DNA mixture (use endofree maxiprep DNA):
5.2 ug vector of interest plasmid
8.7 ug AAV 1 serotype plasmid
10.4 ug DF6 plasmid (adenovirus helper genes)
Vortex to mix
Add 434 uL DMEM (no serum!) Add 130 ul PEI solution
Vortex 5-10 seconds
Add DNA/DMEM/PEI mixture to prewarmed media
Vortex briefly to mix
Replace media in 15cm dish with DNA/DMEM/PEI mixture
Return to 37°C incubator
Incubate 48h before harvesting (advantageously monitor to ensure medium is not turning too acidic)

Virus harvest:

Remove supernatant from 15cm dish
Filter with 0.45 um filter (low protein binding) Aliquot and freeze at -80°C
Transduction (primary neuron cultures in 24-well format, 5DIV)
Replace complete neurobasal media in each well of neurons to be transduced with fresh neurobasal
(usually 400ul out of 500ul per well is replaced)
Thaw AAV supernatant in 37°C waterbath
Let equilibrate in incubator for 30min
Add 250 ul AAV supernatant to each well
Incubate 24h at 37°C
Remove media/supernatant and replace with fresh complete neurobasal
Expression starts to be visible after 48h, saturates around 6-7 Days Post Infection
Constructs for pAAV plasmid with GOI should not exceed 4.8kb including both ITRS.
Example of a human codon optimized sequence (i.e. being optimized for expression in humans) sequence :
SaCas9 is provided below:

ACCGGTGCCACCATGTACCCATACGATGTTCCAGATTACGC

TTCGCCGAAGAAAAAGCGCAAGGTCGAAGCGTCCATGAAAAGGAACTACATTCT

GGGGCTGGACATCGGGATTACAAGCGTGGGGTATGGGATTATTGACTATGAAAC

AAGGGACGTGATCGACGCAGGCGTCAGACTGTTCAAGGAGGCCAACGTGGAAAA

CAATGAGGGACGGAGAAGCAAGAGGGGAGCCAGGCGCCTGAAACGACGGAGAA

GGCACAGAATCCAGAGGGTGAAGAAACTGCTGTTCGATTACAACCTGCTGACCG

ACCATTCTGAGCTGAGTGGAATTAATCCTTATGAAGCCAGGGTGAAAGGCCTGA

GTCAGAAGCTGTCAGAGGAAGAGTTTTCCGCAGCTCTGCTGCACCTGGCTAAGC

GCCGAGGAGTGCATAACGTCAATGAGGTGGAAGAGGACACCGGCAACGAGCTG

TCTACAAAGGAACAGATCTCACGCAATAGCAAAGCTCTGGAAGAGAAGTATGTC

GCAGAGCTGCAGCTGGAACGGCTGAAGAAAGATGGCGAGGTGAGAGGGTCAATT

AATAGGTTCAAGACAAGCGACTACGTCAAAGAAGCCAAGCAGCTGCTGAAAGTG

CAGAAGGCTTACCACCAGCTGGATCAGAGCTTCATCGATACTTATATCGACCTGC

TGGAGACTCGGAGAACCTACTATGAGGGACCAGGAGAAGGGAGCCCCTTCGGAT

GGAAAGACATCAAGGAATGGTACGAGATGCTGATGGGACATTGCACCTATTTTCC

AGAAGAGCTGAGAAGCGTCAAGTACGCTTATAACGCAGATCTGTACAACGCCCT

GAATGACCTGAACAACCTGGTCATCACCAGGGATGAAAACGAGAAACTGGAATA

CTATGAGAAGTTCCAGATCATCGAAAACGTGTTTAAGCAGAAGAAAAAGCCTAC

ACTGAAACAGATTGCTAAGGAGATCCTGGTCAACGAAGAGGACATCAAGGGCTA

CCGGGTGACAAGCACTGGAAAACCAGAGTTCACCAATCTGAAAGTGTATCACGA

TATTAAGGACATCACAGCACGGAAAGAAATCATTGAGAACGCCGAACTGCTGGA

TCAGATTGCTAAGATCCTGACTATCTACCAGAGCTCCGAGGACATCCAGGAAGAG

CTGACTAACCTGAACAGCGAGCTGACCCAGGAAGAGATCGAACAGATTAGTAAT

CTGAAGGGGTACACCGGAACACACAACCTGTCCCTGAAAGCTATCAATCTGATTC

TGGATGAGCTGTGGCATACAAACGACAATCAGATTGCAATCTTTAACCGGCTGAA

GCTGGTCCCAAAAAAGGTGGACCTGAGTCAGCAGAAAGAGATCCCAACCACACT

GGTGGACGATTTCATTCTGTCACCCGTGGTCAAGCGGAGCTTCATCCAGAGCATCAA

AGTGATCAACGCCATCATCAAGAAGTACGGCCTGCCCAATGATATCATTATCGAGCT

GGCTAGGGAGAAGAACAGCAAGGACGCACAGAAGATGATCAATGAGATGCAGAAA

CGAAACCGGCAGACCAATGAACGCATTGAAGAGATTATCCGAACTACCGGGAAAGA

GAACGCAAAGTACCTGATTGAAAAAATCAAGCTGCACGATATGCAGGAGGGAAAGT

GTCTGTATTCTCTGGAGGCCATCCCCCTGGAGGACCTGCTGAACAATCCATTCAACTAC

GAGGTCGATCATATTATCCCCAGAAGCGTGTCCTTCGACAATTCCTTTAACAACAAGGT

GCTGGTCAAGCAGGAAGAGAACTCTAAAAAGGGCAATAGGACTCCTTTCCAGTACCTG

TCTAGTTCAGATTCCAAGATCTCTTACGAAACCTTTAAAAAGCACATTCTGAATCTGGC

CAAAGGAAAGGGCCGCATCAGCAAGACCAAAAAGGAGTACCTGCTGGAAGAGCGGGA

CATCAACAGATTCTCCGTCCAGAAGGATTTTATTAACCGGAATCTGGTGGACACAAGAT

ACGCTACTCGCGGCCTGATGAATCTGCTGCGATCCTATTTCCGGGTGAACAATCTGGAT

GTGAAAGTCAAGTCCATCAACGGCGGGTTCACATCTTTTCTGAGGCGCAAATGGAAGTT

TAAAAAGGAGCGCAACAAAGGGTACAAGCACCATGCCGAAGATGCTCTGATTATCGCA

AATGCCGACTTCATCTTTAAGGAGTGGAAAAAGCTGGACAAAGCCAAGAAAGTGATGG

AGAACCAGATGTTCGAAGAGAAGCAGGCCGAATCTATGCCCGAAATCGAGACAGAAC

AGGAGTACAAGGAGATTTTCATCACTCCTCACCAGATCAAGCATATCAAGGATTTCAAG

GACTACAAGTACTCTCACCGGGTGGATAAAAAGCCCAACAGAGAGCTGATCAATGACA

CCCTGTATAGTACAAGAAAGACGATAAGGGGAATACCCTGATTGTGAACAATCTGAA

CGGACTGTACGACAAAGATAATGACAAGCTGAAAAAGCTGATCAACAAAGTCCCGAG

AAGCTGCTGATGTACCACCATGATCCTCAGACATATCAGAAACTGAAGCTGATTATGGA

GCAGTACGGCGACGAGAAGAACCCACTGTATAAGTACTATGAAGAGACTGGGAACTAC

CTGACCAAGTATAGCAAAAAGGATAATGGCCCCGTGATCAAGAAGATCAAGTACTATG

GGAACAAGCTGAATGCCCATCTGGACATCACAGACGATTACCCTAACAGTCGCAACAA

GGTGGTCAAGCTGTCACTGAAGCCATACAGATTCGATGTCTATCTGGACAACGGCGTGT

ATAAATTTGTGACTGTCAAGAATCTGGATGTCATCAAAAAGGAGAACTACTATGAAGT

GAATAGCAAGTGCTACGAAGAGGCTAAAAAGCTGAAAAAGATTAGCAACCAGGCAGA

GTTCATCGCCTCCTTTTACAACAACGACCTGATTAAGATCAATGGCGAACTGTATAGGG

TCATCGGGGTGAACAATGATCTGCTGAACCGCATTGAAGTGAATATGATTGACATCACT

TACCGAGAGTATCTGGAAAACATGAATGATAAGCGCCCCCCTCGAATTATCAAAACAA

TTGCCTCTAAGACTCAGAGTATCAAAAAGTACTCAACCGACATTCTGGGAAACCTGTAT

GAGGTGAAGAGCAAAAAGCACCCTCAGATTATCAAAAAGGGCTAAGAATTC (SEQ ID NO: 207 )

*Example 35: Minimizing off-target cleavage using Cas9 nickase and two guide RNAs*

Cas9 is a RNA-guided DNA nuclease that may be targeted to specific locations in the genome with the help of a 20bp RNA guide. However the guide sequence may tolerate some mismatches between the guide sequence and the DNA-target sequence. The flexibility is undesirable due to the potential for off-target cleavage, when the guide RNA targets Cas9 to a an off-target sequence that has a few bases different from the guide sequence. For all experimental applications (gene targeting, crop engineering, therapeutic applications, etc) it is important to be able to improve the specificity of Cas9 mediated gene targeting and reduce the likelihood of off-target modification by Cas9.

Applicants developed a method of using a Cas9 nickase mutant in combination with two guide RNAs to facilitate targeted double strand breaks in the genome without off-target modifications. The Cas9 nickase mutant may be generated from a Cas9 nuclease by disabling its cleavage activity so that instead of both strands of the DNA duplex being cleaved only one strand is cleaved. The Cas9 nickase may be generated by inducing mutations in one ore more domains of the Cas9 nuclease, e.g. Ruvc1 or HNH. These mutations may include but are not limited to mutations in a Cas9 catalytic domain, e.g in SpCas9 these mutations may be at positions D10 or H840. These mutations may include but are not limited to D10A, E762A, H840A, N854A, N863A or D986A in SpCas9 but nickases may be generated by inducing mutations at corresponding positions in other CRISPR enzymes or Cas9 orthologs. In a most preferred embodiment of the invention the Cas9 nickase mutant is a SpCas9 nickase with a D10A mutation.

The way this works is that each guide RNA in combination with Cas9 nickase would induce the targeted single strand break of a duplex DNA target. Since each guide RNA nicks one strand, the net result is a double strand break. The reason this method eliminates off-target mutations is because it is very unlikely to have an off-target site that has high degrees of similarity for both guide sequences (20bp+2bp(PAM) = 22bp specificity for each guide, and two guides means any off-target site will have to have close to 44bp of homologous sequence). Although it is still likely that individual guides may have off-targets, but those off-targets will only be nicked, which is unlikely to be repaired by the mutagenic NHEJ process. Therefore the multiplexing of DNA double strand nicking provides a powerful way of introducing targeted DNA double strand breaks without off-target mutagenic effects.

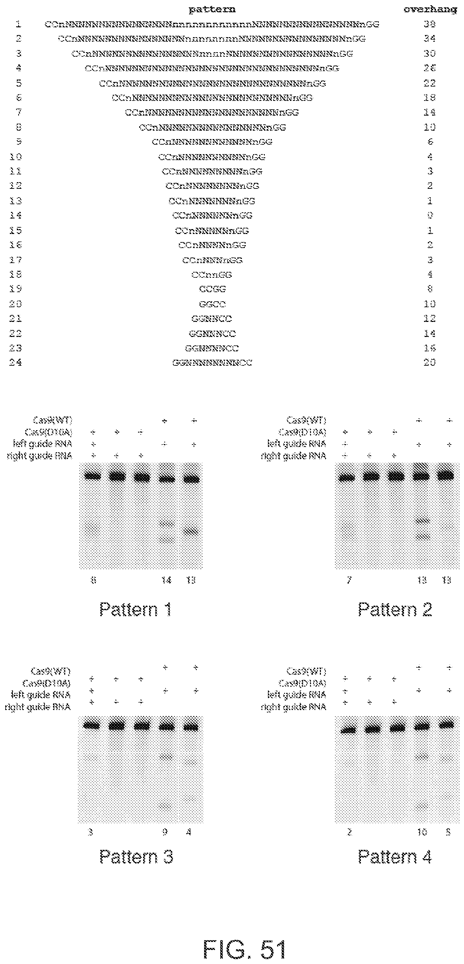

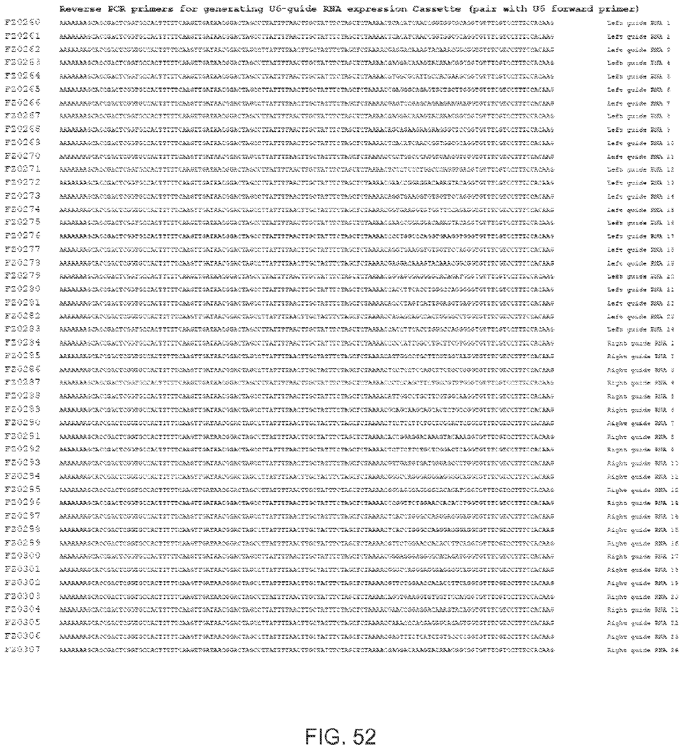

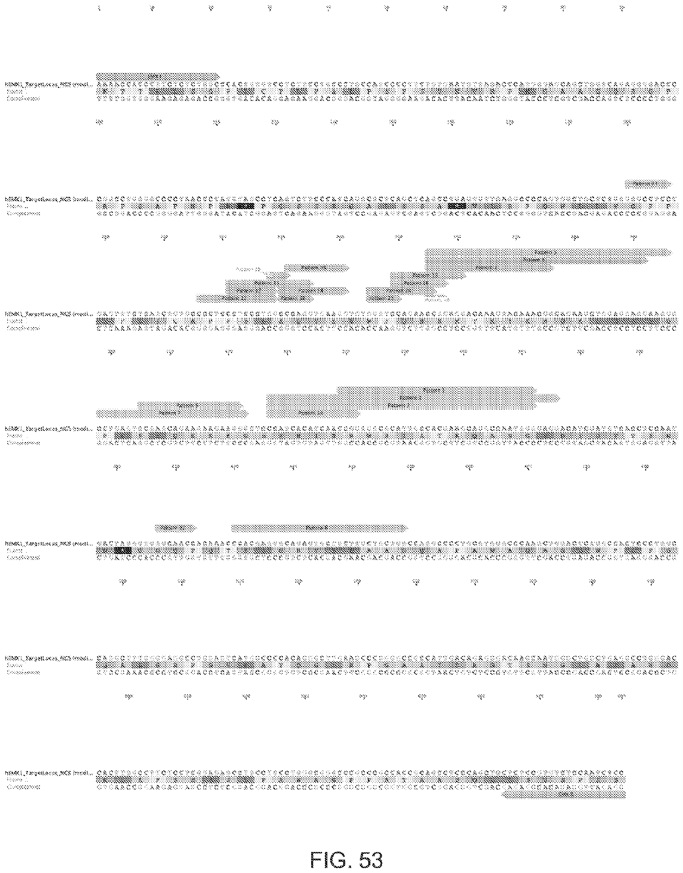

Applicants carried out experiments involving the co-transfection of HEK293FT cells with a plasmid encoding Cas9(D10A) nickase as well as DNA expression cassettes for one or more guides. Applicants transfected cells using Lipofectamine 2000, and transfected cells were harvested 48 or 72 hours after transfections. Double nicking-induced NHEJ were detected using the SURVEYOR nuclease assay as described previously herein (Figs. 51, 52 and 53).

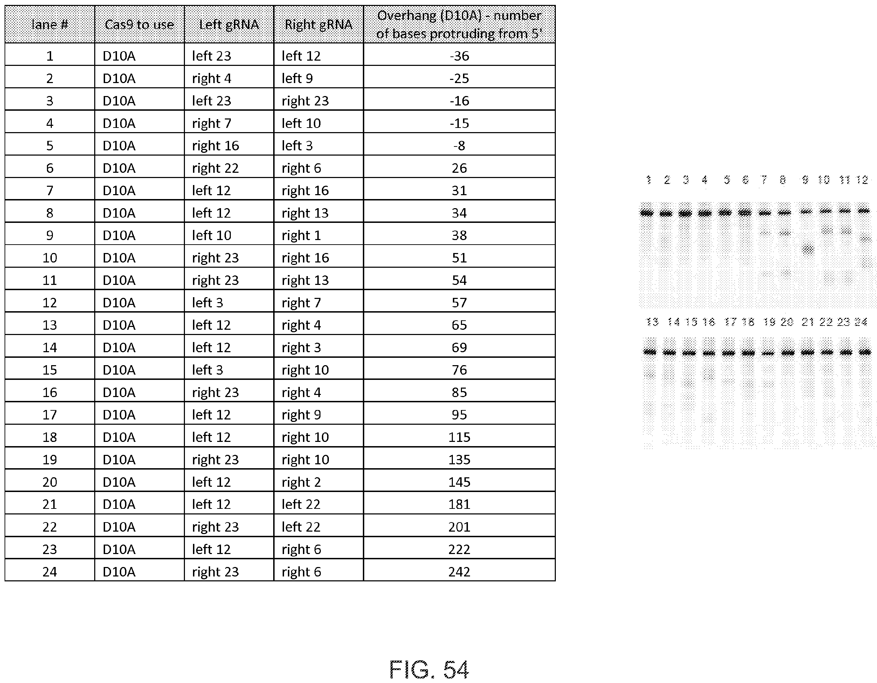

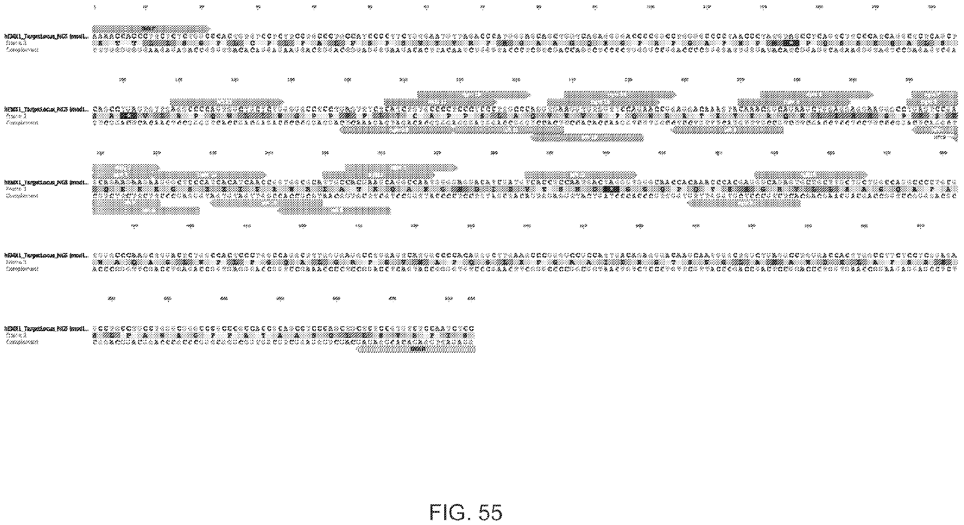

Applicants have further identified parameters that relate to efficient cleavage by the Cas9 nickase mutant when combined with two guide RNAs and these parameters include but are not limited to the length of the 5' overhang. Efficient cleavage is reported for 5' overhang of at least 26 base pairs. In a preferred embodiment of the invention, the 5' overhang is at least 30 base pairs and more preferably at least 34 base pairs. Overhangs of up to 200 base pairs may be acceptable for cleavage, while 5' overhangs less than 100 base pairs are preferred and 5' overhangs less than 50 base pairs are most preferred (Figs. 54 and 55).

*Example 36: ApoB genotypic and phenotypic change seen in vivo with guides and SaCas9 delivered intravenously to the Liver using an AAV vector and a Liver-specific Cas9 promoter*

In this example, inter alia:

- AAV2/8 is a Liver-targeting adenoviral vector;
- TBG is a liver-specific promoter and is used here to drive expression of SaCas9;
- U6 is used here to drive expression of the sgRNA (guide);
- ApoB is a lipid metabolism gene. It can be said to be the "gold-standard" in liver delivery, and is widely used in mouse models of obesity
- "Target1 through Target 4" means that 4 targets within ApoB were chosen, of which Targets 1 and three (T1 and T3) were the most useful;
- Delivery through expression from a viral vector as seen here is an improvement over Anderson/Yin's (NBT 2884) use of hydrodynamic delivery as the delivery method, because hydrodynamic delivery requires several mls of fluid to be injected which is stressful on the murine body and can be fatal. Hydrodynamic delivery is well suited for delivery of plasmid (naked) DNA, whereas Applicants have shown that packaging the guide and Cas9 sequences within a viral delivery vector is preferable in terms of greatly increased efficiency. Indeed, only relatively small volumes need to be introduced, and this can be done intravenously (i.v.), which is likely to be much more acceptable therapeutically.
- What was particularly encouraging was that not only was a genotypic change seen in a "gold-standard" gene for liver such as ApoB, but **phenotypic** changes were also recorded. Previous work with PCSK9 had shown genotypic, but not phenotypic changes, so the phenotypic changes seen with ApoB validate the plausibility of CRISPR delivery to, and its ability to effect phenotypic change in, the Liver. This is in combination with the more therapeutically acceptable means of delivery (i.v. compared to hydrodynamic delivery). As such, viral delivery of CRISPR (guide and Cas9) is preferred, especially intravenously).
- Targets include: *PCSK9, HMGCR, APOB, LDLR, ANGPTL3, F8, F9/FIX, AAT, FAH, HPD, TAT, ATP7B, UGT1A1, OTC, ARH*

## Material and Methods

### *Viruses and Injection Parameters*

Constructs used: -AAV2/8 - TBG-SaCas9-U6-sgRNA (Apob-Target1 through Target 4).

*In vitro* testing: all induced cleavage of Apob locus at 10%-15% efficiency in Hepa cells.

*In vivo* results: Mouse - 8 weeks, C57BL/6 (2 animals each time point and with 1 animal as saline-injected wild type control)

### *Tail Vein Injection:*

*Injection Volume:* 100ul of 0.8E12 vp/ml (vp = viral particle)
*Viral particle delivered:* 0.8E11 total vp/animal

### **Tissue Processing and Data Collection**

*Tissue processing and data collection occurred as follows:*

First time point ~ 1 wk (8 days). Second time point ~ 4 wks.

Saline perfusion followed by acute dissection of liver tissue.

(A) Half liver put into -80C storage for Surveyor & qPCR & Western Blot protein analysis (X12 tubes / animal).
(B) Half liver put into Cryoprotectant and flash-freeze for cryostat processing. Cryosections were subjected to H&E and Oil Red staining.

QuickExtract and Surveyor assays were used to detect and quantify indels from 2 pieces of liver per animal.

### **Results**

*In vivo Indel Assessment*

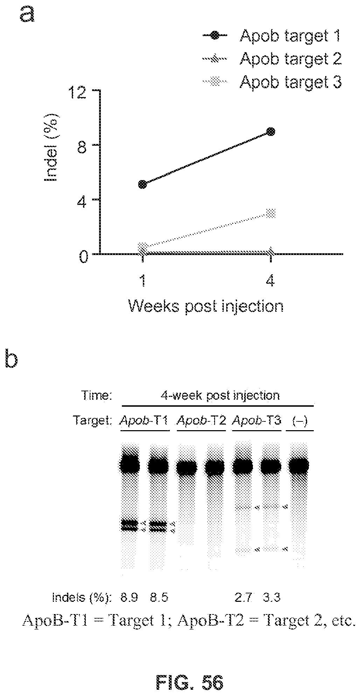

The figures show *in vivo* indel assessment for the ApoB guide (targets) over time (up to 4 weeks post-injection). Figure 56 A shows that guide (target) 1 induced the highest percentage of indels in ApoB. Targets 2 and 4 showed little of no effect, in eh sense that they resulted in only none or very poor indel formation, whilst Target 3 showed some activity. Figure 56 B shows the results of a Surveyor nuclease gel assay for indel formation efficiency, 4 weeks post-injection.

Target 1 can be seen to have almost 9% indel formation, representing significant levels of target locus

*Phenotype Change shown with 2 of the 4 guides designed to target*

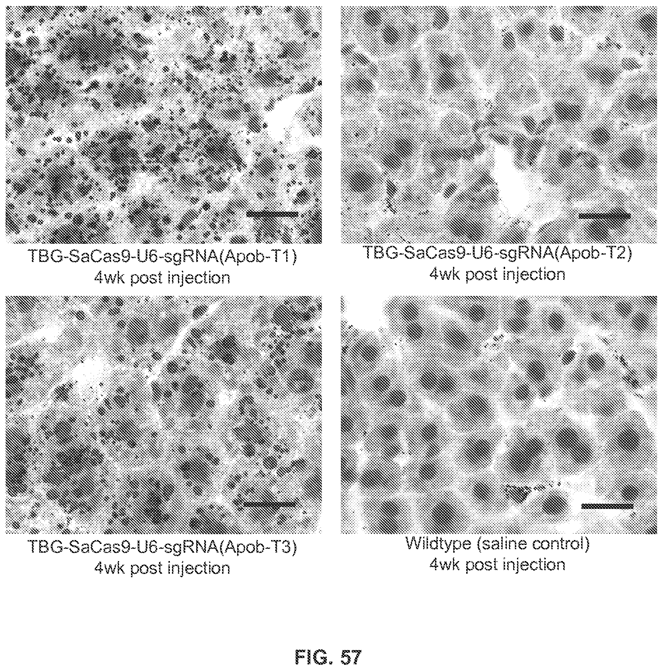

Phenotypic changes were seen with two of the three guides used (targets 1 and 3), as seen in Figure 57B, which shows oil red staining to detect hepatic lipid accumulation phenotype in vivo following AAV-Cas9-sgRNA delivery. The red patches of oil shown accumulating in the 2 Figures on the left, targets 1 and 3, show that ApoB has been disrupted and are compared to the control, bottom right. Apob gene has been disrupted as a result of Cas9-induced targeted genomic cleavage, giving rise to this physiological/phenotypic change Target 2 showed no noticeable difference over the control and target 4 is not shown. This oil red O staining is an assay where the fats in liver are visualized through histological staining. This stain is used frequently in research to assess the amount of fats in liver. In clinical practice, the Oil Red O stain is mainly ordered on frozen sections of liver biopsy specimens to assess the amount of fat in the liver during liver transplantation and other procedures. For a protocol and information on this aspect of the Examples, mention is made of: Mehlem et al, "Imaging of neutral lipids by oil red O for analyzing the metabolic status in health and disease," Nature Protocols 8, 1149-1154(2013); Maczuga et al., "Therapeutic expression of hairpins targeting apolipoprotein B100 induces phenotypic and transcriptome changes in murine liver," Gene Therapy (2014) 21, 60-70; Koornneef et al, "Apolipoprotein B Knockdown by AAV-delivered shRNA Lowers Plasma Cholesterol in Mice," Molecular Therapy (2011) 19 4, 731-740; Tadin-Strapps et al., "siRNA-induced liver ApoB knockdown lowers serum LDL-cholesterol in a mouse model with human-like serum lipids," Journal of Lipid Research Volume 52, 1084-1097 (2011). The scale bar in the figure represents 20 microns.

*Example 37: SaCas9 Optimization Experiments*

The following were investigated: Guide Length Optimization; Intron Test; H1 promoter; D10A Double-nickase Test; Additional Length/DN Test.

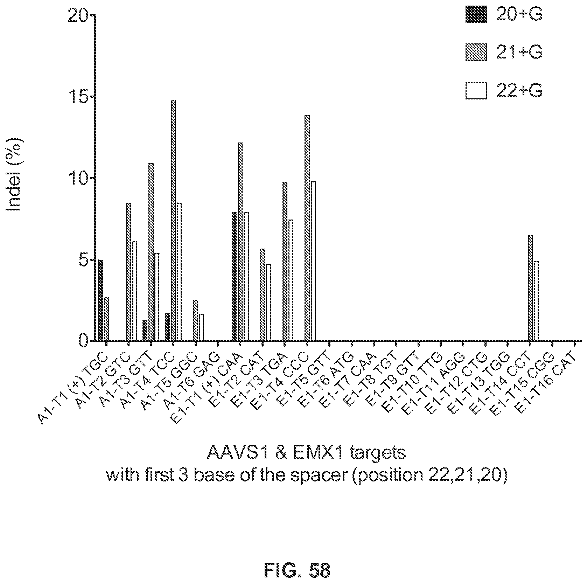

*SaCas9 Guide Length Test:* To determine sgRNA guide lengths: 20 vs. 21 vs. 22 bp as well the effect of a 'G' at the start (5' end) of the guide. Mention is made of Figure 58. In this experiment

*Target sites:*

A1: AAVS1
E1: EMX1
T1, T2, ...: Numbering of target sites
TGC, GTC, ...: Base composition at position 23, 22, 21 nts from 5'-end of PAM

The experiment of this Example is performed by: 1. Select targets using NNGRR as PAM within two gene of interest, AAVS1 and EMX1. 2. Synthesizing oligos corresponding to the targets, but vary the length of the guide sequence part within the sgRNA from 20, to 21, to 22. 3. Use the oligos to create sgRNA expression cassette and co-transfect into HEK 293FT cell line with plasmids expressing the SaCas9 protein. 4. 72 hours post transfection, cells were harvested and then analyzed by Surveyor assay to detect indels. 5. Indel formation frequency induced by Cas9 were then calculated and summarized in the figures herewith.

Figure 58 shows that 21 nts/base pairs (bp), represented by the grey bars is the optimal spacer length, at least compared to 20 or 22 base pairs (represented by the black and the white bars, respectively) across a range of targets and within two different genes (AAVS1 and EMX1). The targets and genes are not thought be important, merely representative. As such, it appears that 21nts or base pairs is optimal for good length, especially in or as to SaCas9. Figure 58 also shows that a G nt at the 5' end of the guide / target sequence is may be advantageous, e.g., for the U6 promoter. The optimal guide length may be specific to each Cas9 protein. For example, for SpCas9, the optimal guide length might be 19-20nt. Hence, this example demonstrates how one can determine and use optimal guide length.

*Intron Test*

This experiment set out to test whether a guide sequence could be inserted into the Cas9 intronic sequence.
The following construct was used. Note the presence of the guide RNA (sgRNA) within the intron (between the Cas9 N' and C' terminal exons).
CMV-SaCas9(N-term)-Intron(sgRNA)-SaCas9(C-term)
The construct was expressed in Hepa cells.
Each intron was tested with2 different guides: *Pcsk9* and *Hmgcr* sgRNA.
A total of 9 constructs shown: three EBV1 three EBV2 and three ADV:

- Lanes 1-3: show EBV1-152 (EBV based, 152bp intron 1 from EBV genome)
- Lanes 4-6: show EBV2 (EBV based, intron from the W repeat of EBV genome)
- Lanes 7-9: show ADV (Adenoviral based intron, similar origin as Kiani et al., "CRISPR transcriptional repression devices and layered circuits in mammalian cells," Nature Methods doi:10.1038/nmeth.2969 Published online 5 May 2014 and Nissim et al, "Multiplexed and Programmable Regulation of Gene Networks with an Integrated RNA and CRISPR/Cas Toolkit in Human Cells," Volume 54, Issue 4, p698-710, 22 May 2014; DOI: http://dx.doi.org/10.1016/j.molcel.2014.04.022).

Within each group of design, the three constructs corresponding to three different insertion site of sgRNA within the intron.

ADV-design 3

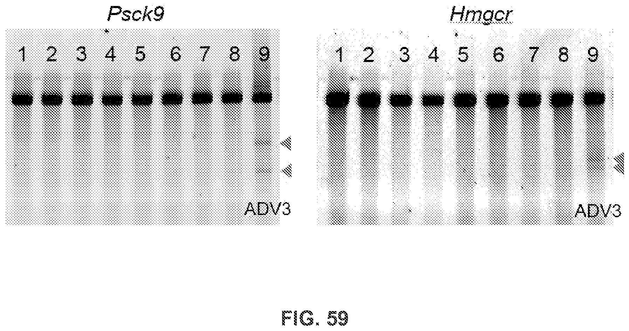

The results are shown in Figure 59. These results provide proof of principle of successful packaging of a guide sequence into a SaCas9 intron is certainly possible. The sgRNA bearing the guide sequence is inserted within a synthetic intron derived from Adenovirus, and then this entire intron-sgRNA cassette is inserted into the SaCas9 gene. Introns can be inserted anywhere within the SaCas9 gene without significantly disrupting the normal expression of the SaCas9 protein. Multiple introns with sgRNAs can be inserted into different positions within the SaCas9 gene Positioning is flexible and this broad approach is advantageous including in the following two ways:
Size minimisation allows for the total number of bp or nts in the construct to be reduced.
Multiplexing allows for greater degrees of multiplexing (co-delivery of multiple guides) as 'space' is always an issue here too. As guides don't necessarily need a specific promoter, one or more guides can similarly be packaged into a/the Cas9 intron.
The foregoing text uses 'a/the' because the as discussed above, a number of synthetic introns can be introduced into Cas9. It may be advantageous to insert the sgRNA into a position close but at least 5-15bp to the 5' end of the intron and also before the branch point of the intron. Some of the intron spacer sequence between the 5' splice donor site and the branch point in the middle of the intron may be deleted if the skilled person wishes to so do. That this was achieved in a Cas9, especially SaCas9 may be surprising, including because the sgRNA structure is different between Sa and Sp.

For now, ADV are preferred, but this approach has broad applicability across a range of viruses and Cas9s (Sa, Sp, etc).

### H1 promoter tests

This experiment set out to investigate alternative promoters to the U6 promoter.

#### A) full-length H1

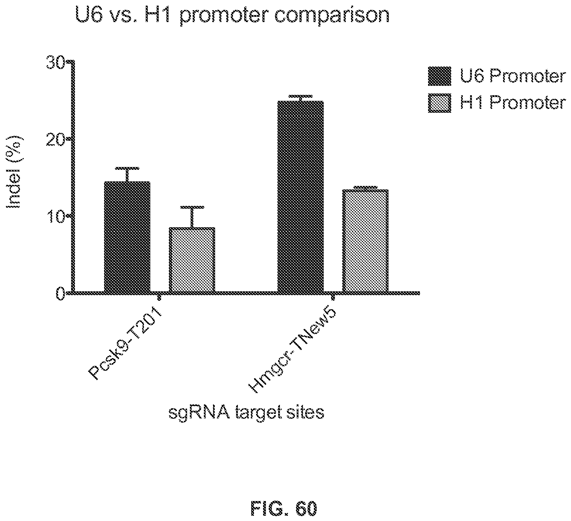

The following constructs were made:
CMV-SaCas9 with original H1 promoter driving one sgRNA (either Pcsk9-Target201 or Hmgcr-NewTarget5)
As can be seen in Figure 60, the full-length H1 promoter (grey bar) is still weaker than U6 promoter (black bar), as the U6 shows increased indel percentage formation for each target tested.

#### B) Double H1 promoter test (short H1)

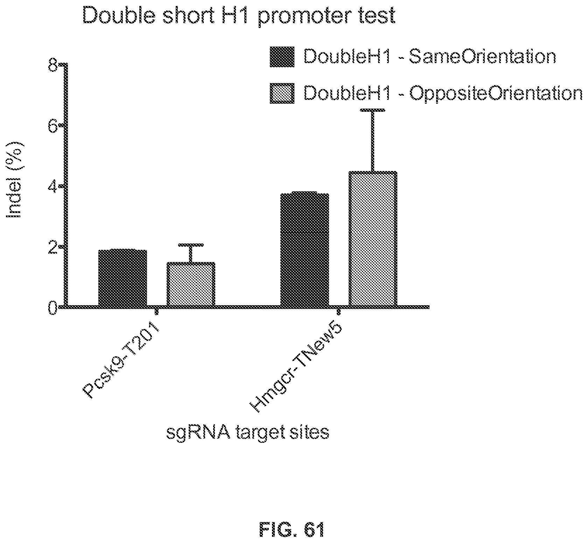

The following constructs were made:
TBG-SaCas9 with two short H1 promoters driving two sgRNAs (Pcsk9-Target201 and Hmgcr-NewTarget5) simultaneously with the Double short H1 promoter used in the same orientation and in opposite orientations.
As can be seen in Figure 61, short H1 promoter is weaker than the full-length H1.

### SaCas9 nickase test (using the D10A mutant)

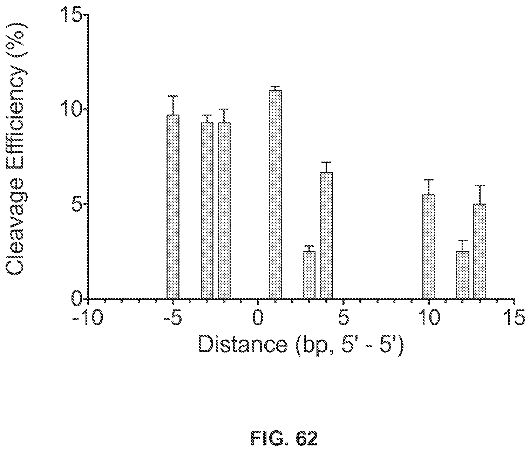

This experiment looked at the distance between the 5' ends of two guide sequences in a construct and then measured this in relation to the cleavage efficiency of the D10A SaCAs9 double nickase. The targets were for the Human AAT1 gene. These tests were done with 20bp+G guides cloned into plasmids.
Optimal results were shown between -5 and +1 bp (5' to 5'), see Figure 62.

*Example 38 : In vivo interrogation of gene function in the mammalian brain using CRISPR-Cas9*

This work presents the following main points: First demonstration of successful AAV-mediated Cas9 delivery in vivo as well as efficient genome modification in post-mitotic neurons. Development of a nuclear tagging technique which enables easy isolation of neuronal nuclei from Cas9 and sgRNA-expressing cells. Demonstration of application toward RNAseq analysis of neuronal transcriptome. Integration of electrophysiological studies with Cas9-mediated genome perturbation. And demonstration of multiplex targeting and the ability to study gene function on rodent behavior using Cas9-mediated genome editing.

Transgenic animal models carrying disease-associated mutations are enormously useful for the study of neurological disorders, helping to elucidate the genetic and pathophysiological mechanism of disease[1]. However, generation of animal models that carry single or multiple genetic modifications is particularly labor intensive and requires time-consuming breeding over many generations. Therefore, to facilitate the rapid dissection of gene function in normal and disease-related brain processes we need ability to precisely and efficiently manipulate the genome of neurons *in vivo.* The CRISPR-associated endonuclease Cas9 from *Streptococcus pyogenes* (SpCas9) has been shown to mediate precise and efficient genome cleavage of single and multiple genes in replicating eukaryotic cells, resulting in frame shifting insertion/deletion (indel) mutations[2, 3]. Here, we integrate Cas9-mediated genome perturbation with biochemical, sequencing, electrophysiological, and behavioral readouts to study the function of individual as wells as groups of genes in neural processes and their roles in brain disorders *in vivo.*

#### Discussion

Adeno-associated viral (AAV) vectors are commonly used to deliver recombinant genes into the mouse brain[4]. The main limitation of the AAV system is its small packaging size, capped at approximately 4.5 kb without ITRs[5], which limits the amount of genetic material that can be packaged into a single vector. Since the size of the SpCas9[6] is already 4.2 kb, leaving less than 0.3 kb for other genetic elements within a single AAV vector, we designed a dual-vector system that packages SpCas9 (AAV-SpCas9) and sgRNA expression cassettes (AAV-SpGuide) on two separate viral vectors (Fig. 67a). While designing the AAV-SpCas9 vector, we compared various short neuron-specific promoters as well as poly adenylation signals to optimize SpCas9 expression. For our final design we chose the mouse *Mecp2* promoter (235 bp, pMecp2)[7] and a minimal polyadenylation signal (48 bp, spA)[8] based on their ability to achieve sufficient levels of SpCas9 expression in cultured primary mouse cortical neurons (Figure 67-c). To facilitate immunofluorescence identification of SpCas9-expressing neurons, we tagged SpCas9 with a HA-epitope tag. For the AAV-SpGuide vector, we packaged an U6-sgRNA expression cassette as well as the green fluorescent protein (GFP)-fused with the KASH nuclear trans-membrane domain[9] driven by the human Synapsin I promoter (Figure 63a). The GFP-KASH fusion protein directs GFP to the outer nuclear membrane (Figure 67c,d) and enables fluorescence-based identification and purification of intact neuronal nuclei transduced by AAV-SpGuide.

To test the delivery efficacy of our dual-vector delivery system, we first transduced cultured primary mouse cortical neurons *in vitro* and observed robust expression by AAV-SpCas9 and AAV-SpGuide (Figure 67e), with greater than 80% co-transduction efficiency (Figure 67e). Importantly, compared with un-transduced neurons, expression of SpCas9 did not adversely affect the morphology and survival rate of transduced neurons (Figure 67c,f).

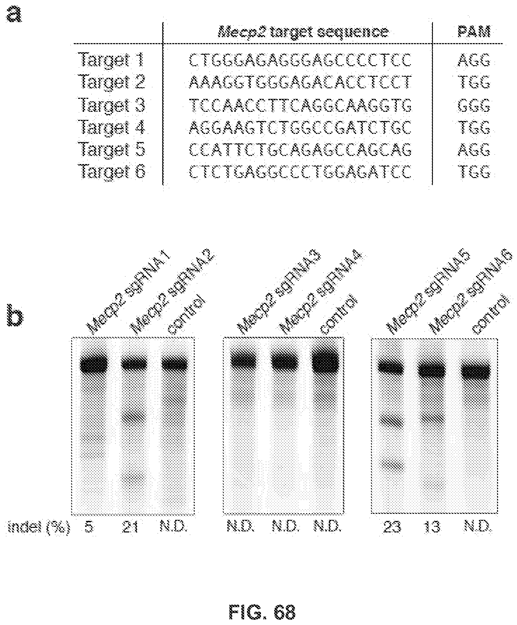

Having established an efficient delivery system, we next sought to test SpCas9-mediated genome editing in mouse primary neurons. Whereas SpCas9 has been used to achieve efficient genome modifications in a variety of dividing cell types, it is unclear whether SpCas9 can be used to efficiently achieve genome editing in post-mitotic neurons. For our initial test we targeted the *Mecp2* gene, which plays a principal role in Rett syndrome[10], a type of autism spectrum disorder. MeCP2 protein is ubiquitously expressed in neurons throughout the brain but nearly absent in glial cells[11, 12] and its deficiency has been shown to be associated with severe morphological and electrophysiological phenotypes in neurons, and both are believed to contribute to the neurological symptoms observed in patients with Rett syndrome[13-16]. To target *Mecp2,* we first designed several sgRNAs targeting exon 3 of the mouse *Mecp2* gene (Figure 68a) and evaluated their efficacy using Neuro-2a cells. The most efficient sgRNA was identified using the SURVEYOR nuclease assay (Figure 68b). We chose the most effective sgRNA (*Mecp2* target 5) for subsequent *in vitro* and *in vivo Mecp2* targeting experiments.

To assess the editing efficiency of our dual-vector system in neurons, we transduced primary mouse cortical neurons at 7 days *in vitro* (7 DIV, Figure 69a) and measured indel rate using the SURVEYOR nuclease assay 7 days post transduction (Figure 69b). Of note, neuron culture co-transduced with AAV-SpCas9 and AAV-SpGuide targeting *Mecp2* showed up to 80% reduction in MeCP2 protein levels compared to control neurons (Figure 69c,d). One possible explanation for the observed discrepancy between relatively low indel frequency (~14%) and robust protein depletion (~80%) could be that mere binding by SpCas9 at the target site may interfere with transcription, which has been shown in *E. coli*[17,18]. We investigated this possibility using a mutant of SpCas9 with both RuvC and HNH catalytic domains inactivated[19,20] (D10A and H840A, dSpCas9). Co-expression of dSpCas9 and Mecp2-targeting sgRNA did not reduce MeCP2 protein levels (Figure 69a,d), suggesting that the observed decrease of MeCP2 level in presence of active SpCas9 is due to occurrence of modification in the *Mecp2* locus. Another possible explanation for the discrepancy between the low level of detected indel and high level of protein depletion may be due to underestimation of the true indel rate by the SURVEYOR nuclease assay - the detection accuracy of SURVEYOR has been previously shown to be sensitive to the indel sequence composition[21]

MeCP2 loss-of-function has been previously shown to be associated with dendritic tree abnormalities and spine morphogenesis defects in neurons[14, 16]. These phenotypes of MeCP2 deprivation have also been reproduced in neurons differentiated from MeCP-KO iPS cells[15]. Therefore, we investigated whether SpCas9-mediated MeCP2-depletion in neurons can similarly recapitulate morphological phenotypes of Rett syndrome. Indeed, neurons co-expressing SpCas9 and *Mecp2*-targeting sgRNA exhibited altered dendritic tree morphology and spine density when compared with control neurons (Figure 70). These results demonstrate that SpCas9 can be used to facilitate the study of gene functions in cellular assays by enabling targeted knockout in post-mitotic neurons.

Given the complexity of the nervous system, which consists of intricate networks of heterogeneous cell types, being able to efficiently edit the genome of neurons *in vivo* would enable direct testing of gene function in relevant cell types embedded in native contexts. Consequently, we stereotactically injected a mixture (1:1 ratio) of high titer AAV-SpCas9 and AAV-SpGuide into the hippocampal dentate gyrus in adult mice. We observed high co-transduction efficiency of both vectors (over 80%) in hippocampal granule cells at 4 weeks after viral injection (Figure 63b,c) resulting in genomic modifications of the *Mecp2* locus. (Figure 63d). Using SURVEYOR nuclease assay we detected ~13% indel frequency in brain punches obtained from injected brain regions (Figure 63e). Similar to our finding in cultured primary neurons, SpCas9-mediated cutting of the *Mecp2* locus efficiently decreased MeCP2 protein levels by over 60%. Additionally the number of MeCP2-positive nuclei in the dentate gyrus decreased by over 75% when injected with AAV-SpCas9 and AAV-SpGuide compared to AAV-SpCas9 alone (Figure 63g-h). These results suggest that SpCas9 can be used to directly perturb specific genes within intact biological contexts.

Targeted genomic perturbations can be coupled with quantitative readouts to provide insights into the biological function of specific genomic elements. To facilitate analysis of AAV-SpCas9 and AAV-SpGuide transduced cells, we developed a method to purify GFP-KASH labeled nuclei using fluorescent activated cell sorting (FACS) (Figure 64a). Sorted nuclei can be directly used to purify nuclear DNA and RNA for downstream biochemical or sequencing analysis.

Using sanger sequencing, we found that 13 out of 14 single GFP-positive nuclei contained an indel mutation at the sgRNA target site.

In addition to genomic DNA sequencing, purified GFP-positive nuclei can also be used for RNAseq analysis to study transcriptional consequences of MeCP2 depletion (Figure 64b and Figure 71). To test the effect of *Mecp2* knockout on transcription of neurons from the dentate gyrus, we prepared RNAseq libraries using FACS purified GFP⁺ nuclei from animals receiving AAV-SpCas9 as well as either a control sgRNA that has been designed to target bacterial *lacZ* gene and not the mouse genome, or a *Mecp2*-targeting sgRNA. All sgRNAs have been optimized to minimize their off-target score (CRISPR Design Tool: http://tools.genome-engineering.org)[2]. We were able to find differentially expressed genes (Figure 63b) between control and *Mecp2* sgRNA expressing nuclei (*p*<0.01). We identified several interesting candidates among genes that were down-regulated in *Mecp2* sgRNA expressing nuclei: *Hpca, Olfm1,* and *Ncdn,* which have been previously reported to play important roles in learning behaviors[22-24]; and *Cplx2,* which has been shown to be involved in synaptic vesicle release and related to neuronal firing rate[25, 26]. These results demonstrate that the combination of SpCas9-mediated genome perturbation and population level RNAseq analysis provides a way to characterize transcriptional regulations in neurons and suggest genes that may be important to specific neuronal functions or disease processes.

SpCas9-mediated *in vivo* genome editing in the brain can also be coupled with electrophysiological recording to study the effect of genomic perturbation on specific cell types or circuit components. To study the functional effect of MeCP2 depletion on neuronal physiology we stereotactically co-delivered AAV-SpCas9 and AAV-SpGuide targeting *Mecp2* into the superficial layer of the primary visual cortex (V1) of male mice. V1 was chosen since the superficial layer cortical excitatory neurons are more accessible to two-photon imaging and two-photon guided targeted recording. Two weeks after SpCas9 delivery, mice were subjected to two-photon guided juxtacellular recordings to compare the electrophysiological response of KASH-GFP⁺ neurons and GFP⁻ neighboring neurons in layer 2/3 of mouse V1 (Figure 64a-c). We measured neuronal responses to 18 drifting gratings in 20-degree increments and calculated evoked firing rate (FR) and orientation selectivity index (OSI) of cells by vector averaging the response. Both FR and OSI were significantly reduced for excitatory GFP⁺, MeCP2 knockout neurons, compared to neighboring GFP⁻ excitatory neurons (Figure 64d-e). In comparison, control sgRNA expression together with SpCas9 did not have any effect on FR and OSI when compared with neighboring uninfected neurons (Figure 64d-e). These results show that SpCas9 mediated depletion of MeCP2 in adult V1 cortical neurons alters the visual response properties of excitatory neurons *in vivo* within two weeks and further demonstrate the versatility of SpCas9 in facilitating targeted gene knockout in the mammalian brain *in vivo,* for studying genes functions and dissection of neuronal circuits.

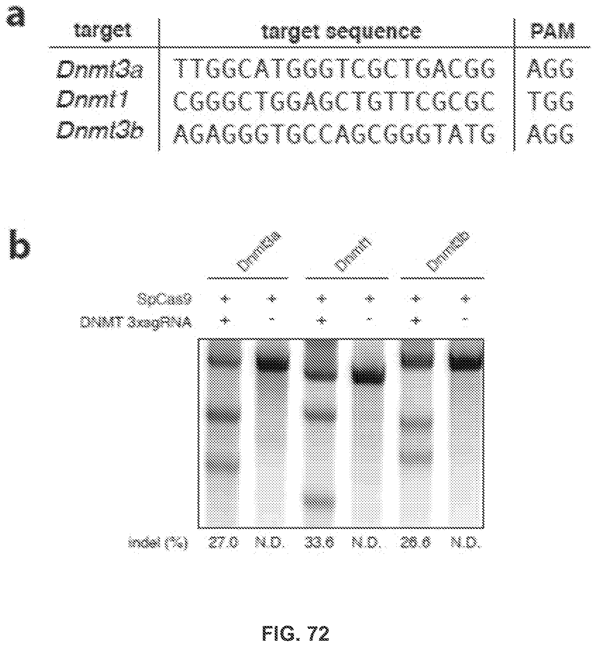

One key advantage of the SpCas9 system is its ability to facilitate multiplex genome editing[2]. Introducing stable knockouts of multiple genes in the brain of living animals will have potentially far-reaching applications, such as causal interrogation of multigenic mechanisms in physiological and neuropathological conditions. To test the possibility of multiplex genome editing in the brain we designed a multiplex sgRNA expression vector consisting of three sgRNAs in tandem, along with GFP-KASH for nuclei labeling (Figure 65a). We chose sgRNAs targeting the DNA methyltransferases gene family (DNMTs), which consists of *Dnmt1, Dnmt3a* and *Dnmt3b. Dnmt1* and *3a* are highly expressed in the adult brain and it was previously shown that DNMT activity alters DNA methylation and both Dnmt3a and Dnmt1 are required for synaptic plasticity and learning and memory formation[27]. We designed individual sgRNAs against *Dnmt3a* and *Dnmt1* with high modification efficiency. To avoid any potential compensatory effects by *Dnmt3b* we decided also to additionally target this gene even though it is expressed mainly during neurodevelopment[27]. We finally selected individual sgRNAs for high simultaneous DNA cleavage for all three targeted genes (Figure 66b and Figure 72).

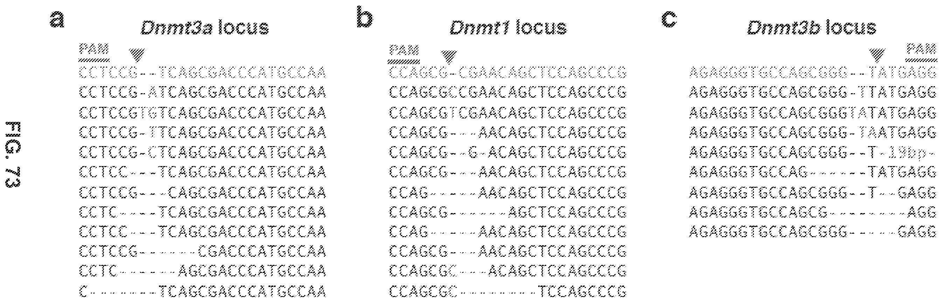

To test the efficacy of multiplex genome editing *in vivo,* we stereotactically delivered a mixture of high titer AAV-SpCas9 and AAV-SpGuide into the dorsal and ventral dentate gyrus of male adult mice. After 4 weeks, hippocampi were dissected and targeted cell nuclei were sorted via FACS. We detected -19% (*Dnmt3a*)*,* 18% (*Dnmt1*) and 4% (*Dnmt3b*) indel frequency in the sorted nuclei population using SURVEYOR nuclease assay (Figure 66c) and sequencing (Figure 73). Targeting multiple loci raises the question about the effective rate of multiple-knockouts in individual cells. By using single nuclei sorting combined with targeted sequencing, we quantified simultaneous targeting of multiple DNMT loci in individual neuronal nuclei (Figure 66d). Of neuronal nuclei carrying modification in at least one *Dnmt* locus, more than 70% of nuclei contained indels in both *Dnmt3a* and *Dnmt1* (~40% contained indels at all 3 loci, and ~30% at both *Dnmt3a* and *Dnmt1* loci). These results are in agreement with Dnmt3a and Dnmt1 protein depletion levels in the dentate gyrus (Figure 66e). Due to the low expression of *Dnmt3b* in the adult brain, we were not able to detect Dnmt3b protein.

Recent studies with SpCas9 have shown that, although each base within the 20-nt sgRNA sequence contributes to overall specificity, genomic loci that partially match the sgRNA can result in off-target double strand brakes and indel formations[28,29]. To assess the rate of off-target modifications, we computationally identified a list of highly similar genomic target sites[2] and quantified the rate of modifications using targeted deep sequencing. Indel analysis of the top predicted off-target loci revealed a 0-1.6% rate of indel formations demonstrating that SpCas9 modification is specific. To increase the specificity of SpCas9-mediated genome editing *in vivo,* future studies may use off-targeting minimization strategies such as double nicking[30, 31] and truncated sgRNAs[28].

Knockdown of Dnmt3a and Dnmt1 have been previous shown to impact hippocampus-dependent memory formation[27]. Consequently, we performed contextual fear-conditioning behavior tests to investigate the effect of SpCas9-mediated triple knockout (*Dnmt3a, Dnmt1* and *Dnmt3b*) on memory acquisition and consolidation. While we did not observe any differences between control and triple knockout mice in the memory acquisition phase, knockout mice showed impaired memory consolidation when tested under trained context conditions (Figure 66f). This effect was abolished when mice were tested in the altered context. Our results demonstrate that CRIPSR-Cas9-mediated knockout of DNMT family members in dentate gyrus neurons is sufficient to probe the function of genes in behavioral tasks.

Together, our results demonstrate that AAV-mediated *in vivo* delivery of SpCas9 and sgRNA provides a rapid and powerful technology for achieving precise genomic perturbations within intact neural circuits. Whereas SpCas9 has been broadly used to engineer dividing cells, we demonstrate that SpCas9 can also be used to engineer the genome of postmitotic neurons with high efficiency via NHEJ-mediated indel generation. SpCas9-mediated genomic perturbations can be combined with biochemical, sequencing, electrophysiological, and behavioral analysis to study the function of the targeted genomic element. We demonstrated that SpCas9-mediated targeting of single or multiple genes can recapitulate morphological, electrophysiological, and behavioral phenotypes observed using classical, more time-consuming genetic mouse models. The current study employed the *Streptococcus pyogenes* Cas9, which not only necessitates the use of two AAV vectors but also limits the size of promoter elements can be used to achieve cell type-specific targeting. Given the diversity of Cas9 orthologues, with some being substantially shorter than SpCas9[2, 32, 33], it should be possible to engineer single AAV vectors expressing both Cas9 and sgRNA, as described herein.

## REFERENCES

1. Nestler, E.J. & Hyman, S.E. Animal models of neuropsychiatric disorders. Nat Neurosci 13, 1161-1169 (2010).

2. Cong, L. et al. Multiplex genome engineering using CRISPR/Cas systems. Science 339, 819-823 (2013).

3. Mali, P. et al. RNA-guided human genome engineering via Cas9. Science 339, 823-826 (2013).

4. Burger, C., Nash, K. & Mandel, R.J. Recombinant adeno-associated viral vectors in the nervous system. Hum Gene Ther 16, 781-791 (2005).

5. Wu, Z., Yang, H. & Colosi, P. Effect of genome size on AAV vector packaging. Mol Ther 18, 80-86 (2010).

6. Deltcheva, E. et al. CRISPR RNA maturation by trans-encoded small RNA and host factor RNase III. Nature 471, 602-607 (2011).

7. Gray, S.J. et al. Optimizing promoters for recombinant adeno-associated virus-mediated gene expression in the peripheral and central nervous system using self-complementary vectors. Hum Gene Ther 22, 1143-1153 (2011).

8. Levitt, N., Briggs, D., Gil, A. & Proudfoot, N.J. Definition of an efficient synthetic poly(A) site. Genes Dev 3, 1019-1025 (1989).

9. Ostlund, C. et al. Dynamics and molecular interactions of linker of nucleoskeleton and cytoskeleton (LINC) complex proteins. J Cell Sci 122, 4099-4108 (2009).

10. Chahrour, M. & Zoghbi, H.Y. The story of Rett syndrome: from clinic to neurobiology. Neuron 56, 422-437 (2007).

11. Kishi, N. & Macklis, J.D. MECP2 is progressively expressed in post-migratory neurons and is involved in neuronal maturation rather than cell fate decisions. Molecular and cellular neurosciences 27, 306-321 (2004).

12. Skene, P.J. et al. Neuronal MeCP2 is expressed at near histone-octamer levels and globally alters the chromatin state. Molecular cell 37, 457-468 (2010).

13. Chen, R.Z., Akbarian, S., Tudor, M. & Jaenisch, R. Deficiency of methyl-CpG binding protein-2 in CNS neurons results in a Rett-like phenotype in mice. Nat Genet 27, 327-331 (2001).

14. Zhou, Z. et al. Brain-specific phosphorylation of MeCP2 regulates activity-dependent Bdnf transcription, dendritic growth, and spine maturation. Neuron 52, 255-269 (2006).

15. Li, Y. et al. Global transcriptional and translational repression in human-embryonic-stem-cell-derived Rett syndrome neurons. Cell Stem Cell 13, 446-458 (2013).

16. Nguyen, M.V. et al. MeCP2 is critical for maintaining mature neuronal networks and global brain anatomy during late stages of postnatal brain development and in the mature adult brain. J Neurosci 32, 10021-10034 (2012).

17. Jiang, W., Bikard, D., Cox, D., Zhang, F. & Marraffini, L.A. RNA-guided editing of bacterial genomes using CRISPR-Cas systems. Nature biotechnology 31, 233-239 (2013).

18. Qi, L.S. et al. Repurposing CRISPR as an RNA-guided platform for sequence-specific control of gene expression. Cell 152, 1173-1183 (2013).

19. Sapranauskas, R. et al. The Streptococcus thermophilus CRISPR/Cas system provides immunity in Escherichia coli. Nucleic acids research 39, 9275-9282 (2011).

20. Jinek, M. et al. A programmable dual-RNA-guided DNA endonuclease in adaptive bacterial immunity. Science 337, 816-821 (2012).

21. Qiu, P. et al. Mutation detection using Surveyor nuclease. BioTechniques 36, 702-707 (2004).

22. Kobayashi, M. et al. Hippocalcin-deficient mice display a defect in cAMP response element-binding protein activation associated with impaired spatial and associative memory. Neuroscience 133, 471-484 (2005).

23. Dateki, M. et al. Neurochondrin negatively regulates CaMKII phosphorylation, and nervous system-specific gene disruption results in epileptic seizure. The Journal of biological chemistry 280, 20503-20508 (2005).

24. Nakaya, N. et al. Deletion in the N-terminal half of olfactomedin 1 modifies its interaction with synaptic proteins and causes brain dystrophy and abnormal behavior in mice. Experimental neurology 250, 205-218 (2013).

25. Reim, K. et al. Complexins regulate a late step in Ca2+-dependent neurotransmitter release. Cell 104, 71-81 (2001).

26. Edwardson, J.M. et al. Expression of mutant huntingtin blocks exocytosis in PC12 cells by depletion of complexin II. The Journal of biological chemistry 278, 30849-30853 (2003).

27. Feng, J. et al. Dnmt1 and Dnmt3a maintain DNA methylation and regulate synaptic function in adult forebrain neurons. Nat Neurosci 13, 423-430 (2010).

28. Fu, Y. et al. High-frequency off-target mutagenesis induced by CRISPR-Cas nucleases in human cells. Nature biotechnology 31, 822-826 (2013).

29. Hsu, P.D. et al. DNA targeting specificity of RNA-guided Cas9 nucleases. Nature biotechnology 31, 827-832 (2013).

30. Ran, F.A. et al. Double nicking by RNA-guided CRISPR Cas9 for enhanced genome editing specificity. Cell 154, 1380-1389 (2013).

31. Mali, P. et al. CAS9 transcriptional activators for target specificity screening and paired nickases for cooperative genome engineering. Nature biotechnology 31, 833-838 (2013).

32. Esvelt, K.M. & Wang, H.H. Genome-scale engineering for systems and synthetic biology. Molecular systems biology 9, 641 (2013).

33. Li, W., Teng, F., Li, T. & Zhou, Q. Simultaneous generation and germline transmission of multiple gene mutations in rat using CRISPR-Cas systems. Nat Biotechnol 31, 684-686 (2013).

**Methods**

**DNA constructs**

For SpCas9 targets selection and generation of single guide RNA (sgRNA), the 20-nt target sequences were selected to precede a 5'-NGG PAM sequence. To minimize off-targeting effects, the CRIPSR design tool was used (http://tools.genome-engineering.org). sgRNA was PCR amplified using U6 promoter as a template with forward primer: 5'-CGCACGCGTAATTCGAACGCTGACGTCATC-3' (SEQ ID NO: 208 and reverse primer containing the sgRNA with 20-nt DNA target site (*Bold*):

5'-CACACGCGTAAAAAAGCACCGACTCGGTGCCACTTTTTCAAGTTGATAACG

GACTAGCCTTATTTTAACTTGCTATTTCTAGCTCTAAAAC*NNNNNNNNNNNNNNNNNN*

*NNC*GGTGTTTCGTCCTTTCCAC-3'. (SEQ ID NO: 209 )

Control sgRNA sequence was designed to target *lacZ* gene from *E. coli:* target sequence: TGCGAATACGCCCACGCGATGGG (SEQ ID NO: 210) EGFP-KASH[1] construct was a generous gift from Prof. Worman (Columbia University, NYC) and was used as PCR template for cloning the coding cassette into AAV backbone under the human *Synapsin* promoter (*hSyn*). Next, U6-Mecp2sgRNA coding sequence was introduced using *Mlu*I site. For the multiplex gene targeting strategy, individual sgRNAs were PCR amplified as described above. All three sgRNAs were ligated with PCR amplified *hSyn*-GFP-KASH-bGHpA cassette by using the Golden Gate cloning strategy. After PCR amplification, the Golden Gate ligation product containing 3 sgRNAs and *hSyn*-GFP-KASH-bGH pA was cloned into AAV backbone. All obtained constructs were sequenced verified. In order to find the optimal promoter sequence to drive SpCas9 expression in neurons we tested: h*syn1,* mouse truncated *Mecp2* (pMecp2), and truncated rat *Map1b* (pMap1b) promoter sequences[2]. Following primers were used to amplify promoter regions:

hSyn_F: 5'-GTGTCTAGACTGCAGAGGGCCCTG-3'; (SEQ ID NO: 211)
hSyn_R: 5'-GTGTCGTGCCTGAGAGCGCAGTCGAGAA-3'; (SEQ ID NO: 212)
Mecp2 F 5'-GAGAAGCTTAGCTGAATGGGGTCCGCCTC-3'; (SEQ ID NO: 213)
Mecp2 R 5'-CTCACCGGTGCGCGCAACCGATGCCGGGACC-3'; (SEQ ID NO: 214)
Map1b-283/-58_F 5'-GAGAAGCTTGGCGAAATGATTTGCTGCAGATG-3'; (SEQ ID NO: 215)

Maplb-283/-58_R 5'-CTCACCGGTGCGCGCGTCGCCTCCCCCTCCGC-3'. (SEQ ID NO: 216)

Another truncation of rat *map1b* promoter was assembled with the following oligos:

5'-AGCTTCGCGCCGGGAGGAGGGGGGACGCAGTGGGCGGAGCGGAGACAGC
ACCTTCGGAGATAATCCTTTCTCCTGCCGCAGAGCAGAGGAGCGGCGGGAGAGGAA
CACTTCTCCCAGGCTTTAGCAGAGCCGGA-3'(SEQ ID NO: 217) and

5'-CCGGTCCGGCTCTGCTAAAGCCTGGGAGAAGTGTTCCTCTCCCGCCGCTC
CTCTGCTCTGCGGCAGGAGAAAGGATTATCTCCGAAGGTGCTGTCTCCGCTCCGCCC
ACTGCGTCCCCCTCCTCCCGGCGCGA-3'. (SEQ ID NO: 218)

Short synthetic polyadenylation signal (spA)[3] was assembled using following oligos: 5'-AATTCAATAAAAGATCTTTATTTTCATTAGATCTGTGTGTTGGTTTTTTG TGTGC-3' (SEQ ID NO: 219) and

5'-GGCCGCACACAAAAAACCAACACACAGATCTAATGAAAATAAAGAT
CTTTTATTG-3'. (SEQ ID NO: 220)

SpCas9 and its D10A mutant version (dSpCas9) were described previously[4, 5]. Plasmid encoding red fluorescent protein (mCherry) under control of EF1α promoter was used for neuron transfection with Lipofectamine®2000 (Life Technologies).

Cell line cultures and transfection

Neuro-2a (N2a) cells were grown in DMEM containing 5% fetal bovine serum (BSA). For HEK293FT cells DMEM containing 10% fetal bovine serum (FBS) was used. Cells were maintained at 37°C in 5% $CO_2$ atmosphere. Cells were transfected using Lipofectamine®2000 or Polyethylenimine (PEI) "MAX" reagent (Polysciences), according to manufacturer's protocols.

**Production of concentrated AAV vectors**

High titer AAV1/2 particles were produced using AAV1 and AAV2 serotype plasmids at equal ratios and pDF6 helper plasmid and purified on heparin affinity column[6]. Titering of viral particles was done by qPCR. High titer AAV1 particles were produced by the UNC Vector Core Services (University of North Carolina at Chapel Hill). Low titer AAV1 particles in DMEM were produced as described previously[7]. Briefly, HEK293FT cells were transfected with transgene plasmid, pAAV1 serotype plasmid and pDF6 helper plasmid using PEI "MAX". Culture medium was collected after 48 h and filtered through a 0.45 μm PVDF filter (Millipore).

**Primary cortical neuron culture**

Animals used to obtain neurons for tissue cultures were sacrificed according to the protocol approved by the MIT Committee on Animal Care (MIT CAC). Primary cultures were prepared from embryonic day 16 mouse brains[8]. Embryos of either sex were used. Cells were plated on poly-D-lysine (PDL) coated 24-well plates (BD Biosciences) or laminin/PDL coated coverslips (VWR). Cultures were grown at 37°C and 5% $CO_2$ in Neurobasal medium, supplemented with B27, Glutamax (Life Technologies) and penicillin/streptomycin mix.
For AAV transduction, cortical neurons in 500 μl Neurobasal culture medium were incubated at 7 DIV with 300 μl (double infection at 1:1 ratio) AAV1-containing conditioned medium from HEK293FT cells[7]. One week after transduction neurons have been harvested for downstream processing or fixed in 4% paraformaldehyde for immunofluorescent stainings or morphology analysis.
For visualization of neuronal morphology, cells at DIV7 were transfected with EF1α-mCherry expression vector using Lipofectamine®2000 (Life Technologies) for one week as previously described[9]. For measurement of total dendrite length, all dendrites of individual neurons were traced using ImageJ software. Quantification of the number of primary dendrites, dendritic tips and the Sholl analysis[10] were performed on images acquired with fluorescent microscope at a 40x objective (Zeiss AxioCam Ax10 microscope, Axiocam MRm camera). For dendrites number, ends of all non-axonal protrusions longer than 10 μm were counted. For Sholl analysis, concentric circles with 5 μm step in diameter were automatically drawn around the cell body, and the number of dendrites crossing each circle was counted using ImageJ software with a Sholl plug-in.

**Stereotactic injection of AAV1/2 into the mouse brain**

The MIT CAC approved all animal procedures described here. Adult (12-16 weeks old) male C57BL/6N mice were anaesthetized by intraperitoneal (i.p.) injection of 100 mg/kg Ketamine and 10 mg/kg Xylazine. Pre-emptive analgesia was given (Buprenex, 1 mg/kg, i.p.). Craniotomy was performed according to approved procedures and 1 μl of 1:1 AAV mixture (1x1013 Vg/ml of sMecp2-SpCas9; 6x1012 Vg/ml of DNMT 3xsgRNA; 3-5x1012 Vg/ml of hSyn-GFP-KASH) was injected into: dorsal dentate gyrus (anterior/posterior: -1.7; mediolateral: 0.6; dorsal/ventral: -2.15) and/or ventral dentate gyrus (anterior/posterior: -3.52; mediolateral: 2.65; dorsal/ventral: -3). For in vivo electrophysiology recordings experiments virus injection coordinates were 3 mm lateral (from Bregma) and 1 mm anterior from the posterior suture. The skull was thinned using a dremel drill with occasional cooling with saline, and the remaining dura was punctured using a glass micropipette filled with the virus suspended in mineral oil. Several injections (3-4) were made at neighboring sites, at a depth of 200-250 μm. A volume of 150-200 nl of virus mixture was injected at 75 nl/min rate at each site. After each injection, the pipette was held in place for 3-5 minutes prior to retraction to prevent leakage. The incision was sutured and proper post-operative analgesics (Meloxicam, 1-2 mg/kg) were administered for three days following surgery.

In vivo two-photon guided targeted loose patch recordings

Two weeks after virus injection, mice were used for electrophysiology experiments. Mice were anesthetized with 2% isoflurane and maintained using 0.8% isoflurane. The skin was excised, cleaned with sugi and a metal head plate was attached to the skull using glue and dental acrylic, and a 2 mm x 2 mm craniotomy was performed over the primary visual cortex (V1). The exposed area was then covered with a thin layer of 1.5% agarose in artificial cerebrospinal fluid (aCSF; 140 mM NaCl, 5 mM KCl, 2 mM CaC12, 1 mM MgC12, 0.01 mM EDTA, 10 mM HEPES, 10 mM glucose; pH 7.4). Animal body temperature was maintained during experiment 37.5°C with a heating blanket.

Borosilicate pipettes (WPI) were pulled using a Sutter P-2000 laser puller (Sutter Instruments). Tip diameter was around 1 μm while the resistance was between 3-5 MΩ. Recordings were made using custom software (Network Prism, Sur lab), written in Matlab (MathWorks), controlling a MultiClamp 700B amplifier (Axon). A glass pipette electrode was inserted into the brain at an angle of 20-35$^0$ and an Ag/AgCl ground electrode pellet (Warner Instruments) was positioned in the same solution as the brain and the objective. For fluorescent visualization, pipettes were filled with Alexa Fluor 594 (Molecular Probes). The pipette was first targeted to the injection site using a 10x lens, and then targeted to individual GFP+ cells using a 25x lens via simultaneous two-photon imaging at 770 nm. Cell proximity was detected through deflections in resistance observed in voltage clamp during a rapidly time-varying 5 mV command voltage pulse. Once resistance had increased by 5-10 MΩ, the amplifier was switched to current clamp, and spikes were recorded with zero injected current, under a Bessel filter of 4 KHz and an AC filter of 300 Hz. Virus injected brains were perfused post hoc and immunohistochemistry was performed.

**Visual stimulation and data analysis from in vivo two-photon guided targeted loose patch recordings**

To assess the orientation selectivity and tuning of genome-edited neurons, we presented oriented gratings using custom software written in Matlab PsychToolbox-3.

Gratings were optimized for cellular responsiveness and were presented by stepping the orientation from 0-360 degrees in steps of 20 degrees, with each grating presentation being preceded for 4 seconds "off" followed by 4 seconds "on", for a total presentation duration of 144 seconds. Data was acquired directly into Matlab and saved as .mat files. Spike detection was performed via analysis routines that used manually defined thresholds followed by spike shape template matching for further verification. Every spike was tagged and displayed on screen in a graphical user interface whereupon it was manually reviewed for false positives and negatives by the experimenter. Spike times in response to every stimulus were then grouped into "on" or "off" periods based on their timing relative to visual stimulation, and "on" spikes for each stimulus were decremented by the number of "off" spikes observed during an equal time period. For orientation experiments, # spikes per stimulus = (# spikes "on") - (# spikes "off') because "on" and "off" periods were the same duration.

For every cell of interest, the methods were used to collect responses for each oriented stimulus (0 to 360 degrees, in steps of 20 degrees). These responses were then turned into a "tuning curve" of orientation vs. response for each trial. Orientation Selectivity Index (OSI) was computed by taking the vector average for the preferred orientation according to the formulae as follows:

$$OSI = \frac{\sqrt{(\sum_i R(\theta_i) \sin(2\theta_i))^2 + (\sum_i R(\theta_i) \cos(2\theta_i))^2}}{\sum_i R(\theta_i)}$$

**Tissue preparation and purification of cell nuclei**

Total hippocampus or dentate gyrus was quickly dissected in ice cold DPBS (Life Sciences) and shock frozen on dry ice. For cell nuclei purification, tissue was gently homogenized in 2 ml ice-cold homogenization buffer (HB) (320 mM Sucrose, 5 mM CaCl, 3 mM Mg(Ac)$_2$, 10 mM Tris pH7.8, 0.1 mM EDTA, 0.1 % NP40, 0.1 mM PMSF, 1 mM betamercaptoethanol) using 2 ml Dounce homogenizer (Sigma); 25 times with pestle A, followed by 25 times with pestle B. Next, 3 ml of HB was added up to 5 ml total and kept on ice for 5 min. For gradient centrifugation, 5 ml of 50% OptiPrep™ density gradient medium (Sigma) containing 5 mM CaCl, 3 mM Mg(Ac)$_2$, 10 mM Tris pH 7.8, 0.1 mM PMSF, 1 mM betamercaptoethanol was added and mixed. The lysate was gently loaded on the top of 10 ml 29% iso-osmolar OptiPrep™ solution in a conical 30 ml centrifuge tube (Beckman Coulter, SW28 rotor). Samples were centrifuged at 10,100 xg (7,500 rpm) for 30 min at 4°C. The supernatant was removed and the nuclei pellet was gently resuspended in 65 mM beta-glycerophosphate (pH 7.0), 2 mM MgCl2, 25 mM KCl, 340 mM sucrose and 5% glycerol. Number and quality of purified nuclei was controlled using bright field microscopy.

**Cell nuclei sorting**

Purified GFP-positive (GFP$^+$) and negative (GFP$^-$) intact nuclei were co-labeled with Vybrant® DyeCycle™ Ruby Stain (1:500, Life Technologies) and sorted using BD FACSAria III (Koch Institute Flow Cytometry Core, MIT). GFP$^+$ and GFP$^-$ nuclei were collected in 1.5 ml Eppendorf tubes coated with 1% BSA and containing 400 $\mu$l of resuspension buffer (65 mM beta-glycerophosphate pH 7.0, 2 mM MgCl$_2$, 25 mM KCl, 340 mM sucrose and 5% glycerol). After sorting, all samples were kept on ice and centrifuged at 10,000 xg for 20 min at 4°C. Nuclei pellets were stored at -80°C or were directly used for downstream processing.

**Genomic DNA extraction and SURVEYOR™ assay**

For functional testing of sgRNA, 50-70% confluent N2a cells were co-transfected with a single PCR amplified sgRNA and SpCas9 vector. Cells transfected with SpCas9 only served as negative control. Cells were harvested 48 h after transfection, and DNA was extracted using DNeasy Blood & Tissue Kit (Qiagen) according to the manufacturer's protocol. To isolate genomic DNA from AAV1 transduced primary neurons, DNeasy Blood & Tissue Kit was used 7 days post AAV transduction, according to the manufacturer's instruction.

Sorted nuclei or dissected tissues were lysed in lysis buffer (10 mM Tris, pH 8.0, 10 mM NaCl, 10 mM EDTA, 0.5 mM SDS, Proteinase K (PK, 1mg/ml) and RNAse A) at 55°C for 30 min. Next, chloroform-phenol extraction was performed followed by DNA precipitation with ethanol, according to standard procedures. DNA was finally resuspended in TE Buffer (10 mM Tris pH 8.0, 0.1 mM EDTA) and used for downstream analysis. Functional testing of individual sgRNAs was performed by SURVEYOR™ nuclease assay (Transgenomics) using PCR primers listed in Supplementary Table 2. Band intensity quantification was performed as described before[11].

**RNA library preparation and sequencing**

Two weeks after bilateral viral delivery of SpCas9 with guide targeting *Mecp2* (4 animals) or SpCas9 with gRNA targeting *lacZ* (4 animals), the dentate gyrus was quickly dissected in ice cold DPBS (Life Sciences) and transferred immediately to RNA-later solution (Ambion). After 24 hours in 4°C the tissue was moved to -80°C. Populations of 100 targeted neuronal nuclei were FACS sorted into 10 $\mu$l TCL buffer supplemented with 1% 2-mercaptoethanol (Qiagen). After centrifuging, samples were frozen immediately at -80°C. The RNA was purified by AMPure RNAcleanXP SPRI beads (Beckman Coulter Genomics) following the manufactures' instructions, and washed three times with 80% ethanol, omitting the final elution. The beads with captured RNA were air-dried and processed immediately for cDNA synthesis. Samples with no nuclei were used as negative controls. Three population samples were used for each animal, total of 24 population sample, in cDNA library preparations following the SMART-seq2 protocol[12] only replacing the reverse transcriptase enzyme with 0.1 ul of Maxima H Minus enzyme (200 U/ul, Thermo Scientific), and scaling down the PCR reaction to a volume of 25 ul. The tagmentation reaction and final PCR amplification were done using the Nextera XT DNA Sample preparation kit (Illumina), with the following modifications. All reaction volumes were scaled down by a factor of 4, and the libraries were pooled after the PCR amplification step by taking 2.5 ul of each sample. The pooled libraries were cleaned and size-selected using two rounds of 0.7 volume of AMPure XP SPRI bead cleanup (Beckman Coulter Genomics). Samples were loaded on a High-Sensitivity DNA chip (Agilent) to check the quality of the library, while quantification was done with Qubit High-Sensitivity DNA kit (Invitrogen). The pooled libraries were diluted to a final concentration of 4 nM and 12 pmol and were sequenced using Illumina Miseq with 75 bp paired end reads.

**RNA libraries data analysis**

Bowtie2 index was created based on the mouse mm9 UCSC genome and known Gene transcriptome[13], and paired-end reads were aligned directly to this index using Bowtie2 with command line options -q --phred33-quals -n 2 -e 99999999 -125 -I 1 -X 1000 -a -m 200 -p 4 --chunkmbs 512. Next, RSEM v1.27 was run with default parameters on the alignments created by Bowtie2 to estimate expression levels. RSEM's gene level expression estimates (tau) were multiplied by 1,000,000 to obtain transcript per million (TPM) estimates for each gene, and TPM estimates were transformed to log-space by taking log2(TPM+1). Genes were considered detected if their transformed expression level equal to or above 2 (in log2(TPM+1) scale). A library is filtered out if it has less than 8000 genes detected. Based on this criterion, 4 libraries were filtered and excluded from the downstream analysis. To find differentially expressed genes between control animals and Mecp2 sgRNA expressing animals, Student's $t$-test (Matlab V2013b) and cross validation was used in 20 random permutation runs, where in each run one library from each animal was randomly chosen to exclude (this results in a total of 12 libraries used in the $t$-test each time). The $t$-test was run on all genes that have mean expression level above 0.9 quantile (usually around 5 log2(TPM+1)) for each sample. Then, genes that were significant ($p<0.01$) in more than one thirds of the permutation runs were chosen. The log2(TPM+1) expression levels of these genes across samples were clustered using hierarchical clustering (Matlab V2013b).

**Immunofluorescent staining**

Cell culture: For immunofluorescent staining of primary neurons, cells were fixed 7 days after viral delivery with 4% paraformaldehyd (PFA) for 20 min at RT. After washing 3 times with PBS, cells were blocked with 5% normal goat serum (NGS) (Life Technologies), 5% donkey serum (DS) (Sigma) and 0.1% Triton-X100 (Sigma) in PBS for 30 min at RT. Cells were incubated with primary antibodies in 2.5% NGS, 2.5% DS and 0.1% Triton-X100 for 1 hour at RT or overnight at 4°C. After washing 3 times with PBST, cells were incubated with secondary antibodies for 1 hour at RT. Finally, coverslips were mounted using VECTASHIELD HardSet Mounting Medium with DAPI (Vector Laboratories) and imaged using an Zeiss AxioCam Ax10 microscope and an Axiocam MRm camera. Images were processed using the Zen 2012 software (Zeiss). Quantifications were performed by using ImageJ software 1.48 h and Neuron detector plugin.

Mice were sacrified 4 weeks after viral delivery by a lethal dose of Ketamine/Xylazine and transcardially perfused with PBS followed by PFA. Fixed tissue was sectioned using vibratome (Leica, VT1000S). Next, 30 μm sections were boiled for 2 min in sodium citrate buffer (10 mM tri-sodium citrate dehydrate, 0.05% Tween20, pH 6.0) and cool down at RT for 20 min. Sections were blocked with 4% normal goat serum (NGS) in TBST (137 mM NaCl, 20 mM Tris pH 7.6, 0.2% Tween-20) for 1 hour. Paraffin sections were cut using a microtom (Leica RM2125 RTS) to 8 μm, and stained as described previously[14].

Sections were incubated with primary antibodies diluted in TBST with 4% NGS overnight at 4°C. After 3 washes in TBST, samples were incubated with secondary antibodies. After washing with TBST 3 times, sections were mounted using VECTASHIELD HardSet Mounting Medium with DAPI and visualized with confocal microscope (Zeiss LSM 710, Ax10 ImagerZ2, Zen 2012 Software).

Following primary antibodies were used: rabbit anti-Dnmt3a (Santa Cruz, 1:100); rabbit anti-MeCP2 (Millipore, 1:200); mouse anti-NeuN (Millipore, 1:50-1:400); chicken anti-GFAP (Abcam, 1:400); mouse anti-Map2 (Sigma, 1:500); chicken anti-GFP (Aves labs, 1:200-1:400); mouse anti-HA (Cell Signaling, 1:100). Secondary antibodies: AlexaFluor®488, 568 or 633 (Life Technologies, 1:500-1:1,000).

**Quantification of LIVE/DEAD® assay**

Control and transduced primary neurons were stained using the LIVE/DEAD® assay (Life technologies) according to the manufacturer's instruction. To avoid interference with the GFP-signal from GFP-KASH expression, cells were stained for DEAD (ethidium homodimer) and DAPI (all cells) only. Stained cells were imaged using fluorescence microscopy and DEAD, GFP and DAPI positive cells were counted by using ImageJ 1.48h software and Neuron detector plugin.

**Western blot analysis**

Transduced primary cortical neurons (24 well, 7 days after viral delivery) and transduced tissue samples (4 weeks after viral delivery) were lysed in 50 μL of ice-cold RIPA buffer (Cell Signaling) containing 0.1% SDS and proteases inhibitors (Roche, Sigma). Cell lysates were sonicated for 5 min in a Bioruptor sonicater (Diagenode) and protein concentration was determined using the BCA Protein Assay Kit (Pierce Biotechnology, Inc.). Protein lysats were dissolved in SDS-PAGE sample buffer, separated under reducing conditions on 4-15% Tris-HCI gels (Bio-Rad) and analyzed by Western blotting using primary antibodies: rabbit anti-Dnmt3a (Santa Cruz, 1:500), mouse anti-Dnmtl (Novus Biologicals, 1:800), rabbit anti-Mecp2 (Millipore, 1:400), rabbit anti-Tubulin (Cell Signaling, 1:10,000) followed by secondary antimouse and anti-rabbbit HRP antibodies (Sigma-Aldrich, 1:10,000). GAPDH was directly visualized with rabbit HRP coupled anti-GAPDH antibody (Cell Signaling, 1:10,000). Tubulin or GAPDH served as loading control. Blots were imaged with ChemiDoc™ MP system with ImageLab 4.1 software (BioRad), and quantified using ImageJ software 1.48h.

**Delay Contextual Fear Conditioning (DCFC)**

8 weeks after bilateral SpCas9/DNMT 3xsgRNA delivery into the dorsal and ventral dentate gyrus of 12 weeks old C57BL/6N male mice, animals were habituated to the experimentor and the behavior room for 7 days. SpCas9/GFP-KASH injected littermates served as controls. At day 1 of DCFC, mouse cages were placed into an islolated anterroom to prevent mice from auditory cues before and after testing. Indivdual mice were placed into the FC chamber (Med Associates Inc.) and a 12 min habituation period was performed. After habituation the mice were placed back to their homecages. The next day (training day) individual mice were placed into the chamber and were allowed to habituate for 4 min. After another 20 sec (pre-tone) interval, the tone (auditory cue) at a level of 85 dB, 2.8 kHz was presented for 20 sec follwed by 18 sec delay interval before the foot-shock was presented (0.5 mA, 2 sec). After the foot-shock, 40 sec interval (post-tone/shock) preceded a next identical trial starting with the 20 sec pre-tone period. The training trial was repeated 6 times before the mice were placed back to their homecages. At day 3 (testing day), mice were first placed in the conditioning context chamber for 3 min. Next, mice underwent 4x 100 sec testing trials starting with a 20 sec interval followed by 20 sec tone and a 60 sec post-tone interval. Finally, mice were placed in an altered context-conditioning chamber (flat floor vs. grid, tetrameric vs. heptameric chamber, vanillin scent) and the testing trial was repeated. Freezing behavior was recorded and analysis was performed blind off-line manually and confirmed with Noldus EthoVision XT software (Noldus Information Technology).

**Deep sequencing analysis and indel detection**

CRISPR Design Tool (http://crispr.mit.edu/) was used to find potential off-targets for DNMT family genes, targeted by CRISPR-SpCas9 in the brain. Targeted cell nuclei from dentate gyrus were FACS sorted 12 weeks after viral delivery and genomic DNA was purified as described above. For each gene of interest, the genomic region flanking the CRISPR target site was amplified by a fusion PCR method to attach the Illumina P5 adapters as well as unique sample-specific barcodes to the target amplicons (for on- and off-target primers see Supplementary Table 3)[15]. Barcoded and purified DNA samples were quantified by Qubit 2.0 Fluorometer (Life Technologies) and pooled in an equimolar ratio. Sequencing libraries were then sequenced with the Illumina MiSeq Personal Sequencer (Life Technologies), with read length 300 bp.

The MiSeq reads were analyzed as described previously in[15]. Briefly, reads were filtered by Phred quality (Q score) and aligned using a Smith-Waterman algorithm to the genomic region 50 nucleotides upstream and downstream of the target site. Indels were estimated in the aligned region from 5 nucleotides upstream to 5 nucleotides downstream of the target site (a total of 30 bp). Negative controls for each sample were used to estimate the inclusion or exclusion of indels as putative cutting events. We computed a maximum-likelihood estimator (MLE) for the fraction of reads having target-regions with true-indels, using the per-target-region-per-read error rate from the data of the negative control sample. The MLE scores and cutting rates for each target are listed in Supplementary Table 1.

**Statistical analysis**

All experiments were performed with a minimum of two independent biological replicates. Statistics were performed with Prism6 (GraphPad) using Student's two tailed *t*-test.

**SUPPLEMENTARY TABLES**

## Supplementary Table 1. Off-target analysis for DNMTs targeting

| | Gene | GI | Potential off-target sequences | MLE (%) | SEM |
|---|---|---|---|---|---|
| Dnmt1 | Abca1 | NM_013454 | GGAGCTGGAGCTGTTCACGTTGG | 0.0000 | 0.00 |
| | Mctp1 | NM_030174 | CGGGCAGCAGATGTTCGCGTAGG | 0.0806 | 0.08 |
| | Exd2 | NM_133798 | AGGGCTTGAGATGTTCGGGCTGG | 0.0612 | 0.06 |
| | Pik3r6 | NM_001004435 | CCGGCTGGGGCTGTCCTCGCTAG | 0.0000 | 0.00 |
| | Sobp | NM_175407 | CGGGGGTGCAGCTGCTCACGCCAG | 0.0000 | 0.00 |
| | Vac14 | NM_146216 | CTGGCGGGAGCTGGTCGCGTGAG | 0.0083 | 0.00 |
| Dnmt3a | Efemp2 | NM_021474 | TGAGCATGGGCCGCTGGCGGTGG | 0.0050 | 0.01 |
| | Bmpr1b | NM_001277217 | ATGGCATAGGCCGCTGACAGAGG | 0.0117 | 0.01 |
| | Syce1 | NM_001143765 | TTGGCATGGTGAGCTGGCGGGGG | 0.0067 | 0.00 |
| | Atp8b3 | NM_026094 | TGGGCAGGGGTCTCTGAGGGCAG | 0.0067 | 0.01 |
| | Rdh11 | NM_021557 | TTGGCATGGGTCTCTTACCAAGG | 0.0017 | 0.00 |
| Dnmt3b | Hecw2 | NM_001001883 | ACATGGTTCCAGTGGGTATGTAG | 0.0000 | 0.00 |
| | Plekhg3 | NM_153804 | GGAGGTGGGCAGCGGGTATGTAG | 0.0954 | 0.01 |
| | Cdc25b | NM_001111075 | AGAAGGTCCCCGCGGGCATGGAG | 0.2421 | 0.12 |

| | | | | | |
|---|---|---|---|---|---|
| Top1mt | NM_028404 | GGAGGGAACCAGCCGGTATGGGG | 0.0167 | 0.01 |
| Sesn2 | NM_144907 | AGAGAGTGGCAGTGGGTAAGCAG | 0.0000 | 0.00 |
| Ncan | NM_007789 | AGAGGTGGCCAGCGGGCAGGAAG | 0.0017 | 0.00 |
| Nacad | NM_001081652 | TGAGGGGGCCAGCTGGGATGCAG | 1.6254 | 0.76 |

(SEQ ID NO: 221 to238

### Supplementary Table 2. PCR primers used in the SURVEYOR assay

| Gene | Forward primer sequence (5'-3') | SEQ ID NO: | Reverse primer sequence (5'-3') | SEQ ID NO: |
|---|---|---|---|---|
| Mecp2 | GGTCTCATGTGTGGCACTCA | 239 | TGTCCAACCTTCAGGCAAGG | 243 |
| Dnmt3a | ATCCCTCCTCAGAGGGTCAGC | 240 | TACCTCATGCACAGCTAGCACC | 244 |
| Dnmt1 | TTCGGGCATAGCATGGTCTTCC | 241 | GTTCTATTTCAGAGGGCTGATCCC | 245 |
| Dnmt3b | GTTCTGAGCCGCACAGTTTGG | 242 | GGATAAGAAGGGACAATACAGG | 246 |

### Supplementary Table 3. Primers used for on- and off-target genomic loci amplification

| Gene | Forward primer sequence (5'-3') | SEQ ID NO: | Reverse primer sequence (5'-3') | SEQ ID NO: |
|---|---|---|---|---|
| Dnmt1 | GCCGGGGTCTC GTTCAGAGCT | 247 | CTACCGCCTGCGGA CATGGT | 268 |
| Dnmt3a | CCTGTCTCTCTGT CCTAGGGCTCC | 248 | CCGTTTGCTGATGTAGTA GGGGTCC | 269 |
| Dnmt3b | CCCACAGGAAA CAATGAAGGGAGAC | 249 | CATCCTTCGTGTCT GAGGACTGGTC | 270 |

(continued)

| Gene | Forward primer sequence (5'-3') | SEQ ID NO: | Reverse primer sequence (5'-3') | SEQ ID NO: |
|---|---|---|---|---|
| *Abca1* | CCCTGACACCAGC TGTTCAGCAC | 250 | CTCTGGGTGAC CACACACGATGC | 271 |
| *Mctp1* | GAGCAGGCAGA GCCGAGCAAG | 251 | GGAGAGCGTCC GCCAGGAG | 272 |
| *Exd2* | GGGTCTTGTTGTG AGTAGGGTGTG | 252 | GAAGCTCTCTTAA CTACTGTTC | 273 |
| *Pik3r6* | CCTGGAATACTAT TTCCACGCCG | 253 | CAGGCCCTAGCAGCG AGCAG | 274 |
| *Sobp* | GCAGCACACTCCA CCCTCACAT | 254 | GGAAGGGGCTTTCC TCCGAGC | 275 |
| *Vac14* | CGGCGTCACG TGACCTGAGTAAC | 255 | GCTCCGACCCTGCT CTCCCA | 276 |
| *Efemp2* | GTGTCTGCCTC GCTCTGCTGC | 256 | CCTGTTCATCAGGCTC GTAGCCC | 277 |
| *Bmpr1b* | CTATCTGAAATCC ACCACCTTAGACGC | 257 | CGATTGCTGGCTTGC CTTGAG | 278 |
| *Syce1* | GCCTGAGGGGG CCAGAGGT | 258 | GGTTCGCGTCCGCC CGCGTGAT | 279 |
| *Atp8b3* | GGGACTCC CCGGGTGGTG | 259 | GAGAGGTGGTC CTGTCGCCTATG | 280 |
| *Rdh11* | GACCCTGTGTTT CAAGTCTCTCTG | 260 | CCCAGCAGGTCACA GCTGACATC | 281 |
| *Hecw2* | GGCCATCCAGTAC ATTCAATACG | 261 | AGCACAGTATGTATTC TATAAAATAATACGAC | 282 |
| *Plekhg3* | GCAGAAGCCGT GACTCACAGCA | 262 | GTGGGAGGGGACAG AGACCATG | 283 |
| *Cdc25b* | CTTGTGCTTG TGATTCTGTCCTTACTGC | 263 | CCTTACCTGTTCCTCT TCCTTATCCAGC | 284 |
| *Top1mt* | CGAGAAGTC GATGCAGACACTTCAA | 264 | ATACCCAGTCCAC ATCCCTGCC | 285 |
| *Sesn2* | GCTGAAGACTGGC GAGCACAGCT | 265 | CCTCTGCATCTCCCTCAGGAA GTATT | 286 |
| *Ncan* | GACCTGAATGTTG TGGCTGAGAGTCC | 266 | GCCTCCTGTC CCCAGGTCCC | 287 |
| *Nacad* | CCCTCACGTTCC TGTCCAGCAA | 267 | CACTAGGCTT GGGCTGCCCTCT | 288 |

## REFERENCES

1. Ostlund, C. et al. Dynamics and molecular interactions of linker of nucleoskeleton and cytoskeleton (LINC) complex proteins. J Cell Sci 122, 4099-4108 (2009).
2. Gray, S.J. et al. Optimizing promoters for recombinant adeno-associated virus-mediated gene expression in the peripheral and central nervous system using self-complementary vectors. Hum Gene Ther 22, 1143-1153 (2011).

3. Levitt, N., Briggs, D., Gil, A. & Proudfoot, N.J. Definition of an efficient synthetic poly(A) site. Genes Dev 3, 1019-1025 (1989).

4. Jinek, M. et al. A programmable dual-RNA-guided DNA endonuclease in adaptive bacterial immunity. Science 337, 816-821 (2012).

5. Cong, L. et al. Multiplex genome engineering using CRISPR/Cas systems. Science 339, 819-823 (2013).

6. McClure, C., Cole, K.L., Wulff, P., Klugmann, M. & Murray, A.J. Production and titering of recombinant adeno-associated viral vectors. J Vis Exp, e3348 (2011).

7. Konermann, S. et al. Optical control of mammalian endogenous transcription and epigenetic states. Nature 500, 472-476 (2013).

8. Banker, G. & Goslin, K. Developments in neuronal cell culture. Nature 336, 185-186 (1988).

9. Swiech, L. et al. CLIP-170 and IQGAP1 cooperatively regulate dendrite morphology. J Neurosci 31, 4555-4568 (2011).

10. Sholl, D.A. Dendritic organization in the neurons of the visual and motor cortices of the cat. J Anat 87, 387-406 (1953).

11. Ran, F.A. et al. Genome engineering using the CRISPR-Cas9 system. Nature protocols 8, 2281-2308 (2013).

12. Picelli, S. et al. Smart-seq2 for sensitive full-length transcriptome profiling in single cells. Nature methods 10, 1096-1098 (2013).

13. Fujita, P.A. et al. The UCSC Genome Browser database: update 2011. Nucleic acids research 39, D876-882 (2011).

14. Tzingounis, A.V. et al. The KCNQ5 potassium channel mediates a component of the afterhyperpolarization current in mouse hippocampus. Proceedings of the National Academy of Sciences of the United States of America 107, 10232-10237 (2010).

15. Hsu, P.D. et al. DNA targeting specificity of RNA-guided Cas9 nucleases. Nature biotechnology 31, 827-832 (2013).

16. Qiu, P. et al. Mutation detection using Surveyor nuclease. BioTechniques 36, 702-707 (2004).

*Example 39: Further Investigation into Nuclear Tagging Technique*

This Example concerns epitope tagging of Cas9. In brief, we found that a triple epitope tag (specifically 3xHA) improves the detection signal.

**Materials and Methods**

*Cell culture and transfection*

Human embryonic kidney (HEK) cell line 293FT (Life Technologies) or mouse Hepal-6 (Sigma-Aldrich) cell line was maintained in Dulbecco's modified Eagle's Medium (DMEM) supplemented with 10 % fetal bovine serum (HyClone), 2mM GlutaMAX (Life Technologies), 100 U/mL penicillin, and $100\mu g/mL$ streptomycin at 37 °C with 5 % $CO_2$ incubation.

Cells were seeded onto 24-well plates (Corning) at a density of 120,000 cells/well, 24 hours prior to transfection. Cells were transfected using Lipofectamine 2000 (Life Technologies) at 80-90 % confluency following the manufacturer's recommended protocol. A total of 500 ng Cas9 plasmid and 100 ng of U6-sgRNA PCR product was transfected.

*SURVEYOR nuclease assay for genome modification*

293FT and HUES62 cells were transfected with DNA as described above. Cells were incubated at 37 °C for 72 hours post-transfection prior to genomic DNA extraction. Genomic DNA was extracted using the QuickExtract DNA Extraction Solution (Epicentre) following the manufacturer's protocol. Briefly, pelleted cells were resuspended in Quick-Extract solution and incubated at 65 °C for 15 minutes, 68 °C for 15 minutes, and 98 °C for 10 minutes.

The genomic region flanking the CRISPR target site for each gene was PCR amplified, and products were purified using QiaQuick Spin Column (Qiagen) following the manufacturer's protocol. 400 ng total of the purified PCR products were mixed with 2 microlitres 10X Taq DNA Polymerase PCR buffer (Enzymatics) and ultrapure water to a final volume of 20 microlitres, and subjected to a re-annealing process to enable heteroduplex formation: 95 °C for 10 min, 95 °C to 85 °C ramping at - 2 °C/s, 85 °C to 25 °C at - 0.25 °C/s, and 25 °C hold for 1 minute. After re-annealing, products were treated with SURVEYOR nuclease and SURVEYOR enhancer S (Transgenomics) following the manufacturer's recommended protocol, and analyzed on 4-20% Novex TBE poly-acrylamide gels (Life Technologies). Gels were stained with SYBR Gold DNA stain (Life Technologies) for 30 minutes and imaged with a Gel Doc gel imaging system (Bio-rad). Quantification was based on relative band intensities. Indel percentage was determined by the formula,

$100 \times (1 - (1 - (b + c) / (a + b + c))^{1/2})$, where a is the integrated intensity of the undigested PCR product, and b and c are the integrated intensities of each cleavage product.

*Western blot*

HEK 293FT cells were transfected and lysed in 1X RIPA buffer (Sigma-Aldrich) supplemented with Protease Inhibitor (Roche). The lysates were loaded onto Bolt 4-12% Bis-Tris Plus Gels (Invitrogen) and transferred to nitrocellulose membranes. The membranes were blocked in Tris-buffered saline containing 0.1% Tween-20 and 5% blocking agent (G-Biosciences). The membranes were probed with rabbit anti-FLAG (1:5,000, Abcam), HRP-conjugated anti-GAPDH (1:5,000 Cell Signaling Technology), and HRP-conjugated anti-rabbit (1:1,000) antibodies and visualized with a Gel Doc XR+ System (Bio-Rad).

**References**

Banker G, Goslin K. Developments in neuronal cell culture. Nature. 1988 Nov 10;336(6195): 185-6.

Bedell, V.M. et al. In vivo genome editing using a high-efficiency TALEN system. Nature 491, 114-U133 (2012).

Bhaya, D., Davison, M. & Barrangou, R. CRISPR-Cas systems in bacteria and archaea: versatile small RNAs for adaptive defense and regulation. Annu Rev Genet 45, 273-297 (2011).

Bobis-Wozowicz, S., Osiak, A., Rahman, S.H. & Cathomen, T. Targeted genome editing in pluripotent stem cells using zinc-finger nucleases. Methods 53, 339-346 (2011).

Boch, J. et al. Breaking the code of DNA binding specificity of TAL-type III effectors. Science 326, 1509-1512 (2009).

Bogenhagen, D.F. & Brown, D.D. Nucleotide sequences in Xenopus 5S DNA required for transcription termination. Cell 24, 261-270 (1981).

Bultmann, S. et al. Targeted transcriptional activation of silent oct4 pluripotency gene by combining designer TALEs and inhibition of epigenetic modifiers. Nucleic Acids Res 40, 5368-5377 (2012).

Carlson, D.F. et al. Efficient TALEN-mediated gene knockout in livestock. Proc Natl Acad Sci U S A 109, 17382-17387 (2012).

Chen, F.Q. et al. High-frequency genome editing using ssDNA oligonucleotides with zinc-finger nucleases. Nat Methods 8, 753-U796 (2011).

Cho, S.W., Kim, S., Kim, J.M. & Kim, J.S. Targeted genome engineering in human cells with the Cas9 RNA-guided endonuclease. Nat Biotechnol 31, 230-232 (2013).

Christian, M. et al. Targeting DNA double-strand breaks with TAL effector nucleases. Genetics 186, 757-761 (2010).

Cong, L. et al. Multiplex genome engineering using CRISPR-Cas systems. Science 339, 819-823 (2013).

Deltcheva, E. et al. CRISPR RNA maturation by trans-encoded small RNA and host factor RNase III. Nature 471, 602-607 (2011).

Deveau, H., Garneau, J.E. & Moineau, S. CRISPR-Cas system and its role in phage-bacteria interactions. Annu Rev Microbiol 64, 475-493 (2010).

Ding, Q. et al. A TALEN genome-editing system for generating human stem cell-based disease models. Cell Stem Cell 12, 238-251 (2013).

Garneau, J.E. et al. The CRISPR-Cas bacterial immune system cleaves bacteriophage and plasmid DNA. Nature 468, 67-71 (2010).

Gasiunas, G., Barrangou, R., Horvath, P. & Siksnys, V. Cas9-crRNA ribonucleoprotein complex mediates specific DNA cleavage for adaptive immunity in bacteria. Proc Natl Acad Sci U S A 109, E2579-2586 (2012).

Geurts, A.M. et al. Knockout Rats via Embryo Microinjection of Zinc-Finger Nucleases. Science 325, 433-433 (2009).

Gray SJ, Foti SB, Schwartz JW, Bachaboina L, Taylor-Blake B, Coleman J, Ehlers MD, Zylka MJ, McCown TJ, Samulski RJ. Optimizing promoters for recombinant adeno-associated virus-mediated gene expression in the peripheral and central nervous system using self-complementary vectors. Hum Gene Ther. 2011 Sep;22(9):1143-53. doi: 10.1089/hum.2010.245.

Guschin, D.Y. et al. A rapid and general assay for monitoring endogenous gene modification. Methods Mol Biol 649, 247-256 (2010).

Hasty, P., Rivera-Perez, J. & Bradley, A. The length of homology required for gene targeting in embryonic stem cells. Mol Cell Biol 11, 5586-5591 (1991).

Horvath, P. & Barrangou, R. CRISPR-Cas, the immune system of bacteria and archaea. Science 327, 167-170 (2010).

Hsu, P.D. & Zhang, F. Dissecting neural function using targeted genome engineering technologies. ACS Chem Neurosci 3, 603-610 (2012).

Hwang, W.Y. et al. Efficient genome editing in zebrafish using a CRISPR-Cas system. Nat Biotechnol 31, 227-229 (2013).

Jiang, W., Bikard, D., Cox, D., Zhang, F. & Marraffini, L.A. RNA-guided editing of bacterial genomes using CRISPR-Cas systems. Nat Biotechnol 31, 233-239 (2013).

Jinek, M. et al. A programmable dual-RNA-guided DNA endonuclease in adaptive bacterial immunity. Science 337, 816-821 (2012).

Jinek, M. et al. RNA-programmed genome editing in human cells. eLife 2, e00471 (2013).

Kaplitt, M.G., et al., Safety and tolerability of gene therapy with an adeno-associated virus (AAV) borne GAD gene for Parkinson's disease: an open label, phase I trial. Lancet. 2007 Jun 23;369(9579):2097-105.

Levitt N. Briggs D. Gil A. Proudfoot N.J. Definition of an efficient synthetic poly(A) site. Genes Dev. 1989;3:1019-1025.

Liu D, Fischer I. Two alternative promoters direct neuron-specific expression of the rat microtubule-associated protein 1B gene. J Neurosci. 1996 Aug 15;16(16):5026-36.

Lopes, V.S., etc al., Retinal gene therapy with a large MYO7A cDNA using adeno-assocaited virus. Gene Ther, 2013 Jan 24. doi: 10.1038/gt 2013.3.[Epub ahead of print]

Mahfouz, M.M. et al. De novo-engineered transcription activator-like effector (TALE) hybrid nuclease with novel DNA binding specificity creates double-strand breaks. Proc Natl Acad Sci U S A 108, 2623-2628 (2011).

Makarova, K.S. et al. Evolution and classification of the CRISPR-Cas systems. Nat Rev Microbiol 9, 467-477 (2011).

Mali, P. et al. RNA-guided human genome engineering via Cas9. Science 339, 823-826 (2013).

McClure C, Cole KL, Wulff P, Klugmann M, Murray AJ. Production and titering of recombinant adeno-associated viral vectors. J Vis Exp. 2011 Nov 27;(57):e3348. doi: 10.3791/3348.

Michaelis, L.M., Maud "Die kinetik der invertinwirkung.". Biochem. z (1913).

Miller, J.C. et al. An improved zinc-finger nuclease architecture for highly specific genome editing. Nat Biotechnol 25, 778-785 (2007).

Miller, J.C. et al. A TALE nuclease architecture for efficient genome editing. Nat Biotechnol 29, 143-148 (2011).

Moscou, M.J. & Bogdanove, A.J. A simple cipher governs DNA recognition by TAL effectors. Science 326, 1501 (2009).Porteus, M.H. & Baltimore, D. Chimeric nucleases stimulate gene targeting in human cells. Science 300, 763 (2003).

Mussolino, C. et al. A novel TALE nuclease scaffold enables high genome editing activity in combination with low toxicity. Nucleic acids research 39, 9283-9293 (2011).

Nathwani, A.C., et al., Adenovirus-associated virus vector-mediated gene transfer in hemophilia B. N Engl J Med. 2011 Dec 22;365(25):2357-65. doi: 10.1056/NEJMoa1108046. Epub 2011 Dec 10.

Oliveira, T.Y. et al. Translocation capture sequencing: a method for high throughput mapping of chromosomal rearrangements. J Immunol Methods 375, 176-181 (2012).

Perez, E.E. et al. Establishment of HIV-1 resistance in CD4(+) T cells by genome editing using zinc-finger nucleases. Nat Biotechnol 26, 808-816 (2008).

Qi, L.S. et al. Repurposing CRISPR as an RNA-guided platform for sequence-specific control of gene expression. Cell 152, 1173-1183 (2013).

REMINGTON'S PHARMACEUTICAL SCIENCES (Mack Pub. Co., N.J. 1991)

Reyon, D. et al. FLASH assembly of TALENs for high-throughput genome editing. Nat Biotechnol 30, 460-465 (2012).

Saleh-Gohari, N. & Helleday, T. Conservative homologous recombination preferentially repairs DNA double-strand breaks in the S phase of the cell cycle in human cells. Nucleic Acids Res 32, 3683-3688 (2004).

Sander, J.D. et al. Selection-free zinc-finger-nuclease engineering by context-dependent assembly (CoDA). Nat Methods 8, 67-69 (2011).

Sanjana, N.E. et al. A transcription activator-like effector toolbox for genome engineering. Nat Protoc 7, 171-192 (2012).

Sapranauskas, R. et al. The Streptococcus thermophilus CRISPR-Cas system provides immunity in Escherichia coli. Nucleic Acids Res 39, 9275-9282 (2011).

Shen, B. et al. Generation of gene-modified mice via Cas9/RNA-mediated gene targeting. Cell Res 23, 720-723 (2013).

Smithies, O., Gregg, R.G., Boggs, S.S., Koralewski, M.A. & Kucherlapati, R.S. Insertion of DNA sequences into the human chromosomal beta-globin locus by homologous recombination. Nature 317, 230-234 (1985).

Soldner, F. et al. Generation of isogenic pluripotent stem cells differing exclusively at two early onset Parkinson point mutations. Cell 146, 318-331 (2011).

Takasu, Y. et al. Targeted mutagenesis in the silkworm Bombyx mori using zinc finger nuclease mRNA injection. Insect Biochem Molec 40, 759-765 (2010).

Tangri S, et al., Rationally engineered therapeutic proteins with reduced immunogenicity, J Immunol. 2005 Mar 15;174(6):3187-96.

Thomas, K.R., Folger, K.R. & Capecchi, M.R. High frequency targeting of genes to specific sites in the mammalian genome. Cell 44, 419-428 (1986).

Tuschl, T. Expanding small RNA interference. Nat Biotechnol 20, 446-448 (2002).

Urnov, F.D., Rebar, E.J., Holmes, M.C., Zhang, H.S. & Gregory, P.D. Genome editing with engineered zinc finger nucleases. Nat Rev Genet 11, 636-646 (2010).

Valton, J. et al. Overcoming transcription activator-like effector (TALE) DNA binding domain sensitivity to cytosine methylation. J Biol Chem 287, 38427-38432 (2012).

Wang, H. et al. One-Step Generation of Mice Carrying Mutations in Multiple Genes by CRISPR-Cas-Mediated Genome Engineering. Cell 153, 910-918 (2013).

Watanabe, T. et al. Non-transgenic genome modifications in a hemimetabolous insect using zinc-finger and TAL effector nucleases. Nat Commun 3 (2012).

Wilson, E.B. Probable inference, the law of succession, and statistical inference. J Am Stat Assoc 22, 209-212 (1927).

Wood, A.J. et al. Targeted genome editing across species using ZFNs and TALENs. Science 333, 307 (2011).

Wu, S., Ying, G.X., Wu, Q. & Capecchi, M.R. A protocol for constructing gene targeting vectors: generating knockout mice for the cadherin family and beyond. Nat Protoc 3, 1056-1076 (2008).

Zhang, F. et al. Efficient construction of sequence-specific TAL effectors for modulating mammalian transcription. Nat Biotechnol 29, 149-153 (2011).

## Claims

1. A composition comprising a CRISPR-Cas system for use in the treatment of a post-mitotic cell disorder selected from Table A or Table B; wherein said CRISPR-Cas system comprises
   a CRISPR enzyme comprising at least one or more nuclear localization sequences;
   a guide sequence capable of hybridizing to a target sequence in a post-mitotic eukaryotic cell;
   a tracr mate sequence; and
   a tracr sequence;
   or one or more polynucleotides encoding said CRISPR enzyme, said guide sequence, said tracr mate sequence, and said tracr sequence.

2. The composition for use according to claim 1, comprising:

   I. polynucleotide sequence comprising:

      (a) a guide sequence capable of hybridizing to a target sequence in a post mitotic eukaryotic cell,
      (b) a tracr mate sequence, and
      (c) a tracr sequence, and

   II. a polynucleotide sequence encoding a CRISPR enzyme comprising at least one or more nuclear localization sequences,

   wherein (a), (b) and (c) are arranged in a 5' to 3' orientation,
   wherein when transcribed, the tracr mate sequence hybridizes to the tracr sequence and the guide sequence directs sequence-specific binding of a CRISPR complex to the target sequence, and
   wherein the polynucleotide sequence encoding a CRISPR enzyme is DNA or RNA.

3. The composition for use according to claim 1, comprising:

   I. a polynucleotide comprising:

      (a) a guide sequence capable of hybridizing to a target sequence in a eukaryotic cell, and
      (b) at least one or more tracr mate sequences,

   II. a polynucleotide sequence encoding a CRISPR enzyme comprising at least one or more nuclear localization sequences, and
   III. a polynucleotide sequence comprising a tracr sequence, wherein when transcribed, the tracr mate sequence hybridizes to the tracr sequence and the guide sequence directs sequence-specific binding of a CRISPR complex to the target sequence, and

   wherein the CRISPR complex comprises the CRISPR enzyme complexed with (1) the guide sequence that is hybridized to the target sequence, and (2) the tracr mate sequence that is hybridized to the tracr sequence, and the polynucleotide sequence encoding a CRISPR enzyme is DNA or RNA.

4. The composition for use according to claim 1, 2 or 3, comprising: a delivery system configured to deliver a CRISPR-Cas system to a cell of a subject, comprising: (a) a first regulatory element operable in the cell, operably linked to polynucleotide sequence encoding a guide sequence that hybridizes with a target sequence of DNA in the cell, and (b) a second regulatory element operable in the cell operably linked to a polynucleotide sequence encoding a CRISPR enzyme comprising at least one or more nuclear localization sequences, whereby the guide sequence targets the target sequence in the cell.

5. The composition for use according to any of the preceding claims, wherein the system comprises a vector system and the polynucleotides are located on same or different vectors.

6. The composition for use of claim 5, wherein the vector system comprises one or more viral vectors.

7. The composition for use of claim 5 or 6, wherein the vector system comprises one or more lentivirus, one or more adenovirus, one or more adeno-associated virus, or one or more herpes virus.

8. The composition for use of any of claims 5 to 7, wherein each viral vector is packaged into a viral particle.

9. The composition for use according to any of claims 5 to 8, wherein at least one of the vectors further comprises a promoter for driving CRISPR enzyme coding nucleic acid molecule expression, and wherein said promoter is for either ubiquitous expression or tissue specific expression.

10. The composition for use according to claim 9, wherein said promoter is for ubiquitous expression and is selected from the group consisting of CMV, CAG, CBh, PGK, SV40, and Ferritin heavy or light chains.

11. The composition for use according to claim 9, wherein said promoter is for tissue specific expression and is selected from the group consisting of synapsin I for all neurons, CaMKIIalpha for excitatory neurons, GAD67 or GAD65 or VGAT for GABAergic neurons, albumin promoter for liver expression, and OG-2 for osteoblasts.

12. The composition for use according to any of claims 2 to 11, comprising more than one guide sequence and wherein each guide sequence is directed to a different target sequence.

13. The composition for use according to any of claims 2 to 12, further comprising one or more repair templates.

14. The composition for use of claim 13, wherein the one or more repair templates results in a mutation comprising an insertion, deletion, or substitution of one or more nucleotides of said target sequence.

15. The composition for use of claim 14, wherein said mutation results in one or more amino acid changes in a protein expressed from the target sequence.

16. The composition for use according to any of claims 2 to 15, wherein the CRISPR enzyme comprises a Cas9.

17. The composition for use according to claim 16, wherein said Cas9 is SaCas9 and wherein said vector system comprises a single viral vector which is an AAV vector.

18. The composition for use according to any of claims 2 to 17, wherein the CRISPR enzyme comprises one or more mutations.

19. The composition for use of claim 18, wherein the one or more mutations are selected from the group consisting of D10A, H840A, N854A, N863A with reference to the position numbering of a *Streptococcus pyogenes* Cas9 (SpCas9) protein; or the CRISPR enzyme comprises one or more mutations in a RuvC1 domain of the CRISPR enzyme.

20. The composition for use according to any of claims 2 to 19, wherein the CRISPR enzyme is a nickase.

21. The composition for use according to any of claims 1 to 20, wherein the CRISPR-Cas system includes a functional domain.

22. The composition for use according to any of claims 1 to 21, wherein the CRISPR-Cas system includes an activator domain.

23. The composition for use according to any one of claims 1 to 22, wherein the cells are brain cells, kidney cells heart cells, digestive system cells, lung cells, eye cells, ear cells, skin cells, muscle cells, bone cells or liver cells.

24. The composition for use according to any one of claims 1 to 22, wherein the cells are immune cells.

25. The composition for use according to any of the preceding claims, wherein the composition is therapeutic as to a disease, disorder or indication of Table A or Table B or a cellular function or gene of Table C.

26. The composition for use according to any of the preceding claims, for use in the treatment of genetic diseases or conditions caused by, or associated with, a deficient genotype in post-mitotic cells.

27. The composition for use according to any of the preceding claims, wherein the composition is therapeutic as to: a trinucleotide repeat disorder; a metabolic, liver, kidney or protein disease or disorder; a muscular or skeletal disease or disorder; a neurological or neuronal disease or disorder; a cell dysregulation or oncology disease or disorder; a blood or coagulation disease or disorder; an inflammation or immune related disease or disorder; or an ocular disease or disorder.

28. The composition for use according to any of the preceding claims, wherein the cell is *ex vivo.*

29. The composition for use according to any of claims 1 to 28, wherein the delivery system comprises a liposome, particle, exosome or microvesicle.

30. An in vitro method of modifying a post-mitotic cell by manipulation of a target sequence in a genomic locus of interest, to thereby invoke a phenotypic change in the cell, comprising
delivering to said post-mitotic cell a non-naturally occurring or engineered composition comprising:

   I. a polynucleotide sequence comprising:

      (a) a guide sequence capable of hybridizing to a target sequence in a post-mitotic eukaryotic cell,
      (b) a tracr mate sequence, and
      (c) a tracr sequence, and

   II. a polynucleotide sequence encoding a CRISPR enzyme comprising at least one or more nuclear localization sequences,

   wherein (a), (b) and (c) are arranged in a 5' to 3' orientation,
   wherein when transcribed, the tracr mate sequence hybridizes to the tracr sequence and the guide sequence directs sequence-specific binding of a CRISPR complex to the target sequence, and
   wherein the polynucleotide sequence encoding a CRISPR enzyme is DNA or RNA.

31. An in vitro method of modifying a post-mitotic cell by manipulation of a target sequence in a genomic locus of interest, to thereby invoke a phenotypic change in the cell, comprising
delivering to said post-mitotic cell a non-naturally occurring or engineered composition comprising:

   I. a polynucleotide comprising:

      (a) a guide sequence capable of hybridizing to a target sequence in a post-mitotic eukaryotic cell, and
      (b) at least one or more tracr mate sequences,

   II. a polynucleotide sequence encoding a CRISPR enzyme comprising at least one or more nuclear localization sequences, and
   III. a polynucleotide sequence comprising a tracr sequence, wherein when transcribed, the tracr mate sequence hybridizes to the tracr sequence and the guide sequence directs sequence-specific binding of a CRISPR complex to the target sequence, and

wherein the CRISPR complex comprises the CRISPR enzyme complexed with (1) the guide sequence that is hybridized to the target sequence, and (2) the tracr mate sequence that is hybridized to the tracr sequence, and the polynucleotide sequence encoding a CRISPR enzyme is DNA or RNA.

**Patentansprüche**

1. Zusammensetzung, umfassend ein CRISPR-Cas-System zur Verwendung bei der Behandlung einer postmitotischen Zellstörung ausgewählt aus Tabelle A oder Tabelle B; wobei das CRISPR-Cas-System Folgendes umfasst:

   ein CRISPR-Enzym, das wenigstens eine oder mehrere Kernlokalisierungssequenzen umfasst;
   eine Leitsequenz mit der Fähigkeit, an eine Zielsequenz in einer postmitotischen eukaryontischen Zelle zu hybridisieren;
   eine "tracr mate"-Sequenz; und
   eine "tracr"-Sequenz;
   oder ein oder mehrere Polynukleotide, die das CRISPR-Enzym, die Leitsequenz, die tracr mate-Sequenz und die tracr-Sequenz codieren.

2. Zusammensetzung zur Verwendung nach Anspruch 1, umfassend:

   I. Polynukleotidsequenz umfassend:

      (a) eine Leitsequenz mit der Fähigkeit, an eine Zielsequenz in einer postmitotischen eukaryontischen Zelle zu hybridisieren,
      (b) eine tracr mate-Sequenz und
      (c) eine tracr-Sequenz und

   II. eine Polynukleotidsequenz, die ein CRISPR-Enzym codiert, das wenigstens eine oder mehrere Kernlokalisierungssequenzen umfasst,

   wobei (a), (b) und (c) in einer 5'-nach-3'-Orientierung angeordnet sind,
   wobei bei Transkription die tracr mate-Sequenz an die tracr-Sequenz hybridisiert und die Leitsequenz sequenzspezifische Bindung eines CRISPR-Komplexes an die Zielsequenz steuert und
   wobei es sich bei der Polynukleotidsequenz, die ein CRISPR-Enzym codiert, um DNA oder RNA handelt.

3. Zusammensetzung zur Verwendung nach Anspruch 1, umfassend:

   I. ein Polynukleotid umfassend:

      (a) eine Leitsequenz mit der Fähigkeit, an eine Zielsequenz in einer eukaryontischen Zelle zu hybridisieren, und
      (b) wenigstens eine oder mehrere tracr mate-Sequenzen,

   II. eine Polynukleotidsequenz, die ein CRISPR-Enzym codiert, das wenigstens eine oder mehrere Kernlokalisierungssequenzen umfasst, und
   III. eine Polynukleotidsequenz, die eine tracr-Sequenz umfasst, wobei bei Transkription die tracr mate-Sequenz an die tracr-Sequenz hybridisiert und die Leitsequenz sequenzspezifische Bindung eines CRISPR-Komplexes an die Zielsequenz steuert und wobei der CRISPR-Komplex das CRISPR-Enzym im Komplex mit (1) der Leitsequenz, die an die Zielsequenz hybridisiert ist, und (2) der tracr mate-Sequenz, die an die tracr-Sequenz hybridisiert ist, umfasst und es sich bei der Polynukleotidsequenz, die ein CRISPR-Enzym codiert, um DNA oder RNA handelt.

4. Zusammensetzung zur Verwendung nach Anspruch 1, 2 oder 3, umfassend: ein Zuführungssystem, das so konfiguriert ist, dass ein CRISPR-Cas-System einer Zelle eines Individuums zugeführt wird, umfassend:

   (a) ein erstes Regulationselement, das in der Zelle funktionsfähig ist, in operativer Verknüpfung mit einer Polynukleotidsequenz, die eine Leitsequenz codiert, die mit einer Zielsequenz von DNA in der Zelle hybridisiert, und
   (b) ein zweites Regulationselement, das in der Zelle funktionsfähig ist, in operativer Verknüpfung mit einer Polynukleotidsequenz, die ein CRISPR-Enzym codiert, das wenigstens eine oder mehrere Kernlokalisierungssequenzen umfasst, womit die Leitsequenz auf die Zielsequenz in der Zelle zielt.

5. Zusammensetzung zur Verwendung nach einem der vorhergehenden Ansprüche, wobei das System ein Vektorsystem umfasst und die Polynukleotide auf dem gleichen oder auf unterschiedlichen Vektoren liegen.

6. Zusammensetzung zur Verwendung gemäß Anspruch 5, wobei das Vektorsystem einen oder mehrere Virusvektoren umfasst.

7. Zusammensetzung zur Verwendung gemäß Anspruch 5 oder 6, wobei das Vektorsystem ein oder mehrere Lentiviren, ein oder mehrere Adenoviren, ein oder mehrere adeno-assoziierte Viren oder ein oder mehrere Herpesviren umfasst.

8. Zusammensetzung zur Verwendung gemäß einem der Ansprüche 5 bis 7, wobei die Virusvektoren jeweils in ein Viruspartikel verpackt werden.

9. Zusammensetzung zur Verwendung nach einem der Ansprüche 5 bis 8, wobei wenigstens einer der Vektoren ferner einen Promotor zum Treiben der Expression CRISPR-Enzym codierender Nukleinsäuremoleküle umfasst und wobei der Promotor entweder ubiquitärer Expression oder gewebespezifischer Expression dient.

10. Zusammensetzung zur Verwendung nach Anspruch 9, wobei der Promotor ubiquitärer Expression dient und aus der Gruppe bestehend aus CMV, CAG, CBh, PGK, SV40 und Ferritin-schwere- oder -leichte-Ketten ausgewählt ist.

11. Zusammensetzung zur Verwendung nach Anspruch 9, wobei der Promotor gewebespezifischer Expression dient und aus der Gruppe bestehend aus Synapsin I für alle Neuronen, CaMKIIalpha für exzitatorische Neuronen, GAD67 oder GAD65 oder VGAT für GABAerge Neuronen, Albumin-Promotor für Leberexpression und OG-2 für Osteoblasten ausgewählt ist.

12. Zusammensetzung zur Verwendung nach einem der Ansprüche 2 bis 11, umfassend mehr als eine Leitsequenz, und wobei jede Leitsequenz auf eine unterschiedliche Zielsequenz gerichtet ist.

13. Zusammensetzung zur Verwendung nach einem der Ansprüche 2 bis 12, ferner umfassend eine oder mehrere Reparatur-Matrizen.

14. Zusammensetzung zur Verwendung gemäß Anspruch 13, wobei die eine oder mehreren Reparatur-Matrizen zu einer Mutation führen, die eine Insertion, Deletion oder Substitution eines oder mehrerer Nukleotide der Zielsequenz umfasst.

15. Zusammensetzung zur Verwendung gemäß Anspruch 14, wobei die Mutation zu einer oder mehreren Aminosäureänderungen in einem von der Zielsequenz exprimierten Protein führt.

16. Zusammensetzung zur Verwendung nach einem der Ansprüche 2 bis 15, wobei das CRISPR-Enzym ein Cas9 umfasst.

17. Zusammensetzung zur Verwendung nach Anspruch 16, wobei es sich bei dem Cas9 um SaCas9 handelt und wobei das Vektorsystem einen einzelnen Virusvektor umfasst, bei dem es sich um einen AAV-Vektor handelt.

18. Zusammensetzung zur Verwendung nach einem der Ansprüche 2 bis 17, wobei das CRISPR-Enzym eine oder mehrere Mutationen umfasst.

19. Zusammensetzung zur Verwendung gemäß Anspruch 18, wobei die eine oder mehreren Mutationen ausgewählt sind aus der Gruppe bestehend aus D10A, H840A, N854A, N863A unter Bezugnahme auf die Positionsnummerierung eines SpCas9(*Streptococcus pyrogenes* Cas9)-Proteins; oder das CRISPR-Enzym eine oder mehrere Mutationen in einer RuvC1-Domäne des CRISPR-Enzyms umfasst.

20. Zusammensetzung zur Verwendung nach einem der Ansprüche 2 bis 19, wobei es sich bei dem CRISPR-Enzym um eine Nickase handelt.

21. Zusammensetzung zur Verwendung nach einem der Ansprüche 1 bis 20, wobei das CRISPR-Cas-System eine funktionelle Domäne enthält.

22. Zusammensetzung zur Verwendung nach einem der Ansprüche 1 bis 21, wobei das CRISPR-Cas-System eine Aktivator-Domäne enthält.

23. Zusammensetzung zur Verwendung nach einem der Ansprüche 1 bis 22, wobei es sich bei den Zellen um Hirnzellen, Nierenzellen, Herzzellen, Zellen des Verdauungssystems, Lungenzellen, Augenzellen, Ohrenzellen, Hautzellen, Muskelzellen, Knochenzellen oder Leberzellen handelt.

24. Zusammensetzung zur Verwendung nach einem der Ansprüche 1 bis 22, wobei es sich bei den Zellen um Immunzellen handelt.

25. Zusammensetzung zur Verwendung nach einem der vorhergehenden Ansprüche, wobei die Zusammensetzung therapeutisch bezüglich einer Zellfunktion bzw. eines Gens in Tabelle C ist.

26. Zusammensetzung zur Verwendung nach einem der vorhergehenden Ansprüche, zur Verwendung bei der Behandlung von Erbkrankheiten oder Leiden, die durch einen fehlerhaften Genotyp in postmitotischen Zellen verursacht werden oder damit verbunden sind.

27. Zusammensetzung zur Verwendung nach einem der vorhergehenden Ansprüche, wobei die Zusammensetzung therapeutisch bezüglich: einer Trinukleotid-Repeat-Erkrankung, einer Stoffwechsel-, Leber-, Nieren- oder Proteinkrankheit oder -störung; einer Krankheit oder Störung von Muskulatur oder Knochengerüst; einer neurologischen oder neuronalen Krankheit oder Störung; einer Zellfehlregulations- oder onkologischen Krankheit oder Störung; einer Entzündung oder Immunkrankheit oder -Störung; oder einer Augenkrankheit oder -störung ist.

28. Zusammensetzung zur Verwendung nach einem der vorhergehenden Ansprüche, wobei sich die Zelle *ex vivo* befindet.

29. Zusammensetzung zur Verwendung nach einem der Ansprüche 1 bis 28, wobei das Zuführungssystem ein Liposom, Partikel, Exosom oder Mikrovesikel umfasst.

30. In-vitro-Verfahren zur Modifizierung einer postmitotischen Zelle durch Manipulation einer Zielsequenz in einem genomischen Locus von Interesse, so dass dadurch eine phänotypische Veränderung in der Zelle hervorgerufen wird, umfassend Zuführen einer nicht natürlich vorkommenden oder konstruierten Zusammensetzung, die

    I. eine Polynukleotidsequenz umfassend:

        (a) eine Leitsequenz mit der Fähigkeit, an eine Zielsequenz in einer postmitotischen eukaryontischen Zelle zu hybridisieren,
        (b) eine tracr mate-Sequenz und
        (c) eine tracr-Sequenz und

    II. eine Polynukleotidsequenz, die ein CRISPR-Enzym codiert, das wenigstens eine oder mehrere Kernlokalisierungssequenzen umfasst,

wobei (a), (b) und (c) in einer 5'-nach-3'-Orientierung angeordnet sind,
wobei bei Transkription die tracr mate-Sequenz an die tracr-Sequenz hybridisiert und die Leitsequenz sequenzspezifische Bindung eines CRISPR-Komplexes an die Zielsequenz steuert und
wobei es sich bei der Polynukleotidsequenz, die ein CRISPR-Enzym codiert, um DNA oder RNA handelt, umfasst,
an die postmitotische Zelle.

31. In-vitro-Verfahren zur Modifizierung einer postmitotischen Zelle durch Manipulation einer Zielsequenz in einem genomischen Locus von Interesse, so dass dadurch eine phänotypische Veränderung in der Zelle hervorgerufen wird, umfassend Zuführen einer nicht natürlich vorkommenden oder konstruierten Zusammensetzung, die

    I. ein Polynukleotid umfassend:

        (a) eine Leitsequenz mit der Fähigkeit, an eine Zielsequenz in einer postmitotischen eukaryontischen Zelle zu hybridisieren, und
(b) wenigstens eine oder mehrere tracr mate-Sequenzen,

II. eine Polynukleotidsequenz, die ein CRISPR-Enzym codiert, das wenigstens eine oder mehrere Kernlokalisierungssequenzen umfasst, und
III. eine Polynukleotidsequenz, die eine tracr-Sequenz umfasst, wobei bei Transkription die tracr mate-Sequenz an die tracr-Sequenz hybridisiert und die Leitsequenz sequenzspezifische Bindung eines CRISPR-Komplexes an die Zielsequenz steuert und wobei der CRISPR-Komplex das CRISPR-Enzym im Komplex mit (1) der Leitsequenz, die an die Zielsequenz hybridisiert ist, und (2) der tracr mate-Sequenz, die an die tracr-Sequenz hybridisiert ist, umfasst und es sich bei der Polynukleotidsequenz, die ein CRISPR-Enzym codiert, um DNA oder RNA handelt, umfasst, an die postmitotische Zelle.

## Revendications

1. Composition comprenant un système CRISPR-Cas pour utilisation dans le traitement d'un trouble cellulaire postmitotique choisi dans le tableau A ou le tableau B ; dans laquelle ledit système CRISPR-Cas comprend
une enzyme CRISPR comprenant au moins une ou plusieurs séquences de localisation nucléaire ;
une séquence de guidage capable de s'hybrider à une séquence cible dans une cellule eucaryote postmitotique ;
une séquence tracr mate; et
une séquence tracr ;
ou un ou plusieurs polynucléotides codant pour ladite enzyme CRISPR, ladite séquence de guidage, ladite séquence tracr mate et ladite séquence tracr.

2. Composition pour utilisation selon la revendication 1, comprenant :

I. une séquence polynucléotidique comprenant :

(a) une séquence de guidage capable de s'hybrider à une séquence cible dans une cellule eucaryote postmitotique,
(b) une séquence tracr mate, et
(c) une séquence tracr, et

II. une séquence polynucléotidique codant pour une enzyme CRISPR comprenant au moins une ou plusieurs séquences de localisation nucléaire,

dans laquelle (a), (b) et (c) sont agencées dans une orientation de 5' vers 3',
dans laquelle, lorsqu'elle est transcrite, la séquence tracr mate s'hybride à la séquence tracr et la séquence de guidage contrôle la liaison spécifique à une séquence d'un complexe CRISPR à la séquence cible, et dans laquelle la séquence polynucléotidique codant pour une enzyme CRISPR est un ADN ou un ARN.

3. Composition pour utilisation selon la revendication 1, comprenant :

I. un polynucléotide comprenant :

(a) une séquence de guidage capable de s'hybrider à une séquence cible dans une cellule eucaryote, et
(b) au moins une ou plusieurs séquences tracr mate,

II. une séquence polynucléotidique codant pour une enzyme CRISPR comprenant au moins une ou plusieurs séquences de localisation nucléaire, et
III. une séquence polynucléotidique comprenant une séquence tracr où, lorsqu'elle est transcrite, la séquence tracr mate s'hybride avec la séquence tracr et la séquence de guidage contrôle la liaison spécifique à une séquence d'un complexe CRISPR à la séquence cible, et où le complexe CRISPR comprend l'enzyme CRISPR complexée avec (1) la séquence de guidage qui est hybridée à la séquence cible, et (2) la séquence tracr mate qui est hybridée à la séquence tracr, et la séquence polynucléotidique codant pour une enzyme CRISPR est un ADN ou un ARN.

4. Composition pour utilisation selon la revendication 1, 2 ou 3, comprenant : un système d'administration configuré pour administrer un système CRISPR-Cas à une cellule d'un sujet, comprenant : (a) un premier élément régulateur opérationnel dans la cellule, fonctionnellement lié à une séquence polynucléotidique codant pour une séquence de guidage qui s'hybride avec une séquence cible d'ADN dans la cellule, et (b) un deuxième élément régulateur opérationnel dans la cellule, fonctionnellement lié à une séquence polynucléotidique codant pour une enzyme CRISPR comprenant au moins une ou plusieurs séquences de localisation nucléaire, de sorte que la séquence de guidage cible la séquence cible dans la cellule.

5. Composition pour utilisation selon l'une quelconque des revendications précédentes, dans laquelle le système comprend un système de vecteur et les polynucléotides sont situés sur des vecteurs identiques ou différents.

6. Composition pour utilisation selon la revendication 5, dans laquelle le système de vecteur comprend un ou plu ou sieurs vecteurs viraux.

7. Composition pour utilisation selon la revendication 5 ou 6, dans laquelle le système de vecteur comprend un ou plusieurs lentivirus, un ou plusieurs adénovirus, un ou plusieurs virus adéno-associés, ou un ou plusieurs virus de l'herpès.

8. Composition pour utilisation selon l'une quelconque des revendications 5 à 7, dans laquelle chaque vecteur viral est encapsulé dans une particule virale.

9. Composition pour utilisation selon l'une quelconque des revendications 5 à 8, dans laquelle au moins un des vecteurs comprend en outre un promoteur pour contrôler l'expression de la molécule d'acide nucléique codant pour l'enzyme CRISPR, et dans laquelle ledit promoteur est pour l'expression ubiquitaire ou l'expression spécifique à un tissu.

10. Composition pour utilisation selon la revendication 9, dans laquelle ledit promoteur est pour l'expression ubiquitaire et est choisi dans le groupe constitué de CMV, CAG, CBh, PGK, SV40, la chaîne lourde ou légère de ferritine.

11. Composition pour utilisation selon la revendication 9, dans laquelle ledit promoteur est pour expression spécifique à un tissu et est choisi dans le groupe constitué de la synapsine I pour tous les neurones, CaMKIIalpha pour les neurones excitateurs, GAD67 ou GAD65 ou VGAT pour les neurones GABAergiques, le promoteur d'albumine pour l'expression hépatique, et OG-2 pour les ostéoblastes.

12. Composition pour utilisation selon l'une quelconque des revendications 2 à 11, comprenant plus d'une séquence de guidage et dans laquelle chaque séquence de guidage est dirigée vers une séquence cible différente.

13. Composition pour utilisation selon l'une quelconque des revendications 2 à 12, comprenant en outre une ou plusieurs matrices de réparation.

14. Composition pour utilisation selon la revendication 13, dans laquelle les une ou plusieurs matrices de réparation conduisent à une mutation comprenant une insertion, une délétion ou une substitution d'un ou plusieurs nucléotides de ladite séquence cible.

15. Composition pour utilisation selon la revendication 14, dans laquelle ladite mutation conduit à un ou plusieurs changements d'acide aminé dans une protéine exprimée par la séquence cible.

16. Composition pour utilisation selon l'une quelconque des revendications 2 à 15, dans laquelle l'enzyme CRISPR comprend un Cas9.

17. Composition pour utilisation selon la revendication 16, dans laquelle ledit Cas9 est SaCas9 et dans laquelle ledit système de vecteur comprend un vecteur viral unique qui est un vecteur AAV.

18. Composition pour utilisation selon l'une quelconque des revendications 2 à 17, dans laquelle l'enzyme CRISPR comprend une ou plusieurs mutations.

19. Composition pour utilisation selon la revendication 18, dans laquelle les une ou plusieurs mutations sont choisies dans le groupe constitué de D10A, H840A, N854A, N863A en référence à la numérotation de position d'une protéine Cas9 de *Streptococcus pyogenes* (SpCas9) ; ou l'enzyme CRISPR comprend une ou plusieurs mutations dans un domaine RuvC1 de l'enzyme CRISPR.

**20.** Composition pour utilisation selon l'une quelconque des revendications 2 à 19, dans laquelle l'enzyme CRISPR est une nickase.

**21.** Composition pour utilisation selon l'une quelconque des revendications 1 à 20, dans laquelle le système CRISPR-Cas comprend un domaine fonctionnel.

**22.** Composition pour utilisation selon l'une quelconque des revendications 1 à 21, dans laquelle le système CRISPR-Cas comprend un domaine d'activateur.

**23.** Composition pour utilisation selon l'une quelconque des revendications 1 à 22, dans laquelle les cellules sont des cellules cérébrales, des cellules rénales, des cellules cardiaques, des cellules du système digestif, des cellules pulmonaires, des cellules oculaires, des cellules auriculaires, des cellules cutanées, des cellules musculaires, des cellules osseuses ou des cellules hépatiques.

**24.** Composition pour utilisation selon l'une quelconque des revendications 1 à 22, dans laquelle les cellules sont des cellules immunitaires.

**25.** Composition pour utilisation selon l'une quelconque des revendications précédentes, la composition étant thérapeutique vis-à-vis d'une fonction cellulaire ou d'un gène du tableau C.

**26.** Composition pour utilisation selon l'une quelconque des revendications précédentes, pour utilisation dans le traitement de maladies ou affections génétiques causées par, ou associées à, un génotype déficient dans des cellules postmitotiques.

**27.** Composition pour utilisation selon l'une quelconque des revendications précédentes, la composition étant thérapeutique pour : une répétition de trinucléotide ; une maladie ou un trouble métabolique, hépatique, rénal ou protéique ; une maladie ou un trouble musculaire ou squelettique ; une maladie ou un trouble neurologique ou neuronal ; un dérèglement cellulaire ou une maladie ou un trouble oncologique; une maladie ou un trouble du sang ou de la coagulation ; une maladie ou un trouble lié à l'inflammation ou l'immunité ; ou une maladie ou un trouble oculaire.

**28.** Composition pour utilisation selon l'une quelconque des revendications précédentes, dans laquelle la cellule est *ex vivo.*

**29.** Composition pour utilisation selon l'une quelconque des revendications 1 à 28, dans laquelle le système d'administration comprend un liposome, une particule, un exosome ou une microvésicule.

**30.** Procédé *in vitro* de modification d'une cellule postmitotique par manipulation d'une séquence cible dans un locus d'intérêt génomique, de façon à induire un changement phénotypique dans la cellule, comprenant l'administration à ladite cellule postmitotique d'une composition d'origine naturelle ou modifiée comprenant :

I. une séquence polynucléotidique comprenant :

(a) une séquence de guidage capable de s'hybrider à une séquence cible dans une cellule eucaryote postmitotique,
(b) une séquence tracr mate, et
(c) une séquence tracr, et

II. une séquence polynucléotidique codant pour une enzyme CRISPR comprenant au moins une ou plusieurs séquences de localisation nucléaire,

dans lequel (a), (b) et (c) sont agencés dans une orientation de 5' vers 3',
dans lequel, lorsqu'elle est transcrite, la séquence tracr mate s'hybride à la séquence tracr et la séquence de guidage contrôle la liaison spécifique à une séquence d'un complexe CRISPR à la séquence cible, et dans laquelle la séquence polynucléotidique codant pour une enzyme CRISPR est un ADN ou un ARN.

**31.** Procédé *in vitro* de modification d'une cellule postmitotique par manipulation d'une séquence cible dans un locus d'intérêt génomique, de façon à induire un changement phénotypique dans la cellule, comprenant l'administration à ladite cellule postmitotique d'une composition d'origine non naturelle ou modifiée comprenant :

I. un polynucléotide comprenant :

(a) une séquence de guidage capable de s'hybrider à une séquence cible dans une cellule eucaryote postmitotique, et
(b) au moins une ou plusieurs séquences tracr mate,

II. une séquence polynucléotidique codant pour une enzyme CRISPR comprenant au moins une ou plusieurs séquences de localisation nucléaire, et
III. une séquence polynucléotidique comprenant une séquence tracr où, lorsqu'elle est transcrite, la séquence tracr mate s'hybride avec la séquence tracr et la séquence de guidage contrôle la liaison à une séquence spécifique d'un complexe CRISPR à la séquence cible, et

où le complexe CRISPR comprend l'enzyme CRISPR complexée avec (1) la séquence de guidage qui est hybridée à la séquence cible, et (2) la séquence tracr mate qui est hybridée à la séquence tracr, et la séquence polynucléotidique codant pour une enzyme CRISPR est un ADN ou un ARN.

**FIG. 1**

**FIG. 2A**

FIG. 2B

C

FIG. 2C

D

| 2xNLS-SpCas9 | + | + | + | + | + |
|---|---|---|---|---|---|
| SpRNAse III | − | + | + | − | + |
| short tracrRNA | − | + | − | + | + |
| DR-EMX1-DR | + | − | + | + | + |

684bp ►

367bp ►
317bp ►

indel (%):                          4.7    5.0

FIG. 2D

E

Target locus 5'-..AGCTGGAGGAGGAAGGGCCTGAGTCCGAGCAGAAGAAGAAGGGCTCCCAC..-3'
                   ||||||||||||||||||||                    ||||||||||
              3'-..TCGACCTCCTCCTTCCCGGACTCAGGCTCGTCTTCTTCTTCCCGAGGGTG..-5'
                                       ||||||||||||||||||||||
crRNA                              5'- GAGUCCGAGCAGAAGAAGAAGUUUUAGAGC..-3'

indel
AGCTGGAGGAGGAAGGGCCTGAGTCCGAGCAGAAGA--GAAGGGCTCCCAT

F

human *EMX1* protospacer target (mutation in 5 of 43 sequenced clones = 11.6%)

WT       5'-..CTGGAGGAGGAAGGGCCTGAGTCCGAGCAGAAG AAGAAGGGCTCCCATCACAT..-3'
Δ1          CTGGAGGAGGAAGGGCCTGAGTCCGAGCAGAAG--AGAAGGGCTCCCATCACAT
+1          CTGGAGGAGGAAGGGCCTGAGTCCGAGCAGAAGAAAGAAGGGCTCCCATCACAT
Δ3          CTGGAGGAGGAAGGGCCTGAGTCCGAGCAGAAG----AAGGGCTCCCATCACAT
m1, Δ6      CTGGAGGAGGAAGGGCCTGAGCCCGAGCAGAAG------GGCTCCCATCACAT

FIG. 2E-F

**A**

```
                                    protospacer (1)  ▼  PAM
human EMX1   5'-..CTGGAGGAGGAAGGGCCTGAGTCCGAGCAGAAGAAGAAGGGCTCCCAT..-3'
locus           |||||||||||||||||||                       ||||||||||
             3'-..GACCTCCTCCTTCCCGGACTCAGGCTCGTCTTCTTCTTCCCGAGGGTA..-5'
                                    |||||||||||||||||||||||
wt crRNA                         5'-GAGUCCGAGCAGAAGAAGAAGUUUUAGAGC..-3'

             m1   GAGUCCGAGCAGAAGAAGAU
             m2   GAGUCCGAGCAGAAGAAGUA
             m3   GAGUCCGAGCAGAAGAACAA
mismatch-    m4   GAGUCCGAGCAGAAGAUGAA
containing   m5   GAGUCCGAGCAGAAGUAGAA
guide        m7   GAGUCCGAGCAGAUGAAGAA
sequences    m9   GAGUCCGAGCACAAGAAGAA
             m11  GAGUCCGAGGAGAAGAAGAA
             m13  GAGUCCGUGCAGAAGAAGAA
             m15  GAGUCGGAGCAGAAGAAGAA
             m17  GAGACCGAGCAGAAGAAGAA
```

**B**

mismatched spacers

| | m17 | m15 | m13 | m11 | m9 | m7 | m5 | m4 | m3 | m2 | m1 | wt |
|---|---|---|---|---|---|---|---|---|---|---|---|---|
| indel (%) | 5.6 | 7.5 | 8.8 | | | | | | | | | 9.7 |

684bp ►
367bp ►
317bp ►

**C**

```
              left TALEN binding site   protospacer (1)  ▼  PAM
human        5'-..CTGGAGGAGGAAGGGCCTGAGTCCGAGCAGAAGAAGAAGGGCTCCCAT..-3'
EMX1            ||||||||||||||||||||||||||||||||||||||||||||||||
locus        3'-..GACCTCCTCCTTCCCGGACTCAGGCTCGTCTTCTTCTTCCCGAGGGTA..-5'
                                                     right TALEN binding site
```

**FIG. 3A-C**

FIG. 3D

FIG. 4A-B

**C**

human *EMX1* locus

HR Template

*Hind*III, *Nhe*I

200bp

**D**

| | | | |
|---|---|---|---|
| hSpCas9 | + | − | − |
| hSpCas9n | − | + | − |
| HR template | + | + | + |

2281bp ►

1189bp ►
1092bp ►

HR (%)    0.70    0.46

**E**

AATGACAAGCTTGCTAGCGGTGGG

*Hind*III    *Nhe*I

FIG. 4C-E

F  crRNA array design

EMX1(1)    PVALB(9)

DR | EMX1(1) | DR | PVALB(9) | DR

5'-...AAAACGGAAGGGCCTGAGTCCGAGCAGAAGAAGAAGTTT...AAACAGGGGCCGAGATTGGGTGTTCAGGGCAGAGGTTTT...-3'

|  | DR-*EMX1(1)*-DR-*PVALB(9)*-DR | | DR-*EMX1(1)*-DR | | DR-*PVALB(9)*-DR | | untransfected | |
|---|---|---|---|---|---|---|---|---|
| Surveyor target | EMX1 | PVALB | EMX1 | PVALB | EMX1 | PVALB | EMX1 | PVALB |

684bp
577bp

381bp
367bp
317bp

196bp

| indel (%) | 27 | 7.3 | 24 | | 15 | | | |

**FIG. 4F**

FIG. 4G

EP 3 011 032 B1

| Cas9 | target species | gene | protospacer ID | protospacer sequence (5' to 3') | PAM | strand | cell line tested | % indel (pre-crRNA + tracrRNA) | % indel (chimeric RNA) |
|---|---|---|---|---|---|---|---|---|---|
| S. pyogenes SF370 type II CRISPR | Homo sapiens | EMX1 | 1 | GGAAGGGCCTGAGTCCGAGCAGAAGAAGAA | GGG | + | 293FT | 20 ± 1.8 | 6.7 ± 0.82 |
| | | EMX1 | 2 | CATTGGAGGTGACATCGATGTCCTCCCCAT | TGG | – | 293FT | 2.1 ± 0.31 | N.D. |
| | | EMX1 | 3 | GGACATCGATGTCACCTCCAATGACTAGGG | TGG | + | 293FT | 14 ± 1.1 | N.D. |
| | | EMX1 | 4 | CATCGATGTCCTCCCCATTGGCCTGCTTCG | TGG | – | 293FT | 11 ± 1.7 | N.D. |
| | | EMX1 | 5 | TTCGTGGCAATGCGCCACCGGTTGATGTGA | TGG | – | 293FT | 4.3 ± 0.48 | 2.1 ± 0.51 |
| | | EMX1 | 6 | TCGTGGCAATGCGCCACCGGTTGATGTGAT | GGG | – | 293FT | 4.0 ± 0.68 | 0.41 ± 0.25 |
| | | EMX1 | 7 | TCCAGCTTCTGCCGTTTGTACTTTGTCCTC | CGG | – | 293FT | 1.5 ± 0.12 | N.D. |
| | | EMX1 | 8 | GGAGGGAGGGGCACAGATGAGAAACTCAGG | AGG | – | 293FT | 7.8 ± 0.83 | 2.3 ± 1.2 |
| | Homo sapiens | PVALB | 9 | AGGGGCCGAGATTGGGTGTTCAGGGCAGAG | AGG | + | 293FT | 21 ± 2.6 | 6.5 ± 0.32 |
| | | PVALB | 10 | ATGCAGGAGGGTGGCGAGAGGGGCCGAGAT | TGG | + | 293FT | N.D. | N.D. |
| | | PVALB | 11 | GGTGGCGAGAGGGGCCGAGATTGGGTGTTC | AGG | + | 293FT | N.D. | N.D. |
| | Mus musculus | Th | 12 | CAAGCACTGAGTGCCATTAGCTAAATGCAT | AGG | – | Neuro2A | 27 ± 4.3 | 4.1 ± 2.2 |
| | | Th | 13 | AATGCATAGGGTACCACCCACAGGTGCCAG | GGG | – | Neuro2A | 4.8 ± 1.2 | N.D. |
| | | Th | 14 | ACACACATGGAAAGCCTCTGGGCCAGGAA | AGG | + | Neuro2A | 11.3 ± 1.3 | N.D. |
| S. thermophilus LMD-9 CRISPR1 | Homo sapiens | EMX1 | 15 | GGAGGAGGTAGTATACAGAAACACAGAGAA | GTAGAAT | – | 293FT | 14 ± 0.88 | N.T. |
| | | EMX1 | 16 | AGAATGTAGAGGAGTCACAGAAACTCAGCA | CTAGAAA | – | 293FT | 7.8 ± 0.77 | N.T. |

FIG. 5

EP 3 011 032 B1

A

B

FIG. 6A-B

FIG. 6C

indel

rehybridize

surveyor nuclease

$a$    $b$

$c$

gel quantification

$$\% \text{ indel} = \left(1 - \sqrt{1 - (a + b)/(a + b + c)}\right) * 100$$

FIG. 7

A

U6 — EF1α FLAG NLS hSpCas9 NLS WPRE
3x

spacer sequence insertion site

BbsI   BbsI

spacer sequence insert

FIG. 8A-B

B

U6 — EF1α FLAG NLS hSpCas9 NLS WPRE
3x

guide sequence insertion site

BbsI   BbsI

guide sequence insert

**A**

Streptococcus pyogenes SF370 type II CRISPR
PAM occurence in human genome (NGG)

Median: 8bp
Mean: 12.7bp

**B**

Streptococcus thermophilus LMD-9 CRISPR1
PAM occurence in human genome (NNAGAAW)

Median: 65bp
Mean: 106.6bp

**C**

| Chr | NGG | | NNAGAAW | |
|---|---|---|---|---|
| | median | mean | median | mean |
| 1 | 7 | 12.8 | 67 | 115.8 |
| 2 | 8 | 12.7 | 64 | 100.8 |
| 3 | 8 | 13.0 | 63 | 98.5 |
| 4 | 9 | 14.0 | 61 | 94.5 |
| 5 | 8 | 13.1 | 63 | 97.9 |
| 6 | 8 | 13.1 | 63 | 98.5 |
| 7 | 8 | 12.4 | 64 | 102.9 |
| 8 | 8 | 12.8 | 64 | 100.9 |
| 9 | 7 | 13.9 | 65 | 120.5 |
| 10 | 7 | 12.1 | 66 | 107.0 |
| 11 | 7 | 12.0 | 65 | 105.8 |
| 12 | 8 | 12.4 | 65 | 103.5 |
| 13 | 8 | 13.6 | 62 | 94.6 |
| 14 | 8 | 12.0 | 65 | 101.5 |
| 15 | 7 | 11.5 | 68 | 107.7 |
| 16 | 7 | 11.7 | 74 | 136.8 |
| 17 | 6 | 10.3 | 76 | 127.9 |
| 18 | 8 | 13.4 | 63 | 101.8 |
| 19 | 6 | 9.4 | 82 | 145.4 |
| 20 | 7 | 11.1 | 72 | 121.8 |
| 21 | 7 | 13.4 | 64 | 111.4 |
| 22 | 6 | 9.2 | 85 | 140.3 |
| X | 8 | 13.2 | 63 | 99.0 |
| Y | 8 | 29.2 | 62 | 223.7 |

**FIG. 9A-C**

EP 3 011 032 B1

**A** *Streptococcus thermophilus* LMD-9 CRISPR1

**B**

**C**

FIG. 10A-C

FIG. 10D

FIG. 11A-C

**A**

**B**

FIG. 12A-B

FIG. 13A-B

FIG. 14

| Primer name | Assay | Genomic Target | Primer sequence |
|---|---|---|---|
| Sp-EMX1-F | SURVEYOR assay, sequencing | *EMX1* | AAAACCACCCTTCTCTCTGGC |
| Sp-EMX1-R | SURVEYOR assay, sequencing | *EMX1* | GGAGATTGGAGACACGGAGAG |
| Sp-PVALB-F | SURVEYOR assay, sequencing | *PVALB* | CTGGAAAGCCAATGCCTGAC |
| Sp-PVALB-R | SURVEYOR assay, sequencing | *PVALB* | GGCAGCAAACTCCTTGTCCT |
| Sp-Th-F | SURVEYOR assay, sequencing | *Th* | GTGCTTTGCAGAGGCCTACC |
| Sp-Th-R | SURVEYOR assay, sequencing | *Th* | CCTGGAGCGCATGCAGTAGT |
| St-EMX1-F | SURVEYOR assay, sequencing | *EMX1* | ACCTTCTGTGTTTCCACCATTC |
| St-EMX1-R | SURVEYOR assay, sequencing | *EMX1* | TTGGGGAGTGCACAGACTTC |
| Sp-EMX1-RFLP-F | RFLP, sequencing | *EMX1* | GGCTCCCTGGGTTCAAAGTA |
| Sp-EMX1-RFLP-R | RFLP, sequencing | *EMX1* | AGAGGGGTCTGGATGTCGTAA |
| Pb_EMX1_spl | Northern Blot Probe | Not applicable | TAGCTCTAAAACTTCTTCTTCTGCTCGGAC |
| Pb_tracrRNA | Northern Blot Probe | Not applicable | CTAGCCTTATTTTAACTTGCTATGCTGTTT |

**FIG. 15**

**FIG. 16A**

FIG. 16B

FIG. 16C

FIG. 17A-B

FIG. 18

EP 3 011 032 B1

FIG. 19A

251

**FIG. 19B**

FIG. 19C

FIG. 19D

FIG. 20A

FIG. 20B

FIG. 20C

FIG. 20D

FIG. 20E

gi|225537057|ref|NP_687908.1| , hypothetical protein SAG0894 (Streptococcus agalactiae 2603V/R)

gi|76788438|ref|YP_329639.1| , hypothetical protein SAK_1017 (Streptococcus agalactiae A909)

gi|25010965|ref|NP_735360.1| , hypothetical protein gbs0911 (Streptococcus agalactiae NEM316)

gi|24379809|ref|NP_721784.1| , hypothetical protein SMU_1405c (Streptococcus mutans UA159)

gi|290580220|ref|YP_003484612.1| , hypothetical protein SmuNN2025_0694 (Streptococcus mutans NN2025)

gi|306833855|ref|ZP_07466980.1| , csn1 family CRISPR-associated protein (Streptococcus bovis ATCC 700338)

gi|325978669|ref|YP_004288385.1| , CRISPR-associated protein (Streptococcus gallolyticus subsp. gallolyticus ATCC BAA-2069)

gi|288905639|ref|YP_003430861.1| , CRISPR-associated protein (Streptococcus gallolyticus UCN34)

gi|320547102|ref|ZP_08041398.1| , csn1 family CRISPR-associated protein (Streptococcus equinus ATCC 9812)

gi|315223162|ref|ZP_07865023.1| , CRISPR-associated protein, Csn1 family (Streptococcus anginosus F0211)

gi|357636408|ref|ZP_09134281.1| , CRISPR-associated protein Cas9/Csn1, subtype II/NMEMI (Streptococcus macacae NCTC 11558)

gi|313890160|ref|ZP_07823795.1| , CRISPR-associated protein, Csn1 family (Streptococcus pseudoporcinus SPIN 20026)

gi|195978435|ref|YP_002123879.1| , CRISPR-Associated Protein Csn1 (Streptococcus equi subsp. zooepidemicus MGCS10565)

gi|94994317|ref|YP_602415.1| , hypothetical protein MGAS10750_Spy0921 (Streptococcus pyogenes MGAS10750)

gi|251782637|ref|YP_002996940.1| , hypothetical protein SDEG_1231 (Streptococcus dysgalactiae subsp. equisimilis GGS_124)

gi|219102213|ref|NP_664481.1| , hypothetical protein SpyM3_0677 (Streptococcus pyogenes MGAS315)

gi|28896088|ref|NP_802438.1| , hypothetical protein SPs1176 (Streptococcus pyogenes SSI-1)

gi|209559356|ref|YP_002285828.1| , hypothetical protein Spy49_0823 (Streptococcus pyogenes NZ131)

gi|15675041|ref|NP_269215.1| , hypothetical protein SPy_1046 (Streptococcus pyogenes M1 GAS)

gi|71910582|ref|YP_282132.1| , hypothetical protein M5005_Spy_0769 (Streptococcus pyogenes MGAS5005)

gi|94990395|ref|YP_598495.1| , putative cytoplasmic protein (Streptococcus pyogenes MGAS10270)

gi|94988516|ref|YP_596617.1| , cytoplasmic protein (Streptococcus pyogenes MGAS9429)

gi|94992340|ref|YP_600439.1| , putative cytoplasmic protein (Streptococcus pyogenes MGAS2096)

gi|71903413|ref|YP_280216.1| , cytoplasmic protein (Streptococcus pyogenes MGAS6180)

**FIG. 20F**

a

FIG. 21A

FIG. 21B

EP 3 011 032 B1

C

hEMX1 locus

genomic target ▼ PAM

5'~..CAGAAGAAGAAGGGC...CCAATGGGGAGGACATCGATGTCACCTCCAATGACTAGGGTGGTGGGCAAC...CTCTGGCCACTCCCT..~3'
         |||||||||||||||    ||||||||||||||||||||||||||||||||||||||||||||||||||||||    |||||||||||||||
3'~..GTCTTCTTCTTCCCG...GGTTACCCCTCCTGTAGCTACAGTGGAGGTTACTGATCCCACCACCCGTTG...GAGACCGGTGAGGGA..~5'

sense      5'~ CAGAAGAAGAAGGGC...ACATCGATGTCACCTCCAATGACAAGCTTGCTAGCGGTGGGCAACCACAAAC...CTCTGGCCACTCCCT ~3'
                                                        HindIII    NheI

antisense  3'~ GTCTTCTTCTTCCCG...TGTAGCTACAGTGGAGGTTACTGTTCGAACGATCGCCACCCGTTGGTGTTTG...GAGACCGGTGAGGGA ~5'
                                                        HindIII    NheI

FIG. 21C

EP 3 011 032 B1

FIG. 21D

FIG. 22A

FIG. 22B

FIG. 23

SpCas9 mutation positions

hSpCas9

5' ATGGACAAGAAGTACAGCATCGGCCTGGACATCGGCACCAACTCTGTGGGCTGGGCCGTG

60

hSpCas9

D10

RuvCI

| M | D | K | K | Y | S | I | G | L | D | I | G | T | N | S | V | G | W | A | V |
|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 |

5' ATCACCGACGAGTACAAGGTGCCCAGCAAGAAATTCAAGGTGCTGGGCAACACCGACCGG

120

hSpCas9

R21

| I | T | D | E | Y | K | V | P | S | K | K | F | K | V | L | G | N | T | D | R |
|---|---|---|---|---|---|---|---|---|---|---|---|---|---|---|---|---|---|---|---|
| 21 | 22 | 23 | 24 | 25 | 26 | 27 | 28 | 29 | 30 | 31 | 32 | 33 | 34 | 35 | 36 | 37 | 38 | 39 | 40 |

5' CACAGCATCAAGAAGAACCTGATCGGAGCCCTGCTGTTCGACAGCGGCGAAACAGCCGAG

180

hSpCas9

| H | S | I | K | K | N | L | I | G | A | L | L | F | D | S | G | E | T | A | E |
|---|---|---|---|---|---|---|---|---|---|---|---|---|---|---|---|---|---|---|---|
| 41 | 42 | 43 | 44 | 45 | 46 | 47 | 48 | 49 | 50 | 51 | 52 | 53 | 54 | 55 | 56 | 57 | 58 | 59 | 60 |

5' GCCACCCGGCTGAAGAGAACCGCCAGAAGAAGATACACCAGACGGAAGAACCGGATCTGC

240

hSpCas9

| A | T | R | L | K | R | T | A | R | R | R | Y | T | R | R | K | N | R | I | C |
|---|---|---|---|---|---|---|---|---|---|---|---|---|---|---|---|---|---|---|---|
| 61 | 62 | 63 | 64 | 65 | 66 | 67 | 68 | 69 | 70 | 71 | 72 | 73 | 74 | 75 | 76 | 77 | 78 | 79 | 80 |

5' TATCTGCAAGAGATCTTCAGCAACGAGATGGCCAAGGTGGACGACAGCTTCTTCCACAGA

300

hSpCas9

| Y | L | Q | E | I | F | S | N | E | M | A | K | V | D | D | S | F | F | H | R |
|---|---|---|---|---|---|---|---|---|---|---|---|---|---|---|---|---|---|---|---|
| 81 | 82 | 83 | 84 | 85 | 86 | 87 | 88 | 89 | 90 | 91 | 92 | 93 | 94 | 95 | 96 | 97 | 98 | 99 | 100 |

FIG. 24A

hSpCas9

5' CTGGAAGAGTCCTTCCTGGTGGAAGAGGATAAGAAGCACGAGCGGCACCCCATCTTCGGC

360

hSpCas9

L  E  E  S  F  L  V  E  E  D  K  K  H  E  R  H  P  I  F  G
101 102 103 104 105 106 107 108 109 110 111 112 113 114 115 116 117 118 119 120

5' AACATCGTGGACGAGGTGGCCTACCACGAGAAGTACCCCACCATCTACCACCTGAGAAAG

420

hSpCas9

N  I  V  D  E  V  A  Y  H  E  K  Y  P  T  I  Y  H  L  R  K
121 122 123 124 125 126 127 128 129 130 131 132 133 134 135 136 137 138 139 140

5' AAACTGGTGGACAGCACCGACAAGGCCGACCTGCGGCTGATCTATCTGGCCCTGGCCCAC

480

hSpCas9

K  L  V  D  S  T  D  K  A  D  L  R  L  I  Y  L  A  L  A  H
141 142 143 144 145 146 147 148 149 150 151 152 153 154 155 156 157 158 159 160

5' ATGATCAAGTTCCGGGGCCACTTCCTGATCGAGGGCGACCTGAACCCCGACAACAGCGAC

540

hSpCas9

M  I  K  F  R  G  H  F  L  I  E  G  D  L  N  P  D  N  S  D
161 162 163 164 165 166 167 168 169 170 171 172 173 174 175 176 177 178 179 180

5' GTGGACAAGCTGTTCATCCAGCTGGTGCAGACCTACAACCAGCTGTTCGAGGAAAACCCC

600

hSpCas9

V  D  K  L  F  I  Q  L  V  Q  T  Y  N  Q  L  F  E  E  N  P
181 182 183 184 185 186 187 188 189 190 191 192 193 194 195 196 197 198 199 200

5' ATCAACGCCAGCGGCGTGGACGCCAAGGCCATCCTGTCTGCCAGACTGAGCAAGAGCAGA

660

hSpCas9

I  N  A  S  G  V  D  A  K  A  I  L  S  A  R  L  S  K  S  R
201 202 203 204 205 206 207 208 209 210 211 212 213 214 215 216 217 218 219 220

FIG. 24B

269

hSpCas9

5'  CGGCTGGAAAATCTGATCGCCCAGCTGCCCGGCGAGAAGAAGAATGGCCTGTTCGGCAAC

720

hSpCas9

R   L   E   N   L   I   A   Q   L   P   G   E   K   K   N   G   L   F   G   N

221 222 223 224 225 226 227 228 229 230 231 232 233 234 235 236 237 238 239 240

5'  CTGATTGCCCTGAGCCTGGGCCTGACCCCCAACTTCAAGAGCAACTTCGACCTGGCCGAG

780

hSpCas9

L   I   A   L   S   L   G   L   T   P   N   F   K   S   N   F   D   L   A   E

241 242 243 244 245 246 247 248 249 250 251 252 253 254 255 256 257 258 259 260

5'  GATGCCAAACTGCAGCTGAGCAAGGACACCTACGACGACGACCTGGACAACCTGCTGGCC

840

hSpCas9

D   A   K   L   Q   L   S   K   D   T   Y   D   D   D   L   D   N   L   L   A

261 262 263 264 265 266 267 268 269 270 271 272 273 274 275 276 277 278 279 280

5'  CAGATCGGCGACCAGTACGCCGACCTGTTTCTGGCCGCCAAGAACCTGTCCGACGCCATC

900

hSpCas9

Q   I   G   D   Q   Y   A   D   L   F   L   A   A   K   N   L   S   D   A   I

281 282 283 284 285 286 287 288 289 290 291 292 293 294 295 296 297 298 299 300

5'  CTGCTGAGCGACATCCTGAGAGTGAACACCGAGATCACCAAGGCCCCCCTGAGCGCCTCT

960

hSpCas9

L   L   S   D   I   L   R   V   N   T   E   I   T   K   A   P   L   S   A   S

301 302 303 304 305 306 307 308 309 310 311 312 313 314 315 316 317 318 319 320

5'  ATGATCAAGAGATACGACGAGCACCACCAGGACCTGACCCTGCTGAAAGCTCTCGTGCGG

1020

hSpCas9

M   I   K   R   Y   D   E   H   H   Q   D   L   T   L   L   K   A   L   V   R

321 322 323 324 325 326 327 328 329 330 331 332 333 334 335 336 337 338 339 340

FIG. 24C

hSpCas9

5' CAGCAGCTGCCTGAGAAGTACAAAGAGATTTTCTTCGACCAGAGCAAGAACGGCTACGCC
1080
hSpCas9
Q Q L P E K Y K E I F F D Q S K N G Y A
341 342 343 344 345 346 347 348 349 350 351 352 353 354 355 356 357 358 359 360

5' GGCTACATTGACGGCGGAGCCAGCCAGGAAGAGTTCTACAAGTTCATCAAGCCCATCCTG
1140
hSpCas9
G Y I D G G A S Q E E F Y K F I K P I L
361 362 363 364 365 366 367 368 369 370 371 372 373 374 375 376 377 378 379 380

5' GAAAAGATGGACGGCACCGAGGAACTGCTCGTGAAGCTGAACAGAGAGGACCTGCTGCGG
1200
hSpCas9
E K M D G T E E L L V K L N R E D L L R
381 382 383 384 385 386 387 388 389 390 391 392 393 394 395 396 397 398 399 400

5' AAGCAGCGGACCTTCGACAACGGCAGCATCCCCCACCAGATCCACCTGGGAGAGCTGCAC
1260
hSpCas9
K Q R T F D N G S I P H Q I H L G E L H
401 402 403 404 405 406 407 408 409 410 411 412 413 414 415 416 417 418 419 420

5' GCCATTCTGCGGCGGCAGGAAGATTTTTACCCATTCCTGAAGGACAACCGGGAAAAGATC
1320
hSpCas9
A I L R R Q E D F Y P F L K D N R E K I
421 422 423 424 425 426 427 428 429 430 431 432 433 434 435 436 437 438 439 440

5' GAGAAGATCCTGACCTTCCGCATCCCCTACTACGTGGGCCCTCTGGCCAGGGGAAACAGC
1380
hSpCas9
E K I L T F R I P Y Y V G P L A R G N S
441 442 443 444 445 446 447 448 449 450 451 452 453 454 455 456 457 458 459 460

FIG. 24D

hSpCas9

```
5'  AGATTCGCCTGGATGACCAGAAAGAGCGAGGAAACCATCACCCCCCTGGAACTTCGAGGAA
                                                                          1440
            hSpCas9
    R   F   A   W   M   T   R   K   S   E   E   T   I   T   P   W   N   F   E   E
    461 462 463 464 465 466 467 468 469 470 471 472 473 474 475 476 477 478 479 480


5'  GTGGTGGACAAGGGCGCTTCCGCCCAGAGCTTCATCGAGCGGATGACCAACTTCGATAAG
                                                                          1500
            hSpCas9
    V   V   D   K   G   A   S   A   Q   S   F   I   E   R   M   T   N   F   D   K
    481 482 483 484 485 486 487 488 489 490 491 492 493 494 495 496 497 498 499 500


5'  AACCTGCCCAACGAGAAGGTGCTGCCCAAGCACAGCCTGCTGTACGAGTACTTCACCGTG
                                                                          1560
            hSpCas9
    N   L   P   N   E   K   V   L   P   K   H   S   L   L   Y   E   Y   F   T   V
    501 502 503 504 505 506 507 508 509 510 511 512 513 514 515 516 517 518 519 520


5'  TATAACGAGCTGACCAAAGTGAAATACGTGACCGAGGGAATGAGAAAGCCCGCCTTTCTG
                                                                          1620
            hSpCas9
    Y   N   E   L   T   K   V   K   Y   V   T   E   G   M   R   K   P   A   F   L
    521 522 523 524 525 526 527 528 529 530 531 532 533 534 535 536 537 538 539 540


5'  AGCGGCGAGCAGAAAAAGGCCATCGTGGACCTGCTGTTCAAGACCAACCGGAAAGTGACC
                                                                          1680
            hSpCas9
    S   G   E   Q   K   K   A   I   V   D   L   L   F   K   T   N   R   K   V   T
    541 542 543 544 545 546 547 548 549 550 551 552 553 554 555 556 557 558 559 560


5'  GTGAAGCAGCTGAAAGAGGACTACTTCAAGAAAATCGAGTGCTTCGACTCCGTGGAAATC
                                                                          1740
            hSpCas9
    V   K   Q   L   K   E   D   Y   F   K   K   I   E   C   F   D   S   V   E   I
    561 562 563 564 565 566 567 568 569 570 571 572 573 574 575 576 577 578 579 580
```

FIG. 24E

272

hSpCas9

5' TCCGGCGTGGAAGATCGGTTCAACGCCTCCCTGGGCACATACCACGATCTGCTGAAAATT
1800
hSpCas9
S G V E D R F N A S L G T Y H D L L K I
581 582 583 584 585 586 587 588 589 590 591 592 593 594 595 596 597 598 599 600

5' ATCAAGGACAAGGACTTCCTGGACAATGAGGAAAACGAGGACATTCTGGAAGATATCGTG
1860
hSpCas9
I K D K D F L D N E E N E D I L E D I V
601 602 603 604 605 606 607 608 609 610 611 612 613 614 615 616 617 618 619 620

5' CTGACCCTGACACTGTTTGAGGACAGAGAGATGATCGAGGAACGGCTGAAAACCTATGCC
1920
hSpCas9
L T L T L F E D R E M I E E R L K T Y A
621 622 623 624 625 626 627 628 629 630 631 632 633 634 635 636 637 638 639 640

5' CACCTGTTCGACGACAAAGTGATGAAGCAGCTGAAGCGGCGGAGATACACCGGCTGGGGC
1980
hSpCas9
H L F D D K V M K Q L K R R R Y T G W G
641 642 643 644 645 646 647 648 649 650 651 652 653 654 655 656 657 658 659 660

5' AGGCTGAGCCGGAAGCTGATCAACGGCATCCGGGACAAGCAGTCCGGCAAGACAATCCTG
2040
hSpCas9
R L S R K L I N G I R D K Q S G K T I L
661 662 663 664 665 666 667 668 669 670 671 672 673 674 675 676 677 678 679 680

5' GATTTCCTGAAGTCCGACGGCTTCGCCAACAGAAACTTCATGCAGCTGATCCACGACGAC
2100
hSpCas9
D F L K S D G F A N R N F M Q L I H D D
681 682 683 684 685 686 687 688 689 690 691 692 693 694 695 696 697 698 699 700

# FIG. 24F

hSpCas9

5'  AGCCTGACCTTTAAAGAGGACATCCAGAAAGCCCAGGTGTCTGGCCAGGGCGATAGCCTG
                                                                          2160

hSpCas9

S   L   T   F   K   E   D   I   Q   K   A   Q   V   S   G   Q   G   D   S   L
701 702 703 704 705 706 707 708 709 710 711 712 713 714 715 716 717 718 719 720

5'  CACGAGCACATTGCCAATCTGGCCGGCAGCCCCGCCATTAAGAAGGGCATCCTGCAGACA
                                                                          2220

hSpCas9

H   E   H   I   A   N   L   A   G   S   P   A   I   K   K   G   I   L   Q   T
721 722 723 724 725 726 727 728 729 730 731 732 733 734 735 736 737 738 739 740

5'  GTGAAGGTGGTGGACGAGCTCGTGAAAGTGATGGGCCGGCACAAGCCCGAGAACATCGTG
                                                                          2280

hSpCas9

RuvC II

V   K   V   V   D   E   L   V   K   V   M   G   R   H   K   P   E   N   I   V
741 742 743 744 745 746 747 748 749 750 751 752 753 754 755 756 757 758 759 760

5'  ATCGCCATGGCCAGAGAGAACCAGACCACCCAGAAGGGACAGAAGAACAGCCGCGAGAGA
                                                                          2340

hSpCas9

RuvC II

E

I   A   M   A   R   E   N   Q   T   T   Q   K   G   Q   K   N   S   R   E   R
761 762 763 764 765 766 767 768 769 770 771 772 773 774 775 776 777 778 779 780

5'  ATGAAGCGGATCGAAGAGGGCATCAAAGAGCTGGGCAGCCAGATCCTGAAAGAACACCCC
                                                                          2400

hSpCas9

M   K   R   I   E   E   G   I   K   E   L   G   S   Q   I   L   K   E   H   P
781 782 783 784 785 786 787 788 789 790 791 792 793 794 795 796 797 798 799 800

FIG. 24G

274

hSpCas9

5' GTGGAAAACACCCAGCTGCAGAACGAGAAGCTGTACCTGTACTACCTGCAGAATGGGCGG
2460

hSpCas9

V E N T Q L Q N E K L Y L Y Y L Q N G R

801 802 803 804 805 806 807 808 809 810 811 812 813 814 815 816 817 818 819 820

5' GATATGTACGTGGACCAGGAACTGGACATCAACCGGCTGTCTGACTACGATGTGGACGCC
2520

hSpCas9

HNH

H...

D M Y V D Q E L D I N R L S D Y D V D A

821 822 823 824 825 826 827 828 829 830 831 832 833 834 835 836 837 838 839 840

5' ATCGTGCCTCAGAGCTTTCTGAAGGACGACTCCATCGACGCCAAGGTGCTGACCAGAAGC
2580

hSpCas9

HNH

N...

I V P Q S F L K D D S I D A K V L T R S

841 842 843 844 845 846 847 848 849 850 851 852 853 854 855 856 857 858 859 860

5' GACAAGGCCCGGGGCAAGAGCGACAACGTGCCCTCCGAAGAGGTCGTGAAGAAGATGAAG
2640

hSpCas9

HNH

N...

D K A R G K S D N V P S E E V V K K M K

861 862 863 864 865 866 867 868 869 870 871 872 873 874 875 876 877 878 879 880

5' AACTACTGGCGGCAGCTGCTGAACGCCAAGCTGATTACCCAGAGAAAGTTCGACAATCTG
2700

hSpCas9

N Y W R Q L L N A K L I T Q R K F D N L

881 882 883 884 885 886 887 888 889 890 891 892 893 894 895 896 897 898 899 900

FIG. 24H

hSpCas9

```
5'   ACCAAGGCCGAGAGAGGCGGCCTGAGCGAACTGGATAAGGCCGGCTTCATCAAGAGACAG
     +++++|+++++|+++++|+++++|+++++|+++++|+++++|+++++|+++++|+++++|   2760
```
hSpCas9
```
     T   K   A   E   R   G   G   L   S   E   L   D   K   A   G   F   I   K   R   Q
     901 902 903 904 905 906 907 908 909 910 911 912 913 914 915 916 917 918 919 920
```

```
5'   CTGGTGGAAACCCGGCAGATCACAAAGCACGTGGCACAGATCCTGGACTCCCGGATGAAC
     +++++|+++++|+++++|+++++|+++++|+++++|+++++|+++++|+++++|+++++|   2820
```
hSpCas9
```
     L   V   E   T   R   Q   I   T   K   H   V   A   Q   I   L   D   S   R   M   N
     921 922 923 924 925 926 927 928 929 930 931 932 933 934 935 936 937 938 939 940
```

```
5'   ACTAAGTACGACGAGAATGACAAGCTGATCCGGGAAGTGAAAGTGATCACCCTGAAGTCC
     +++++|+++++|+++++|+++++|+++++|+++++|+++++|+++++|+++++|+++++|   2880
```
hSpCas9
```
     T   K   Y   D   E   N   D   K   L   I   R   E   V   K   V   I   T   L   K   S
     941 942 943 944 945 946 947 948 949 950 951 952 953 954 955 956 957 958 959 960
```

```
5'   AAGCTGGTGTCCGATTTCCGGAAGGATTTCCAGTTTTACAAAGTGCGCGAGATCAACAAC
     +++++|+++++|+++++|+++++|+++++|+++++|+++++|+++++|+++++|+++++|   2940
```
hSpCas9
```
     K   L   V   S   D   F   R   K   D   F   Q   F   Y   K   V   R   E   I   N   N
     961 962 963 964 965 966 967 968 969 970 971 972 973 974 975 976 977 978 979 980
```

```
5'   TACCACCACGCCCACGCCGCCTACCTGAACGCCGTCGTGGGAACCGCCCTGATCAAAAAG
     +++++|+++++|+++++|+++++|+++++|+++++|+++++|+++++|+++++|+++++|   3000
```
hSpCas9
RuvC III
```
     Y   H   H   A   H   A   A   Y   L   N   A   V   V   G   T   A   L   I   K   K
     981 982 983 984 985 986 987 988 989 990 991 992 993 994 995 996 997 998 999 1000
```

FIG. 24I

hSpCas9

5′ TACCCTAAGCTGGAAAGCGAGTTCGTGTACGGCGACTACAAGGTGTACGACGTGCGGAAG
                                                                              3060

hSpCas9
Y   P   K   L   E   S   E   F   V   Y   G   D   Y   K   V   Y   D   V   R   K

5′ ATGATCGCCAAGAGCGAGCAGGAAATCGGCAAGGCTACCGCCAAGTACTTCTTCTACAGC
                                                                              3120

hSpCas9
M   I   A   K   S   E   Q   E   I   G   K   A   T   A   K   Y   F   F   Y   S

5′ AACATCATGAACTTTTTCAAGACCGAGATTACCCTGGCCAACGGCGAGATCCGGAAGCGG
                                                                              3180

hSpCas9
N   I   M   N   F   F   K   T   E   I   T   L   A   N   G   E   I   R   K   R

5′ CCTCTGATCGAGACAAACGGCGAAACCGGGGAGATCGTGTGGGATAAGGGCCGGGATTTT
                                                                              3240

hSpCas9
P   L   I   E   T   N   G   E   T   G   E   I   V   W   D   K   G   R   D   F

5′ GCCACCGTGCGGAAAGTGCTGAGCATGCCCCAAGTGAATATCGTGAAAAAGACCGAGGTG
                                                                              3300

hSpCas9
A   T   V   R   K   V   L   S   M   P   Q   V   N   I   V   K   K   T   E   V

5′ CAGACAGGCGGCTTCAGCAAAGAGTCTATCCTGCCCAAGAGGAACAGCGATAAGCTGATC
                                                                              3360

hSpCas9
Q   T   G   G   F   S   K   E   S   I   L   P   K   R   N   S   D   K   L   I

FIG. 24J

hSpCas9

5' GCCAGAAAGAAGGACTGGGACCCTAAGAAGTACGGCGGCTTCGACAGCCCCACCGTGGCC
+++++|+++++|+++++|+++++|+++++|+++++|+++++|+++++|+++++|+++++| 3420
hSpCas9
A R K K D W D P K K Y G G F D S P T V A

5' TATTCTGTGCTGGTGGTGGCCAAAGTGGAAAAGGGCAAGTCCAAGAAACTGAAGAGTGTG
+++++|+++++|+++++|+++++|+++++|+++++|+++++|+++++|+++++|+++++| 3480
hSpCas9
Y S V L V V A K V E K G K S K K L K S V

5' AAAGAGCTGCTGGGGATCACCATCATGGAAAGAAGCAGCTTCGAGAAGAATCCCATCGAC
+++++|+++++|+++++|+++++|+++++|+++++|+++++|+++++|+++++|+++++| 3540
hSpCas9
K E L L G I T I M E R S S F E K N P I D

5' TTTCTGGAAGCCAAGGGCTACAAAGAAGTGAAAAAGGACCTGATCATCAAGCTGCCTAAG
+++++|+++++|+++++|+++++|+++++|+++++|+++++|+++++|+++++|+++++| 3600
hSpCas9
F L E A K G Y K E V K K D L I I K L P K

5' TACTCCCTGTTCGAGCTGGAAAACGGTCGGAAGAGAATGCTGGCTTCTGCTGGCGAACTG
+++++|+++++|+++++|+++++|+++++|+++++|+++++|+++++|+++++|+++++| 3660
hSpCas9
Y S L F E L E N G R K R M L A S A G E L

5' CAGAAGGGAAACGAACTGGCCCTGCCCTCCAAATATGTGAACTTCCTGTACCTGGCCAGC
+++++|+++++|+++++|+++++|+++++|+++++|+++++|+++++|+++++|+++++| 3720
hSpCas9
Q K G N E L A L P S K Y V N F L Y L A S

FIG. 24K

hSpCas9

5'  CACTATGAGAAGCTGAAGGGCTCCCCCGAGGATAATGAGCAGAAACAGCTGTTTGTGGAA  3780

hSpCas9

H Y E K L K G S P E D N E Q K Q L F V E
1241 1242 1243 1244 1245 1246 1247 1248 1249 1250 1251 1252 1253 1254 1255 1256 1257 1258 1259 1260

5'  CAGCACAAGCACTACCTGGACGAGATCATCGAGCAGATCAGCGAGTTCTCCAAGAGAGTG  3840

hSpCas9

Q H K H Y L D E I I E Q I S E F S K R V
1261 1262 1263 1264 1265 1266 1267 1268 1269 1270 1271 1272 1273 1274 1275 1276 1277 1278 1279 1280

5'  ATCCTGGCCGACGCTAATCTGGACAAAGTGCTGTCCGCCTACAACAAGCACCGGGATAAG  3900

hSpCas9

I L A D A N L D K V L S A Y N K H R D K
1281 1282 1283 1284 1285 1286 1287 1288 1289 1290 1291 1292 1293 1294 1295 1296 1297 1298 1299 1300

5'  CCCATCAGAGAGCAGGCCGAGAATATCATCCACCTGTTTACCCTGACCAATCTGGGAGCC  3960

hSpCas9

P I R E Q A E N I I H L F T L T N L G A
1301 1302 1303 1304 1305 1306 1307 1308 1309 1310 1311 1312 1313 1314 1315 1316 1317 1318 1319 1320

5'  CCTGCCGCCTTCAAGTACTTTGACACCACCATCGACCGGAAGAGGTACACCAGCACCAAA  4020

hSpCas9

P A A F K Y F D T T I D R K R Y T S T K
1321 1322 1323 1324 1325 1326 1327 1328 1329 1330 1331 1332 1333 1334 1335 1336 1337 1338 1339 1340

5'  GAGGTGCTGGACGCTCACCCTGATCCACCAGAGCATCACCGGCCTGTACGAGACACCGATC  4080

hSpCas9

E V L D A T L I H Q S I T G L Y E T R I
1341 1342 1343 1344 1345 1346 1347 1348 1349 1350 1351 1352 1353 1354 1355 1356 1357 1358 1359 1360

FIG. 24L

hSpCas9

FIG. 24M

Conditional Cas9, Rosa26 targeting vector map

FIG. 25A

EP 3 011 032 B1

Constitutive Cas9, Rosa26 targeting vector map

FIG. 25B

282

FIG. 26

Cas9 Expression in Mouse Hippocampus (AAV)

Cas9 Expression in Mouse Cortex (AAV)

FIG. 27

FIG. 28

FIG. 29

FIG. 30A-C

# Repair Strategy for Cystic Fibrosis deltaF508 Mutation

FIG. 31

A

GAA repeat expansion in *FXN* intron 1

Transcription repression likely due to aberrant
DNA structure or recruitment of heterchromatin
binding proteins to long GAA repeats

B

FIG. 32

FIG. 33

FIG. 34

FIG. 35

FIG. 36

FIG. 37

FIG. 38

# Expression of SpCas9 & SaCas9 in N2a cells

FIG. 39

FIG. 40

CA1 region of hippocampus

HA DAPI

no primary Ab

FIG. 41

FIG. 42

FIG. 43

FIG. 44

FIG. 45

FIG. 46

# SpCas9 *in vivo: Mecp2*

FIG. 47

Purification of cell nuclei from brain

FIG. 48

FIG. 49

FIG. 50

| | pattern | overhang |
|---|---|---|
| 1 | CCnNNNNNNNNNNNNNNNNNnnnnnnnnnnNNNNNNNNNNNNNNNnGG | 38 |
| 2 | CCnNNNNNNNNNNNNNNNNnnnnnnnNNNNNNNNNNNNNNnGG | 34 |
| 3 | CCnNNNNNNNNNNNNNnnnnnNNNNNNNNNNNNNnGG | 30 |
| 4 | CCnNNNNNNNNNNNNNNNNNNNNNNNNNNNnGG | 26 |
| 5 | CCnNNNNNNNNNNNNNNNNNNNNNnGG | 22 |
| 6 | CCnNNNNNNNNNNNNNNNNNnGG | 18 |
| 7 | CCnNNNNNNNNNNNNNNnGG | 14 |
| 8 | CCnNNNNNNNNNNNnGG | 10 |
| 9 | CCnNNNNNNNNNnGG | 6 |
| 10 | CCnNNNNNNNNnGG | 4 |
| 11 | CCnNNNNNNNnGG | 3 |
| 12 | CCnNNNNNNnGG | 2 |
| 13 | CCnNNNNNNnGG | 1 |
| 14 | CCnNNNNNNnGG | 0 |
| 15 | CCnNNNNnGG | 1 |
| 16 | CCnNNNNnGG | 2 |
| 17 | CCnNNNnGG | 3 |
| 18 | CCnnGG | 4 |
| 19 | CCGG | 8 |
| 20 | GGCC | 10 |
| 21 | GGNNCC | 12 |
| 22 | GGNNNCC | 14 |
| 23 | GGNNNNCC | 16 |
| 24 | GGNNNNNNNNCC | 20 |

Pattern 1

Pattern 2

Pattern 3

Pattern 4

FIG. 51

FIG. 52

FIG. 53

| lane # | Cas9 to use | Left gRNA | Right gRNA | Overhang (D10A) - number of bases protruding from 5' |
|---|---|---|---|---|
| 1 | D10A | left 23 | left 12 | -36 |
| 2 | D10A | right 4 | left 9 | -25 |
| 3 | D10A | left 23 | right 23 | -16 |
| 4 | D10A | right 7 | left 10 | -15 |
| 5 | D10A | right 16 | left 3 | -8 |
| 6 | D10A | right 22 | right 6 | 26 |
| 7 | D10A | left 12 | right 16 | 31 |
| 8 | D10A | left 12 | right 13 | 34 |
| 9 | D10A | left 10 | right 1 | 38 |
| 10 | D10A | right 23 | right 16 | 51 |
| 11 | D10A | right 23 | right 13 | 54 |
| 12 | D10A | left 3 | right 7 | 57 |
| 13 | D10A | left 12 | right 4 | 65 |
| 14 | D10A | left 12 | right 3 | 69 |
| 15 | D10A | left 3 | right 10 | 76 |
| 16 | D10A | right 23 | right 4 | 85 |
| 17 | D10A | left 12 | right 9 | 95 |
| 18 | D10A | left 12 | right 10 | 115 |
| 19 | D10A | right 23 | right 10 | 135 |
| 20 | D10A | left 12 | right 2 | 145 |
| 21 | D10A | left 12 | left 22 | 181 |
| 22 | D10A | right 23 | left 22 | 201 |
| 23 | D10A | left 12 | right 6 | 222 |
| 24 | D10A | right 23 | right 6 | 242 |

FIG. 54

FIG. 55

a

b

ApoB-T1 = Target 1; ApoB-T2 = Target 2, etc.

FIG. 56

TBG-SaCas9-U6-sgRNA(Apob-T1)
4wk post injection

TBG-SaCas9-U6-sgRNA(Apob-T2)
4wk post injection

TBG-SaCas9-U6-sgRNA(Apob-T3)
4wk post injection

Wildtype (saline control)
4wk post injection

FIG. 57

FIG. 58

FIG. 59

FIG. 60

FIG. 61

FIG. 62

FIG. 63

FIG. 64

FIG. 65

FIG. 66

FIG. 67

a

| | Mecp2 target sequence | PAM |
|---|---|---|
| Target 1 | CTGGGAGAGGGAGCCCCTCC | AGG |
| Target 2 | AAAGGTGGGAGACACCTCCT | TGG |
| Target 3 | TCCAACCTTCAGGCAAGGTG | GGG |
| Target 4 | AGGAAGTCTGGCCGATCTGC | TGG |
| Target 5 | CCATTCTGCAGAGCCAGCAG | AGG |
| Target 6 | CTCTGAGGCCCTGGAGATCC | TGG |

b

| indel (%) | 5 | 21 | N.D. | N.D. | N.D. | N.D. | 23 | 13 | N.D. |
|---|---|---|---|---|---|---|---|---|---|

FIG. 68

FIG. 69

FIG. 70

FIG. 71

**FIG. 72**

**a**  *Dnmt3a locus*

PAM ▼
CCTCCG--TCAGCGACCCATGCCAA
CCTCCG-ATCAGCGACCCATGCCAA
CCTCCGTGTCAGCGACCCATGCCAA
CCTCCG-TTCAGCGACCCATGCCAA
CCTCCG-CTCAGCGACCCATGCCAA
CCTCC---TCAGCGACCCATGCCAA
CCTCCG---CAGCGACCCATGCCAA
CCTC----TCAGCGACCCATGCCAA
CCTCC---TCAGCGACCCATGCCAA
CCTCCG-----CGACCCATGCCAA
CCTC------AGCGACCCATGCCAA
C-------TCAGCGACCCATGCCAA

**b**  *Dnmt1 locus*

PAM ▼
CCAGCG-CGAACAGCTCCAGCCCG
CCAGCGCCGAACAGCTCCAGCCCG
CCAGCGTCGAACAGCTCCAGCCCG
CCAGCG----AACAGCTCCAGCCCG
CCAGCG--G-ACAGCTCCAGCCCG
CCAGCG----AACAGCTCCAGCCCG
CCAG-----AACAGCTCCAGCCCG
CCAGCG------AGCTCCAGCCCG
CCAG-----AACAGCTCCAGCCCG
CCAGCG----AACAGCTCCAGCCCG
CCAGCGC----ACAGCTCCAGCCCG
CCAGCGC--------TCCAGCCCG

**c**  *Dnmt3b locus*

PAM
AGAGGGTGCCAGCGGG--TATGAGG
AGAGGGTGCCAGCGGG-TTATGAGG
AGAGGGTGCCAGCGGGTATATGAGG
AGAGGGTGCCAGCGGG-TAATGAGG
AGAGGGTGCCAGCGGG--T-19bp-
AGAGGGTGCCAG------TATGAGG
AGAGGGTGCCAGCGGG--T--GAGG
AGAGGGTGCCAGCG-------AGG
AGAGGGTGCCAGCGGG-----GAGG

FIG. 73

330

**FIG. 74**

☐ FLAG

SpCas9 (330)
2. FLAG-reopt-HA
3. FLAG-reopt(Ub)-HA
4. FLAG-reopt(Ub)-HA
5. FLAG-Sa-HA
6. FLAG-Sa-3XHA

0.0    5.0×10⁵    1.0×10⁶    1.5×10⁶    2.0×10⁶

☐ HA

SpCas9 (414)
0. original Sa-HA
2. FLAG-reopt-HA
3. FLAG-reopt(Ub)-HA
5. FLAG-Sa-HA
6. FLAG-Sa-3XHA

0.0    5.0×10⁵    1.0×10⁶    1.5×10⁶    2.0×10⁶

FIG. 75

FIG. 76

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8709843 B
- US 6007845 A
- US 5855913 A
- US 5985309 A
- US 5543158 A
- WO 2012135025 A
- US 20120251560 A
- US 8454972 B
- US 8404658 B
- US 5846946 A
- US 20120017290 A
- US 20110265198 A
- US 20130236946 A
- US 8454972 B2, Nabel
- US 8404658 B2, Hajjar
- US 20120295960 A
- US 7303910 B
- US 7351585 B
- US 20060281180 A
- US 20090007284 A
- US 20110117189 A
- US 20090017543 A
- US 20070054961 A
- US 20100317109 A
- US 20110293571 A
- US 20040013648 A
- US 20070025970 A
- US 20090111106 A
- US 7259015 B
- US 20110293703 A
- US 20130302401 A
- US 7982027 B
- US 7799565 B
- US 8058069 B
- US 8283333 B
- US 7901708 B
- US 7745651 B
- US 7803397 B
- US 8101741 B
- US 8188263 B
- US 7915399 B
- US 8236943 B
- US 7838658 B
- EP 1766035 A
- EP 1519714 A
- EP 1781593 A
- EP 1664316 A
- US 20130252281 A
- US 20130245107 A
- US 20130244279 A
- US 20120251618 A
- US 20050019923 A
- US 20080267903 A
- US 8372951 B
- US 8575305 B
- US 8614194 B
- US 8044019 B
- US 20110195123 A
- US 20130171732 A
- US 20100291048 A
- US 20130137104 A
- US 20130122591 A
- US 20100146651 A
- US 20110016540 A
- WO 2013163628 A2
- US 20130145487 A
- WO 2013126794 A
- US 20110225664 A
- US 20110091441 A
- US 20100229252 A
- US 20090271881 A
- US 20090222937 A
- US 20110182867 A
- US 20120328580 A
- US 20060030837 A, McKenna
- US 7206639 B, Jacobsen
- US 20070093878 A, Edge
- US 20050287127 A, Li
- US 11953797 B, Li
- US 2007084654 W, Edge
- US 20110142917 A
- US 5593972 A
- US 5589466 A
- US 5580859 A
- US 20120204282 A
- US 20130183282 A
- US 20130202678 A
- US 20120159653 A
- US 20110023139 A
- US 20130253040 A
- WO 9703211 A
- WO 9639154 A
- US 81573004 A
- US 20040171156 A1
- US 491026 A
- WO 2011028929 A
- US 511940 A

- US 4873316 A
- EP 264166 A
- US 6750059 B
- US 092085 A
- US 7776321 B
- US 20110059502 A
- US 61736465 B
- US 61721283 B
- US 5049386 A
- US 4946787 A
- US 4897355 A
- WO 9117424 A
- WO 9116024 A
- US 4186183 A
- US 4217344 A
- US 4235871 A
- US 4261975 A
- US 4485054 A
- US 4501728 A
- US 4774085 A
- US 4837028 A

- US 9405700 W
- US 20120164118 A
- US 4797368 A
- WO 9324641 A
- US 5173414 A
- US 20030087817 A
- US 20110230839 A
- US 5210015 A
- US 5445934 A
- US 61736527 B
- US 61748427 B
- US 20110158957 A
- US 20100311124 A
- US 20110023145 A
- US 20110023153 A
- US 20110023146 A
- US 20110023144 A
- US 61064798 A
- US 61836127 A
- US 61836101 A
- US 61835936 B

**Non-patent literature cited in the description**

- **CONG, L. ; RAN, F.A. ; COX, D. ; LIN, S. ; BARRETTO, R. ; HABIB, N. ; HSU, P.D. ; WU, X. ; JIANG, W. ; MARRAFFINI, L.A.** Multiplex genome engineering using CRISPR/Cas systems. *Science,* 15 February 2013, vol. 339 (6121), 819-23
- **JIANG W. ; BIKARD D. ; COX D. ; ZHANG F ; MARRAFFINI LA.** RNA-guided editing of bacterial genomes using CRISPR-Cas systems. *Nat Biotechnol,* March 2013, vol. 31 (3), 233-9
- **WANG H. ; YANG H. ; SHIVALILA CS. ; DAWLATY MM. ; CHENG AW. ; ZHANG F. ; JAENISCH R.** One-Step Generation of Mice Carrying Mutations in Multiple Genes by CRISPR/Cas-Mediated Genome Engineering. *Cell,* 09 May 2013, vol. 153 (4), 910-8
- **KONERMANN S ; BRIGHAM MD ; TREVINO AE ; HSU PD ; HEIDENREICH M ; CONG L ; PLATT RJ ; SCOTT DA ; CHURCH GM ; ZHANG F.** Optical control of mammalian endogenous transcription and epigenetic states. *Nature,* 22 August 2013, vol. 500 (7463), 472-6
- **RAN, FA. ; HSU, PD. ; LIN, CY. ; GOOTENBERG, JS. ; KONERMANN, S. ; TREVINO, AE. ; SCOTT, DA. ; INOUE, A. ; MATOBA, S. ; ZHANG, Y.** Double Nicking by RNA-Guided CRISPR Cas9 for Enhanced Genome Editing Specificity. *Cell,* 28 August 2013
- **HSU, P. ; SCOTT, D. ; WEINSTEIN, J. ; RAN, FA. ; KONERMANN, S. ; AGARWALA, V. ; LI, Y. ; FINE, E. ; WU, X. ; SHALEM, O.** DNA targeting specificity of RNA-guided Cas9 nucleases. *Nat Biotechnol,* 2013

- **RAN, FA. ; HSU, PD. ; WRIGHT, J. ; AGARWALA, V. ; SCOTT, DA. ; ZHANG, F.** Genome engineering using the CRISPR-Cas9 system. *Nature Protocols,* November 2013, vol. 8 (11), 2281-308
- **SHALEM, O. ; SANJANA, NE. ; HARTENIAN, E. ; SHI, X. ; SCOTT, DA. ; MIKKELSON, T. ; HECKL, D. ; EBERT, BL. ; ROOT, DE. ; DOENCH, JG.** Genome-Scale CRISPR-Cas9 Knockout Screening in Human Cells. *Science,* 2013
- **NISHIMASU, H. ; RAN, FA. ; HSU, PD. ; KONERMANN, S. ; SHEHATA, SI ; DOHMAE, N. ; ISHITANI, R. ; ZHANG, F. ; NUREKI, O.** Crystal structure of cas9 in complex with guide RNA and target DNA. *Cell,* 27 February 2014, vol. 156 (5), 935-49
- **WU X. ; SCOTT DA. ; KRIZ AJ. ; CHIU AC. ; HSU PD. ; DADON DB. ; CHENG AW. ; TREVINO AE. ; KONERMANN S. ; CHEN S.** Genome-wide binding of the CRISPR endonuclease Cas9 in mammalian cells. *Nat Biotechnol.,* 20 April 2014
- **HSU et al.** Development and Applications of CRISPR-Cas9 for Genome Engineering. *Cell,* 05 June 2014, vol. 157, 1262-1278
- **RAN.** *Cell,* 21 August 2013, vol. 154
- **KARRA ; DAHM.** *The Journal of Neuroscience,* 05 May 2010, vol. 30 (18), 6171-6177
- **YIN ; ANDERSON.** *Nature Biotech,* 30 March 2014, 2884
- **SCHWANK et al.** *Cell Stem Cell,* 05 December 2013, vol. 13, 653-658

- **OMAR AKIL ; REBECCA P. SEAL ; KEVIN BURKE ; CHUANSONG WANG ; AURASH ALEMI ; MATTHEW DURING ; ROBERT H. EDWARDS ; LAWRENCE R. LUSTIG.** Restoration of Hearing in the VGLUT3 Knockout Mouse Using Virally Mediated Gene Therapy. *Neuron,* 2012, vol. 75 (2), 283
- **KAUFMANN et al.** *EMBO Mol Med,* 2013, vol. 5, 1642-1661
- **KANASTY ; ANDERSON.** *Nature Materials,* November 2013, vol. 12
- **JAMES E. DAHLMAN ; CARMEN BARNES et al.** *Nature Nanotechnology,* 11 May 2014
- **DUMITRACHE et al.** *Genetics,* August 2011, vol. 188 (4), 787-797
- REMINGTON'S PHARMACEUTICAL SCIENCES. Mack Pub. Co, 1991
- **BALAGAAN.** J Gene Med 2006. Wiley InterScience, 21 November 2005, vol. 8, 275-285
- **BINLEY et al.** *HUMAN GENE THERAPY,* September 2012, vol. 23, 980-991
- **DIGIUSTO et al.** *Sci Transl Med,* 2010, vol. 2, 36ra43
- **SU X ; FRICKE J ; KAVANAGH DG ; IRVINE DJ.** In vitro and in vivo mRNA delivery using lipid-enveloped pH-responsive polymer nanoparticles. *Mol Pharm.,* 06 June 2011, vol. 8 (3), 774-87
- **MAZZA, M. et al.** *ACSNano,* 2013, vol. 7 (2), 1016-1026
- **SIEW, A. et al.** *Mol Pharm,* 2012, vol. 9 (1), 14-28
- **LALATSA, A. et al.** *J Contr Rel,* 2012, vol. 161 (2), 523-36
- **LALATSA, A. et al.** *Mol Pharm,* 2012, vol. 9 (6), 1665-80
- **LALATSA, A. et al.** *Mol Pharm,* 2012, vol. 9 (6), 1764-74
- **GARRETT, N.L. et al.** *J Biophotonics,* 2012, vol. 5 (5-6), 458-68
- **GARRETT, N.L. et al.** *J Raman Spect,* 2012, vol. 43 (5), 681-688
- **AHMAD, S. et al.** *J Royal Soc Interface,* 2010, vol. 7, S423-33
- **UCHEGBU, I.F.** *Expert Opin Drug Deliv,* 2006, vol. 3 (5), 629-40
- **QU, X.** *Biomacromolecules,* 2006, vol. 7 (12), 3452-9
- **UCHEGBU, I.F. et al.** *Int J Pharm,* 2001, vol. 224, 185-199
- **ALABI et al.** *Proc Natl Acad Sci U S A.,* 06 August 2013, vol. 110 (32), 12881-6
- **ZHANG et al.** *Adv Mater.,* 06 September 2013, vol. 25 (33), 4641-5
- **JIANG et al.** *Nano Lett.,* 13 March 2013, vol. 13 (3), 1059-64
- **KARAGIANNIS et al.** *ACS Nano.,* 23 October 2012, vol. 6 (10), 8484-7
- **WHITEHEAD et al.** *ACS Nano.,* 28 August 2012, vol. 6 (8), 6922-9
- **LEE et al.** *Nat Nanotechnol.,* 03 June 2012, vol. 7 (6), 389-93
- **COELHO et al.** *N Engl J Med,* 2013, vol. 369, 819-29
- **TABERNERO et al.** *Cancer Discovery,* April 2013, vol. 3 (4), 363-470
- **ROSIN et al.** *Molecular Therapy,* December 2011, vol. 19 (12), 1286-2200
- **CUTLER et al.** *J. Am. Chem. Soc.,* 2011, vol. 133, 9254-9257
- **HAO et al.** *Small,* 2011, vol. 7, 3158-3162
- **ZHANG et al.** *ACS Nano.,* 2011, vol. 5, 6962-6970
- **CUTLER et al.** *J. Am. Chem. Soc.,* 2012, vol. 134, 1376-1391
- **YOUNG et al.** *Nano Lett.,* 2012, vol. 12, 3867-71
- **ZHENG et al.** *Proc. Natl. Acad. Sci. USA.,* 2012, vol. 109, 11975-80
- **MIRKIN.** *Nanomedicine,* 2012, vol. 7, 635-638
- **ZHANG et al.** *J. Am. Chem. Soc.,* 2012, vol. 134, 16488-1691
- **WEINTRAUB.** *Nature,* 2013, vol. 495, S14-S16
- **CHOI et al.** *Proc. Natl. Acad. Sci. USA.,* 2013, vol. 110 (19), 7625-7630
- **JENSEN et al.** *Sci. Transl. Med.,* 2013, vol. 5, 209ra152
- **MIRKIN et al.** *Small*
- **SCHIFFELERS et al.** *Nucleic Acids Research,* 2004, vol. 32 (19
- **BARTLETT et al.** *PNAS,* 25 September 2007, vol. 104 (39
- **DAVIS et al.** *Nature,* 15 April 2010, vol. 464
- **ALVAREZ-ERVITI et al.** *Nat Biotechnol,* 2011, vol. 29, 341
- **EL-ANDALOUSSI et al.** *Nature Protocols,* 2012, vol. 7, 2112-2126
- **WAHLGREN et al.** *Nucleic Acids Research,* 2012, vol. 40 (17), e130
- **SPUCH ; NAVARRO.** *Journal of Drug Delivery,* 2011, vol. 2011, 12
- **MORRISSEY et al.** *Nature Biotechnology,* August 2005, vol. 23 (8
- **ZIMMERMAN et al.** *Nature Letters,* 04 May 2006, vol. 441
- **LI.** *Gene Therapy,* 2012, vol. 19, 775-780
- **GEISBERT et al.** *Lancet,* 2010, vol. 375, 1896-905
- **JUDGE.** *J. Clin. Invest.,* 2009, vol. 119, 661-673
- *Advanced Drug Delivery Reviews,* 2012, vol. 64, 1730-1737
- **SEMPLE et al.** *Nature Niotechnology,* February 2010, vol. 28 (2), 172-177

- **JAYARAMAN.** *Angew. Chem. Int. Ed.,* 2012, vol. 51, 8529-8533
- **MICHAEL S D KORMANN et al.** Expression of therapeutic proteins after delivery of chemically modified mRNA in mice. *Nature Biotechnology,* 09 January 2011, vol. 29, 154-157
- **NOVOBRANTSEVA.** *Molecular Therapy-Nucleic Acids,* 2012, vol. 1, e4
- **MAZZA et al.** *ACS Nano.,* 26 February 2013, vol. 7 (2), 1016-26
- **UCHEGBU ; SIEW.** *J Pharm Sci.,* 2013, vol. 102 (2), 305-10
- **LALATSA et al.** *J Control Release,* 20 July 2012, vol. 161 (2), 523-36
- **LAWRENCE et al.** *Journal of the American Chemical Society,* 2007, vol. 129, 10110-10112
- **AKINC et al.** *Nat. Biotech.,* 2010, vol. 26, 561-569
- **MCNAUGHTON et al.** *Proc. Natl. Acad. Sci. USA,* 2009, vol. 106, 6111-6116
- **CRONICAN et al.** *ACS Chemical Biology,* 2010, vol. 5, 747-752
- **CRONICAN et al.** *Chemistry & Biology,* 2011, vol. 18, 833-838
- **THOMPSON et al.** *Methods in Enzymology,* 2012, vol. 503, 293-319
- **THOMPSON, D.B. et al.** *Chemistry & Biology,* 2012, vol. 19 (7), 831-843
- **SURESH RAMAKRISHNA ; ABU-BONSRAH KWAKU DAD ; JAGADISH BELOOR et al.** Gene disruption by cell-penetrating peptide-mediated delivery of Cas9 protein and guide RNA. *Genome Res.,* 02 April 2014
- **RAN et al.** *Cell,* 12 September 2013, vol. 154 (6), 1380-9
- **BAILEY et al.** *J Mol Med,* January 1999, vol. 77 (1), 244-9
- **SATO et al.** *Nature Biotechnology,* April 2008, vol. 26 (4), 431-442
- **ROZEMA et al.** *PNAS,* 07 August 2007, vol. 104 (32
- **OAKES ; LIEBERMAN.** *Clin Orthop Relat Res.,* October 2000, vol. 379, 101-12
- **XIA CF ; BOADO RJ ; PARDRIDGE WM.** Antibody-mediated targeting of siRNA via the human insulin receptor using avidin-biotin technology. *Mol Pharm.,* May 2009, vol. 6 (3), 747-51
- **ZHANG et al.** *Mol Ther.,* January 2003, vol. 7 (1), 11-8
- **CHO, S. ; GOLDBERG, M. ; SON, S. ; XU, Q. ; YANG, F. ; MEI, Y. ; BOGATYREV, S. ; LANGER, R. ; ANDERSON, D.** Lipid-like nanoparticles for small interfering RNA delivery to endothelial cells. *Advanced Functional Materials,* 2010, vol. 19, 3112-3118
- **SCHROEDER, A. ; LEVINS, C. ; CORTEZ, C. ; LANGER, R. ; ANDERSON, D.** Lipid-based nanotherapeutics for siRNA delivery. *Journal of Internal Medicine,* 2010, vol. 267, 9-21
- **EL-ANDALOUSSI S et al.** Exosome-mediated delivery of siRNA in vitro and in vivo. *Nat Protoc.,* December 2012, vol. 7 (12), 2112-26
- **UNO et al.** *HUMAN GENE THERAPY,* June 2011, vol. 22, 711-719
- **ZOU et al.** *HUMAN GENE THERAPY,* April 2011, vol. 22, 465-475
- **XIA et al.** *Nature Medicine,* August 2004, vol. 10 (8
- **TANGRI S et al.** Rationally engineered therapeutic proteins with reduced immunogenicity. *J Immunol.,* 15 March 2005, vol. 174 (6), 3187-96
- **CAVAZZANA-CALVO et al.** *Annu. Rev. Med.,* 2005, vol. 56, 585-602
- **FISCHER et al.** *Immunol. Rev.,* 2005, vol. 203, 98-109
- **GIBLETT et al.** *Lancet,* 1972, vol. 2, 1067-1069
- **NOGUCHI et al.** *Cell,* 1993, vol. 73, 147-157
- **MACCHI et al.** *Nature,* 1995, vol. 377, 65-68
- **HIRSCHHORN et al.** *Nat. Genet.,* 1996, vol. 13, 290-295
- **STEPHAN et al.** *N. Engl. J. Med.,* 1996, vol. 335, 1563-1567
- **BOUSSO et al.** *Proc. Natl., Acad. Sci. USA,* 2000, vol. 97, 274-278
- **WADA et al.** *Proc. Natl. Acad. Sci. USA,* 2001, vol. 98, 8697-8702
- **NISHIKOMORI et al.** *Blood,* 2004, vol. 103, 4565-4572
- **CANDOTTI et al.** *Blood,* 1996, vol. 87, 3097-3102
- **CAVAZZANA-CALVO et al.** *Blood,* 1996, vol. 88, 3901-3909
- **TAYLOR et al.** *Blood,* 1996, vol. 87, 3103-3107
- **HACEIN-BEY et al.** *Blood,* 1998, vol. 92, 4090-4097
- **SOUDAIS et al.** *Blood,* 2000, vol. 95, 3071-3077
- **TSAI et al.** *Blood,* 2002, vol. 100, 72-79
- **BUNTING et al.** *Nat. Med.,* 1998, vol. 4, 58-64
- **BUNTING et al.** *Hum. Gene Ther.,* 2000, vol. 11, 2353-2364
- **YATES et al.** *Blood,* 2002, vol. 100, 3942-3949
- **CAVAZZANA-CALVO et al.** *Science,* 2000, vol. 288, 669-672
- **AIUTI et al.** *Nat. Med.,* 2002, vol. 8, 423-425
- **GASPAR et al.** *Lancet,* 2004, vol. 364, 2181-2187
- **BAUER et al.** *Science,* 11 October 2013, vol. 342 (6155), 253-257

- **XU et al.** *Science,* 18 November 2011, vol. 334 (6058), 993-996
- **SALT ; PLONTKE.** *Drug Discovery Today,* 2005, vol. 10, 1299-1306
- **TAKAHASHI et al.** *Cell,* 2007, vol. 131 (5), 861-872
- **TAKAHASHI ; YAMANAKA.** *Cell,* 2006, vol. 126, 663-76
- **OKITA et al.** *Nature,* 2007, vol. 448, 260-262
- **YU, J. et al.** *Science,* 2007, vol. 318 (5858), 1917-1920
- **NAKAGAWA et al.** *Nat. Biotechnol.,* 2008, vol. 26, 101-106
- **ZAEHRES ; SCHOLER.** *Cell,* 2007, vol. 131 (5), 834-835
- **SHEN et al.** *FEBS Let.,* 2003, vol. 539, 111-114
- **XIA et al.** *Nat. Biotech.,* 2002, vol. 20, 1006-1010
- **REICH et al.** *Mol. Vision.,* 2003, vol. 9, 210-216
- **SORENSEN et al.** *J. Mol. Biol.,* 2003, vol. 327, 761-766
- **LEWIS et al.** *Nat. Gen.,* 2002, vol. 32, 107-108
- **SIMEONI et al.** *NAR,* 2003, vol. 31 (11), 2717-2724
- **TOLENTINO et al.** *Retina,* vol. 24 (4), 660
- **QI et al.** *Gene Therapy,* 2013, 1-9
- **REJALI et al.** *Hear Res.,* June 2007, vol. 228 (1-2), 180-7
- **MUKHERJEA et al.** *Antioxidants & Redox Signaling,* 2010, vol. 13 (5
- **JUNG et al.** *Molecular Therapy,* April 2013, vol. 21 (4), 834-841
- Genetic Diseases of the Eye. Oxford University Press, 2012
- **CAMPOCHIARO et al.** *Human Gene Therapy,* February 2006, vol. 17, 167-176
- **MILLINGTON-WARD et al.** *Molecular Therapy,* April 2011, vol. 19 (4), 642-649
- **DALKARA et al.** *Sci Transl Med,* 2013, vol. 5, 189ra76
- **WU.** *Cell Stem Cell,* 2013, vol. 13, 659-62
- **LIN-YANGA et al.** *PNAS,* 10 March 2009, vol. 106 (10
- **EULALIO et al.** *Nature,* 2012, vol. 492, 376
- **SOMASUNTHARAM et al.** *Biomaterials,* 2013, vol. 34, 7790
- Delivery Methods to Target RNAs in the Kidney. **CSABA RÉVÉSZ ; PETER HAMAR.** Gene Therapy Applications. 2011
- **LARSON et al.** *Surgery,* August 2007, vol. 142 (2), 262-269
- **HAMAR et al.** *Proc Natl Acad Sci,* October 2004, vol. 101 (41), 14883-14888
- **ZHENG et al.** *Am J Pathol,* October 2008, vol. 173 (4), 973-980
- **FENG et al.** *Transplantation,* May 2009, vol. 87 (9), 1283-1289
- **XIE ; GUO.** *Am Soc Nephrol,* December 2006, vol. 17 (12), 3336-3346
- **Q. ZHANG et al.** *PloS ONE,* July 2010, vol. 5 (7), 1-13
- **KUSHIBIKIA et al.** *J Controlled Release,* July 2005, vol. 105 (3), 318-331
- **WANG et al.** *Gene Therapy,* July 2006, vol. 13 (14), 1097-1103
- **KOBAYASHI et al.** *Journal of Pharmacology and Experimental Therapeutics,* February 2004, vol. 308 (2), 688-693
- **WOLFRUM et al.** *Nature Biotechnology,* September 2007, vol. 25 (10), 1149-1157
- **MOLITORIS et al.** *J Am Soc Nephrol,* August 2009, vol. 20 (8), 1754-1764
- **MIKHAYLOVA et al.** *Cancer Gene Therapy,* March 2011, vol. 16 (3), 217-226
- **YUAN et al.** *Am J Physiol Renal Physiol,* June 2008, vol. 295, F605-F617
- **Y. ZHANG et al.** *J Am Soc Nephrol,* April 2006, vol. 17 (4), 1090-1101
- **SINGHAL et al.** *Cancer Res,* May 2009, vol. 69 (10), 4244-4251
- **MALEK et al.** *Toxicology and Applied Pharmacology,* April 2009, vol. 236 (1), 97-108
- **SHIMIZU et al.** *J Am Soc Nephrology,* April 2010, vol. 21 (4), 622-633
- **JIANG et al.** *Molecular Pharmaceutics,* May 2009, vol. 6 (3), 727-737
- **CAO et al.** *J Controlled Release,* June 2010, vol. 144 (2), 203-212
- **NINICHUK et al.** *Am J Pathol,* March 2008, vol. 172 (3), 628-637
- **PURSCHKE et al.** *Proc Natl Acad Sci,* March 2006, vol. 103 (13), 5173-5178
- **YUAN et al.** *Am J Physiol Renal Physiol,* 2008, vol. 295, F605-F617
- **MOLITORIS et al.** *J Am Soc Nephrol,* 2009, vol. 20, 1754-1764
- **THOMPSON et al.** *Nucleic Acid Therapeutics,* 2012, vol. 22 (4
- **SHIMIZU et al.** *J Am Soc Nephrol,* 2010, vol. 21, 622-633
- **LI et al.** *Molecular Therapy,* December 2009, vol. 17 (12), 2067-2077
- **ZAMORA et al.** *Am J Respir Crit Care Med,* 2011, vol. 183, 531-538
- **SCHWANK et al.** *Cell Stem Cell,* 2013, vol. 13, 653-58
- **BORTOLANZA et al.** *Molecular Therapy,* November 2011, vol. 19 (11), 2055-2064
- **DUMONCEAUX et al.** *Molecular Therapy,* May 2010, vol. 18 (5), 881-887
- **KINOUCHI et al.** *Gene Therapy,* 2008, vol. 15, 1126-1130

- **HAGSTROM et al.** *Molecular Therapy,* August 2004, vol. 10 (2
- **HICKERSON et al.** *Molecular Therapy-Nucleic Acids,* 2013, vol. 2, e129
- **LEACHMAN et al.** *Molecular Therapy,* February 2010, vol. 18 (2), 442-446
- **ZHENG et al.** *PNAS,* 24 July 2012, vol. 109 (30), 11975-11980
- **GRIMM et al.** *Nature,* 26 May 2006, vol. 441
- **CHEN et al.** *Gene Therapy,* 2007, vol. 14, 11-19
- **WOODDELL et al.** *Molecular Therapy,* May 2013, vol. 21 (5), 973-985
- **ROELVINKI et al.** *Molecular Therapy,* September 2012, vol. 20 (9), 1737-1749
- **HAO YIN et al.** *Nature Biotechnology,* 30 March 2014
- **MCBRIDE et al.** *Molecular Therapy,* December 2011, vol. 19 (12), 2152-2162
- **DIFIGLIA et al.** *PNAS,* 23 October 2007, vol. 104 (43), 17204-17209
- **BOUDREAU et al.** *Molecular Therapy,* June 2009, vol. 17 (6
- **YU et al.** *Cell,* 31 August 2012, vol. 150, 895-908
- **STILES et al.** *Experimental Neurology,* 2012, vol. 233, 463-471
- Overview of principles of hybridization and the strategy of nucleic acid probe assay. **TIJSSEN.** Laboratory Techniques In Biochemistry And Molecular Biology-Hybridization With Nucleic Acid Probes. Elsevier, 1993
- **DEVEREUX et al.** *Nucleic Acids Research,* 1984, vol. 12, 387
- **AUSUBEL et al.** NUCLEIC ACIDS RESEARCH. 1999
- **ATSCHUL et al.** *J. Mol. Biol.,* 1990, 403-410
- **AUSUBEL et al.** J. MOL. BIOL. 1999, 7-58, 7-60
- **DEVEREUX et al.** *Nuc. Acids Research,* 1984, vol. 12, 387
- **AUSUBEL et al.** Short Protocols in Molecular Biology. 1999
- **ALTSCHUL et al.** *J. Mol. Biol.,* 1990, 403-410
- **AUSUBEL et al.** *Short Protocols in Molecular Biology,* 1999, 7-58, 7-60
- *FEMS Microbiol Lett.,* 1999, vol. 174 (2), 247-50
- *FEMS Microbiol Lett.,* 1999, vol. 177 (1), 187-8
- **HIGGINS DG ; SHARP PM.** *Gene,* 1988, vol. 73 (1), 237-244
- **LIVINGSTONE C.D. ; BARTON G.J.** Protein sequence alignments: a strategy for the hierarchical analysis of residue conservation. *Comput. Appl. Biosci.,* 1993, vol. 9, 745-756
- **TAYLOR W.R.** The classification of amino acid conservation. *J. Theor. Biol.,* 1986, vol. 119, 205-218
- **SIMON RJ et al.** *PNAS,* 1992, vol. 89 (20), 9367-9371
- **HORWELL DC.** *Trends Biotechnol,* 1995, vol. 13 (4), 132-134
- **SAMBROOK ; FRITSCH ; MANIATIS.** MOLECULAR CLONING: A LABORATORY MANUAL. 1989
- CURRENT PROTOCOLS IN MOLECULAR BIOLOGY. 1987
- METHODS IN ENZYMOLOGY. Academic Press, Inc,
- PCR 2: A PRACTICAL APPROACH. 1995
- ANTIBODIES, A LABORATORY MANUAL, and ANIMAL CELL CULTURE. R.I. Freshney, 1987
- **GOEDDEL.** GENE EXPRESSION TECHNOLOGY: METHODS IN ENZYMOLOGY. Academic Press, 1990, 185
- **BOSHART et al.** *Cell,* 1985, vol. 41, 521-530
- *Mol. Cell. Biol.,* 1988, vol. 8 (1), 466-472
- *Proc. Natl. Acad. Sci. USA.,* 1981, vol. 78 (3), 1527-31
- **SMITH ; JOHNSON.** Gene. Pharmacia Biotech Inc, 1988, vol. 67, 31-40
- **AMRANN et al.** *Gene,* 1988, vol. 69, 301-315
- **STUDIER et al.** GENE EXPRESSION TECHNOLOGY: METHODS IN ENZYMOLOGY. Academic Press, 1990, vol. 185, 60-89
- **BALDARI et al.** *EMBO J.,* 1987, vol. 6, 229-234
- **KUIJAN ; HERSKOWITZ.** *Cell,* 1982, vol. 30, 933-943
- **SCHULTZ et al.** *Gene,* 1987, vol. 54, 113-123
- **SMITH et al.** *Mol. Cell. Biol.,* 1983, vol. 3, 2156-2165
- **LUCKLOW ; SUMMERS.** *Virology,* 1989, vol. 170, 31-39
- **SEED.** *Nature,* 1987, vol. 329, 840
- **KAUFMAN et al.** *EMBO J.,* 1987, vol. 6, 187-195
- **SAMBROOK et al.** MOLECULAR CLONING: A LABORATORY MANUAL. Cold Spring Harbor Laboratory Press, 1989
- **PINKERT et al.** *Genes Dev.,* 1987, vol. 1, 268-277
- **CALAME ; EATON.** *Adv. Immunol.,* 1988, vol. 43, 235-275
- **WINOTO ; BALTIMORE.** *EMBO J.,* 1989, vol. 8, 729-733
- **BANEIJI et al.** *Cell,* 1983, vol. 33, 729-740
- **QUEEN ; BALTIMORE.** *Cell,* 1983, vol. 33, 741-748
- **BYRNE ; RUDDLE.** *Proc. Natl. Acad. Sci. USA,* 1989, vol. 86, 5473-5477
- **EDLUND et al.** *Science,* 1985, vol. 230, 912-916
- **KESSEL ; GRUSS.** *Science,* 1990, vol. 249, 374-379

- **CAMPES ; TILGHMAN.** *Genes Dev.,* 1989, vol. 3, 537-546
- **ISHINO et al.** *J. Bacteriol.,* 1987, vol. 169, 5429-5433
- **NAKATA et al.** *J. Bacteriol.,* 1989, vol. 171, 3553-3556
- **GROENEN et al.** *Mol. Microbiol.,* 1993, vol. 10, 1057-1065
- **HOE et al.** *Emerg. Infect. Dis.,* 1999, vol. 5, 254-263
- **MASEPOHL et al.** *Biochim. Biophys. Acta,* 1996, vol. 1307, 26-30
- **MOJICA et al.** *Mol. Microbiol.,* 1995, vol. 17, 85-93
- **JANSSEN et al.** *OMICS J. Integ. Biol.,* 2002, vol. 6, 23-33
- **MOJICA et al.** *Mol. Microbiol.,* 2000, vol. 36, 244-246
- **VAN EMBDEN et al.** *J. Bacteriol.,* 2000, vol. 182, 2393-2401
- **JANSEN et al.** *Mol. Microbiol.,* 2002, vol. 43, 1565-1575
- **KARGINOV ; HANNON.** The CRISPR system: small RNA-guided defence in bacteria and archaea. *Mole Cell,* 15 January 2010, vol. 37 (1), 7
- **SAPRANAUSKAS et al.** *Nucleic Acis Research,* 2011, vol. 39, 9275
- **GASIUNAS et al.** *Proc. Natl. Acad. Sci. USA,* 2012, vol. 109, E2579
- *Codon Usage Database,* 09 July 2002, www.kazusa.orjp/codon
- **NAKAMURA, Y. et al.** Codon usage tabulated from the international DNA sequence databases: status for the year 2000. *Nucl. Acids Res.,* 2000, vol. 28, 292
- **ZUKER ; STIEGLER.** *Nucleic Acids Res.,* 1981, vol. 9, 133-148
- **A.R. GRUBER et al.** *Cell,* 2008, vol. 106 (1), 23-24
- **PA CARR ; GM CHURCH.** *Nature Biotechnology,* 2009, vol. 27 (12), 1151-62
- **ANDERSON.** *Science,* 1992, vol. 256, 808-813
- **NABEL ; FELGNER.** *TIBTECH,* 1993, vol. 11, 211-217
- **MITANI ; CASKEY.** *TIBTECH,* 1993, vol. 11, 162-166
- **DILLON.** *TIBTECH,* 1993, vol. 11, 167-175
- **MILLER.** *Nature,* 1992, vol. 357, 455-460
- **VAN BRUNT.** *Biotechnology,* 1988, vol. 6 (10), 1149-1154
- **VIGNE.** *Restorative Neurology and Neuroscience,* 1995, vol. 8, 35-36
- **KREMER ; PERRICAUDET.** *British Medical Bulletin,* 1995, vol. 51 (1), 31-44
- **HADDADA et al.** Current Topics in Microbiology and Immunology. 1995
- **YU et al.** *Gene Therapy,* 1994, vol. 1, 13-26
- **CRYSTAL.** *Science,* 1995, vol. 270, 404-410
- **BLAESE et al.** *Cancer Gene Ther.,* 1995, vol. 2, 291-297
- **BEHR et al.** *Bioconjugate Chem.,* 1994, vol. 5, 382-389
- **REMY et al.** *Bioconjugate Chem.,* 1994, vol. 5, 647-654
- **GAO et al.** *Gene Therapy,* 1995, vol. 2, 710-722
- **AHMAD et al.** *Cancer Res.,* 1992, vol. 52, 4817-4820
- **BUCHSCHER et al.** *J. Virol.,* 1992, vol. 66, 2731-2739
- **JOHANN et al.** *J. Virol.,* 1992, vol. 66, 1635-1640
- **SOMMNERFELT et al.** *Virol.,* 1990, vol. 176, 58-59
- **WILSON et al.** *J. Virol.,* 1989, vol. 63, 2374-2378
- **MILLER et al.** *J. Virol.,* 1991, vol. 65, 2220-2224
- **JONKERS et al.** *Am. J. Vet. Res.,* 1964, vol. 25, 236-242
- **TRAVASSOS DA ROSA et al.** *Am. J. Tropical Med. & Hygiene,* 1984, vol. 33, 999-1006
- **WEST et al.** *Virology,* 1987, vol. 160, 38-47
- **KOTIN.** *Human Gene Therapy,* 1994, vol. 5, 793-801
- **MUZYCZKA.** *J. Clin. Invest.,* 1994, vol. 94, 1351
- **TRATSCHIN et al.** *Mol. Cell. Biol.,* 1985, vol. 5, 3251-3260
- **TRATSCHIN et al.** *Mol. Cell. Biol.,* 1984, vol. 4, 2072-2081
- **HERMONAT ; MUZYCZKA.** *PNAS,* 1984, vol. 81, 6466-6470
- **SAMULSKI et al.** *J. Virol.,* 1989, vol. 63, 03822-3828
- **GRIMM, D. et al.** *J. Virol.,* 2008, vol. 82, 5887-5911
- **CHAN-HUI et al.** *Clinical Immunology,* 2003, vol. 111, 162-174
- **ROBERT D. WELLS ; TETSUO ASHIZAWA.** Genetic Instabilities and Neurological Diseases. Academic Press, 13 October 2011
- **MCIVOR EI ; POLAK U ; NAPIERALA M.** *RNA Biol.,* September 2010, vol. 7 (5), 551-8
- Genetics of Epilepsy and Genetic Epilepsies. Mariani Foundation Paediatric Neurology, 2009, vol. 20
- **FREITAG et al.** *Eur. Child. Adolesc. Psychiatry,* 2010, vol. 19, 169-178
- **BUCAN et al.** *PLoS Genetics,* 2009, vol. 5, e1000536
- **WARING et al.** *Arch. Neurol.,* 2008, vol. 65, 329-334
- **GUSCHIN et al.** *Methods Mol Biol,* 2010, vol. 649, 247

- **GRUBER et al.** *Nucleic Acids Research,* 2008, vol. 36, W70
- **SAPRANAUSAKS et al.** *Nucleic Acids Resch,* 2011, vol. 39, 9275
- **JINEK et al.** *Science,* 2012, vol. 337, 816
- **MAKAROVA et al.** *Nat Rev Microbiol,* 2011, vol. 9, 467
- **PLOSKER GL et al.** *Drugs,* 1996, vol. 51 (3), 433-459
- **TRAPANI et al.** *IUBMB Life,* November 2011, vol. 63 (11), 964-971
- **BIRCH AM et al.** *Current Opinion in Drug Discovery & Development,* 2010, vol. 13 (4), 489-496
- **FUCHS et al.** *Oncogene,* 2002, vol. 21 (37), 5716-5724
- **MCMANAMAN JL et al.** *The Journal of Lipid Research,* 12 February 2013
- **TANG J et al.** *Cell Metabolism,* 05 January 2011, vol. 13 (1), 44-56
- **JOAO et al.** *Human Mutation,* 1995, vol. 5 (3), 191-196
- **SHALEK et al.** *Nano Letters,* 2012
- **PARDRIDGE et al.** *Cold Spring Harb Protoc,* 2010
- *DNAeasy Blood & Tissue Kit*
- **MEHLEM et al.** Imaging of neutral lipids by oil red O for analyzing the metabolic status in health and disease. *Nature Protocols,* 2013, vol. 8, 1149-1154
- **MACZUGA et al.** Therapeutic expression of hairpins targeting apolipoprotein B100 induces phenotypic and transcriptome changes in murine liver. *Gene Therapy,* 2014, vol. 21, 60-70
- **KOORNNEEF et al.** Apolipoprotein B Knockdown by AAV-delivered shRNA Lowers Plasma Cholesterol in Mice. *Molecular Therapy,* 2011, vol. 19 (4), 731-740
- **TADIN-STRAPPS et al.** siRNA-induced liver ApoB knockdown lowers serum LDL-cholesterol in a mouse model with human-like serum lipids. *Journal of Lipid Research,* 2011, vol. 52, 1084-1097
- **KIANI et al.** CRISPR transcriptional repression devices and layered circuits in mammalian cells. *Nature Methods,* 05 May 2014
- **NISSIM et al.** Multiplexed and Programmable Regulation of Gene Networks with an Integrated RNA and CRISPR/Cas Toolkit. *Human Cells,* 22 May 2014, vol. 54 (4), 698-710
- **NESTLER, E.J. ; HYMAN, S.E.** Animal models of neuropsychiatric disorders. *Nat Neurosci,* 2010, vol. 13, 1161-1169
- **CONG, L. et al.** Multiplex genome engineering using CRISPR/Cas systems. *Science,* 2013, vol. 339, 819-823
- **MALI, P. et al.** RNA-guided human genome engineering via Cas9. *Science,* 2013, vol. 339, 823-826

- **BURGER, C. ; NASH, K. ; MANDEL, R.J.** Recombinant adeno-associated viral vectors in the nervous system. *Hum Gene Ther,* 2005, vol. 16, 781-791
- **WU, Z. ; YANG, H. ; COLOSI, P.** Effect of genome size on AAV vector packaging. *Mol Ther,* 2010, vol. 18, 80-86
- **DELTCHEVA, E. et al.** CRISPR RNA maturation by trans-encoded small RNA and host factor RNase III. *Nature,* 2011, vol. 471, 602-607
- **GRAY, S.J. et al.** Optimizing promoters for recombinant adeno-associated virus-mediated gene expression in the peripheral and central nervous system using self-complementary vectors. *Hum Gene Ther,* 2011, vol. 22, 1143-1153
- **LEVITT, N. ; BRIGGS, D. ; GIL, A. ; PROUDFOOT, N.J.** Definition of an efficient synthetic poly(A) site. *Genes Dev,* 1989, vol. 3, 1019-1025
- **OSTLUND, C. et al.** Dynamics and molecular interactions of linker of nucleoskeleton and cytoskeleton (LINC) complex proteins. *J Cell Sci,* 2009, vol. 122, 4099-4108
- **CHAHROUR, M. ; ZOGHBI, H.Y.** The story of Rett syndrome: from clinic to neurobiology. *Neuron,* 2007, vol. 56, 422-437
- **KISHI, N. ; MACKLIS, J.D.** MECP2 is progressively expressed in post-migratory neurons and is involved in neuronal maturation rather than cell fate decisions. *Molecular and cellular neurosciences,* 2004, vol. 27, 306-321
- **SKENE, P.J. et al.** Neuronal MeCP2 is expressed at near histone-octamer levels and globally alters the chromatin state. *Molecular cell,* 2010, vol. 37, 457-468
- **CHEN, R.Z. ; AKBARIAN, S. ; TUDOR, M. ; JAENISCH, R.** Deficiency of methyl-CpG binding protein-2 in CNS neurons results in a Rett-like phenotype in mice. *Nat Genet,* 2001, vol. 27, 327-331
- **ZHOU, Z. et al.** Brain-specific phosphorylation of MeCP2 regulates activity-dependent Bdnf transcription, dendritic growth, and spine maturation. *Neuron,* 2006, vol. 52, 255-269
- **LI, Y. et al.** Global transcriptional and translational repression in human-embryonic-stem-cell-derived Rett syndrome neurons. *Cell Stem Cell,* 2013, vol. 13, 446-458
- **NGUYEN, M.V. et al.** MeCP2 is critical for maintaining mature neuronal networks and global brain anatomy during late stages of postnatal brain development and in the mature adult brain. *J Neurosci,* 2012, vol. 32, 10021-10034
- **JIANG, W. ; BIKARD, D. ; COX, D. ; ZHANG, F. ; MARRAFFINI, L.A.** RNA-guided editing of bacterial genomes using CRISPR-Cas systems. *Nature biotechnology,* 2013, vol. 31, 233-239

- **QI, L.S. et al.** Repurposing CRISPR as an RNA-guided platform for sequence-specific control of gene expression. *Cell,* 2013, vol. 152, 1173-1183
- **SAPRANAUSKAS, R. et al.** The Streptococcus thermophilus CRISPR/Cas system provides immunity in Escherichia coli. *Nucleic acids research,* 2011, vol. 39, 9275-9282
- **JINEK, M. et al.** A programmable dual-RNA-guided DNA endonuclease in adaptive bacterial immunity. *Science,* 2012, vol. 337, 816-821
- **QIU, P. et al.** Mutation detection using Surveyor nuclease. *BioTechniques,* 2004, vol. 36, 702-707
- **KOBAYASHI, M. et al.** Hippocalcin-deficient mice display a defect in cAMP response element-binding protein activation associated with impaired spatial and associative memory. *Neuroscience,* 2005, vol. 133, 471-484
- **DATEKI, M. et al.** Neurochondrin negatively regulates CaMKII phosphorylation, and nervous system-specific gene disruption results in epileptic seizure. *The Journal of biological chemistry,* 2005, vol. 280, 20503-20508
- **NAKAYA, N. et al.** Deletion in the N-terminal half of olfactomedin 1 modifies its interaction with synaptic proteins and causes brain dystrophy and abnormal behavior in mice. *Experimental neurology,* 2013, vol. 250, 205-218
- **REIM, K. et al.** Complexins regulate a late step in Ca2+-dependent neurotransmitter release. *Cell,* 2001, vol. 104, 71-81
- **EDWARDSON, J.M. et al.** Expression of mutant huntingtin blocks exocytosis in PC12 cells by depletion of complexin II. *The Journal of biological chemistry,* 2003, vol. 278, 30849-30853
- **FENG, J. et al.** Dnmt1 and Dnmt3a maintain DNA methylation and regulate synaptic function in adult forebrain neurons. *Nat Neurosci,* 2010, vol. 13, 423-430
- **FU, Y. et al.** High-frequency off-target mutagenesis induced by CRISPR-Cas nucleases in human cells. *Nature biotechnology,* 2013, vol. 31, 822-826
- **HSU, P.D. et al.** DNA targeting specificity of RNA-guided Cas9 nucleases. *Nature biotechnology,* 2013, vol. 31, 827-832
- **RAN, F.A. et al.** Double nicking by RNA-guided CRISPR Cas9 for enhanced genome editing specificity. *Cell,* 2013, vol. 154, 1380-1389
- **MALI, P. et al.** CAS9 transcriptional activators for target specificity screening and paired nickases for cooperative genome engineering. *Nature biotechnology,* 2013, vol. 31, 833-838
- **ESVELT, K.M. ; WANG, H.H.** Genome-scale engineering for systems and synthetic biology. *Molecular systems biology,* 2013, vol. 9, 641
- **LI, W. ; TENG, F. ; LI, T. ; ZHOU, Q.** Simultaneous generation and germline transmission of multiple gene mutations in rat using CRISPR-Cas systems. *Nat Biotechnol,* 2013, vol. 31, 684-686
- **MCCLURE, C. ; COLE, K.L. ; WULFF, P. ; KLUGMANN, M. ; MURRAY, A.J.** Production and titering of recombinant adeno-associated viral vectors. *J Vis Exp,* 2011, e3348
- **KONERMANN, S. et al.** Optical control of mammalian endogenous transcription and epigenetic states. *Nature,* 2013, vol. 500, 472-476
- **BANKER, G. ; GOSLIN, K.** Developments in neuronal cell culture. *Nature,* 1988, vol. 336, 185-186
- **SWIECH, L. et al.** CLIP-170 and IQGAP1 cooperatively regulate dendrite morphology. *J Neurosci,* 2011, vol. 31, 4555-4568
- **SHOLL, D.A.** Dendritic organization in the neurons of the visual and motor cortices of the cat. *J Anat,* 1953, vol. 87, 387-406
- **RAN, F.A. et al.** Genome engineering using the CRISPR-Cas9 system. *Nature protocols,* 2013, vol. 8, 2281-2308
- **PICELLI, S. et al.** Smart-seq2 for sensitive full-length transcriptome profiling in single cells. *Nature methods,* 2013, vol. 10, 1096-1098
- **FUJITA, P.A. et al.** The UCSC Genome Browser database: update 2011. *Nucleic acids research,* 2011, vol. 39, D876-882
- **TZINGOUNIS, A.V. et al.** The KCNQ5 potassium channel mediates a component of the afterhyperpolarization current in mouse hippocampus. *Proceedings of the National Academy of Sciences of the United States of America,* 2010, vol. 107, 10232-10237
- **BANKER G ; GOSLIN K.** Developments in neuronal cell culture. *Nature,* 10 November 1988, vol. 336 (6195), 185-6
- **BEDELL, V.M. et al.** In vivo genome editing using a high-efficiency TALEN system. *Nature,* 2012, vol. 491, 114-U133
- **BHAYA, D. ; DAVISON, M. ; BARRANGOU, R.** CRISPR-Cas systems in bacteria and archaea: versatile small RNAs for adaptive defense and regulation. *Annu Rev Genet,* 2011, vol. 45, 273-297
- **BOBIS-WOZOWICZ, S. ; OSIAK, A. ; RAHMAN, S.H. ; CATHOMEN, T.** Targeted genome editing in pluripotent stem cells using zinc-finger nucleases. *Methods,* 2011, vol. 53, 339-346
- **BOCH, J. et al.** Breaking the code of DNA binding specificity of TAL-type III effectors. *Science,* 2009, vol. 326, 1509-1512
- **BOGENHAGEN, D.F. ; BROWN, D.D.** Nucleotide sequences in Xenopus 5S DNA required for transcription termination. *Cell,* 1981, vol. 24, 261-270

- **BULTMANN, S. et al.** Targeted transcriptional activation of silent oct4 pluripotency gene by combining designer TALEs and inhibition of epigenetic modifiers. *Nucleic Acids Res,* 2012, vol. 40, 5368-5377
- **CARLSON, D.F. et al.** Efficient TALEN-mediated gene knockout in livestock. *Proc Natl Acad Sci U S A,* 2012, vol. 109, 17382-17387
- **CHEN, F.Q. et al.** High-frequency genome editing using ssDNA oligonucleotides with zinc-finger nucleases. *Nat Methods,* 2011, vol. 8, 753-U796
- **CHO, S.W. ; KIM, S. ; KIM, J.M. ; KIM, J.S.** Targeted genome engineering in human cells with the Cas9 RNA-guided endonuclease. *Nat Biotechnol,* 2013, vol. 31, 230-232
- **CHRISTIAN, M. et al.** Targeting DNA double-strand breaks with TAL effector nucleases. *Genetics,* 2010, vol. 186, 757-761
- **CONG, L. et al.** Multiplex genome engineering using CRISPR-Cas systems. *Science,* 2013, vol. 339, 819-823
- **DEVEAU, H. ; GARNEAU, J.E. ; MOINEAU, S.** CRISPR-Cas system and its role in phage-bacteria interactions. *Annu Rev Microbiol,* 2010, vol. 64, 475-493
- **DING, Q. et al.** A TALEN genome-editing system for generating human stem cell-based disease models. *Cell Stem Cell,* 2013, vol. 12, 238-251
- **GARNEAU, J.E. et al.** The CRISPR-Cas bacterial immune system cleaves bacteriophage and plasmid DNA. *Nature,* 2010, vol. 468, 67-71
- **GASIUNAS, G. ; BARRANGOU, R. ; HORVATH, P. ; SIKSNYS, V.** Cas9-crRNA ribonucleoprotein complex mediates specific DNA cleavage for adaptive immunity in bacteria. *Proc Natl Acad Sci U S A,* 2012, vol. 109, E2579-2586
- **GEURTS, A.M. et al.** Knockout Rats via Embryo Microinjection of Zinc-Finger Nucleases. *Science,* 2009, vol. 325, 433-433
- **GRAY SJ ; FOTI SB ; SCHWARTZ JW ; BACHABOINA L ; TAYLOR-BLAKE B ; COLEMAN J ; EHLERS MD ; ZYLKA MJ ; MCCOWN TJ ; SAMULSKI RJ.** Optimizing promoters for recombinant adeno-associated virus-mediated gene expression in the peripheral and central nervous system using self-complementary vectors. *Hum Gene Ther.,* September 2011, vol. 22 (9), 1143-53
- **GUSCHIN, D.Y. et al.** A rapid and general assay for monitoring endogenous gene modification. *Methods Mol Biol,* 2010, vol. 649, 247-256
- **HASTY, P. ; RIVERA-PEREZ, J. ; BRADLEY, A.** The length of homology required for gene targeting in embryonic stem cells. *Mol Cell Biol,* 1991, vol. 11, 5586-5591
- **HORVATH, P. ; BARRANGOU, R.** CRISPR-Cas, the immune system of bacteria and archaea. *Science,* 2010, vol. 327, 167-170
- **HSU, P.D. ; ZHANG, F.** Dissecting neural function using targeted genome engineering technologies. *ACS Chem Neurosci,* 2012, vol. 3, 603-610
- **HWANG, W.Y. et al.** Efficient genome editing in zebrafish using a CRISPR-Cas system. *Nat Biotechnol,* 2013, vol. 31, 227-229
- **JIANG, W. ; BIKARD, D. ; COX, D. ; ZHANG, F. ; MARRAFFINI, L.A.** RNA-guided editing of bacterial genomes using CRISPR-Cas systems. *Nat Biotechnol,* 2013, vol. 31, 233-239
- **JINEK, M. et al.** RNA-programmed genome editing in human cells. *eLife,* 2013, vol. 2, e00471
- **KAPLITT, M.G. et al.** Safety and tolerability of gene therapy with an adeno-associated virus (AAV) borne GAD gene for Parkinson's disease: an open label, phase I trial. *Lancet,* 23 June 2007, vol. 369 (9579), 2097-105
- **LEVITT N. ; BRIGGS D. ; GIL A ; PROUDFOOT N.J.** Definition of an efficient synthetic poly(A) site. *Genes Dev.,* 1989, vol. 3, 1019-1025
- **LIU D ; FISCHER I.** Two alternative promoters direct neuron-specific expression of the rat microtubule-associated protein 1B gene. *J Neurosci.,* 15 August 1996, vol. 16 (16), 5026-36
- **LOPES, V.S.** Retinal gene therapy with a large MYO7A cDNA using adeno-assocaited virus. *Gene Ther,* 24 January 2013
- **MAHFOUZ, M.M. et al.** De novo-engineered transcription activator-like effector (TALE) hybrid nuclease with novel DNA binding specificity creates double-strand breaks. *Proc Natl Acad Sci U S A,* 2011, vol. 108, 2623-2628
- **MAKAROVA, K.S. et al.** Evolution and classification of the CRISPR-Cas systems. *Nat Rev Microbiol,* 2011, vol. 9, 467-477
- **MCCLURE C ; COLE KL ; WULFF P ; KLUGMANN M ; MURRAY AJ.** Production and titering of recombinant adeno-associated viral vectors. *J Vis Exp.,* 27 November 2011, vol. 57, e3348
- **MICHAELIS, L.M., MAUD.** Die kinetik der invertinwirkung. *Biochem. z,* 1913
- **MILLER, J.C. et al.** An improved zinc-finger nuclease architecture for highly specific genome editing. *Nat Biotechnol,* 2007, vol. 25, 778-785
- **MILLER, J.C. et al.** A TALE nuclease architecture for efficient genome editing. *Nat Biotechnol,* 2011, vol. 29, 143-148
- **MOSCOU, M.J. ; BOGDANOVE, A.J.** A simple cipher governs DNA recognition by TAL effectors. *Science,* 2009, vol. 326, 1501
- **PORTEUS, M.H. ; BALTIMORE, D.** Chimeric nucleases stimulate gene targeting in human cells. *Science,* 2003, vol. 300, 763
- **MUSSOLINO, C. et al.** A novel TALE nuclease scaffold enables high genome editing activity in combination with low toxicity. *Nucleic acids research,* 2011, vol. 39, 9283-9293

- **NATHWANI, A.C. et al.** Adenovirus-associated virus vector-mediated gene transfer in hemophilia B. *N Engl J Med.,* 22 December 2011, vol. 365 (25), 2357-65
- **OLIVEIRA, T.Y. et al.** Translocation capture sequencing: a method for high throughput mapping of chromosomal rearrangements. *J Immunol Methods,* 2012, vol. 375, 176-181
- **PEREZ, E.E. et al.** Establishment of HIV-1 resistance in CD4(+) T cells by genome editing using zinc-finger nucleases. *Nat Biotechnol,* 2008, vol. 26, 808-816
- **REYON, D. et al.** FLASH assembly of TALENs for high-throughput genome editing. *Nat Biotechnol,* 2012, vol. 30, 460-465
- **SALEH-GOHARI, N. ; HELLEDAY, T.** Conservative homologous recombination preferentially repairs DNA double-strand breaks in the S phase of the cell cycle in human cells. *Nucleic Acids Res,* 2004, vol. 32, 3683-3688
- **SANDER, J.D. et al.** Selection-free zinc-finger-nuclease engineering by context-dependent assembly (CoDA). *Nat Methods,* 2011, vol. 8, 67-69
- **SANJANA, N.E. et al.** A transcription activator-like effector toolbox for genome engineering. *Nat Protoc,* 2012, vol. 7, 171-192
- **SAPRANAUSKAS, R. et al.** The Streptococcus thermophilus CRISPR-Cas system provides immunity in Escherichia coli. *Nucleic Acids Res,* 2011, vol. 39, 9275-9282
- **SHEN, B. et al.** Generation of gene-modified mice via Cas9/RNA-mediated gene targeting. *Cell Res,* 2013, vol. 23, 720-723
- **SMITHIES, O. ; GREGG, R.G. ; BOGGS, S.S. ; KORALEWSKI, M.A. ; KUCHERLAPATI, R.S.** Insertion of DNA sequences into the human chromosomal beta-globin locus by homologous recombination. *Nature,* 1985, vol. 317, 230-234
- **SOLDNER, F. et al.** Generation of isogenic pluripotent stem cells differing exclusively at two early onset Parkinson point mutations. *Cell,* 2011, vol. 146, 318-331
- **TAKASU, Y. et al.** Targeted mutagenesis in the silkworm Bombyx mori using zinc finger nuclease mRNA injection. *Insect Biochem Molec,* 2010, vol. 40, 759-765
- **THOMAS, K.R. ; FOLGER, K.R. ; CAPECCHI, M.R.** High frequency targeting of genes to specific sites in the mammalian genome. *Cell,* 1986, vol. 44, 419-428
- **TUSCHL, T.** Expanding small RNA interference. *Nat Biotechnol,* 2002, vol. 20, 446-448
- **URNOV, F.D. ; REBAR, E.J. ; HOLMES, M.C. ; ZHANG, H.S. ; GREGORY, P.D.** Genome editing with engineered zinc finger nucleases. *Nat Rev Genet,* 2010, vol. 11, 636-646
- **VALTON, J. et al.** Overcoming transcription activator-like effector (TALE) DNA binding domain sensitivity to cytosine methylation. *J Biol Chem,* 2012, vol. 287, 38427-38432
- **WANG, H. et al.** One-Step Generation of Mice Carrying Mutations in Multiple Genes by CRISPR-Cas-Mediated Genome Engineering. *Cell,* 2013, vol. 153, 910-918
- **WATANABE, T. et al.** Non-transgenic genome modifications in a hemimetabolous insect using zinc-finger and TAL effector nucleases. *Nat Commun,* 2012, 3
- **WILSON, E.B.** Probable inference, the law of succession, and statistical inference. *J Am Stat Assoc,* 1927, vol. 22, 209-212
- **WOOD, A.J. et al.** Targeted genome editing across species using ZFNs and TALENs. *Science,* 2011, vol. 333, 307
- **WU, S. ; YING, G.X. ; WU, Q. ; CAPECCHI, M.R.** A protocol for constructing gene targeting vectors: generating knockout mice for the cadherin family and beyond. *Nat Protoc,* 2008, vol. 3, 1056-1076
- **ZHANG, F. et al.** Efficient construction of sequence-specific TAL effectors for modulating mammalian transcription. *Nat Biotechnol,* 2011, vol. 29, 149-153